(19) Europäisches Patentamt
European Patent Office
Office européen des brevets

(11) EP 4 361 156 A1

(12) # EUROPEAN PATENT APPLICATION

published in accordance with Art. 153(4) EPC

(43) Date of publication:
**01.05.2024 Bulletin 2024/18**

(21) Application number: **22820298.2**

(22) Date of filing: **09.06.2022**

(51) International Patent Classification (IPC):
***C07D 495/14*** *(2006.01)* ***C07D 519/00*** *(2006.01)*
***C09K 11/06*** *(2006.01)* ***H01L 51/50*** *(2006.01)*

(52) Cooperative Patent Classification (CPC):
**C07D 495/14; C07D 519/00; C09K 11/06; H10K 50/00**

(86) International application number:
**PCT/JP2022/023247**

(87) International publication number:
**WO 2022/260119 (15.12.2022 Gazette 2022/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.06.2021 JP 2021097473**
**15.09.2021 JP 2021150650**
**15.04.2022 JP 2022067595**

(71) Applicant: **Idemitsu Kosan Co.,Ltd.**
**Tokyo 100-8321 (JP)**

(72) Inventors:
- **YASUKAWA Keiichi**
  **Tokyo 100-8321 (JP)**
- **MATSUMOTO Hisato**
  **Tokyo 100-8321 (JP)**
- **TERADA Kazuki**
  **Tokyo 100-8321 (JP)**
- **IIDA Maiko**
  **Tokyo 100-8321 (JP)**

(74) Representative: **Gille Hrabal Partnerschaftsgesellschaft mbB Patentanwälte**
**Brucknerstraße 20**
**40593 Düsseldorf (DE)**

(54) **COMPOUND, MATERIAL FOR ORGANIC ELECTROLUMINESCENT ELEMENTS, ORGANIC ELECTROLUMINESCENT ELEMENT, AND ELECTRONIC DEVICE**

(57) A compound represented by a formula (1) below. In the formula (1): CN is a cyano group; $D_{11}$ and $D_{12}$ are each independently a group represented by a formula (11), (12) or (13); at least one $D_{11}$ is a group represented by the formula (12) or (13); and R is, for instance, a hydrogen atom or an aryl group.

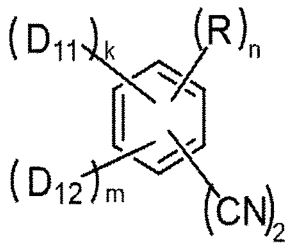

(1)

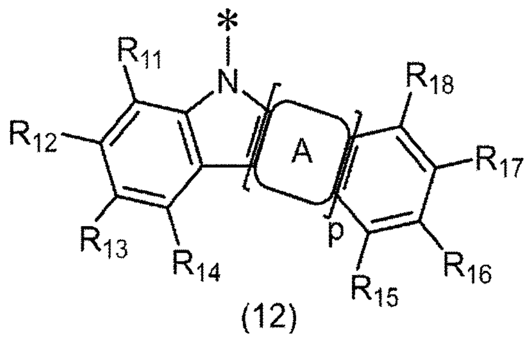

(12)

EP 4 361 156 A1

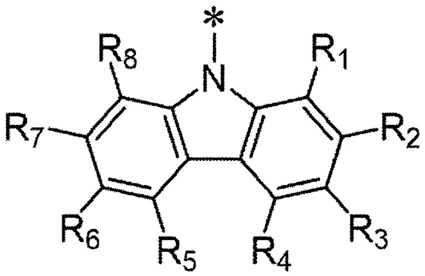

(11)

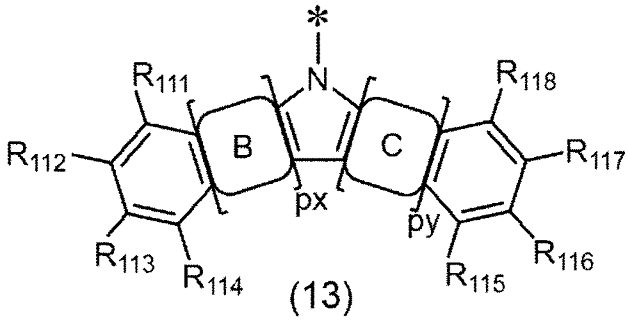

(13)

## Description

### TECHNICAL FIELD

The present invention relates to a compound, an organic-electroluminescence-device material, an organic electroluminescence device, and an electronic device.

### BACKGROUND ART

When a voltage is applied to an organic electroluminescence device (hereinafter, occasionally referred to as "organic EL device"), holes are injected from an anode and electrons are injected from a cathode into an emitting layer. The injected holes and electrons are recombined in the emitting layer to form excitons. Specifically, according to the electron spin statistics theory, singlet excitons and triplet excitons are generated at a ratio of 25%:75%.

A fluorescent organic EL device using light emission from singlet excitons has been applied to a full-color display such as a mobile phone and a television set, but an internal quantum efficiency is said to be at a limit of 25%. Studies have thus been made to improve performance of the organic EL device.

For instance, the organic EL device is expected to emit light more efficiently using triplet excitons in addition to singlet excitons. In view of the above, a highly-efficient fluorescent organic EL device using thermally activated delayed fluorescence (hereinafter sometimes simply referred to as "delayed fluorescence") has been proposed and studied.

A thermally activated delayed fluorescence (TADF) mechanism uses such a phenomenon in which inverse intersystem crossing from triplet excitons to singlet excitons thermally occurs when a material having a small energy difference (ΔST) between singlet energy level and triplet energy level is used. Thermally activated delayed fluorescence is explained in "Yuki Hando-tai no Debaisu Bussei (Device Physics of Organic Semiconductors)" (edited by ADACHI Chihaya, published by Kodansha, issued on April 1, 2012, on pages 261-268).

As a compound exhibiting TADF properties (hereinafter also referred to as a TADF compound), for instance, a compound in which a donor moiety and an acceptor moiety are bonded in a molecule is known.

Patent Literature 1, Patent Literature 2, Patent Literature 3, and Patent Literature 4 are listed as literatures regarding organic EL devices and compounds used for the organic EL devices.

### CITATION LIST

### PATENT LITERATURE(S)

Patent Literature 1: International Publication No. WO 2014/208698
Patent Literature 2: International Publication No. WO 2019/195104
Patent Literature 3: International Publication No. WO 2019/190235
Patent Literature 4: International Publication No. WO 2021/066059


### SUMMARY OF THE INVENTION

### PROBLEM(S) TO BE SOLVED BY THE INVENTION

Further improvement in performance of the organic EL device is desired for improving performance of an electronic device such as a display. The performance of the organic EL device is evaluable in terms of, for instance, luminance, emission wavelength, chromaticity, luminous efficiency, drive voltage, and lifetime. Factors for improving luminous efficiency of the organic EL device are exemplified by use of a compound having a high photoluminescence quantum yield (PLQY). The organic EL device is also desired to have a longer lifetime.

An object of the invention is to provide a compound having a high PLQY. Another object of the invention is to provide an organic-electroluminescence-device material and an organic electroluminescence device each containing a high PLQY, and an electronic device including the organic electroluminescence device. Still another object of the invention is to provide a compound with which high performance, especially at least one of high efficiency or a long lifetime, of the organic electroluminescence device is achieved. A further object of the invention is to provide an organic electroluminescence device whose high performance, especially at least one of high efficiency or a long lifetime, is achieved, and to provide an electronic device including the organic electroluminescence device.

MEANS FOR SOLVING THE PROBLEM(S)

According to an aspect of the invention, there is provided a compound represented by a formula (1) below.

[Formula 1]

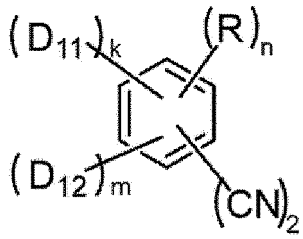

(1)

In the formula (1):

CN is a cyano group;
$D_{11}$ and $D_{12}$ are each independently a group represented by a formula (11), (12) or (13) below; at least one $D_{11}$ is a group represented by the formula (12) or (13);
R is each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{908}$, a group represented by $-COOR_{909}$, a cyano group, a nitro group, a group represented by $-P(=O)(R_{931})(R_{932})$, a group represented by $-Ge(R_{933})(R_{934})(R_{935})$, a group represented by $-B(R_{936})(R_{937})$, a substituted or unsubstituted aryl group having 6 to 50 ring carbon atoms, or a substituted or unsubstituted heterocyclic group having 5 to 50 ring atoms;
at least one R is a substituent and R as at least one substituent is bonded to a benzene ring in the formula (1) via carbon-carbon bonding;
k is 1 or 2;
m is 0, 1, or 2;
n is 1, 2, or 3;
$k + m + n = 4$ is satisfied;
when k is 2, a plurality of $D_{11}$ are mutually the same or different;
when m is 2, a plurality of $D_{12}$ are mutually the same or different; and
when n is 2 or 3, a plurality of R are mutually the same or different.

[Formula 2]

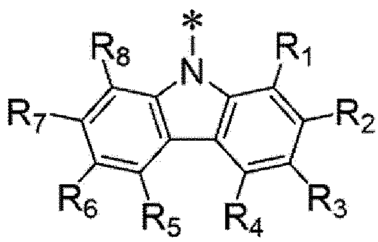

(11)

[Formula 3]

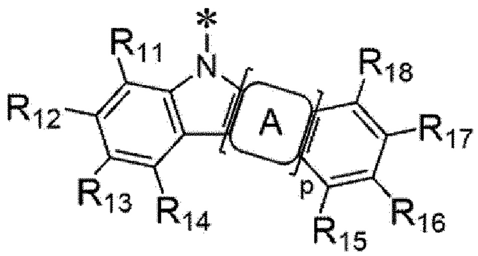

(12)

[Formula 4]

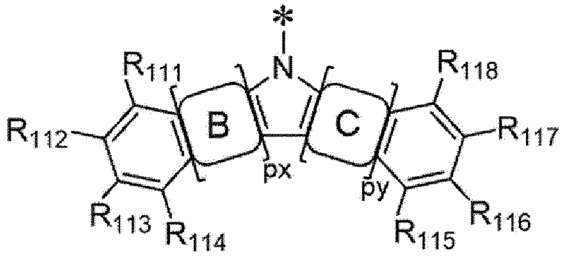

(13)

In the formula (12), at least one combination of adjacent two or more of $R_{11}$ to $R_{18}$ are mutually bonded to form a substituted or unsubstituted monocyclic ring, mutually bonded to form a substituted or unsubstituted fused ring, or not mutually bonded,

in the formula (13), at least one combination of adjacent two or more of $R_{111}$ to $R_{118}$ are mutually bonded to form a substituted or unsubstituted monocyclic ring, mutually bonded to form a substituted or unsubstituted fused ring, or not mutually bonded,

$R_1$ to $R_8$ in the formula (11), $R_{11}$ to $R_{18}$ forming neither the substituted or unsubstituted monocyclic ring nor the substituted or unsubstituted fused ring in the formula (12), and $R_{111}$ to $R_{118}$ forming neither the substituted or unsubstituted monocyclic ring nor the substituted or unsubstituted fused ring in the formula (13) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{908}$, a group represented by $-COOR_{909}$, a halogen atom, a cyano group, a nitro group, a group represented by $-P(=O)(R_{931})(R_{932})$, a group represented by $-Ge(R_{933})(R_{934})(R_{935})$, a group represented by $-B(R_{936})(R_{937})$, a substituted or unsubstituted aryl group having 6 to 50 ring carbon atoms, or a substituted or unsubstituted heterocyclic group having 5 to 50 ring atoms;

in the formulae (12) and (13):

a ring A, a ring B, and a ring C are each independently a cyclic structure selected from the group consisting of cyclic structures represented by formulae (14) and (15) below;

the ring A, the ring B, and the ring C are fused with adjacent rings at any positions;

p, px and py are each independently 1, 2, 3, or 4;

when p is 2, 3, or 4, a plurality of rings A are mutually the same or different;

when px is 2, 3, or 4, a plurality of rings B are mutually the same or different;

when py is 2, 3, or 4, a plurality of rings C are mutually the same or different; and

at least one $D_{11}$ is a group represented by the formula (12) or (13); p is 4 in the formula (12) when the at least one $D_{11}$ is represented by the formula (12), four rings A include two cyclic structures each represented by the formula (14) below and two cyclic structures each represented by the formula (15) below; and px and py are each 2 in the formula (13) when the at least one $D_{11}$ is represented by the formula (13), two rings B include one cyclic structure represented by the formula (14) below and one cyclic structure represented by the formula (15)

below, and two rings C include one cyclic structure represented by the formula (14) below and one cyclic structure represented by the formula (15) below; and
* in the formulae (11) to (13) each represents a bonding position to the benzene ring in the formula (1).

[Formula 5]

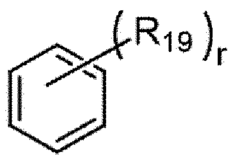

(14)

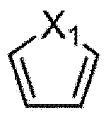

(15)

In the formula (14):

r is 0, 2, or 4;
a combination of a plurality of $R_{19}$ are mutually bonded to form a substituted or unsubstituted monocyclic ring, mutually bonded to form a substituted or unsubstituted fused ring, or not mutually bonded,
in the formula (15), $X_1$ is a sulfur atom or an oxygen atom;
$R_{19}$ forming neither the substituted or unsubstituted monocyclic ring nor the substituted or unsubstituted fused ring is a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{903}$, a group represented by $-COOR_{909}$, a halogen atom, a cyano group, a nitro group, a group represented by $-P(=O)(R_{931})(R_{932})$, a group represented by $-Ge(R_{933})(R_{934})(R_{935})$, a group represented by $-B(R_{936})(R_{937})$, a substituted or unsubstituted aryl group having 6 to 50 ring carbon atoms, or a substituted or unsubstituted heterocyclic group having 5 to 50 ring atoms;
a plurality of $R_{19}$ are mutually the same or different;
a plurality of $X_1$ are mutually the same or different; and
$D_{11}$ that is a group represented by the formula (13) satisfies at least one of a condition (Pv1), a condition (Pv2), or a condition (Pv3),
condition (Pv1): when k is 2, at least one of $X_1$ in a cyclic structure represented by the formula (15) as the ring B or $X_1$ in a cyclic structure represented by the formula (15) as the ring C is an oxygen atom,
condition (Pv2): when k is 2, two $D_{11}$ are mutually different, and
condition (Pv3): when n is 3, $X_1$ in a cyclic structure represented by the formula (15) as the ring B and $X_1$ in a cyclic structure represented by the formula (15) as the ring C are each independently a sulfur atom or an oxygen atom.

In the formulae, $R_{901}$, $R_{902}$, $R_{903}$, $R_{904}$, $R_{905}$, $R_{906}$, $R_{907}$, $R_{908}$, $R_{909}$, $R_{931}$, $R_{932}$, $R_{933}$, $R_{934}$, $R_{935}$, $R_{936}$, and $R_{937}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{908}$ are present, the plurality of $R_{908}$ are mutually the same or different;
when a plurality of $R_{909}$ are present, the plurality of $R_{909}$ are mutually the same or different;
when a plurality of $R_{931}$ are present, the plurality of $R_{931}$ are mutually the same or different;
when a plurality of $R_{932}$ are present, the plurality of $R_{932}$ are mutually the same or different;
when a plurality of Rsss are present, the plurality of $R_{933}$ are mutually the same or different;

when a plurality of $R_{934}$ are present, the plurality of $R_{934}$ are mutually the same or different;
when a plurality of $R_{935}$ are present, the plurality of $R_{935}$ are mutually the same or different;
when a plurality of $R_{936}$ are present, the plurality of $R_{936}$ are mutually the same or different; and
when a plurality of $R_{937}$ are present, the plurality of $R_{937}$ are mutually the same or different.

According to another aspect of the invention, there is provided an organic-electroluminescence-device material containing the compound according to the above aspect of the invention.

According to still another aspect of the invention, there is provided an organic electroluminescence device including an anode, a cathode, and an organic layer containing, as a compound M2, the compound according to the above aspect of the invention.

According to a further aspect of the invention, there is provided an electronic device including the organic electroluminescence device according to the above aspect of the invention.

According to a still further aspect of the invention, a compound having a high PLQY is provided. According to a still further aspect of the invention, there is provided an organic-electroluminescence-device material or an organic electroluminescence device containing a compound having a high PLQY. According to a still further aspect of the invention, an electronic device including the organic electroluminescence device is provided. According to a still further aspect of the invention, there is provided a compound with which high performance, especially at least one of high efficiency or a long lifetime, of the organic electroluminescence device is achieved. According to a still further aspect of the invention, there are provided an organic electroluminescence device whose high performance, especially at least one of high efficiency or a long lifetime, is achieved, and an electronic device including the organic electroluminescence device.

BRIEF EXPLANATION OF DRAWING(S)

Fig. 1 schematically depicts an apparatus for measuring transient PL.
Fig. 2 illustrates an example of decay curves of the transient PL.
Fig. 3 schematically illustrates an exemplary arrangement of an organic electroluminescence device according to a third exemplary embodiment of the invention.
Fig. 4 schematically illustrates a relationship in energy level and energy transfer between a compound M1 and a compound M2 in an emitting layer of an exemplary organic electroluminescence device according to the third exemplary embodiment of the invention.
Fig. 5 schematically illustrates a relationship in energy level and energy transfer between the compound M1, the compound M2 and a compound M3 in an emitting layer of an exemplary organic electroluminescence device according to a fourth exemplary embodiment of the invention.
Fig. 6 schematically illustrates a relationship in energy level and energy transfer between the compound M2 and the compound M3 in an emitting layer of an exemplary organic electroluminescence device according to a fifth exemplary embodiment of the invention.

DESCRIPTION OF EMBODIMENT(S)

Definitions

Herein, a hydrogen atom includes isotope having different numbers of neutrons, specifically, protium, deuterium and tritium.

In chemical formulae herein, it is assumed that a hydrogen atom (i.e. protium, deuterium and tritium) is bonded to each of bondable positions that are not annexed with signs "R" or the like or "D" representing a deuterium.

Herein, the ring carbon atoms refer to the number of carbon atoms among atoms forming a ring of a compound (e.g., a monocyclic compound, fused-ring compound, crosslinking compound, carbon ring compound, and and heterocyclic compound) in which the atoms are bonded to each other to form the ring. When the ring is substituted by a substituent(s), carbon atom(s) contained in the substituent(s) is not counted in the ring carbon atoms. Unless specifically described, the same applies to the "ring carbon atoms" described later. For instance, a benzene ring has 6 ring carbon atoms, a naphthalene ring has 10 ring carbon atoms, a pyridine ring has 5 ring carbon atoms, and a furan ring 4 ring carbon atoms. For instance, a 9,9-diphenylfluorenyl group has 13 ring carbon atoms and 9,9'-spirobifluorenyl group has 25 ring carbon atoms.

When a benzene ring is substituted by a substituent (e.g., an alkyl group), the number of carbon atoms of the alkyl group is not counted in the number of the ring carbon atoms of the benzene ring. Accordingly, the benzene ring

substituted by an alkyl group has 6 ring carbon atoms. When a naphthalene ring is substituted by a substituent in a form of, for instance, an alkyl group, the number of carbon atoms of the alkyl group is not counted in the number of the ring carbon atoms of the naphthalene ring. Accordingly, the naphthalene ring substituted by an alkyl group has 10 ring carbon atoms.

Herein, the ring atoms refer to the number of atoms forming a ring of a compound (e.g., a monocyclic compound, fused-ring compound, cross-linking compound, carbon ring compound, and heterocyclic compound) in which the atoms are bonded to each other to form the ring (e.g., monocyclic ring, fused ring, and ring assembly). Atom(s) not forming the ring (e.g., hydrogen atom(s) for saturating the valence of the atom which forms the ring) and atom(s) in a substituent by which the ring is substituted are not counted as the ring atoms. Unless otherwise specified, the same applies to the "ring atoms" described later. For instance, a pyridine ring has 6 ring atoms, a quinazoline ring has 10 ring atoms, and a furan ring has 5 ring atoms. For instance, the number of hydrogen atom(s) bonded to a pyridine ring or the number of atoms forming a substituent is not counted in the number of the ring atoms of the pyridine ring. Accordingly, a pyridine ring bonded to a hydrogen atom(s) or a substituent(s) has 6 ring atoms. For instance, the hydrogen atom(s) bonded to carbon atom(s) of a quinazoline ring or the atoms forming a substituent are not counted as the quinazoline ring atoms. Accordingly, a quinazoline ring bonded to hydrogen atom(s) or a substituent(s) has 10 ring atoms.

Herein, "XX to YY carbon atoms" in the description of "substituted or unsubstituted ZZ group having XX to YY carbon atoms" represent carbon atoms of an unsubstituted ZZ group and do not include carbon atoms of a substituent(s) of the substituted ZZ group. Herein, "YY" is larger than "XX," "XX" representing an integer of 1 or more and "YY" representing an integer of 2 or more.

Herein, "XX to YY atoms" in the description of "substituted or unsubstituted ZZ group having XX to YY atoms" represent atoms of an unsubstituted ZZ group and does not include atoms of a substituent(s) of the substituted ZZ group. Herein, "YY" is larger than "XX," "XX" representing an integer of 1 or more and "YY" representing an integer of 2 or more.

Herein, an unsubstituted ZZ group refers to an "unsubstituted ZZ group" in a "substituted or unsubstituted ZZ group," and a substituted ZZ group refers to a "substituted ZZ group" in a "substituted or unsubstituted ZZ group."

Herein, the term "unsubstituted" used in a "substituted or unsubstituted ZZ group" means that a hydrogen atom(s) in the ZZ group is not substituted with a substituent(s). The hydrogen atom(s) in the "unsubstituted ZZ group" is protium, deuterium, or tritium.

Herein, the term "substituted" used in a "substituted or unsubstituted ZZ group" means that at least one hydrogen atom in the ZZ group is substituted with a substituent. Similarly, the term "substituted" used in a "BB group substituted by AA group" means that at least one hydrogen atom in the BB group is substituted with the AA group.

Substituents Mentioned Herein

Substituents mentioned herein will be described below.

An "unsubstituted aryl group" mentioned herein has, unless otherwise specified herein, 6 to 50, preferably 6 to 30, and more preferably 6 to 18 ring carbon atoms.

An "unsubstituted heterocyclic group" mentioned herein has, unless otherwise specified herein, 5 to 50, preferably 5 to 30, and more preferably 5 to 18 ring atoms.

An "unsubstituted alkyl group" mentioned herein has, unless otherwise specified herein, 1 to 50, preferably 1 to 20, and more preferably 1 to 6 carbon atoms.

An "unsubstituted alkenyl group" mentioned herein has, unless otherwise specified herein, 2 to 50, preferably 2 to 20, and more preferably 2 to 6 carbon atoms.

An "unsubstituted alkynyl group" mentioned herein has, unless otherwise specified herein, 2 to 50, preferably 2 to 20, and more preferably 2 to 6 carbon atoms.

An "unsubstituted cycloalkyl group" mentioned herein has, unless otherwise specified herein, 3 to 50, preferably 3 to 20, and more preferably 3 to 6 ring carbon atoms.

An "unsubstituted arylene group" mentioned herein has, unless otherwise specified herein, 6 to 50, preferably 6 to 30, and more preferably 6 to 18 ring carbon atoms.

An "unsubstituted divalent heterocyclic group" mentioned herein has, unless otherwise specified herein, 5 to 50, preferably 5 to 30, and more preferably 5 to 18 ring atoms.

An "unsubstituted alkylene group" mentioned herein has, unless otherwise specified herein, 1 to 50, preferably 1 to 20, and more preferably 1 to 6 carbon atoms.

Substituted or Unsubstituted Aryl Group

Specific examples (specific example group G1) of the "substituted or unsubstituted aryl group" mentioned herein include unsubstituted aryl groups (specific example group G1A) below and substituted aryl groups (specific example group G1B). (Herein, an unsubstituted aryl group refers to an "unsubstituted aryl group" in a "substituted or unsubstituted

aryl group", and a substituted aryl group refers to a "substituted aryl group" in a "substituted or unsubstituted aryl group.") A simply termed "aryl group" herein includes both of an "unsubstituted aryl group" and a "substituted aryl group".

The "substituted aryl group" refers to a group derived by substituting at least one hydrogen atom in an "unsubstituted aryl group" with a substituent. Examples of the "substituted aryl group" include a group derived by substituting at least one hydrogen atom in the "unsubstituted aryl group" in the specific example group G1A below with a substituent, and examples of the substituted aryl group in the specific example group G1B below. It should be noted that the examples of the "unsubstituted aryl group" and the "substituted aryl group" mentioned herein are merely exemplary, and the "substituted aryl group" mentioned herein includes a group derived by further substituting a hydrogen atom bonded to a carbon atom of a skeleton of a "substituted aryl group" in the specific example group G1B below, and a group derived by further substituting a hydrogen atom of a substituent of the "substituted aryl group" in the specific example group G1B below.

Unsubstituted Aryl Group (Specific Example Group G1A):

a phenyl group, p-biphenyl group, m-biphenyl group, o-biphenyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-terphenyl-4-yl group, o-terphenyl-3-yl group, o-terphenyl-2-yl group, 1-naphthyl group, 2-naphthyl group, anthryl group, benzanthryl group, phenanthryl group, benzophenanthryl group, phenalenyl group, pyrenyl group, chrysenyl group, benzochrysenyl group, triphenylenyl group, benzotriphenylenyl group, tetracenyl group, pentacenyl group, fluorenyl group, 9,9'-spirobifluorenyl group, benzofluorenyl group, dibenzofluorenyl group, fluoranthenyl group, benzofluoranthenyl group, perylenyl group, and monovalent aryl group derived by removing one hydrogen atom from cyclic structures represented by formulae (TEMP-1) to (TEMP-15) below.

[Formula 6]

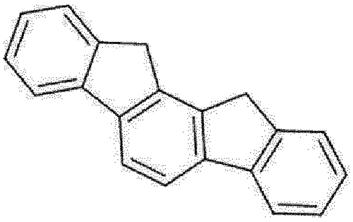
(TEMP-1)

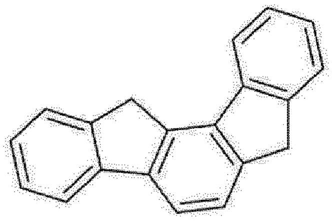
(TEMP-2)

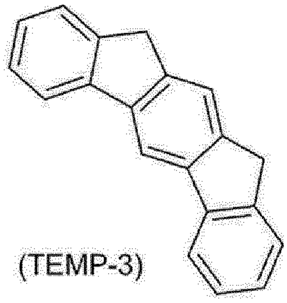
(TEMP-3)

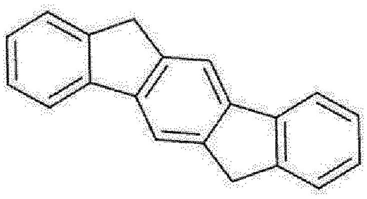
(TEMP-4)

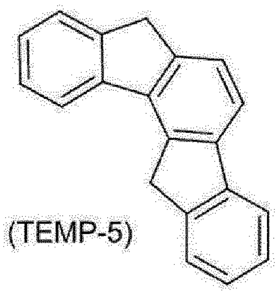
(TEMP-5)

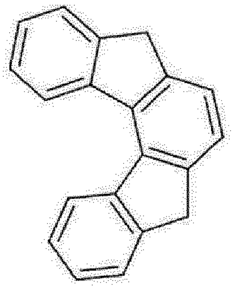
(TEMP-6)

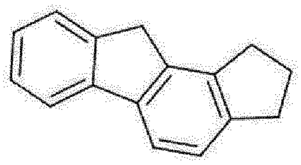
(TEMP-7)

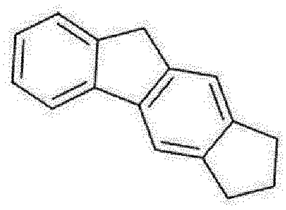
(TEMP-8)

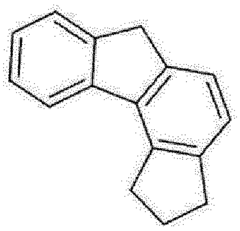
(TEMP-9)

[Formula 7]

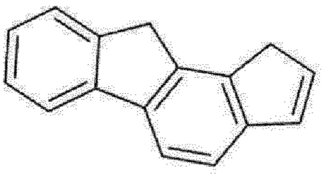
(TEMP-10)

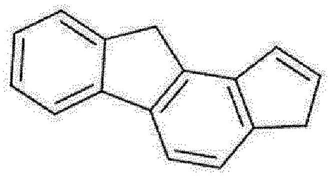
(TEMP-11)

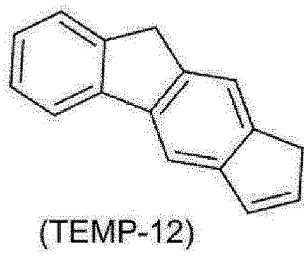
(TEMP-12)

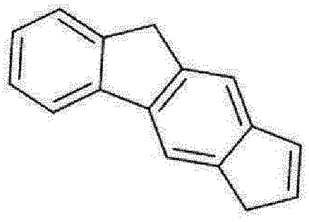
(TEMP-13)

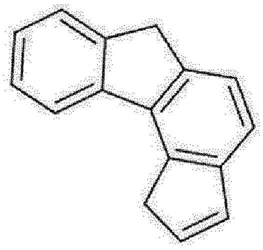
(TEMP-14)

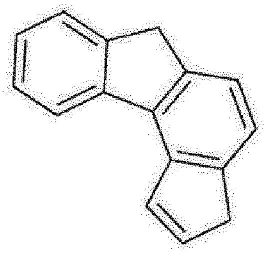
(TEMP-15)

Substituted Aryl Group (Specific Example Group G1B):
an o-tolyl group, m-tolyl group, p-tolyl group, para-xylyl group, meta-xylyl group, ortho-xylyl group, para-isopropylphenyl

group, meta-isopropylphenyl group, ortho-isopropylphenyl group, para-t-butylphenyl group, meta-t-butylphenyl group, ortho-t-butylphenyl group, 3,4,5-trimethylphenyl group, 9,9-dimethylfluorenyl group, 9,9-diphenylfluorenyl group, 9,9-bis(4-methylphenyl)fluorenyl group, 9,9-bis(4-isopropylphenyl)fluorenyl group, 9,9-bis(4-t-butylphenyl)fluorenyl group, cyanophenyl group, triphenylsilylphenyl group, trimethylsilylphenyl group, phenylnaphthyl group, naphthylphenyl group, and group derived by substituting at least one hydrogen atom of a monovalent group derived from one of the cyclic structures represented by the formulae (TEMP-1) to (TEMP-15) with a substituent.

Substituted or Unsubstituted Heterocyclic Group

The "heterocyclic group" mentioned herein refers to a cyclic group having at least one hetero atom in the ring atoms. Specific examples of the hetero atom include a nitrogen atom, oxygen atom, sulfur atom, silicon atom, phosphorus atom, and boron atom.

The "heterocyclic group" mentioned herein is a monocyclic group or a fused-ring group.

The "heterocyclic group" mentioned herein is an aromatic heterocyclic group or a non-aromatic heterocyclic group.

Specific examples (specific example group G2) of the "substituted or unsubstituted heterocyclic group" mentioned herein include unsubstituted heterocyclic groups (specific example group G2A) and substituted heterocyclic groups (specific example group G2B). (Herein, an unsubstituted heterocyclic group refers to an "unsubstituted heterocyclic group" in a "substituted or unsubstituted heterocyclic group," and a substituted heterocyclic group refers to a "substituted heterocyclic group" in a "substituted or unsubstituted heterocyclic group.") A simply termed "heterocyclic group" herein includes both of an "unsubstituted heterocyclic group" and a "substituted heterocyclic group."

The "substituted heterocyclic group" refers to a group derived by substituting at least one hydrogen atom in an "unsubstituted heterocyclic group" with a substituent. Specific examples of the "substituted heterocyclic group" include a group derived by substituting at least one hydrogen atom in the "unsubstituted heterocyclic group" in the specific example group G2A below with a substituent, and examples of the substituted heterocyclic group in the specific example group G2B below. It should be noted that the examples of the "unsubstituted heterocyclic group" and the "substituted heterocyclic group" mentioned herein are merely exemplary, and the "substituted heterocyclic group" mentioned herein includes a group derived by further substituting a hydrogen atom bonded to a ring atom of a skeleton of a "substituted heterocyclic group" in the specific example group G2B below, and a group derived by further substituting a hydrogen atom of a substituent of the "substituted heterocyclic group" in the specific example group G2B below.

The specific example group G2A includes, for instance, unsubstituted heterocyclic groups including a nitrogen atom (specific example group G2A1) below, unsubstituted heterocyclic groups including an oxygen atom (specific example group G2A2) below, unsubstituted heterocyclic groups including a sulfur atom (specific example group G2A3) below, and monovalent heterocyclic groups (specific example group G2A4) derived by removing a hydrogen atom from cyclic structures represented by formulae (TEMP-16) to (TEMP-33) below.

The specific example group G2B includes, for instance, substituted heterocyclic groups including a nitrogen atom (specific example group G2B1) below, substituted heterocyclic groups including an oxygen atom (specific example group G2B2) below, substituted heterocyclic groups including a sulfur atom (specific example group G2B3) below, and groups derived by substituting at least one hydrogen atom of the monovalent heterocyclic groups (specific example group G2B4) derived from the cyclic structures represented by formulae (TEMP-16) to (TEMP-33) below.

Unsubstituted Heterocyclic Groups Including Nitrogen Atom (Specific Example Group G2A1):

a pyrrolyl group, imidazolyl group, pyrazolyl group, triazolyl group, tetrazolyl group, oxazolyl group, isoxazolyl group, oxadiazolyl group, thiazolyl group, isothiazolyl group, thiadiazolyl group, pyridyl group, pyridazynyl group, pyrimidinyl group, pyrazinyl group, triazinyl group, indolyl group, isoindolyl group, indolizinyl group, quinolizinyl group, quinolyl group, isoquinolyl group, cinnolyl group, phthalazinyl group, quinazolinyl group, quinoxalinyl group, benzimidazolyl group, indazolyl group, phenanthrolinyl group, phenanthridinyl group, acridinyl group, phenazinyl group, carbazolyl group, benzocarbazolyl group, morpholino group, phenoxazinyl group, phenothiazinyl group, azacarbazolyl group, and diazacarbazolyl group.

Unsubstituted Heterocyclic Groups Including Oxygen Atom (Specific Example Group G2A2):

a furyl group, oxazolyl group, isoxazolyl group, oxadiazolyl group, xanthenyl group, benzofuranyl group, isobenzofuranyl group, dibenzofuranyl group, naphthobenzofuranyl group, benzoxazolyl group, benzisoxazolyl group, phenoxazinyl group, morpholino group, dinaphthofuranyl group, azadibenzofuranyl group, diazadibenzofuranyl group, azanaphthobenzofuranyl group, and diazanaphthobenzofuranyl group.

Unsubstituted Heterocyclic Groups Including Sulfur Atom (Specific Example Group G2A3):

a thienyl group, thiazolyl group, isothiazolyl group, thiadiazolyl group, benzothiophenyl group (benzothienyl group), isobenzothiophenyl group (isobenzothienyl group), dibenzothiophenyl group (dibenzothienyl group), naphthobenzothiophenyl group (nahthobenzothienyl group), benzothiazolyl group, benzisothiazolyl group, phenothiazinyl group, dinaphthothiophenyl group (dinaphthothienyl group), azadibenzothiophenyl group (azadibenzothienyl group), diazadibenzothiophenyl group (diazadibenzothienyl group), azanaphthobenzothiophenyl group (azanaphthobenzothienyl group), and diazanaphthobenzothiophenyl group (diazanaphthobenzothienyl group).

Monovalent Heterocyclic Groups Derived by Removing One Hydrogen Atom from Cyclic Structures Represented by Formulae (TEMP-16) to (TEMP-33) (Specific Example Group G2A4):

[Formula 8]

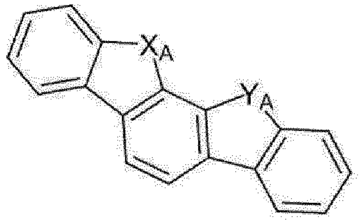
(TEMP-16)

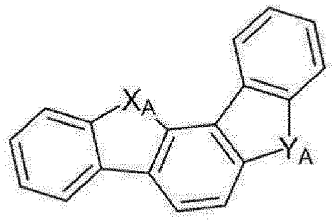
(TEMP-17)

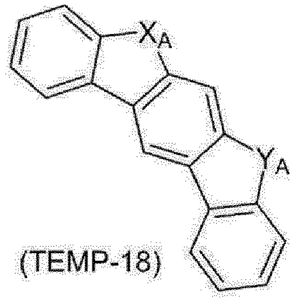
(TEMP-18)

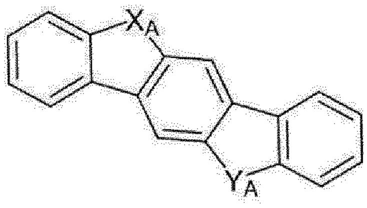
(TEMP-19)

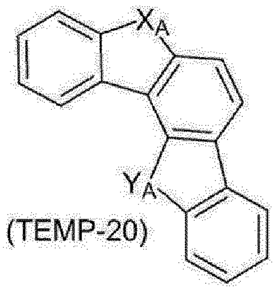
(TEMP-20)

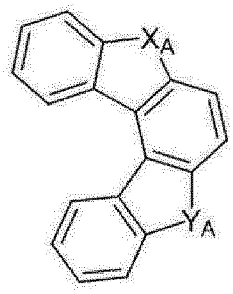
(TEMP-21)

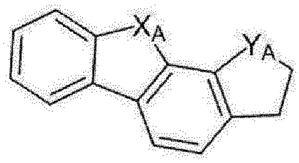
(TEMP-22)

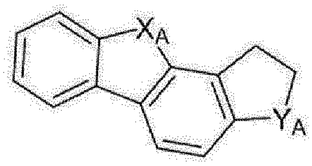
(TEMP-23)

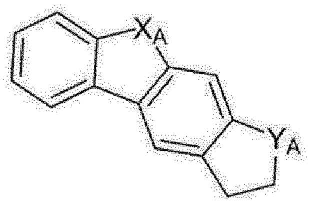
(TEMP-24)

[Formula 9]

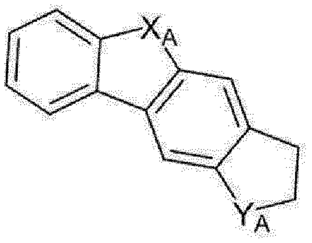
(TEMP-25)

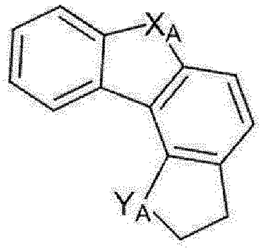
(TEMP-26)

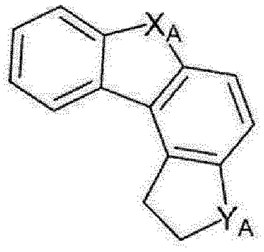
(TEMP-27)

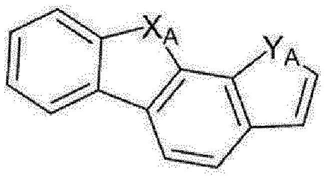
(TEMP-28)

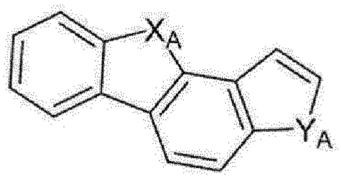
(TEMP-29)

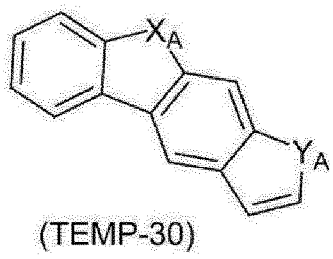
(TEMP-30)

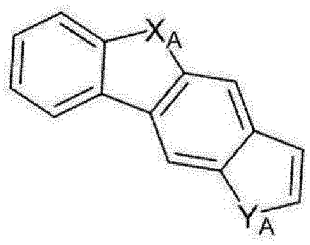
(TEMP-31)

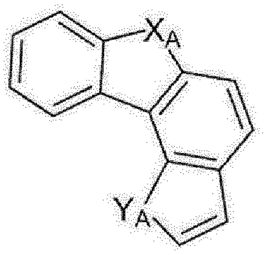
(TEMP-32)

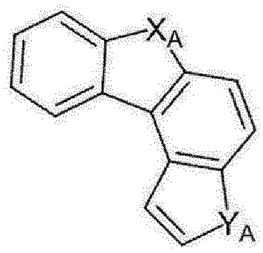
(TEMP-33)

In the formulae (TEMP-16) to (TEMP-33), $X_A$ and $Y_A$ are each independently an oxygen atom, a sulfur atom, NH or $CH_2$, with a proviso that at least one of $X_A$ or $Y_A$ is an oxygen atom, a sulfur atom, or NH.

When at least one of $X_A$ or $Y_A$ in the formulae (TEMP-16) to (TEMP-33) is NH or $CH_2$, the monovalent heterocyclic groups derived from the cyclic structures represented by the formulae (TEMP-16) to (TEMP-33) include a monovalent group derived by removing one hydrogen atom from NH or $CH_2$.

Substituted Heterocyclic Groups Including Nitrogen Atom (Specific Example Group G2B1):

(9-phenyl)carbazolyl group, (9-biphenylyl)carbazolyl group, (9-phenyl)phenylcarbazolyl group, (9-naphthyl)carbazolyl group, diphenylcarbazole-9-yl group, phenylcarbazole-9-yl group, methylbenzimidazolyl group, ethylbenzimidazolyl group, phenyltriazinyl group, biphenylyltriazinyl group, diphenyltriazinyl group, phenylquinazolinyl group, and biphenylquinazolinyl group.

Substituted Heterocyclic Groups Including Oxygen Atom (Specific Example Group G2B2):

phenyldibenzofuranyl group, methyldibenzofuranyl group, t-butyldibenzofuranyl group, and monovalent residue of spiro[9H-xanthene-9,9'-[9H]fluorene].

Substituted Heterocyclic Groups Including Sulfur Atom (Specific Example Group G2B3):

a phenyldibenzothiophenyl group, methyldibenzothiophenyl group, t-butyldibenzothiophenyl group, and monovalent residue of spiro[9H-thioxanthene-9,9'-[9H]fluorene].

Groups Obtained by Substituting at Least One Hydrogen Atom of Monovalent Heterocyclic Group Derived from Cyclic Structures Represented by Formulae (TEMP-16) to (TEMP-33) with Substituent (Specific Example Group G2B4):

The "at least one hydrogen atom of a monovalent heterocyclic group" means at least one hydrogen atom selected from a hydrogen atom bonded to a ring carbon atom of the monovalent heterocyclic group, a hydrogen atom bonded to a nitrogen atom of at least one of $X_A$ or $Y_A$ in a form of NH, and a hydrogen atom of one of $X_A$ and $Y_A$ in a form of a methylene group ($CH_2$).

Substituted or Unsubstituted Alkyl Group

Specific examples (specific example group G3) of the "substituted or unsubstituted alkyl group" mentioned herein include unsubstituted alkyl groups (specific example group G3A) and substituted alkyl groups (specific example group G3B) below. (Herein, an unsubstituted alkyl group refers to an "unsubstituted alkyl group" in a "substituted or unsubstituted alkyl group," and a substituted alkyl group refers to a "substituted alkyl group" in a "substituted or unsubstituted alkyl group.") A simply termed "alkyl group" herein includes both of an "unsubstituted alkyl group" and a "substituted alkyl group".

The "substituted alkyl group" refers to a group derived by substituting at least one hydrogen atom in an "unsubstituted alkyl group" with a substituent. Specific examples of the "substituted alkyl group" include a group derived by substituting at least one hydrogen atom of an "unsubstituted alkyl group" (specific example group G3A) below with a substituent, and examples of the substituted alkyl group (specific example group G3B) below. Herein, the alkyl group for the "unsubstituted alkyl group" refers to a chain alkyl group. Accordingly, the "unsubstituted alkyl group" include linear "unsubstituted alkyl group" and branched "unsubstituted alkyl group." It should be noted that the examples of the "unsubstituted alkyl group" and the "substituted alkyl group" mentioned herein are merely exemplary, and the "substituted alkyl group" mentioned herein includes a group derived by further substituting a hydrogen atom of a skeleton of the "substituted alkyl group" in the specific example group G3B, and a group derived by further substituting a hydrogen atom of a substituent of the "substituted alkyl group" in the specific example group G3B.

Unsubstituted Alkyl Group (Specific Example Group G3A):
a methyl group, ethyl group, n-propyl group, isopropyl group, n-butyl group, isobutyl group, s-butyl group, and t-butyl group.
Substituted Alkyl Group (Specific Example Group G3B):
a heptafluoropropyl group (including isomer thereof), pentafluoroethyl group, 2,2,2-trifluoroethyl group, and trifluoromethyl group.

Substituted or Unsubstituted Alkenyl Group

Specific examples (specific example group G4) of the "substituted or unsubstituted alkenyl group" mentioned herein include unsubstituted alkenyl groups (specific example group G4A) and substituted alkenyl groups (specific example group G4B). (Herein, an unsubstituted alkenyl group refers to an "unsubstituted alkenyl group" in a "substituted or unsubstituted alkenyl group," and a substituted alkenyl group refers to a "substituted alkenyl group" in a "substituted or unsubstituted alkenyl group.") A simply termed "alkenyl group" herein includes both of an "unsubstituted alkenyl group" and a "substituted alkenyl group".

The "substituted alkenyl group" refers to a group derived by substituting at least one hydrogen atom in an "unsubstituted alkenyl group" with a substituent. Specific examples of the "substituted alkenyl group" include an "unsubstituted alkenyl group" (specific example group G4A) substituted by a substituent, and examples of the substituted alkenyl group (specific example group G4B) below. It should be noted that the examples of the "unsubstituted alkenyl group" and the "substituted alkenyl group" mentioned herein are merely exemplary, and the "substituted alkenyl group" mentioned herein includes a group derived by further substituting a hydrogen atom of a skeleton of the "substituted alkenyl group" in the specific example group G4B with a substituent, and a group derived by further substituting a hydrogen atom of a substituent of the "substituted alkenyl group" in the specific example group G4B with a substituent.

Unsubstituted Alkenyl Group (Specific Example Group G4A):
a vinyl group, allyl group, 1-butenyl group, 2-butenyl group, and 3-butenyl group.
Substituted Alkenyl Group (Specific Example Group G4B):
a 1,3-butanedienyl group, 1-methylvinyl group, 1-methylallyl group, 1,1-dimethylallyl group, 2-methylallyl group, and 1,2-dimethylallyl group.

Substituted or Unsubstituted Alkynyl Group

Specific examples (specific example group G5) of the "substituted or unsubstituted alkynyl group" mentioned herein include unsubstituted alkynyl groups (specific example group G5A) below. (Herein, an unsubstituted alkynyl group refers to an "unsubstituted alkynyl group" in a "substituted or unsubstituted alkynyl group.") A simply termed "alkynyl group" herein includes both of "unsubstituted alkynyl group" and "substituted alkynyl group".

The "substituted alkynyl group" refers to a group derived by substituting at least one hydrogen atom in an

"unsubstituted alkynyl group" with a substituent. Specific examples of the "substituted alkynyl group" include a group derived by substituting at least one hydrogen atom of the "unsubstituted alkynyl group" (specific example group G5A) below with a substituent.

Unsubstituted Alkynyl Group (Specific Example Group G5A):
an ethynyl group.

Substituted or Unsubstituted Cycloalkyl Group

Specific examples (specific example group G6) of the "substituted or unsubstituted cycloalkyl group" mentioned herein include unsubstituted cycloalkyl groups (specific example group G6A) and substituted cycloalkyl groups (specific example group G6B). (Herein, an unsubstituted cycloalkyl group refers to an "unsubstituted cycloalkyl group" in a "substituted or unsubstituted cycloalkyl group," and a substituted cycloalkyl group refers to a "substituted cycloalkyl group" in a "substituted or unsubstituted cycloalkyl group.") A simply termed "cycloalkyl group" herein includes both of "unsubstituted cycloalkyl group" and "substituted cycloalkyl group".

The "substituted cycloalkyl group" refers to a group derived by substituting at least one hydrogen atom of an "unsubstituted cycloalkyl group" with a substituent. Specific examples of the "substituted cycloalkyl group" include a group derived by substituting at least one hydrogen atom of the "unsubstituted cycloalkyl group" (specific example group G6A) below with a substituent, and examples of the substituted cycloalkyl group (specific example group G6B) below. It should be noted that the examples of the "unsubstituted cycloalkyl group" and the "substituted cycloalkyl group" mentioned herein are merely exemplary, and the "substituted cycloalkyl group" mentioned herein includes a group derived by substituting at least one hydrogen atom bonded to a carbon atom of a skeleton of the "substituted cycloalkyl group" in the specific example group G6B with a substituent, and a group derived by further substituting a hydrogen atom of a substituent of the "substituted cycloalkyl group" in the specific example group G6B with a substituent.

Unsubstituted Cycloalkyl Group (Specific Example Group G6A):
a cyclopropyl group, cyclobutyl group, cyclopentyl group, cyclohexyl group, 1-adamantyl group, 2-adamantyl group, 1-norbornyl group, and 2-norbornyl group.
Substituted Cycloalkyl Group (Specific Example Group G6B):
a 4-methylcyclohexyl group.

Group Represented by -Si($R_{901}$)($R_{902}$)($R_{903}$)

Specific examples (specific example group G7) of the group represented herein by -Si($R_{901}$)($R_{902}$)($R_{903}$) include:

-Si(G1)(G1)(G1); -Si(G1)(G2)(G2); -Si(G1)(G1)(G2); -Si(G2)(G2)(G2); - Si(G3)(G3)(G3); and -Si(G6)(G6)(G6);
where:

G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3;
G6 represents a "substituted or unsubstituted cycloalkyl group" in the specific example group G6;
a plurality of G1 in -Si(G1)(G1)(G1) are mutually the same or different;
a plurality of G2 in -Si(G1)(G2)(G2) are mutually the same or different;
a plurality of G1 in -Si(G1)(G1)(G2) are mutually the same or different;
a plurality of G2 in -Si(G2)(G2)(G2) are mutually the same or different;
a plurality of G3 in -Si(G3)(G3)(G3) are mutually the same or different; and
a plurality of G6 in -Si(G6)(G6)(G6) are mutually the same or different.

Group Represented by -O-($R_{904}$)

Specific examples (specific example group G8) of a group represented by - O-($R_{904}$) herein include: -O(G1); -O(G2); -O(G3); and -O(G6);
where:

G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3; and

G6 represents a "substituted or unsubstituted cycloalkyl group" in the specific example group G6.

Group Represented by -S-($R_{905}$)

Specific examples (specific example group G9) of a group represented herein by -S-($R_{905}$) include: -S(G1); -S(G2); -S(G3); and -S(G6);
where:

G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3; and
G6 represents a "substituted or unsubstituted cycloalkyl group" in the specific example group G6.

Group Represented by -N($R_{906}$)($R_{907}$)

Specific examples (specific example group G10) of a group represented herein by -N($R_{906}$)($R_{907}$) include: -N(G1)(G1); -N(G2)(G2); -N(G1)(G2); -N(G3)(G3); and -N(G6)(G6),
where:

G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3;
G6 represents a "substituted or unsubstituted cycloalkyl group" in the specific example group G6;
a plurality of G1 in -N(G1)(G1) are mutually the same or different;
a plurality of G2 in -N(G2)(G2) are mutually the same or different;
a plurality of G3 in -N(G3)(G3) are mutually the same or different; and
a plurality of G6 in -N(G6)(G6) are mutually the same or different.

Halogen Atom

Specific examples (specific example group G11) of "halogen atom" mentioned herein include a fluorine atom, chlorine atom, bromine atom, and iodine atom.

Substituted or Unsubstituted Fluoroalkyl Group

The "substituted or unsubstituted fluoroalkyl group" mentioned herein refers to a group derived by substituting at least one hydrogen atom bonded to at least one of carbon atoms forming an alkyl group in the "substituted or unsubstituted alkyl group" with a fluorine atom, and also includes a group (perfluoro group) derived by substituting all of hydrogen atoms bonded to carbon atoms forming the alkyl group in the "substituted or unsubstituted alkyl group" with fluorine atoms. An "unsubstituted fluoroalkyl group" has, unless otherwise specified herein, 1 to 50, preferably 1 to 30, more preferably 1 to 18 carbon atoms. The "substituted fluoroalkyl group" refers to a group derived by substituting at least one hydrogen atom in a "fluoroalkyl group" with a substituent. It should be noted that the examples of the "substituted fluoroalkyl group" mentioned herein include a group derived by further substituting at least one hydrogen atom bonded to a carbon atom of an alkyl chain of a "substituted fluoroalkyl group" with a substituent, and a group derived by further substituting at least one hydrogen atom of a substituent of the "substituted fluoroalkyl group" with a substituent. Specific examples of the "unsubstituted fluoroalkyl group" include a group derived by substituting at least one hydrogen atom of the "alkyl group" (specific example group G3) with a fluorine atom.

Substituted or Unsubstituted Haloalkyl Group

The "substituted or unsubstituted haloalkyl group" mentioned herein refers to a group derived by substituting at least one hydrogen atom bonded to carbon atoms forming the alkyl group in the "substituted or unsubstituted alkyl group" with a halogen atom, and also includes a group derived by substituting all hydrogen atoms bonded to carbon atoms forming the alkyl group in the "substituted or unsubstituted alkyl group" with halogen atoms. An "unsubstituted haloalkyl group" has, unless otherwise specified herein, 1 to 50, preferably 1 to 30, and more preferably 1 to 18 carbon atoms. The "substituted haloalkyl group" refers to a group derived by substituting at least one hydrogen atom in a "haloalkyl group" with a substituent. It should be noted that the examples of the "substituted haloalkyl group" mentioned herein include a group derived by further substituting at least one hydrogen atom bonded to a carbon atom of an alkyl

chain of a "substituted haloalkyl group" with a substituent, and a group derived by further substituting at least one hydrogen atom of a substituent of the "substituted haloalkyl group" with a substituent. Specific examples of the "unsubstituted haloalkyl group" include a group derived by substituting at least one hydrogen atom of the "alkyl group" (specific example group G3) with a halogen atom. The haloalkyl group is sometimes referred to as a halogenated alkyl group.

Substituted or Unsubstituted Alkoxy Group

Specific examples of a "substituted or unsubstituted alkoxy group" mentioned herein include a group represented by -O(G3), G3 being the "substituted or unsubstituted alkyl group" in the specific example group G3. An "unsubstituted alkoxy group" has, unless otherwise specified herein, 1 to 50, preferably 1 to 30, more preferably 1 to 18 carbon atoms.

Substituted or Unsubstituted Alkylthio Group

Specific examples of a "substituted or unsubstituted alkylthio group" mentioned herein include a group represented by -S(G3), G3 being the "substituted or unsubstituted alkyl group" in the specific example group G3. An "unsubstituted alkylthio group" has, unless otherwise specified herein, 1 to 50, preferably 1 to 30, more preferably 1 to 18 carbon atoms.

Substituted or Unsubstituted Aryloxy Group

Specific examples of a "substituted or unsubstituted aryloxy group" mentioned herein include a group represented by -O(G1), G1 being the "substituted or unsubstituted aryl group" in the specific example group G1. An "unsubstituted aryloxy group" has, unless otherwise specified herein, 6 to 50, preferably 6 to 30, more preferably 6 to 18 ring carbon atoms.

Substituted or Unsubstituted Arylthio Group

Specific examples of a "substituted or unsubstituted arylthio group" mentioned herein include a group represented by -S(G1), G1 being the "substituted or unsubstituted aryl group" in the specific example group G1. An "unsubstituted arylthio group" has, unless otherwise specified herein, 6 to 50, preferably 6 to 30, more preferably 6 to 18 ring carbon atoms.

Substituted or Unsubstituted Trialkylsilyl Group

Specific examples of a "trialkylsilyl group" mentioned herein include a group represented by -Si(G3)(G3)(G3), G3 being the "substituted or unsubstituted alkyl group" in the specific example group G3. A plurality of G3 in -Si(G3)(G3)(G3) are mutually the same or different. Each of the alkyl groups in the "trialkylsilyl group" has, unless otherwise specified herein, 1 to 50, preferably 1 to 20, more preferably 1 to 6 carbon atoms.

Substituted or Unsubstituted Aralkyl Group

Specific examples of a "substituted or unsubstituted aralkyl group" mentioned herein include a group represented by -(G3)-(G1), G3 being the "substituted or unsubstituted alkyl group" in the specific example group G3, G1 being the "substituted or unsubstituted aryl group" in the specific example group G1. Accordingly, the "aralkyl group" is a group derived by substituting a hydrogen atom of the "alkyl group" with a substituent in a form of the "aryl group," which is an example of the "substituted alkyl group." An "unsubstituted aralkyl group," which is an "unsubstituted alkyl group" substituted by an "unsubstituted aryl group," has, unless otherwise specified herein, 7 to 50 carbon atoms, preferably 7 to 30 carbon atoms, more preferably 7 to 18 carbon atoms.

Specific examples of the "substituted or unsubstituted aralkyl group" include a benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, and 2-β-naphthylisopropyl group.

Preferable examples of the substituted or unsubstituted aryl group mentioned herein include, unless otherwise specified herein, a phenyl group, p-biphenyl group, m-biphenyl group, o-biphenyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-terphenyl-4-yl group, o-terphenyl-3-yl group, o-terphenyl-2-yl group, 1-naphthyl group, 2-naphthyl group, anthryl group, phenanthryl group, pyrenyl group, chrysenyl group, triphenylenyl group, fluorenyl group, 9,9'-spirobifluorenyl group, 9,9-dimethylfluorenyl group, and 9,9-diphenylfluorenyl group.

Preferable examples of the substituted or unsubstituted heterocyclic group mentioned herein include, unless otherwise specified herein, a pyridyl group, pyrimidinyl group, triazinyl group, quinolyl group, isoquinolyl group, quinazolinyl group, benzimidazolyl group, phenanthrolinyl group, carbazolyl group (1-carbazolyl group, 2-carbazolyl group, 3-carbazolyl group, 4-carbazolyl group, or 9-carbazolyl group), benzocarbazolyl group, azacarbazolyl group, diazacarbazolyl group, dibenzofuranyl group, naphthobenzofuranyl group, azadibenzofuranyl group, diazadibenzofuranyl group, dibenzothiophenyl group, naphthobenzothiophenyl group, azadibenzothiophenyl group, diazadibenzothiophenyl group, (9-phenyl)carbazolyl group ((9-phenyl)carbazole-1-yl group, (9-phenyl)carbazole-2-yl group, (9-phenyl)carbazole-3-yl group, or (9-phenyl)carbazole-4-yl group), (9-biphenylyl)carbazolyl group, (9-phenyl)phenylcarbazolyl group, diphenylcarbazole-9-yl group, phenylcarbazole-9-yl group, phenyltriazinyl group, biphenylyltriazinyl group, diphenyltriazinyl group, phenyldibenzofuranyl group, and phenyldibenzothiophenyl group.

The carbazolyl group mentioned herein is, unless otherwise specified herein, specifically a group represented by one of formulae below.

[Formula 10]

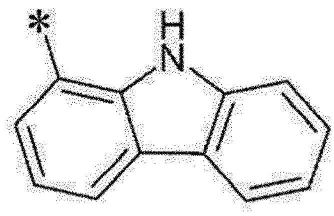

(TEMP-Cz1)

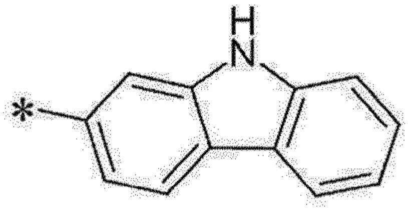

(TEMP-Cz2)

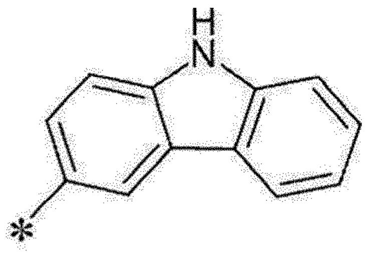

(TEMP-Cz3)

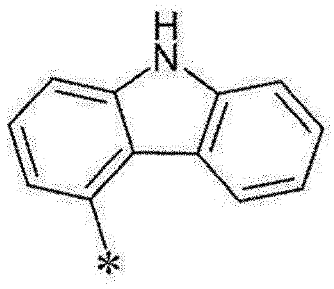

(TEMP-Cz4)

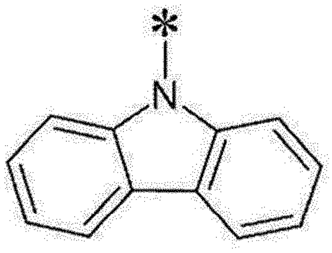

(TEMP-Cz5)

The (9-phenyl)carbazolyl group mentioned herein is, unless otherwise specified herein, specifically a group represented by one of formulae below.

[Formula 11]

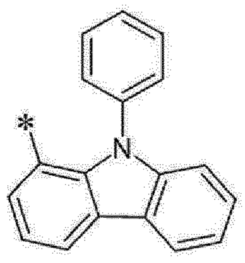

(TEMP-Cz6)

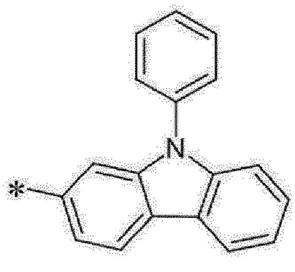

(TEMP-Cz7)

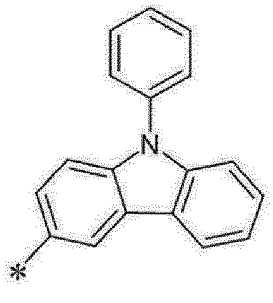

(TEMP-Cz8)

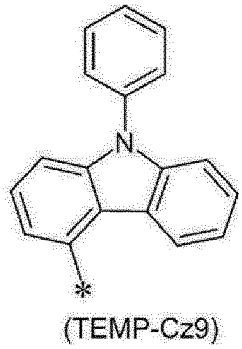

(TEMP-Cz9)

In the formulae (TEMP-Cz1) to (TEMP-Cz9), * represents a bonding position.

The dibenzofuranyl group and dibenzothiophenyl group mentioned herein are, unless otherwise specified herein, each specifically represented by one of formulae below.

[Formula 12]

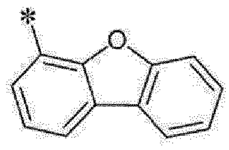

(TEMP-34)

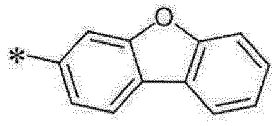

(TEMP-35)

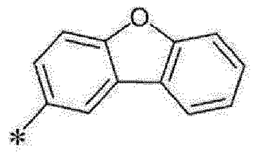

(TEMP-36)

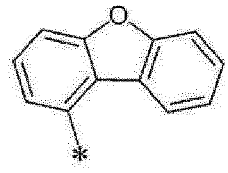

(TEMP-37)

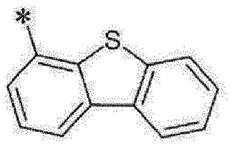

(TEMP-38)

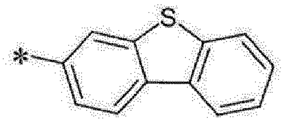

(TEMP-39)

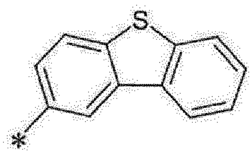

(TEMP-40)

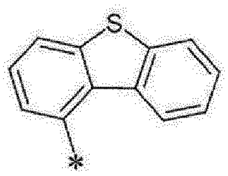

(TEMP-41)

In the formulae (TEMP-34) to (TEMP-41), * each represents a bonding position.

Preferable examples of the substituted or unsubstituted alkyl group mentioned herein include, unless otherwise specified herein, a methyl group, ethyl group, propyl group, isopropyl group, n-butyl group, isobutyl group, and t-butyl group.

Substituted or Unsubstituted Arylene Group

The "substituted or unsubstituted arylene group" mentioned herein is, unless otherwise specified herein, a divalent group derived by removing one hydrogen atom on an aryl ring of the "substituted or unsubstituted aryl group." Specific examples of the "substituted or unsubstituted arylene group" (specific example group G12) include a divalent group derived by removing one hydrogen atom on an aryl ring of the "substituted or unsubstituted aryl group" in the specific example group G1.

Substituted or Unsubstituted Divalent Heterocyclic Group

The "substituted or unsubstituted divalent heterocyclic group" mentioned herein is, unless otherwise specified herein, a divalent group derived by removing one hydrogen atom on a heterocyclic ring of the "substituted or unsubstituted heterocyclic group." Specific examples of the "substituted or unsubstituted divalent heterocyclic group" (specific example group G13) include a divalent group derived by removing one hydrogen atom on a heterocyclic ring of the "substituted or unsubstituted heterocyclic group" in the specific example group G2.

Substituted or Unsubstituted Alkylene Group

The "substituted or unsubstituted alkylene group" mentioned herein is, unless otherwise specified herein, a divalent group derived by removing one hydrogen atom on an alkyl chain of the "substituted or unsubstituted alkyl group." Specific examples of the "substituted or unsubstituted alkylene group" (specific example group G14) include a divalent group derived by removing one hydrogen atom on an alkyl chain of the "substituted or unsubstituted alkyl group" in the specific example group G3.

The substituted or unsubstituted arylene group mentioned herein is, unless otherwise specified herein, preferably any one of groups represented by formulae (TEMP-42) to (TEMP-68) below.

[Formula 13]

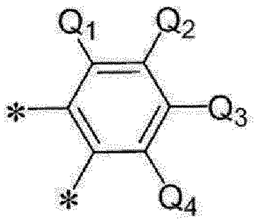

(TEMP-42)

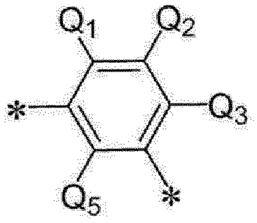

(TEMP-43)

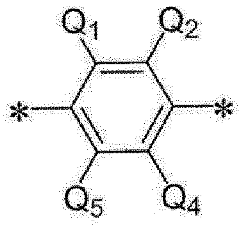

(TEMP-44)

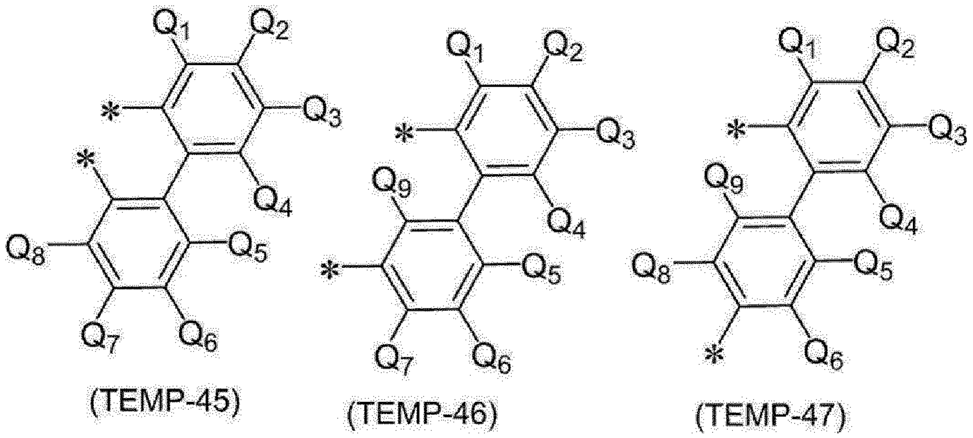

(TEMP-45) (TEMP-46) (TEMP-47)

[Formula 14]

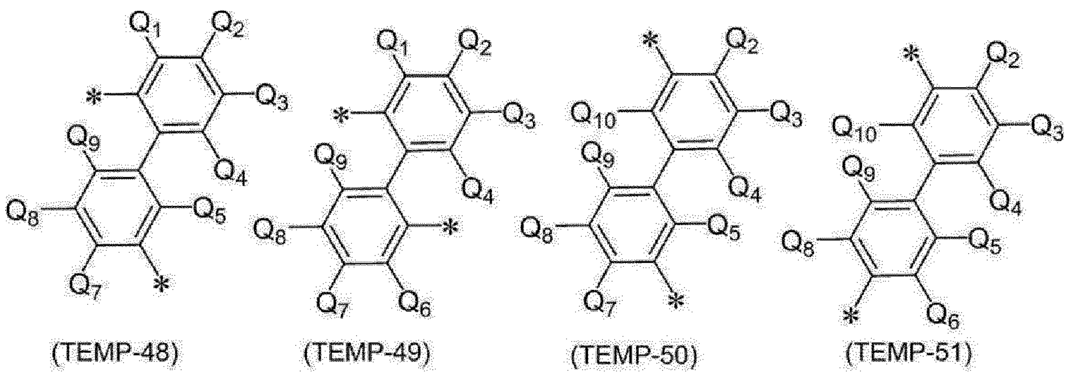

(TEMP-48) (TEMP-49) (TEMP-50) (TEMP-51)

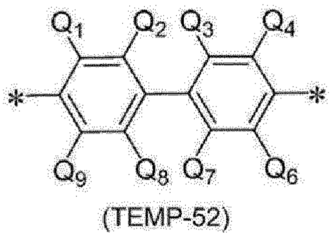

(TEMP-52)

In the formulae (TEMP-42) to (TEMP-52), $Q_1$ to $Q_{10}$ are each independently a hydrogen atom or a substituent.

In the formulae (TEMP-42) to (TEMP-52), * each represents a bonding position.

[Formula 15]

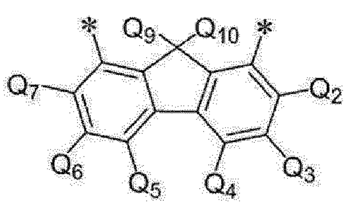

(TEMP-53)

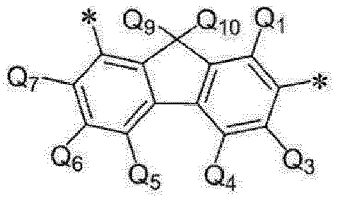

(TEMP-54)

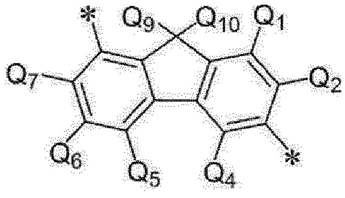

(TEMP-55)

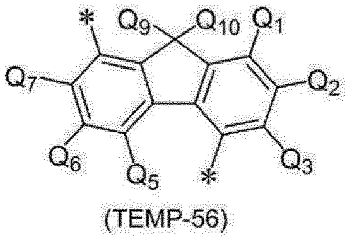

(TEMP-56)

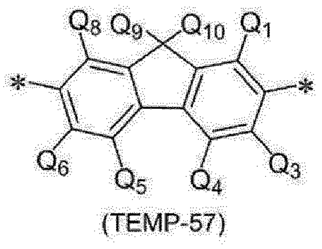

(TEMP-57)

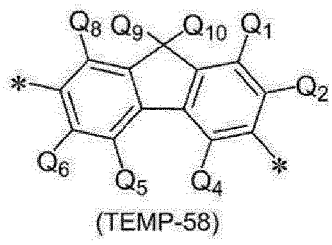

(TEMP-58)

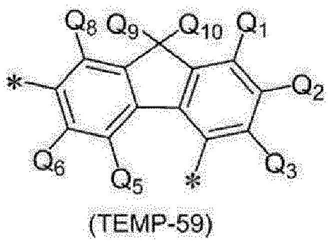

(TEMP-59)

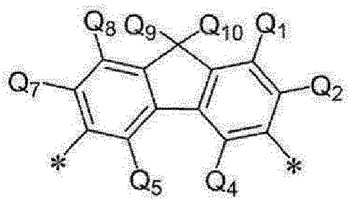

(TEMP-60)

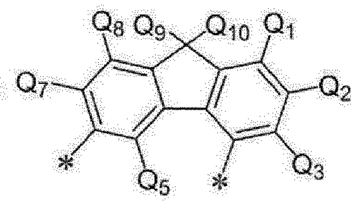

(TEMP-61)

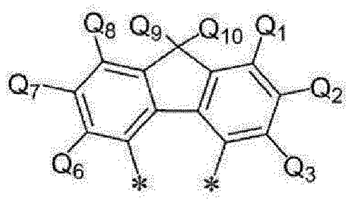

(TEMP-62)

In the formulae (TEMP-53) to (TEMP-62), $Q_1$ to $Q_{10}$ are each independently a hydrogen atom or a substituent.

In the formulae, $Q_9$ and $Q_{10}$ may be mutually bonded through a single bond to form a ring.

In the formulae (TEMP-53) to (TEMP-62), * each represents a bonding position.

[Formula 16]

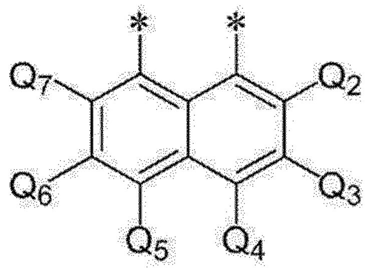
(TEMP-63)

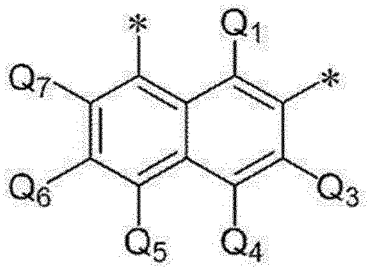
(TEMP-64)

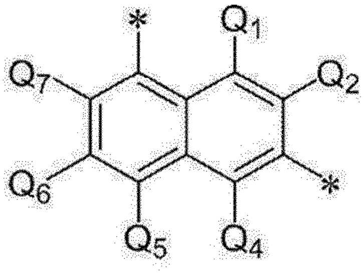
(TEMP-65)

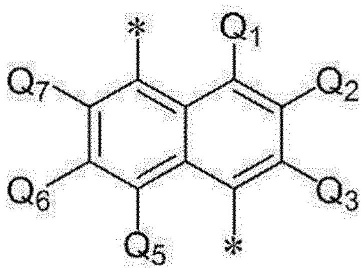
(TEMP-66)

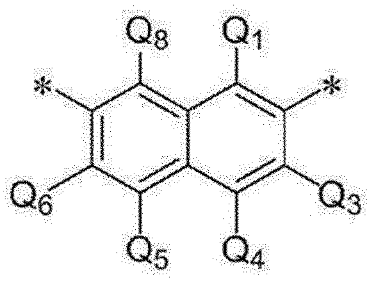
(TEMP-67)

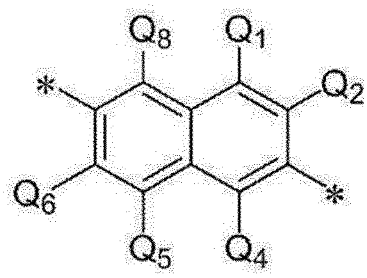
(TEMP-68)

In the formulae (TEMP-63) to (TEMP-68), $Q_1$ to $Q_8$ are each independently a hydrogen atom or a substituent.

In the formulae (TEMP-63) to (TEMP-68), * each represents a bonding position.

The substituted or unsubstituted divalent heterocyclic group mentioned herein is, unless otherwise specified herein, preferably a group represented by any one of formulae (TEMP-69) to (TEMP-102) below.

[Formula 17]

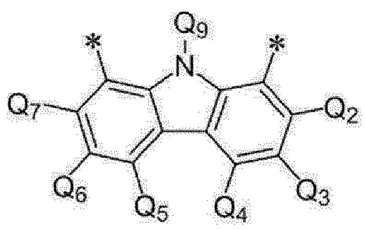
(TEMP-69)

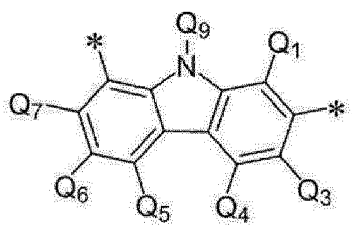
(TEMP-70)

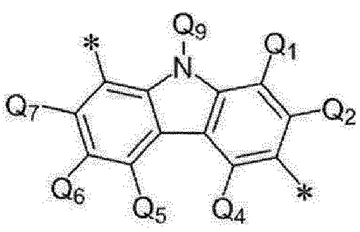
(TEMP-71)

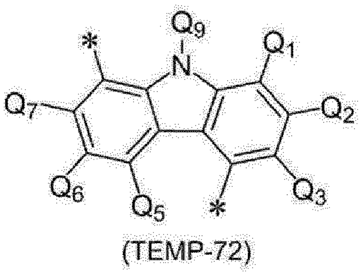
(TEMP-72)

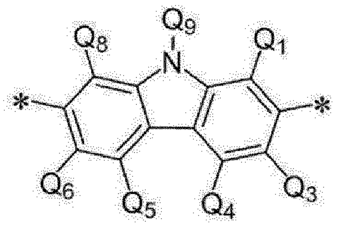
(TEMP-73)

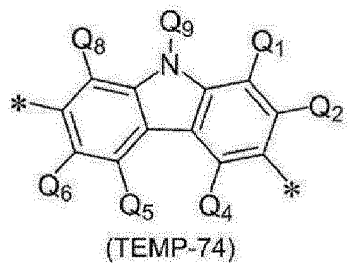
(TEMP-74)

[Formula 18]

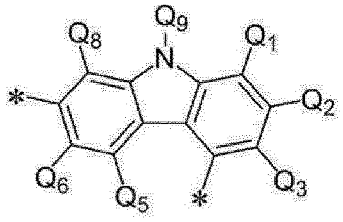
(TEMP-75)

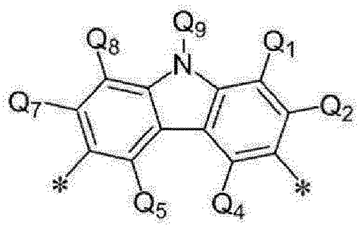
(TEMP-76)

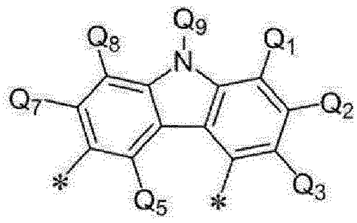
(TEMP-77)

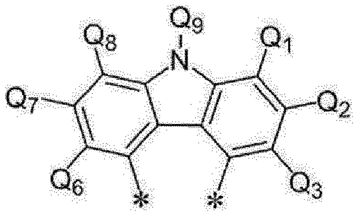
(TEMP-78)

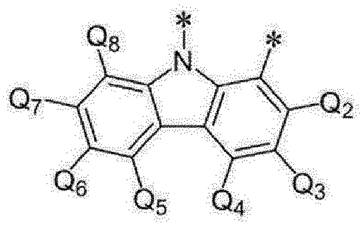
(TEMP-79)

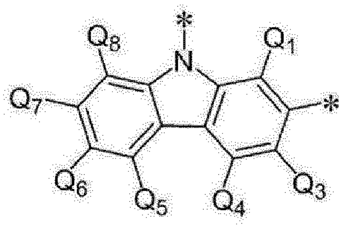
(TEMP-80)

[Formula 19]

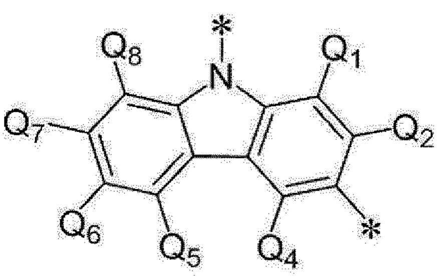
(TEMP-81)

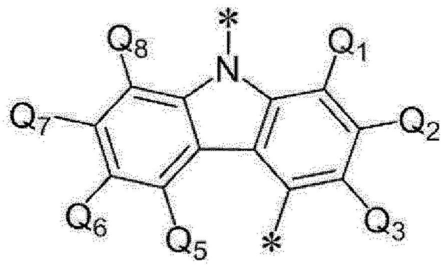
(TEMP-82)

In the formulae (TEMP-69) to (TEMP-82), $Q_1$ to $Q_9$ are each independently a hydrogen atom or a substituent.

[Formula 20]

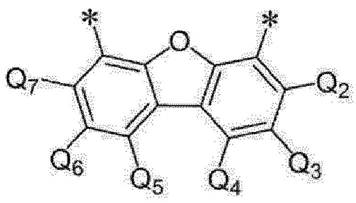
(TEMP-83)

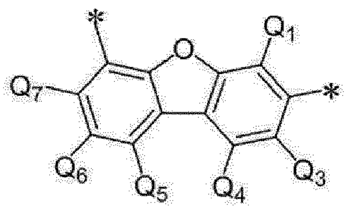
(TEMP-84)

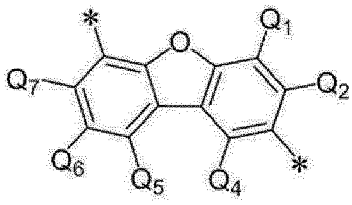
(TEMP-85)

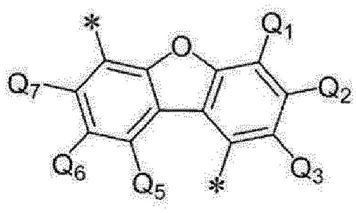
(TEMP-86)

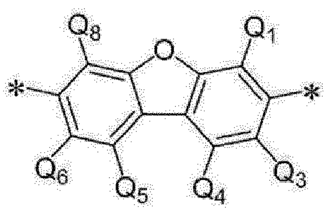
(TEMP-87)

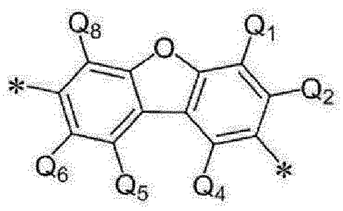
(TEMP-88)

[Formula 21]

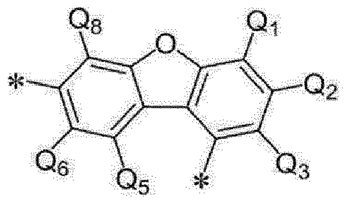
(TEMP-89)

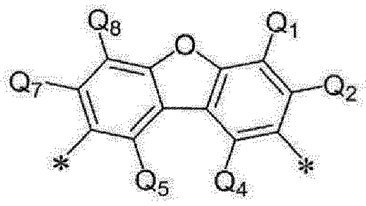
(TEMP-90)

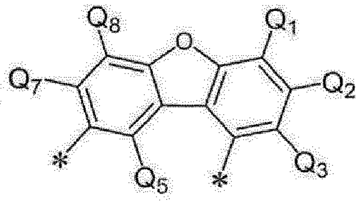
(TEMP-91)

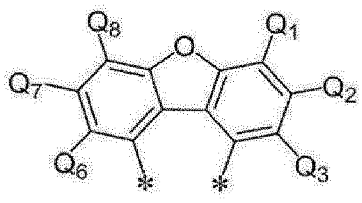
(TEMP-92)

[Formula 22]

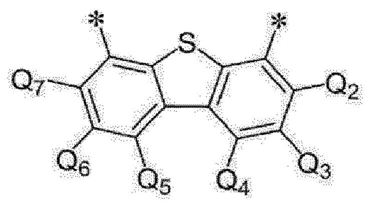
(TEMP-93)

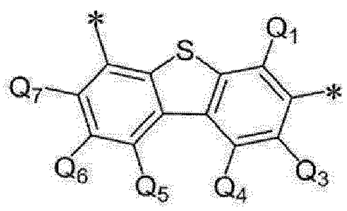
(TEMP-94)

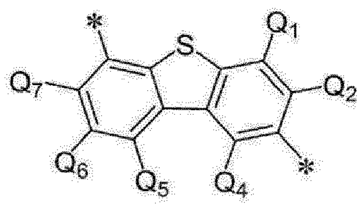
(TEMP-95)

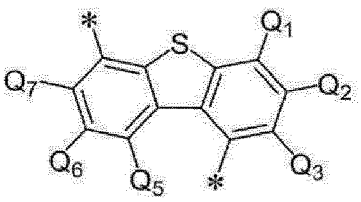
(TEMP-96)

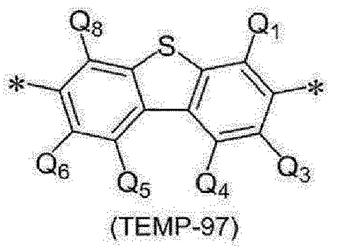
(TEMP-97)

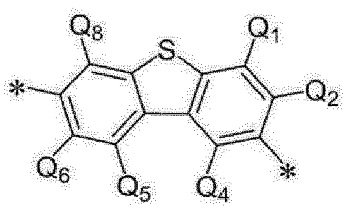
(TEMP-98)

[Formula 23]

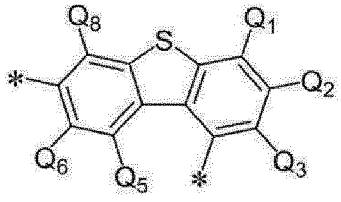

(TEMP-99)

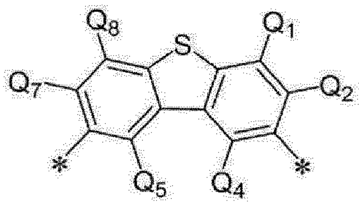

(TEMP-100)

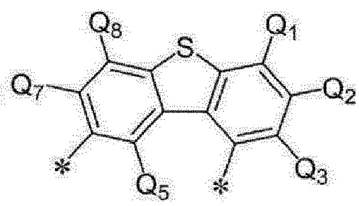

(TEMP-101)

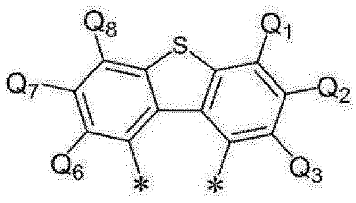

(TEMP-102)

In the formulae (TEMP-83) to (TEMP-102), $Q_1$ to $Q_8$ are each independently a hydrogen atom or a substituent.

The substituent mentioned herein has been described above.

Instance of "Bonded to Form Ring"

Instances where "at least one combination of adjacent two or more (of... ) are mutually bonded to form a substituted or unsubstituted monocyclic ring, mutually bonded to form a substituted or unsubstituted fused ring, or not mutually bonded" mentioned herein refer to instances where "at least one combination of adjacent two or more (of...) are mutually bonded to form a substituted or unsubstituted monocyclic ring, "at least one combination of adjacent two or more (of... ) are mutually bonded to form a substituted or unsubstituted fused ring," and "at least one combination of adjacent two or more (of...) are not mutually bonded."

Instances where "at least one combination of adjacent two or more (of... ) are mutually bonded to form a substituted or unsubstituted monocyclic ring" and "at least one combination of adjacent two or more (of...) are mutually bonded to form a substituted or unsubstituted fused ring" mentioned herein (these instances will be sometimes collectively referred to as an instance of "bonded to form a ring" hereinafter) will be described below. An anthracene compound having a basic skeleton in a form of an anthracene ring and represented by a formula (TEMP-103) below will be used as an example for the description.

[Formula 24]

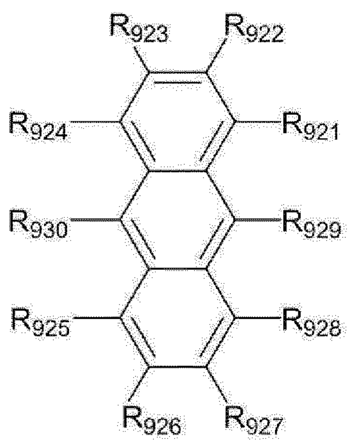

(TEMP-103)

For instance, when "at least one combination of adjacent two or more of $R_{921}$ to $R_{930}$ are mutually bonded to form a ring," the combination of adjacent ones of $R_{921}$ to $R_{930}$ (i.e. the combination at issue) is a combination of $R_{921}$

and $R_{922}$, a combination of $R_{922}$ and $R_{923}$, a combination of $R_{923}$ and $R_{924}$, a combination of $R_{924}$ and $R_{930}$, a combination of $R_{930}$ and $R_{925}$, a combination of $R_{925}$ and $R_{926}$, a combination of $R_{926}$ and $R_{927}$, a combination of $R_{927}$ and $R_{928}$, a combination of $R_{928}$ and $R_{929}$, or a combination of $R_{929}$ and $R_{921}$.

The term "at least one combination" means that two or more of the above combinations of adjacent two or more of $R_{921}$ to $R_{930}$ may simultaneously form rings. For instance, when $R_{921}$ and $R_{922}$ are mutually bonded to form a ring $Q_A$ and $R_{925}$ and $R_{926}$ are simultaneously mutually bonded to form a ring $Q_B$, the anthracene compound represented by the formula (TEMP-103) is represented by a formula (TEMP-104) below.

[Formula 25]

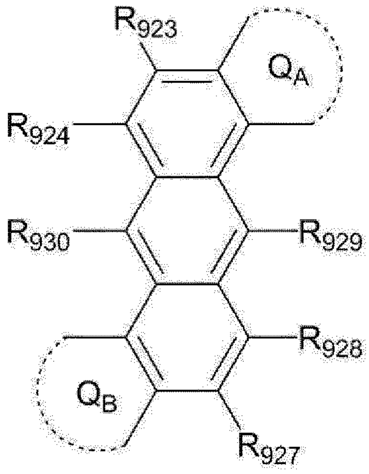

(TEMP-104)

The instance where the "combination of adjacent two or more" form a ring means not only an instance where the "two" adjacent components are bonded but also an instance where adjacent "three or more" are bonded. For instance, $R_{921}$ and $R_{922}$ are mutually bonded to form a ring $Q_A$ and $R_{922}$ and $R_{923}$ are mutually bonded to form a ring Qc, and mutually adjacent three components ($R_{921}$, $R_{922}$ and $R_{923}$) are mutually bonded to form a ring fused to the anthracene basic skeleton. In this case, the anthracene compound represented by the formula (TEMP-103) is represented by a formula (TEMP-105) below. In the formula (TEMP-105) below, the ring $Q_A$ and the ring Qc share $R_{922}$.

[Formula 26]

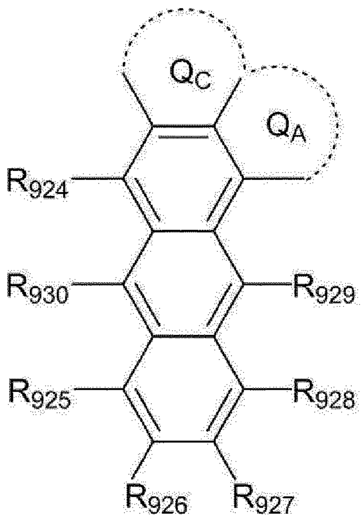

(TEMP-105)

The formed "monocyclic ring" or "fused ring" may be, in terms of the formed ring in itself, a saturated ring or an unsaturated ring. When the "combination of adjacent two" form a "monocyclic ring" or a "fused ring," the "monocyclic

ring" or "fused ring" may be a saturated ring or an unsaturated ring. For instance, the ring $Q_A$ and the ring $Q_B$ formed in the formula (TEMP-104) are each independently a "monocyclic ring" or a "fused ring." Further, the ring $Q_A$ and the ring Qc formed in the formula (TEMP-105) are each a "fused ring." The ring $Q_A$ and the ring Qc in the formula (TEMP-105) are fused to form a fused ring. When the ring $Q_A$ in the formula (TEMP-104) is a benzene ring, the ring $Q_A$ is a monocyclic ring. When the ring $Q_A$ in the formula (TEMP-104) is a naphthalene ring, the ring $Q_A$ is a fused ring.

The "unsaturated ring" represents an aromatic hydrocarbon ring or an aromatic heterocycle. The "saturated ring" represents an aliphatic hydrocarbon ring or a non-aromatic heterocycle.

Specific examples of the aromatic hydrocarbon ring include a ring formed by terminating a bond of a group in the specific examples of the specific example group G1 with a hydrogen atom.

Specific examples of the aromatic heterocyclic ring include a ring formed by terminating a bond of an aromatic heterocyclic group in the specific examples of the specific example group G2 with a hydrogen atom.

Specific examples of the aliphatic hydrocarbon ring include a ring formed by terminating a bond of a group in the specific examples of the specific example group G6 with a hydrogen atom.

The phrase "to form a ring" herein means that a ring is formed only by a plurality of atoms of a basic skeleton, or by a combination of a plurality of atoms of the basic skeleton and one or more optional atoms. For instance, the ring $Q_A$ formed by mutually bonding $R_{921}$ and $R_{922}$ shown in the formula (TEMP-104) is a ring formed by a carbon atom of the anthracene skeleton bonded to $R_{921}$, a carbon atom of the anthracene skeleton bonded to $R_{922}$, and one or more optional atoms. Specifically, when the ring $Q_A$ is a monocyclic unsaturated ring formed by $R_{921}$ and $R_{922}$, the ring formed by a carbon atom of the anthracene skeleton bonded to $R_{921}$, a carbon atom of the anthracene skeleton bonded to $R_{922}$, and four carbon atoms is a benzene ring.

The "optional atom" is, unless otherwise specified herein, preferably at least one atom selected from the group consisting of a carbon atom, nitrogen atom, oxygen atom, and sulfur atom. A bond of the optional atom (e.g. a carbon atom and a nitrogen atom) not forming a ring may be terminated by a hydrogen atom or the like or may be substituted by an "optional substituent" described later. When the ring includes an optional element other than carbon atom, the resultant ring is a heterocycle.

The number of "one or more optional atoms" forming the monocyclic ring or fused ring is, unless otherwise specified herein, preferably in a range from 2 to 15, more preferably in a range from 3 to 12, further preferably in a range from 3 to 5.

Unless otherwise specified herein, the ring, which may be a "monocyclic ring" or "fused ring," is preferably a "monocyclic ring."

Unless otherwise specified herein, the ring, which may be a "saturated ring" or "unsaturated ring," is preferably an "unsaturated ring."

Unless otherwise specified herein, the "monocyclic ring" is preferably a benzene ring.

Unless otherwise specified herein, the "unsaturated ring" is preferably a benzene ring.

When "at least one combination of adjacent two or more" (of...) are "mutually bonded to form a substituted or unsubstituted monocyclic ring" or "mutually bonded to form a substituted or unsubstituted fused ring," unless otherwise specified herein, at least one combination of adjacent two or more of components are preferably mutually bonded to form a substituted or unsubstituted "unsaturated ring" formed of a plurality of atoms of the basic skeleton, and 1 to 15 atoms of at least one element selected from the group consisting of carbon, nitrogen, oxygen and sulfur.

When the "monocyclic ring" or the "fused ring" has a substituent, the substituent is the substituent described in later-described "optional substituent." When the "monocyclic ring" or the "fused ring" has a substituent, specific examples of the substituent are the substituents described in the above under the subtitle "Substituent Mentioned Herein."

When the "saturated ring" or the "unsaturated ring" has a substituent, the substituent is the substituent described in later-described "optional substituent." When the "monocyclic ring" or the "fused ring" has a substituent, specific examples of the substituent are the substituents described in the above under the subtitle "Substituent Mentioned Herein."

The above is the description for the instances where "at least one combination of adjacent two or more (of...) are mutually bonded to form a substituted or unsubstituted monocyclic ring" and "at least one combination of adjacent two or more (of...) are mutually bonded to form a substituted or unsubstituted fused ring" mentioned herein (sometimes referred to as an instance of "bonded to form a ring").

Substituent for Substituted or Unsubstituted Group

In an exemplary embodiment herein, the substituent for the substituted or unsubstituted group (sometimes referred to as an "optional substituent" hereinafter) is, for instance, a group selected from the group consisting of an unsubstituted alkyl group having 1 to 50 carbon atoms, an unsubstituted alkenyl group having 2 to 50 carbon atoms, an unsubstituted alkynyl group having 2 to 50 carbon atoms, an unsubstituted cycloalkyl group having 3 to 50 ring carbon atoms, $-Si(R_{901})(R_{902})(R_{903})$, $-O-(R_{904})$, $-S-(R_{905})$, $-N(R_{906})(R_{907})$, a halogen atom, a cyano group, a nitro group, an unsubstituted aryl group having 6 to 50 ring carbon atoms, and an unsubstituted heterocyclic ring having 5 to 50 ring atoms.

$R_{901}$ to $R_{907}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when two or more $R_{901}$ are present, the two or more $R_{901}$ are mutually the same or different;
when two or more $R_{902}$ are present, the two or more $R_{902}$ are mutually the same or different;
when two or more $R_{903}$ are present, the two or more $R_{903}$ are mutually the same or different;
when two or more $R_{904}$ are present, the two or more $R_{904}$ are mutually the same or different;
when two or more $R_{905}$ are present, the two or more $R_{905}$ are mutually the same or different;
when two or more $R_{906}$ are present, the two or more $R_{906}$ are mutually the same or different; and
when two or more $R_{907}$ are present, the two or more $R_{907}$ are mutually the same or different.

In an exemplary embodiment, the substituent for the substituted or unsubstituted group is a group selected from the group consisting of an alkyl group having 1 to 50 carbon atoms, an aryl group having 6 to 50 ring carbon atoms, and a heterocyclic ring having 5 to 50 ring atoms.
In an exemplary embodiment, the substituent for the substituted or unsubstituted group is a group selected from the group consisting of an alkyl group having 1 to 18 carbon atoms, an aryl group having 6 to 18 ring carbon atoms, and a heterocyclic ring having 5 to 18 ring atoms.
Specific examples of the above optional substituent are the same as the specific examples of the substituent described in the above under the subtitle "Substituent Mentioned Herein."
Unless otherwise specified herein, adjacent ones of the optional substituents may form a "saturated ring" or an "unsaturated ring," preferably a substituted or unsubstituted saturated five-membered ring, a substituted or unsubstituted saturated six-membered ring, a substituted or unsubstituted unsaturated five-membered ring, or a substituted or unsubstituted unsaturated six-membered ring, more preferably a benzene ring.
Unless otherwise specified herein, the optional substituent may further include a substituent. Examples of the substituent for the optional substituent are the same as the examples of the optional substituent.
Herein, numerical ranges represented by "AA to BB" represent a range whose lower limit is the value (AA) recited before "to" and whose upper limit is the value (BB) recited after "to."

First Exemplary Embodiment

Compounds

A compound according to the exemplary embodiment is represented by a formula (1) below.

[Formula 27]

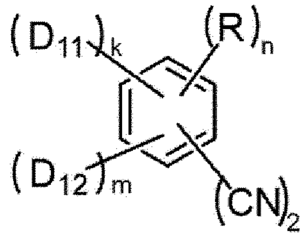

(1)

In the formula (1):

CN is a cyano group;
$D_{11}$ and $D_{12}$ are each independently a group represented by a formula (11), (12) or (13) below; at least one $D_{11}$ is a group represented by the formula (12) or (13);
R is each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by

-C(=O)$R_{908}$, a group represented by -COOR$_{909}$, a cyano group, a nitro group, a group represented by -P(=O)($R_{931}$)($R_{932}$), a group represented by - Ge($R_{933}$)($R_{934}$)($R_{935}$), a group represented by -B($R_{936}$)($R_{937}$), a substituted or unsubstituted aryl group having 6 to 50 ring carbon atoms, or a substituted or unsubstituted heterocyclic group having 5 to 50 ring atoms;

at least one R is a substituent, and R as at least one substituent is bonded to a benzene ring in the formula (1) via carbon-carbon bonding;

k is 1 or 2;

m is 0, 1, or 2;

n is 1, 2, or 3;

k + m + n = 4 is satisfied;

when k is 2, a plurality of $D_{11}$ are mutually the same or different;

when m is 2, a plurality of $D_{12}$ are mutually the same or different; and

when n is 2 or 3, a plurality of R are mutually the same or different.

[Formula 28]

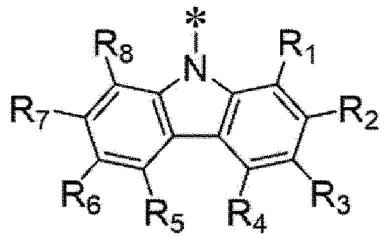

(11)

[Formula 29]

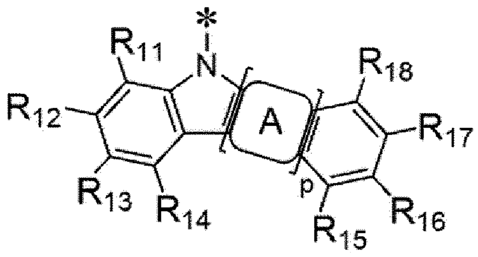

(12)

[Formula 30]

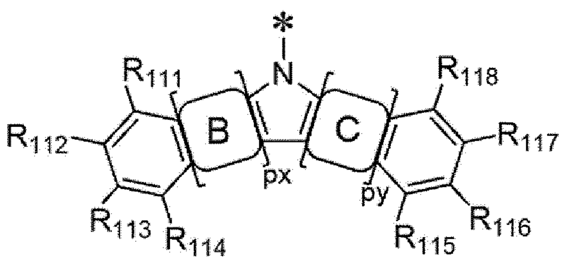

(13)

In the formula (12), at least one combination of adjacent two or more of $R_{11}$ to $R_{18}$ are mutually bonded to form a substituted or unsubstituted monocyclic ring, mutually bonded to form a substituted or unsubstituted fused ring, or not mutually bonded,

in the formula (13), at least one combination of adjacent two or more of $R_{111}$ to $R_{118}$ are mutually bonded to form a substituted or unsubstituted monocyclic ring, mutually bonded to form a substituted or unsubstituted fused ring, or not mutually bonded,

$R_1$ to $R_8$ in the formula (11), $R_{11}$ to $R_{18}$ forming neither the substituted or unsubstituted monocyclic ring nor the

substituted or unsubstituted fused ring in the formula (12), and $R_{111}$ to $R_{118}$ forming neither the substituted or unsubstituted monocyclic ring nor the substituted or unsubstituted fused ring in the formula (13) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{908}$, a group represented by $-COOR_{909}$, a halogen atom, a cyano group, a nitro group, a group represented by $-P(=O)(R_{931})(R_{932})$, a group represented by $-Ge(R_{933})(R_{934})(R_{935})$, a group represented by $-B(R_{936})(R_{937})$, a substituted or unsubstituted aryl group having 6 to 50 ring carbon atoms, or a substituted or unsubstituted heterocyclic group having 5 to 50 ring atoms;
in the formulae (12) and (13):

a ring A, a ring B, and a ring C are each independently a cyclic structure selected from the group consisting of cyclic structures represented by formulae (14) and (15) below;
the ring A, the ring B, and the ring C are fused with adjacent rings at any positions;
p, px and py are each independently 1, 2, 3, or 4;
when p is 2, 3, or 4, a plurality of rings A are mutually the same or different;
when px is 2, 3, or 4, a plurality of rings B are mutually the same or different;
when py is 2, 3, or 4, a plurality of rings C are mutually the same or different;
at least one $D_{11}$ is a group represented by the formula (12) or (13); p is 4 in the formula (12) when the at least one $D_{11}$ is represented by the formula (12), four rings A include two cyclic structures each represented by the formula (14) below and two cyclic structures each represented by the formula (15) below; and px and py are each 2 in the formula (13) when the at least one $D_{11}$ is represented by the formula (13), two rings B include one cyclic structure represented by the formula (14) below and one cyclic structure represented by the formula (15) below, and two rings C include one cyclic structure represented by the formula (14) below and one cyclic structure represented by the formula (15) below; and
* in the formulae (11) to (13) each represents a bonding position to the benzene ring in the formula (1).

[Formula 31]

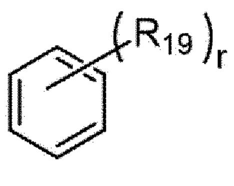
(14)

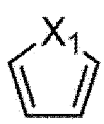
(15)

In the formula (14):

r is 0, 2, or 4;
a combination of a plurality of $R_{19}$ are mutually bonded to form a substituted or unsubstituted monocyclic ring, mutually bonded to form a substituted or unsubstituted fused ring, or not mutually bonded,
in the formula (15), $X_1$ is a sulfur atom or an oxygen atom;
$R_{19}$ forming neither the substituted or unsubstituted monocyclic ring nor the substituted or unsubstituted fused ring is a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{908}$, a group represented by $-COOR_{909}$, a halogen atom, a cyano group, a nitro group, a group represented by $-P(=O)(R_{931})(R_{932})$, a group represented by $-Ge(R_{933})(R_{934})(R_{935})$, a group represented by $-B(R_{936})(R_{937})$, a substituted or unsubstituted aryl group having 6 to 50 ring carbon atoms, or a substituted or unsubstituted heterocyclic group having 5 to 50 ring atoms;

a plurality of $R_{19}$ are mutually the same or different;
a plurality of $X_1$ are mutually the same or different; and
$D_{11}$ that is a group represented by the formula (13) satisfies at least one of a condition (Pv1), a condition (Pv2), or a condition (Pv3),
condition (Pv1): when k is 2, at least one of $X_1$ in a cyclic structure represented by the formula (15) as the ring B or $X_1$ in a cyclic structure represented by the formula (15) as the ring C is an oxygen atom,
condition (Pv2): when k is 2, two $D_{11}$ are mutually different, and
condition (Pv3): when n is 3, $X_1$ in a cyclic structure represented by the formula (15) as the ring B and $X_1$ in a cyclic structure represented by the formula (15) as the ring C are each independently a sulfur atom or an oxygen atom.

In the formulae, $R_{901}$, $R_{902}$, $R_{903}$, $R_{904}$, $R_{905}$, $R_{906}$, $R_{907}$, $R_{908}$, $R_{909}$, $R_{931}$, $R_{932}$, $R_{933}$, $R_{934}$, $R_{935}$, $R_{936}$, and $R_{937}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{908}$ are present, the plurality of $R_{908}$ are mutually the same or different;
when a plurality of $R_{909}$ are present, the plurality of $R_{909}$ are mutually the same or different;
when a plurality of $R_{931}$ are present, the plurality of $R_{931}$ are mutually the same or different;
when a plurality of $R_{932}$ are present, the plurality of $R_{932}$ are mutually the same or different;
when a plurality of $R_{933}$ are present, the plurality of $R_{933}$ are mutually the same or different;
when a plurality of $R_{934}$ are present, the plurality of $R_{934}$ are mutually the same or different;
when a plurality of $R_{935}$ are present, the plurality of $R_{935}$ are mutually the same or different;
when a plurality of $R_{936}$ are present, the plurality of $R_{936}$ are mutually the same or different; and
when a plurality of $R_{937}$ are present, the plurality of $R_{937}$ are mutually the same or different.

The exemplary embodiment provides a compound having a high PLQY.

When the compound according to the exemplary embodiment in which $D_{11}$ and $D_{12}$ are mutually different groups or a plurality of $D_{11}$ are mutually different groups is used in an organic layer of an organic EL device, hole injectability is improved to improve at least one of luminous efficiency or lifetime. Specifically, this is because holes are injected into the organic layer in stages since $D_{11}$ and $D_{12}$ have mutually different oxidation potentials.

When all of $D_{11}$ and $D_{12}$ are mutually the same group in chemical structure except for $X_1$ in the compound according to the exemplary embodiment, at least one $X_1$ being an oxygen atom is enough for an organic EL device to prolong a lifetime. The group with at least one $X_1$ being an oxygen atom has a smaller bonding angle to a benzene ring represented by the formula (1) than that of a group with all $X_1$ being sulfur atoms. Therefore, it is considered that use of the compound according to the exemplary embodiment in the organic layer prolongs a lifetime of an organic EL device.

In the compound according to the exemplary embodiment, a benzene ring in the formula (1) to which groups represented by the formulae (11) to (13) and the like are bonded is a benzene ring per se explicitly depicted in the formula (1), not a benzene ring included in R, $D_{11}$, and $D_{12}$.

Also in compounds represented by the later-described formulae (110), (120), (130), (126), (127), (126A), (127A), (127B), (111), (112), and (113), for instance, the groups represented by the formulae (11) to (13) are bonded to a benzene ring per se explicitly depicted in each of the formulae (110), (120), (130), (126), (127), (126A), (127A), (127B), (111), (112), and (113) in the same manner as to the benzene ring in the formula (1).

In the compound according to the exemplary embodiment, at least one $D_{11}$ is preferably a group represented by a formula (121), (122), or (131) below.

[Formula 32]

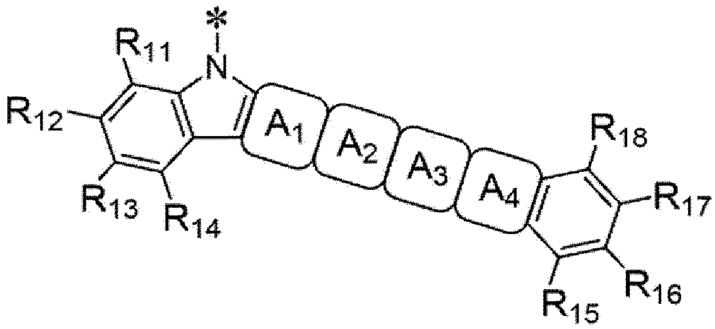

(121)

[Formula 33]

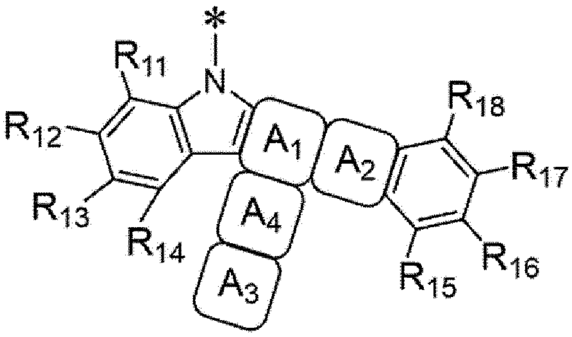

(122)

[Formula 34]

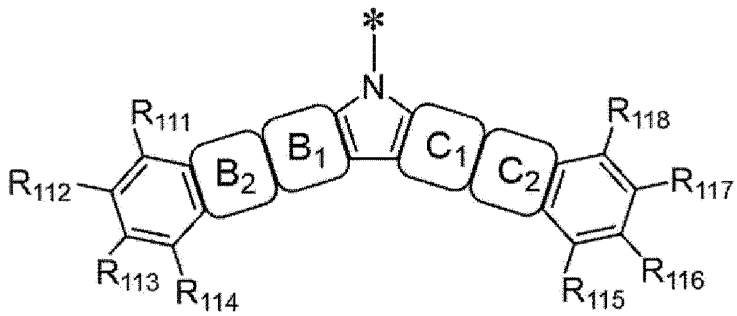

(131)

In the formulae (121) and (122): $R_{11}$ to $R_{18}$ represent the same as $R_{11}$ to $R_{18}$ in the formula (12); of a ring $A_1$, a ring $A_2$, a ring $A_3$, and a ring $A_4$, two of the rings $A_1$, $A_2$, $A_3$, and $A_4$ are each a cyclic structure represented by the formula (14) and the remaining two of the rings $A_1$, $A_2$, $A_3$, and $A_4$ are each a cyclic structure represented by the formula (15).

In the formula (131): $R_{111}$ to $R_{118}$ represent the same as $R_{111}$ to $R_{118}$ in the formula (13);

one of a ring $B_1$ and a ring $B_2$ is a cyclic structure represented by the formula (14) and the other of the ring $B_1$ and the ring $B_2$ is a cyclic structure represented by the formula (15); and
one of a ring $C_1$ and a ring $C_2$ is a cyclic structure represented by the formula (14) and the other of the ring $C_1$ and the ring $C_2$ is a cyclic structure represented by the formula (15).
* in the formulae (121), (122), and (131) each represents a bonding position to the benzene ring in the formula (1).

In the compound according to the exemplary embodiment, preferably, the ring $A_1$ and the ring $A_3$ are each a cyclic structure represented by the formula (14) and the ring $A_2$ and the ring $A_4$ are each a cyclic structure represented by the formula (15).

In the compound according to the exemplary embodiment, preferably, the ring $B_1$ is a cyclic structure represented

by the formula (14), the ring $B_2$ is a cyclic structure represented by the formula (15), the ring $C_1$ is a cyclic structure represented by the formula (14), the ring $C_2$ is a cyclic structure represented by the formula (15).

In the compound according to the exemplary embodiment, at least one $D_{11}$ is preferably a group represented by the formula (131).

In the compound according to the exemplary embodiment, at least one $D_{11}$ is preferably a group represented by a formula (123), (124), (125), or (132) below.

[Formula 35]

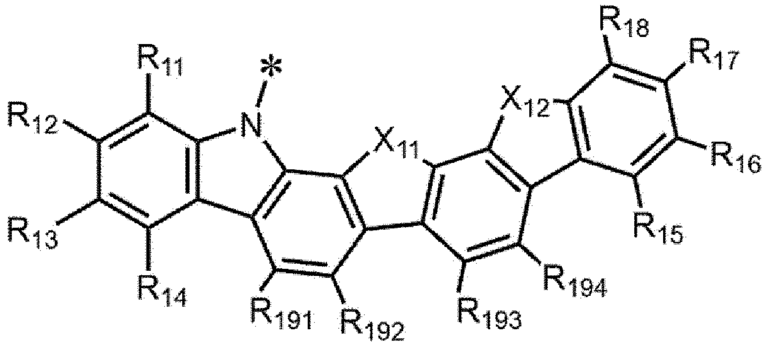

(123)

[Formula 36]

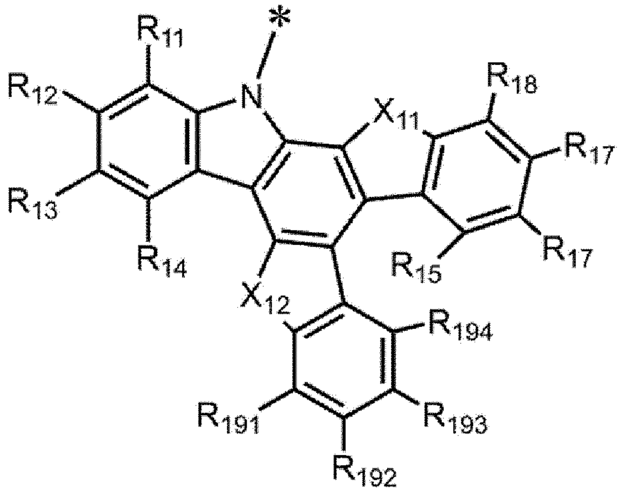

(124)

[Formula 37]

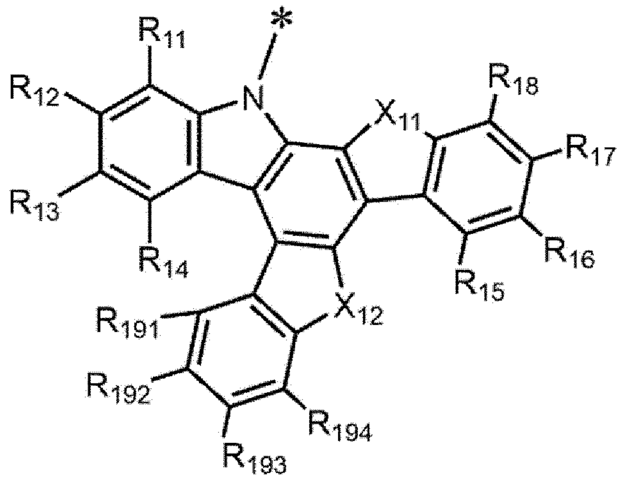

(125)

[Formula 38]

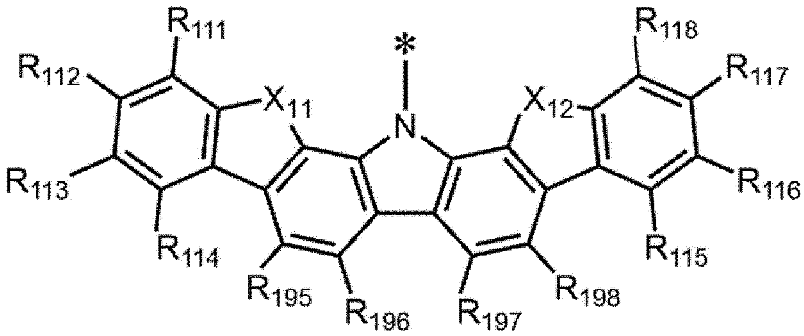

(132)

In the formulae (123), (124), and (125): $R_{11}$ to $R_{18}$ represent the same as $R_{11}$ to $R_{18}$ in the formula (12); and $R_{191}$ to $R_{194}$ each independently represent the same as $R_{19}$ in the formula (14).

In the formula (132): $R_{111}$ to $R_{118}$ represent the same as $R_{111}$ to $R_{118}$ in the formula (13); and $R_{195}$ to $R_{198}$ each independently represent the same as $R_{19}$ in the formula (14).

In the formulae (123), (124), (125), and (132): $X_{11}$ and $X_{12}$ each independently represent the same as $X_1$ in the formula (15), and each * represents a bonding position to the benzene ring in the formula (1).

In the compound according to the exemplary embodiment, preferably, none of combinations of adjacent two or more of $R_{191}$ to $R_{194}$ are bonded to each other.

In the compound according to the exemplary embodiment, preferably, none of combinations of adjacent two or more of $R_{195}$ to $R_{198}$ are bonded to each other.

In the compound according to the exemplary embodiment, $X_{11}$ is preferably a sulfur atom.

In the compound according to the exemplary embodiment, $X_{11}$ in a group represented by each of the formulae (123), (124), and (125) is preferably a sulfur atom.

In the compound according to the exemplary embodiment, at least one $D_{11}$ is preferably a group represented by the formula (132).

In the compound according to the exemplary embodiment, $X_{11}$ in a group represented by the formula (132) is preferably a sulfur atom. In the compound according to the exemplary embodiment, more preferably, $X_{11}$ in a group represented by the formula (132) is a sulfur atom and $X_{12}$ is a sulfur atom or an oxygen atom.

In the compound according to the exemplary embodiment, $D_{12}$ is preferably a group represented by the formula (11) or (12).

In the compound according to the exemplary embodiment, $D_{12}$ is preferably a group represented by the formula (12).

In the compound according to the exemplary embodiment, a group represented by the formula (12) is preferably a group selected from the group consisting of groups represented by formulae (12A), (12B), (12C), (12D), (12E), and (12F) below.

[Formula 39]

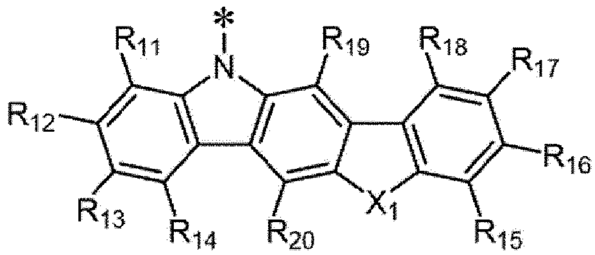

(12A)

[Formula 40]

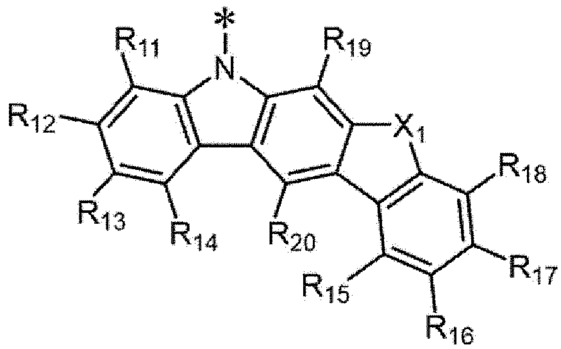

(12B)

[Formula 41]

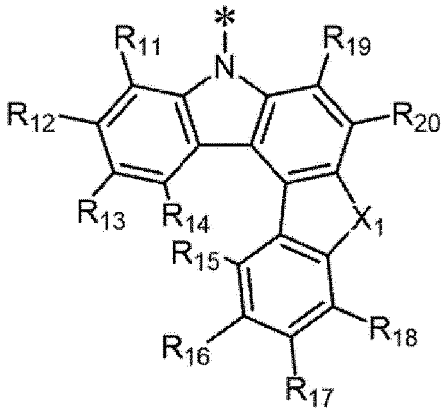

(12C)

[Formula 42]

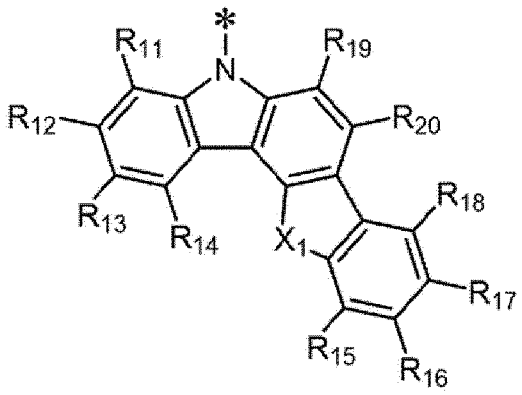

(12D)

[Formula 43]

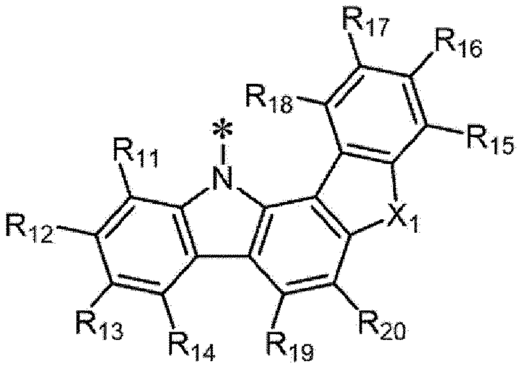

(12E)

[Formula 44]

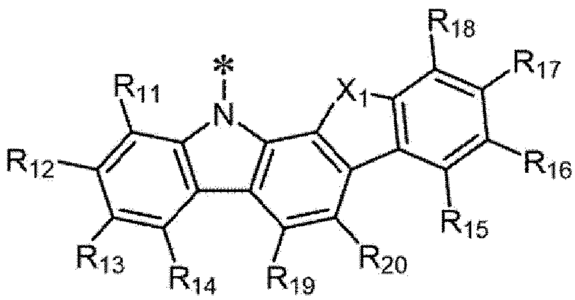

(12F)

In the formulae (12A), (12B), (12C), (12D), (12E), and (12F):

$R_{11}$ to $R_{18}$ each independently represent the same as $R_{11}$ to $R_{18}$ in the formula (12);
$R_{19}$ and $R_{20}$ each independently represent the same as $R_{19}$ in the formula (14); and
$X_1$ represents the same as $X_1$ in the formula (15); and
each * in the formulae (12A), (12B), (12C), (12D), (12E), and (12F) represents a bonding position to a benzene ring in the formula (1).

In the compound according to the exemplary embodiment, a compound represented by the formula (1) is preferably represented by a formula (110), (120) or (130) below.

[Formula 45]

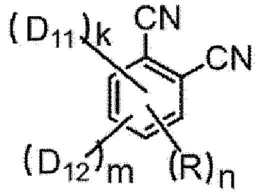
(110)

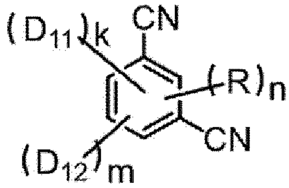
(120)

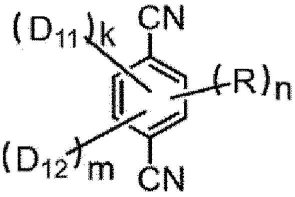
(130)

In the formulae (110), (120), and (130), $D_{11}$, $D_{12}$, R, k, m, and n respectively represent the same as $D_{11}$, $D_{12}$, R, k, m, and n in the formula (1).

In the compound according to the exemplary embodiment, n in the formula (1) is preferably 2 or 3.

In the compound according to the exemplary embodiment, n in the formula (1) is also preferably 2.

In the compound according to the exemplary embodiment, a compound represented by the formula (1) is also preferably represented by a formula (126) or (127) below.

[Formula 46]

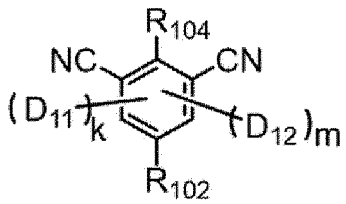
(126)

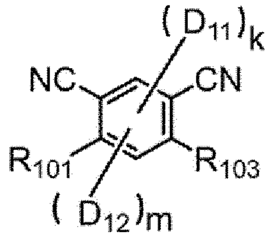
(127)

In the formulae (126) and (127): $D_{11}$ represents the same as $D_{11}$ in the formula (1); $D_{12}$ represents the same as $D_{12}$ in the formula (1); $R_{101}$ to $R_{104}$ each independently represent the same as R in the formula (1); k is 1 or 2; m is 0 or 1; and k + m = 2 is satisfied.

In the compound according to the exemplary embodiment, also preferably, k is 2, one of two $D_{11}$ is a group represented by the formula (12) and the other of the two $D_{11}$ is a group represented by the formula (13).

In the compound according to the exemplary embodiment, also preferably, k is 2, two $D_{11}$ are each a group represented by the formula (13) and two groups represented by the formula (13) as $D_{11}$ are mutually different.

In the compound according to the exemplary embodiment, also preferably, k and m are each 1, one of $D_{11}$ and $D_{12}$ is a group represented by the formula (12) and the other of $D_{11}$ and $D_{12}$ is a group represented by the formula (13).

In the compound according to the exemplary embodiment, a compound represented by the formula (1) is also preferably represented by a formula (126A), (127A) or (127B) below.

[Formula 47]

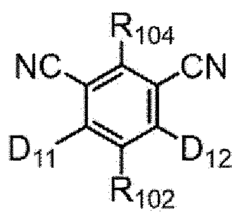
(126A)

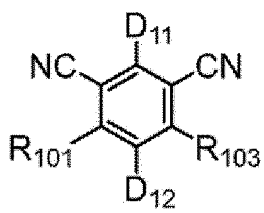
(127A)

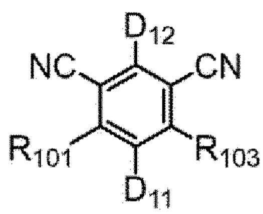
(127B)

In the formulae (126A), (127A) and (127B): $D_{11}$ represents the same as $D_{11}$ in the formula (1); $D_{12}$ represents the same as $D_{12}$ in the formula (1); and $R_{101}$ to $R_{104}$ each independently represent the same as R in the formula (1).

In the formulae (126A), (127A) and (127B), $D_{11}$ and $D_{12}$ are preferably mutually different groups.

In the compound according to the exemplary embodiment, n in the formula (1) is also preferably 3.

In the compound according to the exemplary embodiment, a compound represented by the formula (1) is also preferably represented by a formula (111), (112) or (113) below.

[Formula 48]

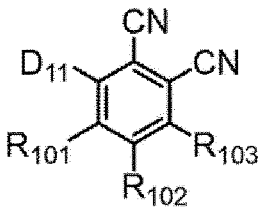
(111)

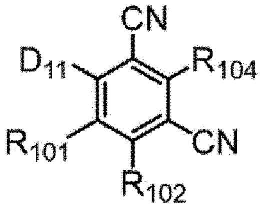
(112)

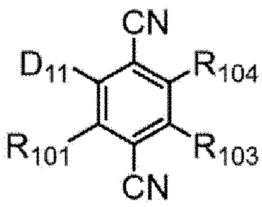
(113)

In the formulae (111), (112), and (113): $D_{11}$ represents the same as $D_{11}$ in the formula (1); and $R_{101}$ to $R_{104}$ each independently represent the same as R in the formula (1).

In the compound according to the exemplary embodiment, none of combinations of adjacent two or more of a plurality of R are bonded to each other.

In the compound according to the exemplary embodiment, none of combinations of adjacent two or more of $R_{101}$ to $R_{104}$ are bonded to each other.

In the compound according to the exemplary embodiment, R in the formula (1) is preferably each independently a substituted or unsubstituted aryl group having 6 to 14 ring carbon atoms or a substituted or unsubstituted heterocyclic group having 5 to 14 ring atoms.

In the compound according to the exemplary embodiment, R in the formula (1) is preferably each independently a substituted or unsubstituted phenyl group or a substituted or unsubstituted heterocyclic group having 6 ring atoms.

In the compound according to the exemplary embodiment, $R_{101}$ to $R_{104}$ are preferably each independently a substituted or unsubstituted aryl group having 6 to 14 ring carbon atoms or a substituted or unsubstituted heterocyclic group having 5 to 14 ring atoms.

In the compound according to the exemplary embodiment, $R_{101}$ to $R_{104}$ are preferably each independently a substituted or unsubstituted phenyl group or a substituted or unsubstituted heterocyclic group having 6 ring atoms.

In the compound according to the exemplary embodiment, a compound represented by the formula (1) is also preferably represented by a formula (126C) or (127C) below.

[Formula 49]

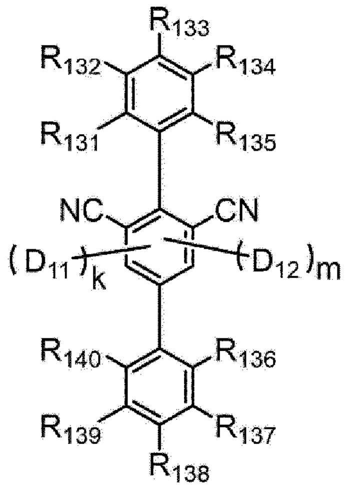

(126C)

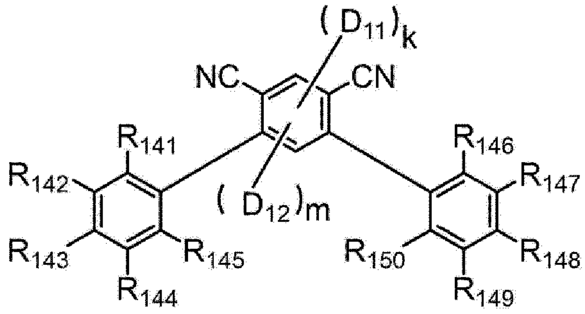

(127C)

In the formulae (126C) and (127C): $D_{11}$ represents the same as $D_{11}$ in the formula (1); $D_{12}$ represents the same as $D_{12}$ in the formula (1); $R_{131}$ to $R_{140}$ and $R_{141}$ to $R_{150}$ each independently represent the same as R in the formula (1); k is 1 or 2; m is 0 or 1; and k + m = 2 is satisfied.

In the compound according to the exemplary embodiment, a compound represented by the formula (1) is also preferably represented by a formula (126D) or (127D) below.

[Formula 50]

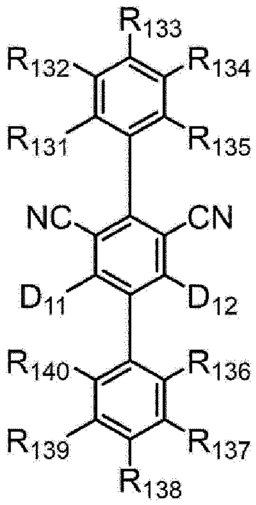

(126D)

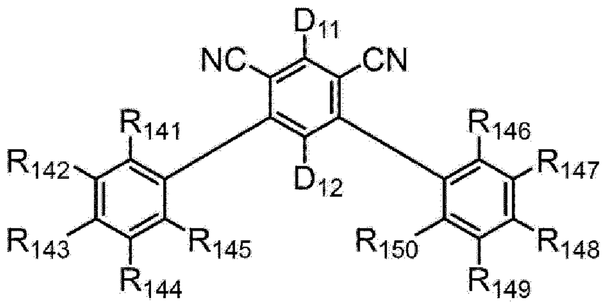

(127D)

In the formulae (126D) and (127D): $D_{11}$ represents the same as $D_{11}$ in the formula (1); $D_{12}$ represents the same as $D_{12}$ in the formula (1); and $R_{131}$ to $R_{140}$ and $R_{141}$ to $R_{150}$ each independently represent the same as R in the formula (1).

In the compound according to the exemplary embodiment, preferably, $D_{11}$ is a group represented by the formula (132) and $D_{12}$ is a group represented by one of the formulae (12A) to (12F).

In the compound according to the exemplary embodiment, $R_{131}$ to $R_{140}$ and $R_{141}$ to $R_{150}$ are preferably each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms, more preferably a hydrogen atom, a substituted or unsubstituted alkyl group having 1 to 50 carbon atoms, or a substituted or unsubstituted aryl group having 6 to 50 ring carbon atoms.

In the compound according to the exemplary embodiment, none of combinations of adjacent two or more of $R_1$ to $R_8$ are bonded to each other.

In the compound according to the exemplary embodiment, preferably, none of combinations of adjacent two or more of $R_{11}$ to $R_{18}$ are bonded to each other.

In the compound according to the exemplary embodiment, preferably, none of combinations of adjacent two or more of $R_{11}$ to $R_{20}$ are bonded to each other.

In the compound according to the exemplary embodiment, preferably, none of combinations of adjacent two or more of $R_{111}$ to $R_{118}$ are bonded to each other.

In the compound according to the exemplary embodiment, $R_1$ to $R_8$ in the formula (11), $R_{11}$ to $R_{18}$ in the formula (12), $R_{111}$ to $R_{118}$ in the formula (13), and $R_{19}$ in the formula (14) are preferably each independently a hydrogen atom, a substituted or unsubstituted alkyl group having 1 to 50 carbon atoms, a substituted or unsubstituted cycloalkyl group having 3 to 50 ring carbon atoms, or a substituted or unsubstituted aryl group having 6 to 50 ring carbon atoms.

In the compound according to the exemplary embodiment, $R_1$ to $R_8$ in the formula (11), $R_{11}$ to $R_{18}$ in the formula (12), $R_{111}$ to $R_{118}$ in the formula (13), and $R_{19}$ in the formula (14) are preferably each independently a hydrogen atom, an unsubstituted alkyl group having 1 to 50 carbon atoms, an unsubstituted cycloalkyl group having 3 to 50 ring carbon atoms, or an unsubstituted aryl group having 6 to 50 ring carbon atoms.

In the compound according to the exemplary embodiment, $R_{191}$ to $R_{198}$ are preferably each independently a hydrogen atom, a substituted or unsubstituted alkyl group having 1 to 50 carbon atoms, a substituted or unsubstituted cycloalkyl group having 3 to 50 ring carbon atoms, or a substituted or unsubstituted aryl group having 6 to 50 ring carbon atoms, and more preferably a hydrogen atom, an unsubstituted alkyl group having 1 to 50 carbon atoms, an unsubstituted cycloalkyl group having 3 to 50 ring carbon atoms, or an unsubstituted aryl group having 6 to 50 ring carbon atoms.

The compound according to the exemplary embodiment is preferably a compound exhibiting delayed fluorescence.

Delayed Fluorescence

Delayed fluorescence is explained in "Yuki Hando-tai no Debaisu Bussei (Device Physics of Organic Semiconductors)" (edited by ADACHI, Chihaya, published by Kodansha, on pages 261-268). This document describes that, if an energy difference $\Delta E_{13}$ of a fluorescent material between a singlet state and a triplet state is reducible, a reverse energy transfer from the triplet state to the singlet state, which usually occurs at a low transition probability, would occur at a high efficiency to express thermally activated delayed fluorescence (TADF). Further, a generation mechanism of delayed fluorescence is explained in Fig. 10.38 in the document. The compound according to the exemplary embodiment is preferably a compound exhibiting thermally activated delayed fluorescence generated by such a mechanism.

In general, emission of delayed fluorescence can be confirmed by measuring the transient PL (Photo Luminescence).

The behavior of delayed fluorescence can also be analyzed based on the decay curve obtained from the transient PL measurement. The transient PL measurement is a method of irradiating a sample with a pulse laser to excite the sample, and measuring the decay behavior (transient characteristics) of PL emission after the irradiation is stopped. PL emission in TADF materials is classified into a light emission component from a singlet exciton generated by the first PL excitation and a light emission component from a singlet exciton generated via a triplet exciton. The lifetime of the singlet exciton generated by the first PL excitation is on the order of nanoseconds and is very short. Therefore, light emission from the singlet exciton rapidly attenuates after irradiation with the pulse laser.

On the other hand, the delayed fluorescence is gradually attenuated due to light emission from a singlet exciton generated via a triplet exciton having a long lifetime. As described above, there is a large temporal difference between the light emission from the singlet exciton generated by the first PL excitation and the light emission from the singlet exciton generated via the triplet exciton. Therefore, the luminous intensity derived from delayed fluorescence can be determined.

Fig. 1 is a schematic diagram of an exemplary apparatus for measuring the transient PL. An example of a method of measuring a transient PL as illustrated in Fig. 1 and an example of behavior analysis of delayed fluorescence will be described.

A transient PL measuring apparatus 100 in Fig. 1 includes: a pulse laser 101 capable of radiating a light having a predetermined wavelength; a sample chamber 102 configured to house a measurement sample; a spectrometer 103 configured to divide a light radiated from the measurement sample; a streak camera 104 configured to provide a two-dimensional image; and a personal computer 105 configured to import and analyze the two-dimensional image. An apparatus for measuring the transient PL is not limited to the apparatus illustrated in Fig. 1

The sample housed in the sample chamber 102 is obtained by forming a thin film, in which a matrix material is doped with a doping material at a concentration of 12 mass%, on the quartz substrate.

The thin film sample housed in the sample chamber 102 is irradiated with the pulse laser from the pulse laser

101 to excite the doping material. Emission is extracted in a direction of 90 degrees with respect to a radiation direction of the excited light. The extracted emission is divided by the spectrometer 103 to form a two-dimensional image in the streak camera 104. As a result, the two-dimensional image is obtainable in which the ordinate axis represents a time, the abscissa axis represents a wavelength, and a bright spot represents a luminous intensity. When this two-dimensional image is taken out at a predetermined time axis, an emission spectrum in which the ordinate axis represents the luminous intensity and the abscissa axis represents the wavelength is obtainable. Moreover, when this two-dimensional image is taken out at the wavelength axis, a decay curve (transient PL) in which the ordinate axis represents a logarithm of the luminous intensity and the abscissa axis represents the time is obtainable.

For instance, a thin film sample A was prepared as described above from a reference compound H1 as the matrix material and a reference compound D1 as the doping material and was measured in terms of the transient PL.

[Formula 51]

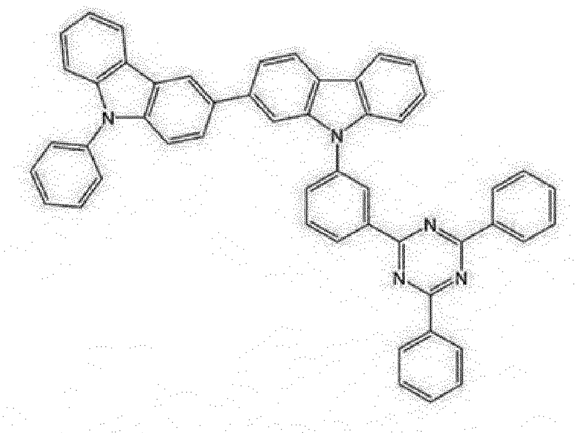

Reference Compound H1

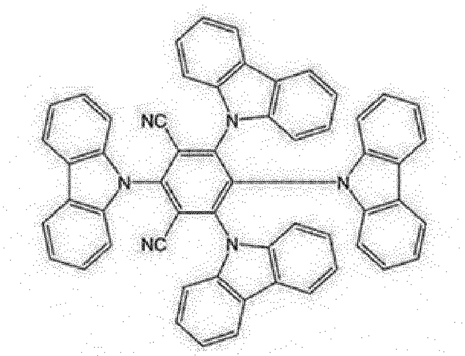

Reference Compound D1

The decay curve was analyzed with respect to the above thin film sample A and a thin film sample B. The thin film sample B was produced in the same manner as described above from a reference compound H2 as the matrix material and the reference compound D1 as the doping material.

Fig. 2 illustrates decay curves obtained from transient PL obtained by measuring the thin film samples A and B.

[Formula 52]

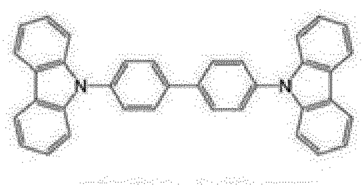

Reference Compound H2

As described above, an emission decay curve in which the ordinate axis represents the luminous intensity and the abscissa axis represents the time can be obtained by the transient PL measurement. Based on the emission decay curve, a fluorescence intensity ratio between fluorescence emitted from a singlet state generated by photo-excitation and delayed fluorescence emitted from a singlet state generated by reverse energy transfer via a triplet state can be estimated. In a delayed fluorescent material, a ratio of the intensity of the slowly decaying delayed fluorescence to the intensity of the promptly decaying fluorescence is relatively large.

Specifically, Prompt emission and Delay emission are present as emission from the delayed fluorescent material. Prompt emission is observed promptly when the excited state is achieved by exciting the compound of the exemplary embodiment with a pulse beam (i.e., a beam emitted from a pulse laser) having a wavelength absorbable by the delayed

fluorescent material. Delay emission is observed not promptly when the excited state is achieved but after the excited state is achieved.

An amount of Prompt emission, an amount of Delay emission and a ratio between the amounts thereof can be obtained according to the method as described in "Nature 492,234-238,2012" (Reference Document 1). The amount of Prompt emission and the amount of Delay emission may be calculated using an apparatus different from one described in Reference Document 1 or the apparatus illustrated in Fig. 1.

A sample produced by the following method is used for measuring delayed fluorescence of the compound according to the exemplary embodiment. For instance, the compound according to the exemplary embodiment is dissolved in toluene to prepare a dilute solution with an absorbance of 0.05 or less at the excitation wavelength to eliminate the contribution of self-absorption. In order to prevent quenching due to oxygen, the sample solution is frozen and degassed and then sealed in a cell with a lid under an argon atmosphere to obtain an oxygen-free sample solution saturated with argon.

The fluorescence spectrum of the sample solution is measured with a spectrofluorometer FP-8600 (produced by JASCO Corporation), and the fluorescence spectrum of a 9,10-diphenylanthracene ethanol solution is measured under the same conditions. Using the fluorescence area intensities of both spectra, the total fluorescence quantum yield is calculated by an equation (1) in Morris et al. J. Phys. Chem. 80 (1976) 969.

In the exemplary embodiment, provided that an amount of Prompt emission of a measurement target compound is denoted by $X_P$ and an amount of Delay emission is denoted by $X_D$, a value of $X_D/X_P$ is preferably 0.05 or more.

The amounts of Prompt emission and Delay emission and a ratio of the amounts thereof in compounds other than the compound according to the exemplary embodiment herein are measured in the same manner as those of the compound according to the exemplary embodiment.

ΔST

In the exemplary embodiment, a difference ($S_1$ - $T_{77K}$) between the lowest singlet energy $S_1$ and the energy gap $T_{77K}$ at 77K is defined as ΔST.

A difference ΔST(M1) between the lowest singlet energy $S_1$(M1) of the compound according to the exemplary embodiment and the energy gap $T_{77K}$(M1) at 77K of the compound according to the exemplary embodiment is preferably less than 0.3 eV, more preferably less than 0.2 eV, still more preferably less than 0.1 eV, and still further more preferably less than 0.01 eV. In other words, ΔST(M1) preferably satisfies a relationship of a numerical formula (Numerical Formula (10), Numerical Formula (11), Numerical Formula (12), or Numerical Formula (13)) below.

$$\Delta ST(M1) = S_1(M1) - T_{77K}(M1) < 0.3eV \quad ...(\text{Numerical Formula 10})$$

$$\Delta ST(M1) = S_1(M1) - T_{77K}(M1) < 0.2eV \quad ...(\text{Numerical Formula 11})$$

$$\Delta ST(M1) = S_1(M1) - T_{77K}(M1) < 0.1eV \quad ...(\text{Numerical Formula 12})$$

$$\Delta ST(M1) = S_1(M1) - T_{77K}(M1) < 0.01eV \quad ...(\text{Numerical Formula 13})$$

Relationship between Triplet Energy and Energy Gap at 77K

Here, a relationship between a triplet energy and an energy gap at 77K will be described. In the exemplary embodiment, the energy gap at 77K is different from a typically defined triplet energy in some aspects.

The triplet energy is measured as follows. First, a solution in which a compound (measurement target) is dissolved in an appropriate solvent is encapsulated in a quartz glass tube to prepare a sample. A phosphorescence spectrum (ordinate axis: phosphorescent luminous intensity, abscissa axis: wavelength) of the sample is measured at a low temperature (77K). A tangent is drawn to the rise of the phosphorescence spectrum close to the short-wavelength region. The triplet energy is calculated by a predetermined conversion equation based on a wavelength value at an intersection of the tangent and the abscissa axis.

Herein, among the compounds of the exemplary embodiment, the thermally activated Formula delayed fluorescent compound is preferably a compound having a small ΔST. When ΔST is small, intersystem crossing and inverse intersystem crossing are likely to occur even at a low temperature (77K), so that the singlet state and the triplet state coexist. As a result, the spectrum to be measured in the same manner as the above includes emission from both the

singlet state and the triplet state. Although it is difficult to distinguish from which state, the singlet state or the triplet state, light is emitted, the value of the triplet energy is basically considered dominant.

Accordingly, in the exemplary embodiment, the triplet energy is measured by the same method as a typical triplet energy T, but a value measured in the following manner is referred to as an energy gap T77K in order to differentiate the measured energy from the typical triplet energy in a strict meaning. The measurement target compound is dissolved in EPA (diethylether:isopentane:ethanol = 5:5:2 in volume ratio) at a concentration of 10 μmol/L, and the obtained solution is put in a quartz cell to provide a measurement sample. A phosphorescence spectrum (ordinate axis: phosphorescent luminous intensity, abscissa axis: wavelength) of the sample is measured at a low temperature (77K). A tangent is drawn to the rise of the phosphorescence spectrum close to the short-wavelength region. An energy amount is calculated by a conversion equation (F1) below based on a wavelength value $\lambda_{edge}$ [nm] at an intersection of the tangent and the abscissa axis and is defined as an energy gap $T_{77K}$ at 77K.

$$\text{Conversion Equation (F1): } T_{77K}\ [\text{eV}] = 1239.85/\lambda_{edge}$$

The tangent to the rise of the phosphorescence spectrum close to the short-wavelength region is drawn as follows. While moving on a curve of the phosphorescence spectrum from the short-wavelength region to the local maximum value closest to the short-wavelength region among the local maximum values of the phosphorescence spectrum, a tangent is checked at each point on the curve toward the long-wavelength of the phosphorescence spectrum. An inclination of the tangent is increased along the rise of the curve (i.e., a value of the ordinate axis is increased). A tangent drawn at a point of the local maximum inclination (i.e., a tangent at an inflection point) is defined as the tangent to the rise of the phosphorescence spectrum close to the short-wavelength region.

A local maximum point where a peak intensity is 15% or less of the maximum peak intensity of the spectrum is not counted as the above-mentioned local maximum peak intensity closest to the short-wavelength region. The tangent drawn at a point that is closest to the local maximum peak intensity closest to the short-wavelength region and where the inclination of the curve is the local maximum is defined as a tangent to the rise of the phosphorescence spectrum close to the short-wavelength region.

For phosphorescence measurement, a spectrophotofluorometer body F-4500 (manufactured by Hitachi High-Technologies Corporation) is usable. Any device for phosphorescence measurement is usable. A combination of a cooling unit, a low temperature container, an excitation light source and a light-receiving unit may be used for phosphorescence measurement.

Lowest Singlet Energy $S_1$

A method of measuring the lowest singlet energy $S_1$ with use of a solution (occasionally referred to as a solution method.) is exemplified by a method below.

A toluene solution of a measurement target compound at a concentration of 10 μmol/L is prepared and put in a quartz cell. An absorption spectrum (ordinate axis: absorption intensity, abscissa axis: wavelength.) of the thus-obtained sample is measured at a normal temperature (300K). A tangent is drawn to the fall of the absorption spectrum close to the long-wavelength region, and a wavelength value λedge (nm) at an intersection of the tangent and the abscissa axis is assigned to a conversion equation (F2) below to calculate the lowest singlet energy.

$$\text{Conversion Equation (F2): } S_1\ [\text{eV}] = 1239.85/\lambda\text{edge}$$

Any apparatus for measuring absorption spectrum is usable. For instance, a spectrophotometer (U3310 manufactured by Hitachi, Ltd.) is usable.

The tangent to the fall of the absorption spectrum close to the long-wavelength region is drawn as follows. While moving on a curve of the absorption spectrum from the local maximum value closest to the long-wavelength region, among the local maximum values of the absorption spectrum, in a long-wavelength direction, a tangent at each point on the curve is checked. An inclination of the tangent is decreased and increased in a repeated manner as the curve falls (i.e., a value of the ordinate axis is decreased). A tangent drawn at a point where the inclination of the curve is the local minimum closest to the long-wavelength region (except when absorbance is 0.1 or less) is defined as the tangent to the fall of the absorption spectrum close to the long-wavelength region.

The local maximum absorbance of 0.2 or less is not counted as the above-mentioned local maximum absorbance closest to the long-wavelength region.

Producing Method of Compound in the Exemplary Embodiment

The compound according to the exemplary embodiment can be produced by application of known substitution reactions and materials depending on a target compound, according to a synthesis method described in Examples described later or in a similar manner as the synthesis method.

Specific Examples of Compound in the Exemplary Embodiment

Specific examples of the compound in the exemplary embodiment include compounds below. However, the invention is by no means limited to the specific examples. Herein, a deuterium atom is denoted as D in formulae, and a protium atom is denoted as H or omitted.

[Formula 53]

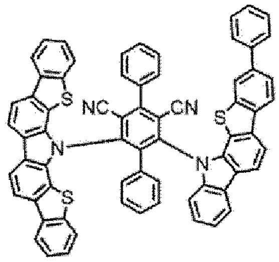
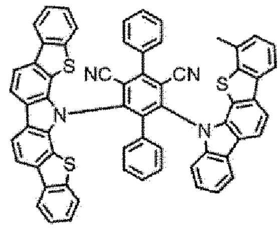
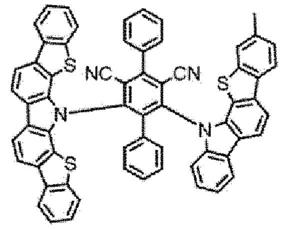
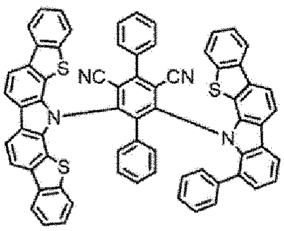
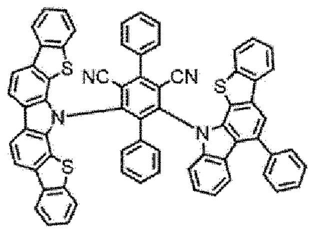
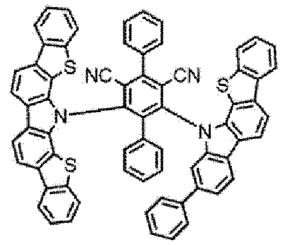
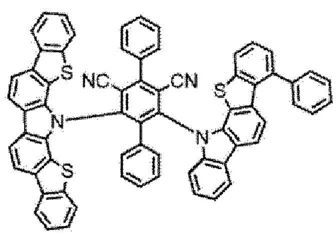
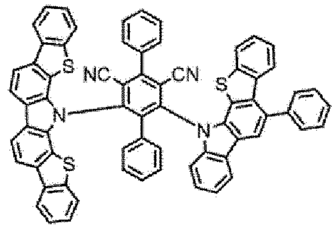
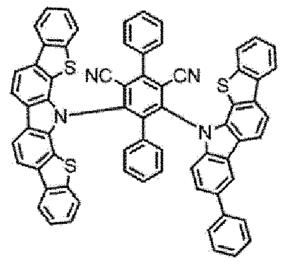
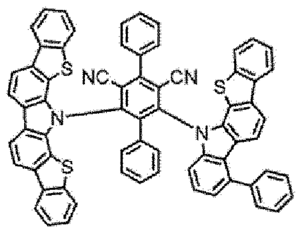
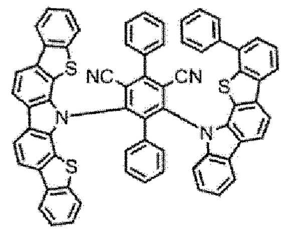
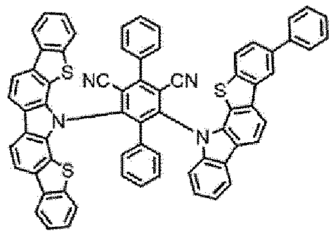
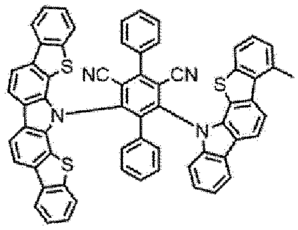
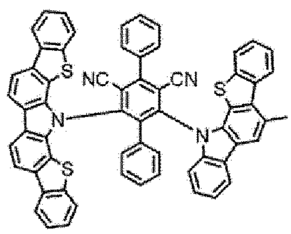
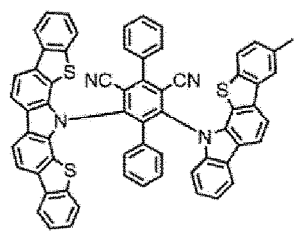

[Formula 54]

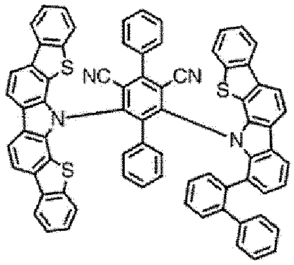
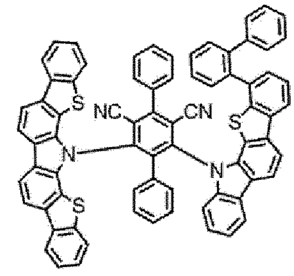
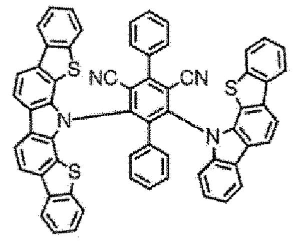
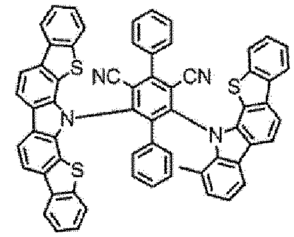
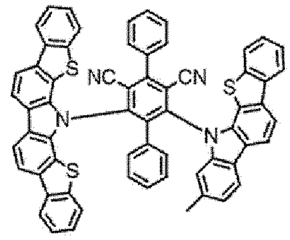
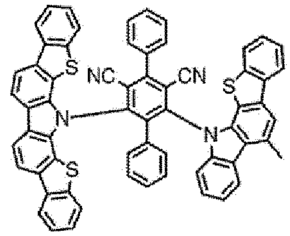
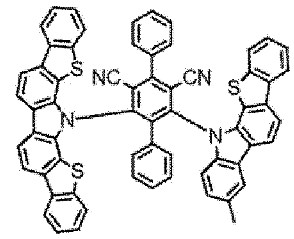
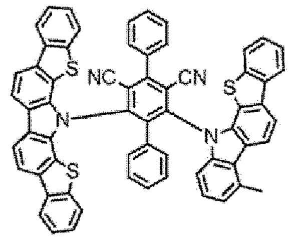

[Formula 55]

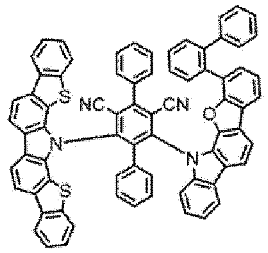
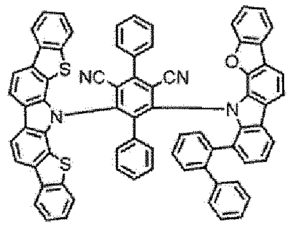
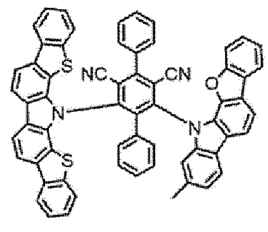

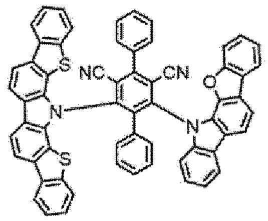
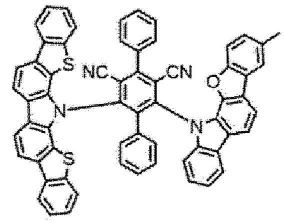
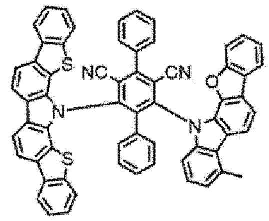

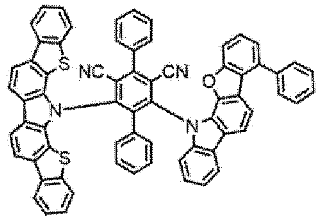
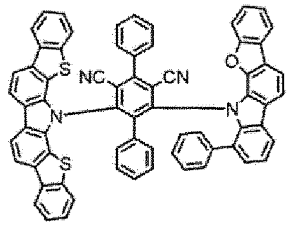
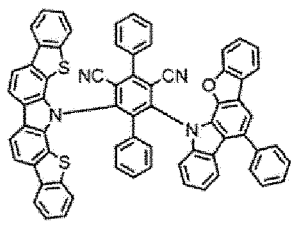

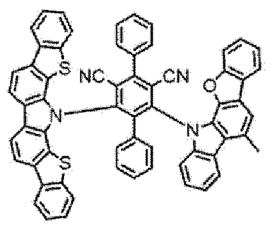
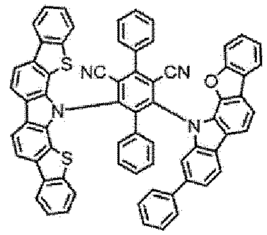
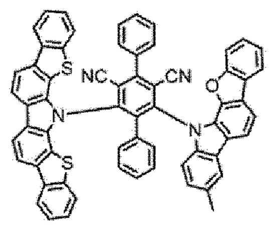

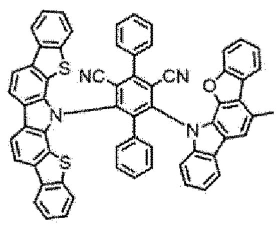
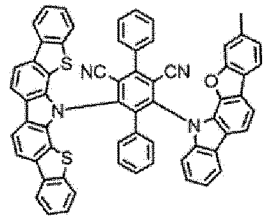
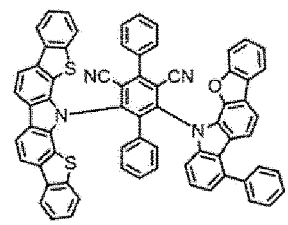

[Formula 56]

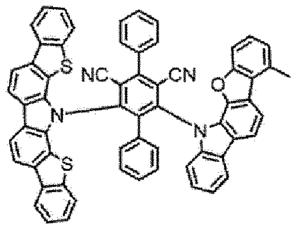

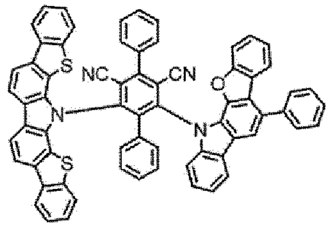

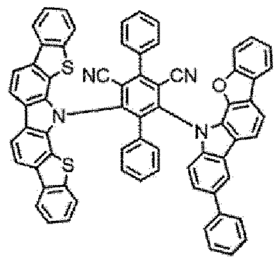

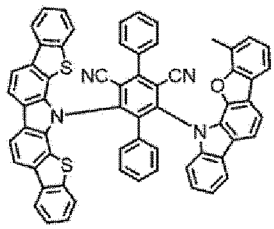

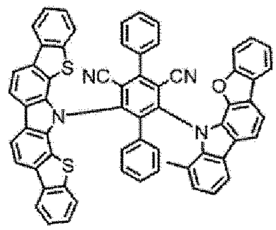

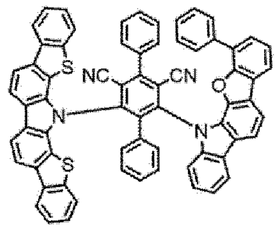

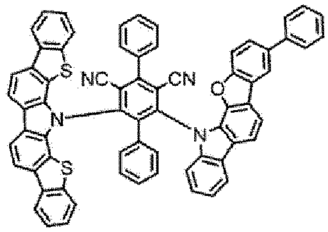

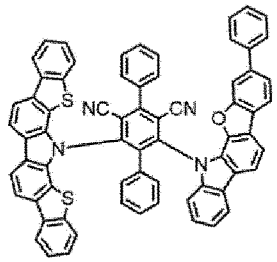

[Formula 57]

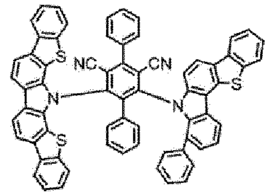 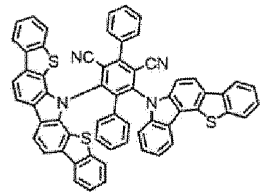 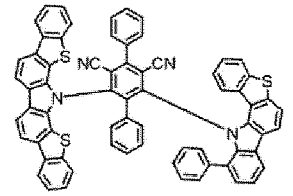

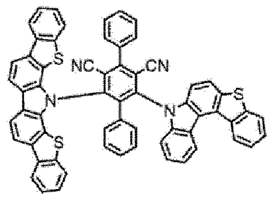 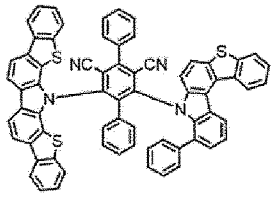 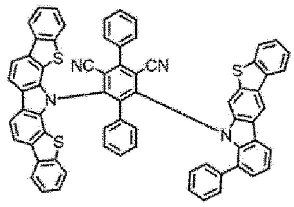

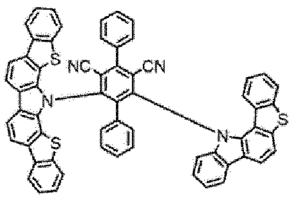 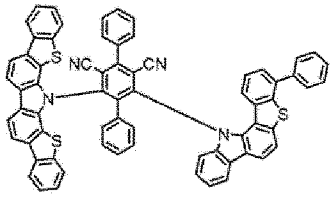 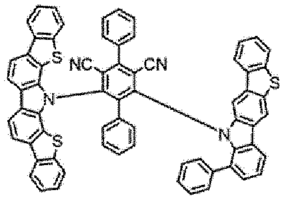

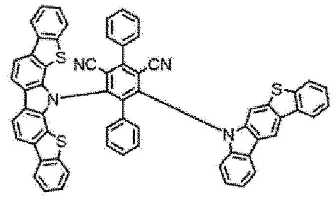 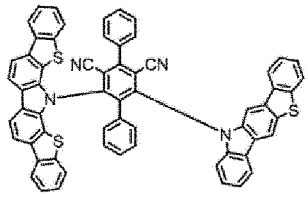

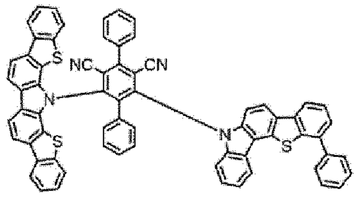 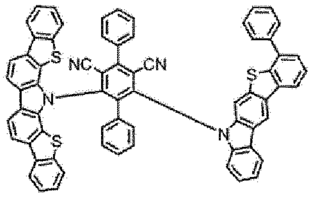

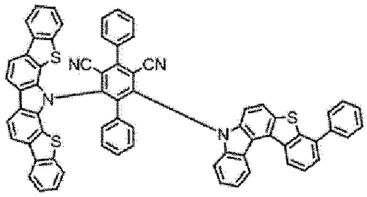 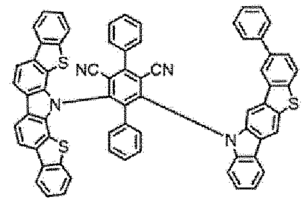

[Formula 58]

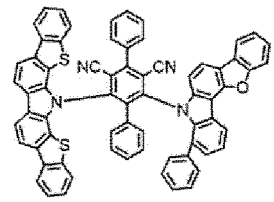
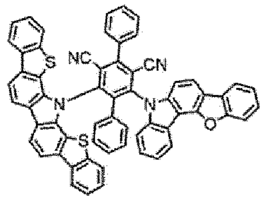
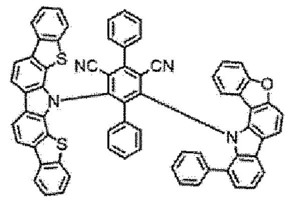
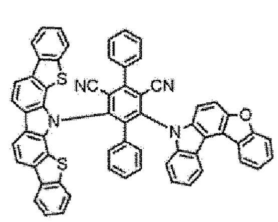
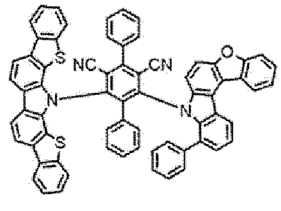
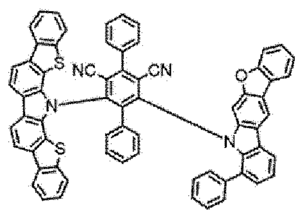
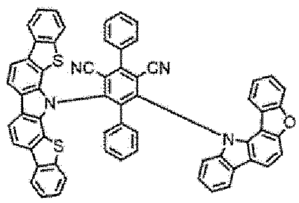
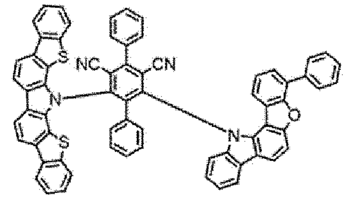
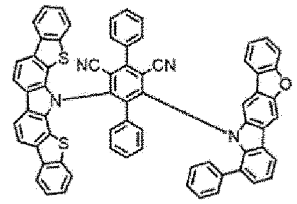
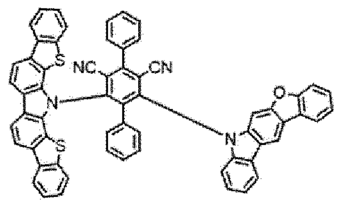
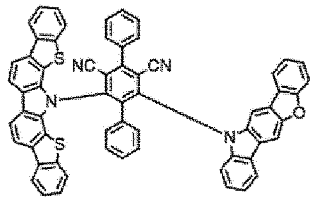
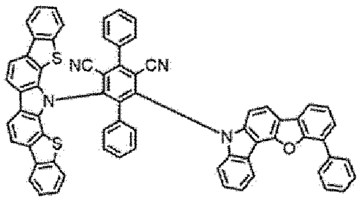
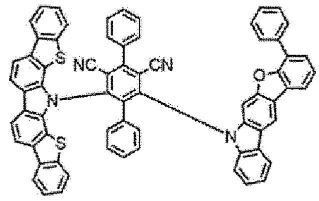
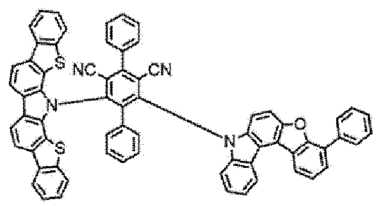

[Formula 59]

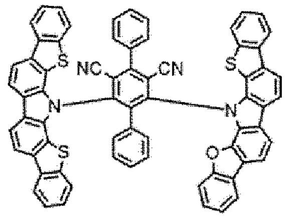
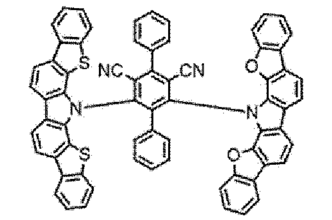
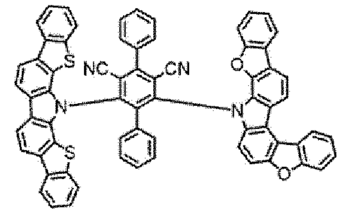
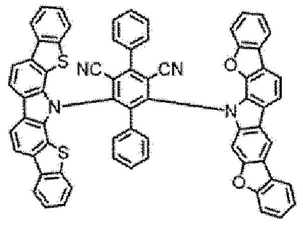
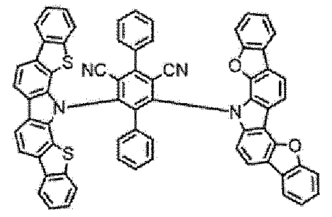
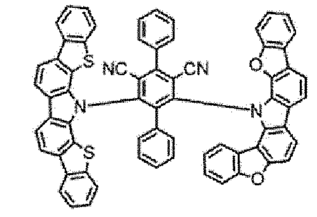
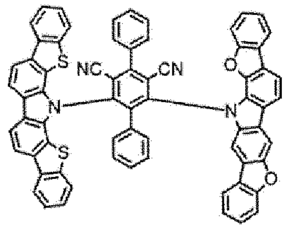
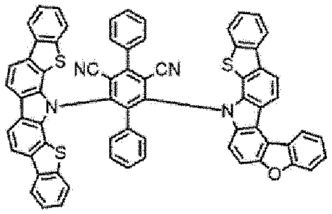
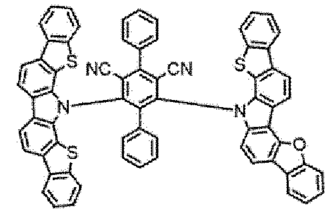
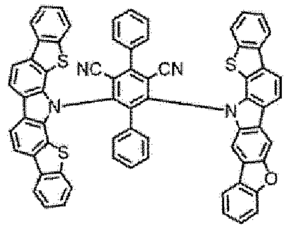
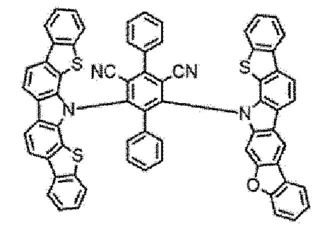
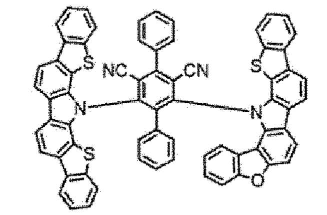

[Formula 60]

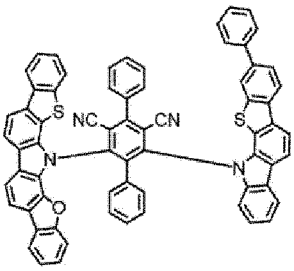 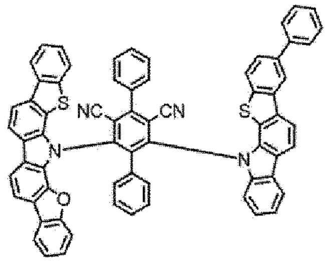 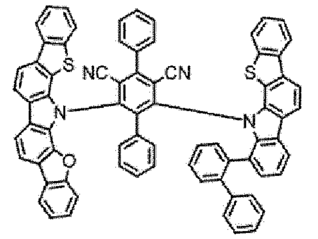

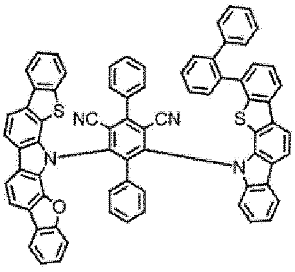 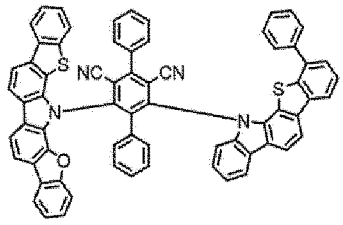 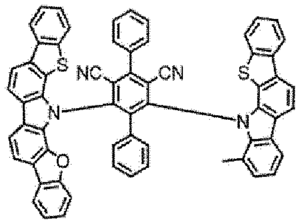

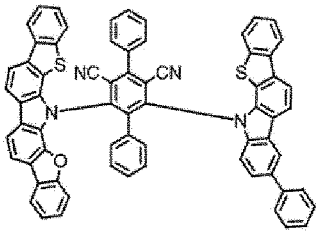 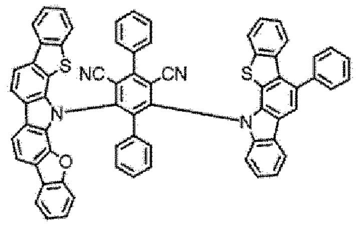 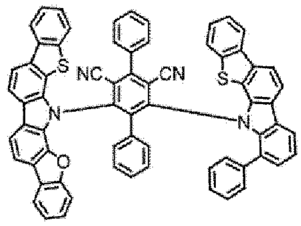

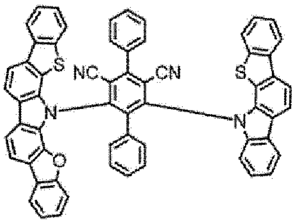 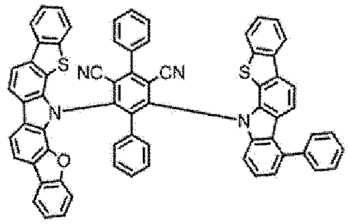 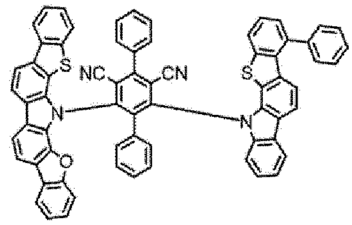

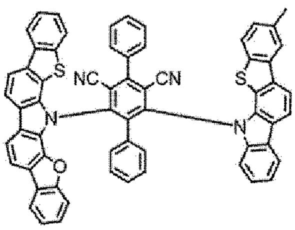 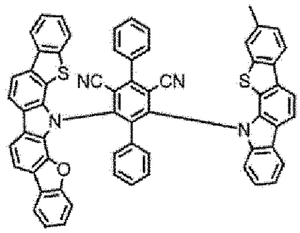 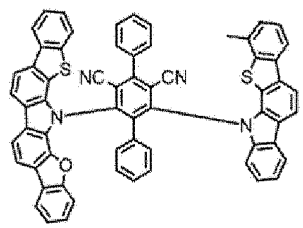

[Formula 61]

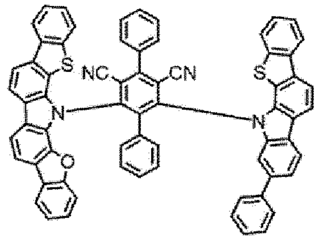
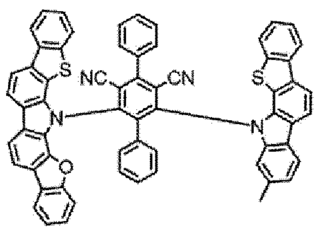
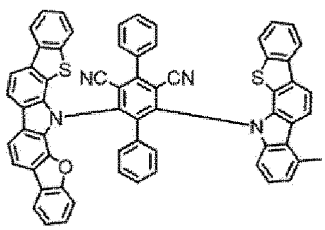
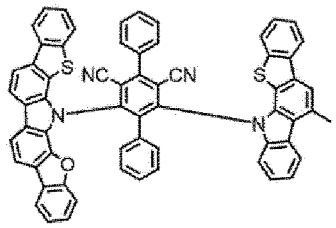
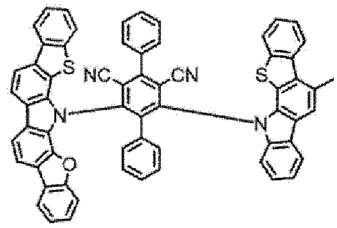
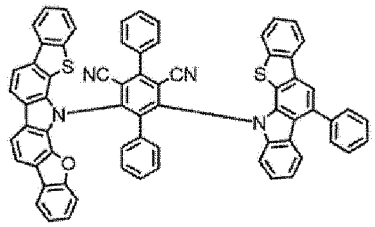
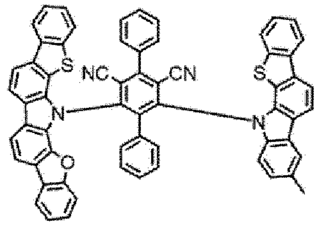
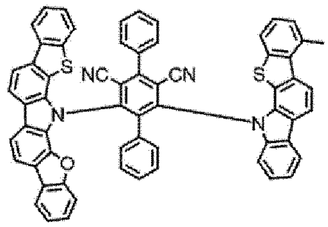

[Formula 62]

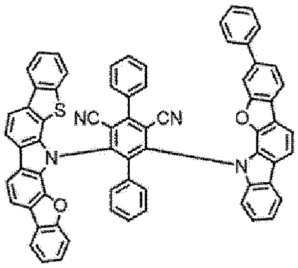
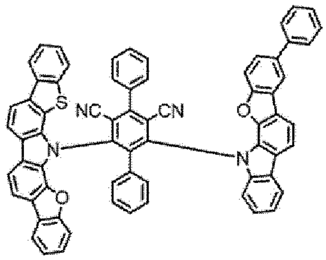
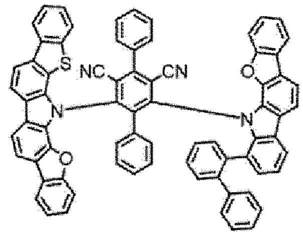

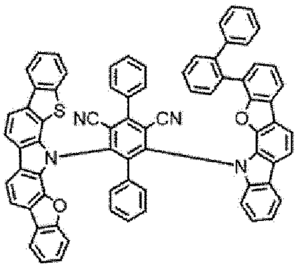
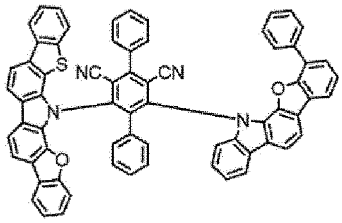
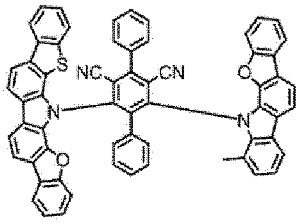

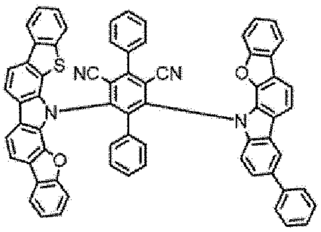
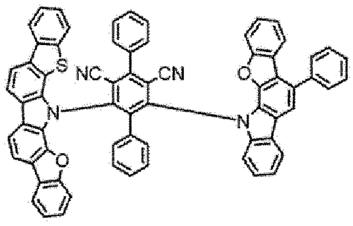
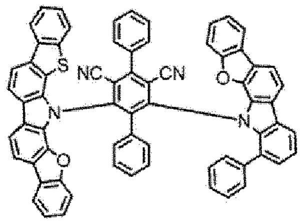

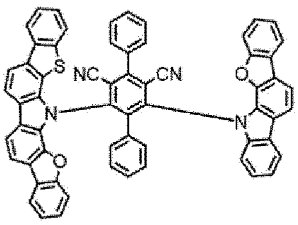
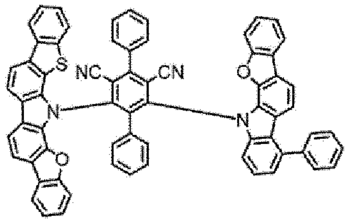
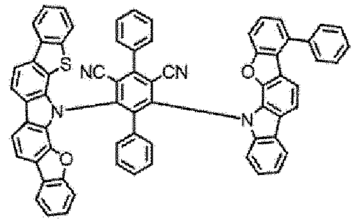

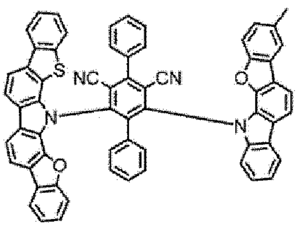
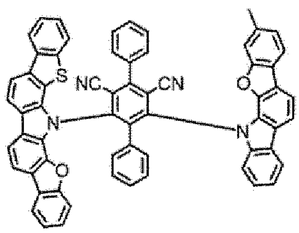
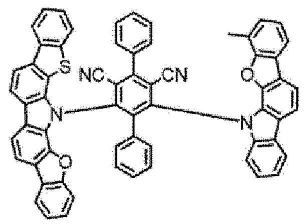

[Formula 63]

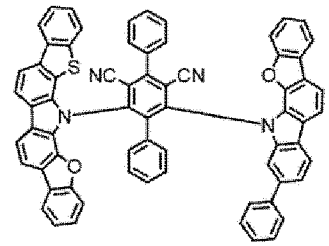
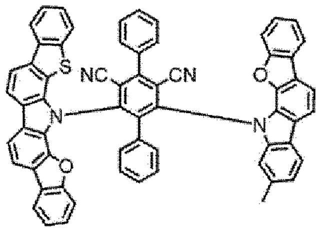
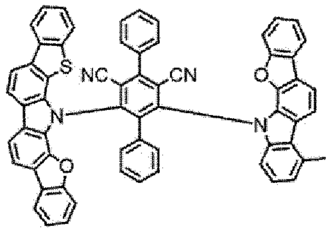
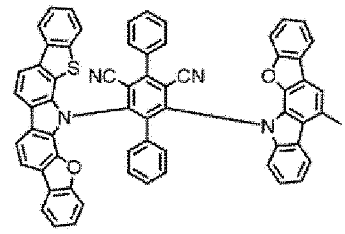
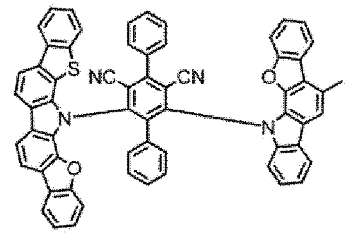
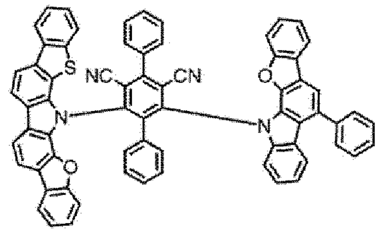
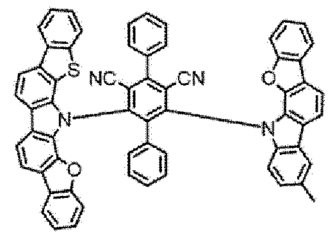
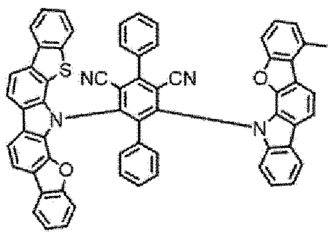

[Formula 64]

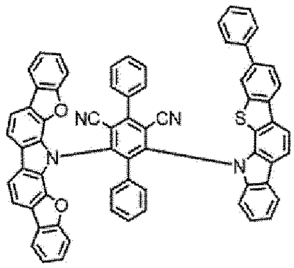
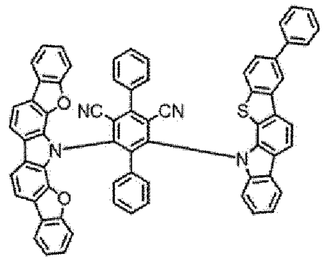
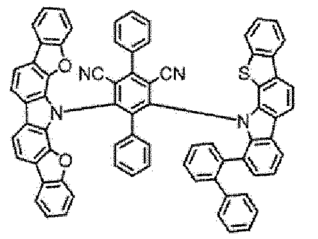

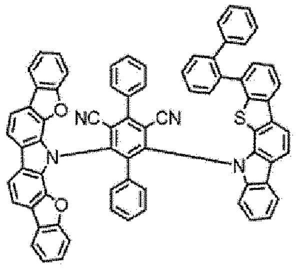
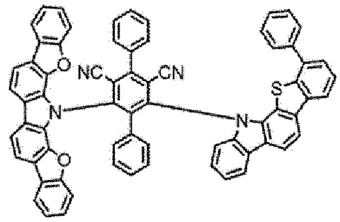
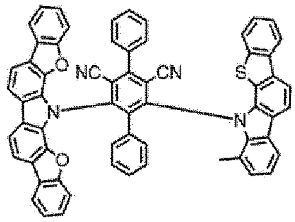

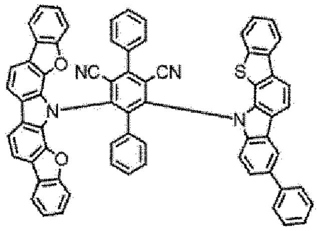
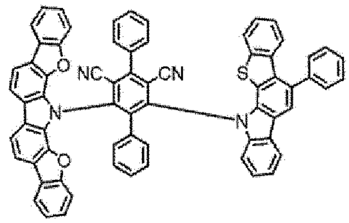
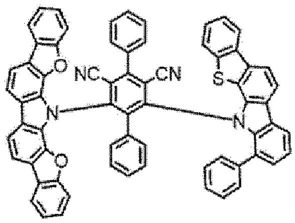

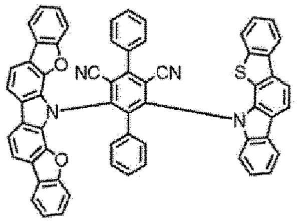
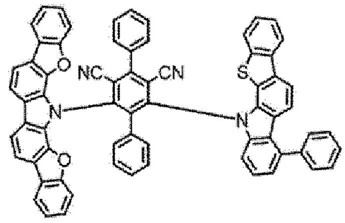
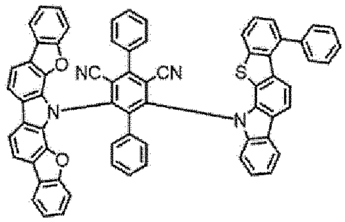

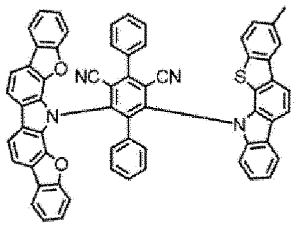
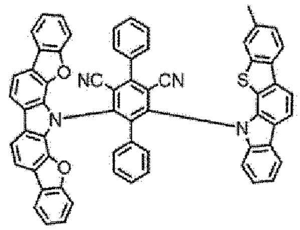
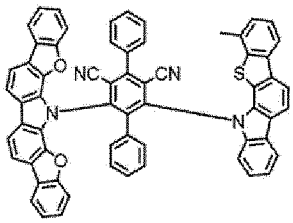

[Formula 65]

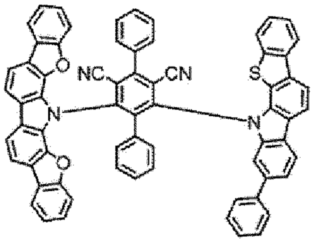
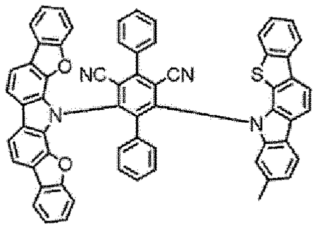
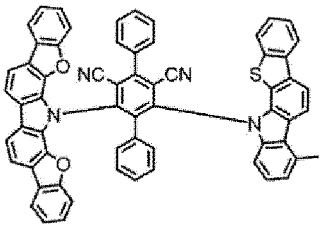
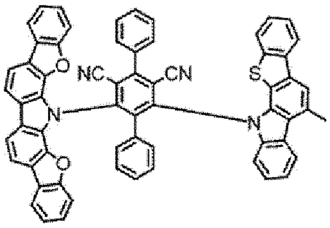
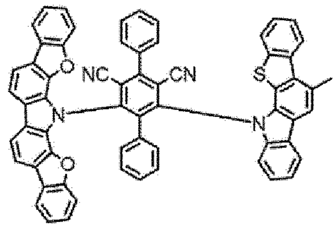
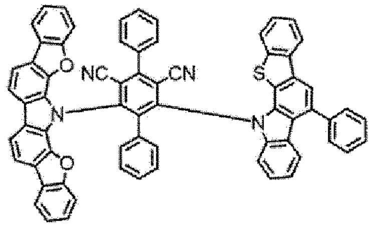
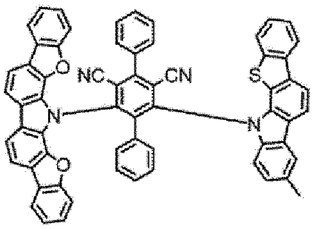
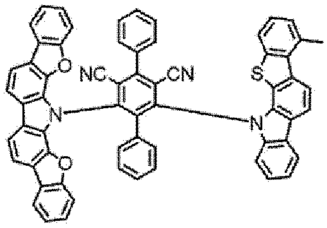

[Formula 66]

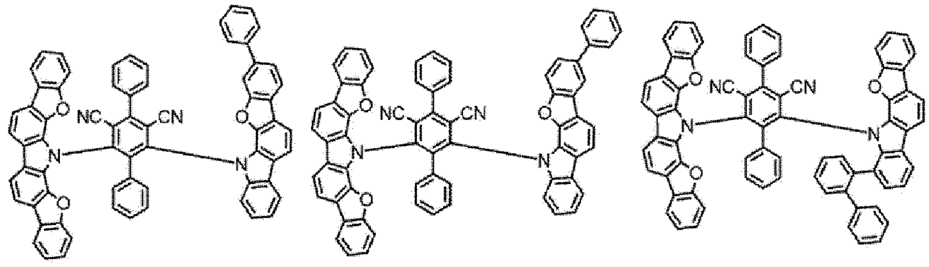

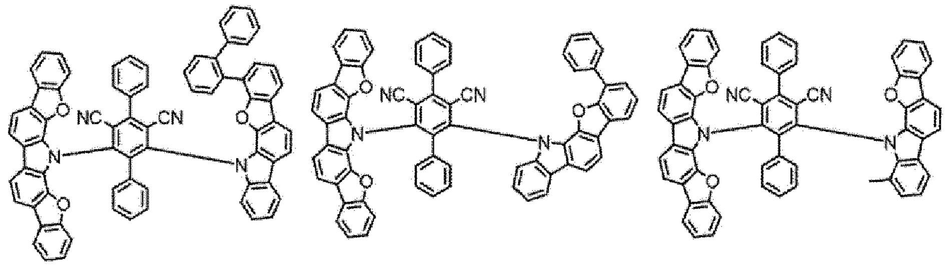

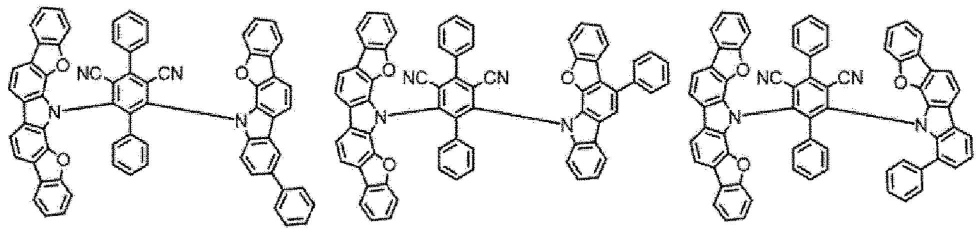

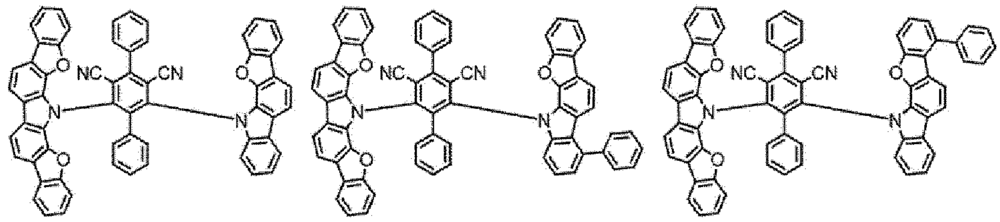

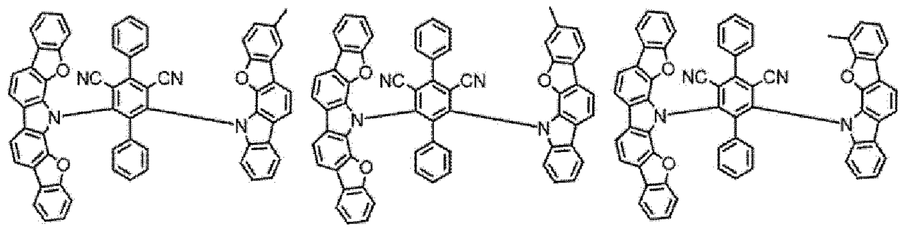

[Formula 67]

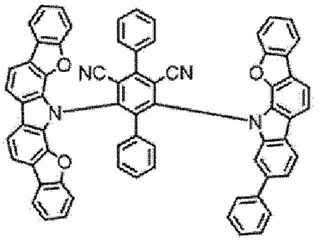
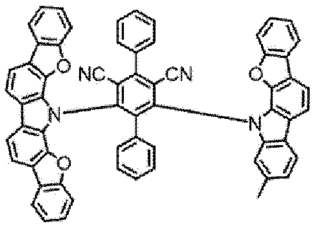
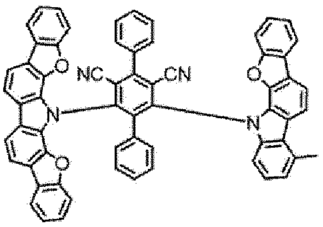

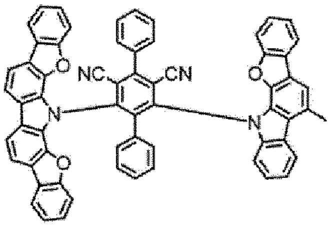
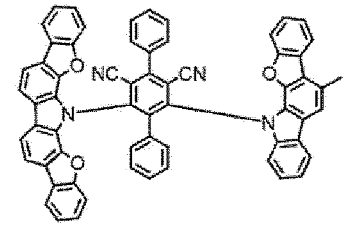
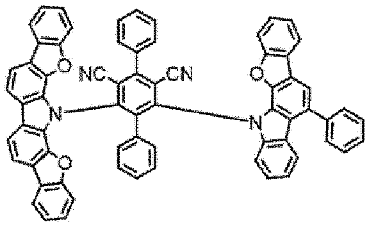

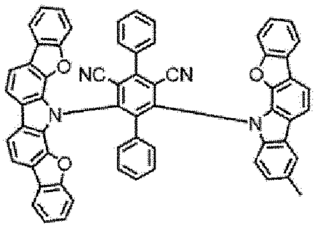
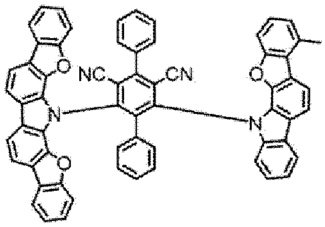

[Formula 68]

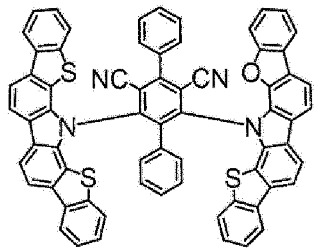
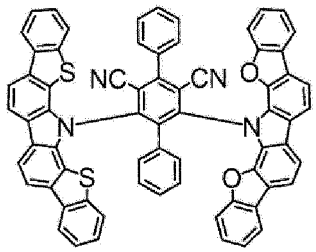

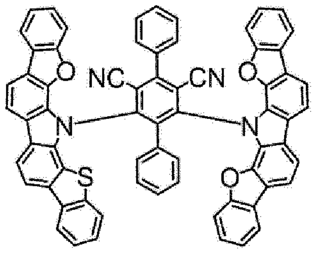
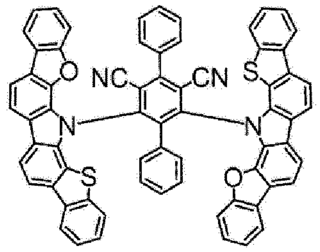
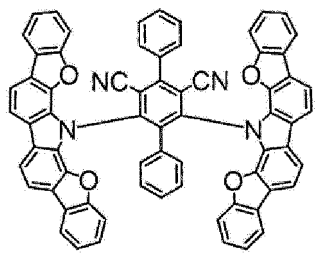

[Formula 69]

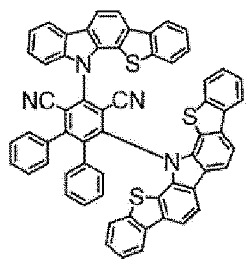
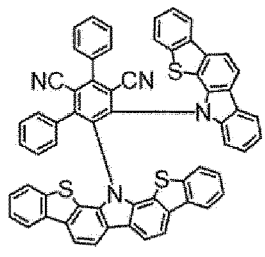
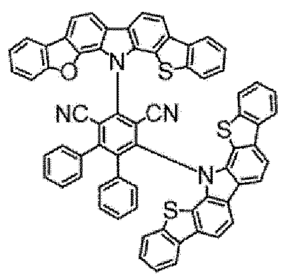
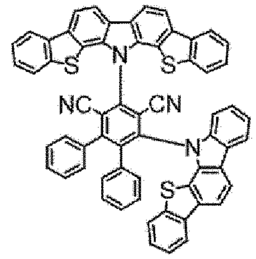
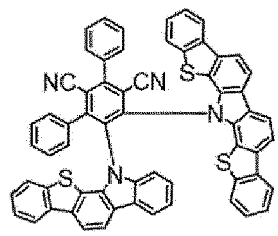
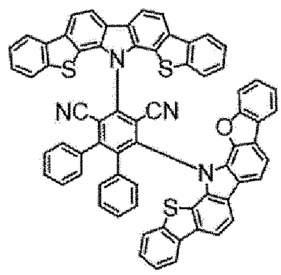
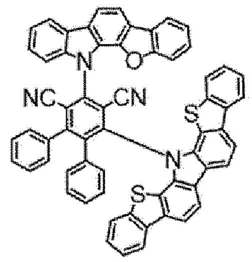
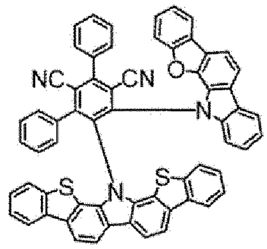
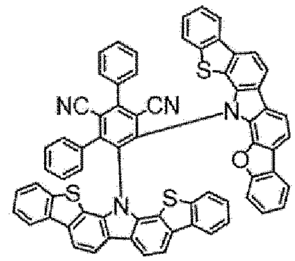
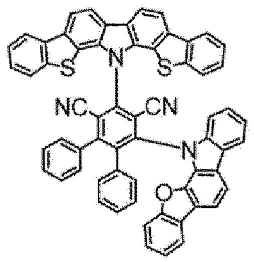
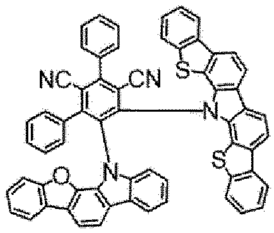
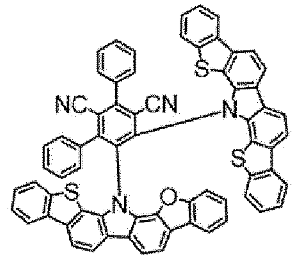

[Formula 70]

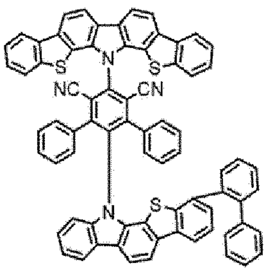
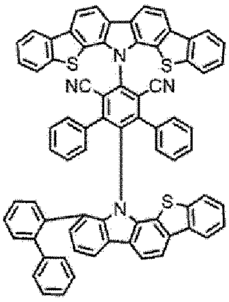
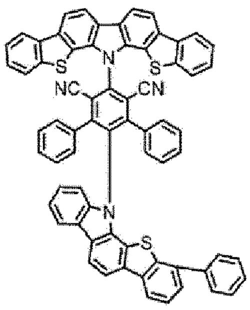
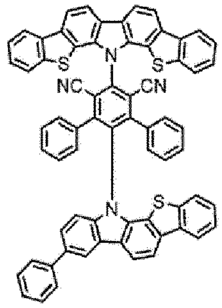
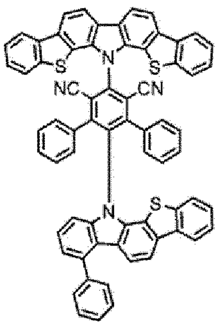
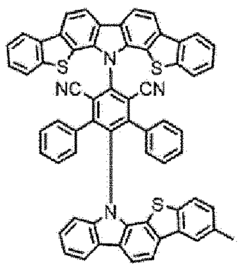
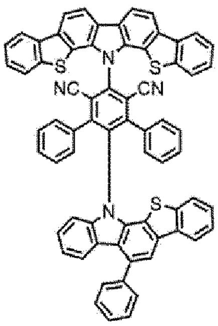
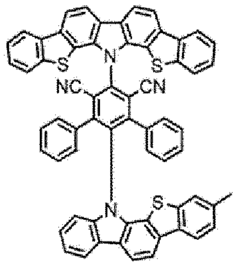
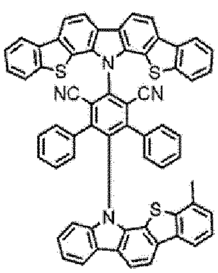
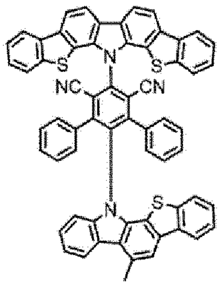
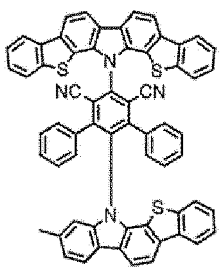
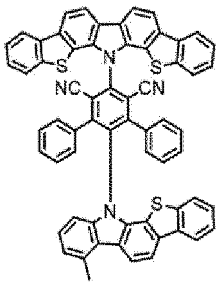
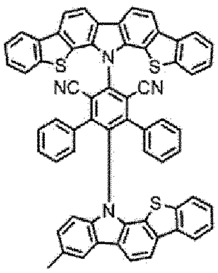
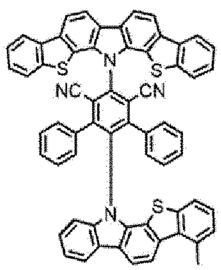
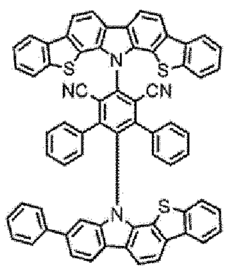
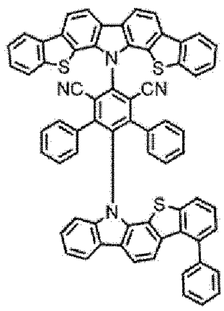

[Formula 71]

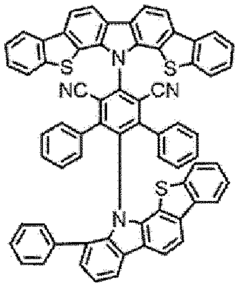
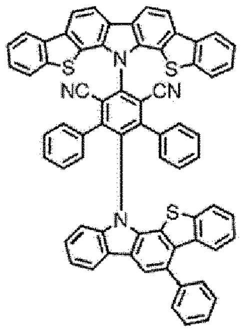
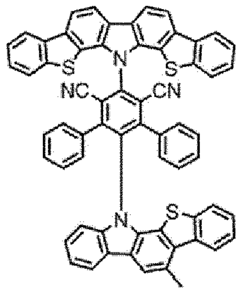
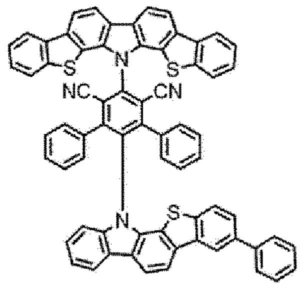

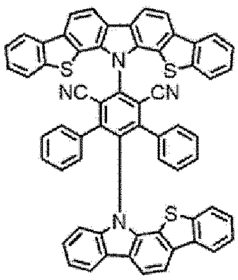
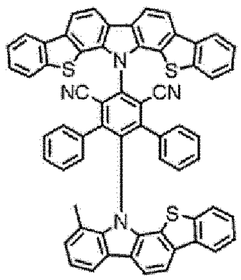
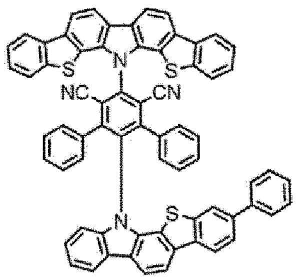

[Formula 72]

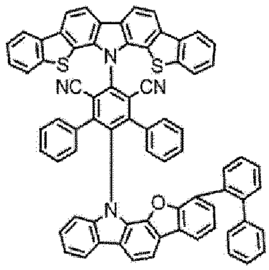
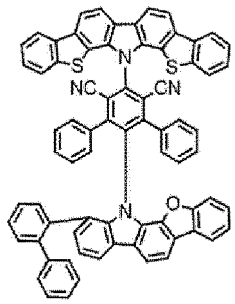
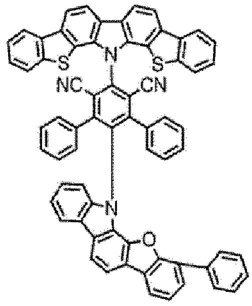
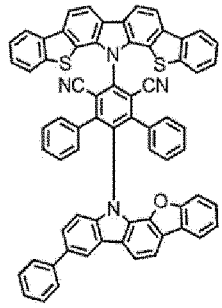
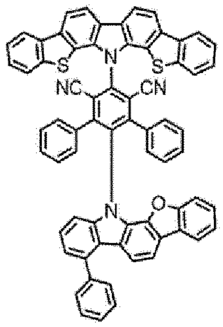
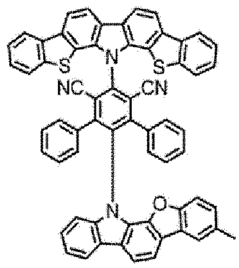
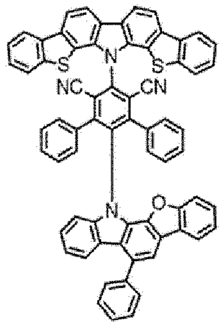
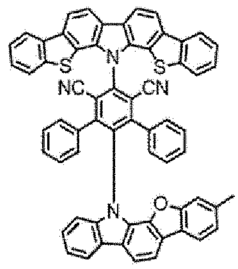
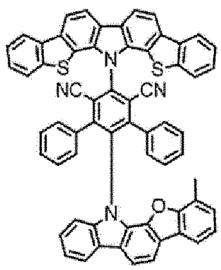
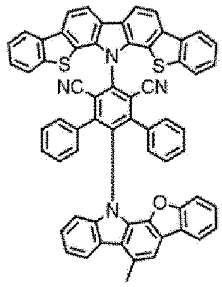
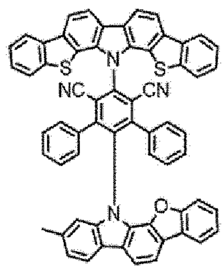
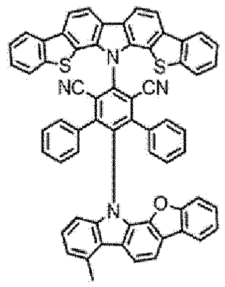
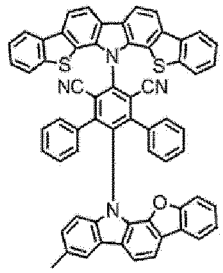
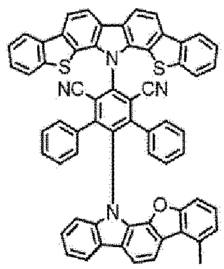
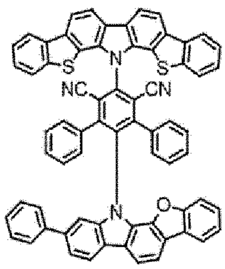
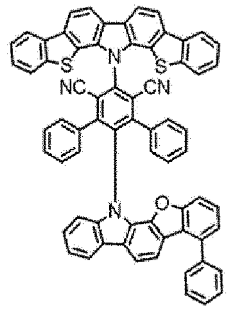

[Formula 73]

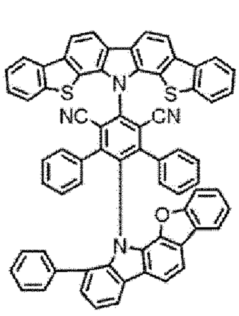
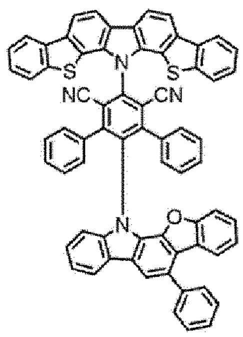
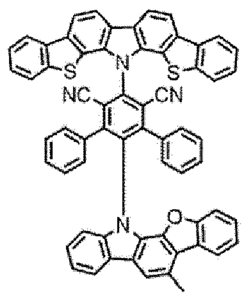
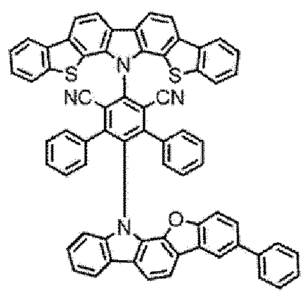
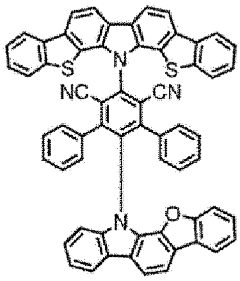
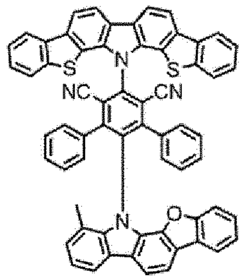
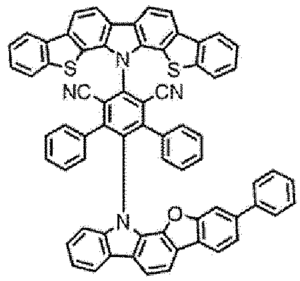

[Formula 74]

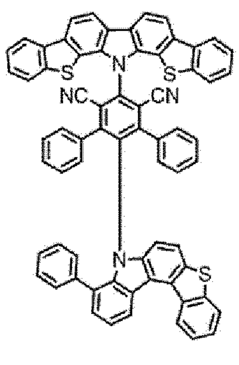
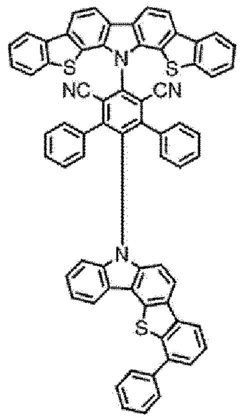
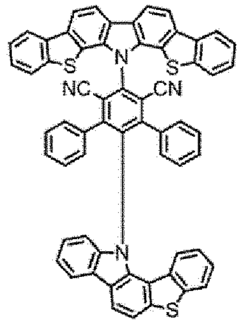
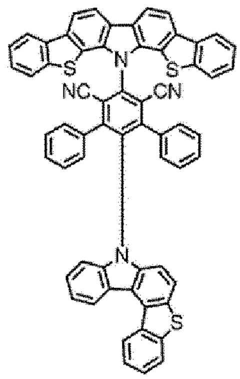
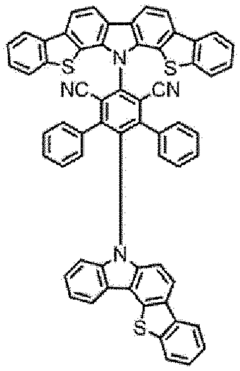
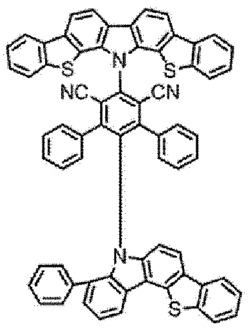
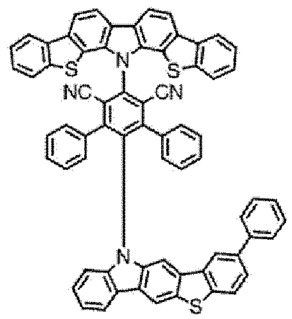
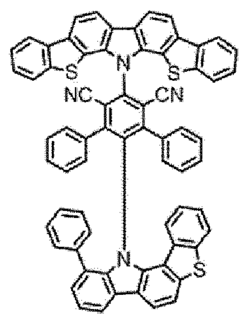

[Formula 75]

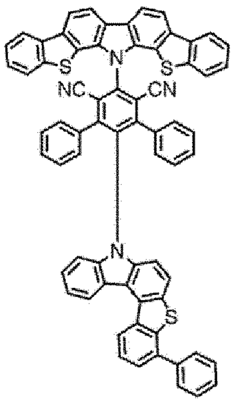
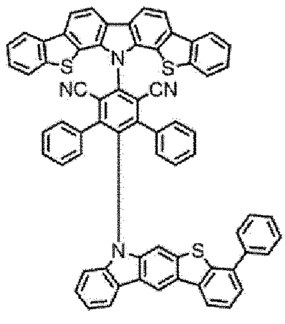
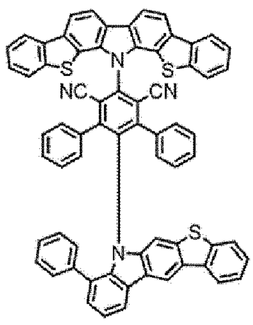
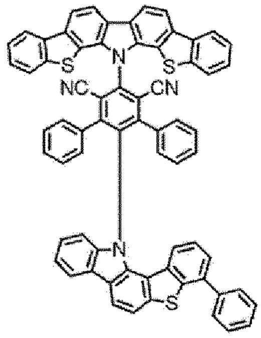
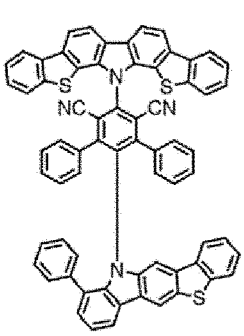
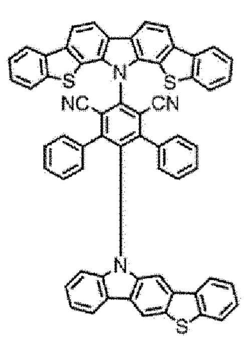
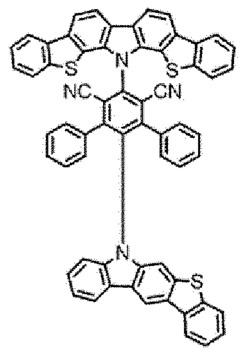

[Formula 76]

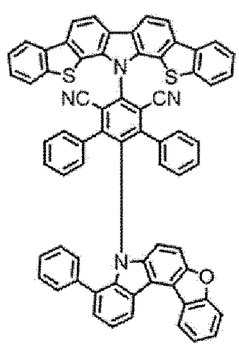
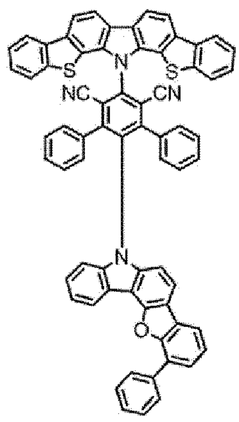
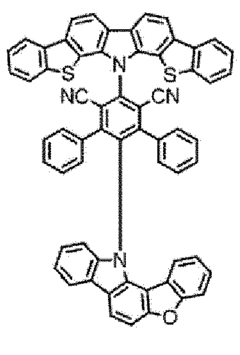
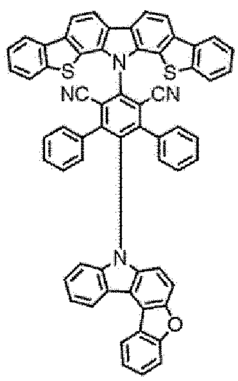
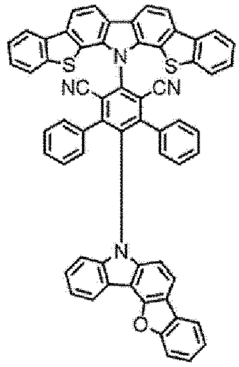
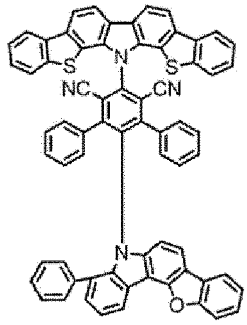
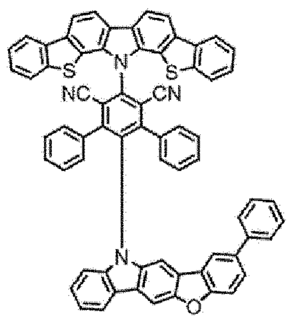
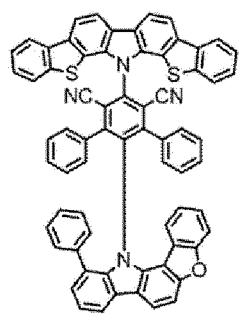

[Formula 77]

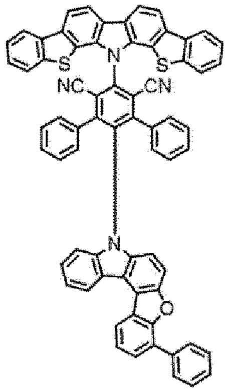
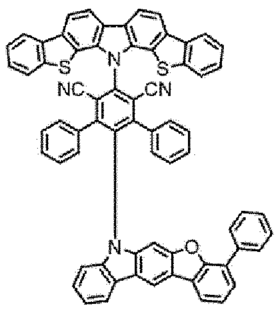
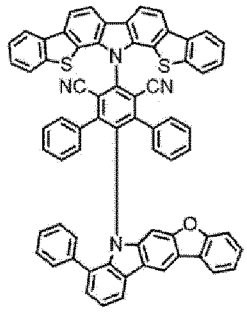
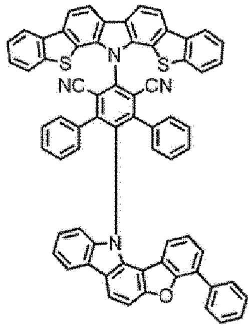
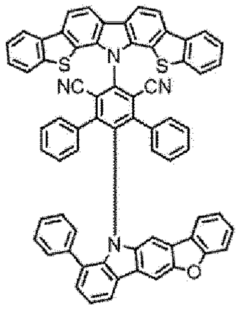
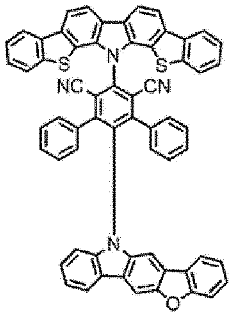
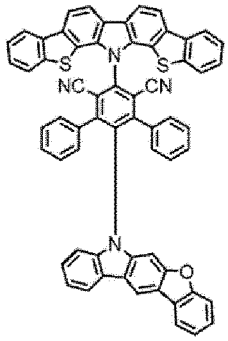

[Formula 78]

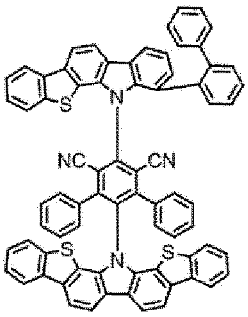
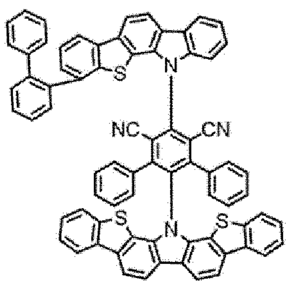
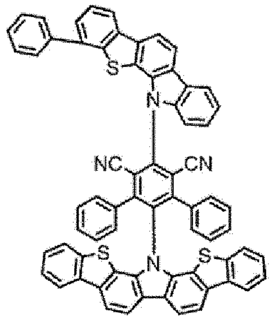
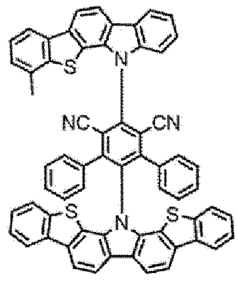
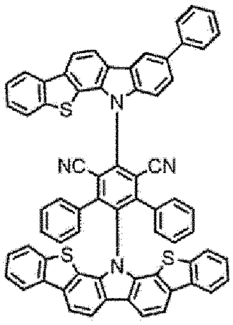
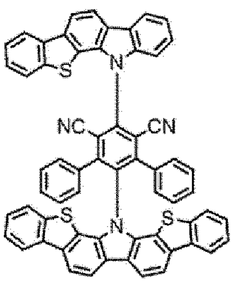
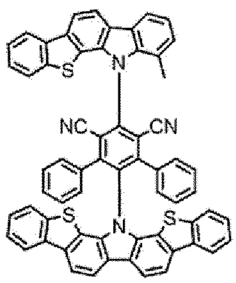
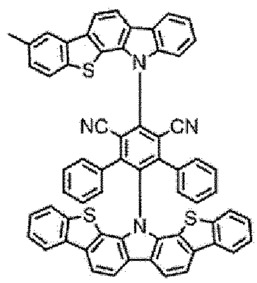

[Formula 79]

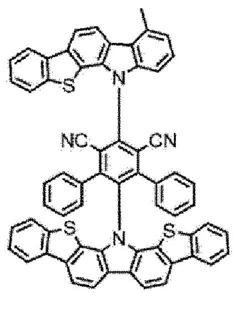
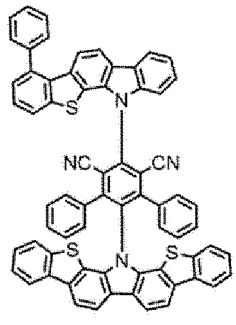
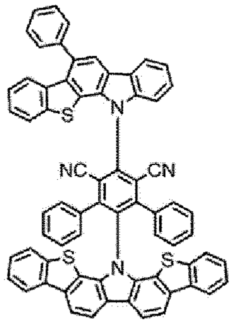
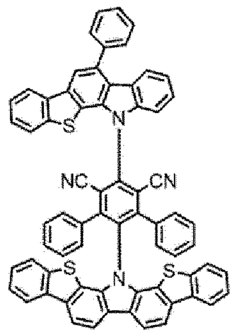
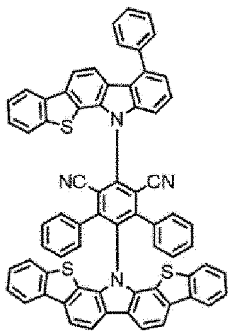
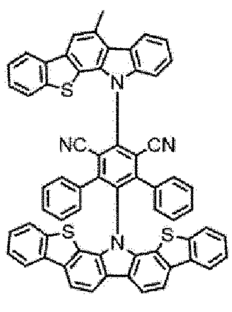
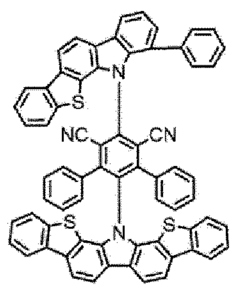
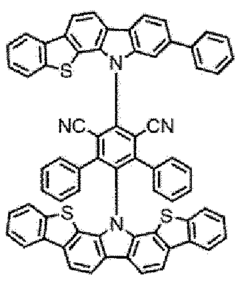
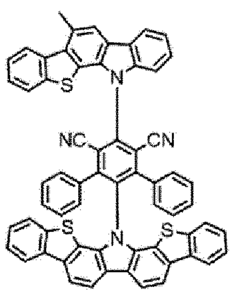
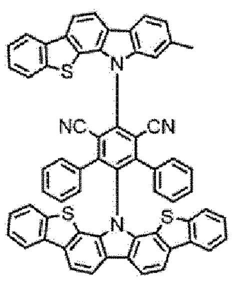
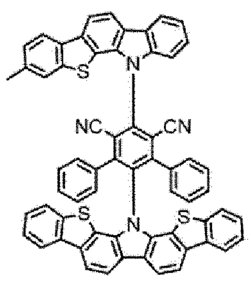
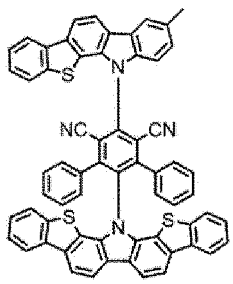
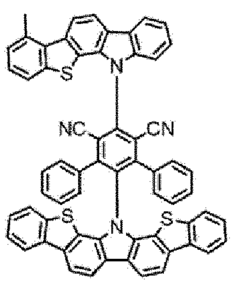
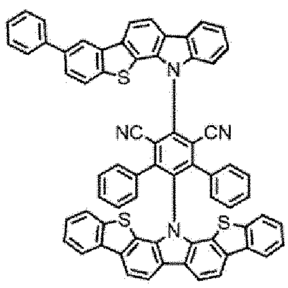
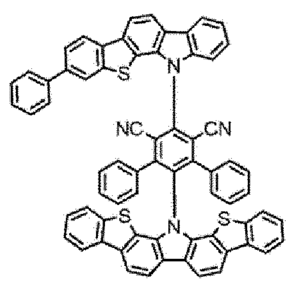

[Formula 80]

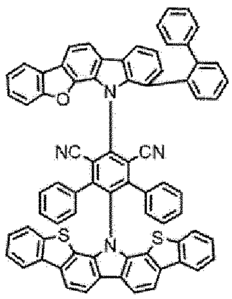
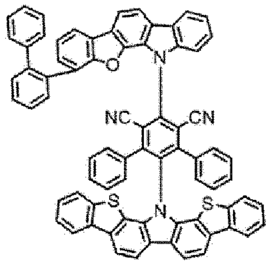
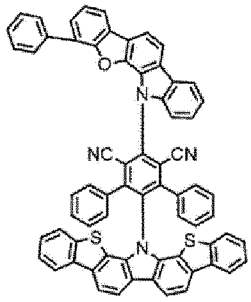
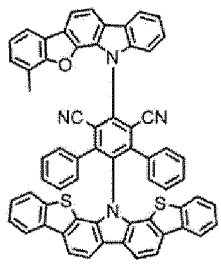
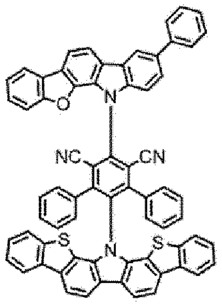
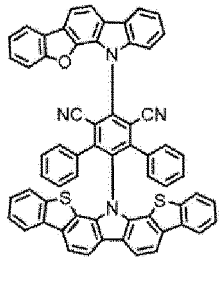
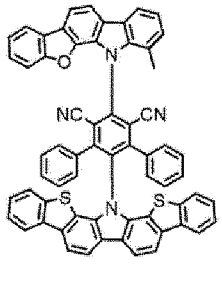
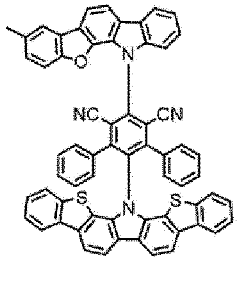
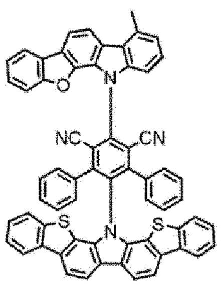
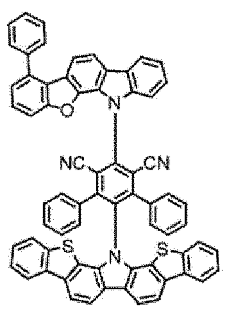
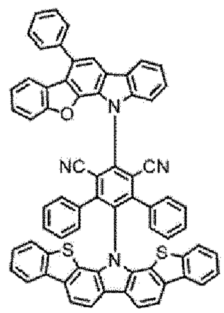
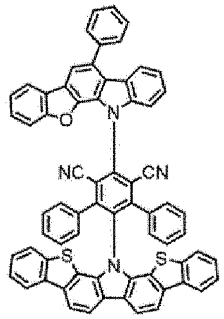
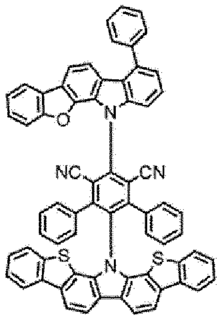
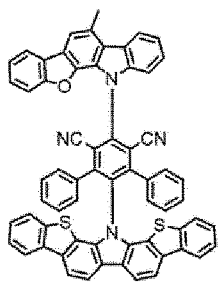
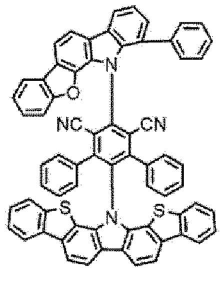
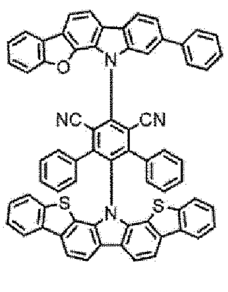

[Formula 81]

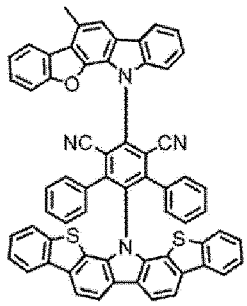 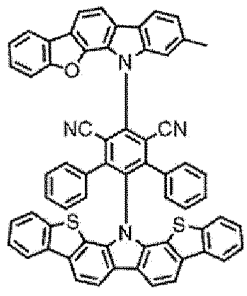 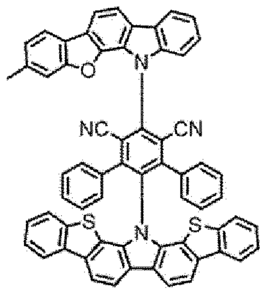 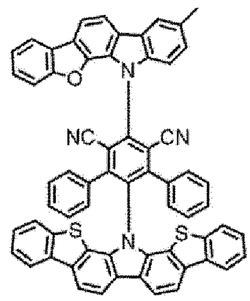

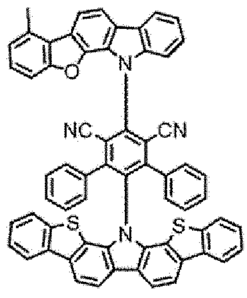 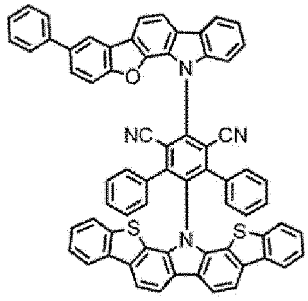 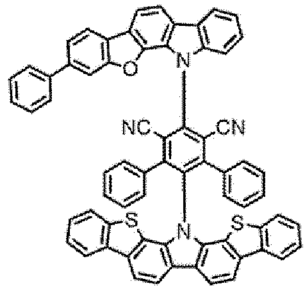

[Formula 82]

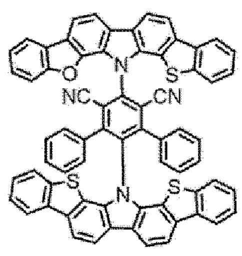 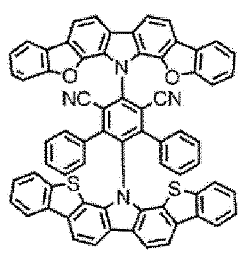 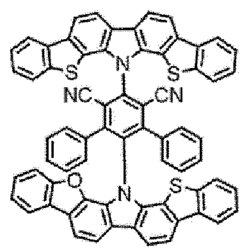

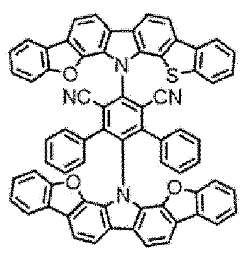 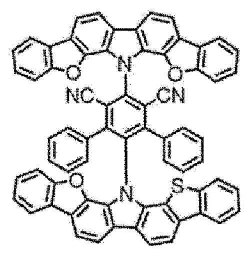 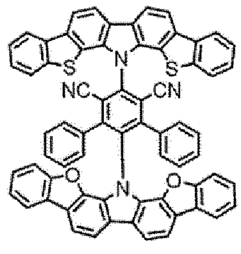

[Formula 83]

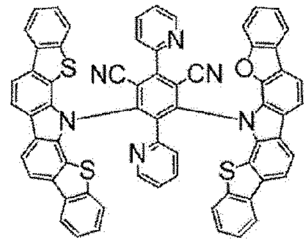
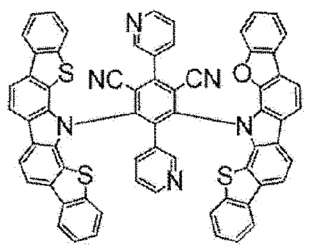
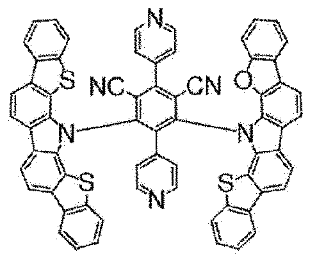

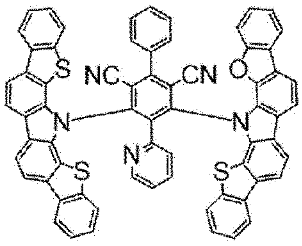
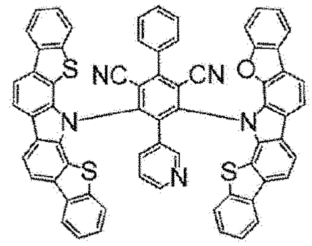
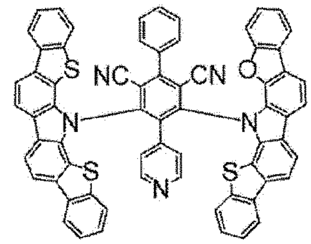

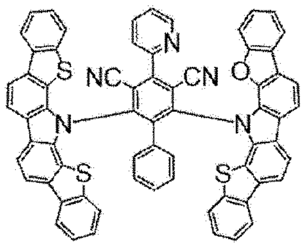
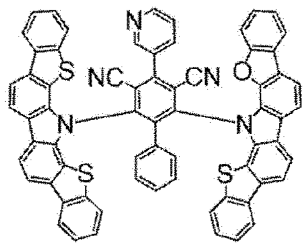
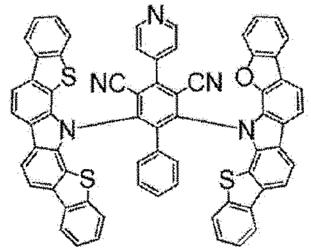

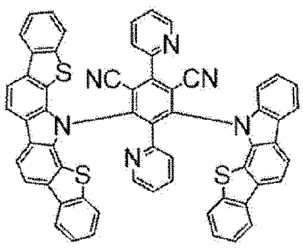
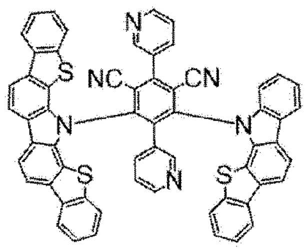
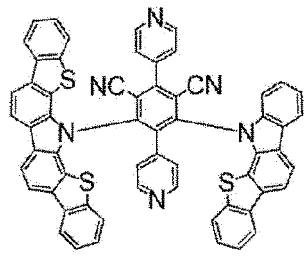

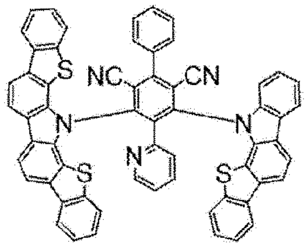
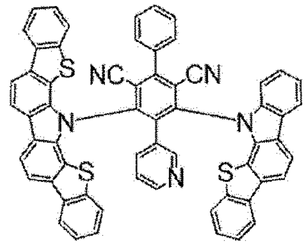
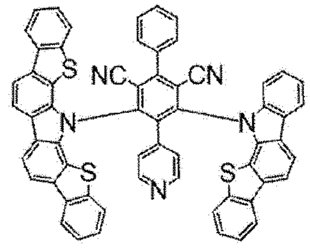

[Formula 84]

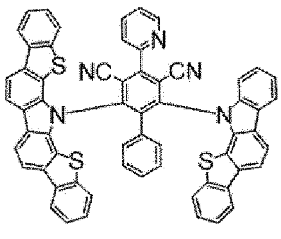
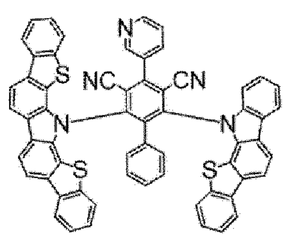
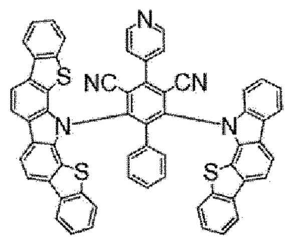

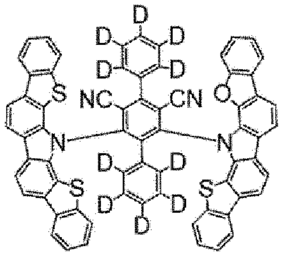
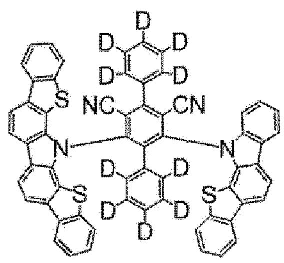
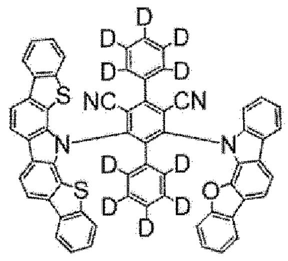

[Formula 85]

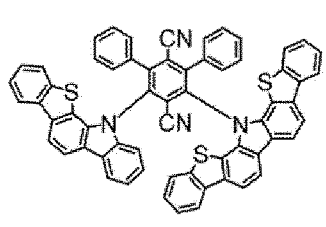
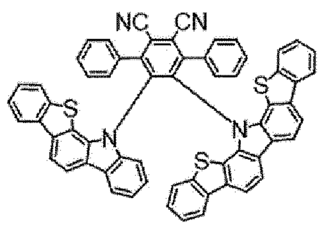
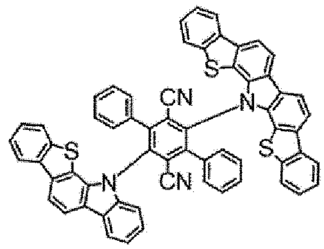

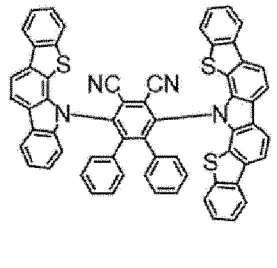
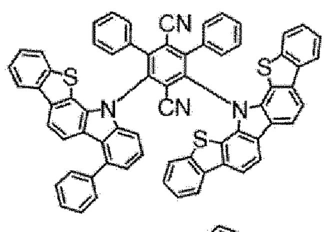
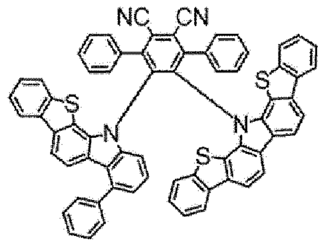

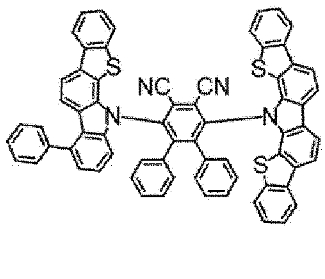
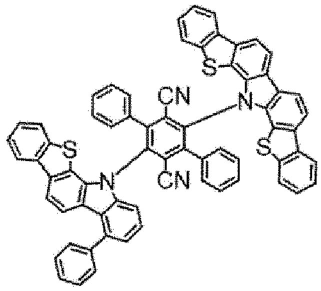

[Formula 86]

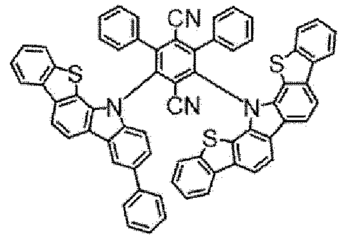
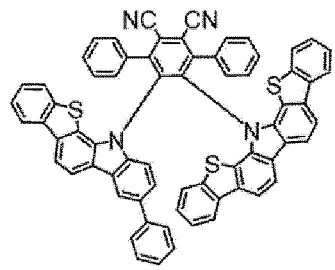
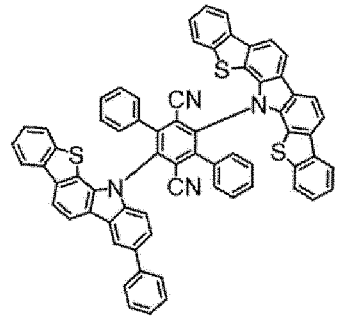
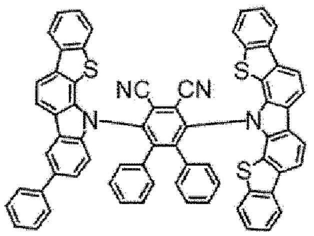
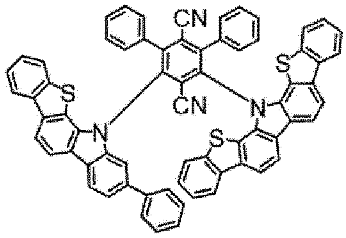
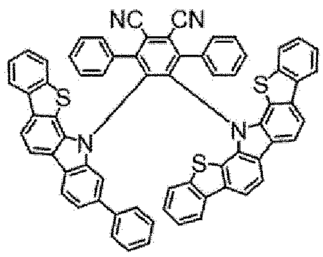
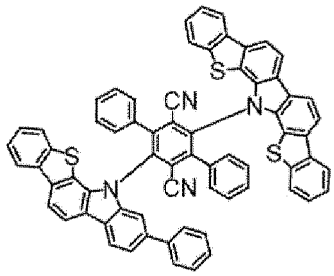
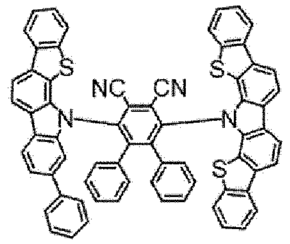

[Formula 87]

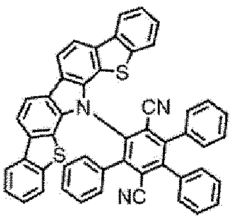
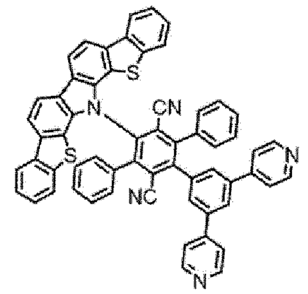
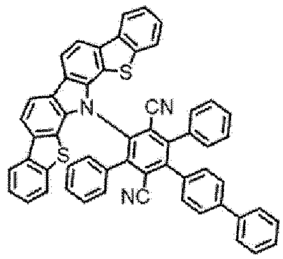
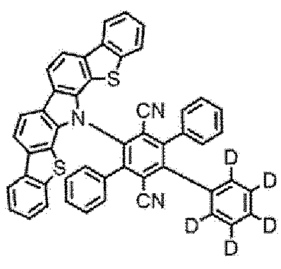
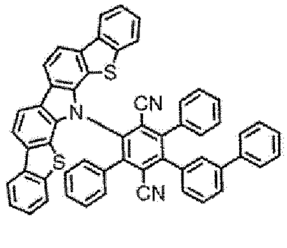
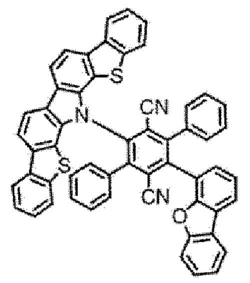

[Formula 88]

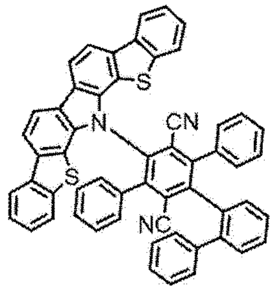

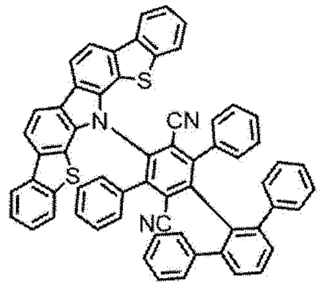

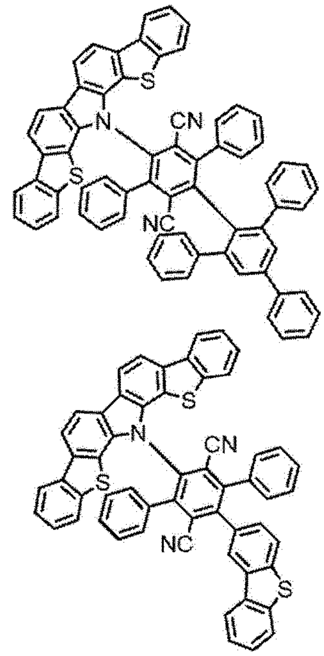

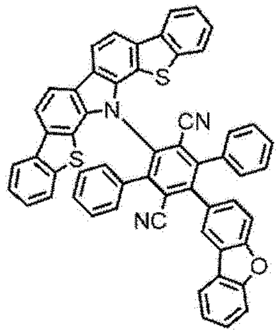

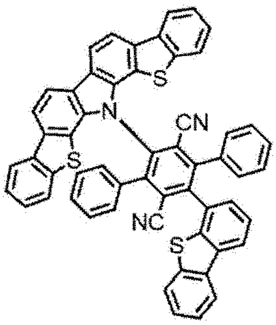

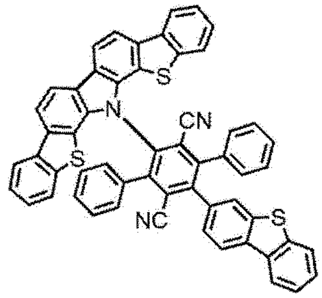

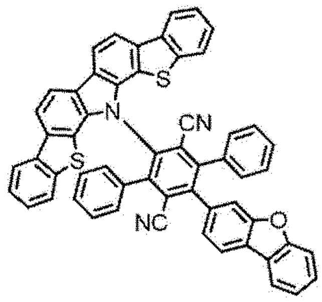

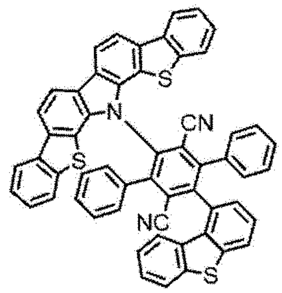

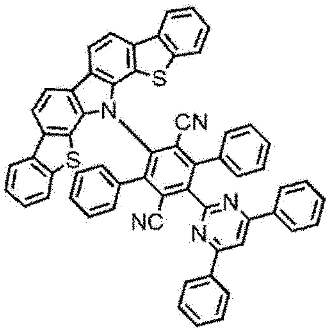

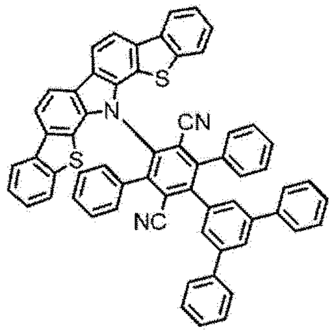

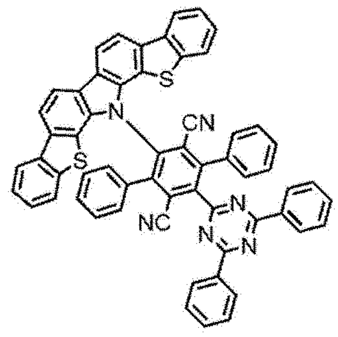

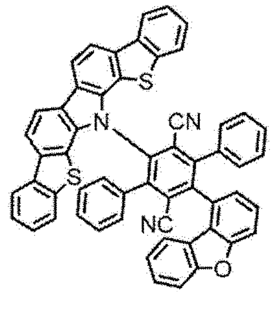

[Formula 89]

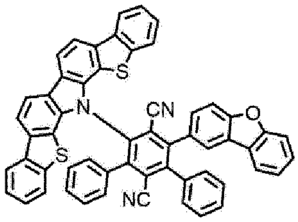
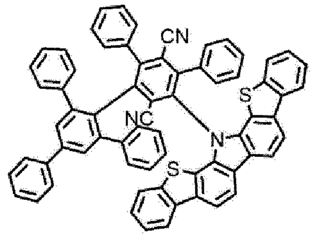
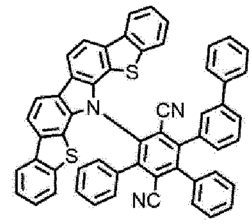
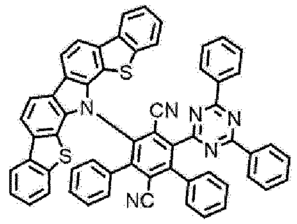
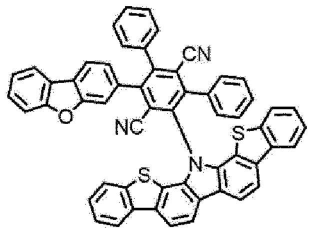
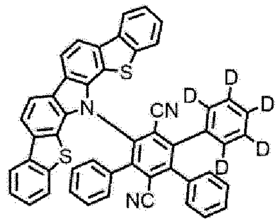
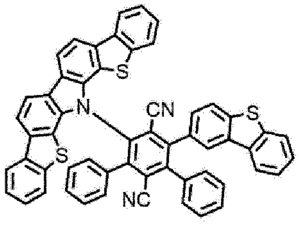
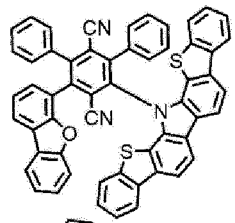
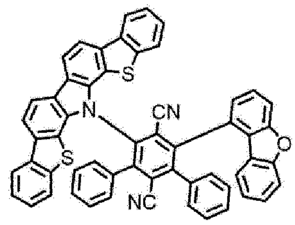
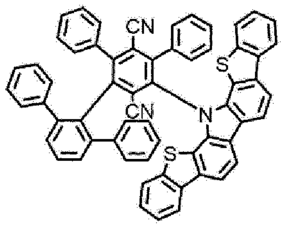
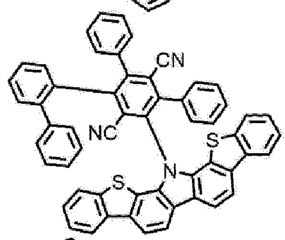
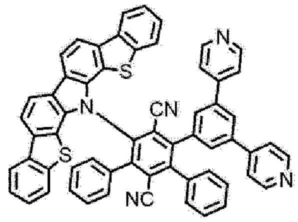
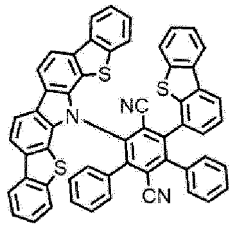
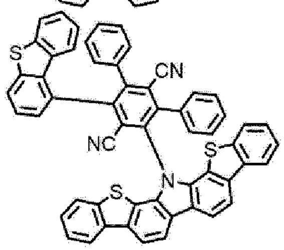
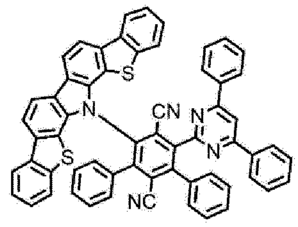

[Formula 90]

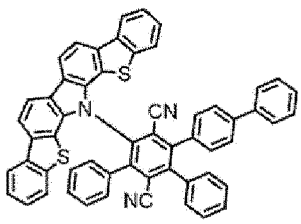
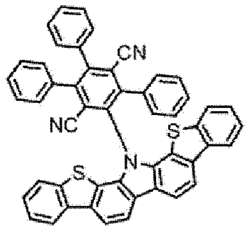
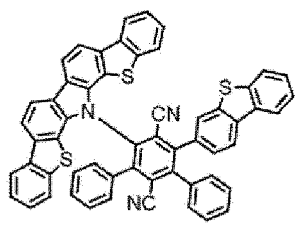
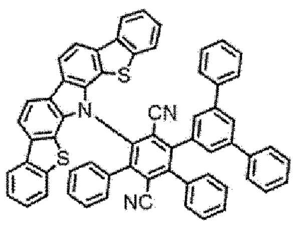

[Formula 91]

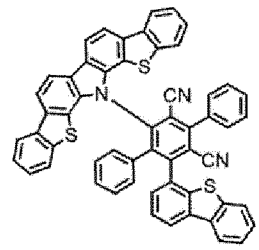
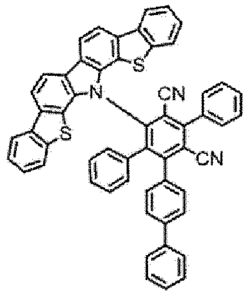
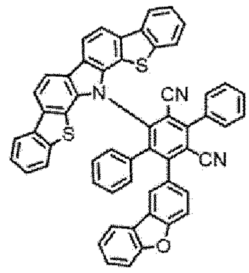
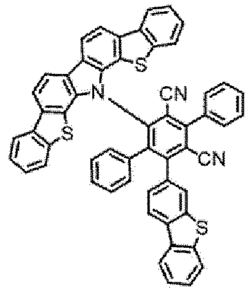
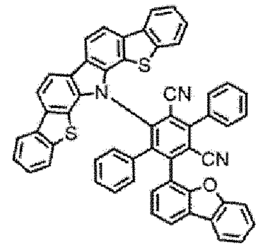
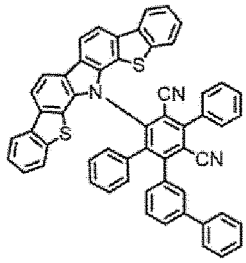
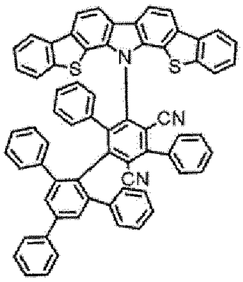
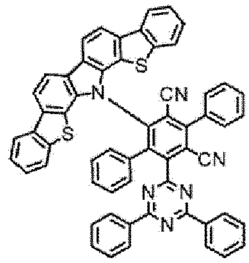
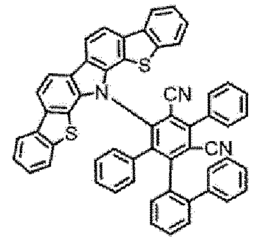
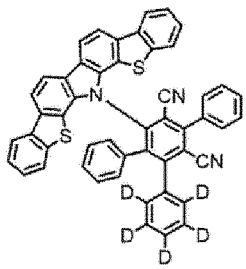
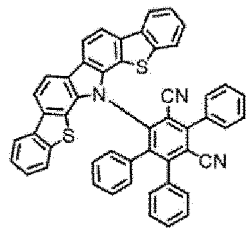
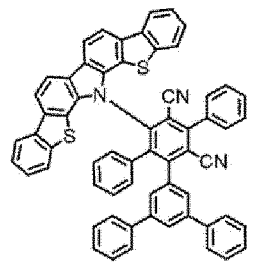

[Formula 92]

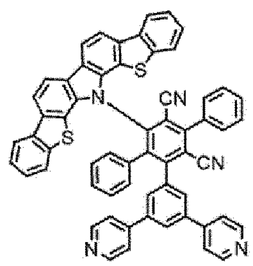
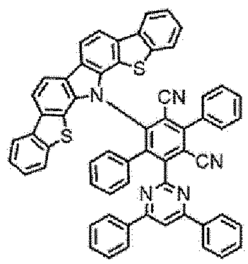
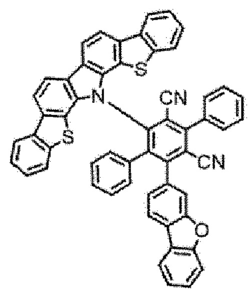
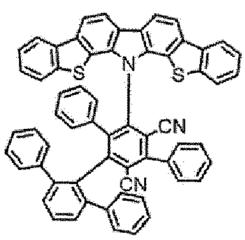
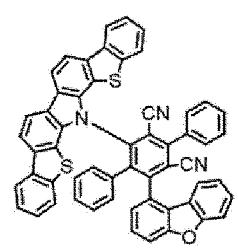
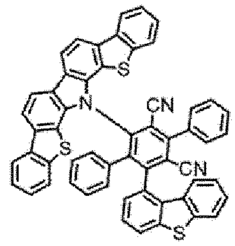
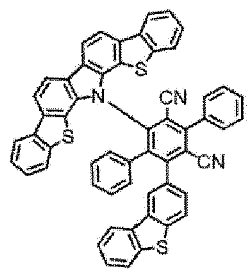

[Formula 93]

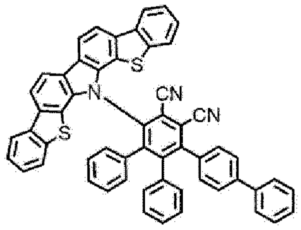
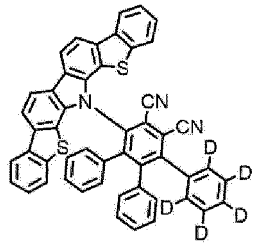
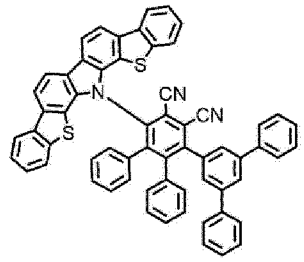
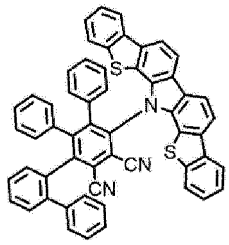
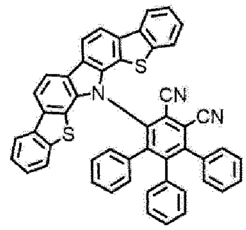
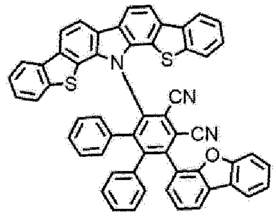
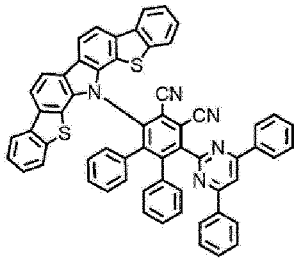
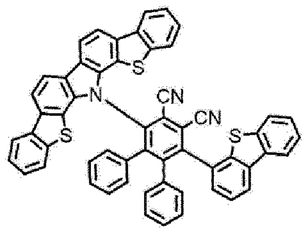
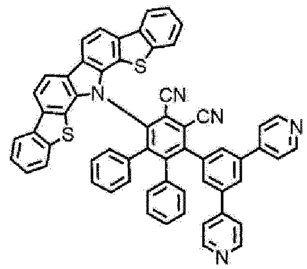
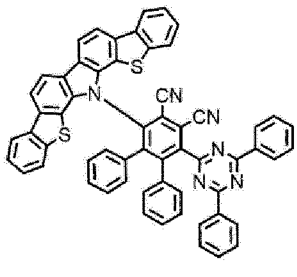
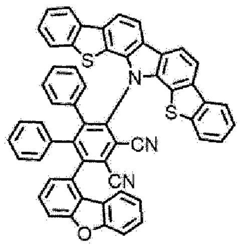
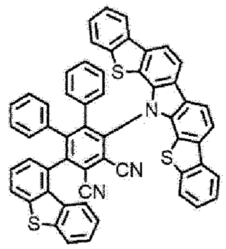

[Formula 94]

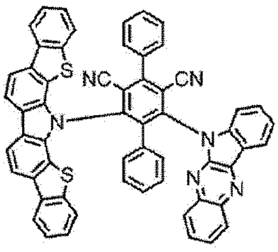
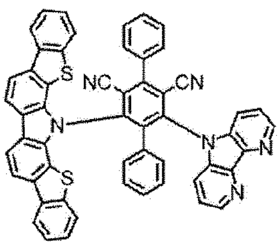
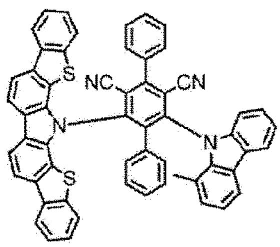
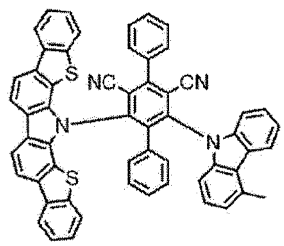

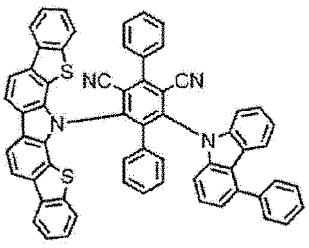
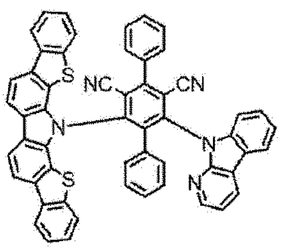
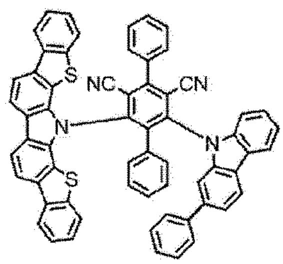

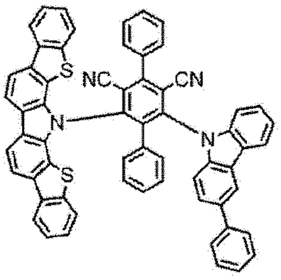
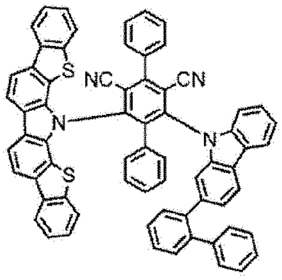
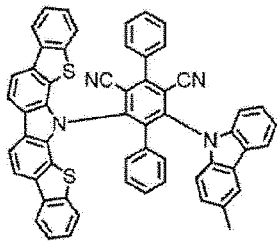
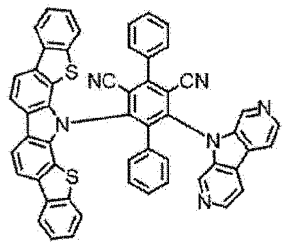

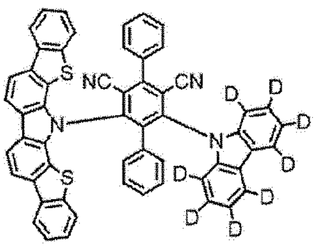
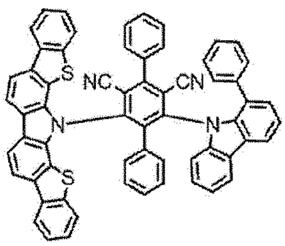
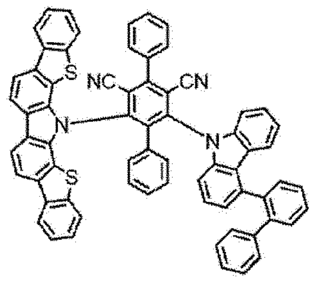

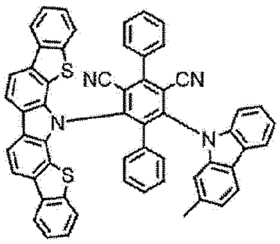
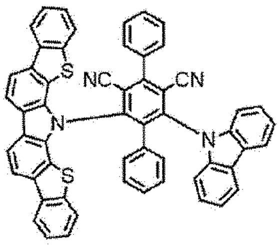
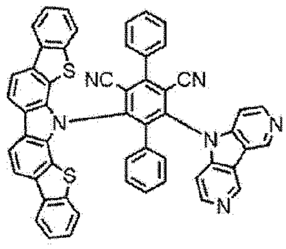
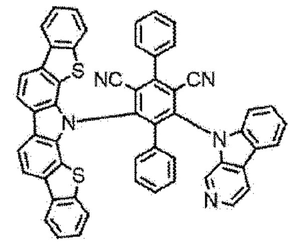

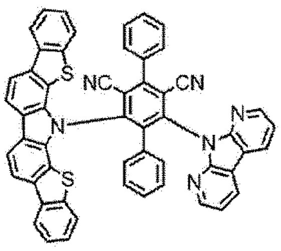
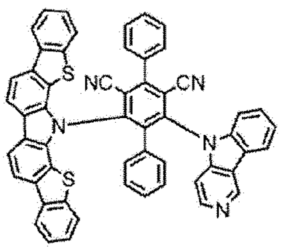
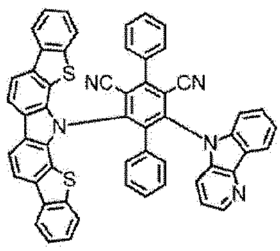

[Formula 95]

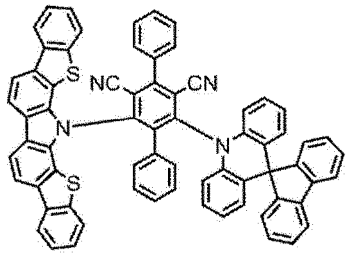
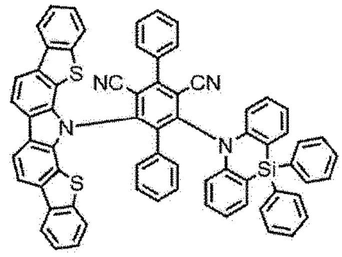
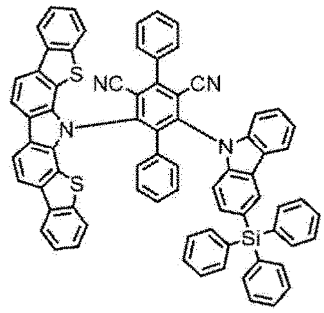
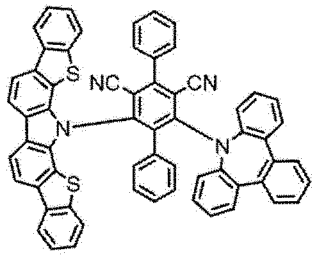
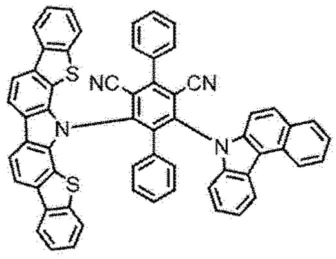
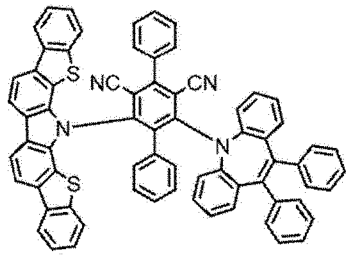
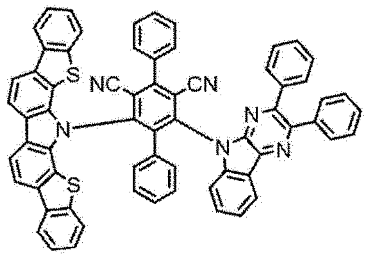
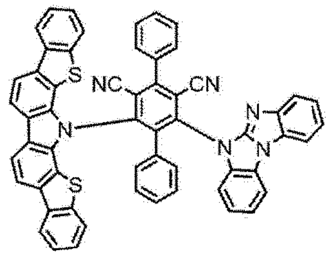
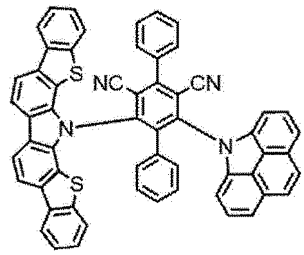
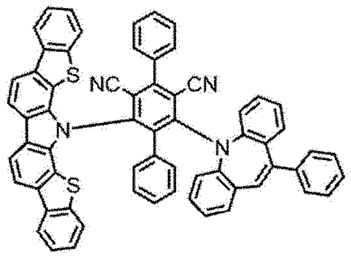
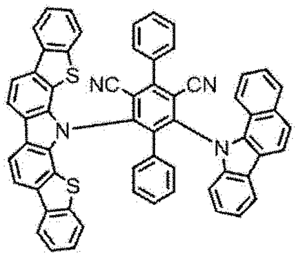
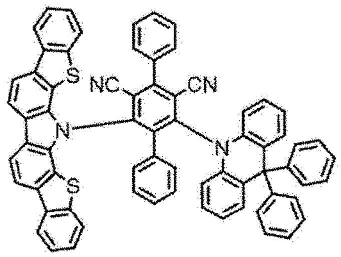
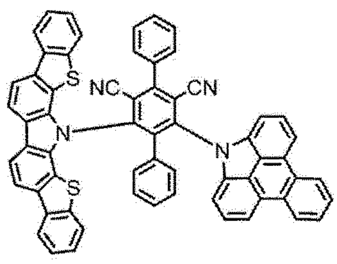
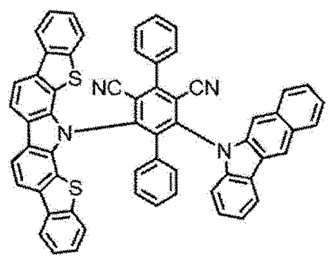
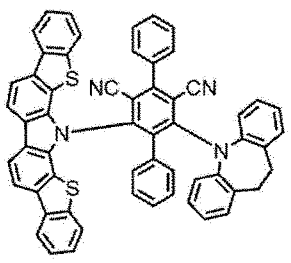
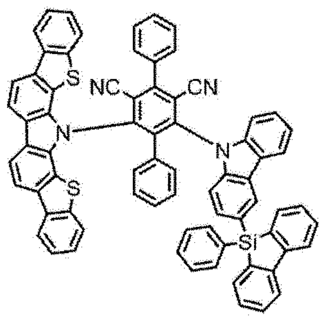

[Formula 96]

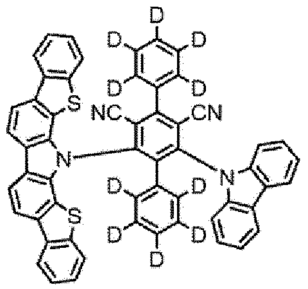
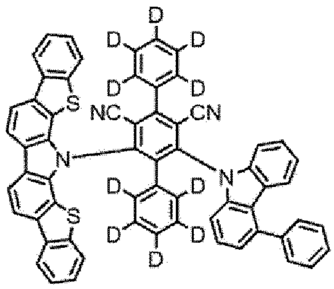
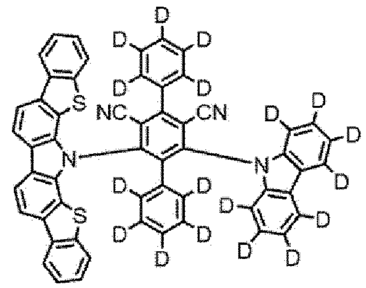
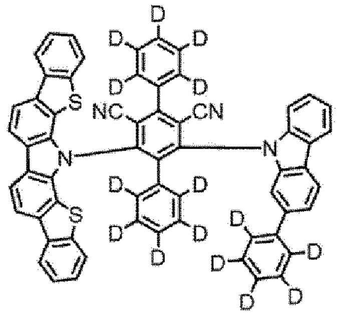
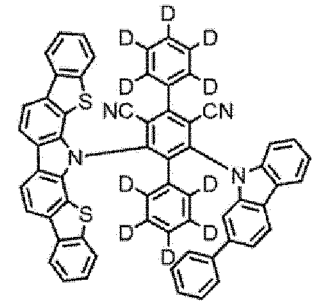
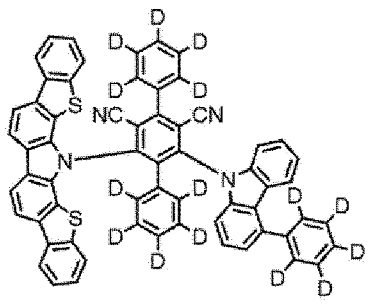
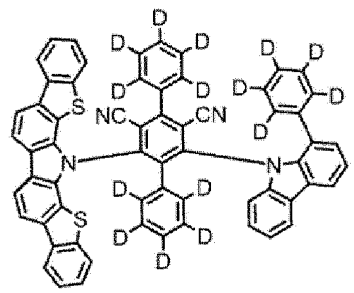
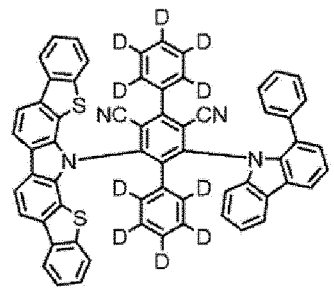

[Formula 97]

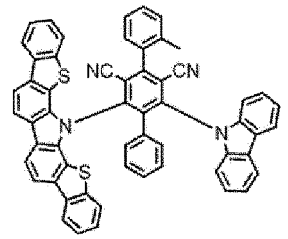

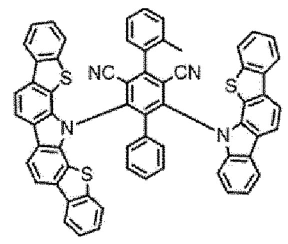

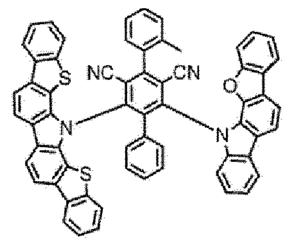

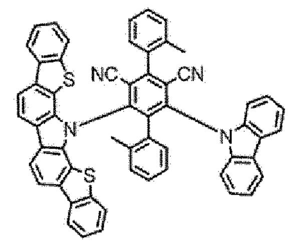

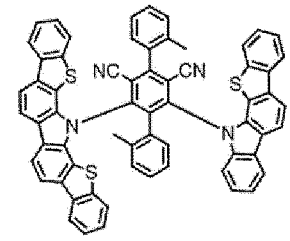

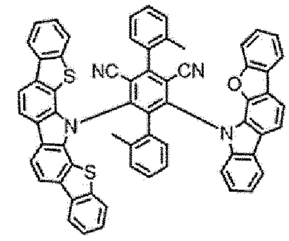

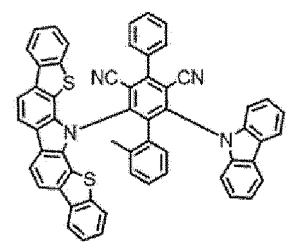

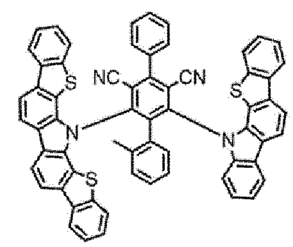

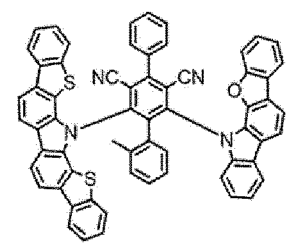

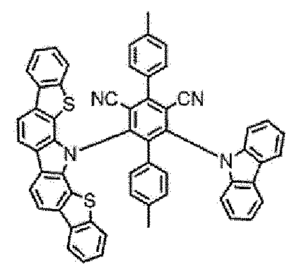

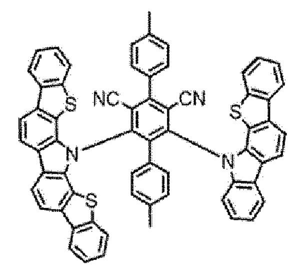

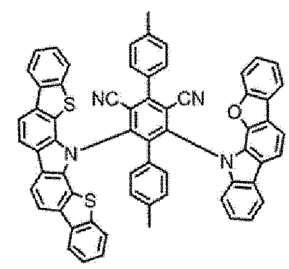

[Formula 98]

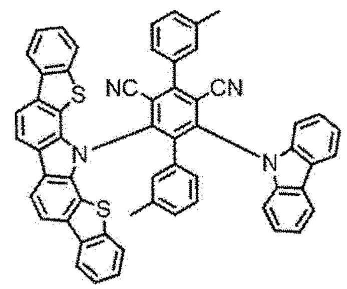
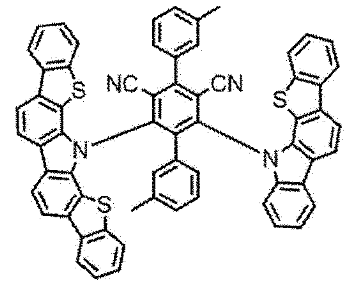
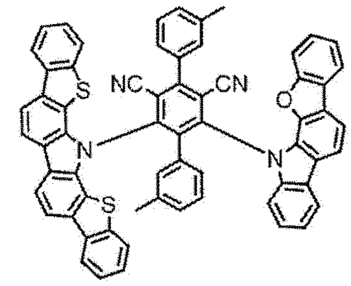
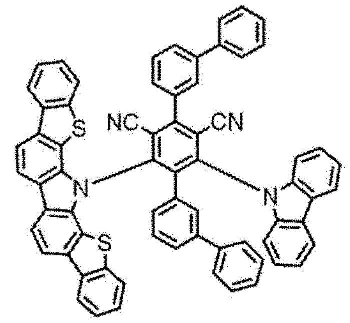
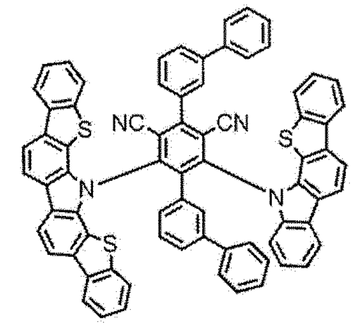
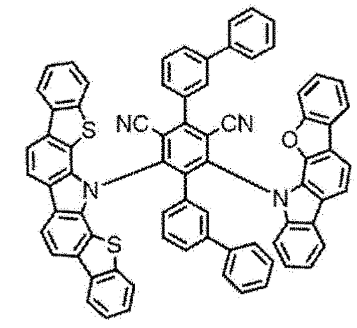
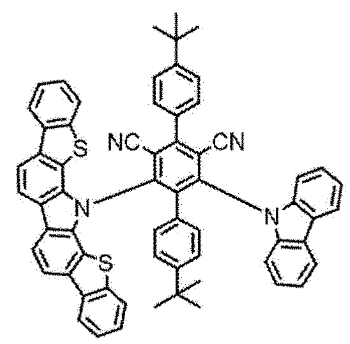
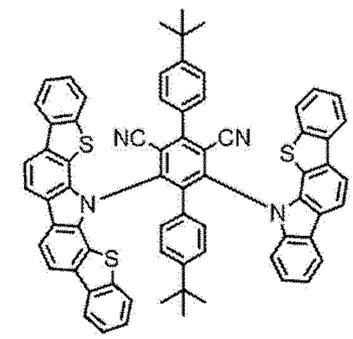
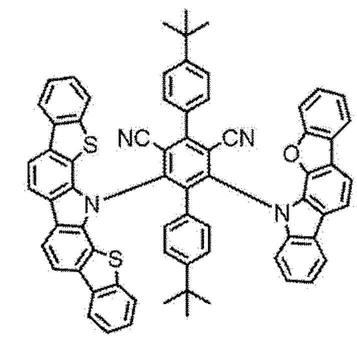
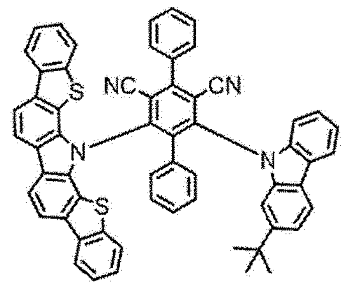
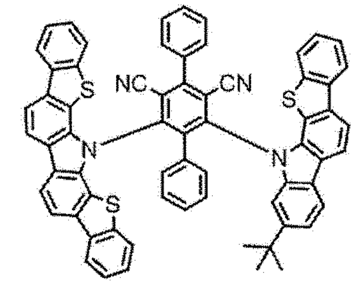
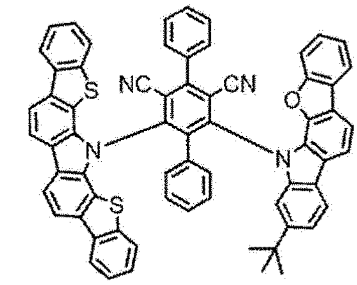
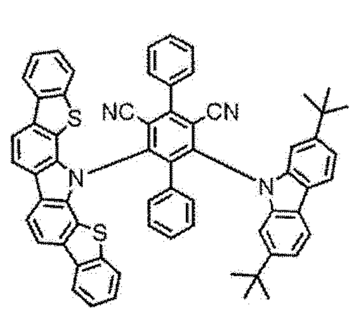

[Formula 99]

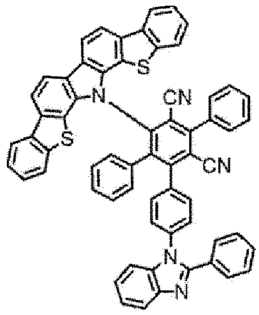 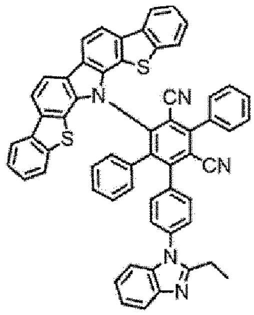 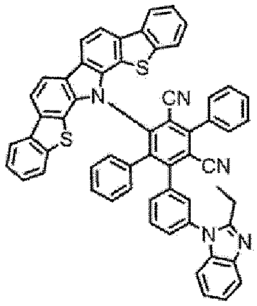 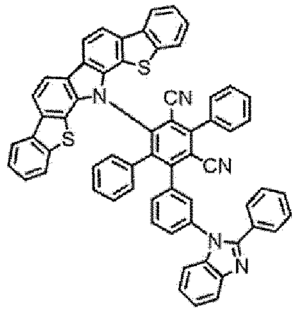

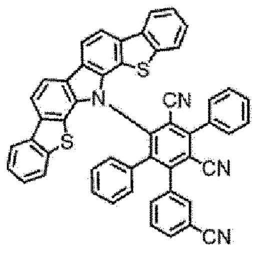 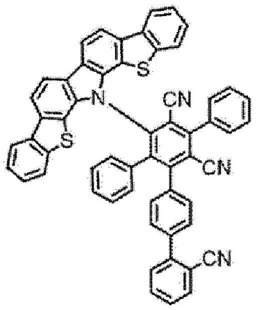 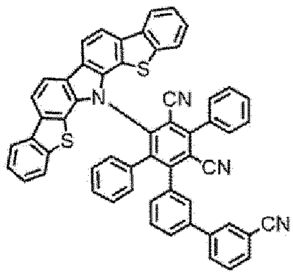 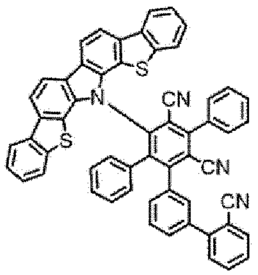

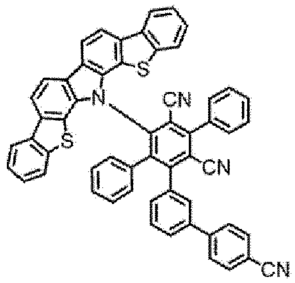 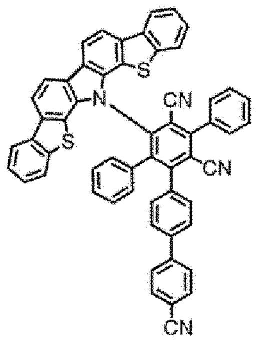 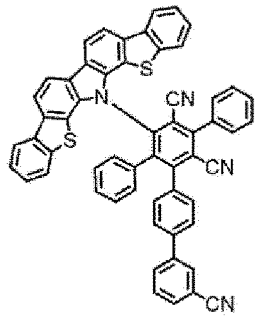

[Formula 100]

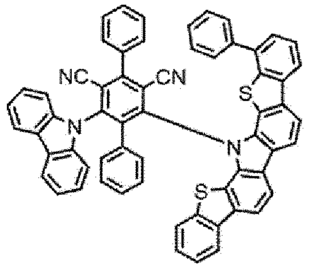
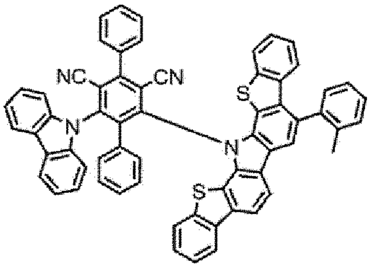
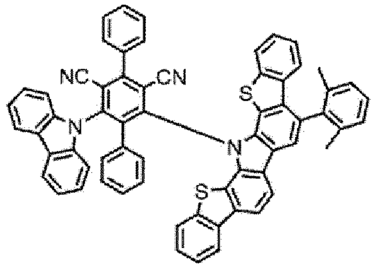

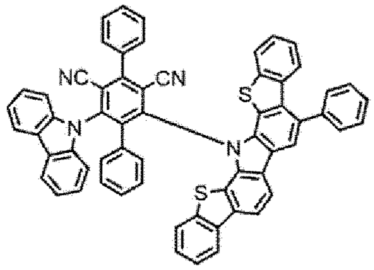
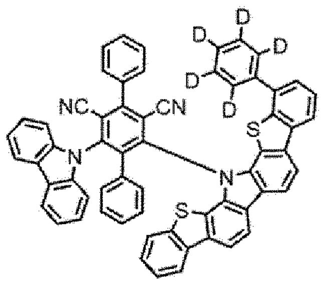
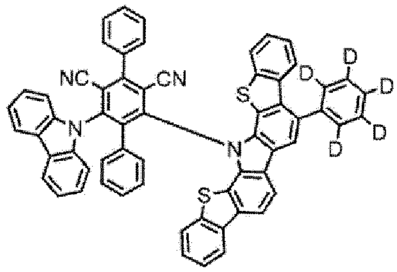

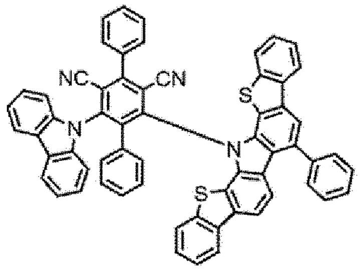

[Formula 101]

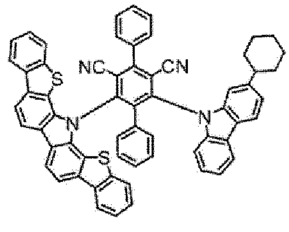

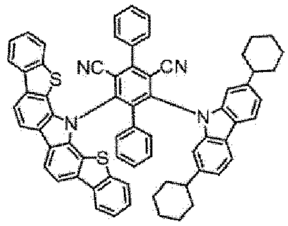

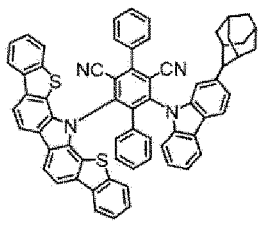

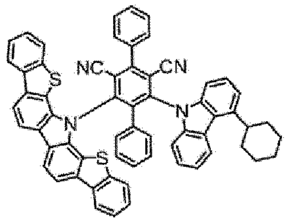

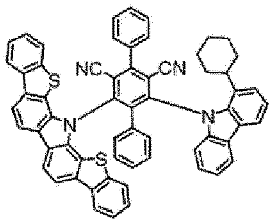

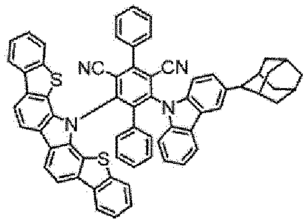

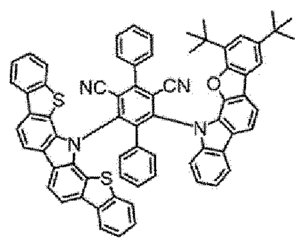

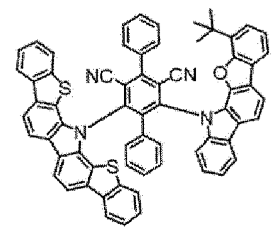

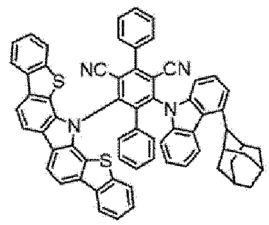

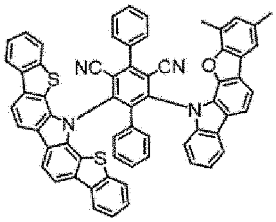

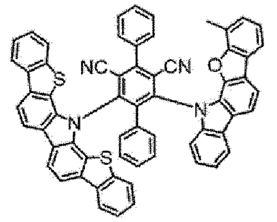

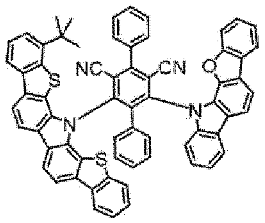

[Formula 102]

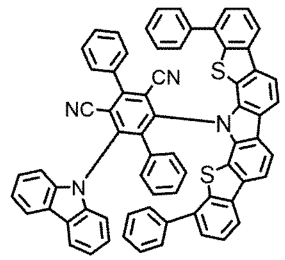
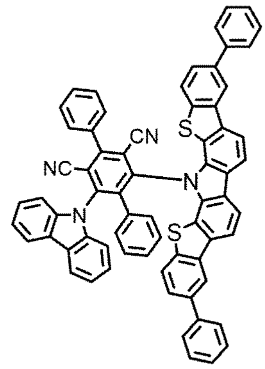
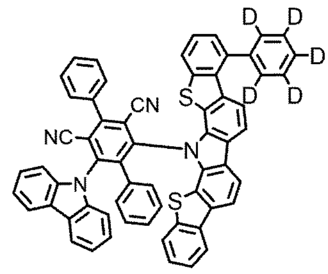
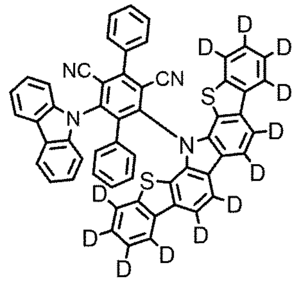
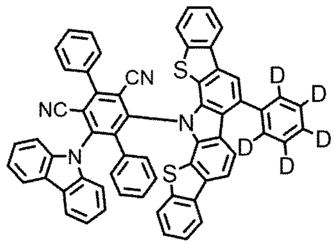
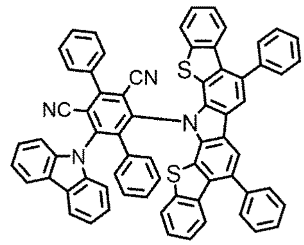
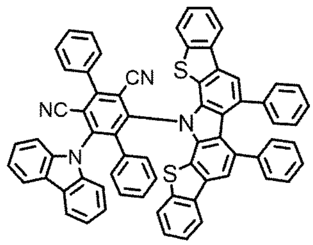
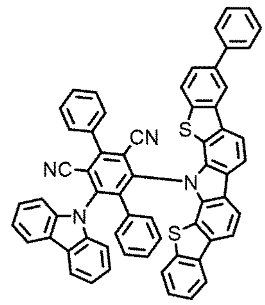
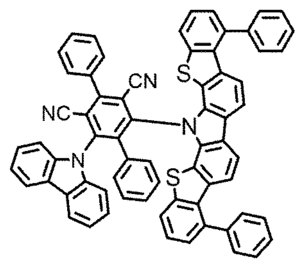
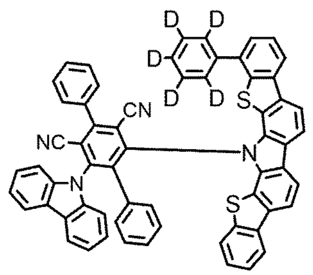
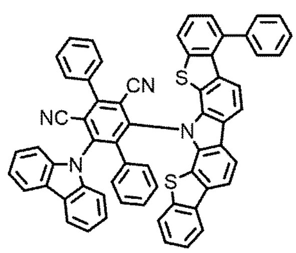

[Formula 103]

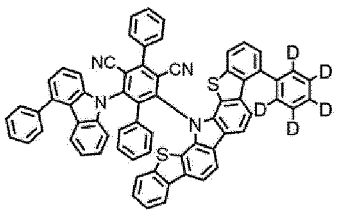
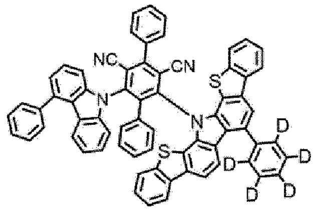
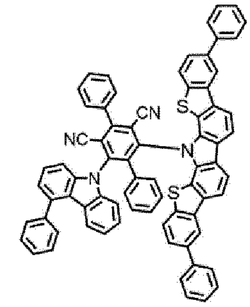
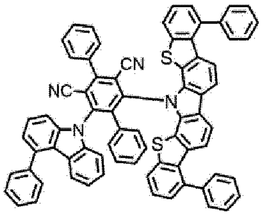
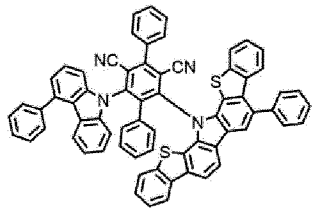
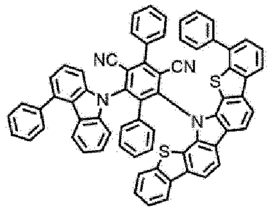
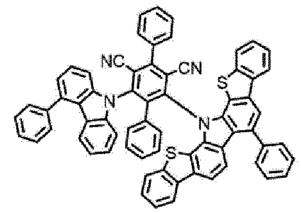
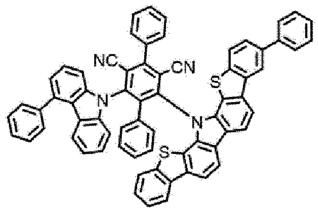
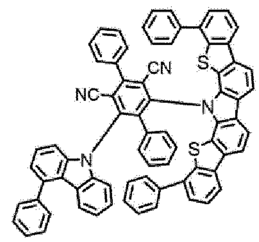
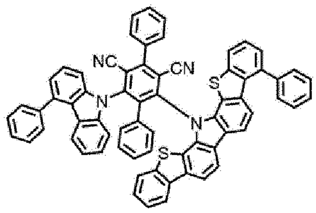
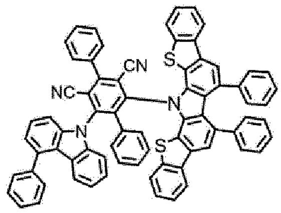
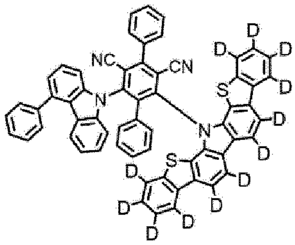
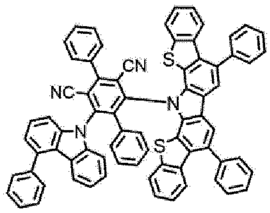
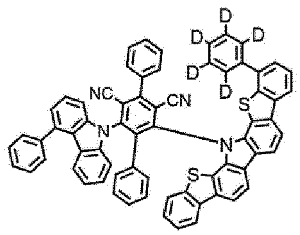

[Formula 104]

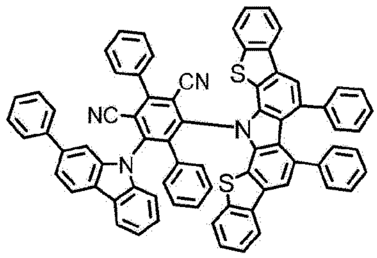

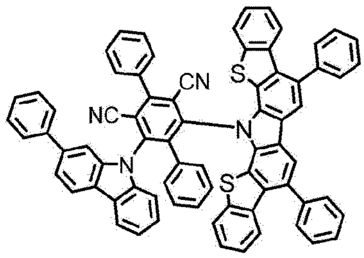

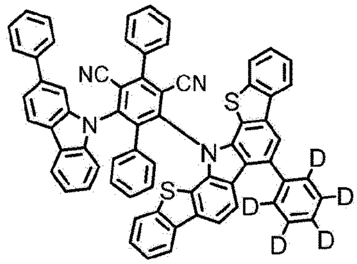

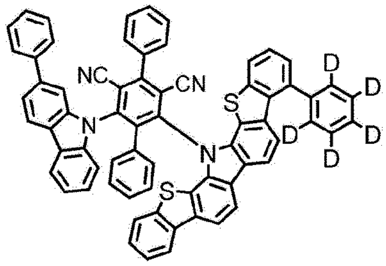

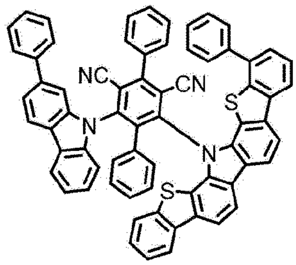

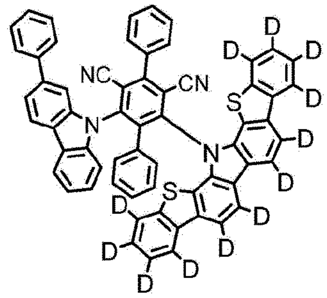

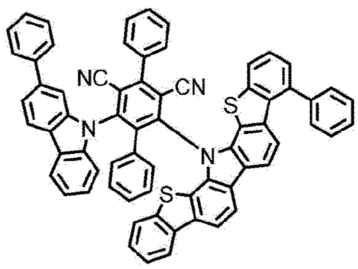

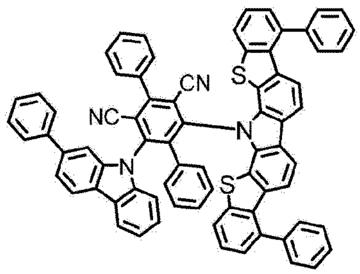

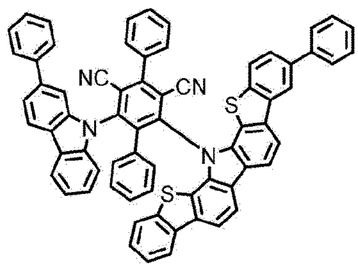

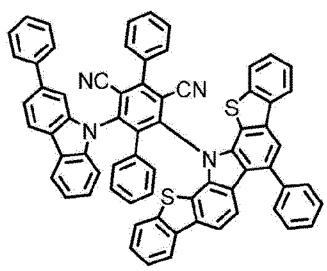

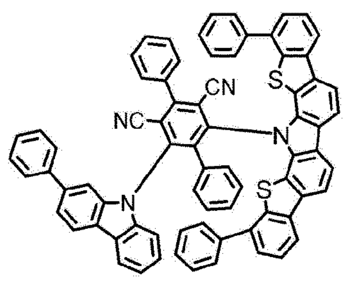

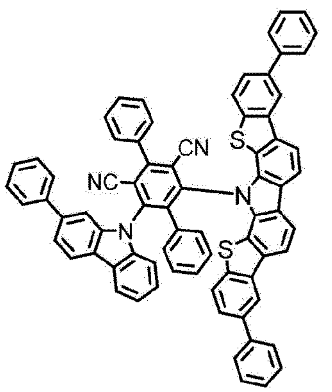

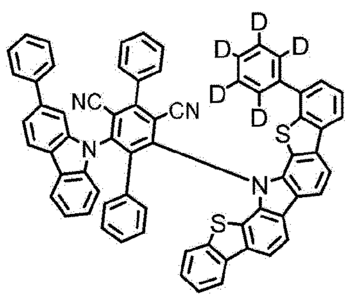

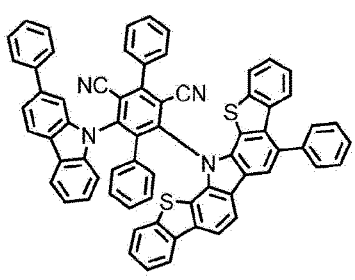

[Formula 105]

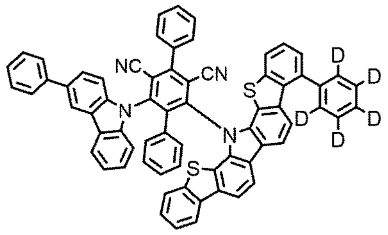
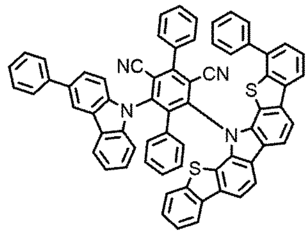
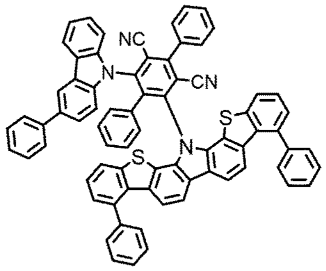
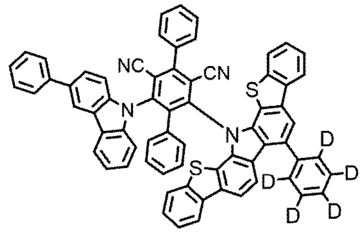
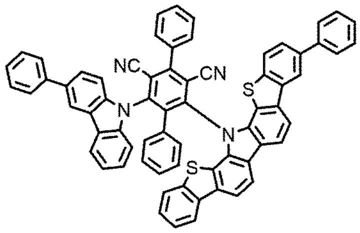
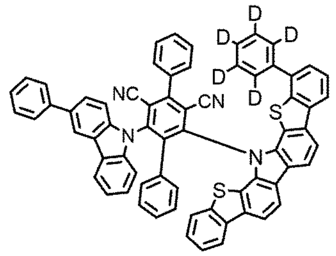
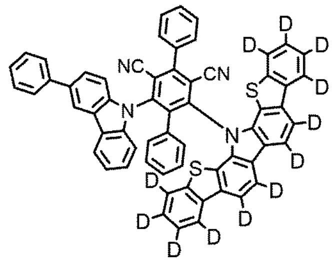
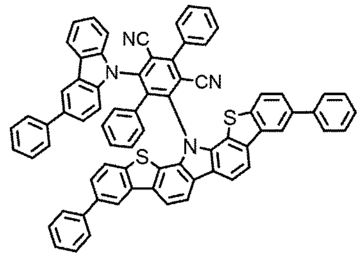
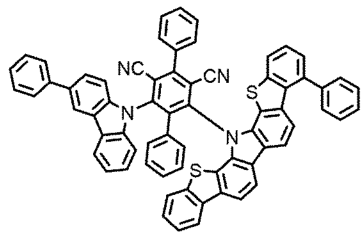
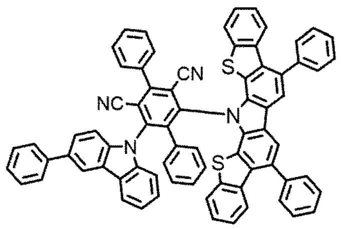

[Formula 106]

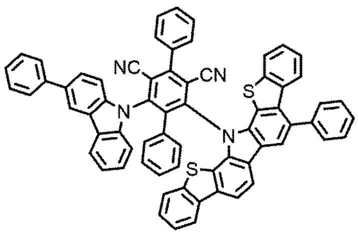
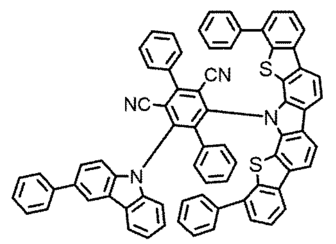
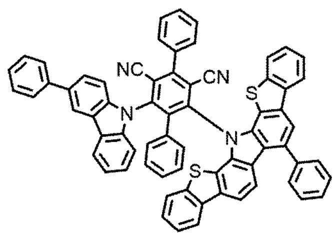
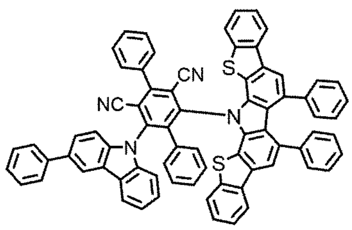

[Formula 107]

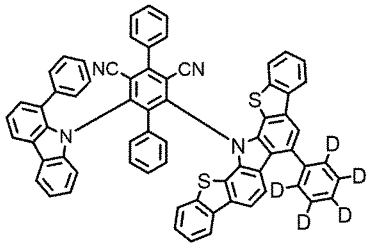
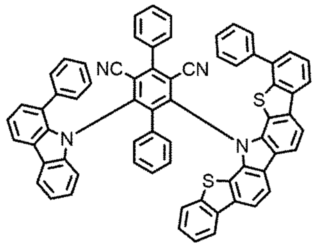
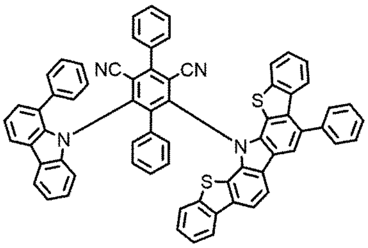
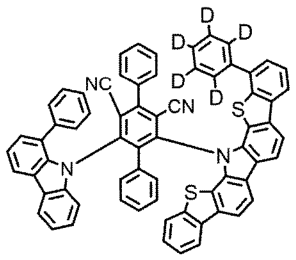
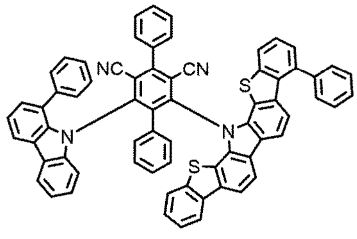
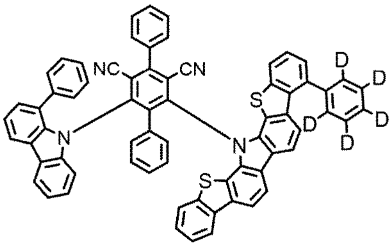
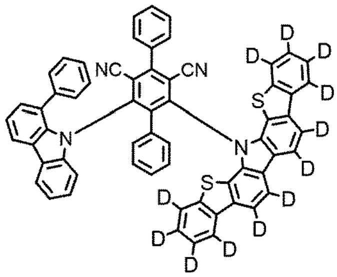
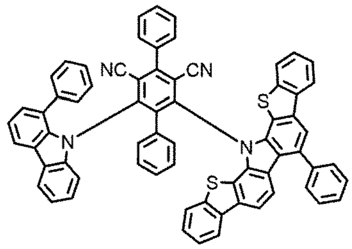

[Formula 108]

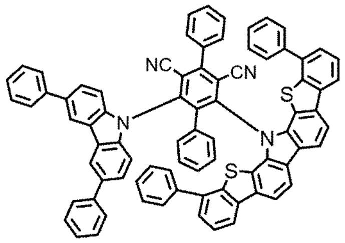
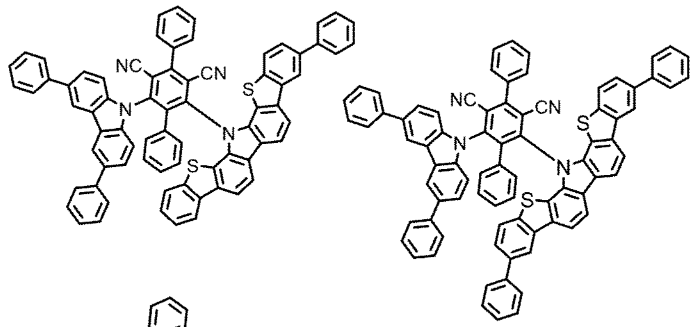
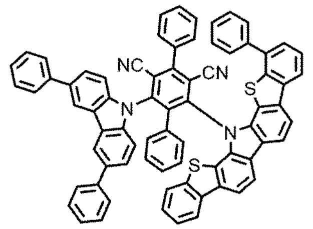
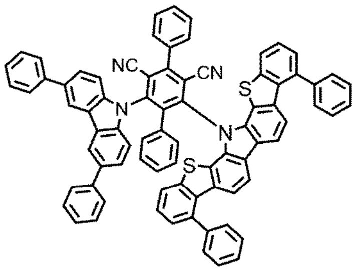
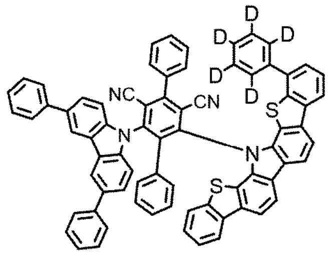
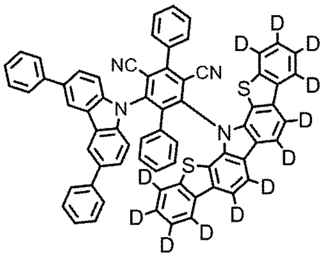
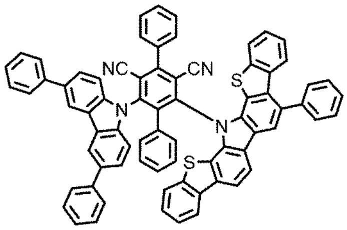
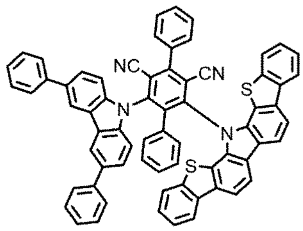
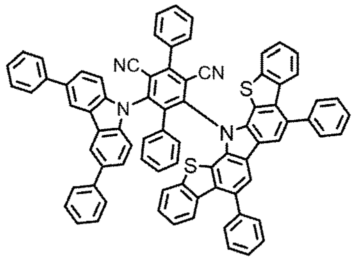
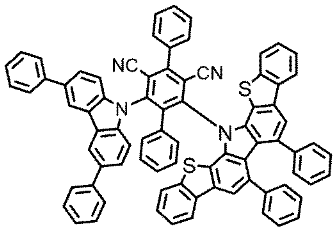

[Formula 109]

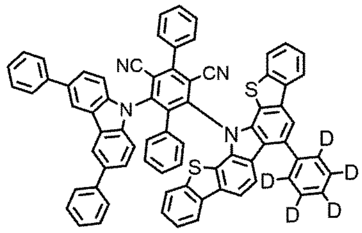

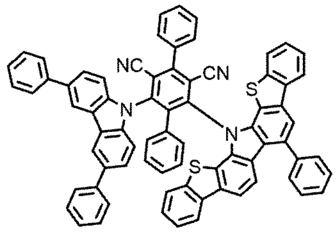

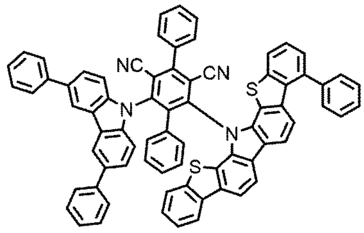

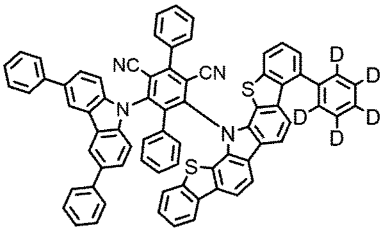

[Formula 110]

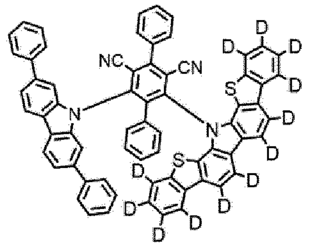
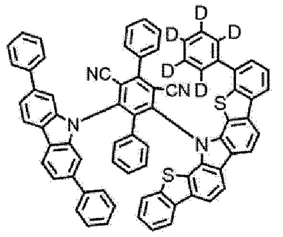
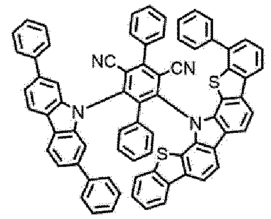
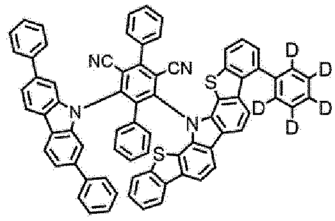
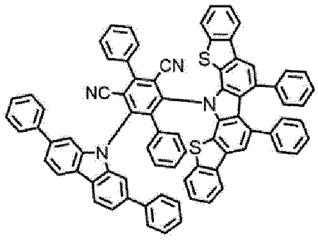
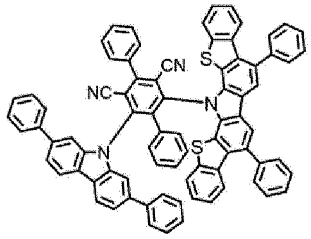
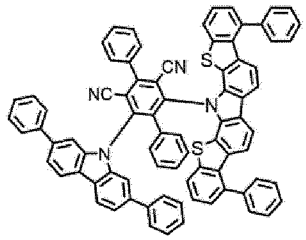
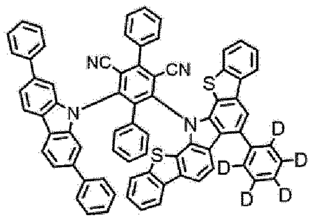
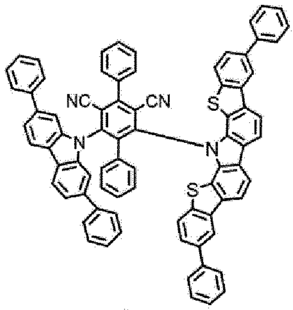
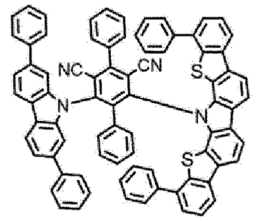
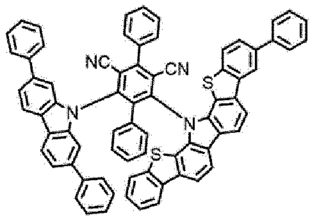
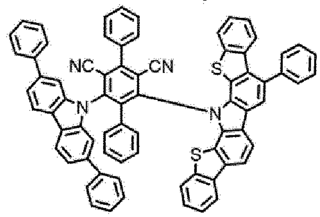
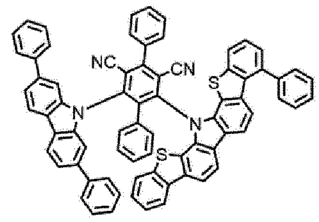
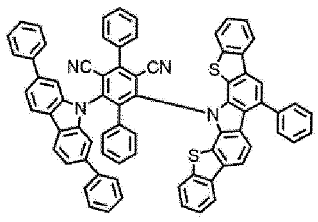
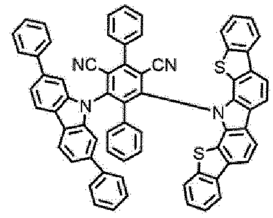

[Formula 111]

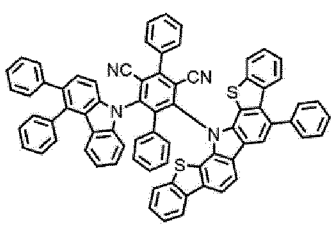
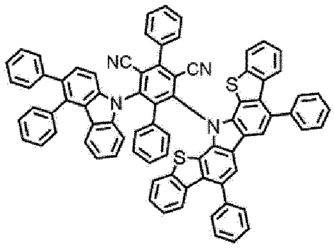
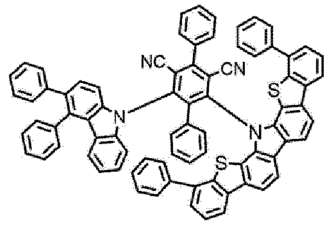
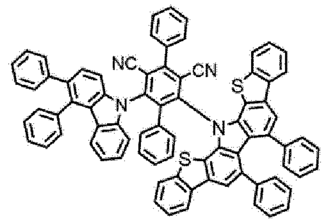
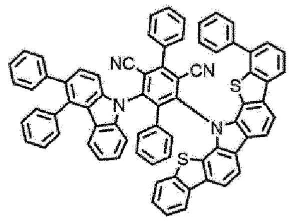
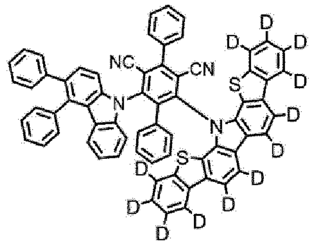
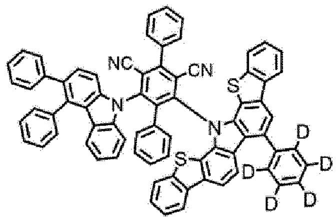
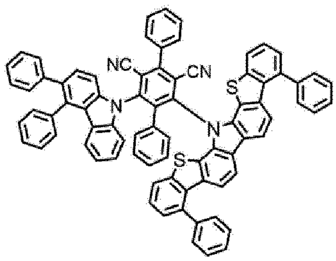
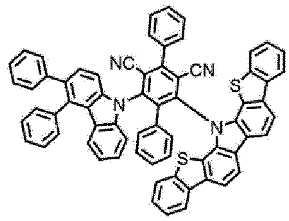
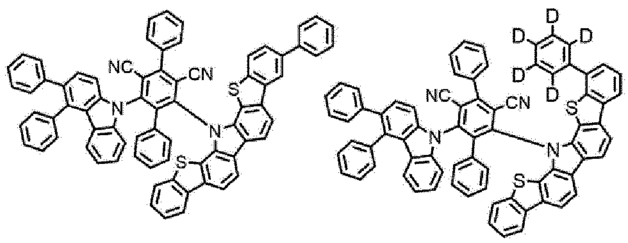
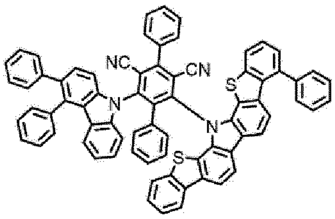
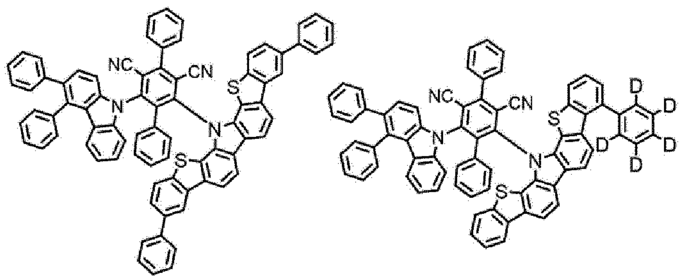
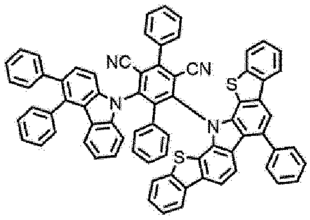

[Formula 112]

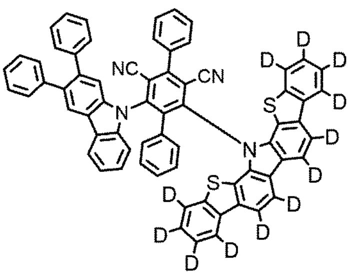
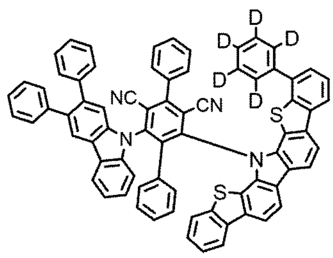
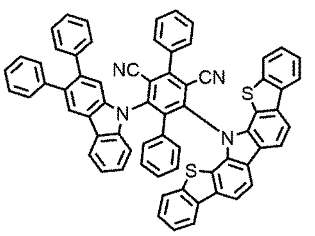

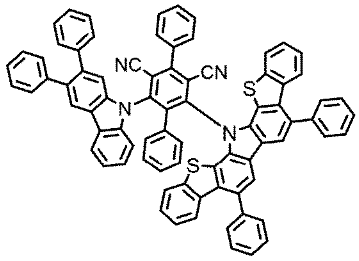
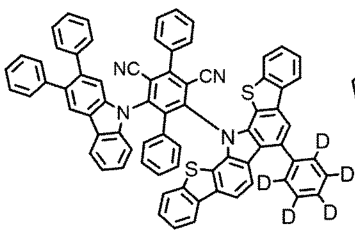
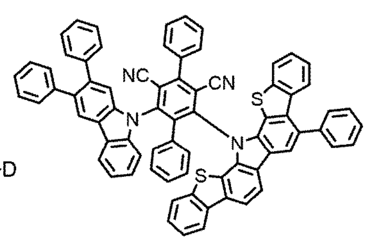

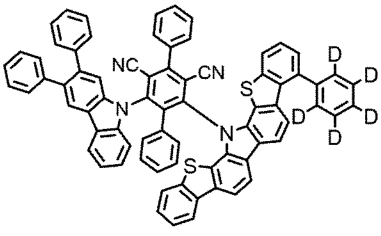
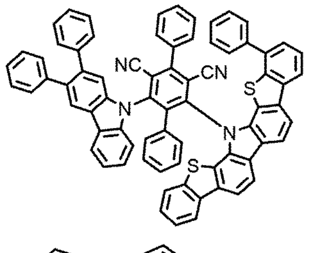
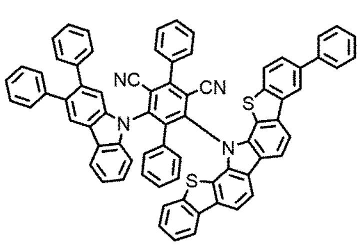

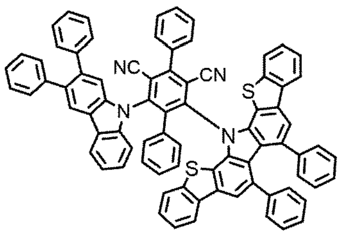
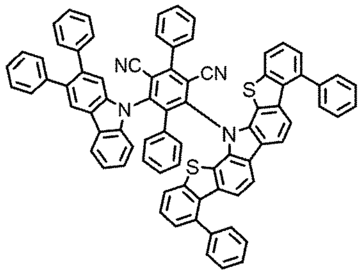

[Formula 113]

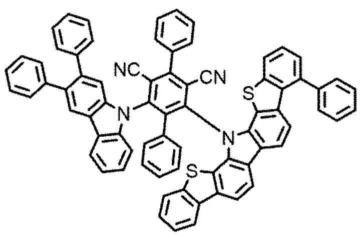
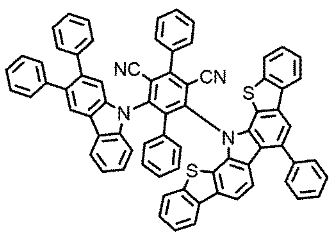

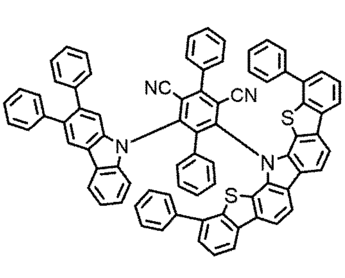
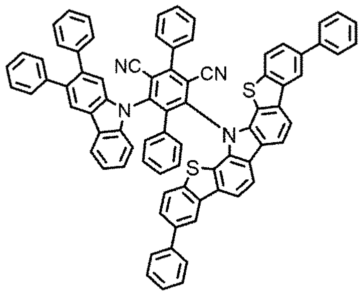

[Formula 114]

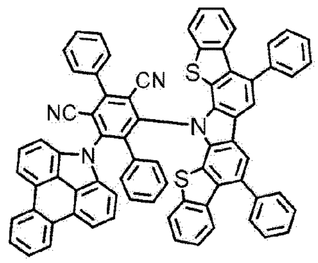
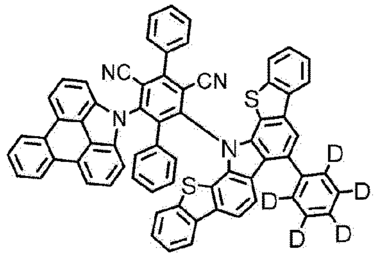
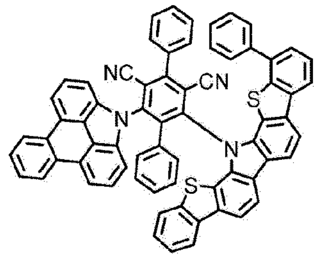
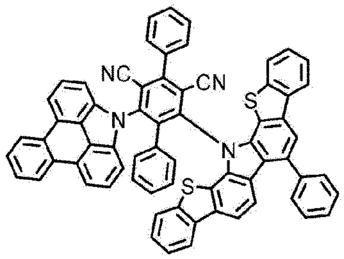
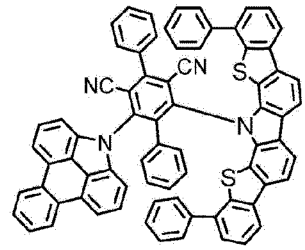
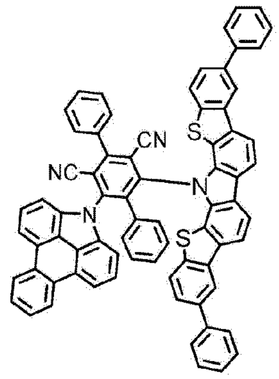
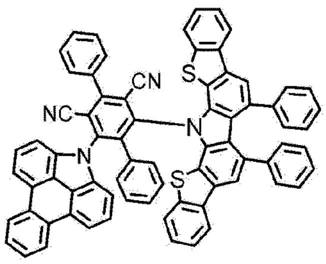
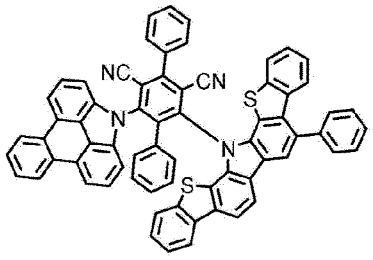
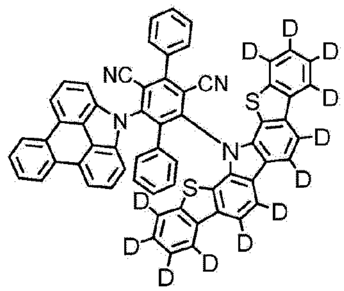
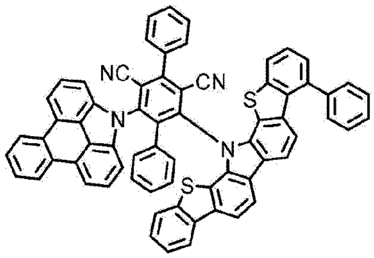
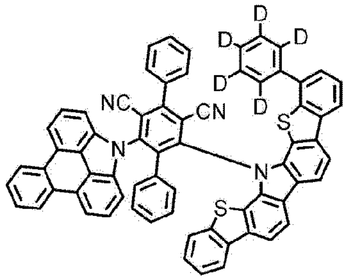
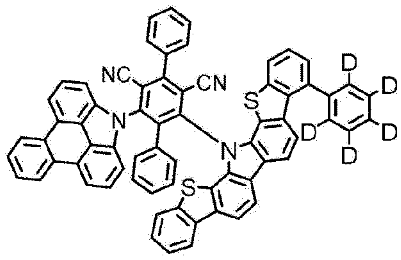
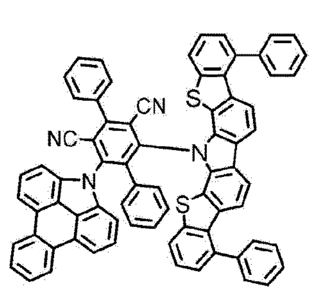
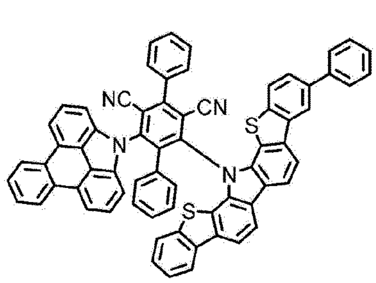

[Formula 115]

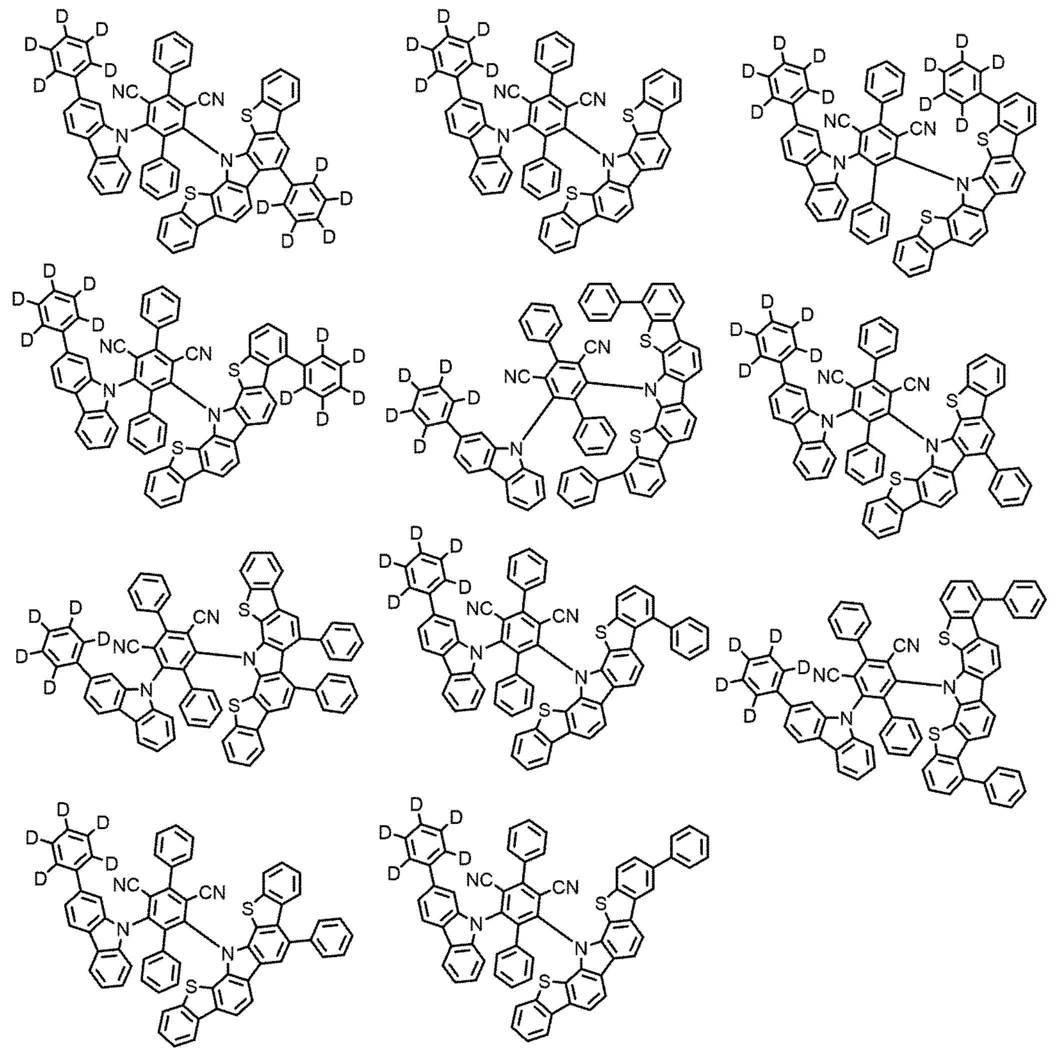

[Formula 116]

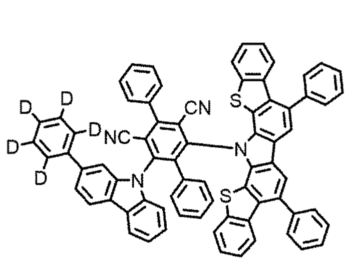
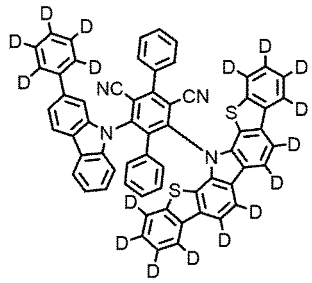
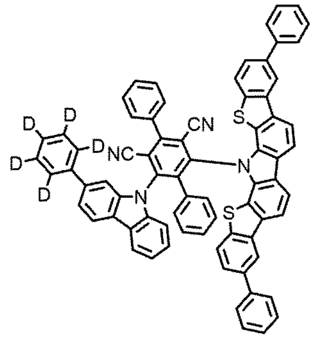
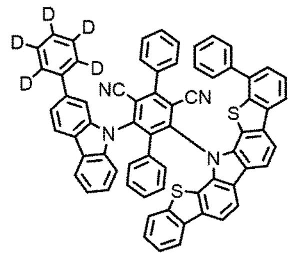

[Formula 117]

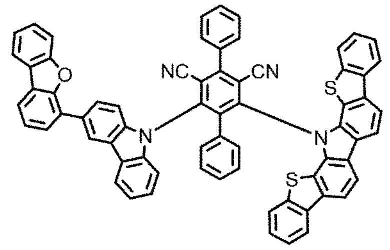
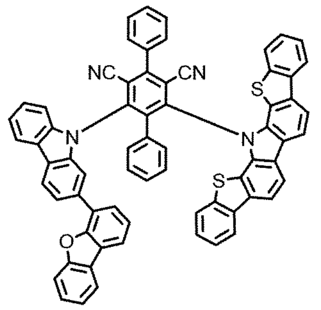
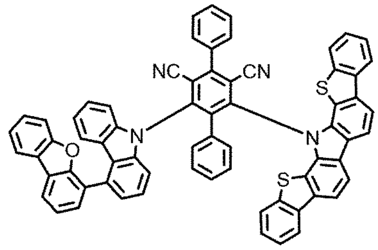
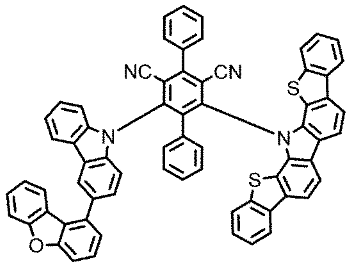
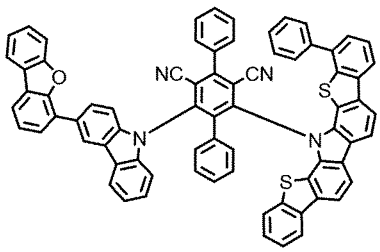
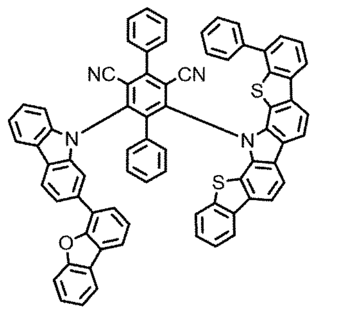

[Formula 118]

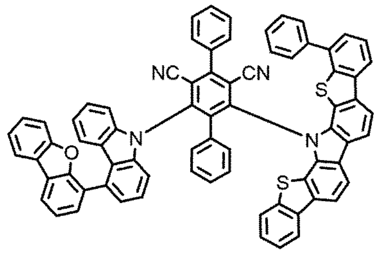

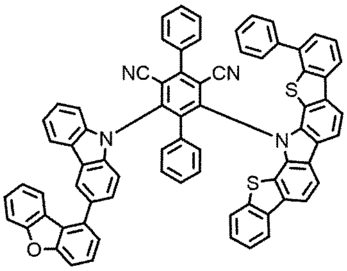

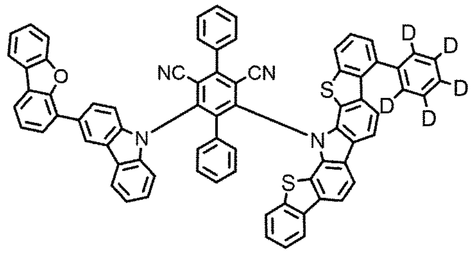

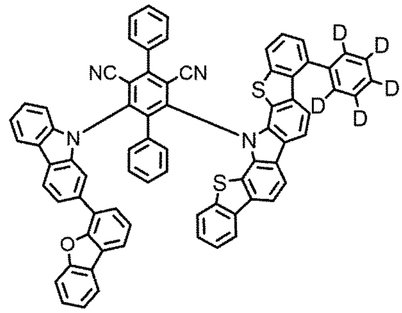

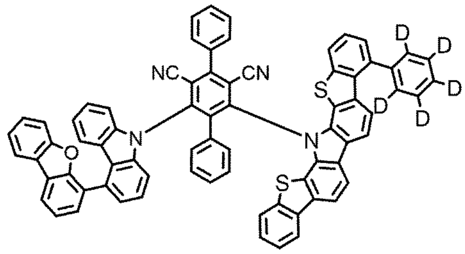

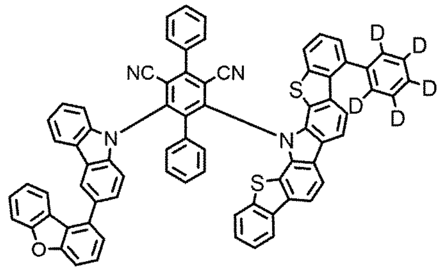

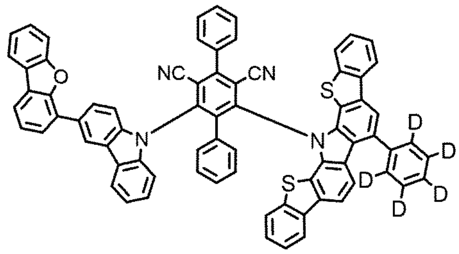

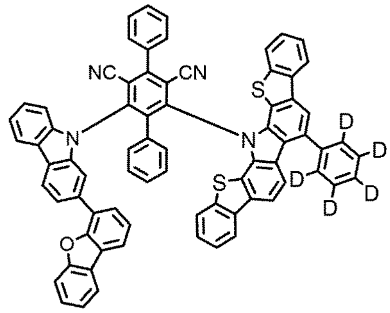

[Formula 119]

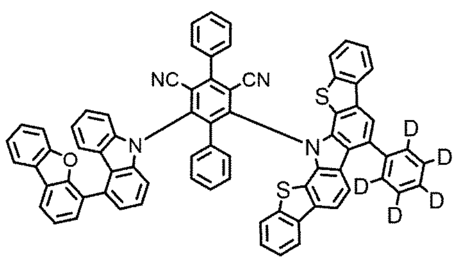
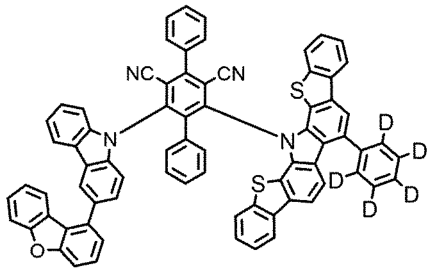
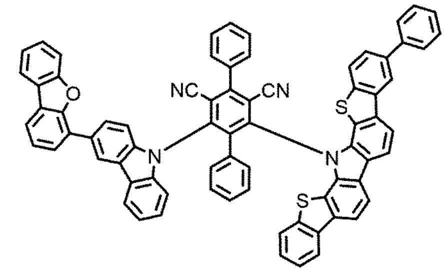
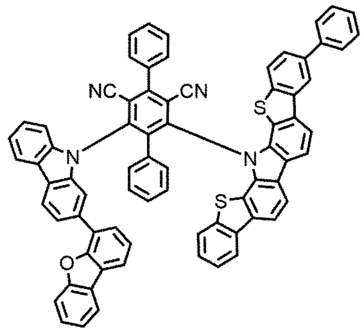
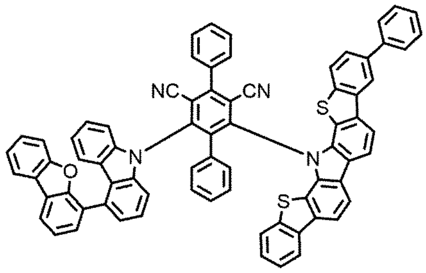
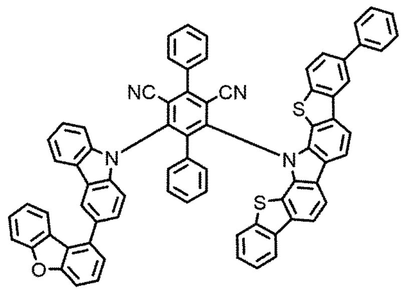
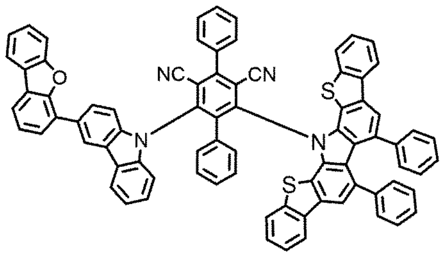
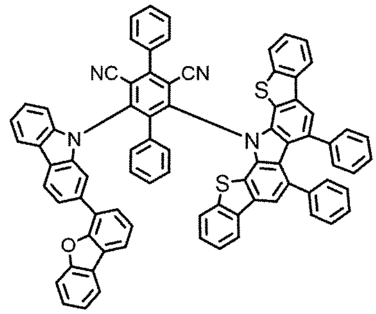

[Formula 120]

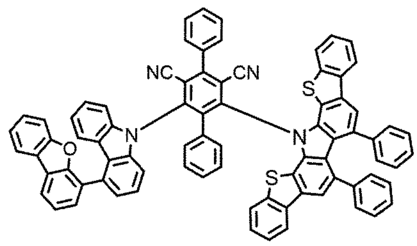
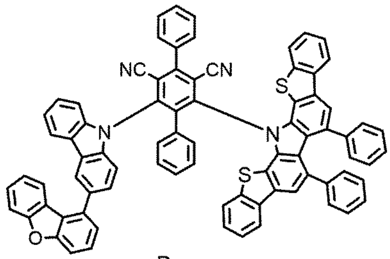

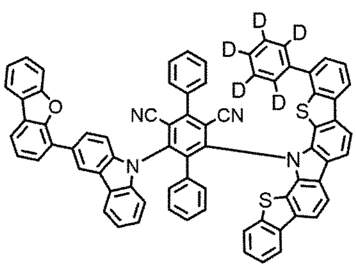
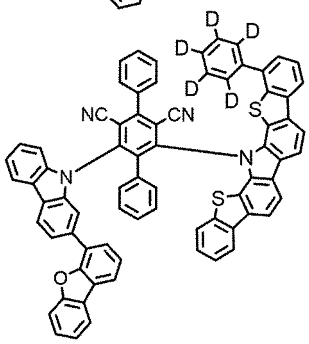
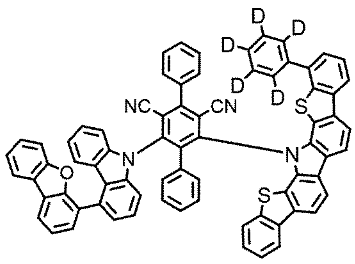

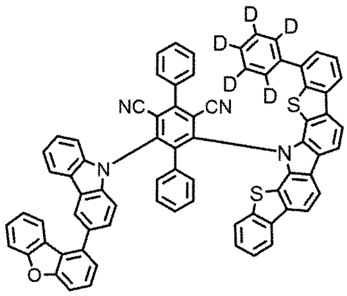
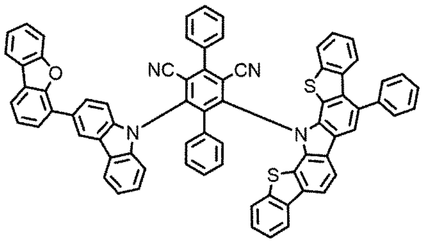

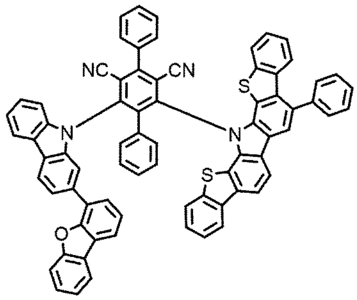
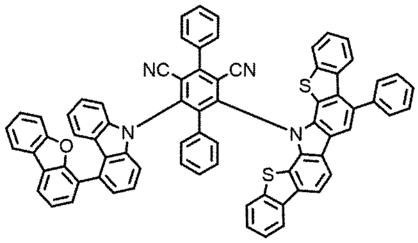

[Formula 121]

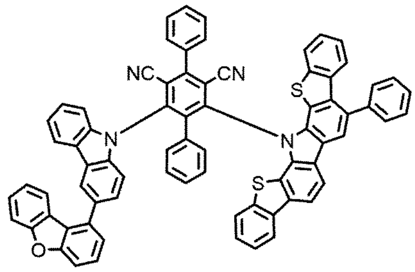
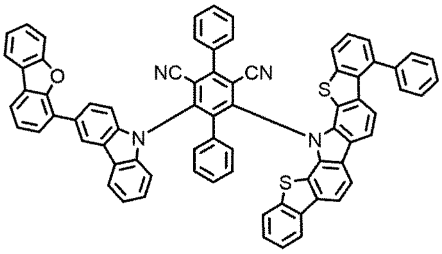
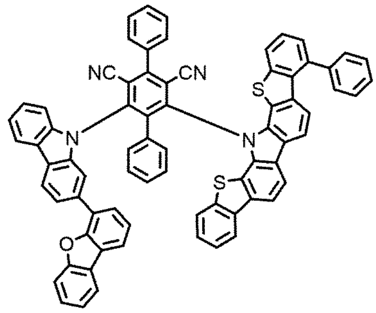
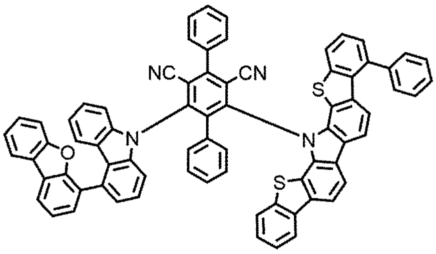
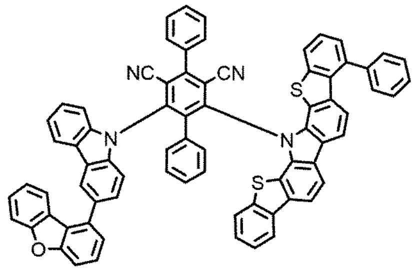
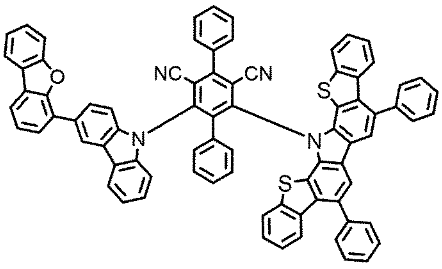
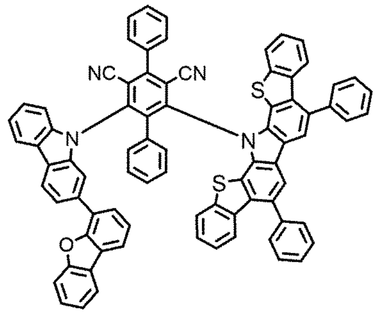
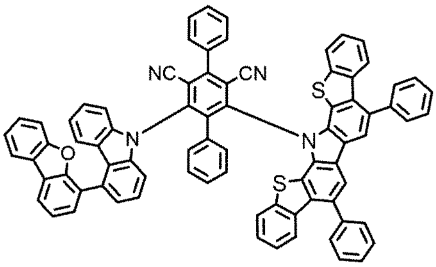

[Formula 122]

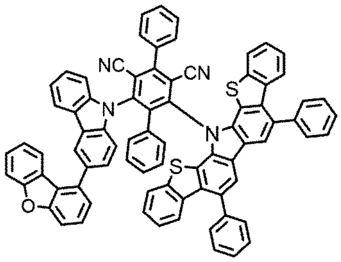
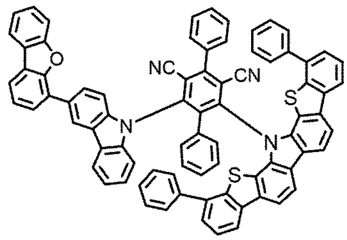
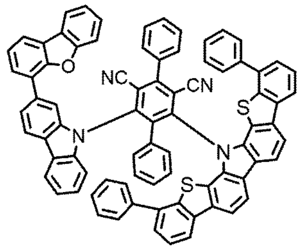
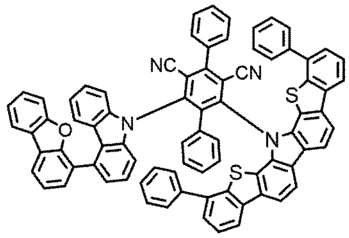
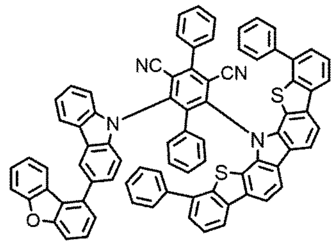
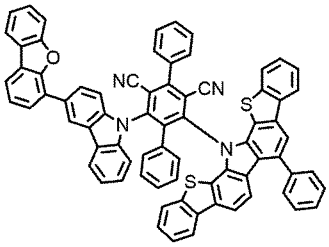
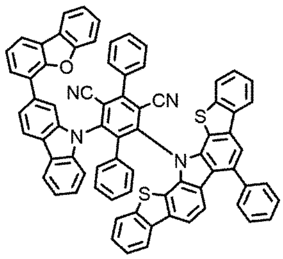
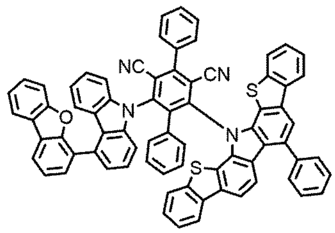
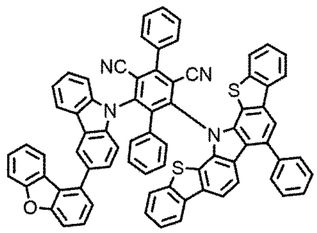

[Formula 123]

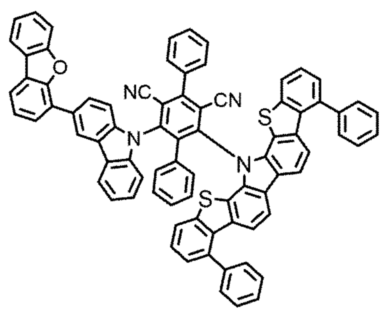

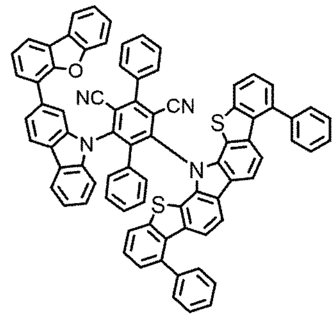

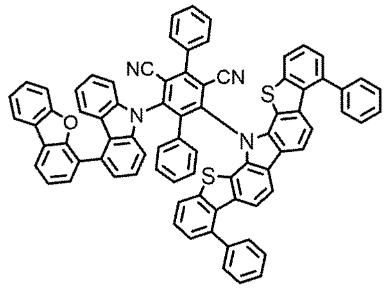

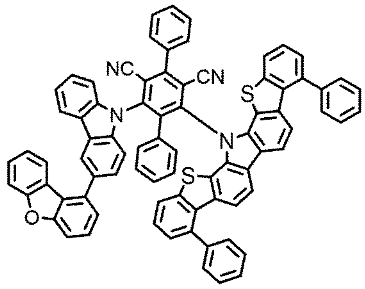

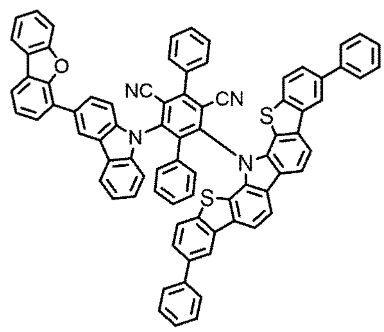

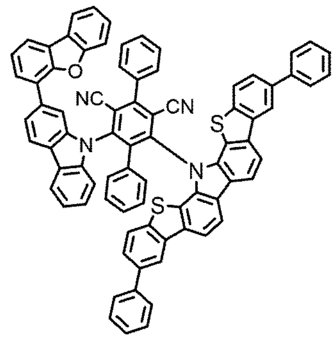

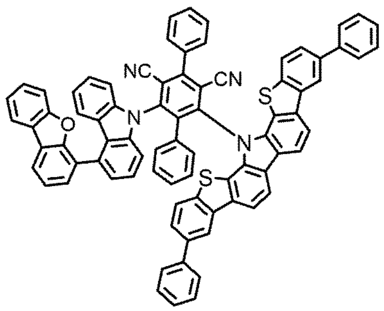

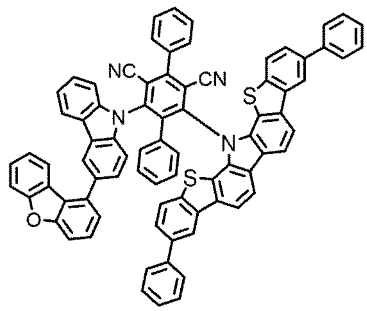

[Formula 124]

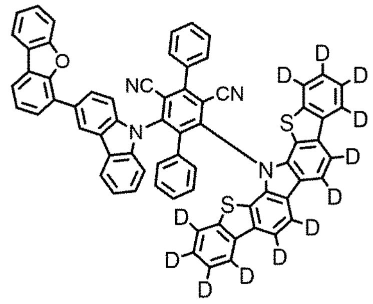

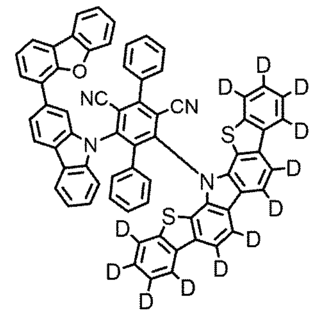

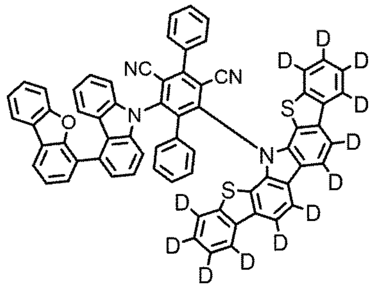

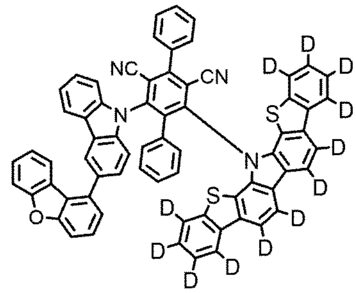

[Formula 125]

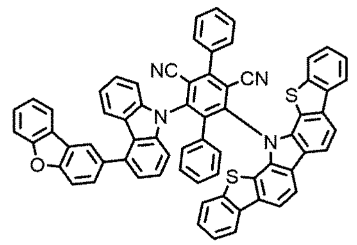

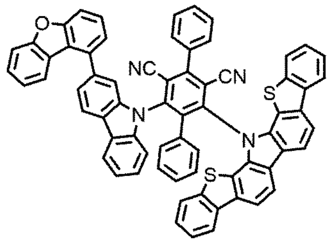

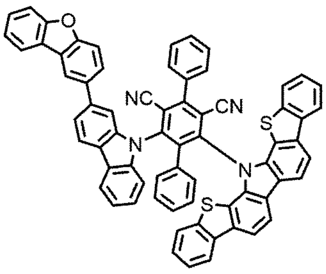

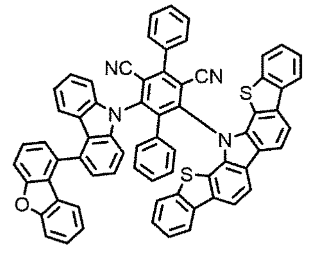

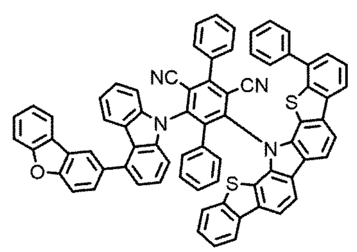

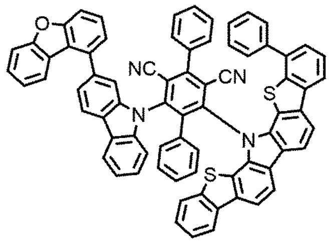

[Formula 126]

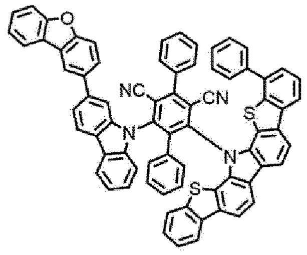
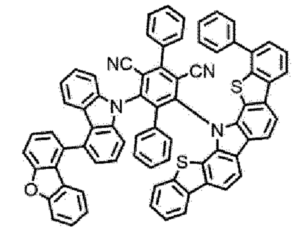
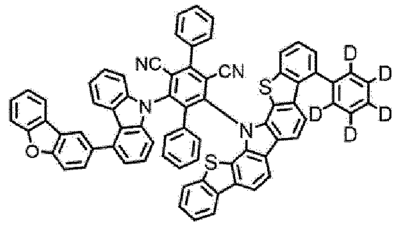
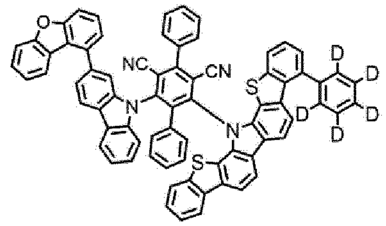
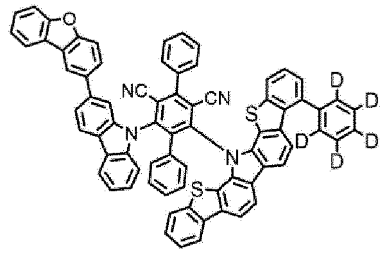
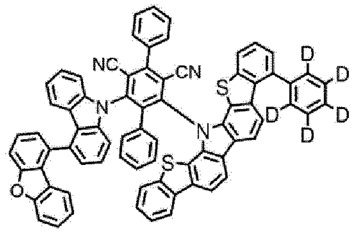
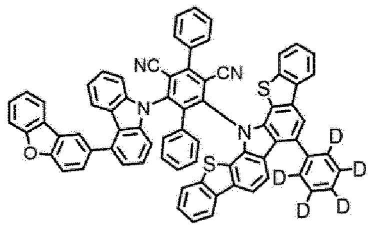
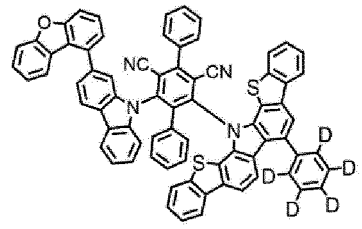
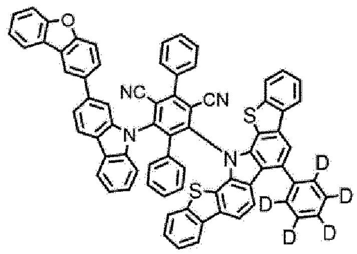
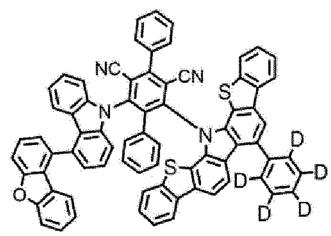

[Formula 127]

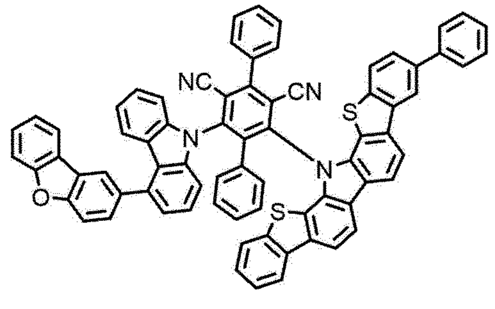
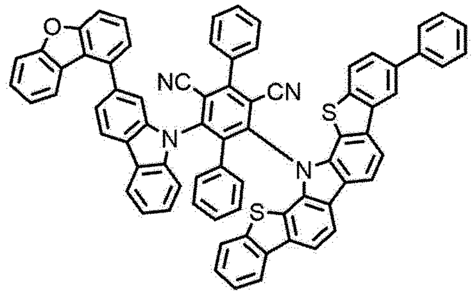
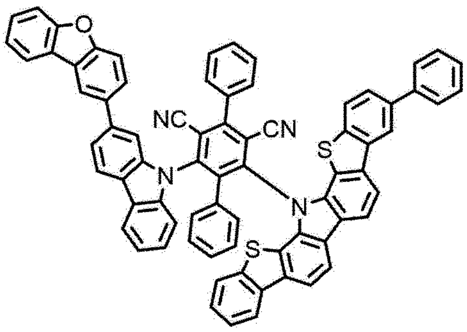
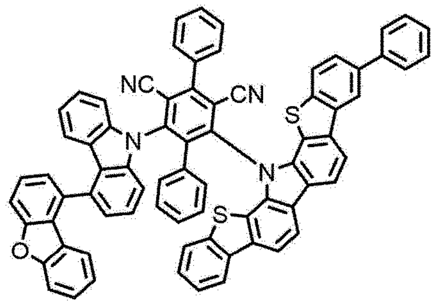
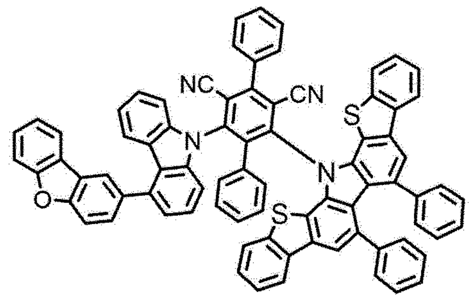
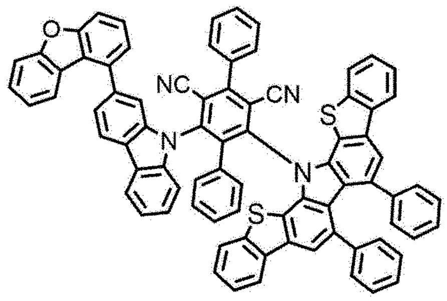
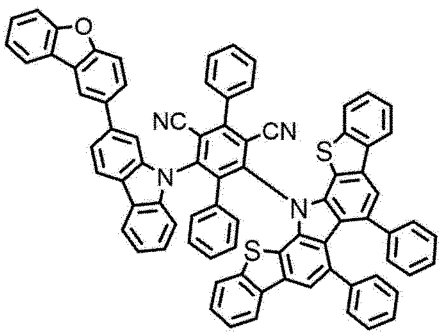
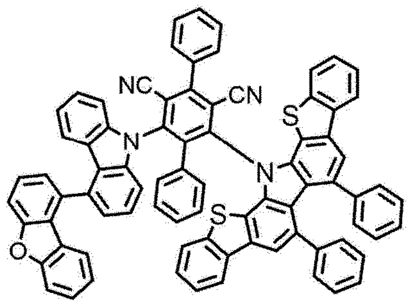
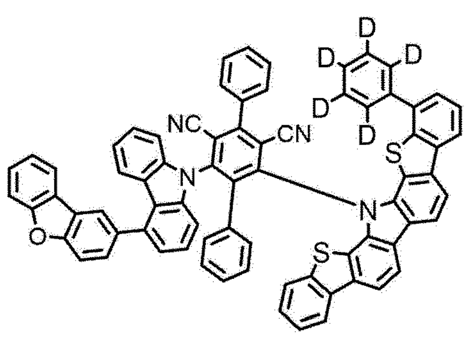
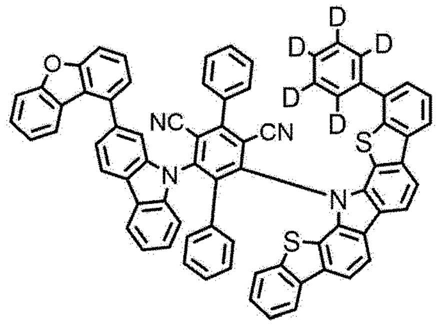

[Formula 128]

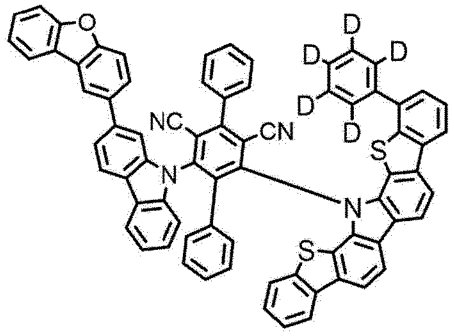

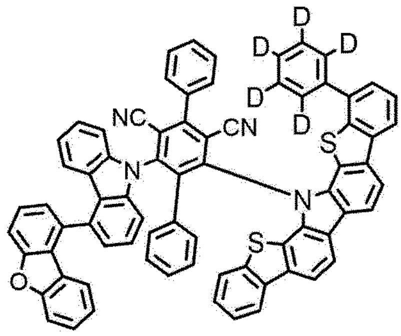

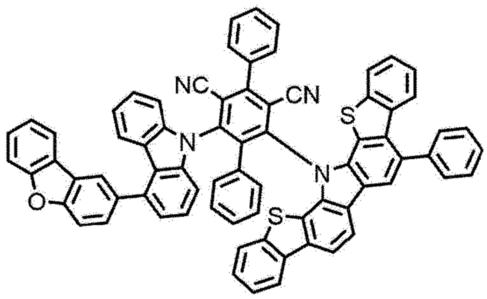

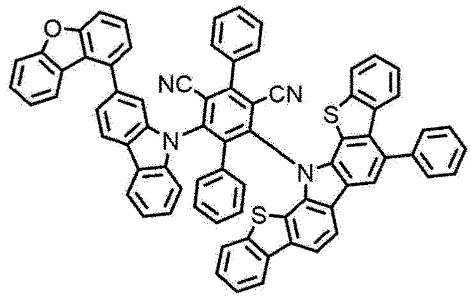

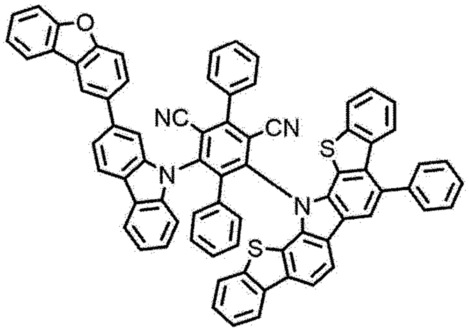

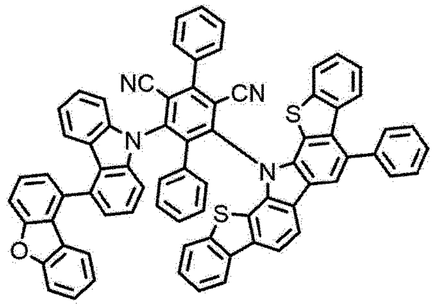

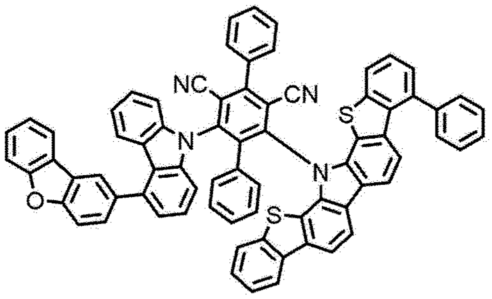

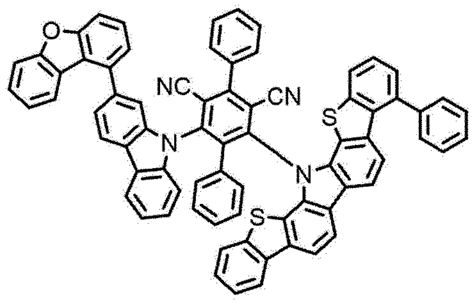

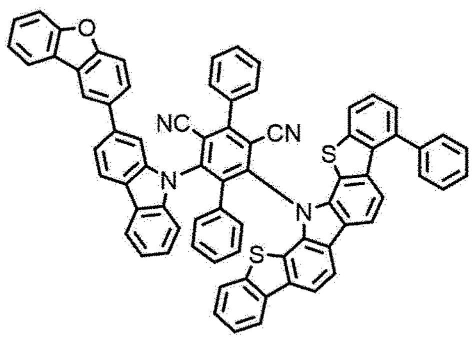

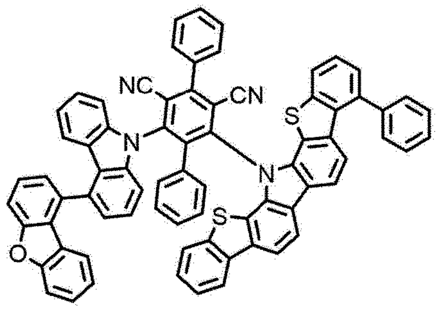

[Formula 129]

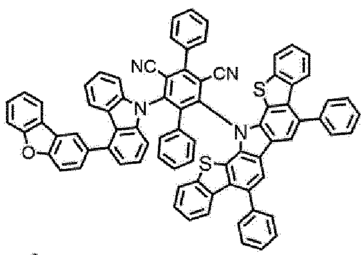

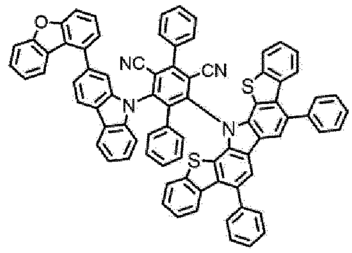

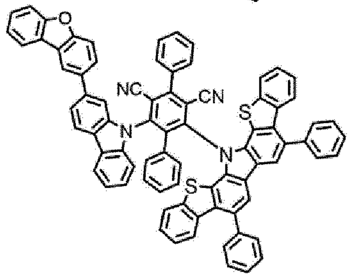

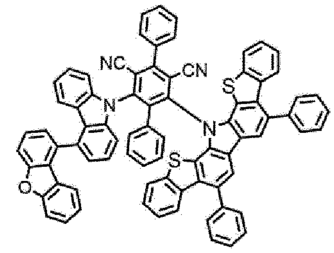

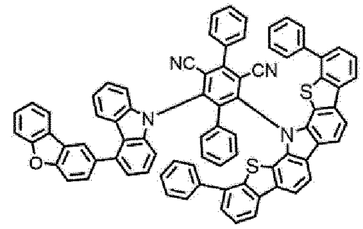

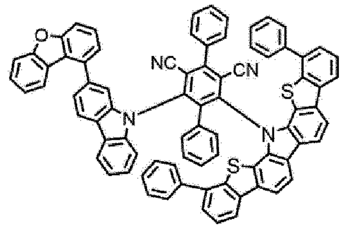

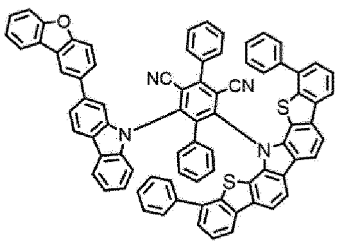

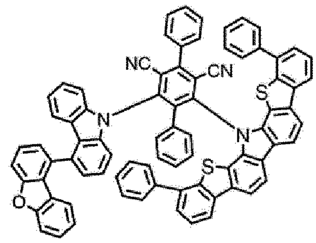

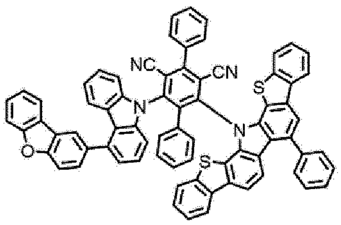

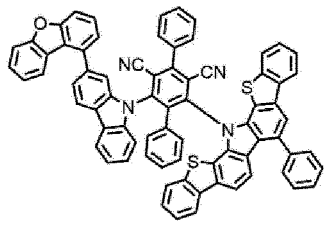

[Formula 130]

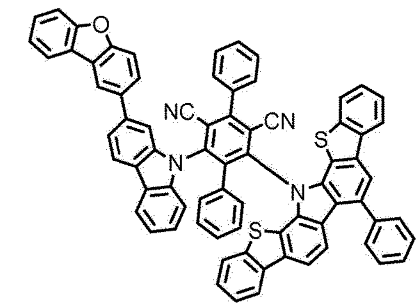

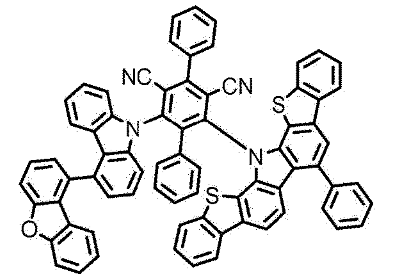

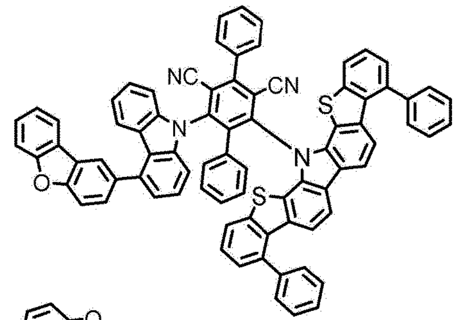

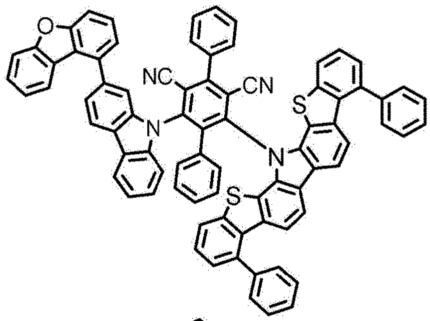

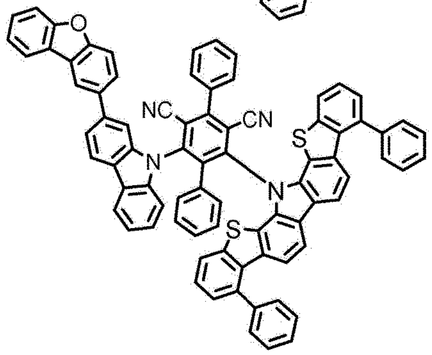

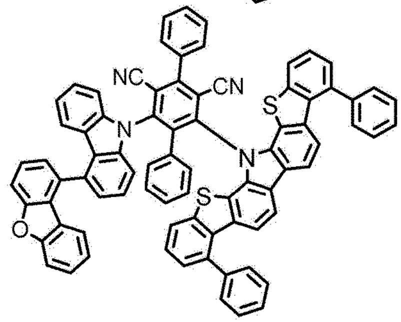

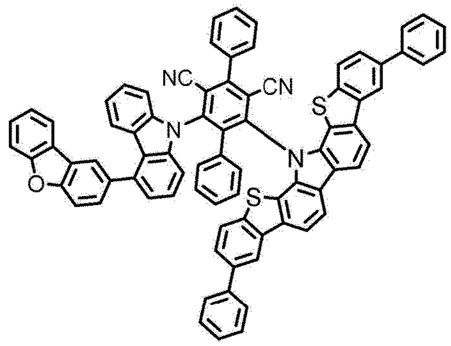

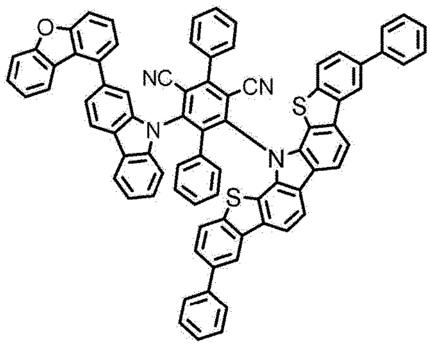

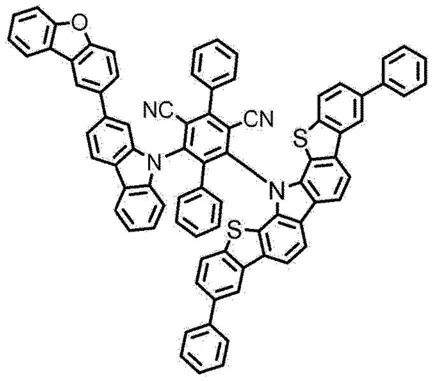

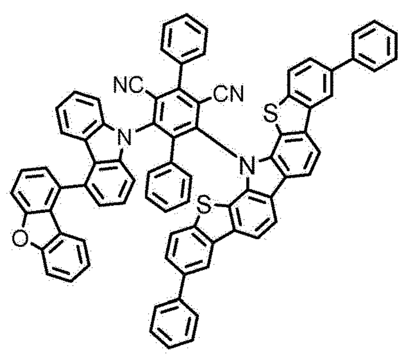

[Formula 131]

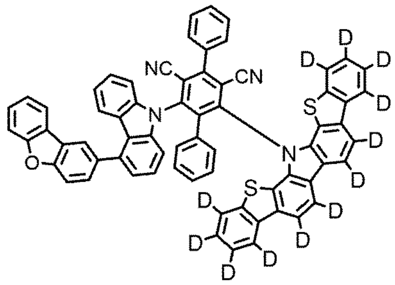

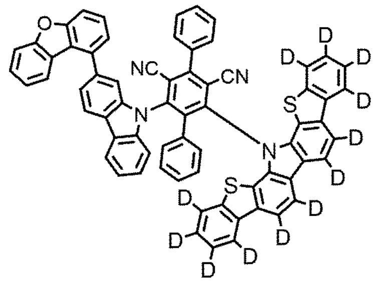

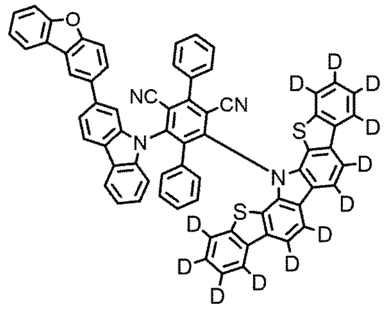

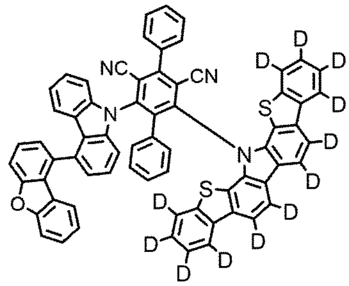

[Formula 132]

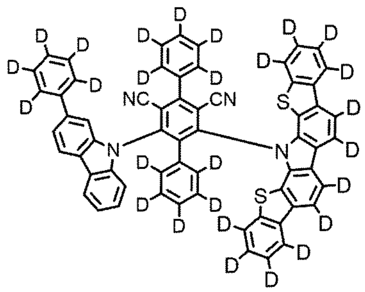

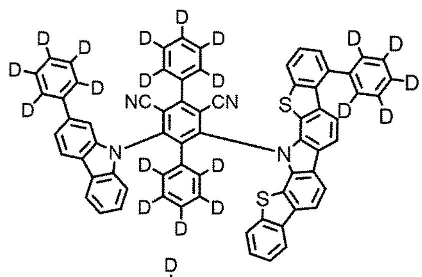

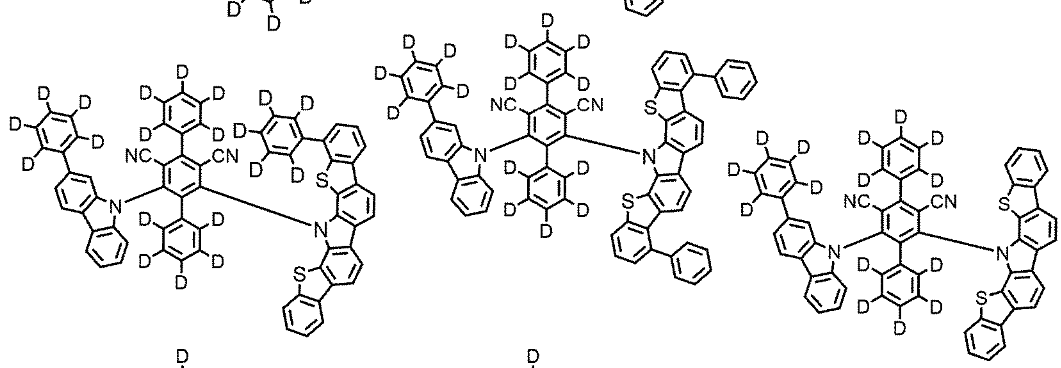

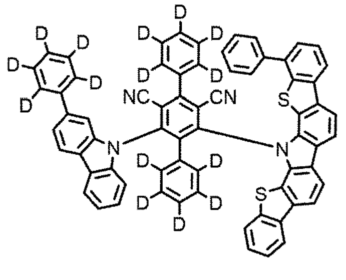

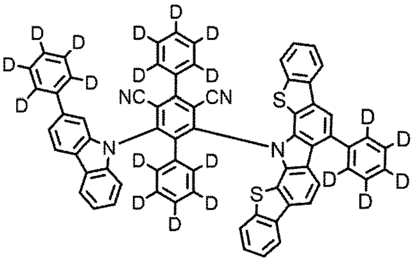

[Formula 133]

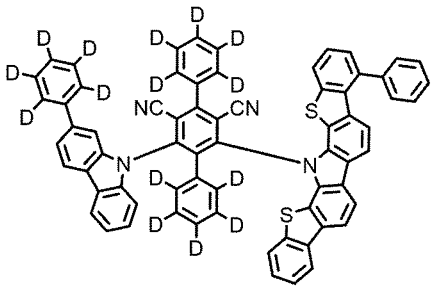

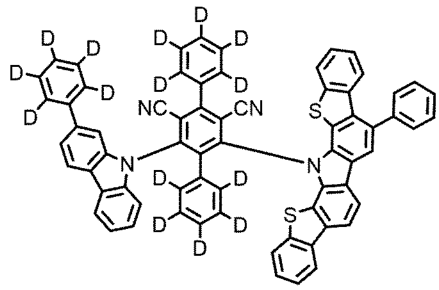

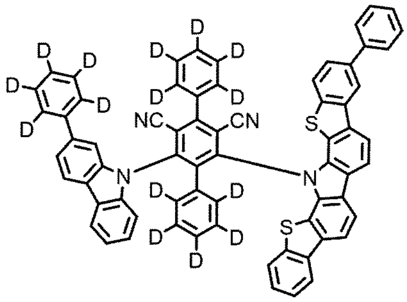

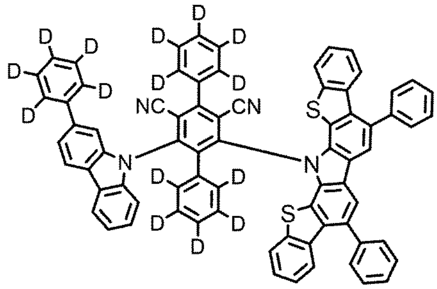

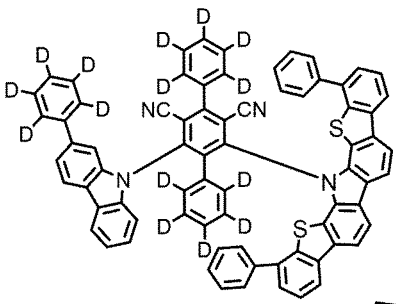

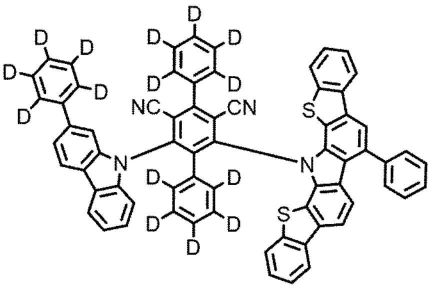

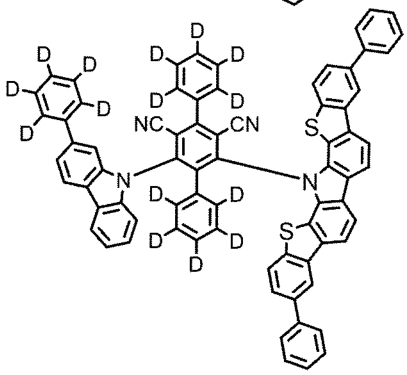

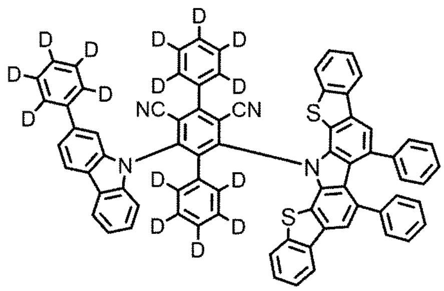

[Formula 134]

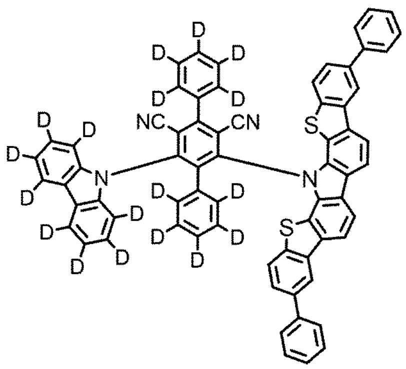

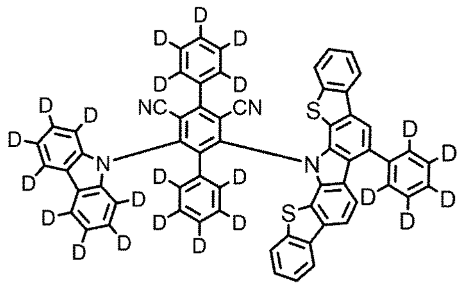

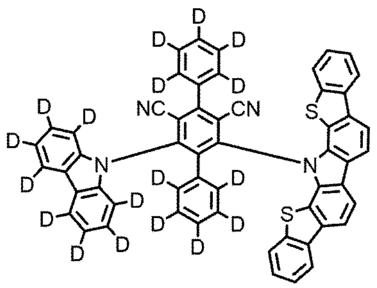

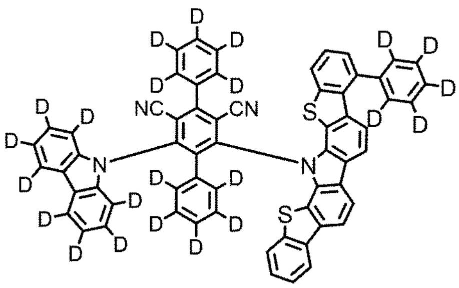

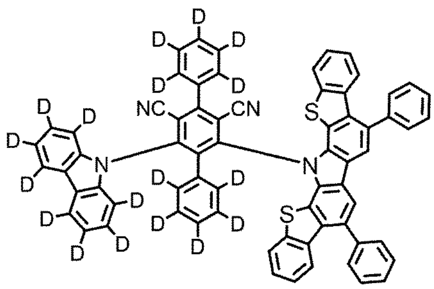

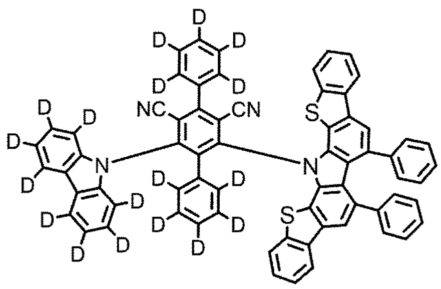

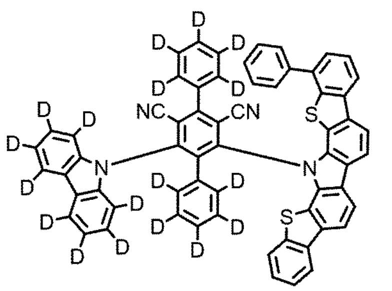

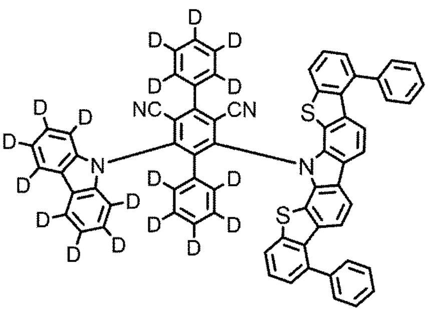

[Formula 135]

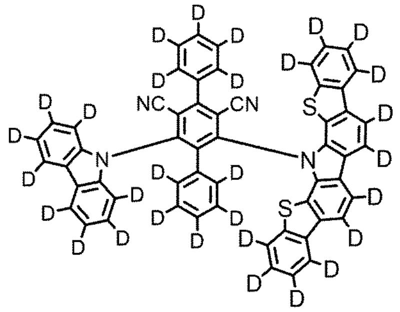

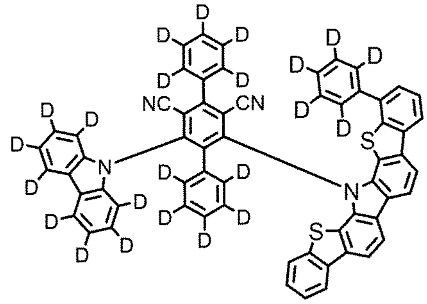

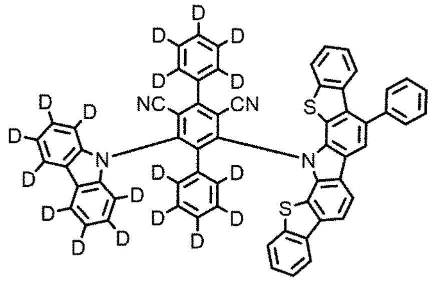

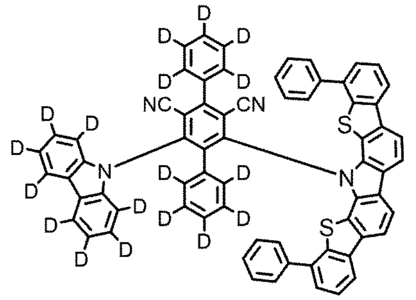

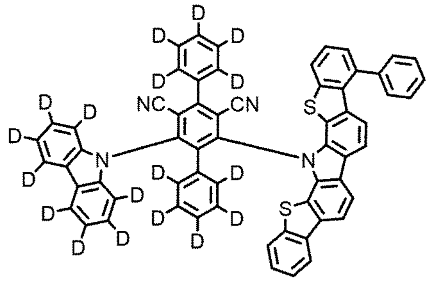

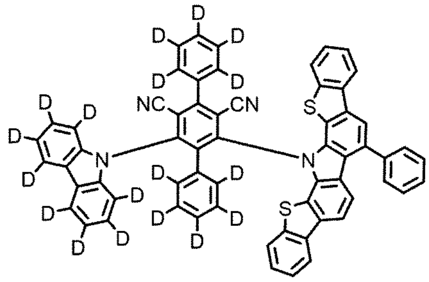

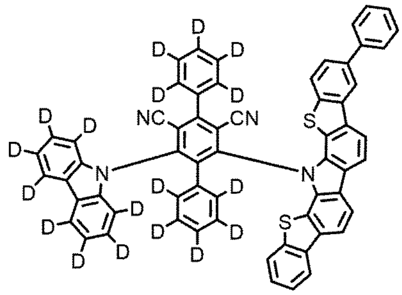

[Formula 136]

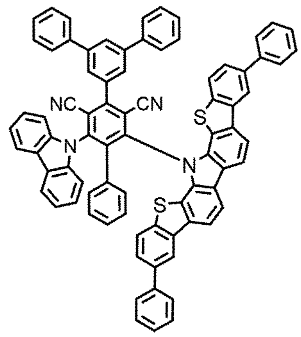
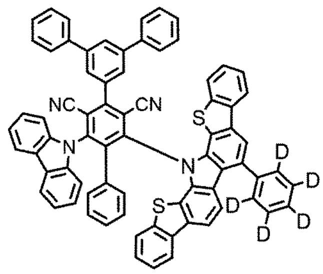
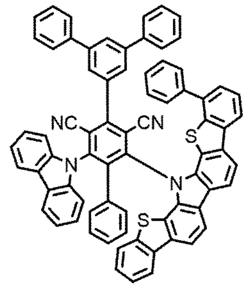
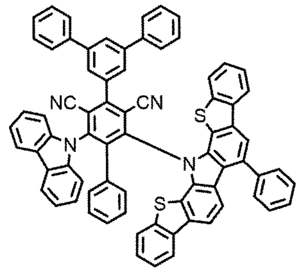
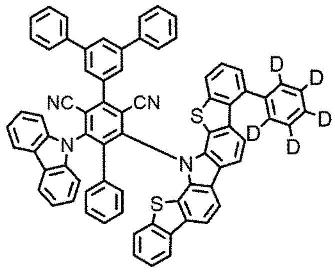
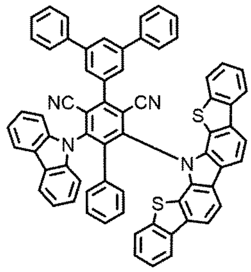
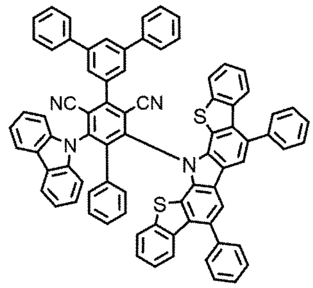
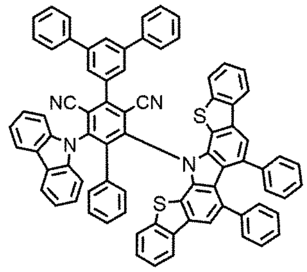
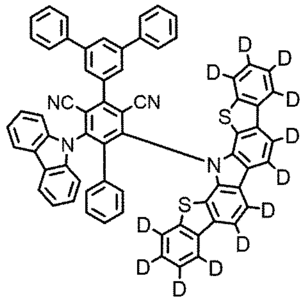
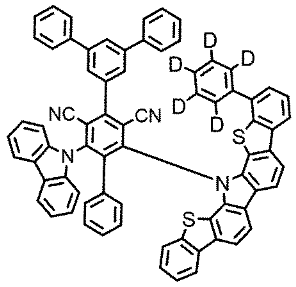
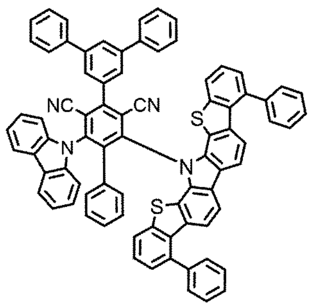
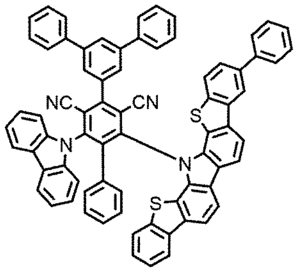

[Formula 137]

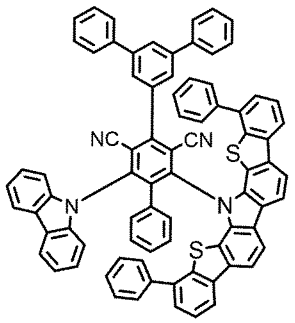
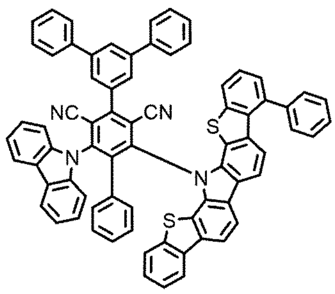
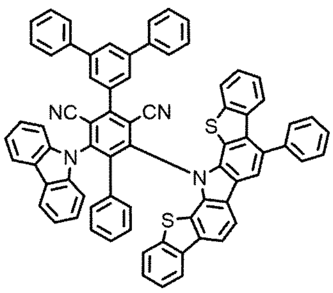

[Formula 138]

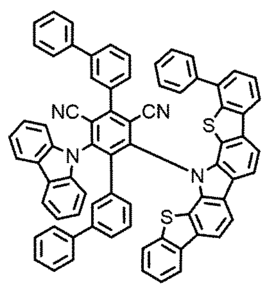

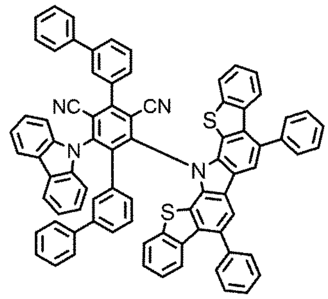

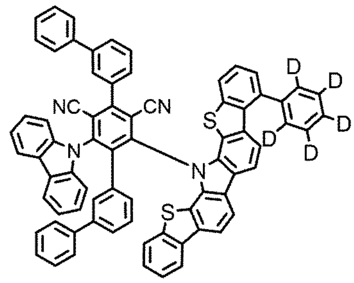

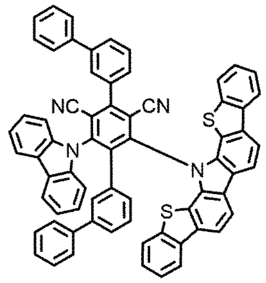

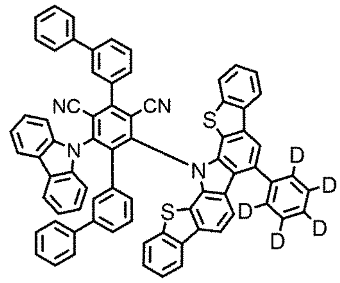

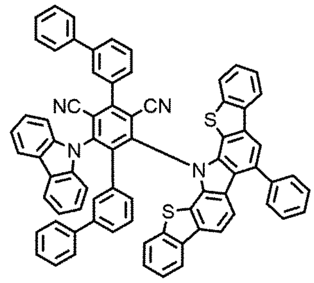

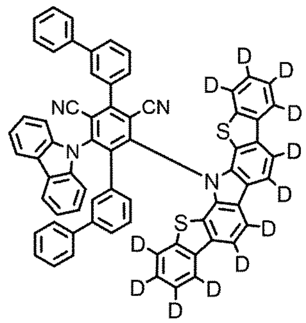

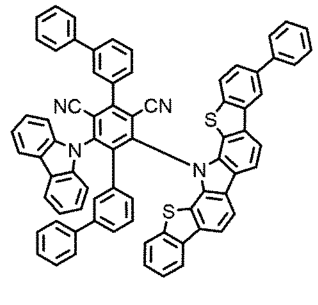

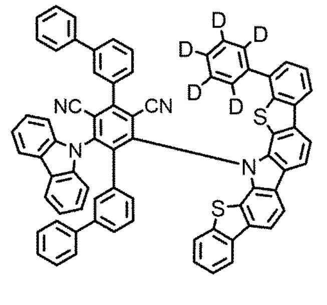

[Formula 139]

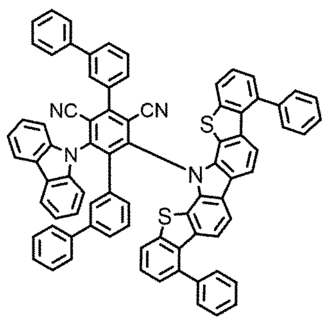

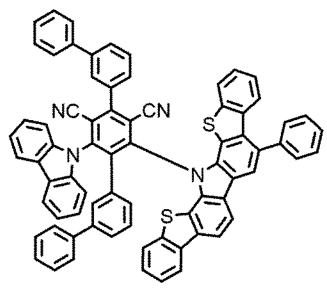

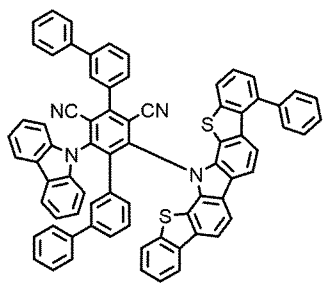

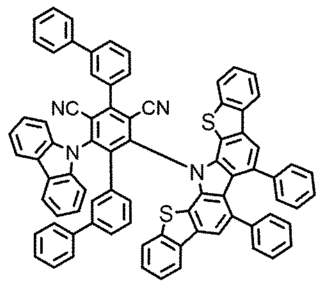

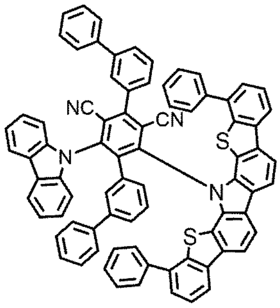

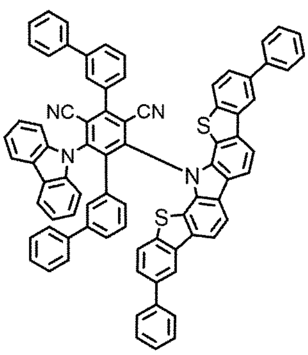

[Formula 140]

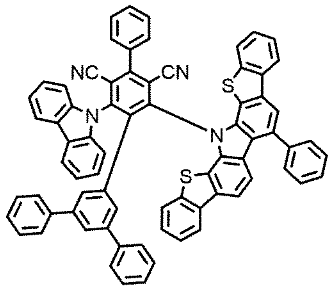
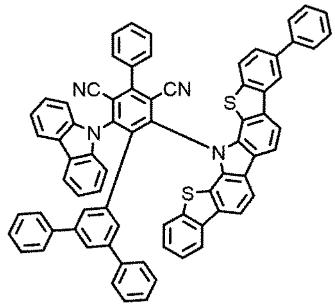
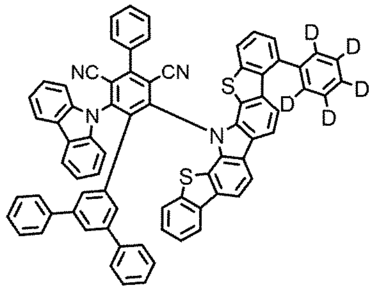
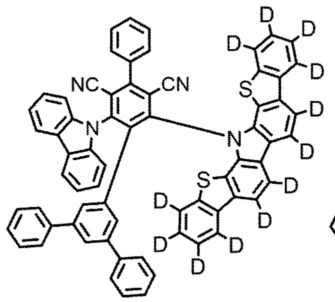
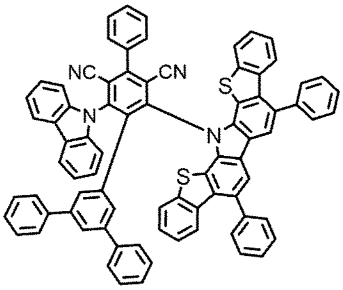
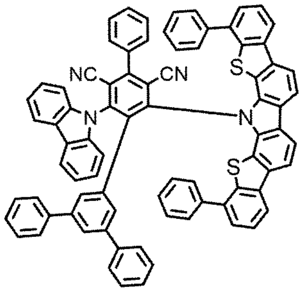

[Formula 141]

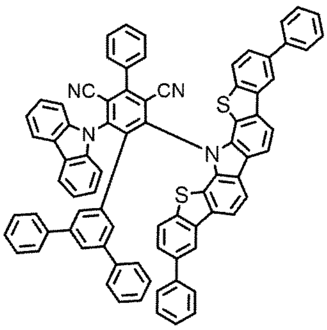
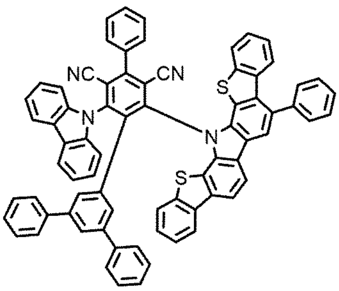
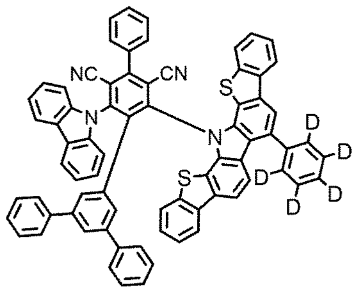
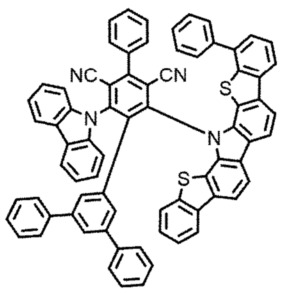
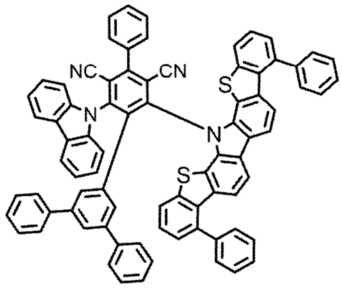
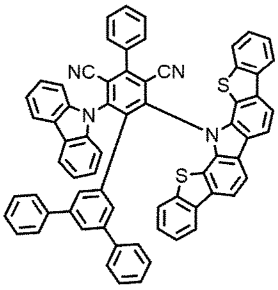
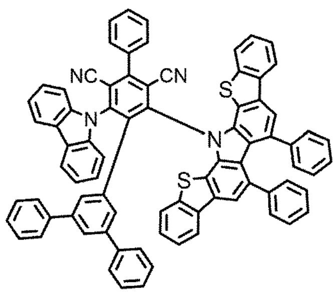
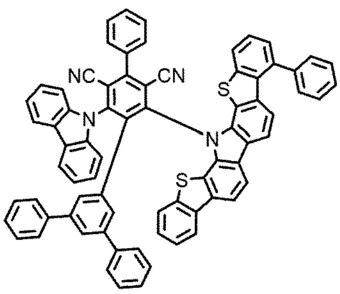
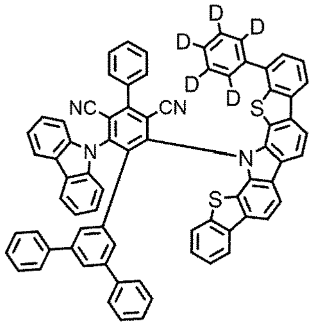

Second Exemplary Embodiment

Organic-Electroluminescence-Device Material

An organic-electroluminescence-device material according to the exemplary embodiment contains the compound according to the first exemplary embodiment. One example is an organic-electroluminescence-device material containing only the compound according to the first exemplary embodiment. Another example is an organic-electroluminescence-device material containing the compound according to the first exemplary embodiment and a compound different from the compound according to the first exemplary embodiment.

In the organic-electroluminescence-device material according to the exemplary embodiment, the compound according to the first exemplary embodiment is preferably a host material. In this case, the organic-electroluminescence-device material may contain the compound according to the first exemplary embodiment as a host material and other compound(s) such as a dopant material.

Moreover, in the organic-electroluminescence-device material according to the exemplary embodiment, the compound according to the first exemplary embodiment is preferably a delayed fluorescence material.

Third Exemplary Embodiment

Organic Electroluminescence Device

An organic EL device according to a third exemplary embodiment will be described.

The organic EL device according to the exemplary embodiment includes an anode, a cathode, and an organic layer between the anode and the cathode. The organic layer includes at least one layer formed from an organic compound(s). Alternatively, the organic layer includes a plurality of layers formed from an organic compound(s). The organic layer may further contain an inorganic compound(s).

In the organic EL device according to the exemplary embodiment, the organic layer contains the compound according to the first exemplary embodiment. Specifically, the organic EL device according to the exemplary embodiment includes the anode, the cathode, and the organic layer in which the organic layer contains the compound according to the first exemplary embodiment as a compound M2.

In the organic EL device according to the exemplary embodiment, the organic layer preferably includes at least one emitting layer, in which the emitting layer preferably contains the compound according to the first exemplary embodiment as the compound M2.

For instance, the organic layer may be one emitting layer, or may further include a layer(s) usable in the organic EL device. Examples of the layer usable in the organic EL device, which are not particularly limited, include at least one selected from the group consisting of a hole injecting layer, a hole transporting layer, an electron blocking layer, a hole blocking layer, an electron transporting layer, and an electron injecting layer.

In an exemplary arrangement, the emitting layer may contain a metal complex.

In an exemplary arrangement, the emitting layer also preferably does not contain a metal complex.

In an exemplary arrangement, the emitting layer preferably does not contain a phosphorescent material (dopant material).

In an exemplary arrangement, the emitting layer preferably does not contain a heavy-metal complex and a phosphorescent rare earth metal complex. Examples of the heavy-metal complex include iridium complex, osmium complex, and platinum complex.

Fig. 3 schematically illustrates an exemplary arrangement of the organic EL device according to the exemplary embodiment.

An organic EL device 1 includes a light-transmissive substrate 2, an anode 3, a cathode 4, and organic layers 10 provided between the anode 3 and the cathode 4. The organic layers 10 are a hole injecting layer 6, a hole transporting layer 7, an emitting layer 5, an electron transporting layer 8, and an electron injecting layer 9 that are layered on the anode 3 in this order. The organic EL device of the invention may have any arrangement without being limited to the arrangement of the organic EL device illustrated in Fig. 3.

Emitting Layer

In the organic EL device according to the exemplary embodiment, the emitting layer contains the compound M1 and the compound M2. The compound M2 in the emitting layer is preferably the compound according to the first exemplary embodiment. In this arrangement, the compound M2 is preferably a host material (also referred to as a matrix material) and the compound M1 is also preferably a dopant material (also referred to as a guest material, emitter or luminescent material).

In the exemplary embodiment, in a case where the emitting layer contains the compound of the first exemplary embodiment, the emitting layer preferably does not contain a phosphorescent metal complex and also preferably does not contain a metal complex other than the phosphorescent metal complex.

Compound M2

The compound M2 is the compound of the first exemplary embodiment. The compound M2 of the exemplary embodiment is preferably a compound exhibiting thermally activated delayed fluorescence.

Compound M1

The compound M1 is preferably a fluorescent compound. The compound M1 is preferably a compound not exhibiting thermally activated delayed fluorescence.

The compound M1 of the exemplary embodiment is not a phosphorescent metal complex. The compound M1 is preferably not a heavy-metal complex. The compound M1 is also preferably not a metal complex.

A fluorescent material is usable as the compound M1 of the exemplary embodiment. Specific examples of the fluorescent material include a bisarylaminonaphthalene derivative, aryl-substituted naphthalene derivative, bisarylaminoanthracene derivative, aryl-substituted anthracene derivative, bisarylaminopyrene derivative, aryl-substituted pyrene derivative, bisarylamino chrysene derivative, aryl-substituted chrysene derivative, bisarylaminofluoranthene derivative, aryl-substituted fluoranthene derivative, indenoperylene derivative, acenaphthofluoranthene derivative, compound including a boron atom, pyromethene boron complex compound, compound having a pyromethene skeleton, metal complex of the compound having a pyrromethene skeleton, diketopyrrolopyrrole derivative, perylene derivative, and naphthacene derivative.

The compound M1 preferably emits light having a maximum peak wavelength in a range from 400 nm to 700 nm.

Herein, the maximum peak wavelength means a peak wavelength of a fluorescence spectrum exhibiting a maximum luminous intensity among fluorescence spectra measured in a toluene solution in which a measurement target compound is dissolved at a concentration ranging from $10^{-6}$ mol/l to $10^{-5}$ mol/l. A spectrophotofluorometer (F-7000 manufactured by Hitachi High-Tech Science Corporation) is used as a measurement apparatus.

The compound M1 preferably exhibits red or green light emission.

Herein, the red light emission refers to light emission whose maximum peak wavelength of fluorescence spectrum is in a range from 600 nm to 660 nm.

When the compound M1 is a red fluorescent compound, the maximum peak wavelength of the compound M1 is preferably in a range from 600 nm to 660 nm, more preferably in a range from 600 nm to 640 nm, and still more preferably in a range from 610 nm to 630 nm.

Herein, the green light emission refers to light emission whose maximum peak wavelength of fluorescence spectrum is in a range from 500 nm to 560 nm.

When the compound M1 is a green fluorescent compound, the maximum peak wavelength of the compound M1 is preferably in a range from 500 nm to 560 nm, more preferably in a range from 500 nm to 540 nm, and still more preferably in a range from 510 nm to 540 nm.

Herein, the blue light emission refers to light emission whose maximum peak wavelength of fluorescence spectrum is in a range from 430 nm to 480 nm.

When the compound M1 is a blue fluorescent compound, the maximum peak wavelength of the compound M1 is preferably in a range from 430 nm to 480 nm, more preferably in a range from 440 nm to 480 nm.

The maximum peak wavelength of the light emitted from the organic EL device is measured as follows.

Voltage is applied on the organic EL device such that a current density becomes 10 mA/$cm^2$, where spectral radiance spectrum is measured by a spectroradiometer CS-2000 (manufactured by Konica Minolta, Inc.). A peak wavelength of an emission spectrum, at which the luminous intensity of the resultant spectral radiance spectrum is at the maximum, is measured and defined as the maximum peak wavelength (unit: nm).

Compound Represented by Formula (D1)

The compound M1 of the exemplary embodiment is also preferably a compound represented by a formula (D1) below.

[Formula 142]

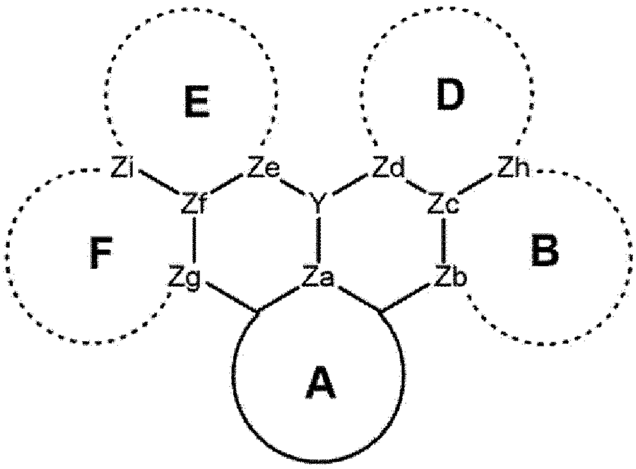

(D1)

In the formula (D1):

a ring A, a ring B, a ring C, a ring D, a ring E, and a ring F are each independently a cyclic structure selected from the group consisting of a substituted or unsubstituted aryl ring having 6 to 30 ring carbon atoms, and a substituted or unsubstituted heterocyclic ring having 5 to 30 ring atoms;
one of the ring B and the ring D is present or both of the ring B and the ring D are present;
when both of the ring B and the ring D are present, the ring B and the ring D share a bond connecting Zc to Zh;
one of the ring E and the ring F is present or both of the ring E and the ring F are present;
when both of the ring E and the ring F are present, the ring E and the ring F share a bond connecting Zf to Zi;
Za is a nitrogen atom or a carbon atom;
Zb is a nitrogen atom or a carbon atom when the ring B present;
Zb is an oxygen atom, a sulfur atom, NRb, $C(Rb_1)(Rb_2)$, or $Si(Rb_3)(Rb_4)$ when the ring B is not present;
Zc is a nitrogen atom or a carbon atom;
Zd is a nitrogen atom or a carbon atom when the ring D is present;
Zd is an oxygen atom, a sulfur atom, or NRd when the ring D is not present;
Ze is a nitrogen atom or a carbon atom when the ring E is present;
Ze is an oxygen atom, a sulfur atom, or NRe when the ring E is not present;
Zf is a nitrogen atom or a carbon atom;
Zg is a nitrogen atom or a carbon atom when the ring F is present;
Zg is an oxygen atom, a sulfur atom, NRg, $C(Rg_1)(Rg_2)$, or $Si(Rg_3)(Rg_4)$ when the ring F is not present;
Zh is a nitrogen atom or a carbon atom;
Zi is a nitrogen atom or a carbon atom;
Y is a boron atom, a phosphorus atom, SiRh, P=O, or P=S;
Rb, $Rb_1$, $Rb_2$, $Rb_3$, $Rb_4$, Rd, Re, Rg, $Rg_1$, $Rg_2$, $Rg_3$, $Rg_4$, and Rh are each independently a hydrogen atom or a substituent;
Rb, $Rb_1$, $Rb_2$, $Rb_3$, $Rb_4$, Rd, Re, Rg, $Rg_1$, $Rg_2$, $Rg_3$, $Rg_4$, and Rh as a substituent are each independently a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted cycloalkyl group having 3 to 30 ring carbon atoms, a group represented by $-Si(R_{911})(R_{912})(R_{913})$, a group represented by $-O-(R_{914})$, a group represented by $-S-(R_{915})$, or a group represented by $-N(R_{916})(R_{917})$; and a bond between Y and Za, a bond between Y and Zd, and a bond between Y and Ze are each a single bond.

In the compound M1, $R_{911}$ to $R_{917}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{911}$ are present, the plurality of $R_{911}$ are mutually the same or different;
when a plurality of $R_{912}$ are present, the plurality of $R_{912}$ are mutually the same or different;
when a plurality of $R_{913}$ are present, the plurality of $R_{913}$ are mutually the same or different;

when a plurality of $R_{914}$ are present, the plurality of $R_{914}$ are mutually the same or different;
when a plurality of $R_{915}$ are present, the plurality of $R_{915}$ are mutually the same or different;
when a plurality of $R_{916}$ are present, the plurality of $R_{916}$ are mutually the same or different; and
when a plurality of $R_{917}$ are present, the plurality of $R_{917}$ are mutually the same or different.

A bond between Y and Za, a bond between Y and Zd, and a bond between Y and Ze are each a single bond and the single bond is not a coordinate bond but a covalent bond.
Herein, a heterocyclic ring is exemplified by a cyclic structure (heterocyclic ring) obtained by removing a bond from a "heterocyclic group" exemplified by the above "Substituent Mentioned Herein." The heterocyclic ring may be substituted or unsubstituted.
Herein, an aryl ring is exemplified by a cyclic structure (aryl ring) obtained by removing a bond from an "aryl group" exemplified by the above "Substituent Mentioned Herein." The aryl ring may be substituted or unsubstituted.
The compound M1 of the exemplary embodiment is also preferably a compound represented by a formula (D11) below.

[Formula 143]

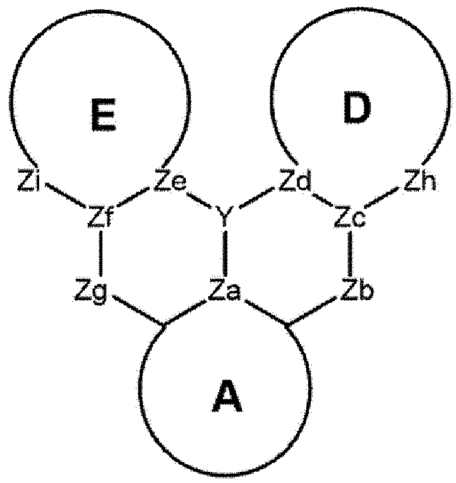

(D11)

In the formula (D11):

a ring A, a ring D, and a ring E are each independently a cyclic structure selected from the group consisting of a substituted or unsubstituted aryl ring having 6 to 30 ring carbon atoms, and a substituted or unsubstituted heterocyclic ring having 5 to 30 ring atoms;
Za is a nitrogen atom or a carbon atom;
Zb is an oxygen atom, a sulfur atom, NRb, $C(Rb_1)(Rb_2)$, or $Si(Rb_3)(Rb_4)$;
Zc is a nitrogen atom or a carbon atom;
Zd is a nitrogen atom or a carbon atom;
Ze is a nitrogen atom or a carbon atom;
Zf is a nitrogen atom or a carbon atom;
Zg is an oxygen atom, a sulfur atom, NRg, $C(Rg_1)(Rg_2)$, or $Si(Rg_3)(Rg_4)$;
Zh is a nitrogen atom or a carbon atom;
Zi is a nitrogen atom or a carbon atom;
Y is a boron atom, a phosphorus atom, SiRh, P=O, or P=S; and
Rb, $Rb_1$, $Rb_2$, $Rb_3$, $Rb_4$, Rg, $Rg_1$, $Rg_2$, $Rg_3$, $Rg_4$, and Rh each independently represent the same as Rb, $Rb_1$, $Rb_2$, $Rb_3$, $Rb_4$, Rg, $Rg_1$, $Rg_2$, $Rg_3$, $Rg_4$, and Rh in the formula (D1).

The compound M1 of the exemplary embodiment is also preferably a compound represented by a formula (D16) below.

[Formula 144]

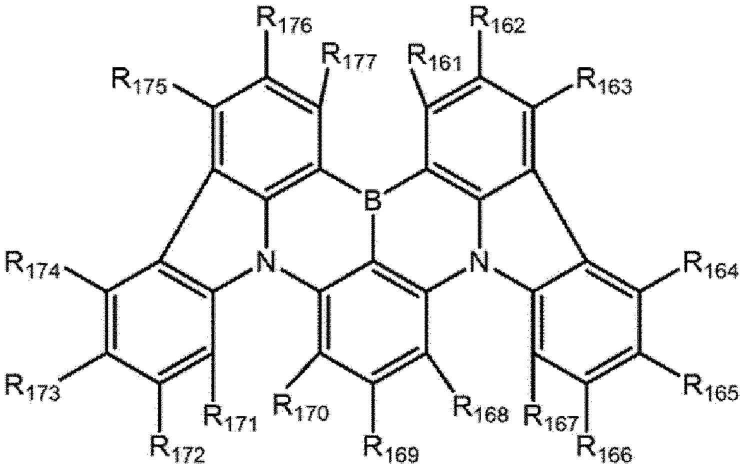

(D16)

In the formula (D16):

at least one combination of adjacent two or more of $R_{161}$ to $R_{177}$ are mutually bonded to form a substituted or unsubstituted monocyclic ring, mutually bonded to form a substituted or unsubstituted fused ring, or not mutually bonded;

$R_{161}$ to $R_{177}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aralkyl group having 7 to 50 carbon atoms, a group represented by -Si($R_{961}$)($R_{962}$)($R_{963}$), a group represented by -O-($R_{964}$), a group represented by -S-($R_{965}$), a group represented by -N($R_{966}$)($R_{967}$), a group represented by -C(=O)$R_{968}$, a group represented by -COO$R_{969}$, a halogen atom, a cyano group, a nitro group, a substituted or unsubstituted aryl group having 6 to 50 ring carbon atoms, or a substituted or unsubstituted heterocyclic group having 5 to 50 ring atoms;

$R_{961}$ to $R_{969}$ are each independently a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms;

when a plurality of $R_{961}$ are present, the plurality of $R_{961}$ are mutually the same or different;
when a plurality of $R_{962}$ are present, the plurality of $R_{962}$ are mutually the same or different;
when a plurality of $R_{963}$ are present, the plurality of $R_{963}$ are mutually the same or different;
when a plurality of $R_{964}$ are present, the plurality of $R_{964}$ are mutually the same or different;
when a plurality of $R_{965}$ are present, the plurality of $R_{965}$ are mutually the same or different;
when a plurality of $R_{966}$ are present, the plurality of $R_{966}$ are mutually the same or different;
when a plurality of $R_{967}$ are present, the plurality of $R_{967}$ are mutually the same or different;
when a plurality of $R_{968}$ are present, the plurality of $R_{968}$ are mutually the same or different; and
when a plurality of $R_{969}$ are present, the plurality of $R_{969}$ are mutually the same or different.

Compound Represented by Formula (D10)

In the organic EL device according to the exemplary embodiment, the compound M1 is also preferably a compound represented by a formula (D10) below. A compound represented by the formula (D1) is also preferably a compound represented by the formula (D10) below.

[Formula 145]

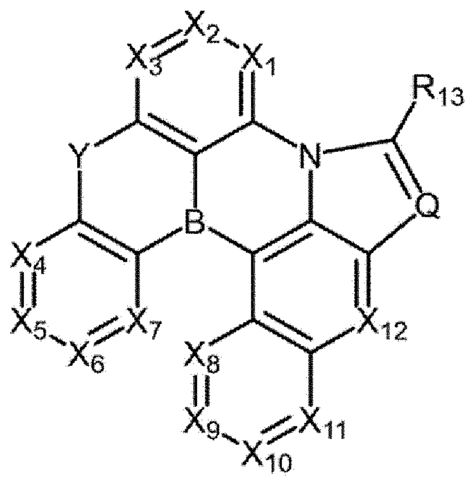

(D10)

In the formula (D10):

$X_1$ is $CR_1$ or a nitrogen atom;
$X_2$ is $CR_2$ or a nitrogen atom;
$X_3$ is $CR_3$ or a nitrogen atom;
$X_4$ is $CR_4$ or a nitrogen atom;
$X_5$ is $CR_5$ or a nitrogen atom;
$X_6$ is $CR_6$ or a nitrogen atom;
$X_7$ is $CR_7$, a nitrogen atom, or a carbon atom bonded to $X_8$ with a single bond;
$X_8$ is $CR_8$, a nitrogen atom, or a carbon atom bonded to $X_7$ with a single bond;
$X_9$ is $CR_9$ or a nitrogen atom;
$X_{10}$ is $CR_{10}$ or a nitrogen atom;
$X_{11}$ is $CR_{11}$ or a nitrogen atom;
$X_{12}$ is $CR_{12}$ or a nitrogen atom;
Q is $CR_Q$ or a nitrogen atom;
Y is $NR_{Y1}$, an oxygen atom, a sulfur atom, $C(R_{Y2})(R_{Y3})$, or $Si(R_{Y4})(R_{Y5})$;
at least one combination of adjacent two or more of $R_1$ to $R_6$ and $R_9$ to $R_{11}$ are mutually bonded to form a substituted or unsubstituted monocyclic ring, mutually bonded to form a substituted or unsubstituted fused ring, or not mutually bonded;
at least one combination of adjacent two or more of $R_3$, $R_4$, and $R_{Y1}$ are mutually bonded to form a substituted or unsubstituted monocyclic ring, mutually bonded to form a substituted or unsubstituted fused ring, or not mutually bonded;
at least one hydrogen atom in a monocycllic ring or a fused ring formed by mutually bonding at least one combination of adjacent two or more of $R_3$, $R_4$, and $R_{Y1}$ is substituted or not substituted by at least one substituent selected from the group consisting of an alkyl group having 1 to 50 carbon atoms, an aryl group having 6 to 50 ring carbon atoms, a heterocyclic group having 5 to 50 ring atoms, a group represented by $-O-(R_{920})$, and a group represented by $-N(R_{921})(R_{922})$;
at least one hydrogen atom of the substituent is substituted or not substituted by an aryl group having 6 to 50 ring carbon atoms or an alkyl group having 1 to 50 carbon atoms;
$R_1$ to $R_{11}$ forming neither the substituted or unsubstituted monocyclic ring nor the substituted or unsubstituted fused ring, $R_{12}$ to $R_{13}$, and $R_Q$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{911})(R_{912})(R_{913})$, a group represented by $-O-(R_{914})$, a group represented by $-S-(R_{915})$, a group represented by $-N(R_{916})(R_{917})$, a substituted or unsubstituted aralkyl group having 7 to 50 carbon atoms, a group represented by $-C(=O)R_{918}$, a group represented by $-COOR_{919}$, a halogen atom, a cyano group, a nitro group, a substituted or unsubstituted aryl group having 6 to 50 ring carbon atoms, or a substituted or unsubstituted heterocyclic group having 5 to 50 ring atoms;
$R_{Y1}$ forming neither the substituted or unsubstituted monocyclic ring nor the substituted or unsubstituted fused ring

is a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
a combination of $R_{Y2}$ and $R_{Y3}$ are mutually bonded to form a substituted or unsubstituted monocyclic ring, mutually bonded to form a substituted or unsubstituted fused ring, or not mutually bonded;
$R_{Y2}$ and $R_{Y3}$ forming neither the substituted or unsubstituted monocyclic ring nor the substituted or unsubstituted fused ring and $R_{Y4}$ and $R_{Y5}$ are each independently a hydrogen atom, a halogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms;
$R_{911}$ to $R_{922}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
when a plurality of $R_{911}$ are present, the plurality of $R_{911}$ are mutually the same or different;
when a plurality of $R_{912}$ are present, the plurality of $R_{912}$ are mutually the same or different;
when a plurality of $R_{913}$ are present, the plurality of $R_{913}$ are mutually the same or different;
when a plurality of $R_{914}$ are present, the plurality of $R_{914}$ are mutually the same or different;
when a plurality of $R_{915}$ are present, the plurality of $R_{915}$ are mutually the same or different;
when a plurality of $R_{916}$ are present, the plurality of $R_{916}$ are mutually the same or different;
when a plurality of $R_{917}$ are present, the plurality of $R_{917}$ are mutually the same or different;
when a plurality of $R_{918}$ are present, the plurality of $R_{918}$ are mutually the same or different;
when a plurality of $R_{919}$ are present, the plurality of $R_{919}$ are mutually the same or different;
when a plurality of $R_{920}$ are present, the plurality of $R_{920}$ are mutually the same or different;
when a plurality of $R_{921}$ are present, the plurality of $R_{921}$ are mutually the same or different; and
when a plurality of $R_{922}$ are present, the plurality of $R_{922}$ are mutually the same or different.

In a compound represented by the formula (D10), when $X_7$ is a carbon atom bonded to $X_8$ with a single bond and $X_8$ is a carbon atom bonded to $X_7$ with a single bond, the formula (D10) is represented by a formula (D10A) below.

[Formula 146]

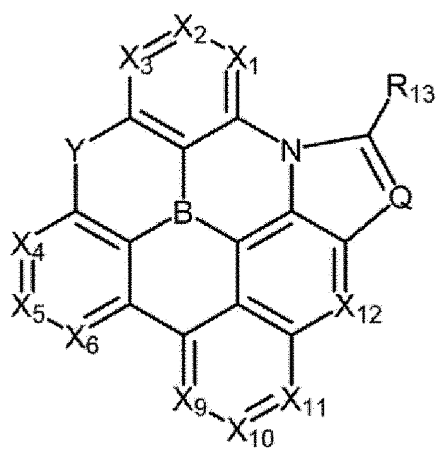

(D10A)

In the formula (D10A), $X_1$ to $X_6$, $X_9$ to $X_{12}$, Y, Q, and $R_{13}$ are each independently defined as in the formula (D10).
The compound represented by the formula (D10) is also preferably represented by a formula (D12) below.

[Formula 147]

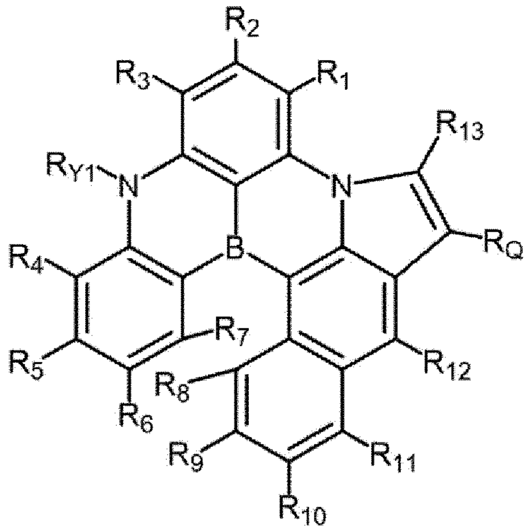

(D12)

In the formula (D12), $R_1$ to $R_{13}$, $R_{Y1}$, and $R_Q$ are each independently defined the same as in the formula (D10).

The compound represented by the formula (D10) is also preferably represented by a formula (D12A) below.

[Formula 148]

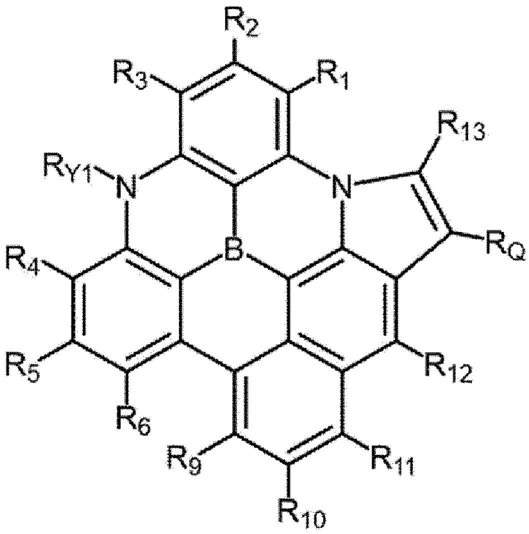

(D12A)

In the formula (D12A), $R_1$ to $R_6$, $R_9$ to $R_{13}$, $R_{Y1}$, and $R_Q$ are each independently defined as in the formula (D10).

The compound represented by the formula (D10) is also preferably represented by a formula (D13) below.

[Formula 149]

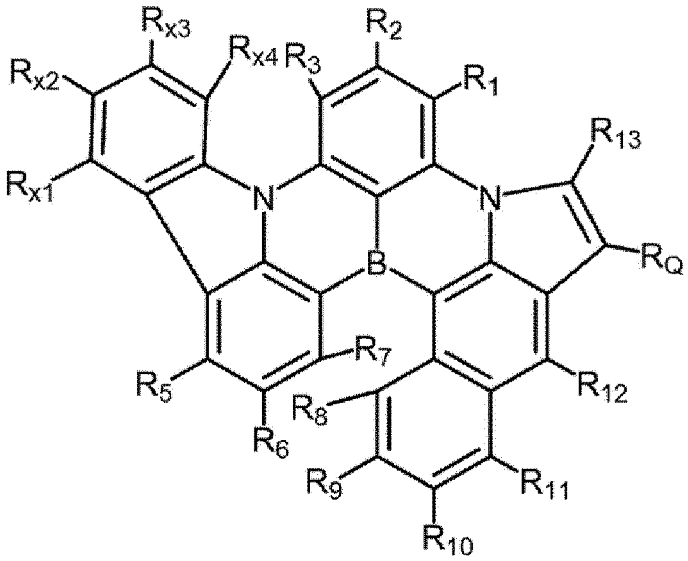

(D13)

In the formula (D13):

$R_1$ to $R_3$, $R_5$ to $R_{13}$, and $R_Q$ are each independently defined as in the formula (D10);
at least one combination of adjacent two or more of $R_{x1}$ to $R_{x4}$ are mutually bonded to form a substituted or unsubstituted monocyclic ring, mutually bonded to form a substituted or unsubstituted fused ring, or not mutually bonded;
$R_{x1}$ to $R_{x4}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aralkyl group having 7 to 50 carbon atoms, a group represented by -Si($R_{931}$)($R_{932}$)($R_{933}$), a group represented by -O-($R_{934}$), a group represented by -S-(Rsss), a group represented by -N($R_{936}$)($R_{937}$), a substituted or unsubstituted aralkyl group having 7 to 50 carbon atoms, a group represented by -C(=O)$R_{938}$, a group represented by -COOR$_{939}$, a halogen atom, a cyano group, a nitro group, a substituted or unsubstituted aryl group having 6 to 50 ring carbon atoms, or a substituted or unsubstituted heterocyclic group having 5 to 50 ring atoms;
$R_{931}$ to $R_{939}$ are each independently a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms;
when a plurality of $R_{931}$ are present, the plurality of $R_{931}$ are mutually the same or different;
when a plurality of $R_{932}$ are present, the plurality of $R_{932}$ are mutually the same or different;
when a plurality of $R_{933}$ are present, the plurality of $R_{933}$ are mutually the same or different;
when a plurality of $R_{934}$ are present, the plurality of $R_{934}$ are mutually the same or different;
when a plurality of $R_{935}$ are present, the plurality of $R_{935}$ are mutually the same or different;
when a plurality of $R_{936}$ are present, the plurality of $R_{936}$ are mutually the same or different;
when a plurality of $R_{937}$ are present, the plurality of $R_{937}$ are mutually the same or different;
when a plurality of $R_{938}$ are present, the plurality of $R_{938}$ are mutually the same or different; and
when a plurality of $R_{939}$ are present, the plurality of $R_{939}$ are mutually the same or different.

In the formula (D13), for instance, a combination of $R_5$ and $R_6$ are mutually bonded to form a substituted or unsubstituted monocyclic ring, mutually bonded to form a substituted or unsubstituted fused ring, or not bonded.
The compound represented by the formula (D10) is also preferably represented by a formula (D13A) below.

[Formula 150]

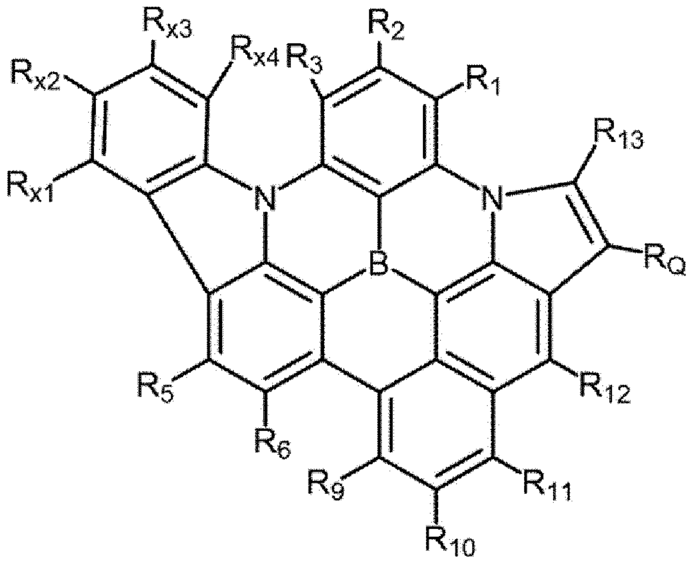

(D13A)

In the formula (D13A): $R_1$ to $R_3$, $R_5$ to $R_6$, $R_9$ to $R_{13}$, and $R_Q$ are each independently defined as in the formula (D10); and $R_{x1}$ to $R_{x4}$ are each independently defined as in the formula (D13).

It is also preferable that $R_1$ to $R_{13}$ and $R_Q$ in the compound represented by the formula (D10) are each independently a hydrogen atom, a substituted or unsubstituted alkyl group having 1 to 50 carbon atoms, a substituted or unsubstituted aryl group having 6 to 50 ring carbon atoms, or a substituted or unsubstituted heteroaryl group having 5 to 50 ring atoms.

It is also preferable that $R_1$ to $R_{13}$ and $R_Q$ in the compound represented by the formula (D10) are each independently a hydrogen atom, a substituted or unsubstituted alkyl group having 1 to 25 carbon atoms, a substituted or unsubstituted aryl group having 6 to 25 ring carbon atoms, or a substituted or unsubstituted heteroaryl group having 5 to 25 ring atoms.

It is also preferable that $R_1$ to $R_3$, $R_5$ to $R_{13}$, $R_Q$, and $R_{x1}$ to $R_{x4}$ in a compound represented by the formula (D10) are each independently a hydrogen atom, a substituted or unsubstituted alkyl group having 1 to 50 carbon atoms, a substituted or unsubstituted aryl group having 6 to 50 ring carbon atoms, or a substituted or unsubstituted heteroaryl group having 5 to 50 ring atoms.

It is also preferable that $R_1$ to $R_3$, $R_5$ to $R_{13}$, $R_Q$, and $R_{x1}$ to $R_{x4}$ in a compound represented by the formula (D10) are each independently a hydrogen atom, a substituted or unsubstituted alkyl group having 1 to 25 carbon atoms, a substituted or unsubstituted aryl group having 6 to 25 ring carbon atoms, or a substituted or unsubstituted heteroaryl group having 5 to 25 ring atoms.

It is preferable that $R_1$ to $R_{13}$, $R_Q$, and $R_{x1}$ to $R_{x4}$ in a compound represented by the formula (D10) are each independently a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

It is preferable that $R_1$ to $R_{13}$, $R_Q$, and $R_{x1}$ to $R_{x4}$ in a compound represented by the formula (D10) are each independently a hydrogen atom, a substituted or unsubstituted alkyl group having 1 to 25 carbon atoms, a substituted or unsubstituted aryl group having 6 to 25 ring carbon atoms, or a substituted or unsubstituted heteroaryl group having 5 to 25 ring atoms.

The compound represented by the formula (D10) is also preferably represented by a formula (D14) below.

[Formula 151]

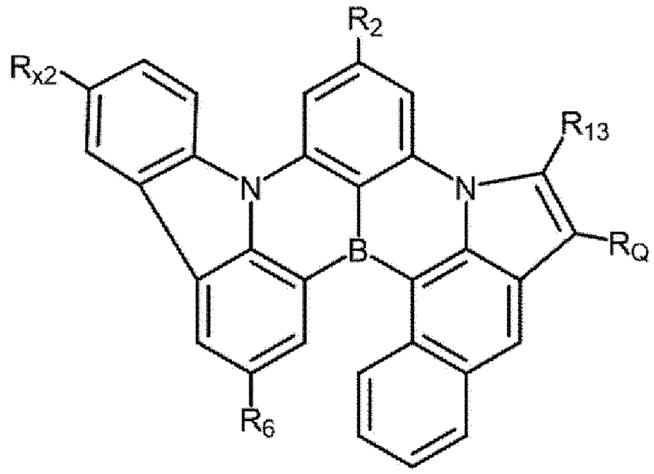

(D14)

In the formula (D14), $R_2$, $R_6$, $R_{13}$, $R_Q$, and $R_{x2}$ are each independently a hydrogen atom, a substituted or unsubstituted alkyl group having 1 to 10 carbon atoms, a substituted or unsubstituted aryl group having 6 to 12 ring carbon atoms, or a substituted or unsubstituted heteroaryl group having 5 to 18 ring atoms.

The compound represented by the formula (D10) is also preferably represented by a formula (D15) below.

[Formula 152]

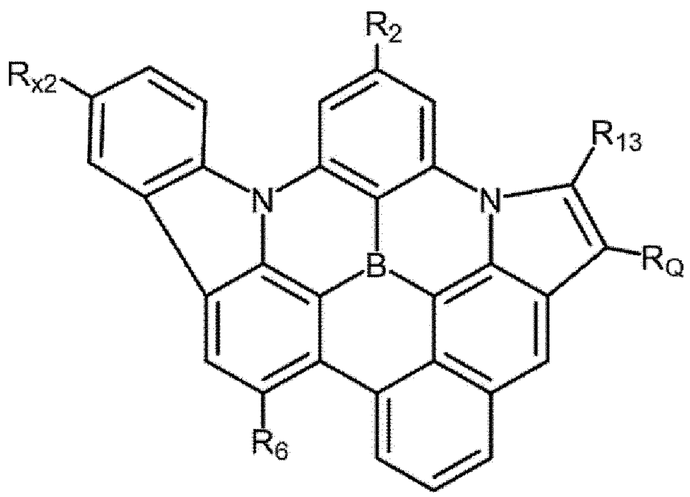

(D15)

In the formula (D15), $R_2$, $R_6$, $R_{13}$, $R_Q$, and $R_{x2}$ are each independently a hydrogen atom, a substituted or unsubstituted alkyl group having 1 to 10 carbon atoms, a substituted or unsubstituted aryl group having 6 to 12 ring carbon atoms, or a substituted or unsubstituted heteroaryl group having 5 to 18 ring atoms.

It is preferable that $R_{13}$ and $R_Q$ in the compound represented by the formula (D10) are each independently a substituted or unsubstituted alkyl group having 1 to 10 carbon atoms, a substituted or unsubstituted phenyl group, a substituted or unsubstituted naphthyl group, or a substituted or unsubstituted dibenzofuranyl group.

In a compound represented by the formula (D10), $R_6$ and $R_{x2}$ are preferably each independently a hydrogen atom or a substituted or unsubstituted alkyl group having 1 to 10 carbon atoms.

Compound Represented by Formula (20)

The compound M1 of the exemplary embodiment is also preferably a compound represented by a formula (20) below.

[Formula 153]

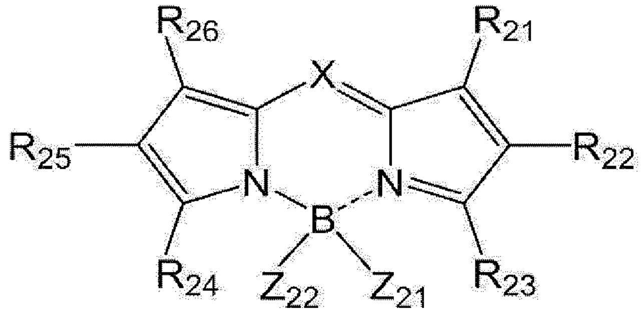

(20)

In the formula (20):

X is a nitrogen atom or a carbon atom bonded to Y;
Y is a hydrogen atom or a substituent;
$R_{21}$ to $R_{26}$ are each independently a hydrogen atom or a substituent, or at least one combination of a combination of $R_{21}$ and $R_{22}$, a combination of $R_{22}$ and $R_{23}$, a combination of $R_{24}$ and $R_{25}$, or a combination of $R_{25}$ and $R_{26}$ are mutually bonded to form a ring;
Y and $R_{21}$ to $R_{26}$ as a substituent are each independently selected from the group consisting of a substituted or unsubstituted alkyl group having 1 to 30 carbon atoms, a substituted or unsubstituted alkyl halide group having 1 to 30 carbon atoms, a substituted or unsubstituted cycloalkyl group having 3 to 30 ring carbon atoms, a substituted or unsubstituted aryl group having 6 to 30 ring carbon atoms, a substituted or unsubstituted alkoxy group having 1 to 30 carbon atoms, a substituted or unsubstituted alkoxy halide group having 1 to 30 carbon atoms, a substituted or unsubstituted alkylthio group having 1 to 30 carbon atoms, a substituted or unsubstituted aryloxy group having 6 to 30 ring carbon atoms, a substituted or unsubstituted arylthio group having 6 to 30 ring carbon atoms, a substituted or unsubstituted alkenyl group having 2 to 30 carbon atoms, a substituted or unsubstituted aralkyl group having 7 to 30 carbon atoms, a substituted or unsubstituted heteroaryl group having 5 to 30 ring atoms, a halogen atom, a carboxy group, a substituted or unsubstituted ester group, a substituted or unsubstituted carbamoyl group, a substituted or unsubstituted amino group, a nitro group, a cyano group, a substituted or unsubstituted silyl group, and a substituted or unsubstituted siloxanyl group;
$Z_{21}$ and $Z_{22}$ are each independently a substituent, or $Z_{21}$ and $Z_{22}$ are mutually bonded to form a ring; and
$Z_{21}$ and $Z_{22}$ as a substituent are each independently selected from the group consisting of a halogen atom, a substituted or unsubstituted alkyl group having 1 to 30 carbon atoms, a substituted or unsubstituted alkyl halide group having 1 to 30 carbon atoms, a substituted or unsubstituted aryl group having 6 to 30 ring carbon atoms, a substituted or unsubstituted alkoxy group having 1 to 30 carbon atoms, a substituted or unsubstituted alkoxy halide group having 1 to 30 carbon atoms, and a substituted or unsubstituted aryloxy group having 6 to 30 ring carbon atoms.

Producing Method of Compound M1

The compound M1 according to the exemplary embodiment can be produced by application of known substitution reactions and materials depending on a target compound, according to a known synthesis method or in a similar manner as the synthesis method.

Specific Examples of Compound M1

Specific examples of the compound M1 in the exemplary embodiment include compounds below. However, the invention is by no means limited to the specific examples of the compound. A coordinate bond between a boron atom and a nitrogen atom in a pyrromethene skeleton is shown by various means such as a solid line, a broken line, an arrow, and omission. Herein, the coordinate bond is shown by a solid line or a broken line, or the description of the coordinate bond is omitted.

[Formula 154]

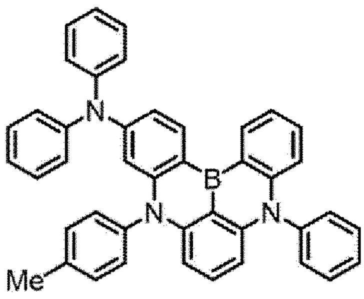

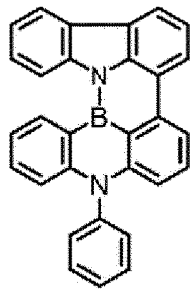

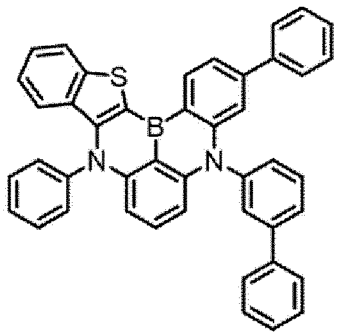

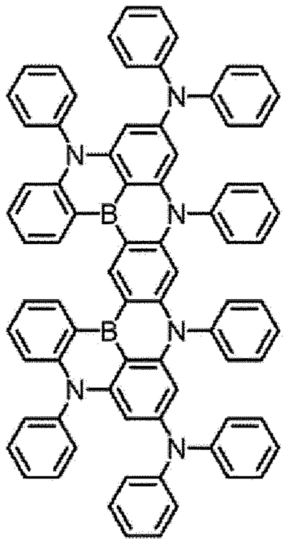

[Formula 155]

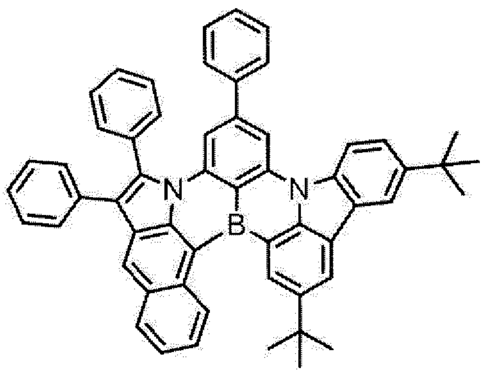

[Formula 156]

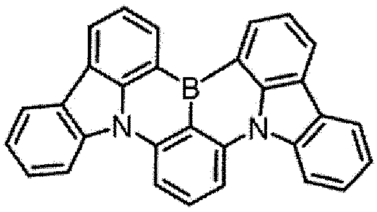

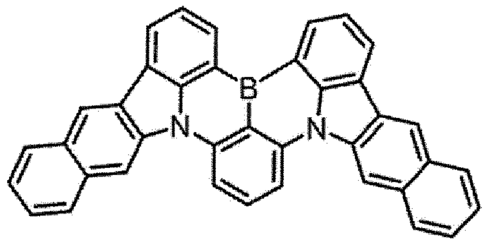

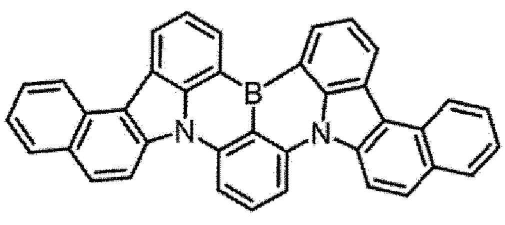

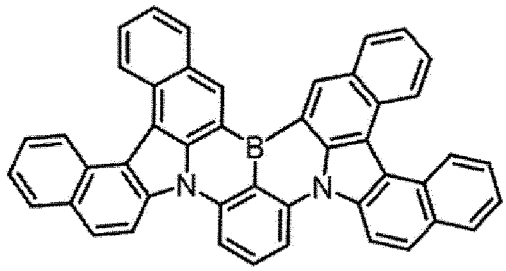

[Formula 157]

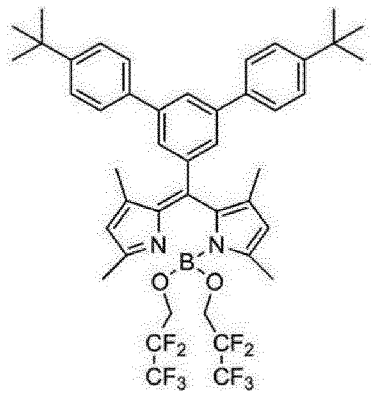

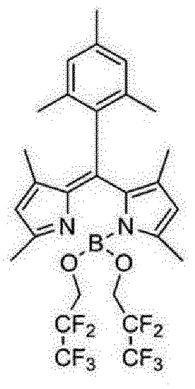

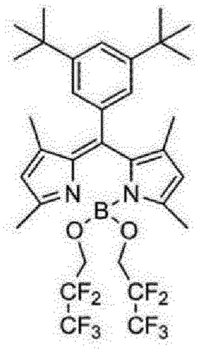

[Formula 158]

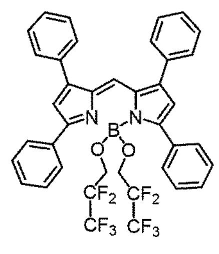

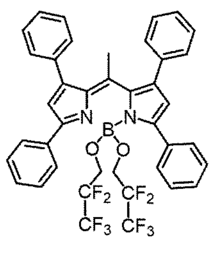

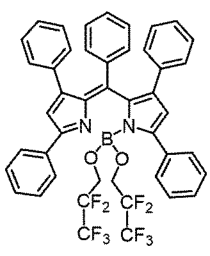

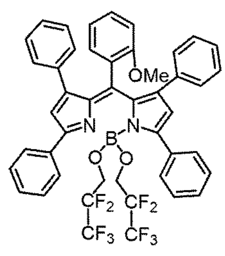

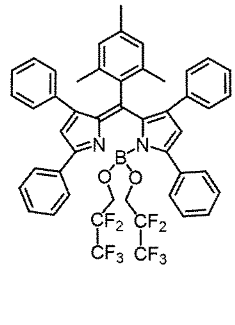

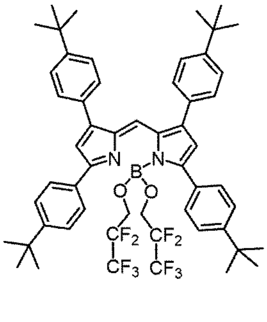

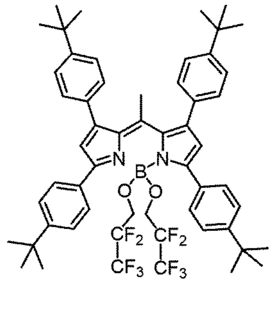

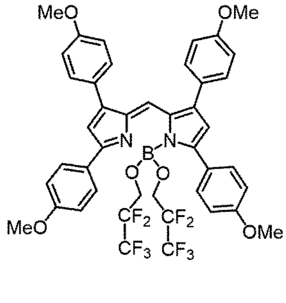

[Formula 159]

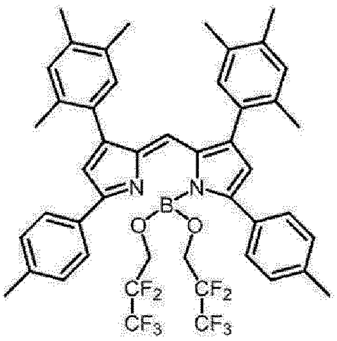
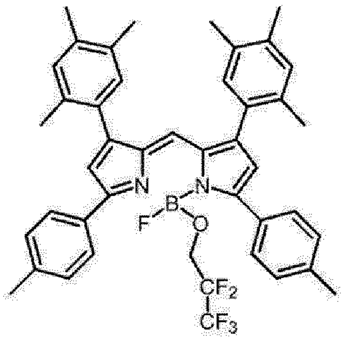
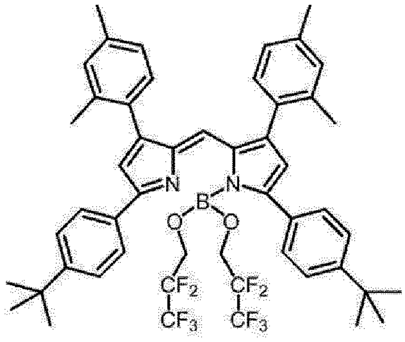

[Formula 160]

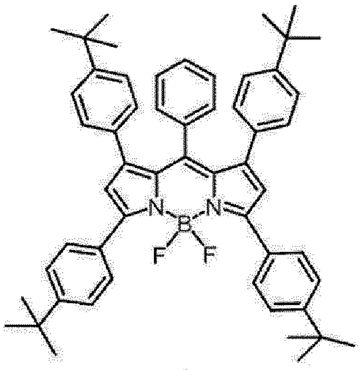
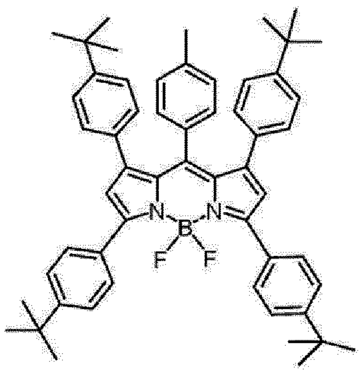
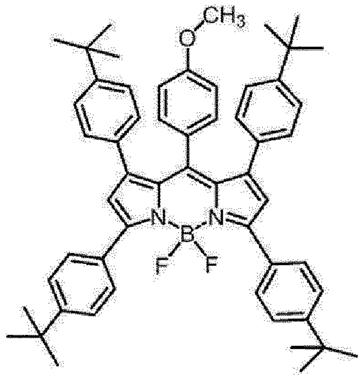
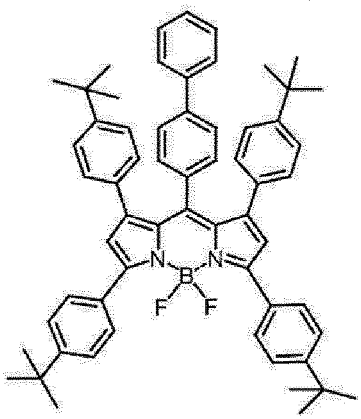
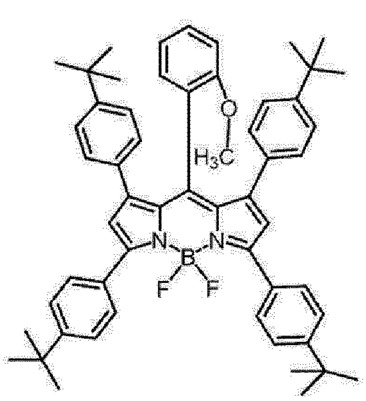

[Formula 161]

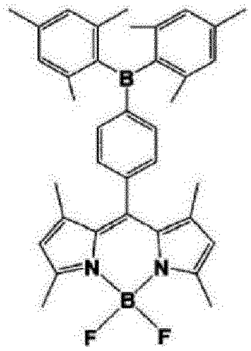
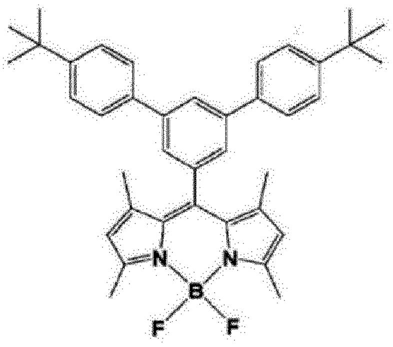
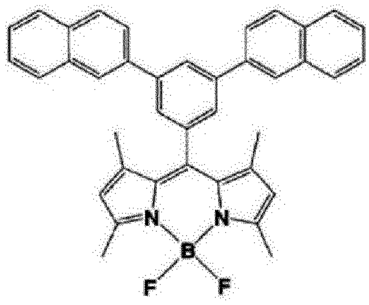

[Formula 162]

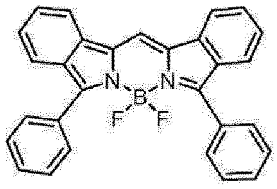
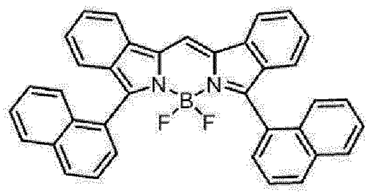
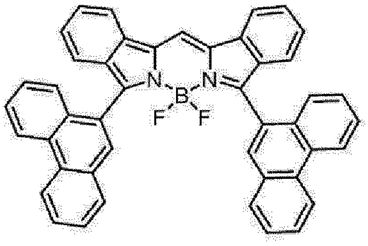
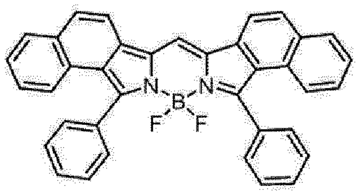
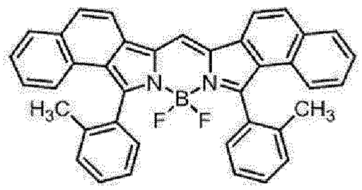
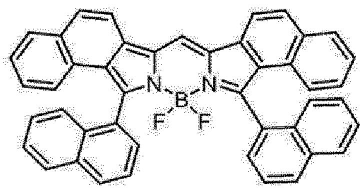

[Formula 163]

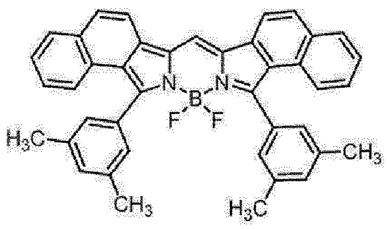
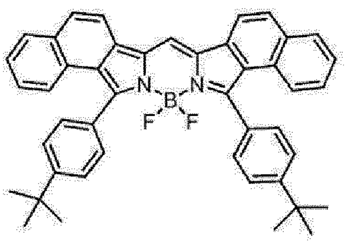
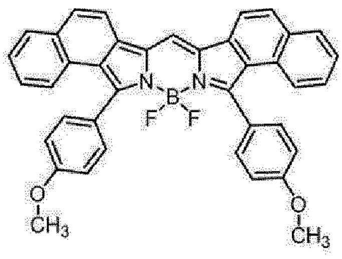
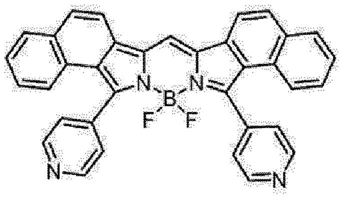
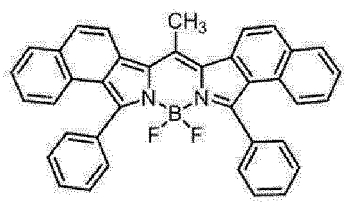
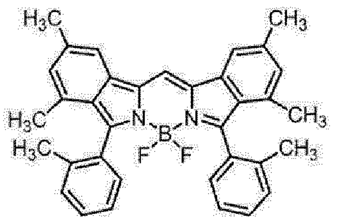

Relationship between Compound M1 and Compound M2 in Emitting Layer

In the organic EL device according to the exemplary embodiment, a lowest singlet energy $S_1$(M2) of the compound M2 and a lowest singlet energy $S_1$(M1) of the compound M1 preferably satisfy a relationship of a numerical

formula (Numerical Formula 1) below.

$$S_1(M2) > S_1(M1) \quad \text{...(Numerical Formula 1)}$$

An energy gap $T_{77K}(M2)$ at 77K of the compound M2 is preferably larger than an energy gap $T_{77K}(M1)$ at 77K of the compound M1. In other words, a relationship of a numerical formula (Numerical Formula 5) below is preferably satisfied.

$$T_{77K}(M2) > T_{77K}(M1) \text{ ...(Numerical Formula 5)}$$

When the organic EL device according to the exemplary embodiment emits light, it is preferable that the compound M1 mainly emits light in the emitting layer.

TADF Mechanism

Fig. 4 illustrates an example of a relationship between energy levels of the compound M2 and the compound M1 in the emitting layer. In Fig. 4, S0 represents a ground state. S1(M1) represents a lowest singlet state of the compound M1. T1(M1) represents a lowest triplet state of the compound M1. S1(M2) represents a lowest singlet state of the compound M2. T1(M2) represents a lowest triplet state of the compound M2.

A dashed arrow directed from S1(M2) to S1(M1) in Fig. 4 represents Förster energy transfer from the lowest singlet state of the compound M2 to the compound M1.

As illustrated in Fig. 4, when a compound having a small ΔST(M2) is used as the compound M2, inverse intersystem crossing from the lowest triplet state T1(M2) to the lowest singlet state S1(M2) can be caused by a heat energy. Subsequently, Förster energy transfer from the lowest singlet state S1(M2) of the compound M2 to the compound M1 occurs to generate the lowest singlet state S1(M1). Consequently, fluorescence from the lowest singlet state S1(M1) of the compound M1 can be observed. It is inferred that the internal quantum efficiency can be theoretically raised up to 100% also by using delayed fluorescence by the TADF mechanism.

The organic EL device according to the exemplary embodiment preferably emits red light or green light.

When the organic EL device according to the exemplary embodiment emits a green light, a main peak wavelength of the light emitted from the organic EL device is preferably in a range from 500 nm to 560 nm.

When the organic EL device according to the exemplary embodiment emits a red light, a main peak wavelength of the light emitted from the organic EL device is preferably in a range from 600 nm to 660 nm.

When the organic EL device according to the exemplary embodiment emits a blue light, a main peak wavelength of the light emitted from the organic EL device is preferably in a range from 430 nm to 480 nm.

The main peak wavelength of the light emitted from the organic EL device is measured as follows.

Voltage is applied on the organic EL device such that a current density becomes 10 $mA/cm^2$, where spectral radiance spectrum is measured by a spectroradiometer CS-2000 (manufactured by Konica Minolta, Inc.).

A peak wavelength of an emission spectrum, at which the luminous intensity of the resultant spectral radiance spectrum is at the maximum, is measured and defined as a main peak wavelength (unit: nm).

Film Thickness of Emitting Layer

A film thickness of the emitting layer of the organic EL device according to the exemplary embodiment is preferably in a range from 5 nm to 50 nm, more preferably in a range from 7 nm to 50 nm, and still more preferably in a range from 10 nm to 50 nm. When the film thickness of the emitting layer is 5 nm or more, the formation of the emitting layer and the adjustment of the chromaticity are likely to be easy. When the film thickness of the emitting layer is 50 nm or less, an increase in the drive voltage is likely to be inhibited.

Content Ratios of Compounds in Emitting Layer

For instance, content ratios of the compound M2 and the compound M1 in the emitting layer preferably fall within ranges shown below.

The content ratio of the compound M2 may be in a range from 90 mass% to 99.9 mass%, may be in a range from 95 mass% to 99.9 mass%, and may be in a range from 99 mass% to 99.9 mass%.

The content ratio of the compound M1 is preferably in a range from 0.01 mass% to 10 mass%, more preferably in a range from 0.01 mass% to 5 mass%, and still more preferably in a range from 0.01 mass% to 1 mass%.

It should be noted that the emitting layer of the exemplary embodiment may contain a material other than the compound M2 and the compound M1.

The emitting layer may contain a single type of the compound M2 or may contain two or more types of the compound M2. The emitting layer may contain a single type of the compound M1 or may contain two or more types of the compound M1.

Substrate

The substrate is used as a support for the organic EL device. For instance, glass, quartz, plastics and the like are usable for the substrate. A flexible substrate is also usable. The flexible substrate is a bendable substrate. Examples of the flexible substrate include a plastic substrate made using polycarbonate, polyarylate, polyethersulfone, polypropylene, polyester, polyvinyl fluoride, and polyvinyl chloride. Further, an inorganic vapor deposition film is also usable.

Anode

Metal, an alloy, an electrically conductive compound, a mixture thereof, or the like having a large work function (specifically, 4.0 eV or more) is preferably used as the anode formed on the substrate. Specific examples of the material include ITO (Indium Tin Oxide), indium oxide-tin oxide containing silicon or silicon oxide, indium oxide-zinc oxide, indium oxide containing tungsten oxide and zinc oxide, and graphene. In addition, gold (Au), platinum (Pt), nickel (Ni), tungsten (W), chrome (Cr), molybdenum (Mo), iron (Fe), cobalt (Co), copper (Cu), palladium (Pd), titanium (Ti), and nitrides of a metal material (e.g., titanium nitride) are usable.

The material is typically formed into a film by a sputtering method. For instance, the indium oxide-zinc oxide can be formed into a film by the sputtering method using a target in which zinc oxide in a range from 1 mass% to 10 mass% is added to indium oxide. Moreover, for instance, the indium oxide containing tungsten oxide and zinc oxide can be formed by the sputtering method using a target in which tungsten oxide in a range from 0.5 mass% to 5 mass% and zinc oxide in a range from 0.1 mass% to 1 mass% are added to indium oxide. In addition, the anode may be formed by a vacuum deposition method, a coating method, an inkjet method, a spin coating method or the like.

Among the organic layers formed on the anode, since the hole injecting layer adjacent to the anode is formed of a composite material into which holes are easily injectable irrespective of the work function of the anode, a material usable as an electrode material (e.g., metal, an alloy, an electroconductive compound, a mixture thereof, and the elements belonging to the group 1 or 2 of the periodic table) is also usable for the anode.

A material having a small work function such as elements belonging to Groups 1 and 2 in the periodic table of the elements, specifically, an alkali metal such as lithium (Li) and cesium (Cs), an alkaline earth metal such as magnesium (Mg), calcium (Ca) and strontium (Sr), alloys (e.g., MgAg and AlLi) including the alkali metal or the alkaline earth metal, a rare earth metal such as europium (Eu) and ytterbium (Yb), and alloys including the rare earth metal are also usable for the anode. It should be noted that the vacuum deposition method and the sputtering method are usable for forming the anode using the alkali metal, alkaline earth metal and the alloy thereof. Further, when a silver paste is used for the anode, the coating method and the inkjet method are usable.

Cathode

It is preferable to use metal, an alloy, an electroconductive compound, a mixture thereof, or the like having a small work function (specifically, 3.8 eV or less) for the cathode. Examples of the material for the cathode include elements belonging to Groups 1 and 2 in the periodic table of the elements, specifically, an alkali metal such as lithium (Li) and cesium (Cs), an alkaline earth metal such as magnesium (Mg), calcium (Ca) and strontium (Sr), alloys (e.g., MgAg and AlLi) including the alkali metal or the alkaline earth metal, a rare earth metal such as europium (Eu) and ytterbium (Yb), and alloys including the rare earth metal.

It should be noted that the vacuum deposition method and the sputtering method are usable for forming the cathode using the alkali metal, alkaline earth metal and the alloy thereof. Further, when a silver paste is used for the cathode, the coating method and the inkjet method are usable.

By providing the electron injecting layer, various conductive materials such as Al, Ag, ITO, graphene, and indium oxide-tin oxide containing silicon or silicon oxide may be used for forming the cathode regardless of the work function. The conductive materials can be formed into a film using the sputtering method, inkjet method, spin coating method and the like.

Hole Injecting Layer

The hole injecting layer is a layer containing a substance exhibiting a high hole injectability. Examples of the

substance exhibiting a high hole injectability include molybdenum oxide, titanium oxide, vanadium oxide, rhenium oxide, ruthenium oxide, chrome oxide, zirconium oxide, hafnium oxide, tantalum oxide, silver oxide, tungsten oxide, and manganese oxide.

In addition, the examples of the highly hole-injectable substance include: an aromatic amine compound, which is a low-molecule organic compound, such that 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), 4,4'-bis[N-(4-diphenylaminophenyl)-N-phenylamino]biphenyl (abbreviation: DPAB), 4,4'-bis(N-{4-[N'-(3-methylphenyl)-N'-phenylamino]phenyl} -N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylamino]-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCN1).

In addition, a high polymer compound (e.g., oligomer, dendrimer and polymer) is usable as the substance exhibiting a high hole injectability. Examples of the high-molecule compound include poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N'-phenylaminolphenyl)methacrylamide] (abbreviation: PTPDMA), and poly[N,N'-bis(4-butylphenyl)-N,N'-bis(phenyl)benzidine] (abbreviation: Poly-TPD). Moreover, an acid-added high polymer compound such as poly(3,4-ethylenedioxythiophene)/poly(styrene sulfonic acid) (PEDOT/PSS) and polyaniline/poly(styrene sulfonic acid) (PAni/PSS) are also usable.

Hole Transporting Layer

The hole transporting layer is a layer containing a highly hole-transporting substance. An aromatic amine compound, carbazole derivative, anthracene derivative and the like are usable for the hole transporting layer. Specific examples of a material for the hole transporting layer include 4,4'-bis[N-(1-naphthyl)-N-phenylamino]biphenyl (abbreviation: NPB), N,N'-bis(3-methylphenyl)-N,N'-diphenyl-[1,1'-biphenyl]-4,4'-diamine (abbreviation: TPD), 4-phenyl-4'-(9-phenylfluorene-9-yl)triphenylamine (abbreviation: BAFLP), 4,4'-bis[N-(9,9-dimethylfluorene-2-yl)-N-phenylamino]biphenyl (abbreviation: DFLDPBi), 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), and 4,4'-bis[N-(spiro-9,9'-bifluorene-2-yl)-N-phenylamino]biphenyl (abbreviation: BSPB). The above-described substances mostly have a hole mobility of $10^{-6}cm^2/Vs$ or more.

For the hole transporting layer, a carbazole derivative such as CBP, CzPA, and PCzPA and an anthracene derivative such as t-BuDNA, DNA, and DPAnth may be used. A high polymer compound such as poly(N-vinylcarbazole) (abbreviation: PVK) and poly(4-vinyltriphenylamine) (abbreviation: PVTPA) is also usable.

However, in addition to the above substances, any substance exhibiting a higher hole transportability than an electron transportability may be used. It should be noted that the layer containing the substance exhibiting a high hole transportability may be not only a single layer but also a laminate of two or more layers formed of the above substance(s).

Electron Transporting Layer

The electron transporting layer is a layer containing a highly electron-transporting substance. For the electron transporting layer, 1) a metal complex such as an aluminum complex, beryllium complex, and zinc complex, 2) a hetero aromatic compound such as imidazole derivative, benzimidazole derivative, azine derivative, carbazole derivative, and phenanthroline derivative, and 3) a high polymer compound are usable. Specifically, as a low-molecule organic compound, for instance, a metal complex such as Alq, tris(4-methyl-8-quinolinato)aluminum (abbreviation: $Almq_3$), bis(10-hydroxybenzo[h]quinolinato)beryllium (abbreviation: $BeBq_2$), BAlq, Znq, ZnPBO and ZnBTZ is usable. In addition to the metal complex, a heteroaromatic compound such as 2-(4-biphenylyl)-5-(4-tert-butylphenyl)-1,3,4-oxadiazole (abbreviation: PBD), 1,3-bis[5-(ptert-butylphenyl)-1,3,4-oxadiazole-2-yl]benzene (abbreviation: OXD-7), 3-(4-tert-butylphenyl)-4-phenyl-5-(4-biphenylyl)-1,2,4-triazole (abbreviation: TAZ), 3-(4-tert-butylphenyl)-4-(4-ethylphenyl)-5-(4-biphenylyl)-1,2,4-triazole (abbreviation: p-EtTAZ), bathophenanthroline (abbreviation: BPhen), bathocuproine (abbreviation: BCP), and 4,4'-bis(5-methylbenzoxazole-2-yl)stilbene (abbreviation: BzOs) is usable. The above-described substances mostly have an electron mobility of $10^{-6}$ $cm^2/(V•s)$ or more. It should be noted that any other substance than the above substance may be used for the electron transporting layer as long as exhibiting a higher electron transportability than the hole transportability. It should be noted that the electron transporting layer may be not only a single layer but also a laminate of two or more layers formed of the above substance(s).

Further, a high polymer compound is usable for the electron transporting layer. For instance, poly[(9,9-dihexylfluorene-2,7-diyl)-co-(pyridine-3,5-diyl)] (abbreviation: PF-Py), and poly[(9,9-dioctylfluorene-2,7-diyl)-co-(2,2'-bipyridine-6,6'-diyl)] (abbreviation: PF-BPy) are usable.

## Electron Injecting Layer

The electron injecting layer is a layer containing a highly electron-injectable substance. Examples of a material for the electron injecting layer include an alkali metal, alkaline earth metal and a compound thereof, examples of which include lithium (Li), cesium (Cs), calcium (Ca), lithium fluoride (LiF), cesium fluoride (CsF), calcium fluoride ($CaF_2$), and lithium oxide (LiOx). In addition, the alkali metal, alkaline earth metal or the compound thereof may be added to the substance exhibiting the electron transportability in use. Specifically, for instance, magnesium (Mg) added to Alq may be used. In this case, the electrons can be more efficiently injected from the cathode.

Alternatively, the electron injecting layer may be provided by a composite material in a form of a mixture of the organic compound and the electron donor. Such a composite material exhibits excellent electron injectability and electron transportability since electrons are generated in the organic compound by the electron donor. In this case, the organic compound is preferably a material excellent in transporting the generated electrons. Specifically, the above examples (e.g., the metal complex and the hetero aromatic compound) of the substance forming the electron transporting layer are usable. As the electron donor, any substance exhibiting electron donating property to the organic compound is usable. Specifically, the electron donor is preferably alkali metal, alkaline earth metal and rare earth metal such as lithium, cesium, magnesium, calcium, erbium and ytterbium. The electron donor is also preferably alkali metal oxide and alkaline earth metal oxide such as lithium oxide, calcium oxide, and barium oxide. Moreover, a Lewis base such as magnesium oxide is usable. Further, the organic compound such as tetrathiafulvalene (abbreviation: TTF) is usable.

## Layer Formation Method

A method for forming each layer of the organic EL device in the exemplary embodiment is subject to no limitation except for the above particular description. However, known methods of dry film-forming such as vacuum deposition, sputtering, plasma or ion plating and wet film-forming such as spin coating, dipping, flow coating or ink-jet are applicable.

## Film Thickness

A thickness of each of the organic layers in the organic EL device according to the exemplary embodiment is not limited except for the above particular description. In general, the thickness preferably ranges from several nanometers to 1 μm because excessively small film thickness is likely to cause defects (e.g. pin holes) and excessively large thickness leads to the necessity of applying high voltage and consequent reduction in efficiency.

The organic EL device according to the third exemplary embodiment contains, in the emitting layer, the compound of the first exemplary embodiment as the compound M2 and the compound M1 having the lowest singlet energy smaller than that of the compound M2. Since the organic EL device according to the third exemplary embodiment contains the compound having a high PLQY according to the first exemplary embodiment, the third exemplary embodiment provides a high-performance organic EL device that achieves at least one of high efficiency or a long lifetime.

## Fourth Exemplary Embodiment

An arrangement of an organic EL device according to a fourth exemplary embodiment will be described below. In the description of the fourth exemplary embodiment, the same components as those in the third exemplary embodiment are denoted by the same reference signs and names to simplify or omit explanation of the components. In the fourth exemplary embodiment, the same materials and compounds as described in the third exemplary embodiment are usable, unless otherwise specified.

The organic EL device according to the fourth exemplary embodiment is different from the organic EL device according to the third exemplary embodiment in that the emitting layer further includes a compound M3. The fourth exemplary embodiment is the same as the third exemplary embodiment in other respects.

Specifically, in the fourth exemplary embodiment, the emitting layer contains the compound M3, the compound M2, and the compound M1. In this arrangement, it is preferable that the compound M2 is a host material and the compound M1 is a dopant material.

## Compound M3

The compound M3 of the exemplary embodiment may be a thermally activated delayed fluorescent compound or a compound exhibiting no thermally activated delayed fluorescence. However, the compound M3 is preferably a compound exhibiting no thermally activated delayed fluorescence.

The compound M3, which is not particularly limited, is preferably a compound other than an amine compound. As the compound M3, for instance, a carbazole derivative, dibenzofuran derivative, and a dibenzothiophen derivative

are usable. However, the compound M3 is not limited to these derivatives.

The compound M3 of the exemplary embodiment is preferably a compound represented by a formula (3X) or (3Y) below.

Compound Represented by Formula (3X)

The compound M3 is also preferably a compound represented by a formula (3X) below.

[Formula 164]

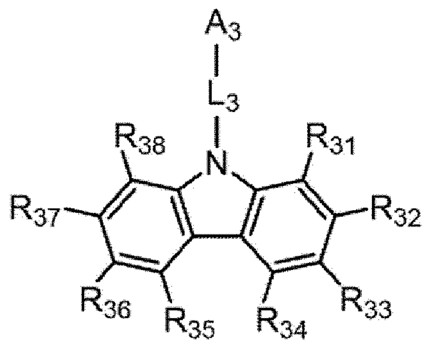

(3X)

In the formula (3X):

$A_3$ is a substituted or unsubstituted aryl group having 6 to 50 ring carbon atoms, or a substituted or unsubstituted heterocyclic group having 5 to 50 ring atoms;

$L_3$ is a single bond, a substituted or unsubstituted arylene group having 6 to 50 ring carbon atoms, a substituted or unsubstituted divalent heterocyclic group having 5 to 50 ring atoms, or a divalent group formed by bonding two groups selected from the group consisting of a substituted or unsubstituted arylene group having 6 to 50 ring carbon atoms and a substituted or unsubstituted divalent heterocyclic group having 5 to 50 ring atoms, or a divalent group formed by bonding three groups selected from the group consisting of a substituted or unsubstituted arylene group having 6 to 30 ring carbon atoms and a substituted or unsubstituted divalent heterocyclic group having 5 to 30 ring atoms;

at least one combination of adjacent two or more of $R_{31}$ to $R_{38}$ are mutually bonded to form a substituted or unsubstituted monocyclic ring, mutually bonded to form a substituted or unsubstituted fused ring, or not mutually bonded;

$R_{31}$ to $R_{38}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{906}$, a group represented by $-COOR_{909}$, a halogen atom, a cyano group, a nitro group, a group represented by $-P(=O)(R_{931})(R_{932})$, a group represented by $-Ge(R_{933})(R_{934})(R_{935})$, a group represented by $-B(R_{936})(R_{937})$, a substituted or unsubstituted aryl group having 6 to 50 ring carbon atoms, a substituted or unsubstituted heterocyclic group having 5 to 50 ring atoms, or a group represented by a formula (3A) below.

[Formula 165]

$$*-L_{31}-\left(L_{32}-R_B\right)_{n3} \qquad (3A)$$

In the formula (3A):

$R_B$ is a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a

substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{906}$, a group represented by $-COOR_{909}$, a halogen atom, a cyano group, a nitro group, a group represented by $-P(=O)(R_{931})(R_{932})$, a group represented by $-Ge(R_{933})(R_{934})(R_{935})$, a group represented by $-B(R_{936})(R_{937})$, a substituted or unsubstituted aryl group having 6 to 50 ring carbon atoms, or a substituted or unsubstituted heterocyclic group having 5 to 50 ring atoms;

when a plurality of $R_B$ are present, the plurality of $R_B$ are mutually the same or different;

$L_{31}$ is a single bond, a substituted or unsubstituted arylene group having 6 to 50 ring carbon atoms, a trivalent group, tetravalent group, pentavlent group, or hexavalent group derived from the arylene group, a substituted or unsubstituted divalent heterocyclic group having 5 to 50 ring atoms, a trivalent group, tetravalent group, pentavlent group, or hexavalent group derived from the heterocyclic group, a divalent group formed by bonding two groups selected from the group consisting of a substituted or unsubstituted arylene group having 6 to 50 ring carbon atoms and a substituted or unsubstituted divalent heterocyclic group having 5 to 50 ring atoms, or a trivalent group, tetravalent group, pentavlent group, or hexavalent group derived from the divalent group;

$L_{32}$ is a single bond, a substituted or unsubstituted arylene group having 6 to 50 ring carbon atoms, or a substituted or unsubstituted divalent heterocyclic group having 5 to 50 ring atoms;

ns is 1, 2, 3, 4, or 5;

when $L_{31}$ is a single bond, ns is 1 and $L_{32}$ is bonded to a carbon atom in a six-membered ring in the formula (3X);

when a plurality of $L_{32}$ are present, the plurality of $L_{32}$ are mutually the same or different; and

* represents a bonding position to a carbon atom in a six-membered ring in the formula (3X).

In the compound M3, $R_{901}$, $R_{902}$, $R_{903}$, $R_{904}$, $R_{905}$, $R_{906}$, $R_{907}$, $R_{908}$, $R_{909}$, $R_{931}$, $R_{932}$, $R_{933}$, $R_{934}$, $R_{935}$, $R_{936}$, and $R_{937}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{906}$ are present, the plurality of $R_{908}$ are mutually the same or different;
when a plurality of $R_{909}$ are present, the plurality of $R_{909}$ are mutually the same or different;
when a plurality of $R_{931}$ are present, the plurality of $R_{931}$ are mutually the same or different;
when a plurality of $R_{932}$ are present, the plurality of $R_{932}$ are mutually the same or different;
when a plurality of $R_{933}$ are present, the plurality of $R_{933}$ are mutually the same or different;
when a plurality of $R_{934}$ are present, the plurality of $R_{934}$ are mutually the same or different;
when a plurality of $R_{935}$ are present, the plurality of $R_{935}$ are mutually the same or different;
when a plurality of $R_{936}$ are present, the plurality of $R_{936}$ are mutually the same or different; and
when a plurality of $R_{937}$ are present, the plurality of $R_{937}$ are mutually the same or different.

The compound M3 is also preferably a compound represented by one of formulae (31) to (36) below.

[Formula 166]

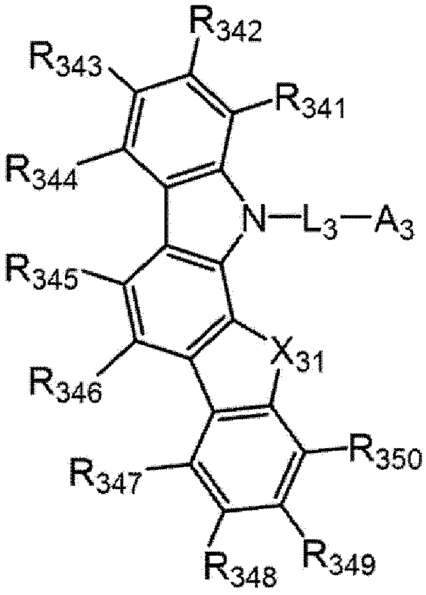

(31)

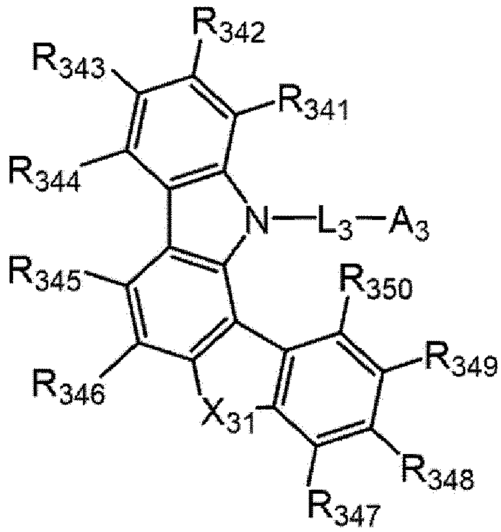

(32)

[Formula 167]

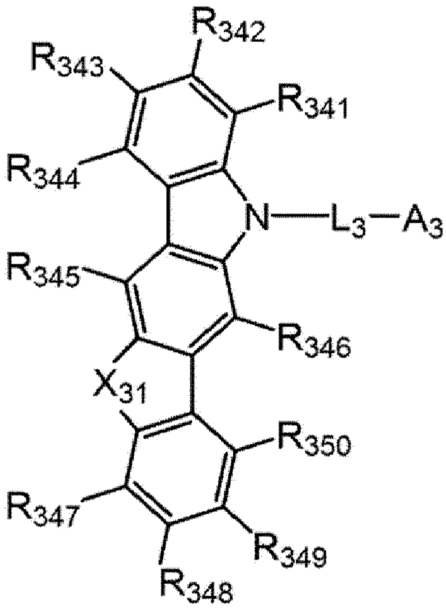

(33)

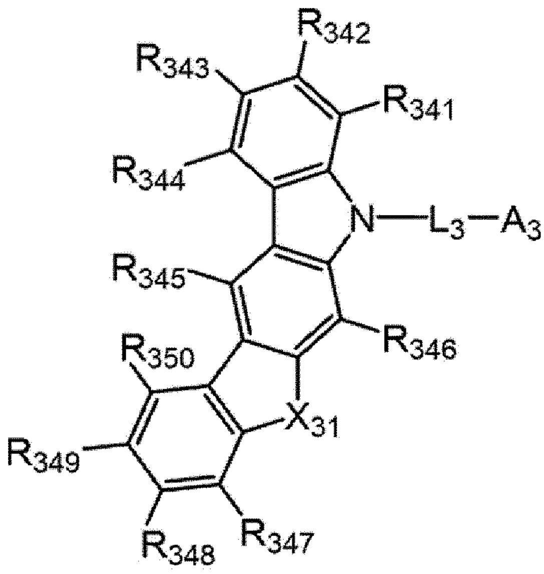

(34)

[Formula 168]

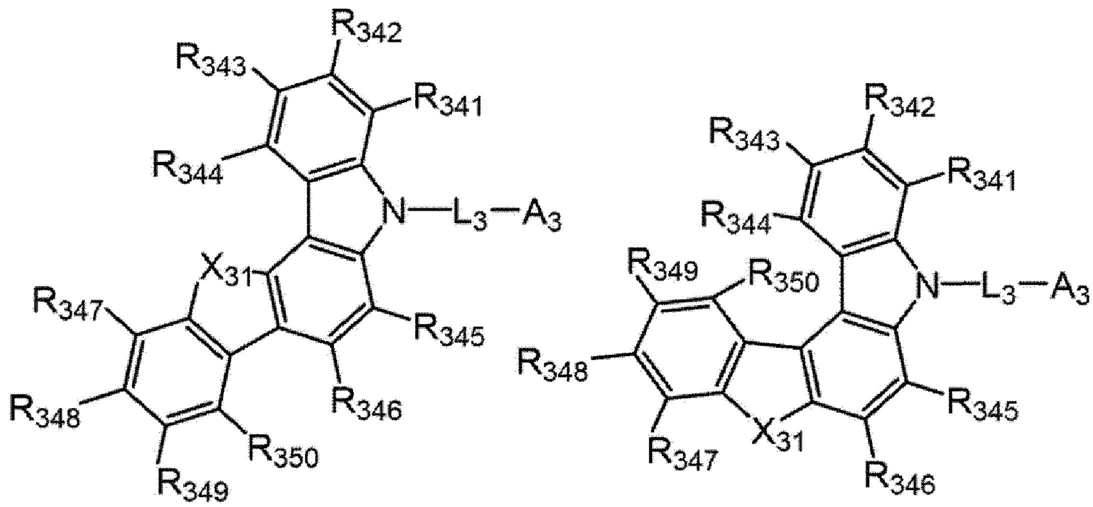

(35) (36)

In the formulae (31) to (36):

$A_3$ and $L_3$ respectively represent the same as $A_3$ and $L_3$ in the formula (3X);
at least one combination of adjacent two or more of $R_{341}$ to $R_{350}$ are mutually bonded to form a substituted or unsubstituted monocyclic ring, mutually bonded to form a substituted or unsubstituted fused ring, or not mutually bonded;
$X_{31}$ is a sulfur atom, an oxygen atom, $NR_{352}$, or $CR_{353}R_{354}$;
a combination of $R_{353}$ and $R_{354}$ are mutually bonded to form a substituted or unsubstituted monocyclic ring, mutually bonded to form a substituted or unsubstituted fused ring, or not mutually bonded;
$R_{341}$ to $R_{350}$ forming neither the substituted or unsubstituted monocyclic ring nor the substituted or unsubstituted fused ring; $R_{352}$; and $R_{353}$ and $R_{354}$ forming neither the substituted or unsubstituted monocyclic ring nor the substituted or unsubstituted fused ring each independently represent the same as $R_{31}$ to $R_{38}$ forming neither the substituted or unsubstituted monocyclic ring nor the substituted or unsubstituted fused ring.

In the compound M3, $R_{352}$ is preferably a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

In the compound M3, it is preferable that a combination of $R_{353}$ and $R_{354}$ are mutually bonded to form a substituted or unsubstituted monocyclic ring, mutually bonded to form a substituted or unsubstituted fused ring, or not mutually bonded;
$R_{353}$ and $R_{354}$ forming neither the substituted or unsubstituted monocyclic ring nor the substituted or unsubstituted fused ring are each independently a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

In the compound M3, $X_{31}$ is preferably a sulfur atom or an oxygen atom.

In the compound M3, $A_3$ is preferably a group represented by one of formulae (A31) to (A37) below.

[Formula 169]

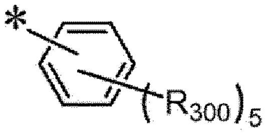
(A31)

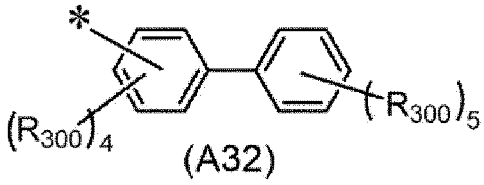
(A32)

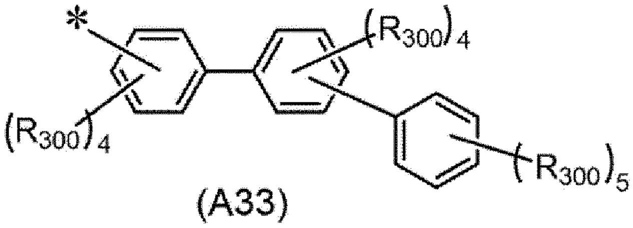
(A33)

[Formula 170]

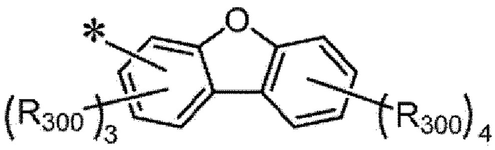
(A34)

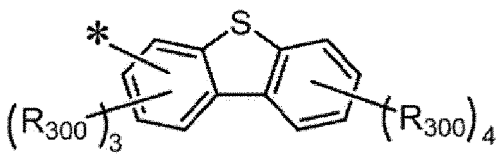
(A35)

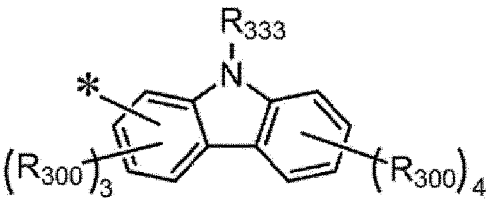
(A36)

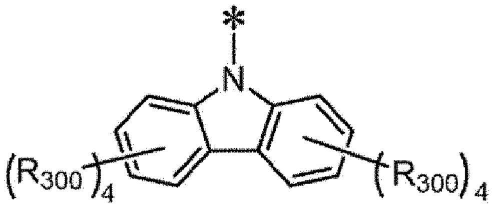
(A37)

In the formulae (A31) to (A37):

at least one combination of adjacent two or more of a plurality of $R_{300}$ are mutually bonded to form a substituted or unsubstituted monocyclic ring, mutually bonded to form a substituted or unsubstituted fused ring, or not mutually bonded;
$R_{300}$ forming neither the substituted or unsubstituted monocyclic ring nor the substituted or unsubstituted fused ring, and $R_{333}$ each independently represent the same as $R_{31}$ to $R_{38}$ forming neither the substituted or unsubstituted monocyclic ring nor the substituted or unsubstituted fused ring; and
* in the formulae (A31) to (A37) each represents a bonding position to $L_3$ in the compound M3.

In the compound M3, $A_3$ is also preferably a group represented by the formula (A34), (A35), or (A37).
The compound M3 is also preferably a compound represented by one of formulae (311) to (316) below.

[Formula 171]

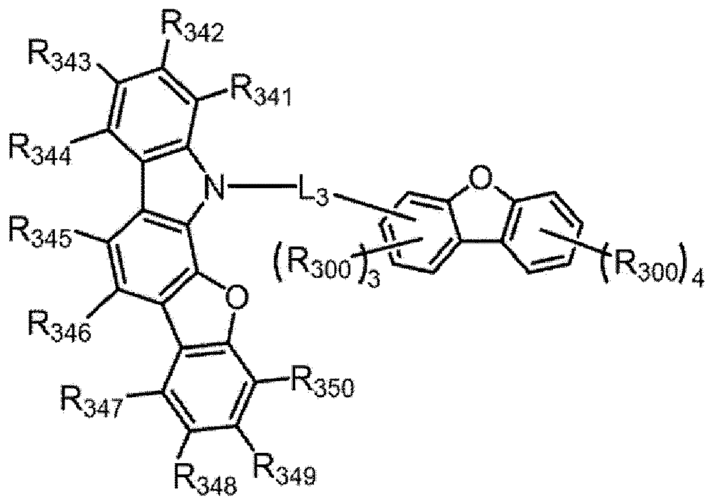

(311)

[Formula 172]

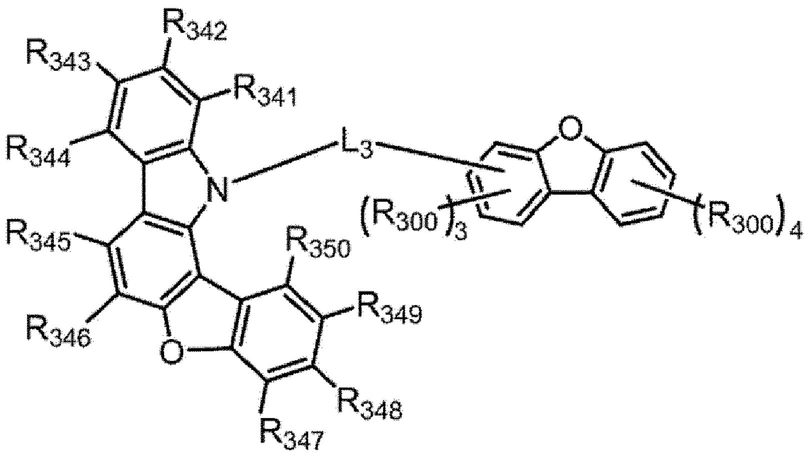

(312)

[Formula 173]

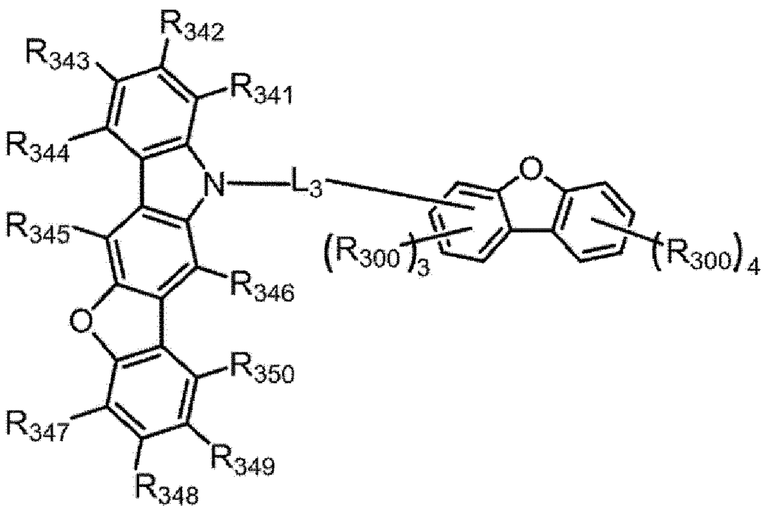

(313)

[Formula 174]

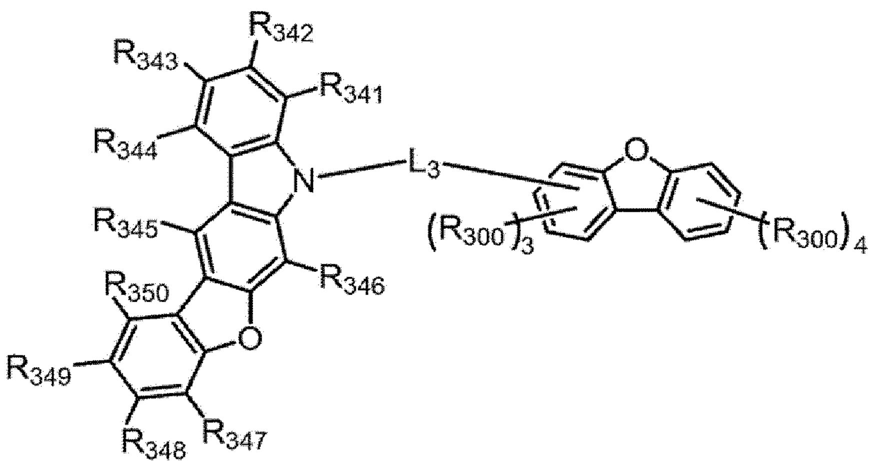

(314)

[Formula 175]

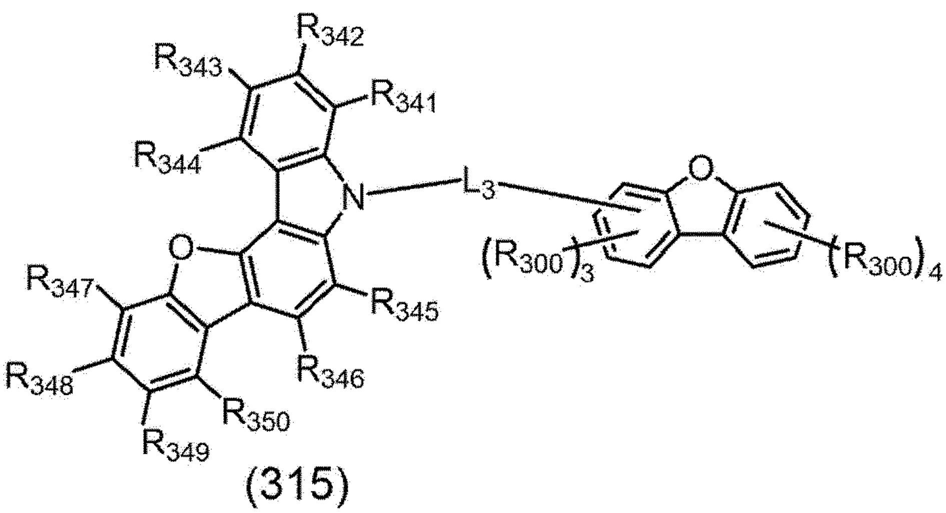

(315)

[Formula 176]

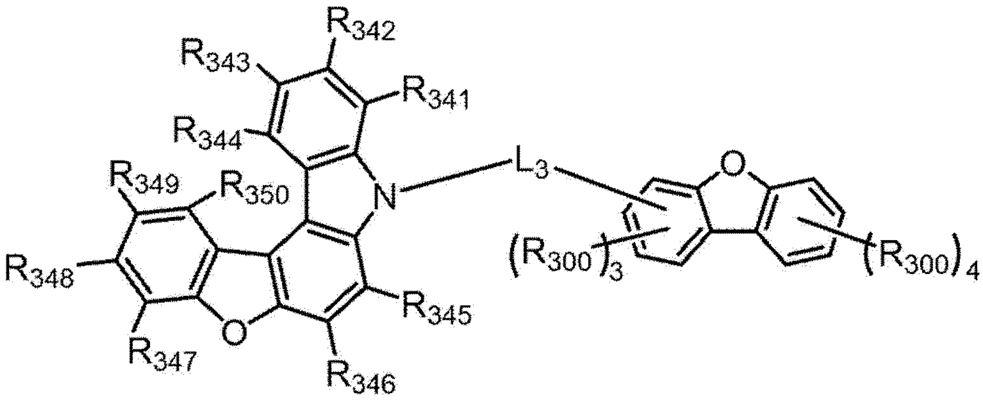

(316)

In the formulae (311) to (316):

$L_3$ represents the same as $L_3$ in the formula (3X);
at least one combination of adjacent two or more of a plurality of $R_{300}$ are mutually bonded to form a substituted or unsubstituted monocyclic ring, mutually bonded to form a substituted or unsubstituted fused ring, or not mutually bonded;
at least one combination of adjacent two or more of $R_{341}$ to $R_{350}$ are mutually bonded to form a substituted or unsubstituted monocyclic ring, mutually bonded to form a substituted or unsubstituted fused ring, or not mutually bonded; and
$R_{300}$ forming neither the substituted or unsubstituted monocyclic ring nor the substituted or unsubstituted fused ring, and $R_{341}$ to $R_{350}$ forming neither the substituted or unsubstituted monocyclic ring nor the substituted or unsubstituted fused ring each independently represent the same as $R_{31}$ to $R_{38}$ forming neither the substituted or unsubstituted monocyclic ring nor the substituted or unsubstituted fused ring.

The compound M3 is also preferably a compound represented by a formula (321) below.

[Formula 177]

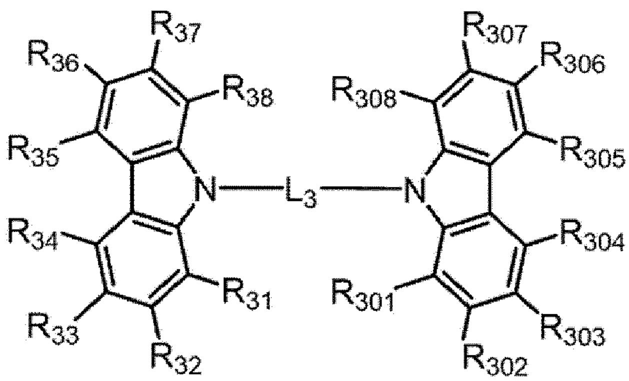

(321)

In the formula (321):

$L_3$ represents the same as $L_3$ in the formula (3X); and
$R_{31}$ to $R_{38}$ and $R_{301}$ to $R_{308}$ each independently represent the same as $R_{31}$ to $R_{38}$ forming neither the substituted or unsubstituted monocyclic ring nor the substituted or unsubstituted fused ring.

In the compound M3, $L_3$ is preferably a single bond or a substituted or unsubstituted arylene group having 6 to 50 ring carbon atoms.
In the compound M3, $L_3$ is preferably a single bond, a substituted or unsubstituted phenylene group, a substituted or unsubstituted biphenylene group, or a substituted or unsubstituted terphenylene group.
In the compound M3, $L_3$ is preferably a group represented by a formula (317) below.

[Formula 178]

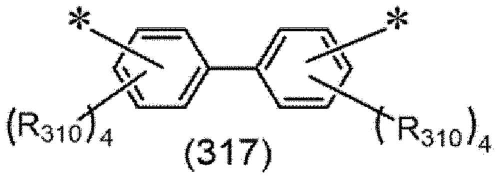

(317)

In the formula (317): $R_{310}$ each independently represents the same as $R_{31}$ to $R_{38}$ forming neither the substituted or unsubstituted monocyclic ring nor the substituted or unsubstituted fused ring, and each * independently represents a bonding position.
In the compound M3, $L_3$ also preferably contains a divalent group represented by a formula (318) or (319) below.
In the compound M3, $L_3$ is also preferably a divalent group represented by the formula (318) or (319) below.
The compound M3 is also preferably a compound represented by a formula (322) or (323) below.

[Formula 179]

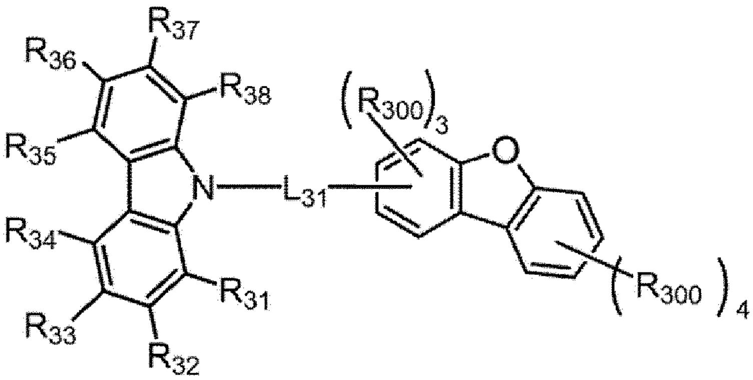

(322)

[Formula 180]

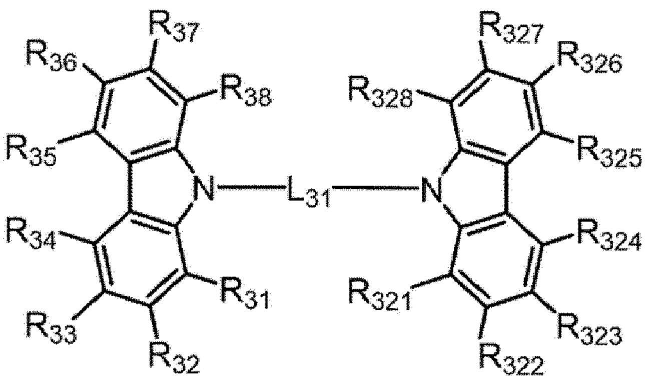

(323)

In the formulae (322) and (323):

$L_{31}$ is a substituted or unsubstituted arylene group having 6 to 50 ring carbon atoms, a substituted or unsubstituted divalent heterocyclic group having 5 to 50 ring atoms, or a divalent group formed by bonding two groups selected from the group consisting of a substituted or unsubstituted arylene group having 6 to 50 ring carbon atoms and a substituted or unsubstituted divalent heterocyclic group having 5 to 50 ring atoms;
$L_{31}$ contains a divalent group represented by the formula (318) or (319) below; and
$R_{31}$ to $R_{38}$, $R_{300}$, and $R_{321}$ to $R_{328}$ each independently represent the same as $R_{31}$ to $R_{38}$ forming neither the substituted or unsubstituted monocyclic ring nor the substituted or unsubstituted fused ring.

[Formula 181]

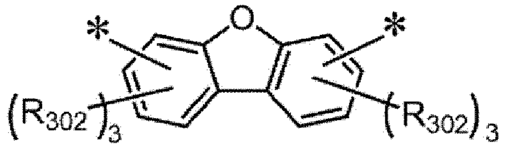

(318)

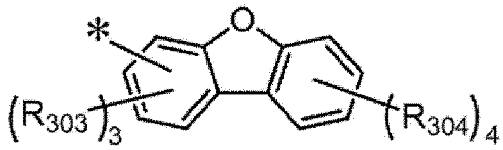

(319)

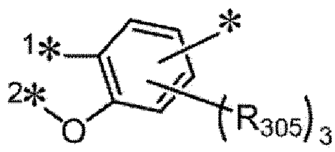

(320)

In the formula (319):

a combination of adjacent two of a plurality of $R_{304}$ are mutually bonded to form a ring represented by the formula (320).
In the formulae (320), 1* and 2* each independently represent a bonding position to a ring bonded to $R_{304}$.
$R_{302}$ in the formula (318), $R_{303}$ in the formula (319), $R_{304}$ not forming a ring represented by the formula (320), and $R_{305}$ in the formula (320) each independently represent the same as $R_{31}$ to $R_{38}$ forming neither the substituted or unsubstituted monocyclic ring nor the substituted or unsubstituted fused ring.
* in the formulae (318) to (320) each represents a bonding position.

In the compound M3, a group represented by the formula (319) for $L_3$ or $L_{31}$ is, for instance, a group represented by a formula (319A) below.

[Formula 182]

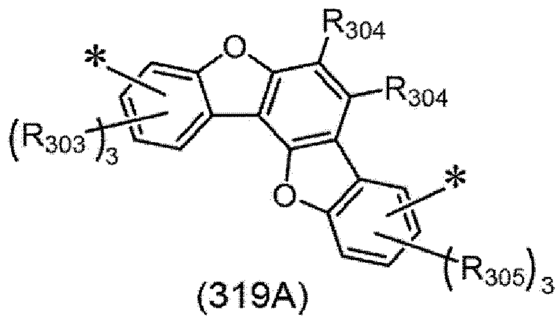

(319A)

In the formula (319A), $R_{303}$, $R_{304}$, and $R_{305}$ each independently represent the same as $R_{31}$ to $R_{38}$ forming neither the substituted or unsubstituted monocyclic ring nor the substituted or unsubstituted fused ring, and each * in the formula (319A) represents a bonding position.
It is also preferable that the compound M3 is a compound represented by the formula (322) and $L_{31}$ is a group represented by the formula (318).
The compound M3 is also preferably a compound represented by a formula (324) below.

[Formula 183]

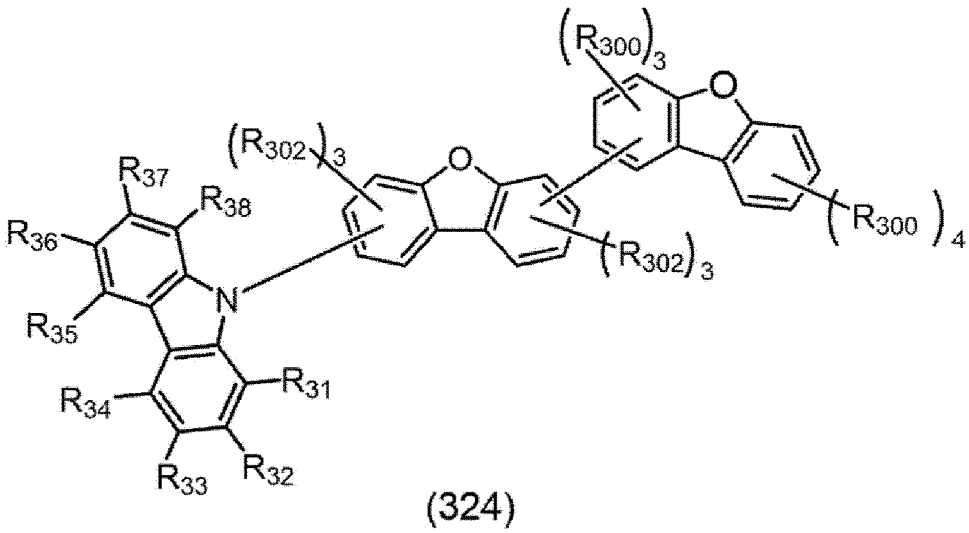

(324)

In the formula (324), $R_{31}$ to $R_{38}$, $R_{300}$, and $R_{302}$ each independently represent the same as $R_{31}$ to $R_{38}$ forming neither the substituted or unsubstituted monocyclic ring nor the substituted or unsubstituted fused ring.

It is preferable that $R_{31}$ to $R_{38}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted aryl group having 6 to 50 ring carbon atoms, a substituted or unsubstituted heterocyclic group having 5 to 50 ring atoms, or a group represented by the formula (3A), and

$R_B$ in the formula (3A) is a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

It is preferable that $R_{31}$ to $R_{38}$ forming neither the substituted or unsubstituted monocyclic ring nor the substituted or unsubstituted fused ring are each independently a hydrogen atom, a substituted or unsubstituted aryl group having 6 to 50 ring carbon atoms, or a group represented by the formula (3A), and

$R_B$ in the formula (3A) is a substituted or unsubstituted aryl group having 6 to 50 ring carbon atoms.

It is preferable that $R_{31}$ to $R_{38}$ forming neither the substituted or unsubstituted monocyclic ring nor the substituted or unsubstituted fused ring are each independently a hydrogen atom, a substituted or unsubstituted phenyl group, or a group represented by the formula (3A), and

$R_B$ in the formula (3A) is a substituted or unsubstituted phenyl group.

The compound M3 is also preferably a compound not having a pyridine ring, a pyrimidine ring, and a triazine ring.

Compound Represented by Formula (3Y)

The compound M3 is also preferably a compound represented by a formula (3Y) below.

[Formula 184]

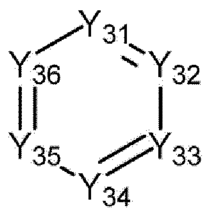

(3Y)

In the formula (3Y):

$Y_{31}$ to $Y_{36}$ are each independently $CR_3$ or a nitrogen atom;

two or more of $Y_{31}$ to $Y_{36}$ are each a nitrogen atom;

when a plurality of $R_3$ are present, at least one combination of adjacent two or more of the plurality of $R_3$ are mutually bonded to form a substituted or unsubstituted monocyclic ring, mutually bonded to form a substituted or unsubstituted fused ring, or not mutually bonded; and

$R_3$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by - $S-(R_{905})$, a group represented by $-N(R_{906})(R_{907})$, a substituted or unsubstituted aralkyl group having 7 to 50 carbon atoms, a group represented by $-C(=O)R_{908}$, a group represented by $-COOR_{909}$, a halogen atom, a cyano group, a nitro group, a group represented by $-P(=O)(R_{931})(R_{932})$, a group represented by $-Ge(R_{933})(R_{934})(R_{935})$, a group represented by $-B(R_{936})(R_{937})$, a substituted or unsubstituted aryl group having 6 to 50 ring carbon atoms, a substituted or unsubstituted heterocyclic group having 5 to 50 ring atoms, or a group represented by a formula (3B) below.

[Formula 185]

$$*-L_{31}-\left(L_{32}-R_B\right)_{n3} \qquad (3B)$$

In the formula (3B): $R_B$, $L_{31}$, $L_{32}$, and ns each independently represent the same as $R_B$, $L_{31}$, $L_{32}$, and $n_3$ in the formula (3A),

when a plurality of $R_B$ are present, the plurality of $R_B$ are mutually the same or different;

when $L_{31}$ is a single bond, ns is 1 and $L_{32}$ is bonded to a carbon atom in a six-membered ring in the formula (3Y);

when a plurality of $L_{32}$ are present, the plurality of $L_{32}$ are mutually the same or different; and

* represents a bonding position to a carbon atom in a six-membered ring in the formula (3Y).

The compound M3 preferably does not include a pyridine ring in a molecule.

The compound M3 is also preferably a compound represented by a formula (31a) or (32a) below.

[Formula 186]

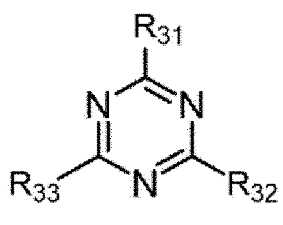

(31a)

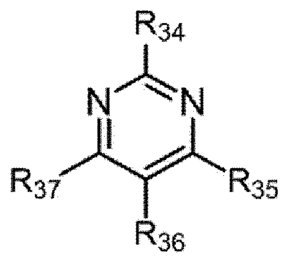

(32a)

In the formula (32a):

at least one combination of adjacent two or more of $R_{35}$ to $R_{37}$ are mutually bonded to form a substituted or unsubstituted monocyclic ring, mutually bonded to form a substituted or unsubstituted fused ring, or not mutually bonded; and

$R_{31}$ to $R_{33}$ in the formula (31a), $R_{34}$ in the formula (32a), and $R_{35}$ to $R_{37}$ forming neither the substituted or unsubstituted monocyclic ring nor the substituted or unsubstituted fused ring each independently represent the same as $R_3$ in the formula (3Y).

The compound M3 is also preferably a compound represented by the formula (31a).

$R_3$ in the formula (3Y) is preferably each independently a hydrogen atom, a substituted or unsubstituted alkyl group having 1 to 50 carbon atoms, a substituted or unsubstituted aryl group having 6 to 50 ring carbon atoms, a substituted or unsubstituted heterocyclic group having 5 to 50 ring atoms, or a group represented by the formula (3B).

$R_3$ in the formula (3Y) is preferably each independently a hydrogen atom, a substituted or unsubstituted alkyl group having 1 to 50 carbon atoms, a substituted or unsubstituted aryl group having 6 to 50 ring carbon atoms, or a group represented by the formula (3B).

The compound M3 represented by the formula (3Y) preferably has at least one group selected from the group consisting of groups represented by formulae (B31) to (B44) below in a molecule.

[Formula 187]

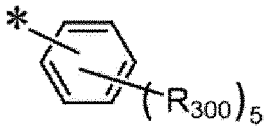

(B31)

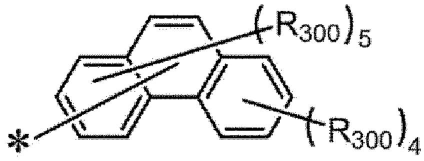

(B32)

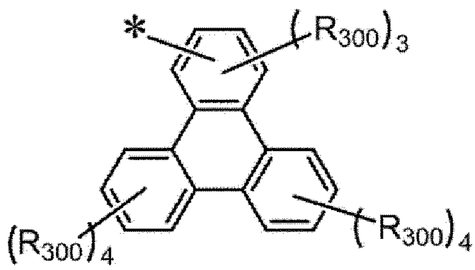

(B33)

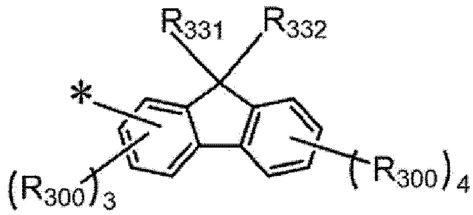

(B34)

[Formula 188]

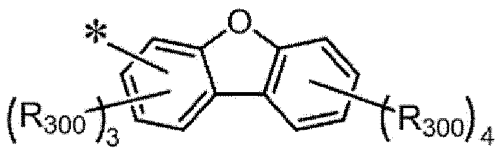

(B35)

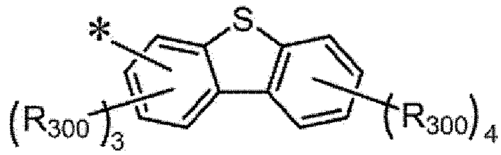

(B36)

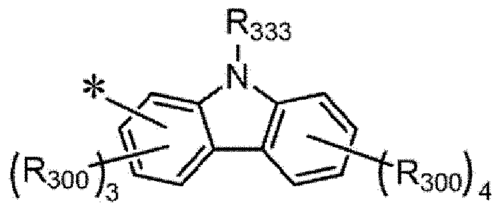

(B37)

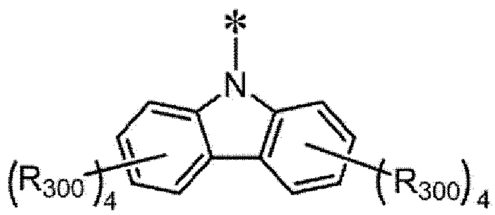

(B38)

In the formulae (B31) to (B38):

at least one combination of adjacent two or more of a plurality of $R_{300}$ are mutually bonded to form a substituted or unsubstituted monocyclic ring, mutually bonded to form a substituted or unsubstituted fused ring, or not mutually bonded;

a combination of $R_{331}$ and $R_{332}$ are mutually bonded to form a substituted or unsubstituted monocyclic ring, mutually

bonded to form a substituted or unsubstituted fused ring, or not mutually bonded;

$R_{300}$, $R_{331}$, and $R_{332}$ forming neither the substituted or unsubstituted monocyclic ring nor the substituted or unsubstituted fused ring, and $R_{333}$ are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{908}$, a group represented by $-COOR_{909}$, a halogen atom, a cyano group, a nitro group, a substituted or unsubstituted aryl group having 6 to 50 ring carbon atoms, or a substituted or unsubstituted heterocyclic group having 5 to 50 ring atoms; and

* in the formulae (B31) to (B38) each represents a bonding position to another atom in a molecule of the compound M3.

[Formula 189]

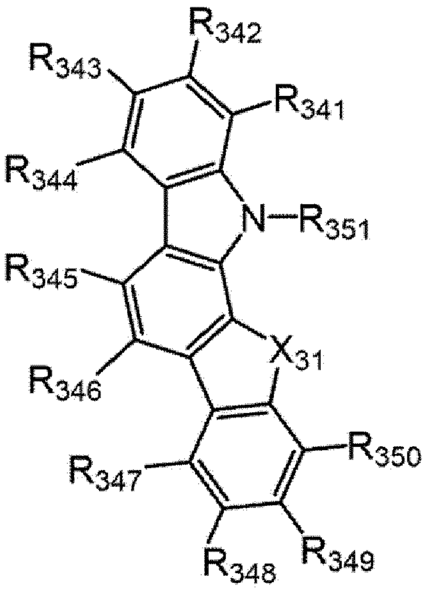

(B39)

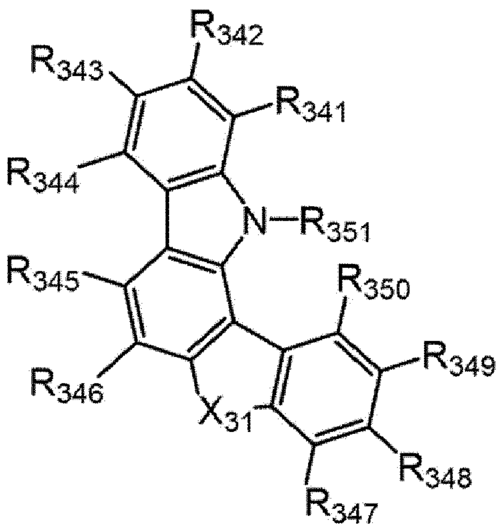

(B40)

[Formula 190]

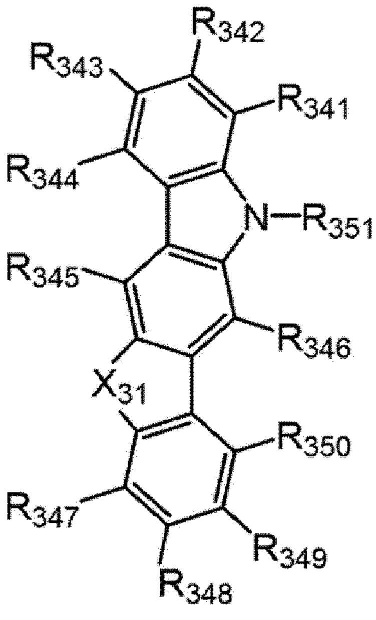

(B41)

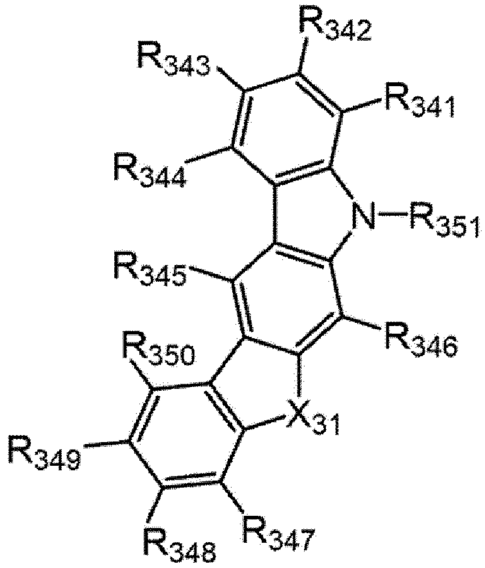

(B42)

[Formula 191]

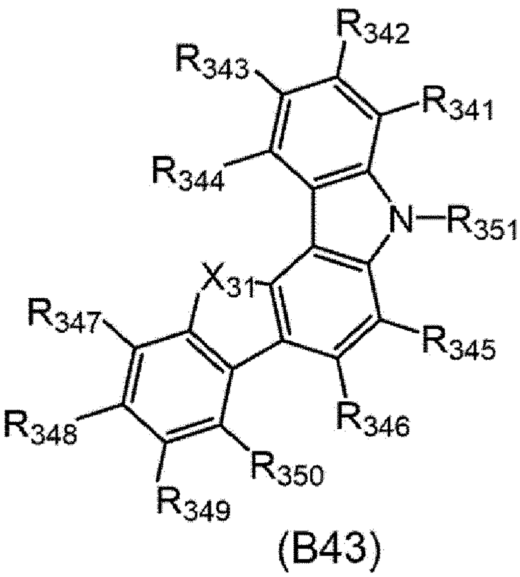

(B43)

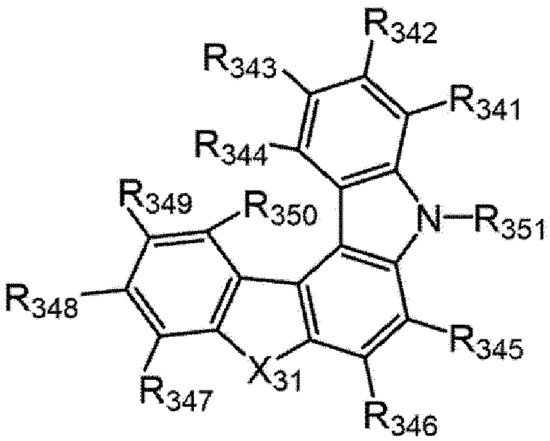

(B44)

In the formulae (B39) to (B44):

at least one combination of adjacent two or more of $R_{341}$ to $R_{350}$ are mutually bonded to form a substituted or unsubstituted monocyclic ring, mutually bonded to form a substituted or unsubstituted fused ring, or not mutually bonded;

at least one of $R_{341}$ to $R_{351}$ represents a bonding position to another atom in a molecule of the compound M3;

$X_{31}$ is a sulfur atom, an oxygen atom, $NR_{352}$, or $CR_{353}R_{354}$;

a combination of $R_{353}$ and $R_{354}$ are mutually bonded to form a substituted or unsubstituted monocyclic ring, mutually bonded to form a substituted or unsubstituted fused ring, or not mutually bonded; and

$R_{341}$ to $R_{351}$ not being a bonding position to another atom in a molecule of the compound M3 and forming neither the substituted or unsubstituted monocyclic ring nor the substituted or unsubstituted fused ring, $R_{353}$ and $R_{354}$ forming neither the substituted or unsubstituted monocyclic ring nor the substituted or unsubstituted fused ring, and

$R_{352}$ are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{908}$, a group represented by $-COOR_{909}$, a halogen atom, a cyano group, a nitro group, a substituted or unsubstituted aryl group having 6 to 50 ring carbon atoms, or a substituted or unsubstituted heterocyclic group having 5 to 50 ring atoms.

The compound M3 represented by the formula (3Y) preferably has at least one group selected from the group consisting of groups represented by formulae (B38) to (B44) in a molecule.

In the formula (3Y), it is preferable that at least one of $Y_{31}$ to $Y_{36}$ is $CR_3$, at least one $R_3$ is a group represented by the formula (3B), and $R_B$ is a group represented by one of the formulae (B31) to (B44).

In the formula (3Y), it is preferable that at least one of $Y_{31}$ to $Y_{36}$ is $CR_3$, at least one $R_3$ is a group represented by the formula (3B), and $R_B$ is a group represented by one of the formulae (B38) to (B44).

In the formulae (3A) and (3B): it is preferable that $L_{31}$ is a single bond, a substituted or unsubstituted arylene group having 6 to 50 ring carbon atoms, a trivalent group, tetravalent group, pentavlent group, or hexavalent group derived from the arylene group, or a divalent group formed by bonding two groups selected from the group consisting of a substituted or unsubstituted arylene group having 6 to 50 ring carbon atoms, or a trivalent group, tetravalent group, pentavlent group, or hexavalent group derived from the divalent group; and

$L_{32}$ is each independently a single bond, or a substituted or unsubstituted arylene group having 6 to 50 ring carbon atoms.

In the formulae (3A) and (3B): it is preferable that $L_{31}$ is a single bond, or a substituted or unsubstituted arylene group having 6 to 50 ring carbon atoms;

$n_3$ is 1; and

$L_{32}$ is a single bond, or a substituted or unsubstituted arylene group having 6 to 50 ring carbon atoms.

In the formulae (3A) and (3B): it is preferable that $L_{31}$ is a single bond, a substituted or unsubstituted phenylene group, a substituted or unsubstituted biphenylene group, or a divalent group formed by bonding two groups selected from the group consisting of a substituted or unsubstituted phenylene group and a substituted or unsubstituted biphenylene group, or a trivalent group, tetravalent group, pentavlent group, or hexavalent group derived from the divalent group;

ns is 1; and

$L_{32}$ is a single bond, a substituted or unsubstituted phenylene group, or a substituted or unsubstituted biphenylene group.

In the compounds represented by the formulae (3X) and (3Y), $R_{352}$ is preferably a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

In the compounds represented by the formulae (3X) and (3Y), it is preferable that a combination of $R_{353}$ and $R_{354}$ are mutually bonded to form a substituted or unsubstituted monocyclic ring, mutually bonded to form a substituted or unsubstituted fused ring, or not mutually bonded;

$R_{353}$ and $R_{354}$ forming neither the substituted or unsubstituted monocyclic ring nor the substituted or unsubstituted fused ring are each independently a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

In the compounds represented by the formulae (3X) and (3Y), it is preferable that the substituent for "the substituted or unsubstituted" group is an unsubstituted alkyl group having 1 to 25 carbon atoms, an unsubstituted alkenyl group having 2 to 25 carbon atoms, an unsubstituted alkynyl group having 2 to 25 carbon atoms, an unsubstituted cycloalkyl group having 3 to 25 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, an unsubstituted aralkyl group having 7 to 50 carbon atoms, a group represented by $-C(=O)R_{908}$, a group represented by $-COOR_{909}$, a group represented by $-P(=O)(R_{931})(R_{932})$, a group represented by $-Ge(R_{933})(R_{934})(R_{935})$, a group represented by $-B(R_{936})(R_{937})$, a group represented by $-S(=O)_2R_{938}$, a halogen atom, a cyano group, a nitro group, an unsubstituted aryl group having 6 to 25 ring carbon atoms, or an unsubstituted heterocyclic group having 5 to 25 ring atoms; and

$R_{901}$ to $R_{909}$ and $R_{931}$ to $R_{938}$ are each independently a hydrogen atom, an unsubstituted alkyl group having 1 to 25 carbon atoms, an unsubstituted aryl group having 6 to 25 ring carbon atoms, or an unsubstituted heterocyclic group having 5 to 25 ring atoms.

In the compounds represented by the formulae (3X) and (3Y), it is preferable that the substituent for "the substituted or unsubstituted" group is a halogen atom, an unsubstituted alkyl group having 1 to 25 carbon atoms, an unsubstituted aryl group having 6 to 25 ring carbon atoms, or an unsubstituted heterocyclic group having 5 to 25 ring atoms.

In the compounds represented by the formulae (3X) and (3Y), it is preferable that the substituent for "the substituted or unsubstituted" group is an unsubstituted alkyl group having 1 to 10 carbon atoms, an unsubstituted aryl group having 6 to 12 ring carbon atoms, or an unsubstituted heterocyclic group having 5 to 12 ring atoms.

In the compounds represented by the formulae (3X) and (3Y), the groups specified to be "substituted or unsubstituted" are each also preferably an "unsubstituted" group.

Producing Method of Compound M3

The compound M3 of the exemplary embodiment can be produced by a known method.

Specific Examples of Compound M3

Specific examples of the compound M3 in the exemplary embodiment include compounds below. However, the invention is by no means limited to the specific examples of the compound.

[Formula 192]

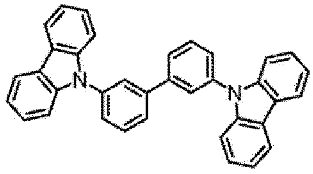
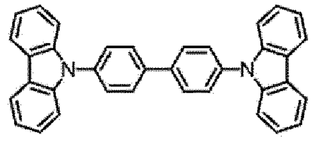
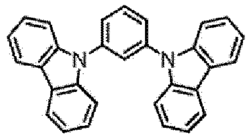
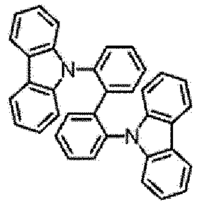
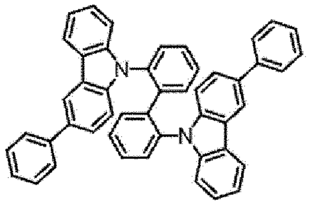
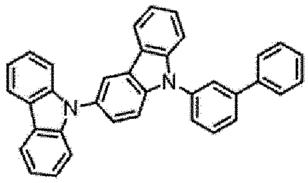
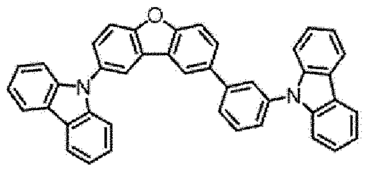
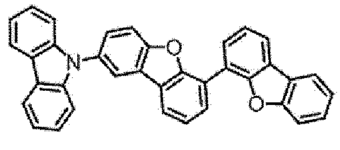
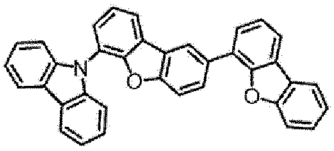
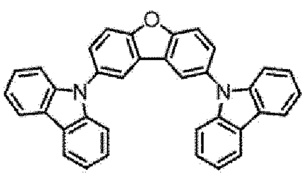
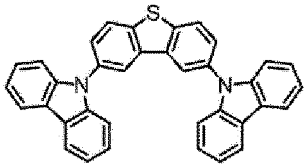
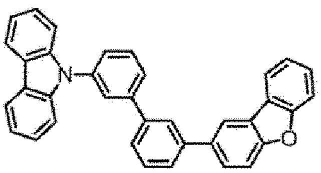
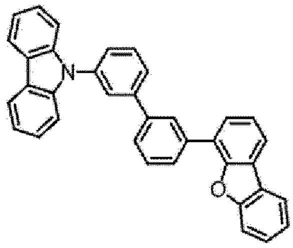
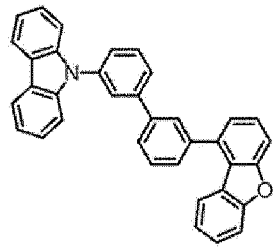
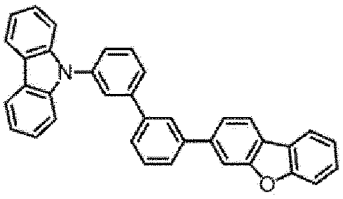
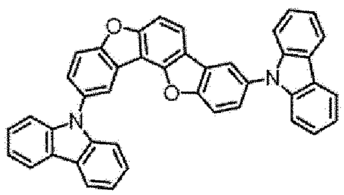

[Formula 193]

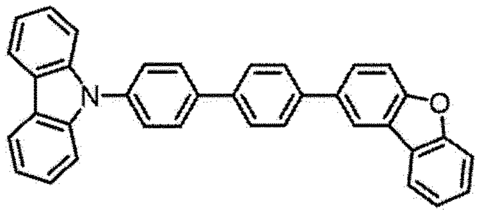
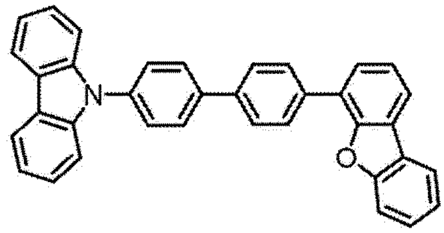
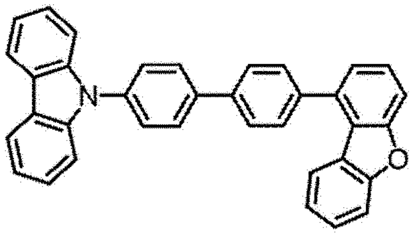
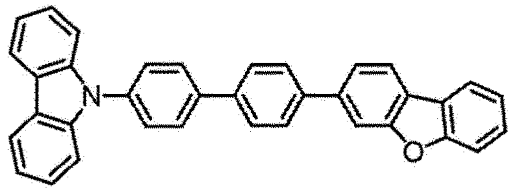
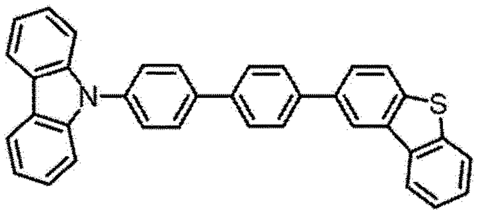
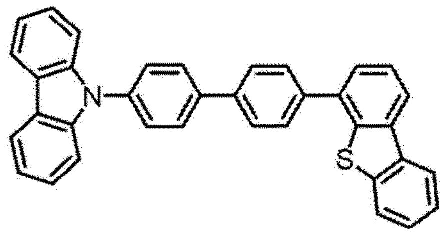
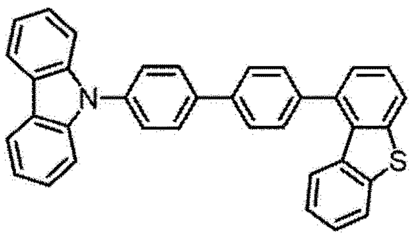
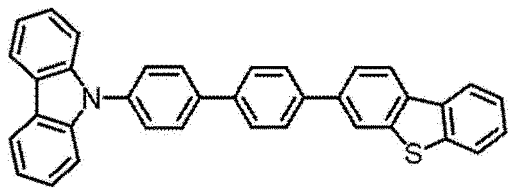
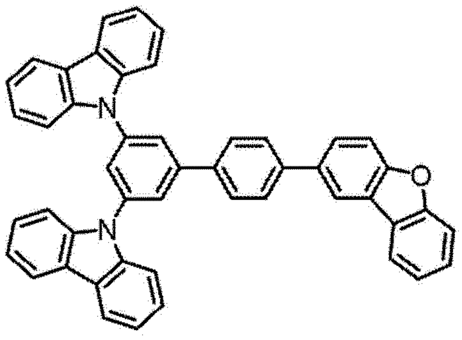
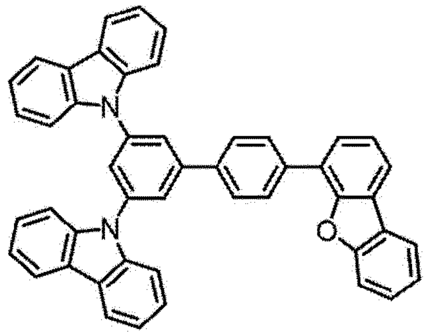
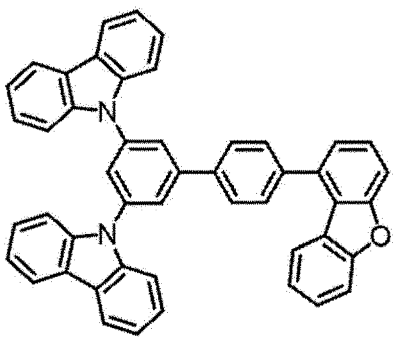
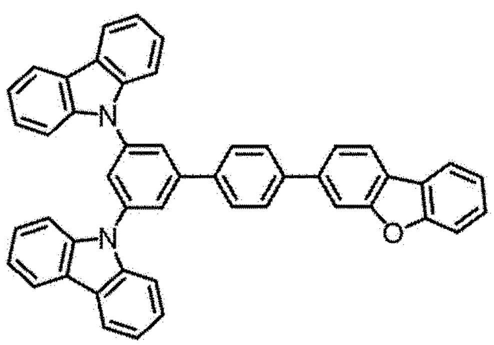

[Formula 194]

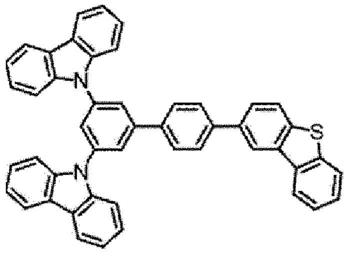
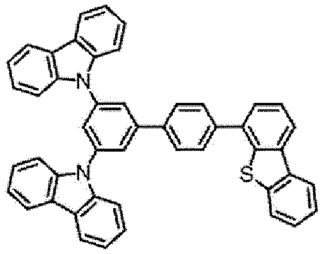
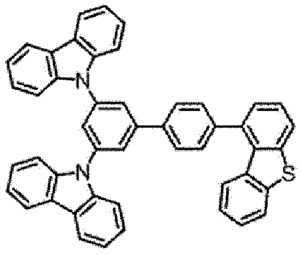
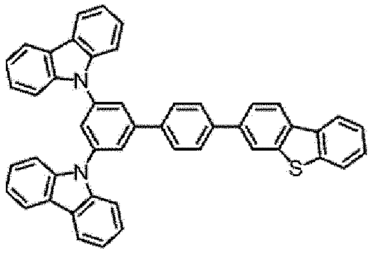
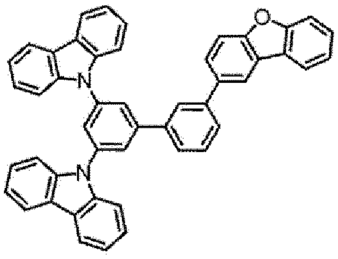
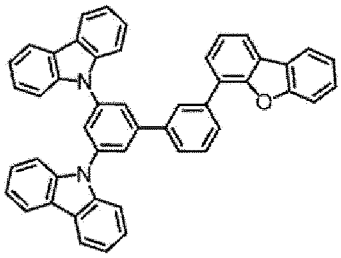
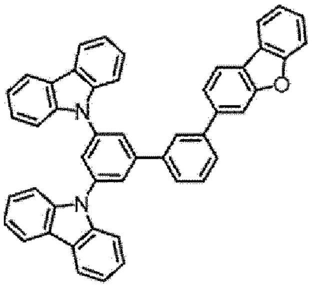
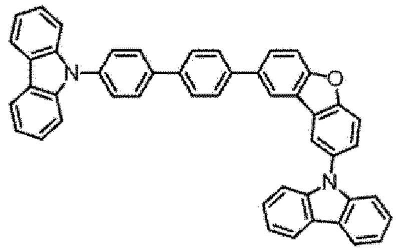
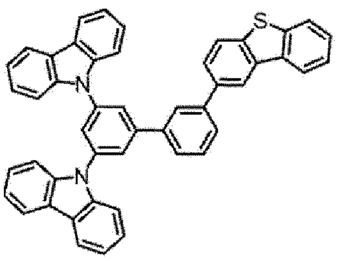
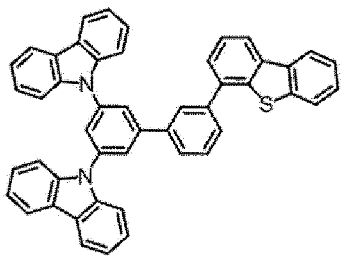
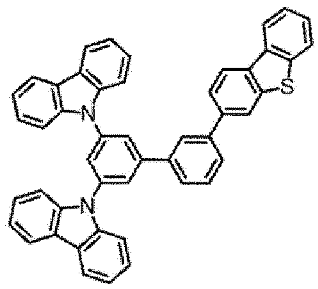

[Formula 195]

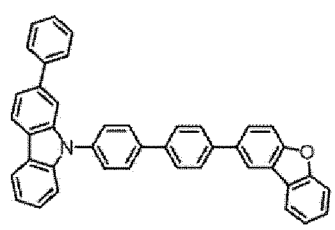
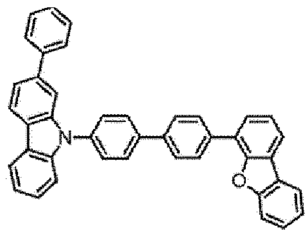
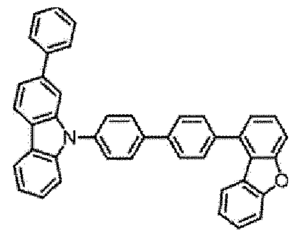
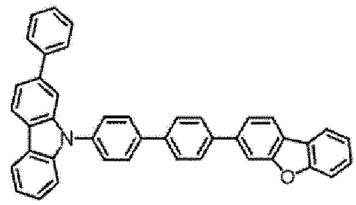
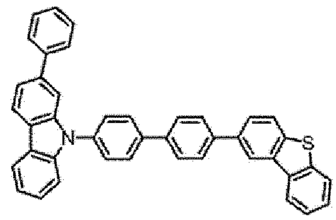
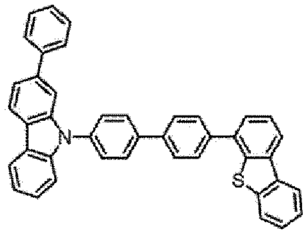
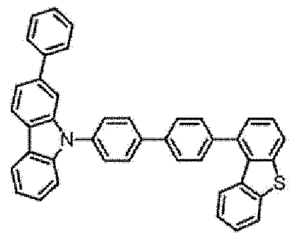
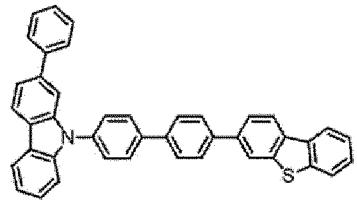
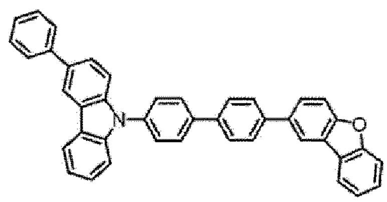
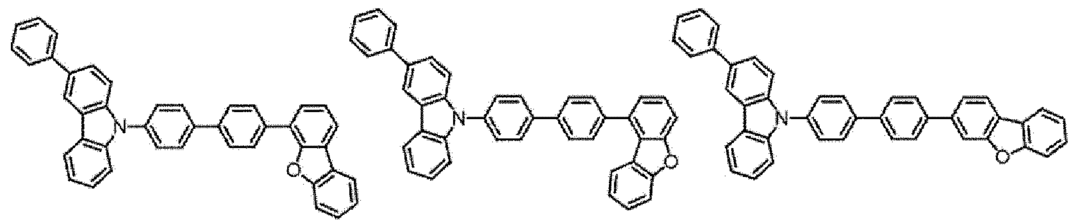

[Formula 196]

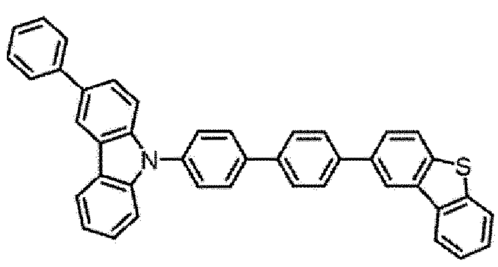
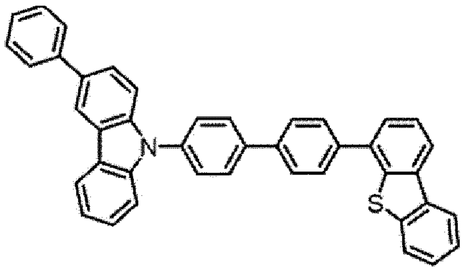
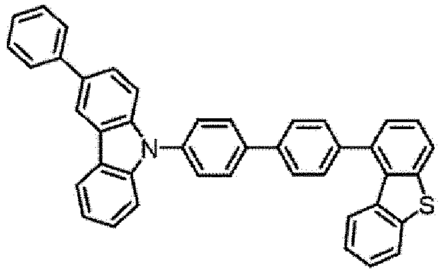
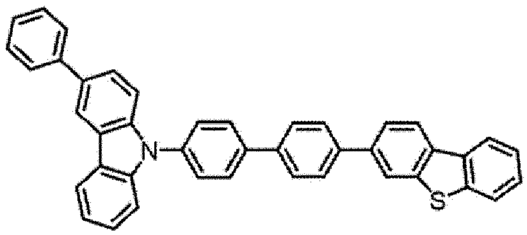
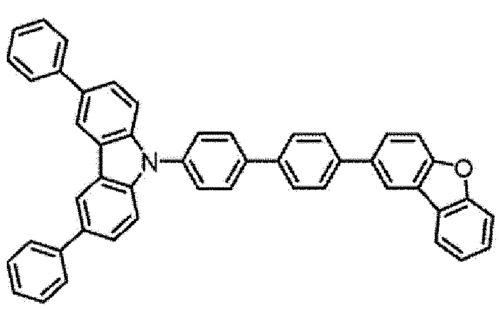
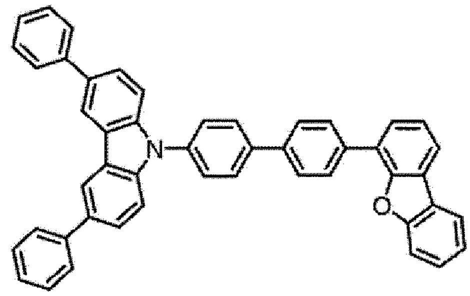
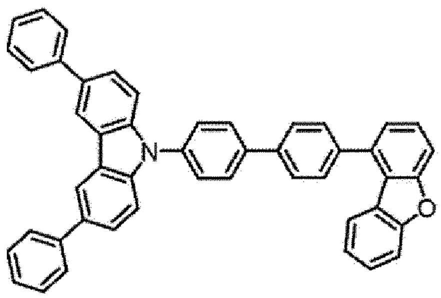
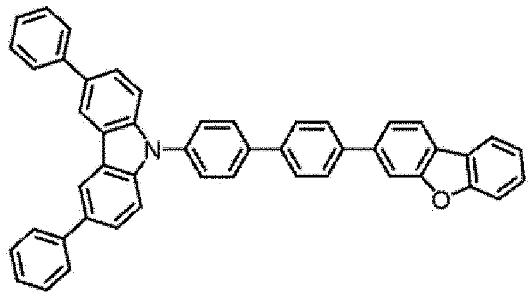

[Formula 197]

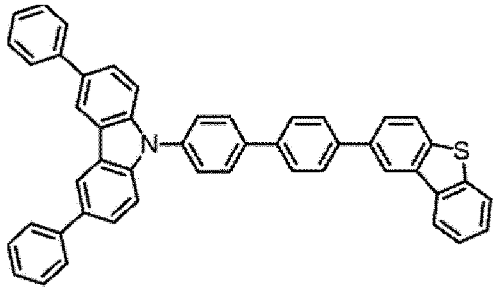
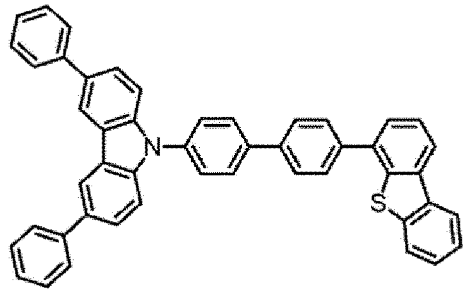
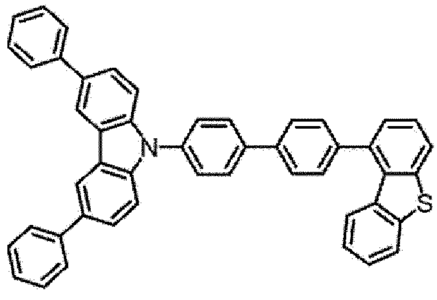
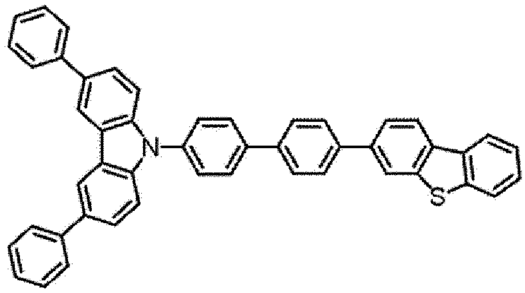

[Formula 198]

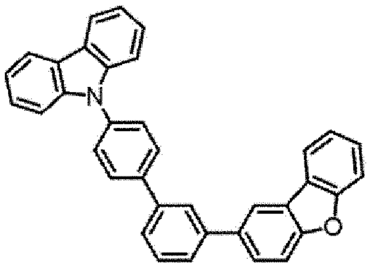
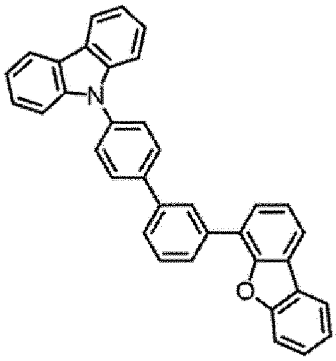
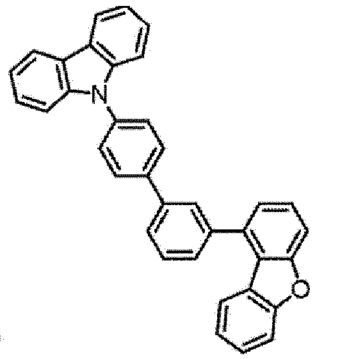
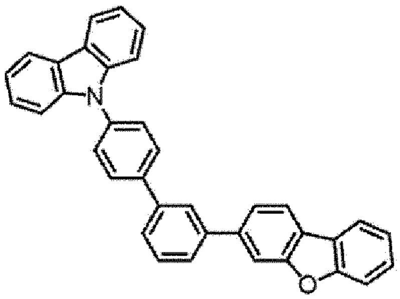
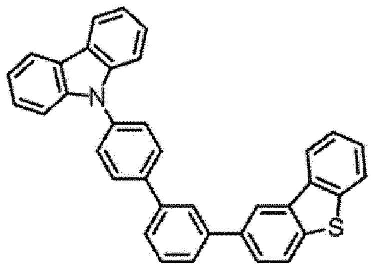
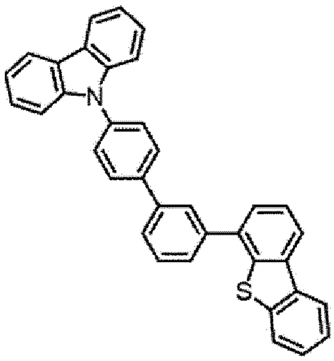
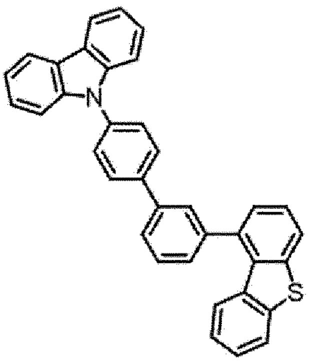
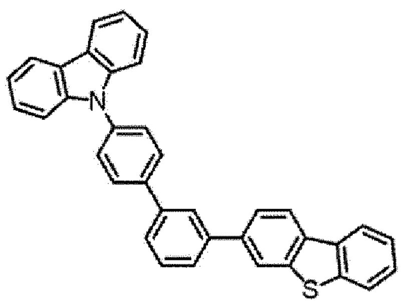

[Formula 199]

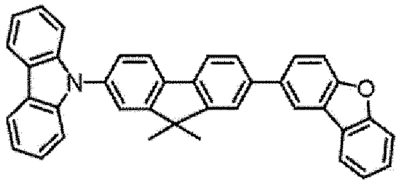
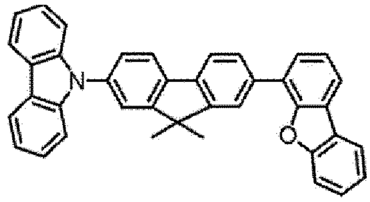
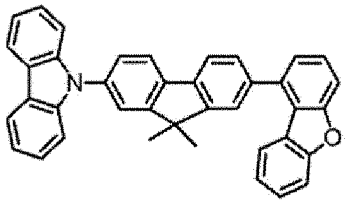
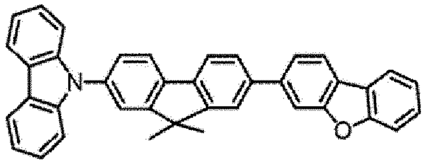
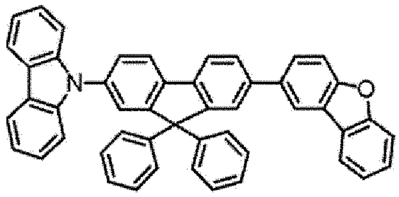
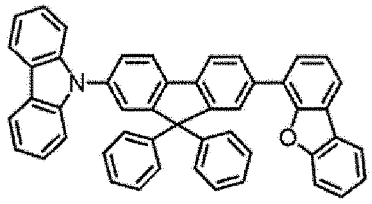
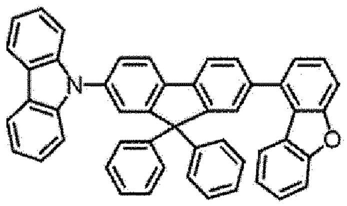
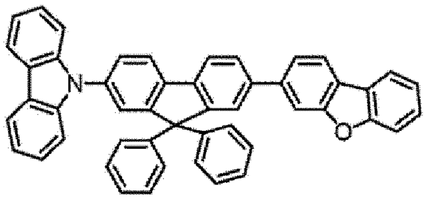
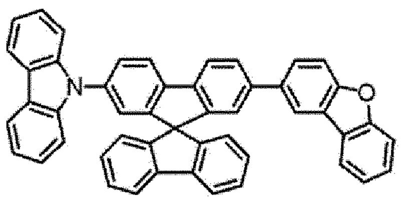
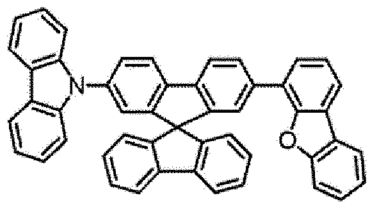
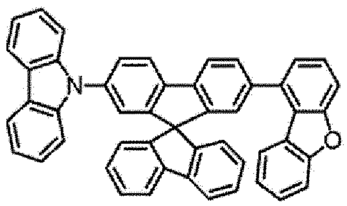
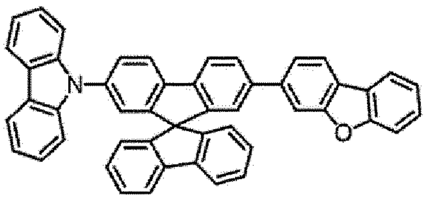

[Formula 200]

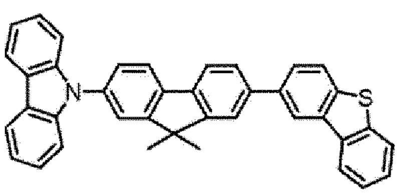 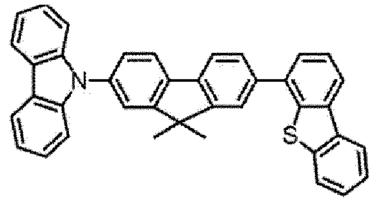

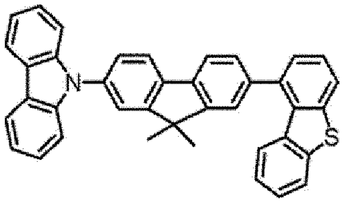 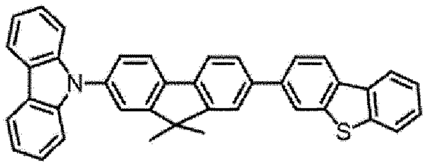

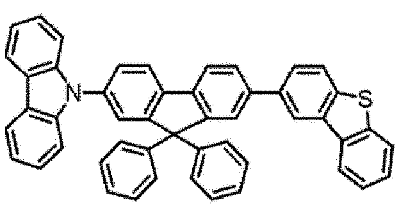 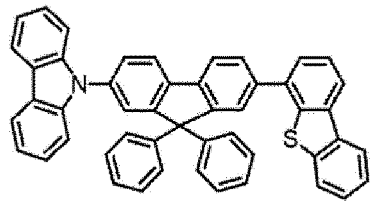

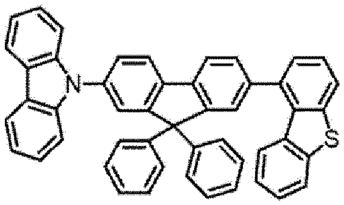 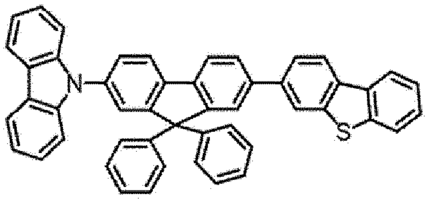

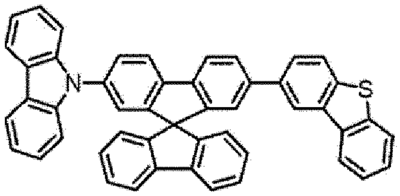 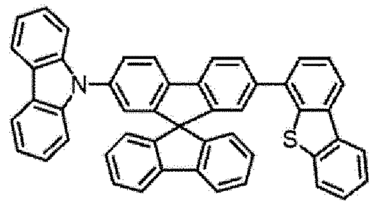

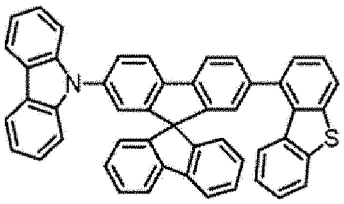 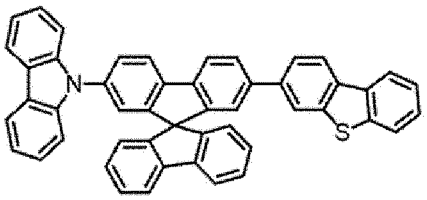

[Formula 201]

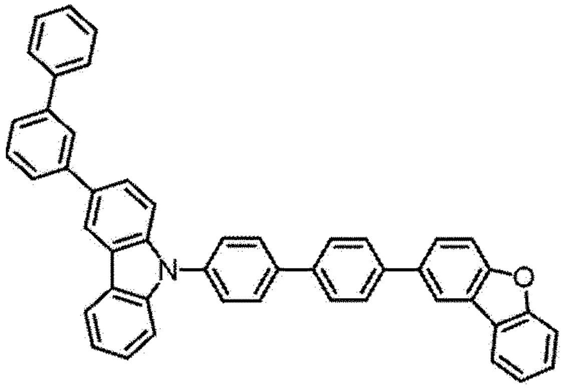

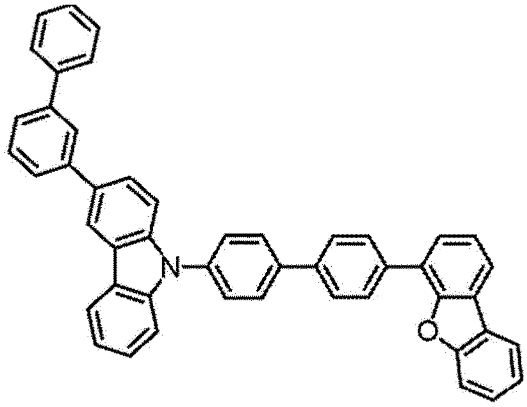

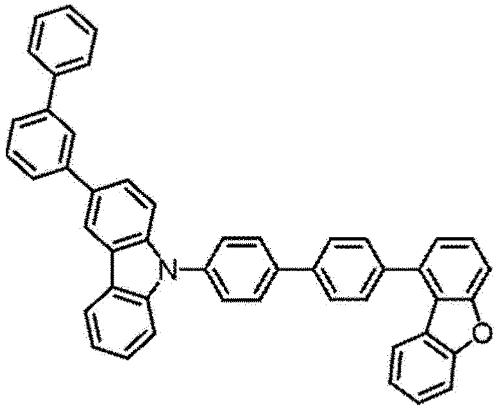

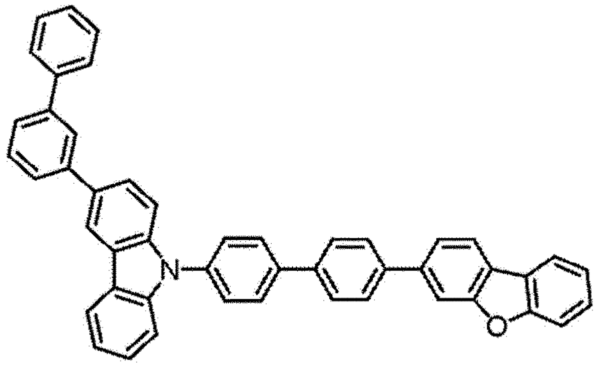

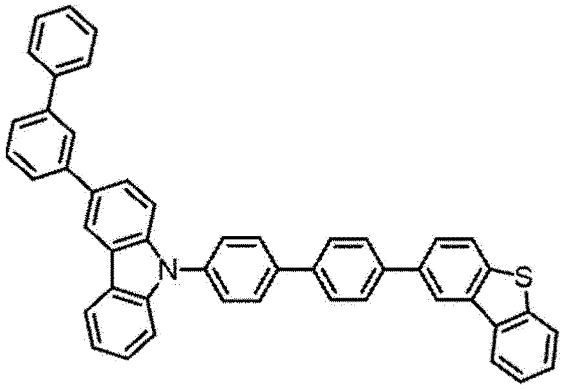

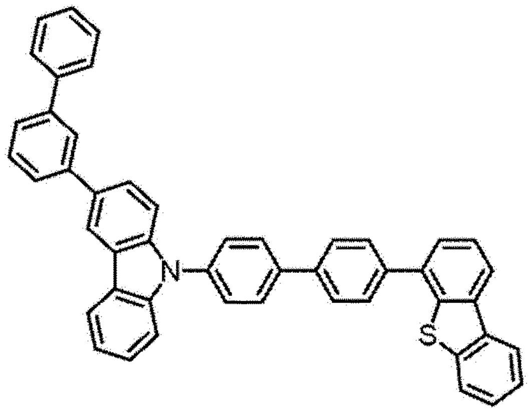

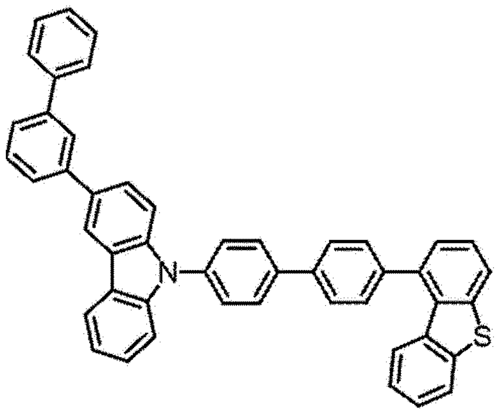

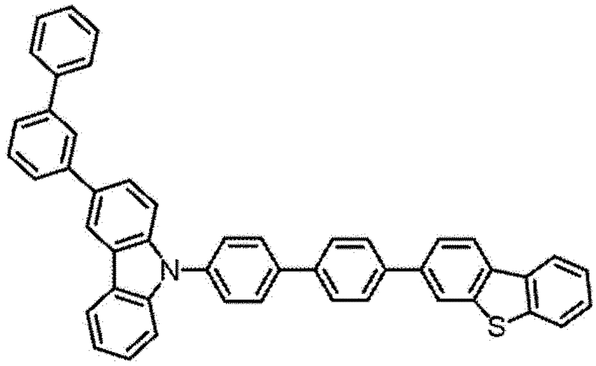

[Formula 202]

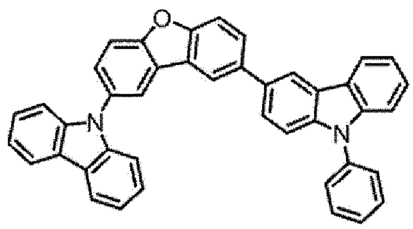
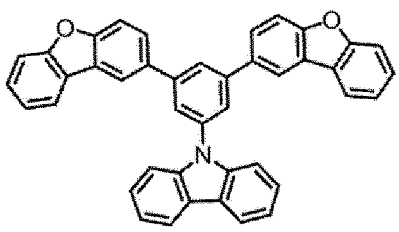

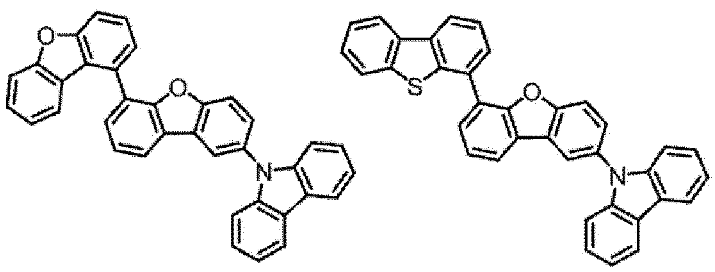
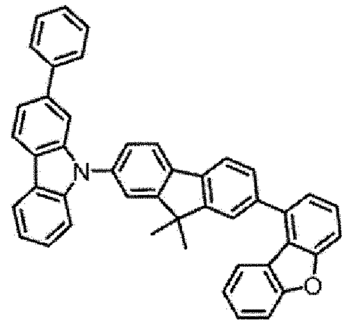

[Formula 203]

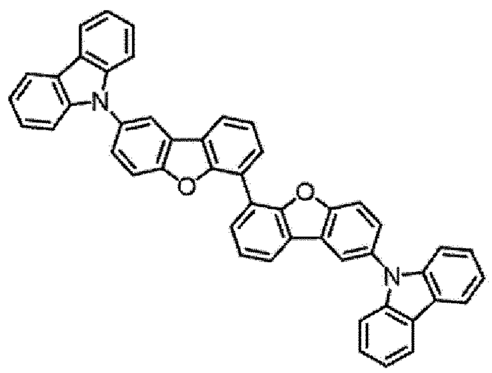
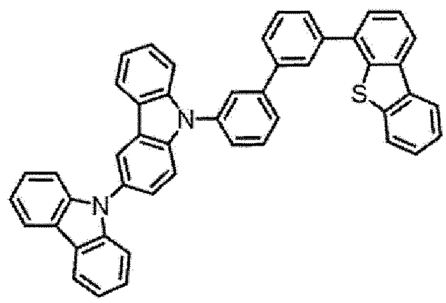

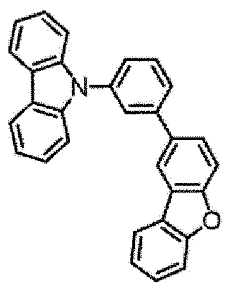
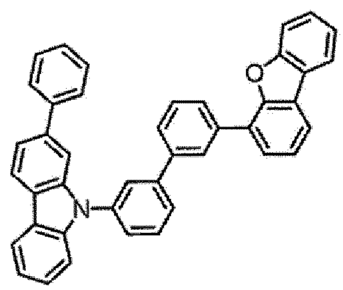
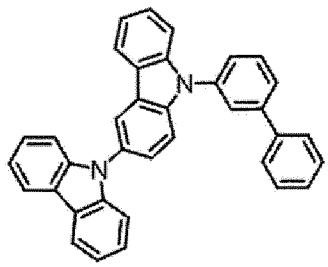

[Formula 204]

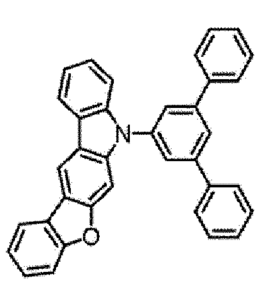
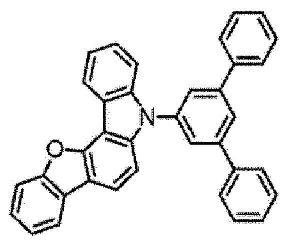
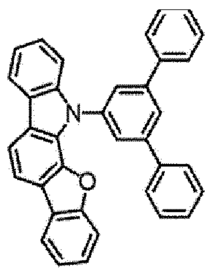

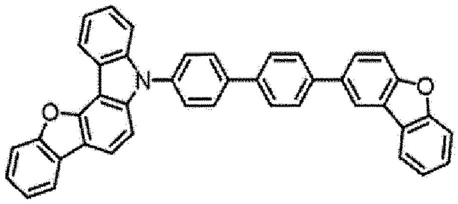
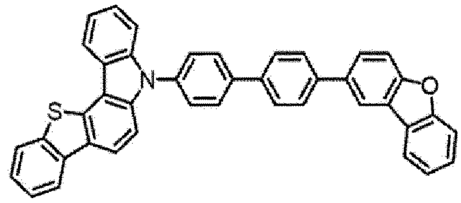

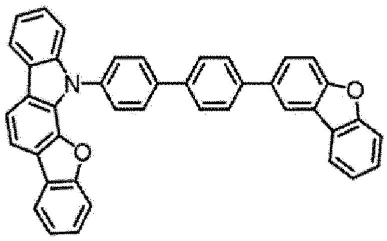
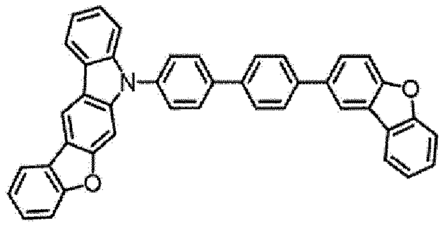

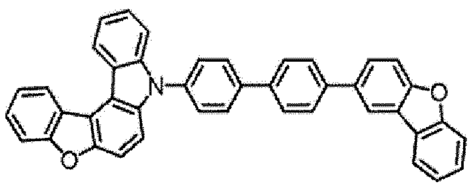
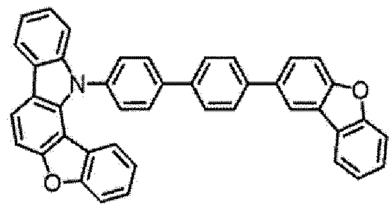

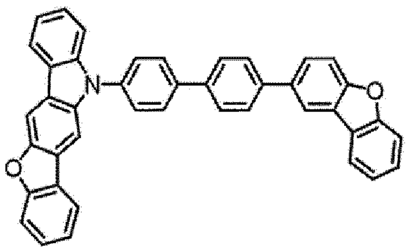
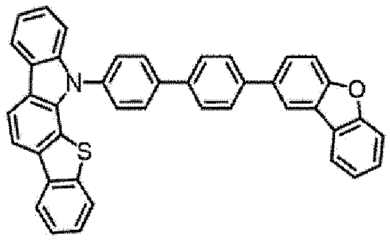

[Formula 205]

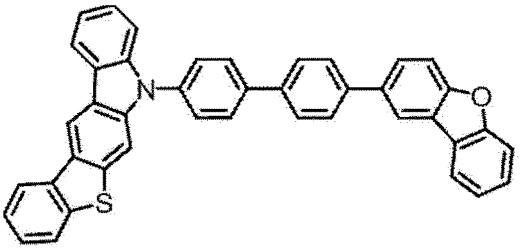
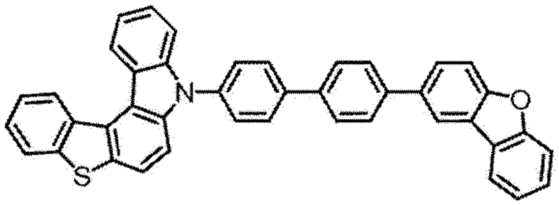
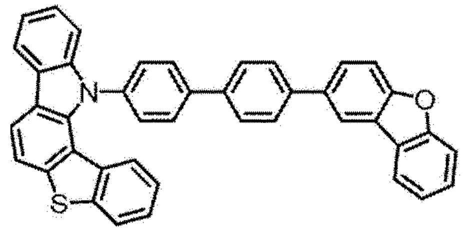
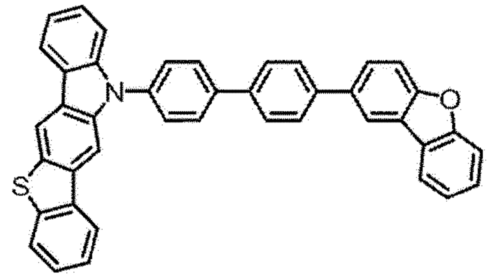
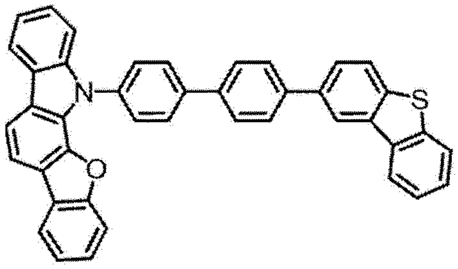
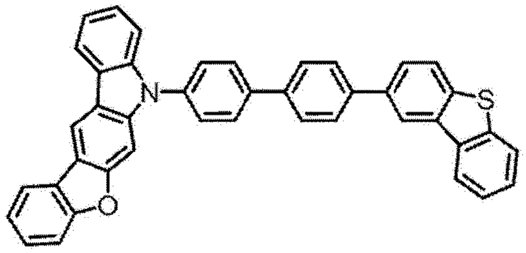
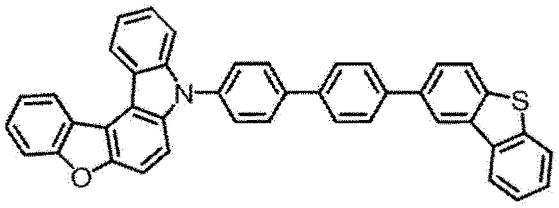
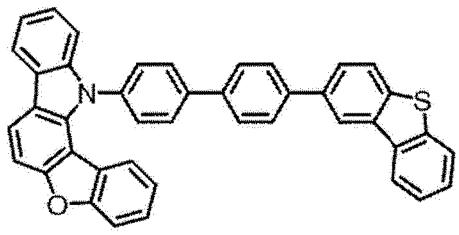
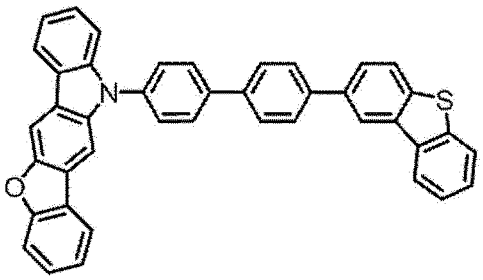
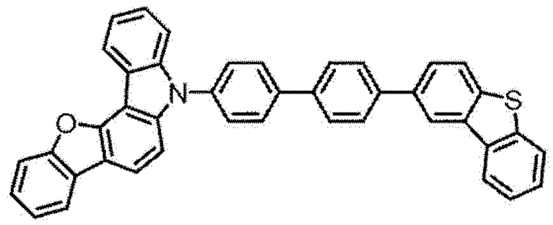
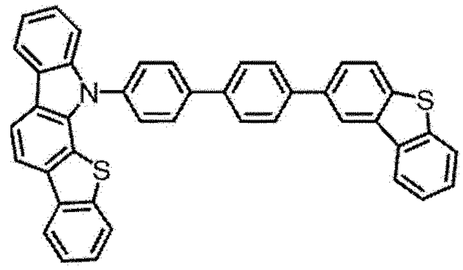
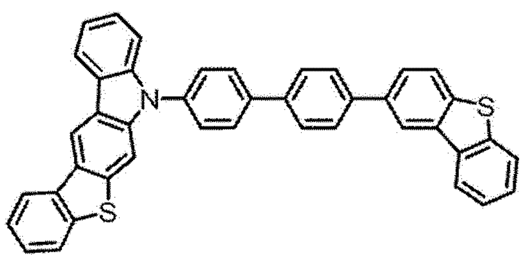

[Formula 206]

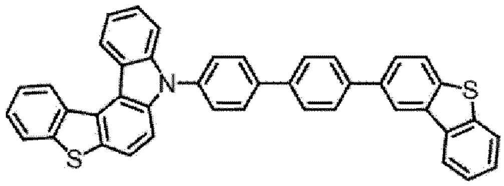

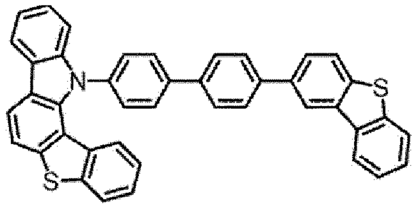

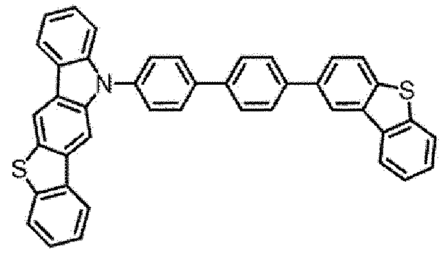

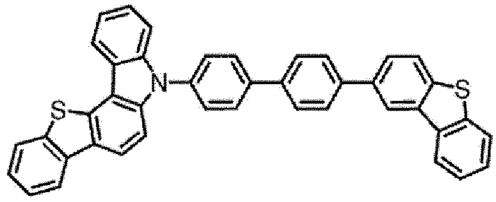

[Formula 207]

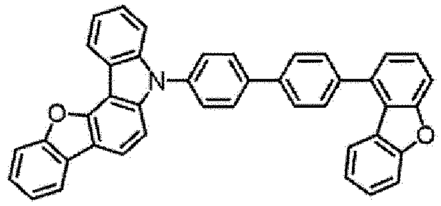

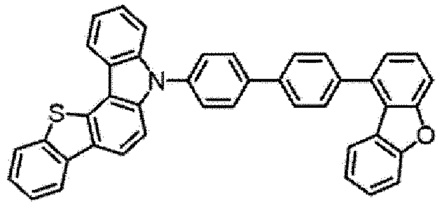

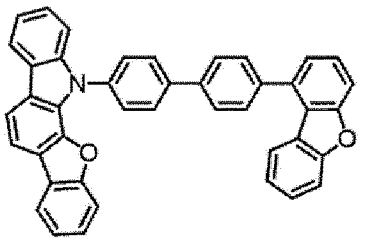

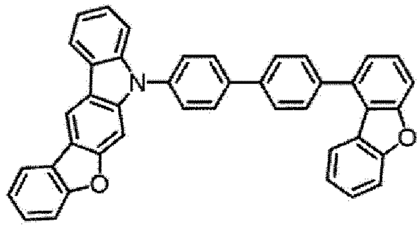

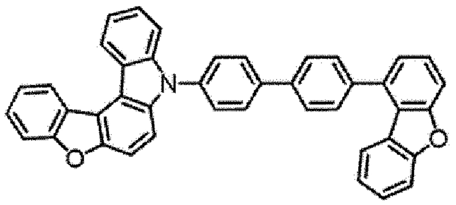

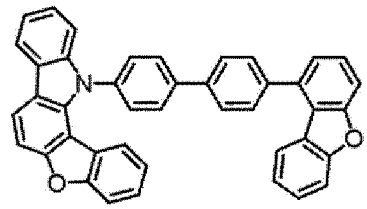

[Formula 208]

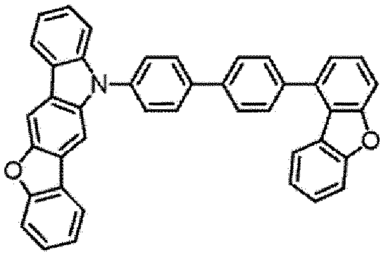

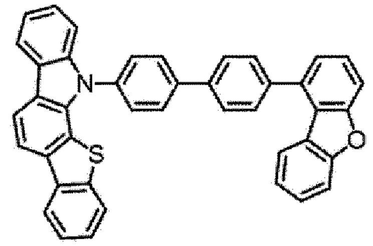

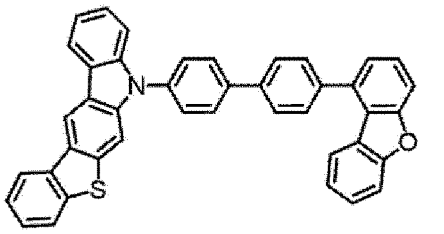

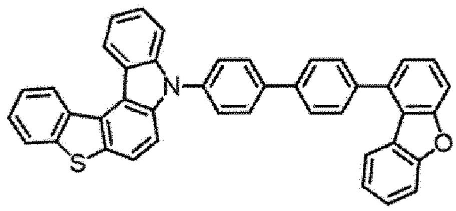

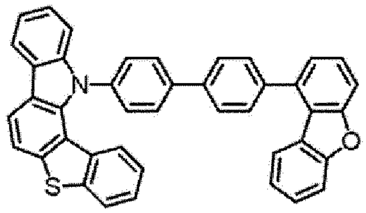

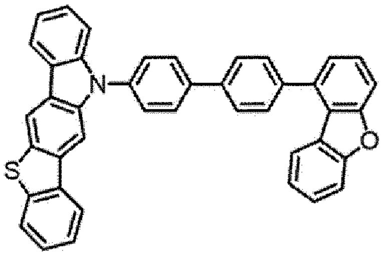

[Formula 209]

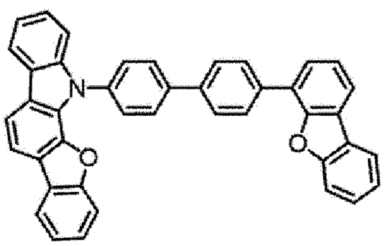

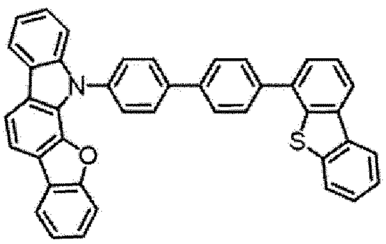

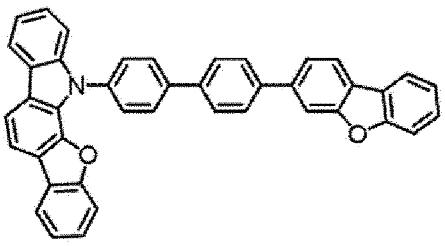

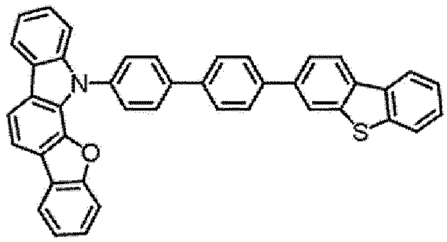

[Formula 210]

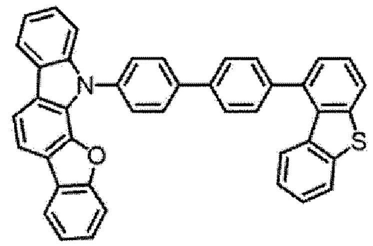

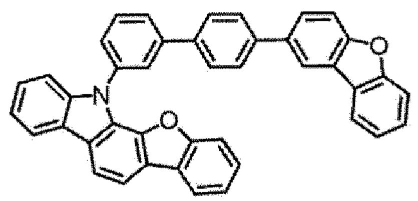

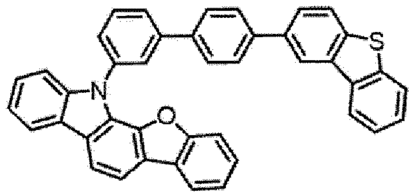

[Formula 211]

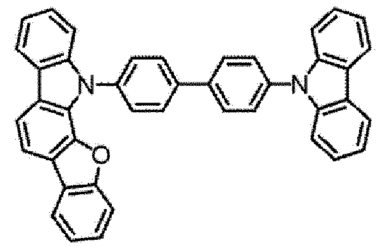

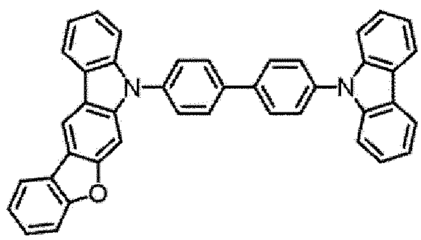

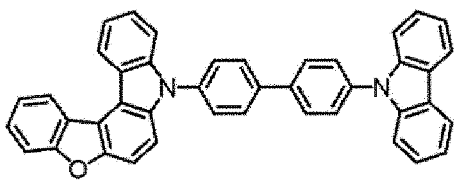

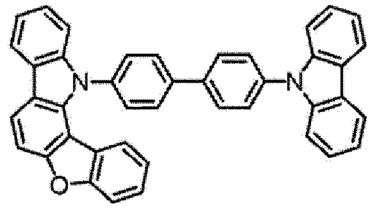

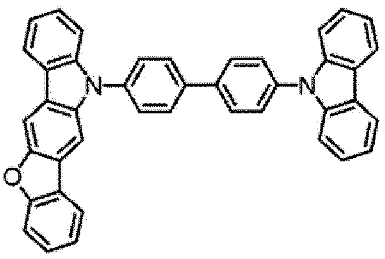

[Formula 212]

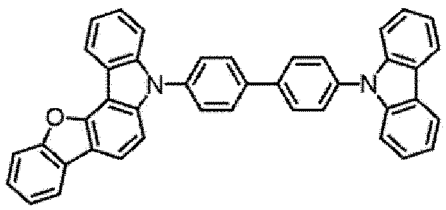
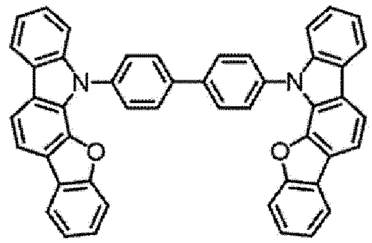
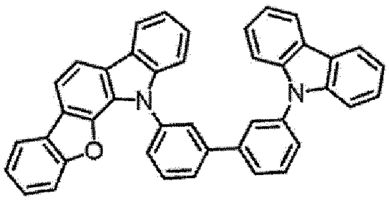
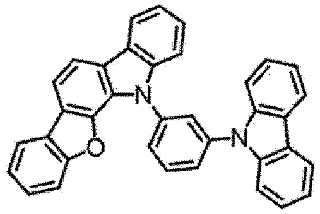
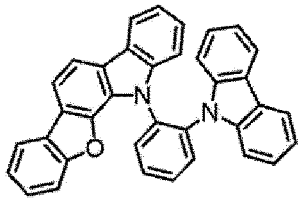
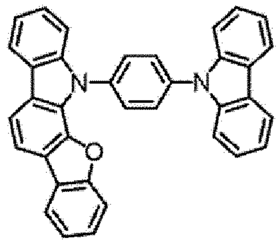
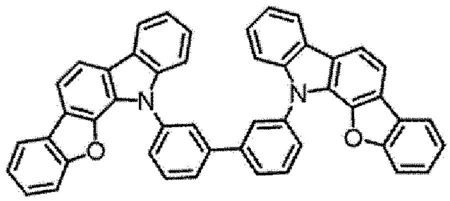

[Formula 213]

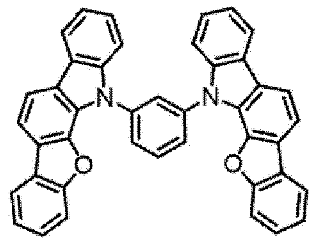
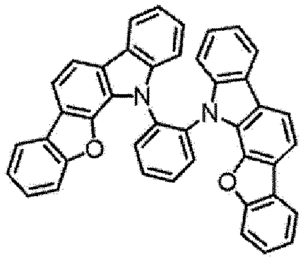
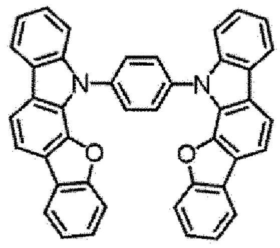
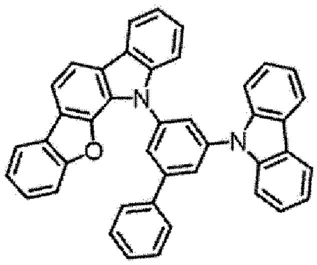
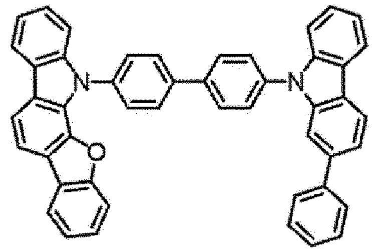

[Formula 214]

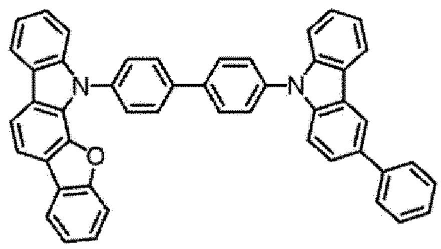
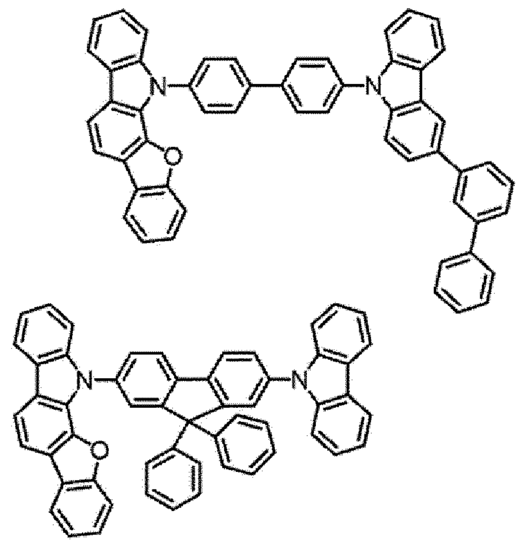
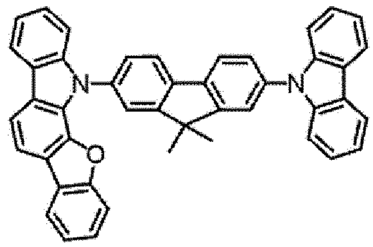
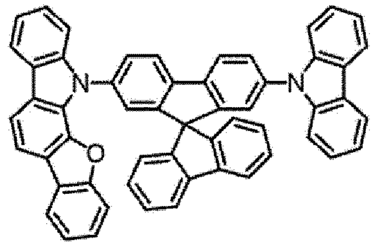
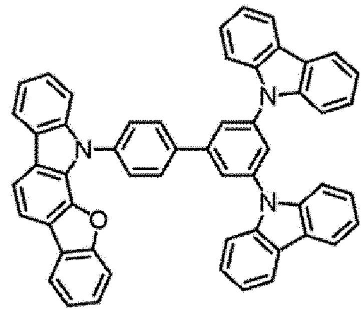
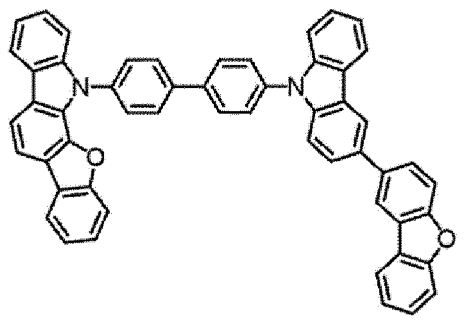

[Formula 215]

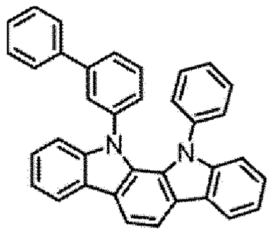
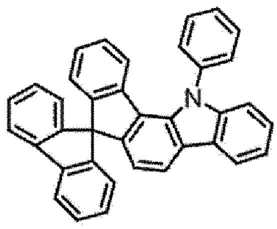
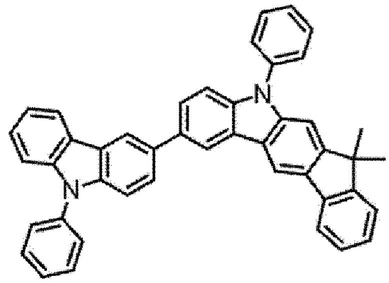
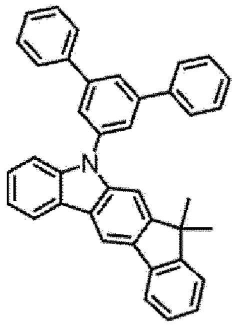
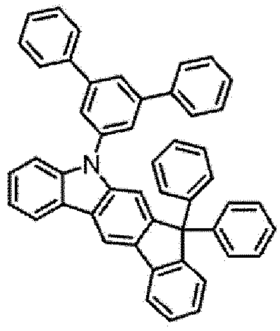
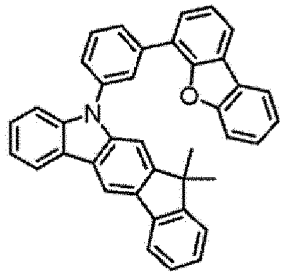
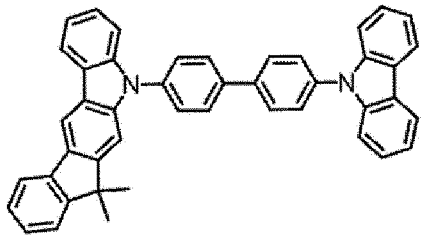
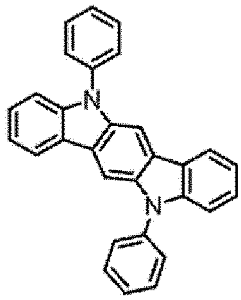
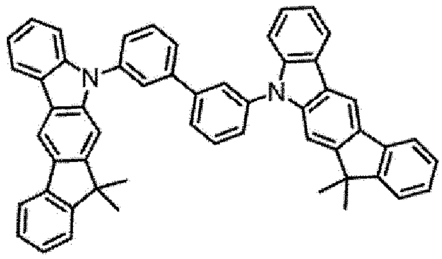
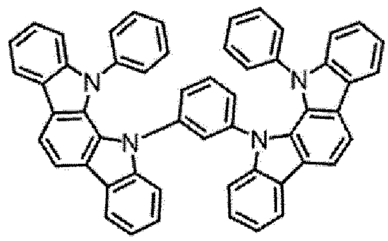

[Formula 216]

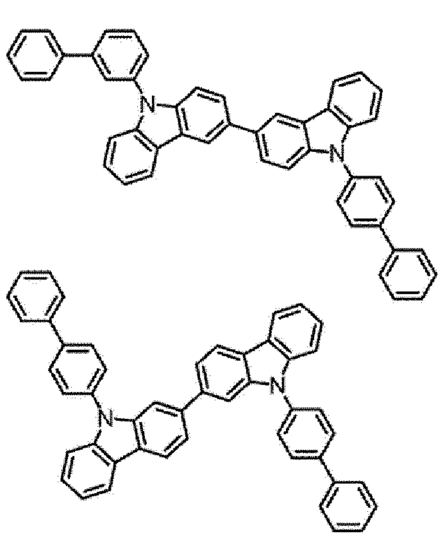

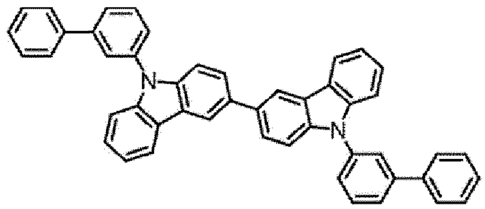

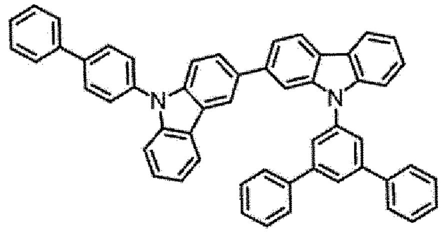

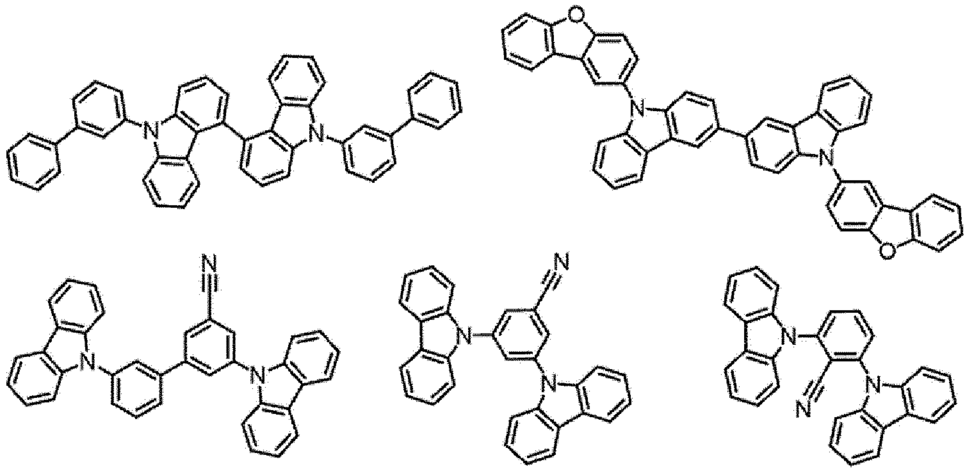

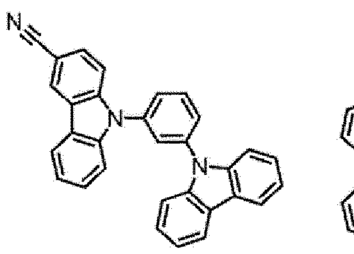

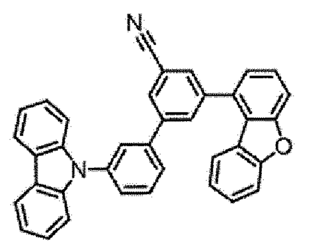

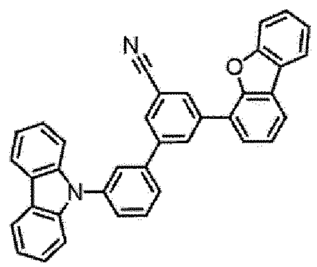

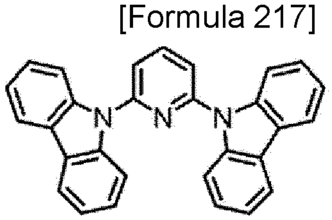

[Formula 217]

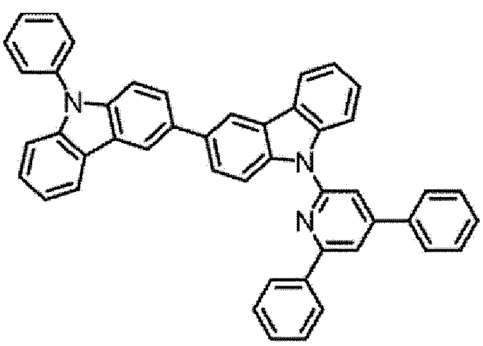

[Formula 218]

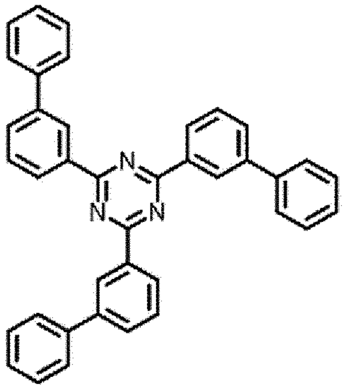
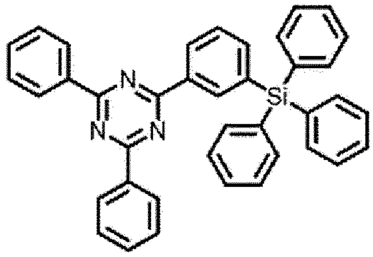
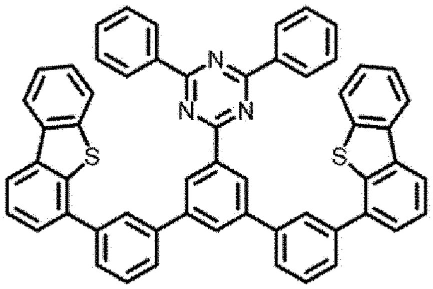
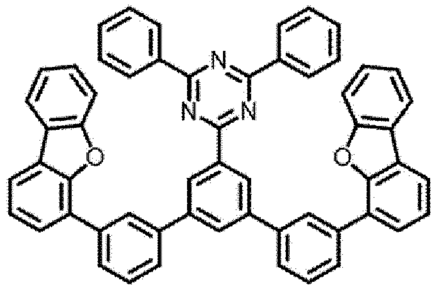
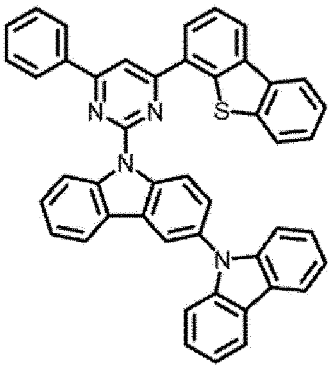
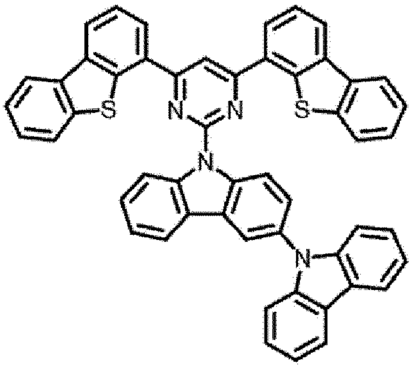

[Formula 219]

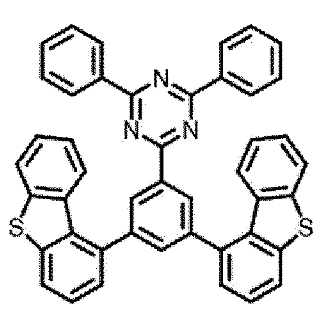

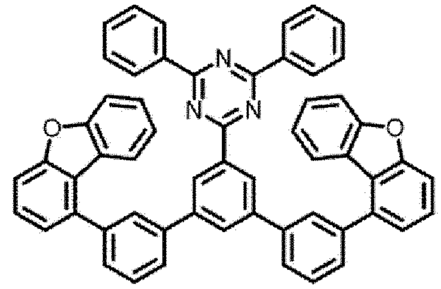

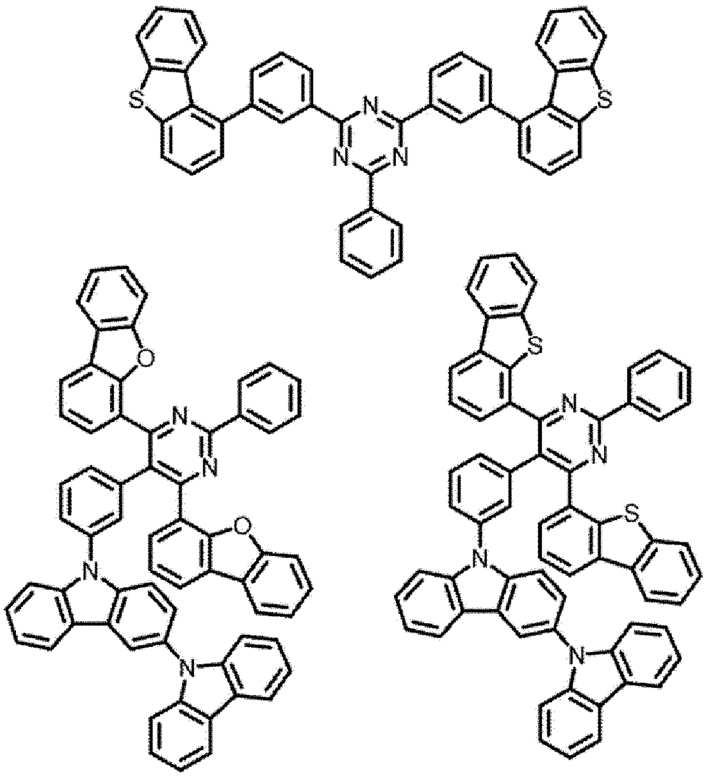

[Formula 220]

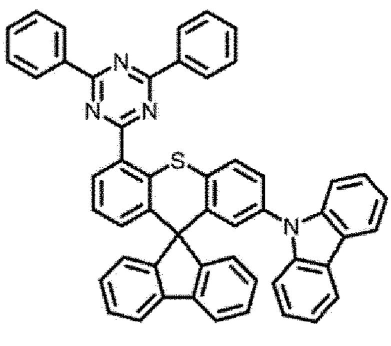

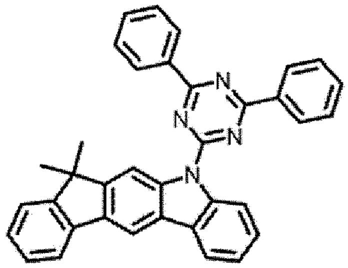

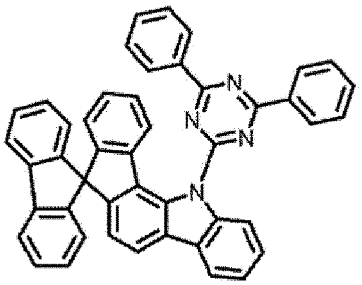

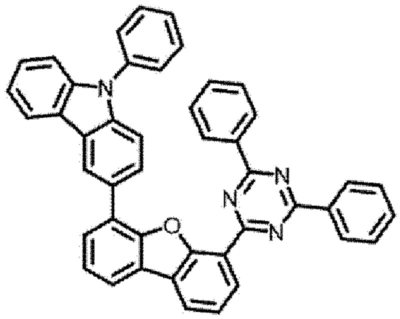

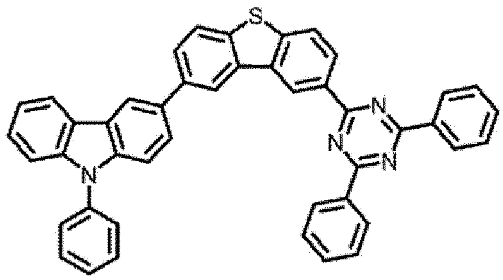

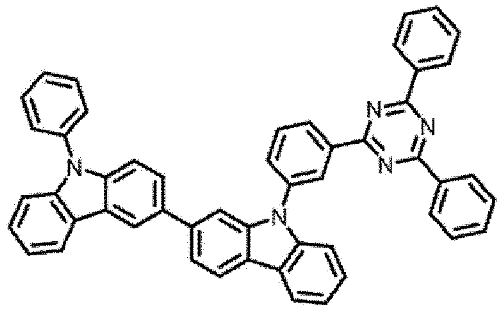

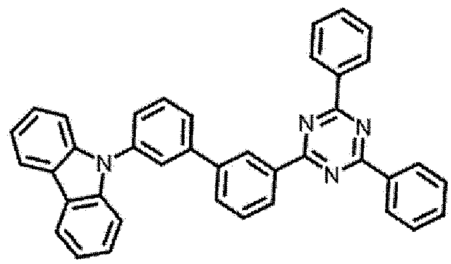

[Formula 221]

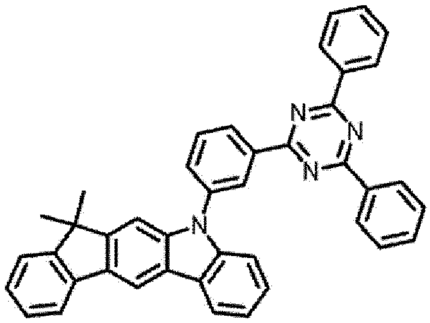

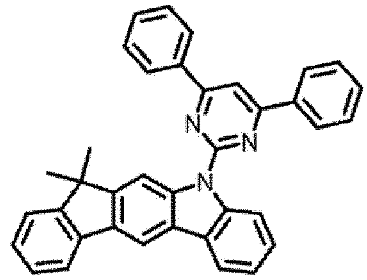

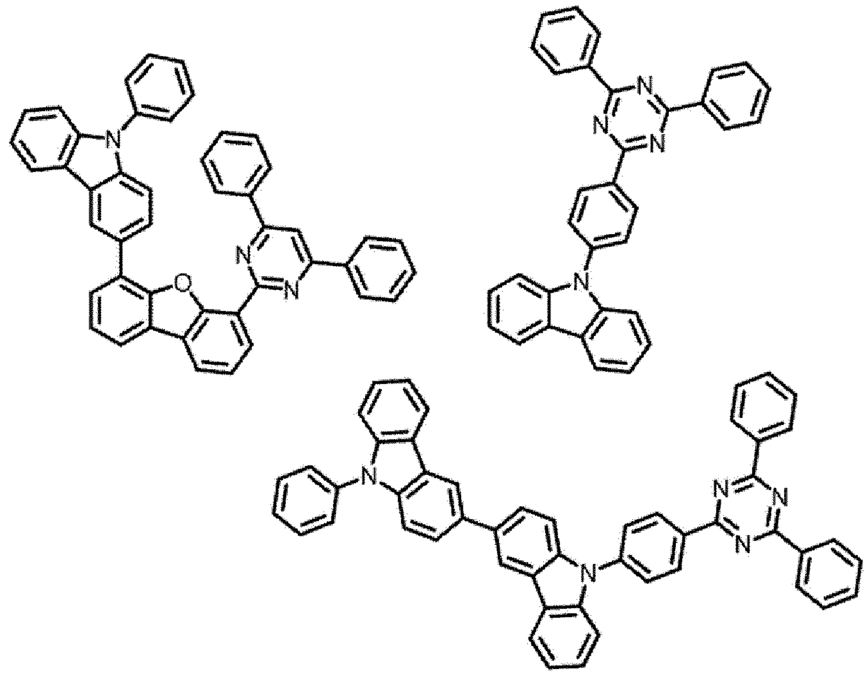

[Formula 222]

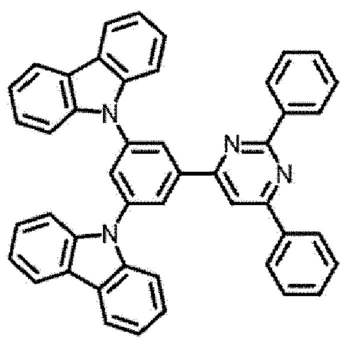

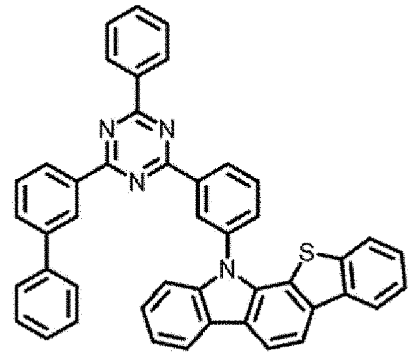

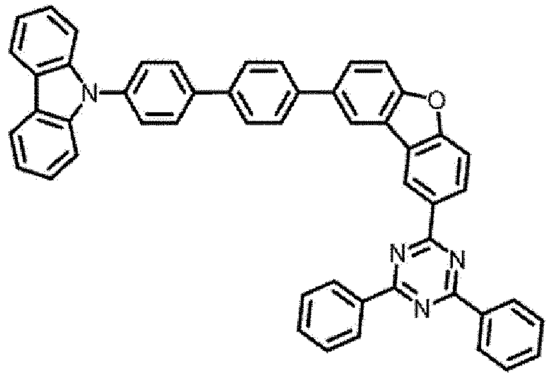

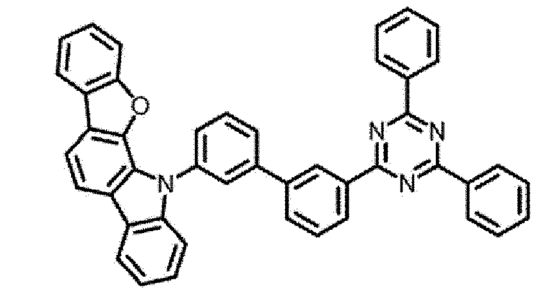

[Formula 223]

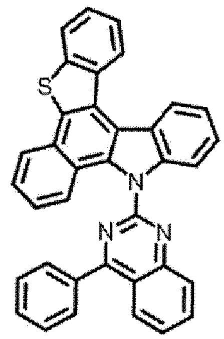

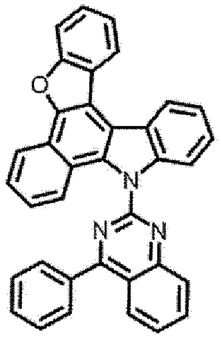

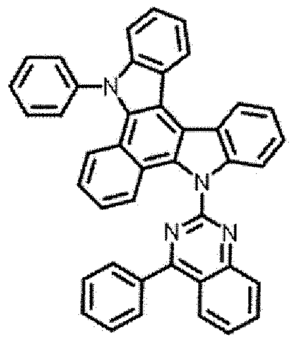

[Formula 224]

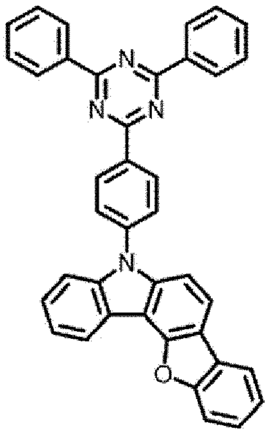

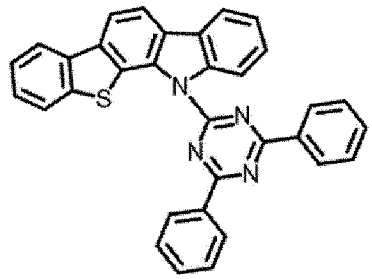

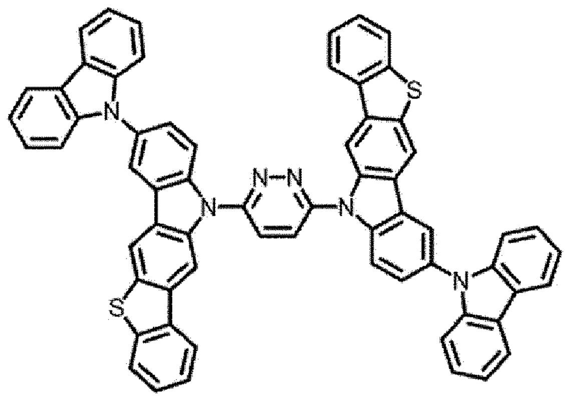

[Formula 225]

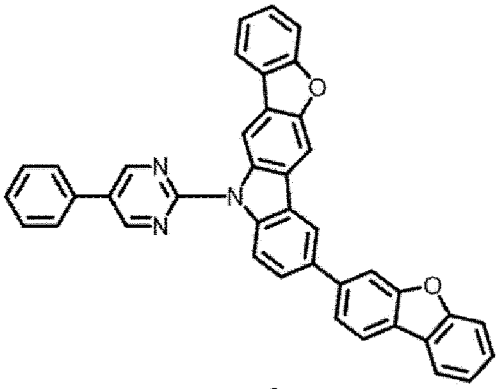

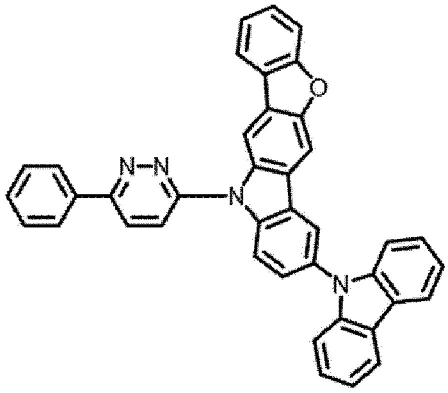

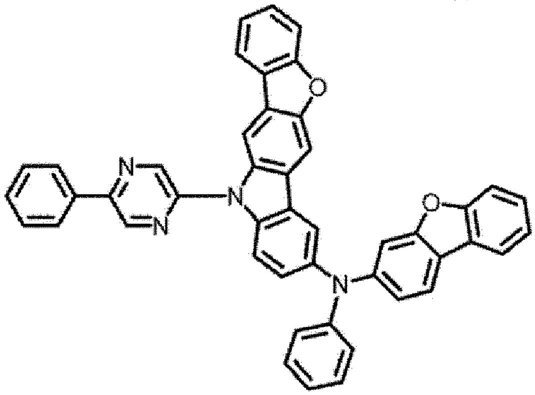

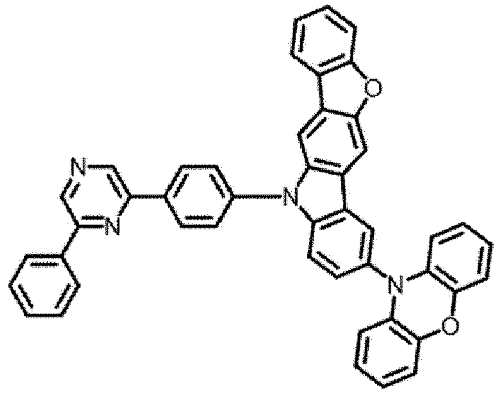

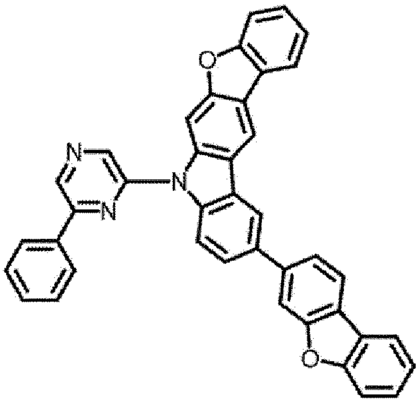

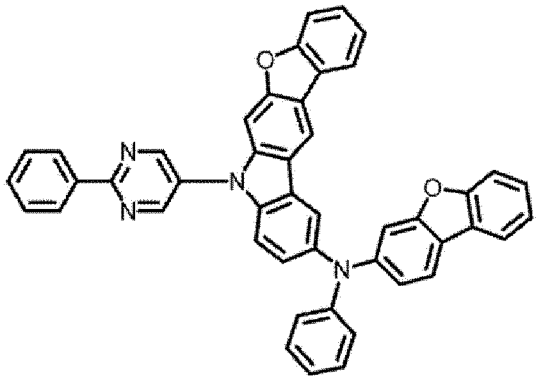

[Formula 226]

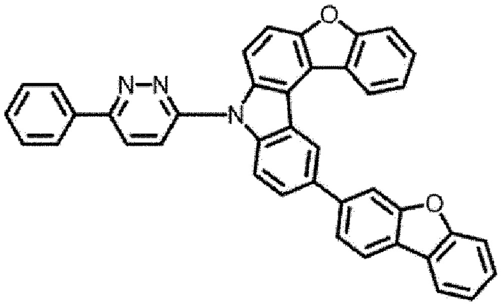
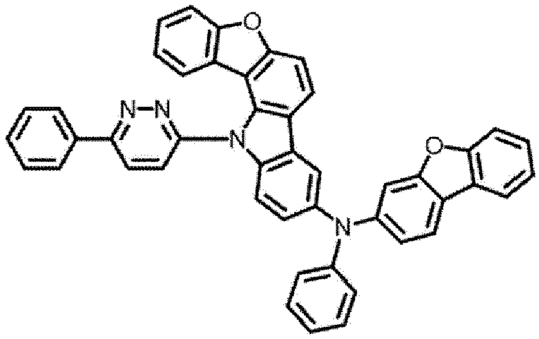
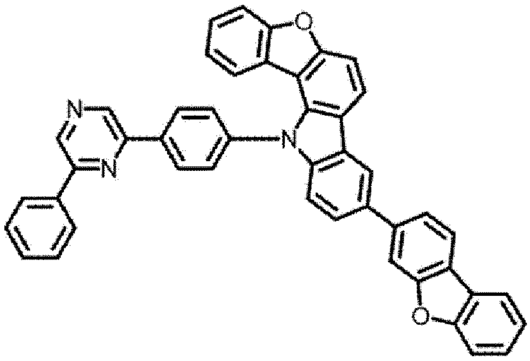
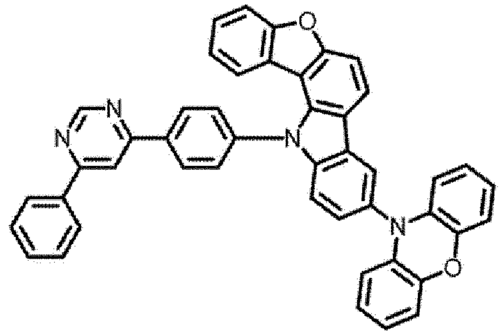

[Formula 227]

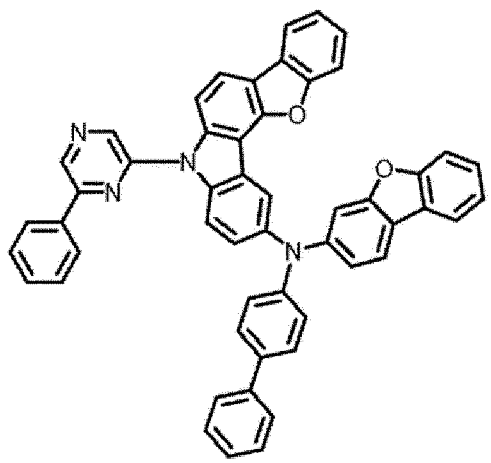
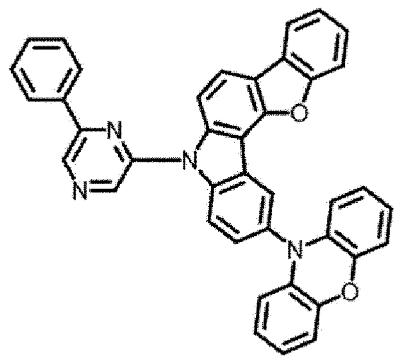
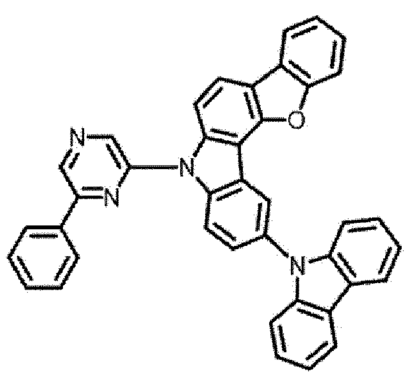
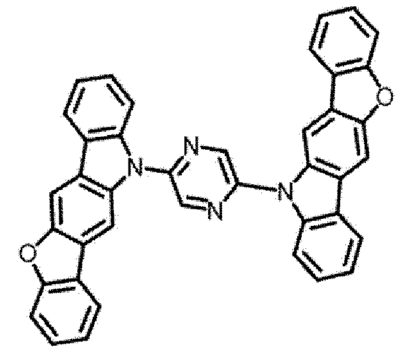

[Formula 228]

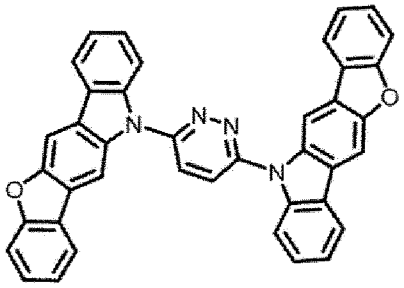

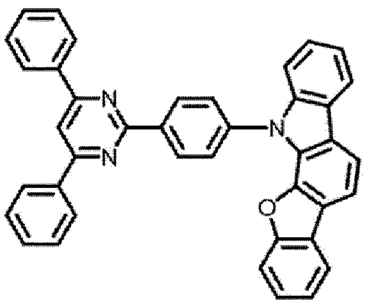

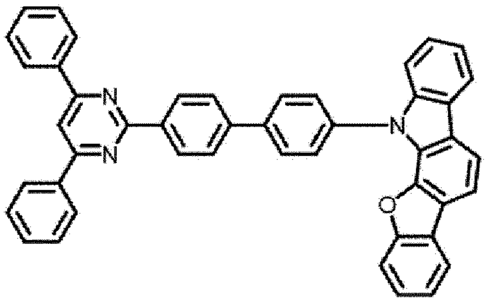

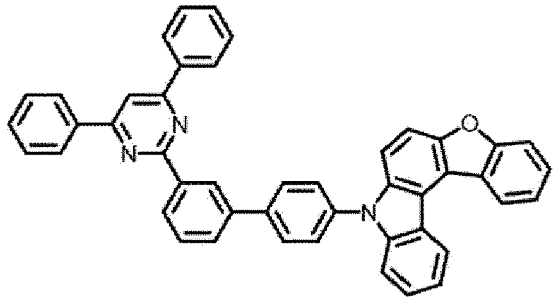

[Formula 229]

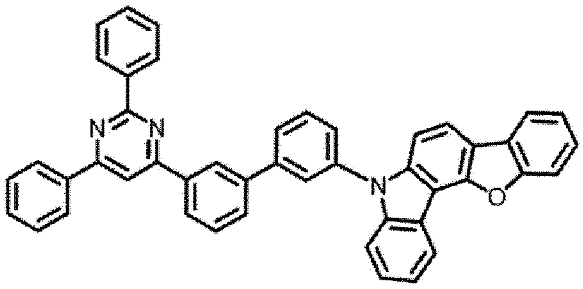

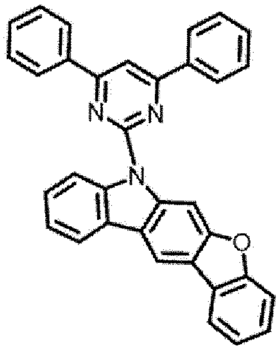

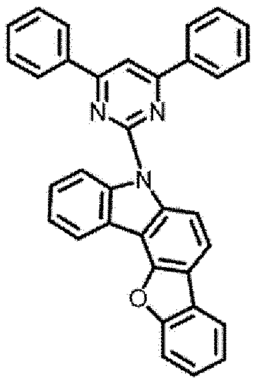

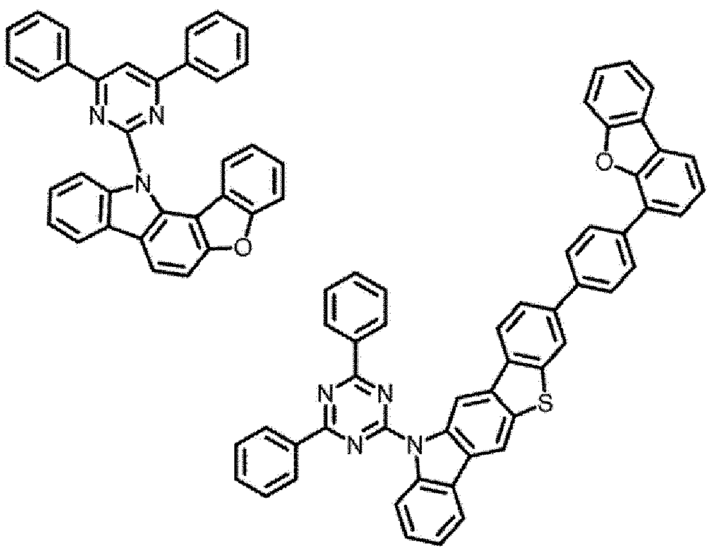

[Formula 230]

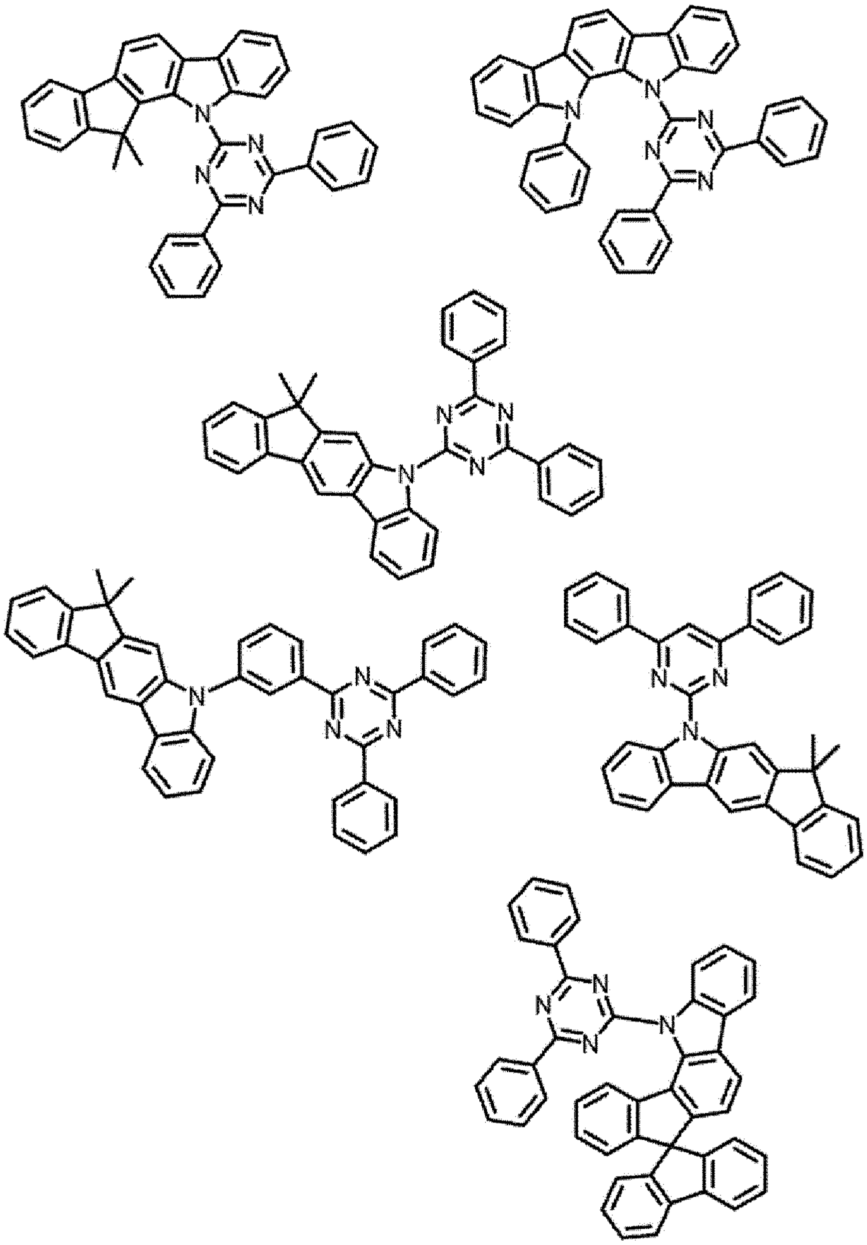

[Formula 231]

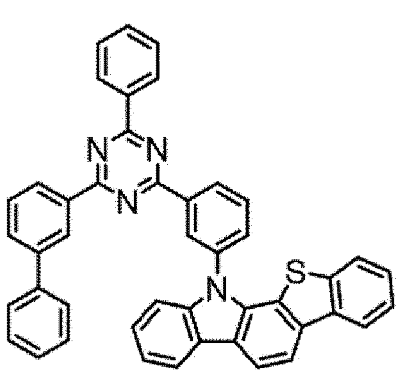

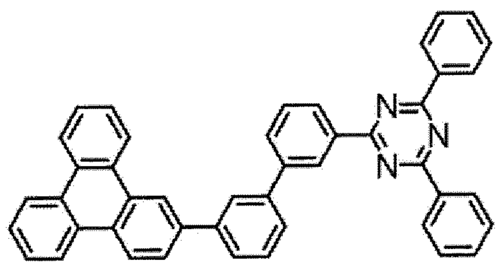

Relationship between Compound M3, Compound M2 and Compound M1 in Emitting Layer

In the organic EL device according to the exemplary embodiment, the lowest singlet energy $S_1(M2)$ of the compound M2 and the lowest singlet energy $S_1(M1)$ of the compound M1 preferably satisfy a relationship of a numerical formula (Numerical Formula 1) below.

$$S_1(M2) > S_1(M1) \quad \text{...(Numerical Formula 1)}$$

In the organic EL device according to the exemplary embodiment, the lowest singlet energy $S_1(M2)$ of the compound M2 and the lowest singlet energy $S_1(M3)$ of the compound M3 preferably satisfy a relationship of a numerical formula (Numerical Formula 2) below.

$$S_1(M3) > S_1(M2) \quad \text{...(Numerical Formula 2)}$$

Moreover, the lowest singlet energy $S_1(M3)$ of the compound M3 is preferably larger than the lowest singlet energy $S_1(M1)$ of the compound M1.

$$S_1(M3) > S_1(M1) \quad \text{...(Numerical Formula 2A)}$$

The lowest singlet energy $S_1(M3)$ of the compound M3, the lowest singlet energy $S_1(M2)$ of the compound M2, and the lowest singlet energy $S_1(M1)$ of the compound M1 preferably satisfy a relationship of a numerical formula (Numerical Formula 2B) below.

$$S_1(M3) > S_1(M2) > S_1(M1) \quad \text{...(Numerical Formula 2B)}$$

In the organic EL device according to the exemplary embodiment, an energy gap $T_{77K}(M3)$ at 77K of the compound M3 is preferably larger than an energy gap $T_{77K}(M2)$ at 77K of the compound M2.

In the organic EL device according to the exemplary embodiment, an energy gap $T_{77K}(M2)$ at 77K of the compound M2 is preferably larger than an energy gap $T_{77K}(M1)$ at 77K of the compound M1.

In the organic EL device according to the exemplary embodiment, the compound M3, the compound M2, and the compound M1 preferably satisfy a relationship of a numerical formula (Numerical Formula 5A) below.

$$T_{77K}(M3) > T_{77K}(M2) > T_{77K}(M1) \quad \text{...(Numerical Formula 5A)}$$

It is preferable that mainly the fluorescent compound M1 emits light in the emitting layer when the organic EL device of the exemplary embodiment emits light.

The organic EL device according to the exemplary embodiment preferably emits red light or green light.

Content Ratios of Compounds in Emitting Layer

Content ratios of the compound M3, the compound M2, and the compound M1 in the emitting layer preferably fall, for instance, within ranges below.

The content ratio of the compound M3 is preferably in a range from 10 mass% to 80 mass%.

The content ratio of the compound M2 is preferably in a range from 10 mass% to 80 mass%, more preferably in a range from 10 mass% to 60 mass%, and still more preferably in a range from 20 mass% to 60 mass%.

The content ratio of the compound M1 is preferably in a range from 0.01 mass% to 10 mass%, more preferably in a range from 0.01 mass% to 5 mass%, and still more preferably in a range from 0.01 mass% to 1 mass%.

The upper limit of a total of the content ratios of the compound M3, the compound M2, and the compound M1 in the emitting layer is 100 mass%. It should be noted that the emitting layer of the exemplary embodiment may further contain material(s) other than the compounds M3, M2 and M1.

The emitting layer may contain a single type of the compound M3 or may contain two or more types of the compound M3. The emitting layer may contain a single type of the compound M2 or may contain two or more types of the compound M2. The emitting layer may contain a single type of the compound M1 or may contain two or more types

of the compound M1.

Fig. 5 illustrates an example of a relationship between energy levels of the compound M3, the compound M2, and the compound M1 in the emitting layer. In Fig. 5, S0 represents a ground state. S1(M1) represents the lowest singlet state of the compound M1. T1(M1) represents the lowest triplet state of the compound M1. S1(M2) represents the lowest singlet state of the compound M2. T1(M2) represents the lowest triplet state of the compound M2. S1(M3) represents the lowest singlet state of the compound M3. T1(M3) represents the lowest triplet state of the compound M3. A dashed arrow directed from S1(M2) to S1(M1) in Fig. 5 represents Förster energy transfer from the lowest singlet state of the compound M2 to the lowest singlet state of the compound M1.

As shown in Fig. 5, when a compound having a small ΔST(M2) is used as the compound M2, inverse intersystem crossing from the lowest triplet state T1(M2) to the lowest singlet state S1(M2) can be caused by a heat energy. Subsequently, Förster energy transfer from the lowest singlet state S1(M2) of the compound M2 to the compound M1 occurs to generate the lowest singlet state S1(M1). Consequently, fluorescence from the lowest singlet state S1(M1) of the compound M1 can be observed. It is inferred that the internal quantum efficiency can be theoretically raised up to 100% also by using delayed fluorescence by the TADF mechanism.

The organic EL device according to the fourth exemplary embodiment contains in the emitting layer the compound of the first exemplary embodiment as the compound M2, the compound M1 having the lowest singlet energy smaller than that of the compound M2, and the compound M3 having the lowest singlet energy larger than that of the compound M2. Since the organic EL device according to the fourth exemplary embodiment contains the compound having a high PLQY according to the first exemplary embodiment (the compound M2), the fourth exemplary embodiment provides a high-performance organic EL device that achieves at least one of high efficiency or a long lifetime.

## Fifth Exemplary Embodiment

An arrangement of an organic EL device according to a fifth exemplary embodiment will be described below. In the description of the fifth exemplary embodiment, the same components as those in the third or fourth exemplary embodiment are denoted by the same reference signs and names to simplify or omit explanation of the components. In the fifth exemplary embodiment, the same materials and compounds as described in the third or fourth exemplary embodiment are usable, unless otherwise specified.

The organic EL device according to the fifth exemplary embodiment is different from the organic EL device according to the third or fourth exemplary embodiment in that the emitting layer contains the compounds M2 and M3 but does not contain the compound M1. The fifth exemplary embodiment is the same as the third or fourth exemplary embodiment in other respects.

Specifically, in the fifth exemplary embodiment, the emitting layer contains the compound M2 and the compound M3.

In this arrangement, it is preferable that the compound M3 is a host material and the compound M2 is a dopant material.

In the exemplary embodiment, in a case where the emitting layer contains the compound of the first exemplary embodiment, the emitting layer preferably does not contain a phosphorescent metal complex and also preferably does not contain a metal complex other than the phosphorescent metal complex.

### Compound M2

The compound M2 is the compound of the first exemplary embodiment.

The compound M2 is preferably a delayed fluorescence compound.

### Compound M3

The compound M3 is the same as the compound M3 described in the fourth exemplary embodiment.

### Relationship between Compound M2 and Compound M3 in Emitting Layer

In the organic EL device according to the exemplary embodiment, the lowest singlet energy $S_1$(M2) of the compound M2 and the lowest singlet energy $S_1$(M3) of the compound M3 preferably satisfy a relationship of a numerical formula (Numerical Formula 2) below.

$$S_1(M3) > S_1(M2) \qquad \text{...(Numerical Formula 2)}$$

An energy gap $T_{77K}$(M3) at 77K of the compound M3 is preferably larger than an energy gap $T_{77K}$(M2) at 77K of the compound M2.

Fig. 6 is an illustration for explaining the principle of light emission according to an exemplary embodiment of the invention.

In Fig. 6, S0 represents a ground state. S1(M2) represents the lowest singlet state of the compound M2. T1(M2) represents the lowest triplet state of the compound M2. S1(M3) represents the lowest singlet state of the compound M3. T1(M3) represents the lowest triplet state of the compound M3.

As illustrated in Fig. 6, when a compound having a small ΔST(M2) is used as the compound M2, inverse intersystem crossing from the lowest triplet state T1(M2) to the lowest singlet state S1(M2) can be caused by a heat energy.

With use of inverse intersystem crossing occurring in the compound M2, for instance, light emission as described in (i) or (ii) below can be observed.

(i) Light emission from the lowest singlet state S1(M2) of the compound M2 can be observed when the emitting layer does not contain a fluorescent dopant with the lowest singlet state S1 smaller than the lowest singlet state S1(M2) of the compound M2.

(ii) Light emission from a fluorescent dopant can be observed when the emitting layer contains the fluorescent dopant with the lowest singlet state S1 smaller than the lowest singlet state S1(M2) of the compound M2 (i.e., the fluorescent compound M1 in the third or fourth exemplary embodiment).

In the organic EL device of the fifth exemplary embodiment, light emission described in the above (i) can be observed. In the organic EL device of the third or fourth exemplary embodiment, light emission described in the above (ii) can be observed.

Content Ratios of Compounds in Emitting Layer

For instance, content ratios of the compound M2 and the compound M3 in the emitting layer preferably fall within ranges shown below.

The content ratio of the compound M2 is preferably in a range from 10 mass% to 90 mass%, more preferably in a range from 10 mass% to 80 mass%, still more preferably in a range from 10 mass% to 60 mass%, and still further more preferably in a range from 20 mass% to 60 mass%.

The content ratio of the compound M3 is preferably in a range from 10 mass% to 90 mass%.

The upper limit of a total of the content ratios of the compound M2 and the compound M3 in the emitting layer is 100 mass%.

The emitting layer may contain a single type of the compound M2 or may contain two or more types of the compound M2. The emitting layer may contain a single type of the compound M3 or may contain two or more types of the compound M3.

The organic EL device according to the fifth exemplary embodiment contains, in the emitting layer, the compound of the first exemplary embodiment as the compound M2 and the compound M3 having the lowest singlet energy larger than that of the compound M2. Since the organic EL device according to the fifth exemplary embodiment contains the compound having a high PLQY according to the first exemplary embodiment (the compound M2), the fifth exemplary embodiment provides a high-performance organic EL device that achieves at least one of high efficiency or a long lifetime.

Sixth Exemplary Embodiment

Electronic Device

An electronic device according to a sixth exemplary embodiment is installed with any one of the organic EL devices according to the above exemplary embodiments. Examples of the electronic device include a display device and a light-emitting unit. Examples of the display device include a display component (e.g., an organic EL panel module), TV, mobile phone, tablet and personal computer. Examples of the light-emitting unit include an illuminator and a vehicle light.

Modification of Embodiment(s)

The scope of the invention is not limited by the above exemplary embodiments but includes any modification and improvement as long as such modification and improvement are compatible with the invention.

For instance, the emitting layer is not limited to a single layer, but may be provided by laminating a plurality of emitting layers. When the organic EL device has the plurality of emitting layers, it is only required that at least one of

the emitting layers satisfies the conditions described in the above exemplary embodiments. For instance, the rest of the emitting layers may be a fluorescent emitting layer or a phosphorescent emitting layer with use of emission caused by electron transfer from the triplet excited state directly to the ground state.

In a case where the organic EL device includes a plurality of emitting layers, these emitting layers may be mutually adjacently provided, or may form a so-called tandem organic EL device, in which a plurality of emitting units are layered via an intermediate layer.

For instance, a blocking layer may be provided adjacent to at least one of a side of the emitting layer close to the anode or a side of the emitting layer close to the cathode. The blocking layer is preferably provided in contact with the emitting layer to block at least any of holes, electrons, or excitons.

For instance, when the blocking layer is provided in contact with the side of the emitting layer close to the cathode, the blocking layer permits transport of electrons, and blocks holes from reaching a layer provided closer to the cathode (e.g., the electron transporting layer) beyond the blocking layer. When the organic EL device includes the electron transporting layer, the blocking layer is preferably interposed between the emitting layer and the electron transporting layer.

When the blocking layer is provided in contact with the side of the emitting layer close to the anode, the blocking layer permits transport of holes and blocks electrons from reaching a layer provided closer to the anode (e.g., the hole transporting layer) beyond the blocking layer. When the organic EL device includes the hole transporting layer, the blocking layer is preferably interposed between the emitting layer and the hole transporting layer.

Alternatively, the blocking layer may be provided adjacent to the emitting layer so that the excitation energy does not leak out from the emitting layer toward neighboring layer(s). The blocking layer blocks excitons generated in the emitting layer from being transferred to a layer(s) (e.g., the electron transporting layer and the hole transporting layer) closer to the electrode(s) beyond the blocking layer.

The emitting layer is preferably bonded with the blocking layer.

Specific structure, shape and the like of the components in the invention may be designed in any manner as long as an object of the invention can be achieved. Examples

The invention will be described in more detail below with reference to Examples. The scope of the invention is by no means limited to Examples.

Compounds

Structures of a compound represented by the formula (1) used for producing organic EL devices in Examples 1-1 to 1-5, Examples 2-1 to 2-11, Examples 3-1 to 3-24, and Examples 4-1 to 4-25 are shown below.

[Formula 232]

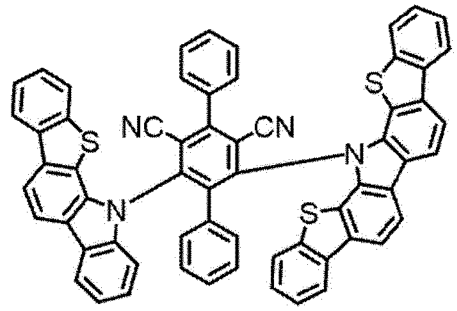

**A-1**

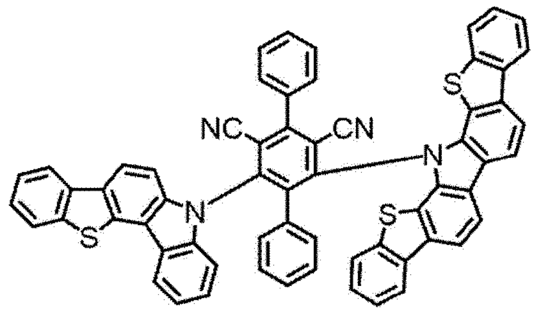

**A-2**

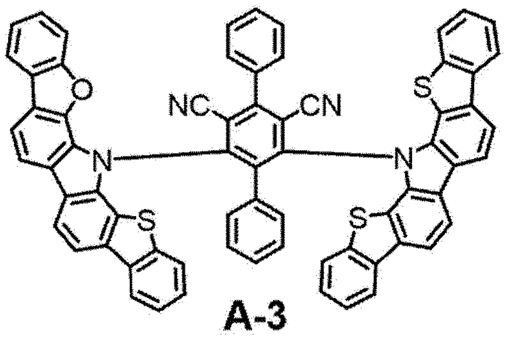

**A-3**

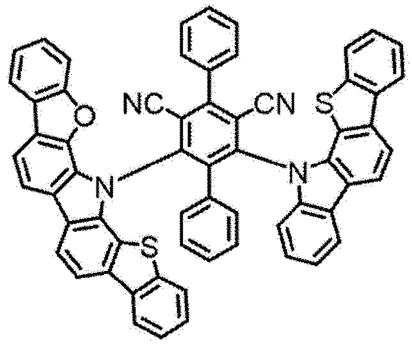

**A-4**

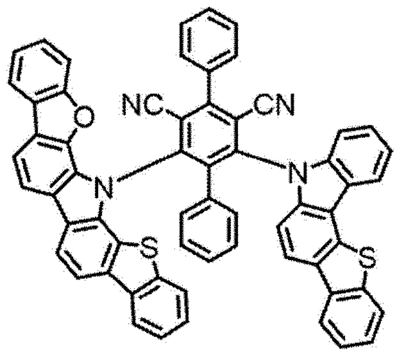

**A-5**

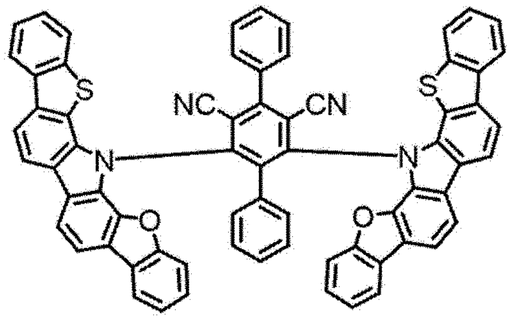

**A-6**

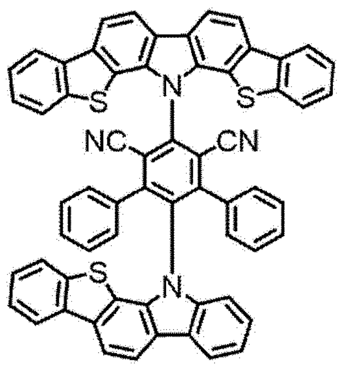

**A-7**

[Formula 233]

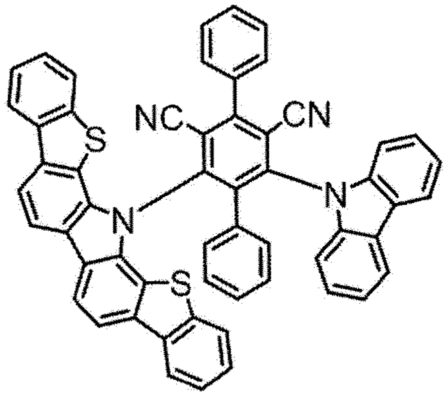

A-9

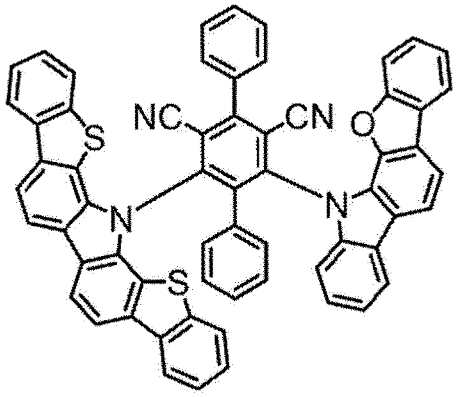

A-10

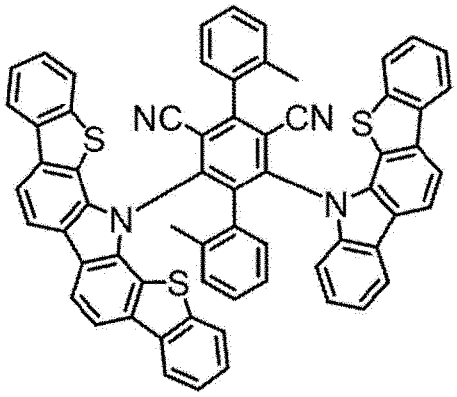

A-11

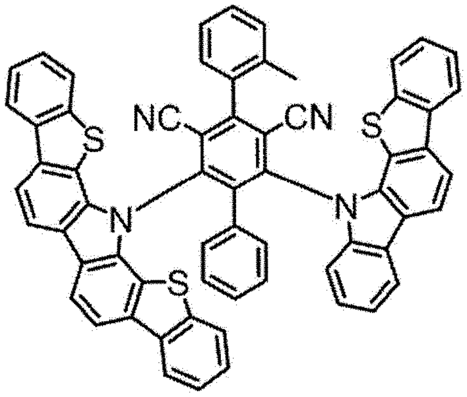

A-12

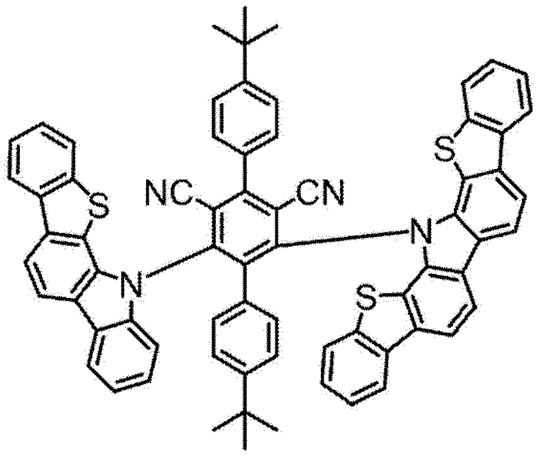

A-13

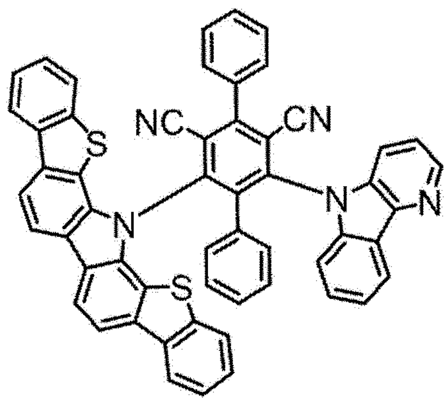

A-14

[Formula 234]

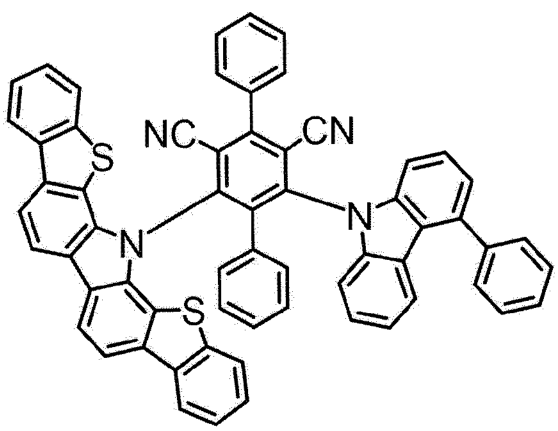

A-15

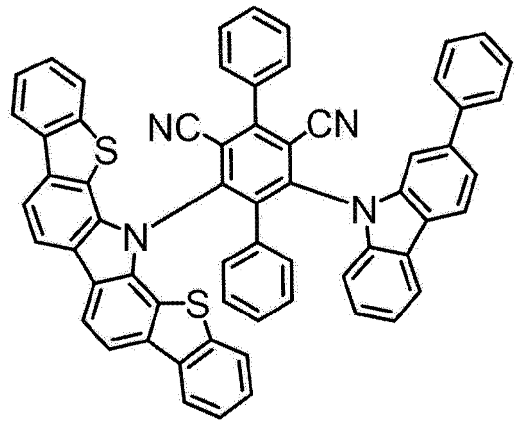

A-16

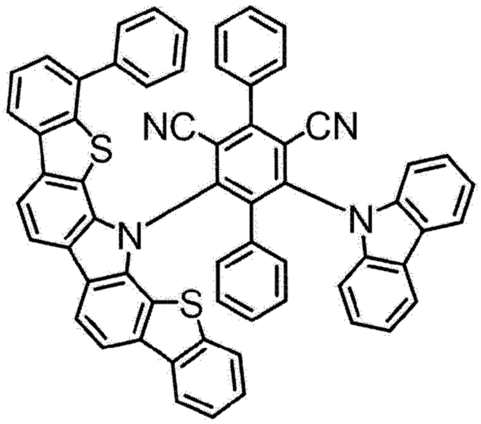

A-17

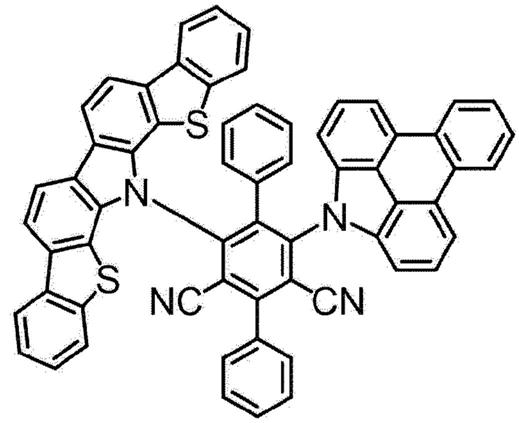

A-19

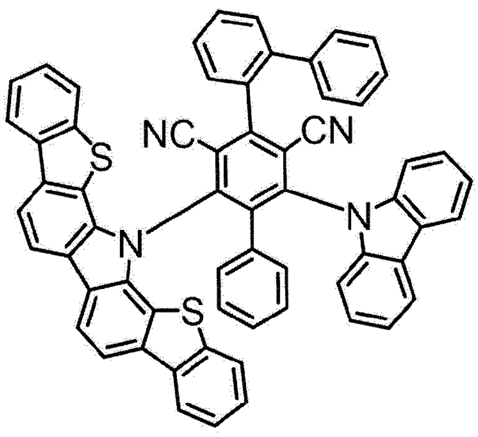

A-20

[Formula 235]

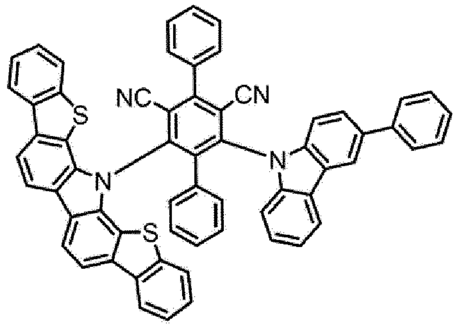

A-21

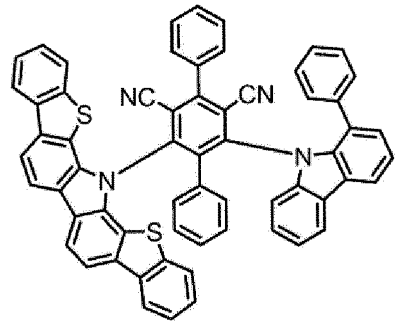

A-22

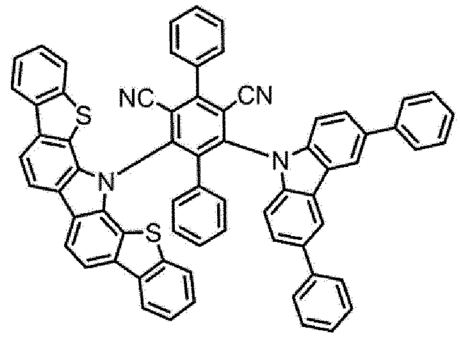

A-23

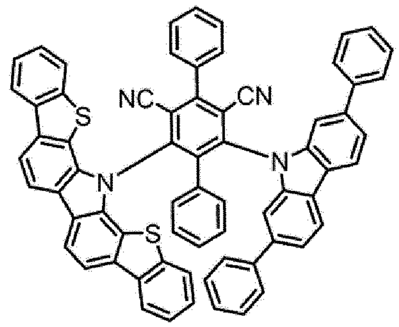

A-24

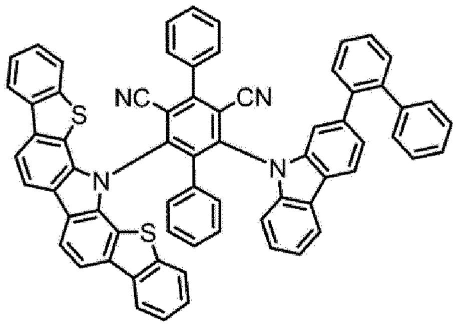

A-25

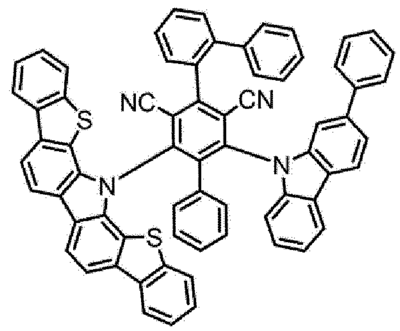

A-26

[Formula 236]

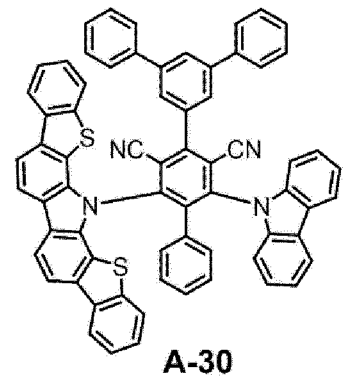

**A-30**

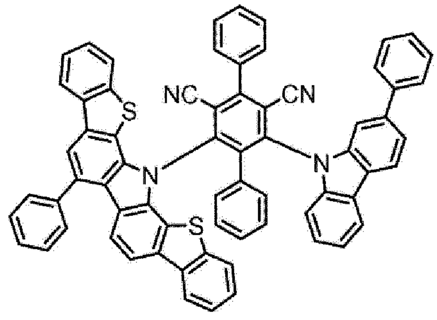

**A-31**

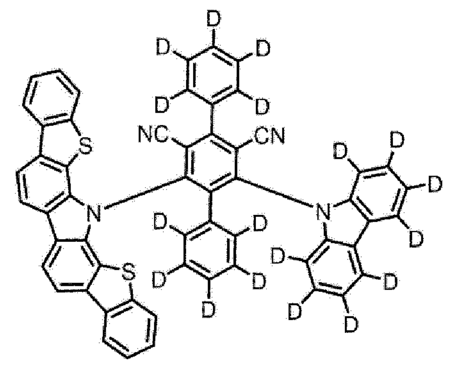

**A-32**

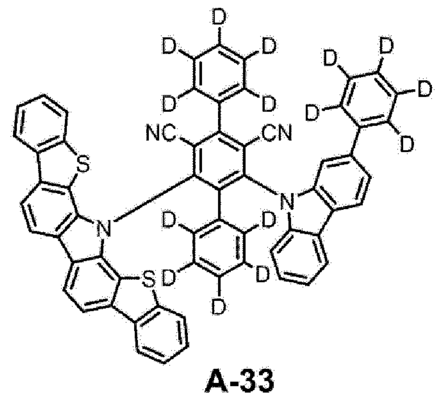

**A-33**

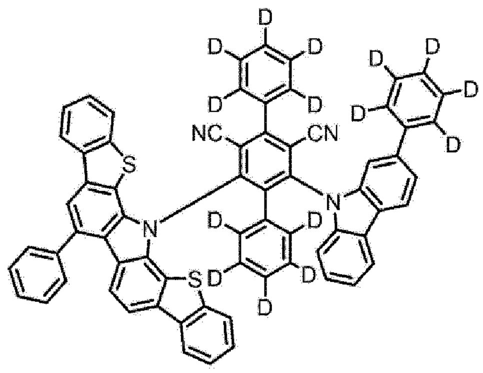

**A-34**

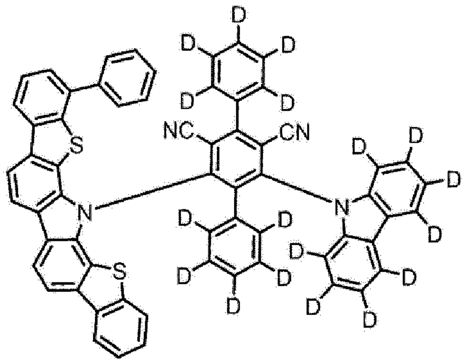

**A-35**

[Formula 237]

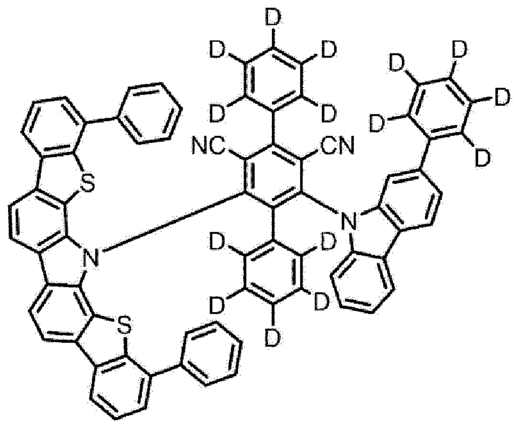

A-36

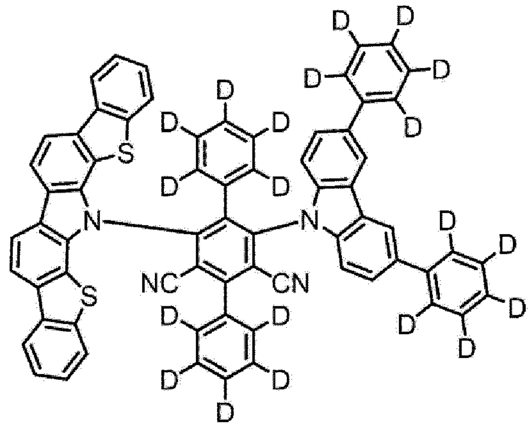

A-37

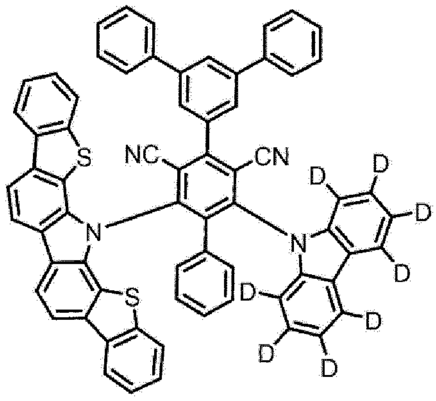

A-38

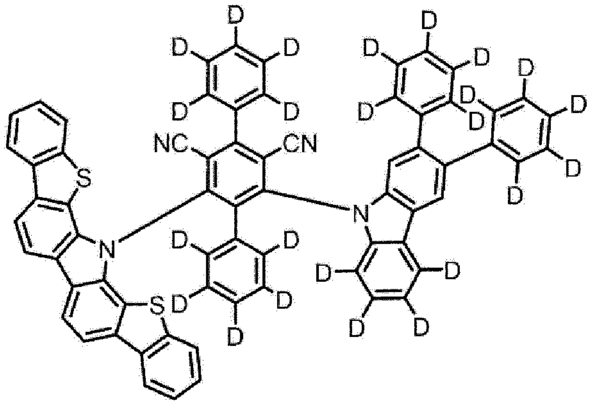

A-39

[Formula 238]

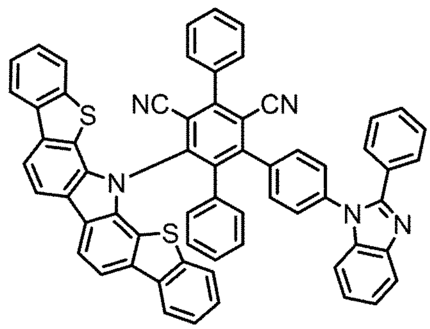

A-40

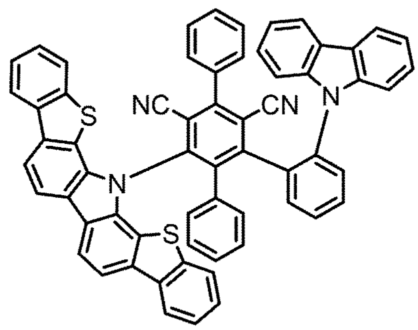

A-41

[Formula 239]

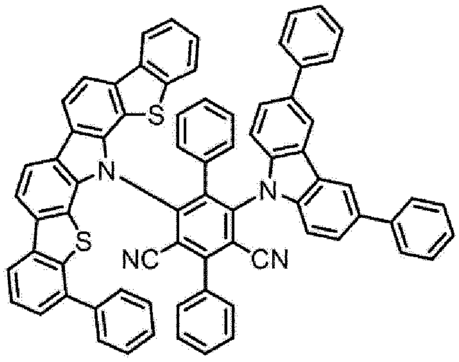

A-42

Structures of comparative compounds used for producing organic EL devices in Comparative 1-1, Comparative 2-1, Comparative 3-1, and Comparative 4-1 are shown below.

[Formula 240]

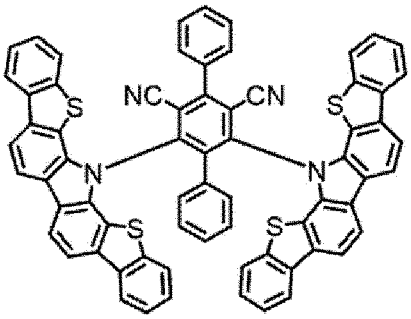

Ref-1

Structures of other compounds used for producing the organic EL devices in Examples 1-1 to 1-5, Examples 2-1 to 2-11, Examples 3-1 to 3-24, Examples 4-1 to 4-25, Comparative 1-1, Comparative 2-1, Comparative 3-1, and Comparative 4-1 are shown below.

[Formula 241]

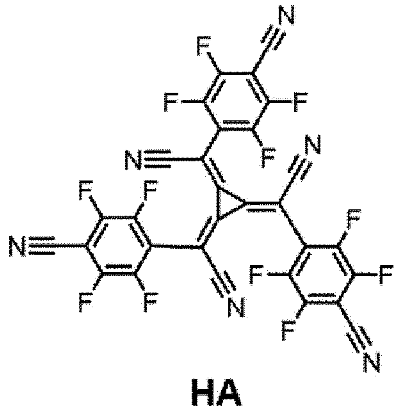

HA

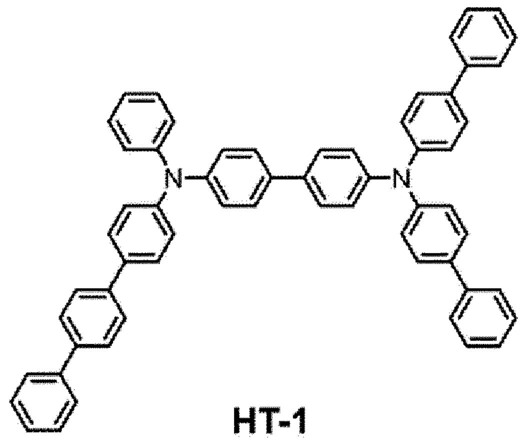

HT-1

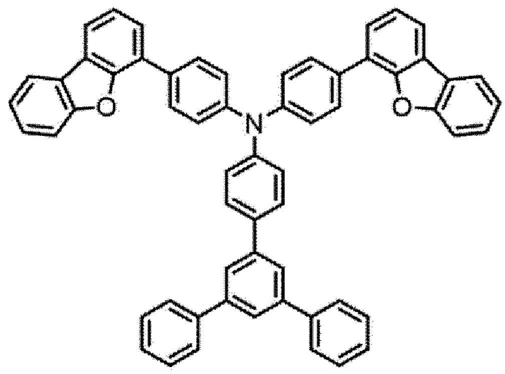

HT-2

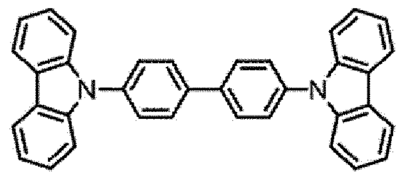

CBP

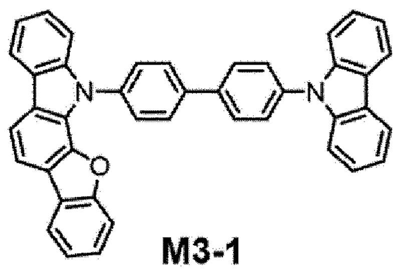

M3-1

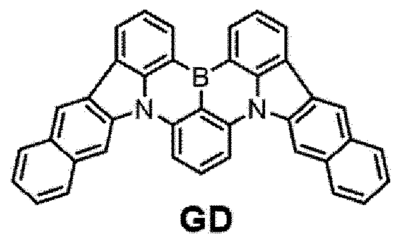

GD

[Formula 242]

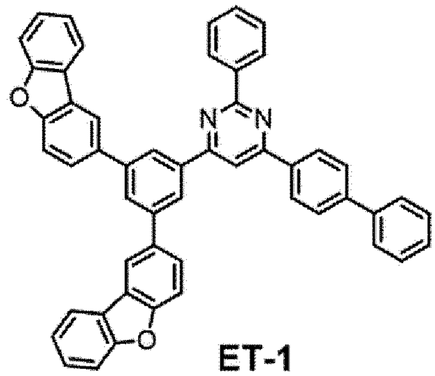

ET-1

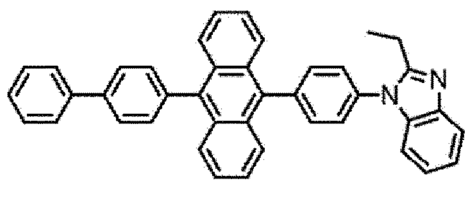

ET-2

[Formula 243]

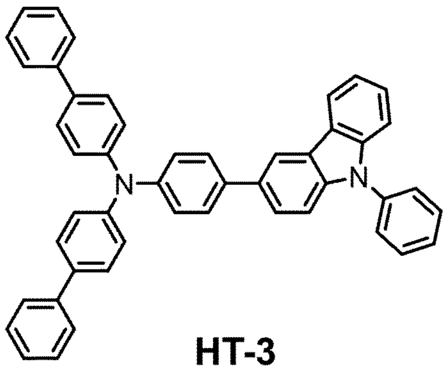

HT-3

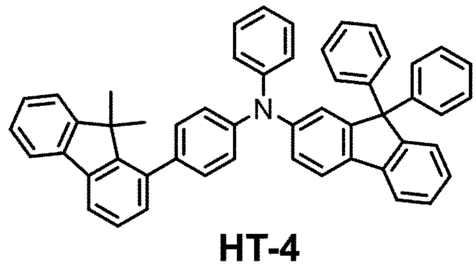

HT-4

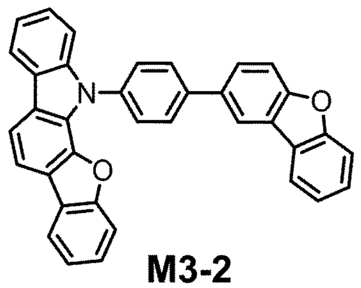

M3-2

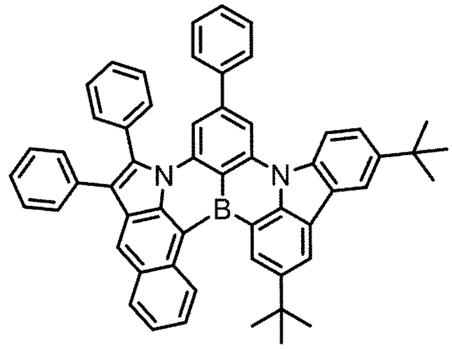

GD2

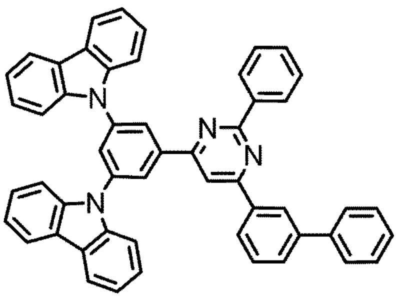

ET-3

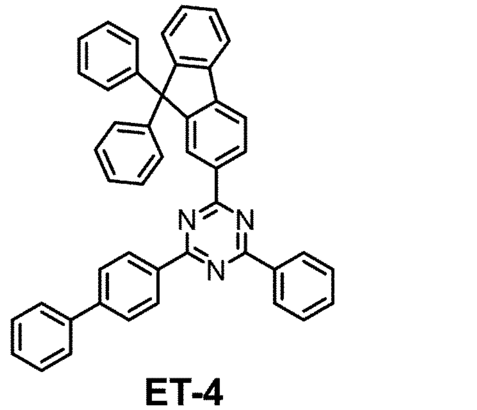

ET-4

Production (1) of Organic EL Devices

Organic EL devices were produced and evaluated as follows.

Example 1-1

A glass substrate (size: 25 mm × 75 mm × 1.1 mm thick, produced by Geomatec Co., Ltd.) having an ITO transparent electrode (anode) was ultrasonic-cleaned in isopropyl alcohol for five minutes, and then UV-ozone-cleaned for one minute. The film thickness of ITO was 130 nm.

After the glass substrate having the transparent electrode line was cleaned, the glass substrate was mounted on a substrate holder of a vacuum deposition apparatus. Firstly, a compound HT-1 and a compound HA were co-deposited on a surface of the glass substrate where the transparent electrode line was provided in a manner to cover the transparent electrode, thereby forming a 10-nm-thick hole injecting layer. Concentrations of the compound HT-1 and the compound HA in the hole injecting layer were 97 mass% and 3 mass%, respectively.

The compound HT-1 was then vapor-deposited on the hole injecting layer to form a 110-nm-thick first hole transporting layer.

A compound HT-2 was then vapor-deposited on the first hole transporting layer to form a 5-nm-thick second hole transporting layer.

A compound CBP was then vapor-deposited on the second hole transporting layer to form a 5-nm-thick electron blocking layer.

Next, a compound M3-1 as the compound M3 and a compound A-1 as the compound M2 were co-deposited on the electron blocking layer to form a 25-nm-thick emitting layer. Concentrations of the compound M3-1 and the compound A-1 in the emitting layer were 75 mass% and 25 mass%, respectively.

Next, a compound ET-1 was vapor-deposited on the emitting layer to form a 5-nm-thick hole blocking layer.

A compound ET-2 was then vapor-deposited on the hole blocking layer to form a 50-nm-thick electron transporting layer.

Lif was then vapor-deposited on the electron transporting layer to form a 1-nm-thick electron injecting layer.

Subsequently, metal aluminum (Al) was vapor-deposited on the electron injecting layer to form an 80-nm-thick metal Al cathode.

A device arrangement of the organic EL device in Example 1-1 is roughly shown as follows.

ITO(130) / HT-1:HA(10,97%:3%) / HT-1(110) / HT-2(5) / CBP(5) / M3-1:A-1(25,75%:25%) / ET-1(5) / ET-2(50) / LiF(1) / Al(80)

Numerals in parentheses represent a film thickness (unit: nm).

The numerals (97%:3%) represented by percentage in the same parentheses indicate a ratio (mass%) between the compound HT-1 and the compound HA in the hole injecting layer. The numerals (75%:25%) represented by percentage in the same parentheses indicate a ratio (mass%) between the compound M3-1 and the compound A-1 in the emitting layer. Similar notations apply to the description below.

Examples 1-2 to 1-5

Organic EL devices in Examples 1-2 to 1-5 were produced in the same manner as in Example 1-1 except that the compound A-1 used as the compound M2 in the emitting layer of Example 1-1 was replaced by compounds as the compound M2 shown in Table 1.

Comparative 1-1

An organic EL device in Comparative 1-1 was produced in the same manner as in Example 1-1 except that the compound A-1 used as the compound M2 in the emitting layer of Example 1-1 was replaced by a compound as the compound M2 shown in Table 1.

Example 2-1

An organic EL device in Example 2-1 was produced in the same manner as in Example 1-1 except that, in place of the emitting layer of Example 1-1, the compound M3-1 as the compound M3, the compound A-1 as the compound M2, and the compound GD as the compound M1 were co-deposited to form a 25-nm thick emitting layer in which concentrations of the compound M3-1, the compound A-1, and the compound GD were 74 mass%, 25 mass%, and 1 mass%, respectively.

A device arrangement of the organic EL device in Example 2-1 is roughly shown as follows.

ITO(130) / HT-1:HA(10,97%:3%) / HT-1(110) / HT-2(5) / CBP(5) / M3-1:A-1:GD(25,74%:25%:1%) / ET-1(5) / ET-2(50) / LiF(1) / Al(80)

Examples 2-2 to 2-11

Organic EL devices in Examples 2-2 to 2-11 were produced in the same manner as in Example 2-1 except that the compound A-1 used as the compound M2 in the emitting layer of Example 2-1 was replaced by compounds as the compound M2 shown in Table 2.

Comparative 2-1

An organic EL device in Comparative 2-1 was produced in the same manner as in Example 2-1 except that the compound A-1 used as the compound M2 in the emitting layer of Example 2-1 was replaced by a compound as the compound M2 shown in Table 2.

Evaluation (1) of Organic EL Devices

The organic EL devices produced were evaluated as follows. Tables 1 and 2 show evaluation results. It should be noted that a comparative compound Ref-1 used in Comparatives 1-1 and 2-1 does not correspond to the compound M2, but is listed in the same column as the compound M2 for convenience. Tables 1 and 2 also show the evaluation results of the compounds used in the emitting layer in each Example.

Lifetime LT95

Voltage was applied to the produced organic EL device such that a current density was 50 mA/cm$^2$, where a time (LT95 (unit: hr)) elapsed before a luminance intensity was reduced to 95% of the initial luminance intensity was measured as a lifetime. The luminance intensity was measured with a spectroradiometer CS-2000 (manufactured by Konica Minolta, Inc.). Table 1 shows "LT95 (relative value)" (unit: %).

LT95 (relative value) (unit: %) shown in Table 1 was calculated based on the measurement value of LT95 in each Example (Examples 1-1 to 1-5 and Comparative 1-1) according to a numerical formula (Numerical Formula 1X) below.

LT95 (relative value) = (LT95 of each Example / LT95 of Comparative 1-1) $\times$ 100 (Numerical Formula 1X)

External Quantum Efficiency EQE

Voltage was applied on each of the produced organic EL device such that a current density was 10.00 mA/cm$^2$, where spectral radiance spectrum was measured by a spectroradiometer CS-2000 (manufactured by Konica Minolta, Inc.). The external quantum efficiency EQE (unit: %) was calculated based on the obtained spectral radiance spectra, assuming that the spectra was provided under a Lambertian radiation. Table 2 shows "EQE (relative value)" (unit: %).

EQE (relative value) (unit: %) shown in Table 2 was calculated based on the measurement value of EQE in each Example (Examples 2-1 to 2-11 and Comparative 2-1) according to a numerical formula (Numerical Formula 2X) below.

EQE (relative value) = (EQE of each Example / EQE of Comparative 2-1) $\times$ 100 (Numerical Formula 2X)

Maximum Peak Wavelength $\lambda_p$ and Full Width at Half Maximum (FWHM)

Voltage was applied to each organic EL device so that a current density of the organic EL device was 10.00 mA/cm$^2$, where spectral radiance spectrum was measured by a spectroradiometer CS-2000 (produced by Konica Minolta, Inc.). The maximum peak wavelength $\lambda_p$ (unit: nm) and full width at half maximum (FWHM, unit: nm) were calculated based on the obtained spectral-radiance spectra. FWHM is an abbreviation of Full Width at Half Maximum.

CIE1931 Chromaticity

Voltage was applied to each organic EL device so that a current density of the organic EL device was 10.00 mA/cm$^2$, where coordinates of CIE1931 chromaticity (x, y) were measured by a spectroradiometer CS-2000 (produced by Konica Minolta, Inc.).

Table 1

| | Compound M2 | | | | | Compound M3 | | | Device Evaluation | | | | |
|---|---|---|---|---|---|---|---|---|---|---|---|---|---|
| | Name | λ [nm] | PLQY | $S_1$ [eV] | ΔST [eV] | Name | $S_1$ [eV] | $T_{77K}$ [eV] | CIEx | CIEy | LT95 (relative value) [%] | λp [nm] | FWHM [nm] |
| Ex.1-1 | A-1 | 498 | 0.90 | 2.58 | < 0.01 | M3-1 | 3.42 | 2.89 | 0.367 | 0.592 | 504 | 535 | 82 |
| Ex.1-2 | A-2 | 496 | 0.96 | 2.62 | < 0.01 | M3-1 | 3.42 | 2.89 | 0.365 | 0.590 | 112 | 534 | 84 |
| Ex.1-3 | A-3 | 493 | 0.99 | 2.52 | < 0.01 | M3-1 | 3.42 | 2.89 | 0.341 | 0.603 | 216 | 528 | 80 |
| Ex.1-4 | A-4 | 491 | 0.94 | 2.67 | < 0.01 | M3-1 | 3.42 | 2.89 | 0.343 | 0.589 | 259 | 531 | 87 |
| Ex.1-5 | A-7 | 490 | 0.91 | 2.75 | < 0.01 | M3-1 | 3.42 | 2.89 | 0.370 | 0.571 | 137 | 542 | 93 |
| Comp.1-1 | Ref-1 | 504 | 0.87 | 2.52 | < 0.01 | M3-1 | 3.42 | 2.89 | 0.395 | 0.582 | 100 | 542 | 80 |

Table 2

| | Compound M2 | | | | | Compound M3 | Compound M1 | | | CIEx | CIEy | EQE (relative value) [%] | λp [nm] | FWHM [nm] |
|---|---|---|---|---|---|---|---|---|---|---|---|---|---|---|
| | Name | λ [nm] | PLQY | $S_1$ [eV] | ΔST [eV] | Name | Name | $S_1$ [eV] | $T_{77K}$ [eV] | | | | | |
| Ex.2-1 | A-1 | 498 | 0.90 | 2.58 | < 0.01 | M3-1 | GD | 2.45 | 2.18 | 0.295 | 0.639 | 107 | 516 | 40.0 |
| Ex.2-2 | A-3 | 493 | 0.99 | 2.52 | < 0.01 | M3-1 | GD | 2.45 | 2.18 | 0.257 | 0.665 | 121 | 515 | 33.2 |
| Ex.2-3 | A-4 | 491 | 0.94 | 2.67 | < 0.01 | M3-1 | GD | 2.45 | 2.18 | 0.238 | 0.672 | 110 | 514 | 31.0 |
| Ex.2-4 | A-5 | 484 | 0.98 | 2.70 | < 0.01 | M3-1 | GD | 2.45 | 2.18 | 0.225 | 0.677 | 103 | 514 | 30.0 |
| Ex.2-5 | A-6 | 475 | 0.95 | 2.69 | < 0.01 | M3-1 | GD | 2.45 | 2.18 | 0.212 | 0.686 | 115 | 515 | 30.0 |
| Ex.2-6 | A-9 | 494 | 0.90 | 2.65 | < 0.01 | M3-1 | GD | 2.45 | 2.18 | 0.230 | 0.676 | 122 | 513 | 29.0 |
| Ex.2-7 | A-10 | 489 | 0.90 | 2.69 | < 0.01 | M3-1 | GD | 2.45 | 2.18 | 0.244 | 0.668 | 138 | 514 | 30.0 |
| Ex.2-8 | A-11 | 495 | 0.85 | 2.65 | < 0.01 | M3-1 | GD | 2.45 | 2.18 | 0.259 | 0.665 | 122 | 515 | 32.0 |
| Ex.2-9 | A-12 | 499 | 0.87 | 2.58 | < 0.01 | M3-1 | GD | 2.45 | 2.18 | 0.291 | 0.645 | 114 | 515 | 38.0 |
| Ex.2-10 | A-13 | 496 | 100 | 2.58 | < 0.01 | M3-1 | GD | 2.45 | 2.18 | 0.227 | 0.677 | 123 | 513 | 29.0 |
| Ex.2-11 | A-14 | 505 | 0.99 | 2.64 | < 0.01 | M3-1 | GD | 2.45 | 2.18 | 0.290 | 0.643 | 112 | 514 | 36.0 |
| Comp.2-1 | Ref-1 | 504 | 0.87 | 2.52 | < 0.01 | M3-1 | GD | 2.45 | 2.18 | 0.368 | 0.597 | 100 | 522 | 82.5 |

Production (2) of Organic EL Devices

Example 3-1

A glass substrate (size: 25 mm × 75 mm × 1.1 mm thick, produced by Geomatec Co., Ltd.) having an ITO transparent electrode (anode) was ultrasonic-cleaned in isopropyl alcohol for five minutes, and then UV-ozone-cleaned for one minute. The film thickness of ITO was 130 nm.

After the glass substrate having the transparent electrode line was cleaned, the glass substrate was mounted on a substrate holder of a vacuum deposition apparatus. Firstly, a compound HT-3 and the compound HA were co-deposited on a surface of the glass substrate where the transparent electrode line was provided in a manner to cover the transparent electrode, thereby forming a 10-nm-thick hole injecting layer. Concentrations of the compound HT-3 and the compound HA in the hole injecting layer were 97 mass% and 3 mass%, respectively.

The compound HT-3 was then vapor-deposited on the hole injecting layer to form a 90-nm-thick first hole transporting layer.

A compound HT-4 was then vapor-deposited on the first hole transporting layer to form a 30-nm-thick second hole transporting layer.

Next, a compound M3-2 as the compound M3, and the compound A-1 as the compound M2 were co-deposited on the second hole transporting layer to form a 25-nm-thick emitting layer. Concentrations of the compound M3-2 and the compound A-1 in the emitting layer were 75 mass% and 25 mass%, respectively.

Next, a compound ET-3 was vapor-deposited on the emitting layer to form a 5-nm-thick hole blocking layer.

A compound ET-4 and a compound Liq were then co-deposited on the hole blocking layer to form a 50-nm-thick electron transporting layer. Concentrations of the compound ET-4 and the compound Liq in the electron transporting layer were 50 mass% and 50 mass%, respectively. Liq is an abbreviation of (8-quinolinolato)lithium ((8-Quinolinolato)lithium).

Next, ytterbium (Yb) was vapor-deposited on the electron transporting layer to form a 1-nm-thick electron injecting layer.

Subsequently, metal aluminum (Al) was vapor-deposited on the electron injecting layer to form an 80-nm-thick metal Al cathode.

A device arrangement of the organic EL device in Example 3-1 is roughly shown as follows.

ITO(130) / HT-3:HA(10,97%:3%) / HT-3(90) / HT-4(30) / M3-2:A-1(25,75%:25%) / ET-3(5) / ET-4:Liq(50,50%:50%) / Yb(1) / Al(80)

Examples 3-2 to 3-20

Organic EL devices in Examples 3-2 to 3-20 were produced in the same manner as in Example 3-1 except that the compound A-1 used as the compound M2 in the emitting layer of Example 3-1 was replaced by compounds as the compound M2 shown in Table 3.

Examples 3-21 to 3-24

Organic EL devices in Examples 3-21 to 3-24 were produced in the same manner as in Example 3-1 except that the compound A-1 used as the compound M2 in the emitting layer of Example 3-1 was replaced by compounds as the compound M2 shown in Table 4.

Comparative 3-1

An organic EL device in Comparative 3-1 was produced in the same manner as in Example 3-1 except that the compound A-1 used as the compound M2 in the emitting layer of Example 3-1 was replaced by a compound as the compound M2 shown in Table 3.

Example 4-1

An organic EL device in Example 4-1 was produced in the same manner as in Example 3-1 except that, in place of the emitting layer of Example 3-1, the compound M3-2 as the compound M3, a compound A-40 as the compound M2, and a compound GD2 as the compound M1 were co-deposited to form a 25-nm thick emitting layer in which concentrations of the compound M3-2, the compound A-40, and the compound GD2 were 74.4 mass%, 25 mass%, and 0.6 mass%, respectively.

A device arrangement of the organic EL device in Example 4-1 is roughly shown as follows.

ITO(130) / HT-3:HA(10,97%:3%) / HT-3(90) / HT-4(30) / M3-2:A-40:GD2(25,74.4%:25%:0.6%) / ET-3(5) / ET-4:Liq(50,50%:50%) / Yb(1) / Al(80)

Example 4-2

An organic EL device in Example 4-2 was produced in the same manner as in Example 4-1 except that the compound A-40 used as the compound M2 in the emitting layer of Example 4-1 was replaced by a compound as the compound M2 shown in Table 5.

Examples 4-3 to 4-25

Organic EL devices in Examples 4-3 to 4-25 were produced in the same manner as in Example 4-1 except that the compound A-40 used as the compound M2 in the emitting layer of Example 4-1 was replaced by compounds as the compound M2 shown in Tables 6 and 7.

Comparative 4-1

An organic EL device in Comparative 4-1 was produced in the same manner as in Example 4-1 except that the compound A-40 used as the compound M2 in the emitting layer of Example 4-1 was replaced by a compound as the compound M2 shown in Table 5.

Evaluation (2) of Organic EL Devices

The organic EL devices produced in Examples 3-1 to 3-24, Examples 4-1 to 4-25, Comparative 3-1, and Comparative 4-1 were evaluated in terms of items shown in Tables 3, 4, 5, 6, and 7 according to the method described in "Evaluation (1) of Organic EL Devices". Tables 3, 4, 5, 6, and 7 show evaluation results.

EQE (relative value) shown in Tables 3 and 4 was calculated based on the measurement value of EQE in each Example (Examples 3-1 to 3-24 and Comparative 3-1) according to a numerical formula (Numerical Formula 3X) below.

$$\text{EQE (relative value)} = (\text{EQE of each Example} / \text{EQE of Comparative 3-1}) \times 100 \qquad \text{(Numerical Formula 3X)}$$

EQE (relative value) shown in Tables 5, 6, and 7 was calculated based on the measurement value of EQE in each Example (Examples 4-1 to 4-25 and Comparative 4-1) according to a numerical formula (Numerical Formula 4X) below.

$$\text{EQE (relative value)} = (\text{EQE of each Example} / \text{EQE of Comparative 4-1}) \times 100 \qquad \text{(Numerical Formula 4X)}$$

Table 3

| | Compound M2 | | | | | Compound M3 | | | Device Evaluation | | | | |
|---|---|---|---|---|---|---|---|---|---|---|---|---|---|
| | Name | $\lambda$ [nm] | PLQY | $S_1$ [eV] | $\Delta ST$ [eV] | Name | $S_1$ [eV] | $T_{77K}$ [eV] | CIEx | CIEy | EQE (relative value) [%] | $\lambda p$ [nm] | FWHM [nm] |
| Ex.3-1 | A-1 | 498 | 0.90 | 2.58 | < 0.01 | M3-2 | 3.43 | 2.89 | 0.369 | 0.597 | 105 | 535 | 78 |
| Ex.3-2 | A-9 | 494 | 0.90 | 2.65 | < 0.01 | M3-2 | 3.43 | 2.89 | 0.333 | 0.605 | 113 | 527 | 78 |
| Ex.3-3 | A-15 | 494 | 0.91 | 2.65 | <0.01 | M3-2 | 3.43 | 2.89 | 0.333 | 0.602 | 113 | 528 | 81 |
| Ex.3-4 | A-16 | 495 | 0.83 | 2.66 | <0.01 | M3-2 | 3.43 | 2.89 | 0.332 | 0.606 | 110 | 527 | 77 |
| Ex.3-5 | A-17 | 498 | 0.99 | 2.64 | <0.01 | M3-2 | 3.43 | 2.89 | 0.327 | 0.605 | 114 | 526 | 79 |
| Ex.3-6 | A-20 | 488 | 0.85 | 2.68 | <0.01 | M3-2 | 3.43 | 2.89 | 0.292 | 0.604 | 123 | 519 | 75 |
| Ex.3-7 | A-21 | 495 | 0.87 | 2.63 | <0.01 | M3-2 | 3.43 | 2.89 | 0.344 | 0.602 | 111 | 528 | 79 |
| Ex.3-8 | A-22 | 492 | 0.87 | 2.67 | <0.01 | M3-2 | 3.43 | 2.89 | 0.301 | 0.601 | 114 | 521 | 78 |
| Ex.3-9 | A-23 | 499 | 0.90 | 2.60 | <0.01 | M3-2 | 3.43 | 2.89 | 0.352 | 0.604 | 116 | 531 | 77 |
| Ex.3-10 | A-24 | 497 | 0.84 | 2.63 | <0.01 | M3-2 | 3.43 | 2.89 | 0.338 | 0.607 | 108 | 527 | 76 |
| Ex.3-11 | A-25 | 493 | 0.78 | 2.66 | <0.01 | M3-2 | 3.43 | 2.89 | 0.308 | 0.606 | 122 | 522 | 76 |
| Ex.3-12 | A-26 | 491 | 0.86 | 2.66 | <0.01 | M3-2 | 3.43 | 2.89 | 0.311 | 0.606 | 108 | 522 | 77 |
| Ex.3-13 | A-31 | 504 | 0.90 | 2.60 | <0.01 | M3-2 | 3.43 | 2.89 | 0.342 | 0.607 | 125 | 529 | 77 |
| Ex.3-14 | A-32 | 493 | 0.92 | 2.67 | <0.01 | M3-2 | 3.43 | 2.89 | 0.324 | 0.606 | 114 | 524 | 77 |
| Ex.3-15 | A-33 | 495 | 0.89 | 2.67 | <0.01 | M3-2 | 3.43 | 2.89 | 0.328 | 0.607 | 113 | 525 | 77 |
| Ex.3-16 | A-34 | 502 | 0.89 | 2.59 | <0.01 | M3-2 | 3.43 | 2.89 | 0.335 | 0.609 | 129 | 526 | 76 |
| Ex.3-17 | A-35 | 498 | 0.95 | 2.65 | <0.01 | M3-2 | 3.43 | 2.89 | 0.321 | 0.607 | 118 | 525 | 78 |
| Ex.3-18 | A-36 | 495 | 0.99 | 2.69 | <0.01 | M3-2 | 3.43 | 2.89 | 0.305 | 0.610 | 117 | 521 | 75 |
| Ex.3-19 | A-38 | 497 | 0.88 | 2.64 | <0.01 | M3-2 | 3.43 | 2.89 | 0.332 | 0.604 | 105 | 527 | 79 |
| Ex.3-20 | A-39 | 495 | 0.90 | 2.64 | <0.01 | M3-2 | 3.43 | 2.89 | 0.327 | 0.607 | 114 | 526 | 77 |
| Comp.3-1 | Ref-1 | 504 | 0.87 | 2.52 | <0.01 | M3-2 | 3.43 | 2.89 | 0.416 | 0.570 | 100 | 548 | 75 |

Table 4

| | Compound M2 | | | | | Compound M3 | | | Device Evaluation | | | | |
|---|---|---|---|---|---|---|---|---|---|---|---|---|---|
| | Name | λ [nm] | PLQY | $S_1$ [eV] | ΔST [eV] | Name | $S_1$ [eV] | $T_{77K}$ [eV] | CIEx | CIEy | EQE (relative value) [%] | λp [nm] | FWHM [nm] |
| Ex.3-21 | A-19 | 492 | 0.85 | 2.66 | <0.01 | M3-2 | 3.43 | 2.89 | 0.338 | 0.604 | 112 | 530 | 79 |
| Ex.3-22 | A-30 | 497 | 0.92 | 2.64 | <0.01 | M3-2 | 3.43 | 2.89 | 0.330 | 0.605 | 107 | 527 | 79 |
| Ex.3-23 | A-37 | 499 | 0.86 | 2.60 | <0.01 | M3-2 | 3.43 | 2.89 | 0.364 | 0.598 | 118 | 535 | 79 |
| Ex.3-24 | A-42 | 500 | 0.84 | 2.60 | <0.01 | M3-2 | 3.43 | 2.89 | 0.359 | 0.601 | 112 | 533 | 77 |
| Comp.3-1 (re-shown) | Ref-1 | 504 | 0.87 | 2.52 | <0.01 | M3-2 | 3.43 | 2.89 | 0.416 | 0.570 | 100 | 548 | 75 |

Table 5

| | Compound M2 | | | | | Compound M3 | Compound M1 | | | Device Evaluation | | | | |
|---|---|---|---|---|---|---|---|---|---|---|---|---|---|---|
| | Name | λ [nm] | PLQY | $S_1$ [eV] | ΔST [eV] | Name | Name | $S_1$ [eV] | $T_{77K}$ [eV] | CIEx | CIEy | EQE (relative value) [%] | λp [nm] | FWHM [nm] |
| Ex.4-1 | A-40 | 489 | 0.82 | 2.71 | < 0.01 | M3-2 | GD2 | 2.39 | 1.99 | 0.209 | 0.693 | 127 | 518 | 28.3 |
| Ex.4-2 | A-41 | 495 | 0.70 | 2.70 | < 0.01 | M3-2 | GD2 | 2.39 | 1.99 | 0.203 | 0.698 | 137 | 518 | 27.4 |
| Comp. 4-1 | Ref-1 | 504 | 0.87 | 2.52 | <0.01 | M3-2 | GD2 | 2.39 | 1.99 | 0.291 | 0.660 | 100 | 521 | 34.0 |

Table 6

| | Compound M2 | | | | | Compound M3 | Compound M1 | | | Device Evaluation | | | | |
|---|---|---|---|---|---|---|---|---|---|---|---|---|---|---|
| | Name | λ [nm] | PLQY | $S_1$ [eV] | ΔST [eV] | Name | Name | $S_1$ [eV] | $T_{77K}$ [eV] | CIEx | CIEy | EQE (relative value) [%] | λp [nm] | FWHM [nm] |
| Ex.4-3 | A-9 | 494 | 0.90 | 2.65 | < 0.01 | M3-2 | GD2 | 2.39 | 1.99 | 0.230 | 0.693 | 122 | 519 | 29.0 |
| Ex.4-4 | A-15 | 494 | 0.91 | 2.65 | <0.01 | M3-2 | GD2 | 2.39 | 1.99 | 0.240 | 0.692 | 123 | 519 | 30.0 |
| Ex.4-5 | A-16 | 495 | 0.83 | 2.66 | <0.01 | M3-2 | GD2 | 2.39 | 1.99 | 0.250 | 0.690 | 119 | 519 | 29.0 |
| Ex.4-6 | A-17 | 498 | 0.99 | 2.64 | <0.01 | M3-2 | GD2 | 2.39 | 1.99 | 0.230 | 0.690 | 132 | 519 | 29.0 |
| Ex.4-7 | A-19 | 492 | 0.85 | 2.66 | <0.01 | M3-2 | GD2 | 2.39 | 1.99 | 0.250 | 0.690 | 124 | 520 | 28.0 |
| Ex.4-8 | A-20 | 488 | 0.85 | 2.68 | <0.01 | M3-2 | GD2 | 2.39 | 1.99 | 0.220 | 0.690 | 137 | 518 | 28.0 |
| Ex.4-9 | A-21 | 495 | 0.87 | 2.63 | <0.01 | M3-2 | GD2 | 2.39 | 1.99 | 0.250 | 0.680 | 121 | 519 | 32.0 |
| Ex.4-10 | A-22 | 492 | 0.87 | 2.67 | <0.01 | M3-2 | GD2 | 2.39 | 1.99 | 0.220 | 0.700 | 136 | 518 | 28.0 |
| Ex.4-11 | A-23 | 499 | 0.90 | 2.60 | <0.01 | M3-2 | GD2 | 2.39 | 1.99 | 0.290 | 0.660 | 121 | 520 | 35.0 |
| Ex.4-12 | A-24 | 497 | 0.84 | 2.63 | <0.01 | M3-2 | GD2 | 2.39 | 1.99 | 0.250 | 0.680 | 122 | 519 | 30.0 |
| Ex.4-13 | A-25 | 493 | 0.78 | 2.66 | <0.01 | M3-2 | GD2 | 2.39 | 1.99 | 0.230 | 0.690 | 141 | 518 | 29.0 |
| Ex.4-14 | A-26 | 491 | 0.86 | 2.66 | <0.01 | M3-2 | GD2 | 2.39 | 1.99 | 0.230 | 0.690 | 125 | 518 | 29.0 |
| Comp.4-1 (re-shown) | Ref-1 | 504 | 0.87 | 2.52 | <0.01 | M3-2 | GD2 | 2.39 | 1.99 | 0.291 | 0.660 | 100 | 521 | 34.0 |

Table 7

| | Compound M2 | | | | | Compound M3 | Compound M1 | | | Device Evaluation | | | | |
|---|---|---|---|---|---|---|---|---|---|---|---|---|---|---|
| | Name | λ [nm] | PLQY | $S_1$ [eV] | ΔST [eV] | Name | Name | $S_1$ [eV] | $T_{77K}$ [eV] | CIEx | CIEy | EQE (relative value) [%] | λp [nm] | FWHM [nm] |
| Ex.4-15 | A-30 | 497 | 0.92 | 2.64 | <0.01 | M3-2 | GD2 | 2.39 | 1.99 | 0.240 | 0.690 | 126 | 519 | 29.0 |
| Ex.4-16 | A-31 | 504 | 0.90 | 2.60 | <0.01 | M3-2 | GD2 | 2.39 | 1.99 | 0.260 | 0.680 | 130 | 519 | 31.0 |
| Ex.4-17 | A-32 | 493 | 0.92 | 2.67 | <0.01 | M3-2 | GD2 | 2.39 | 1.99 | 0.240 | 0.690 | 126 | 518 | 29.0 |
| Ex.4-18 | A-33 | 495 | 0.89 | 2.67 | <0.01 | M3-2 | GD2 | 2.39 | 1.99 | 0.240 | 0.680 | 124 | 519 | 29.0 |
| Ex.4-19 | A-34 | 502 | 0.89 | 2.59 | <0.01 | M3-2 | GD2 | 2.39 | 1.99 | 0.250 | 0.690 | 132 | 519 | 29.0 |
| Ex.4-20 | A-35 | 498 | 0.95 | 2.65 | <0.01 | M3-2 | GD2 | 2.39 | 1.99 | 0.220 | 0.700 | 135 | 519 | 28.0 |
| Ex.4-21 | A-36 | 495 | 0.99 | 2.69 | <0.01 | M3-2 | GD2 | 2.39 | 1.99 | 0.220 | 0.700 | 143 | 518 | 28.0 |
| Ex.4-22 | A-37 | 499 | 0.86 | 2.60 | <0.01 | M3-2 | GD2 | 2.39 | 1.99 | 0.290 | 0.660 | 120 | 520 | 28.0 |
| Ex.4-23 | A-38 | 497 | 0.88 | 2.64 | <0.01 | M3-2 | GD2 | 2.39 | 1.99 | 0.240 | 0.690 | 126 | 519 | 29.0 |
| Ex.4-24 | A-39 | 495 | 0.90 | 2.64 | <0.01 | M3-2 | GD2 | 2.39 | 1.99 | 0.230 | 0.690 | 129 | 519 | 28.0 |
| Ex.4-25 | A-42 | 500 | 0.84 | 2.60 | <0.01 | M3-2 | GD2 | 2.39 | 1.99 | 0.300 | 0.650 | 122 | 520 | 34.0 |
| Comp.4-1 (re-shown) | Ref-1 | 504 | 0.87 | 2.52 | <0.01 | M3-2 | GD2 | 2.39 | 1.99 | 0.291 | 0.660 | 100 | 521 | 34.0 |

The organic EL devices of Examples using a compound represented by the formula (1) had an improved device performance compared with the organic EL devices of Comparatives.

Evaluation of Compounds

The compounds used for producing the organic EL devices in Examples and the following compounds were evaluated.

[Formula 244]

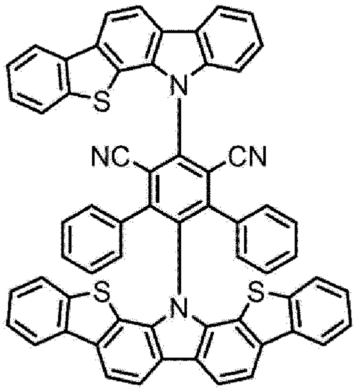

**A-8**

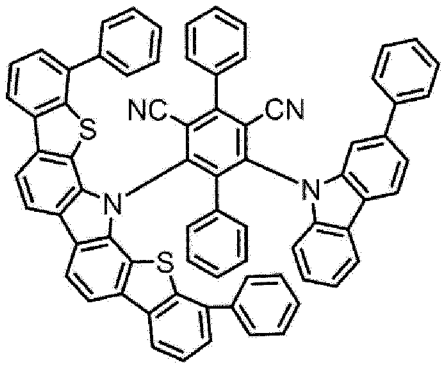

**A-18**

[Formula 245]

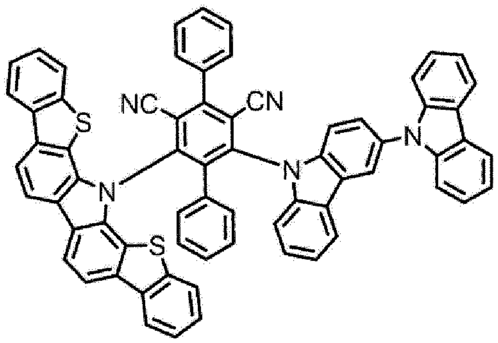

A-27

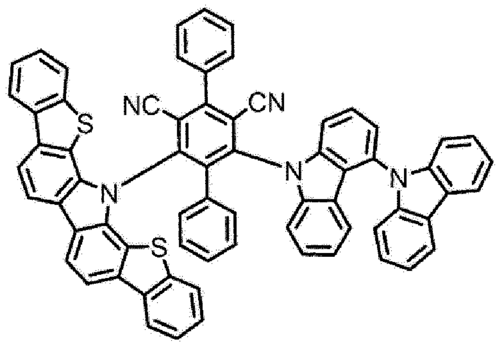

A-28

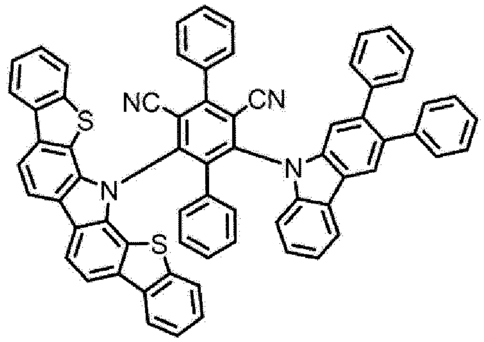

A-29

Measurement of Fluorescence Quantum Yield (PLQY)

Each measurement target compound was dissolved in toluene at a concentration of 5 μmol/L to prepare a toluene solution. Subsequently, the prepared solution was bubbled with nitrogen for five minutes and sealed so as not to be mixed with outside air.

PLQY of the prepared toluene solution of the measurement target compound was measured using an absolute photoluminescence (PL) quantum yield measurement machine Quantaurus-QY (manufactured by Hamamatsu Photonics K.K.).

Maximum Peak Wavelength of Compounds

A maximum peak wavelength λ of each of the compounds was measured according to the following method.

A toluene solution of each measurement target compound at a concentration of 5 μmol/L was prepared and put in a quartz cell. An emission spectrum (ordinate axis: luminous intensity, abscissa axis: wavelength) of the thus-obtained sample was measured at a normal temperature (300K). In Examples, the emission spectrum was measured with a spectrophotometer (device name: F-7000) manufactured by Hitachi High-Tech Corporation. It should be noted that the apparatus for measuring the emission spectrum is not limited to the apparatus used herein. A peak wavelength of the emission spectrum exhibiting the maximum luminous intensity was defined as the maximum peak wavelength λ.

Delayed Fluorescence of Compounds

Delayed fluorescence was checked by measuring transient PL using an apparatus illustrated in Fig. 1. The compound A-1 was dissolved in toluene to prepare a dilute solution with an absorbance of 0.05 or less at the excitation wavelength to eliminate contribution of self-absorption. In order to prevent quenching due to oxygen, the sample solution was frozen and degassed and then sealed in a cell with a lid under an argon atmosphere to obtain an oxygen-free

sample solution saturated with argon.

The fluorescence spectrum of the sample solution was measured with a spectrofluorometer FP-8600 (produced by JASCO Corporation), and the fluorescence spectrum of a 9,10-diphenylanthracene ethanol solution was measured under the same conditions. Using the fluorescence area intensities of both spectra, the total fluorescence quantum yield was calculated by an equation (1) in Morris et al. J. Phys. Chem. 80 (1976) 969.

Prompt emission was observed immediately when the excited state was achieved by exciting the compound A-1 with a pulse beam (i.e., a beam emitted from a pulse laser) having a wavelength to be absorbed by the compound A-1, and Delay emission was observed not immediately when the excited state was achieved but after the excited state was achieved. The delayed fluorescence in Examples means that an amount of Delay emission is 5% or more relative to an amount of Prompt emission. Specifically, provided that the amount of Prompt emission is denoted by $X_P$ and the amount of Delay emission is denoted by $X_D$, the delayed fluorescence means that a value of $X_D/X_P$ is 0.05 or more.

An amount of Prompt emission, an amount of Delay emission and a ratio between the amounts thereof can be obtained according to the method as described in "Nature 492, 234-238, 2012" (Reference Document 1). The amount of Prompt emission and the amount of Delay emission may be calculated using an apparatus different from one described in the above Reference Document 1 or the apparatus illustrated in Fig. 1.

The compounds A-2 to A-42 and the comparative compound Ref-1 were also measured in the same manner as the compound A-1.

It was confirmed that the amount of Delay emission was 5% or more relative to the amount of Prompt emission as for the compounds A-1 to A-42 and the comparative compound Ref-1. Specifically, the value of $X_D/X_P$ was 0.05 or more with respect to the compounds A-1 to A-42 and the comparative compound Ref-1.

Lowest Singlet Energy $S_1$

The lowest singlet energy $S_1$ of a measurement target compound was measured according to the above-described solution method.

Energy Gap $T_{77K}$ at 77K and ΔST

An energy gap $T_{77K}$ of each of the measurement target compounds was measured according to the measurement method of the energy gap $T_{77K}$ described in the above "Relationship between Triplet Energy and Energy Gap at 77K."

ΔST of each of the compounds A-1 to A-42 and the comparative compound Ref-1 was determined from the corresponding one of the values of energy gap $T_{77K}$ and the corresponding one of the values of the lowest singlet energy $S_1$ described above. In the table, "<0.01" represents that ΔST is less than 0.01 eV.

Table 8

| Compound | λ [nm] | PLQY | $S_1$ [eV] | ΔST [eV] | | Compound | λ [nm] | PLQY | $S_1$ [eV] |
|---|---|---|---|---|---|---|---|---|---|
| Name | | | | | | Name | | | |

(continued)

| Compound Name | λ [nm] | PLQY | $S_1$ [eV] | ΔST [eV] | | Compound Name | λ [nm] | PLQY | $S_1$ [eV] |
|---|---|---|---|---|---|---|---|---|---|
| A-1 | 498 | 0.90 | 2.58 | < 0.01 | | A-26 | 491 | 0.86 | 2.66 |
| A-2 | 496 | 0.96 | 2.62 | < 0.01 | | A-27 | 531 | 0.48 | 2.57 |
| A-3 | 493 | 0.99 | 2.52 | < 0.01 | | A-28 | 502 | 0.93 | 2.57 |
| A-4 | 491 | 0.94 | 2.67 | < 0.01 | | A-29 | 495 | 0.87 | 2.65 |
| A-5 | 484 | 0.98 | 2.70 | < 0.01 | | A-30 | 497 | 0.92 | 2.64 |
| A-6 | 475 | 0.95 | 2.69 | < 0.01 | | A-31 | 504 | 0.90 | 2.60 |
| A-7 | 490 | 0.91 | 2.75 | < 0.01 | | A-32 | 493 | 0.92 | 2.67 |
| A-8 | 489 | 0.86 | 2.80 | < 0.01 | | A-33 | 495 | 0.89 | 2.67 |
| A-9 | 494 | 0.90 | 2.65 | < 0.01 | | A-34 | 502 | 0.89 | 2.59 |
| A-10 | 489 | 0.90 | 2.69 | < 0.01 | | A-35 | 498 | 0.95 | 2.65 |
| A-11 | 495 | 0.85 | 2.65 | < 0.01 | | A-36 | 495 | 0.99 | 2.69 |
| A-12 | 499 | 0.87 | 2.58 | < 0.01 | | A-37 | 499 | 0.86 | 2.60 |
| A-13 | 496 | 1.00 | 2.58 | < 0.01 | | A-38 | 497 | 0.88 | 2.64 |
| A-14 | 505 | 0.99 | 2.64 | < 0.01 | | A-39 | 495 | 0.90 | 2.64 |
| A-15 | 494 | 0.91 | 2.65 | <0.01 | | A-40 | 489 | 0.82 | 2.71 |
| A-16 | 495 | 0.83 | 2.66 | <0.01 | | A-41 | 495 | 0.70 | 2.70 |
| A-17 | 498 | 0.99 | 2.64 | <0.01 | | A-42 | 500 | 0.84 | 2.60 |
| A-18 | 495 | 0.99 | 2.70 | <0.01 | | Ref-1 | 504 | 0.87 | 2.52 |
| A-19 | 492 | 0.85 | 2.66 | <0.01 | | M3-1 | - | - | 3.42 |
| A-20 | 488 | 0.85 | 2.68 | <0.01 | | M3-2 | - | - | 3.43 |
| A-21 | 495 | 0.87 | 2.63 | <0.01 | | GD | 508 | 0.75 | 2.45 |
| A-22 | 492 | 0.87 | 2.67 | <0.01 | | GD2 | 512 | 0.91 | 2.39 |
| A-23 | 499 | 0.90 | 2.60 | <0.01 | | | | | |
| A-24 | 497 | 0.84 | 2.63 | <0.01 | | | | | |
| A-25 | 493 | 0.78 | 2.66 | <0.01 | | | | | |

Synthesis Examples

Synthesis of Compound A-1

A synthesis method of the compound A-1 is described below.

[Formula 246]

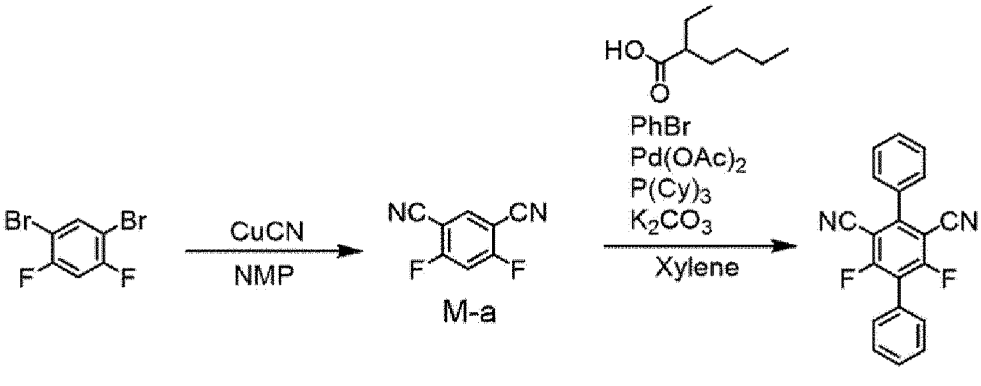

M-a

M-b

Under a nitrogen atmosphere, 1,5-dibromo-2,4-difluorobenzene (165 g, 607 mmol), cyanocopper (120 g, 1335 mmol), and NMP (800 ml) were put into a 2-L three-necked flask and stirred for five hours at 150 degrees C. 1L of methylene chloride was added to the reaction mixture, filtered through Celite, and the filtrate was concentrated using an evaporator. The solid obtained after the concentration was purified by silica gel column chromatography to obtain 58 g of a white solid. The obtained white solid was identified as an intermediate M-a by analysis of Gas Chromatograph Mass Spectrometer (GC-MS) (a yield of 58%). NMP is an abbreviation for N-methyl-2-pyrrolidone.

Under a nitrogen atmosphere, the intermediate M-a (20 g,122 mmol), potassium carbonate (33.7 g, 244 mmol), diacetoxypalladium (1.368 g, 6.09 mmol), tricyclohexylphosphine (5.13 g, 18.28 mmol), bromobenzene (31.9 ml, 305 mmol), 2-ethylhexanoic acid (7.81 ml, 48.7 mmol), and xylene (250 ml) were put into a 500-mL three-necked flask and stirred at 100 degrees C for five hours. 200 ml of methylene chloride was added to the reaction solution and passed through Celite. Methylene chloride in the obtained solution was removed and the precipitated solid was filtered. The obtained solid was purified by silica gel column chromatography to obtain 12 g of a white solid. The obtained white solid was identified as an intermediate M-b by analysis of GC-MS (a yield of 31%).

[Formula 247]

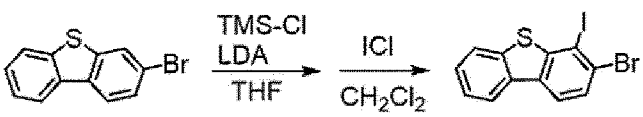

M-c

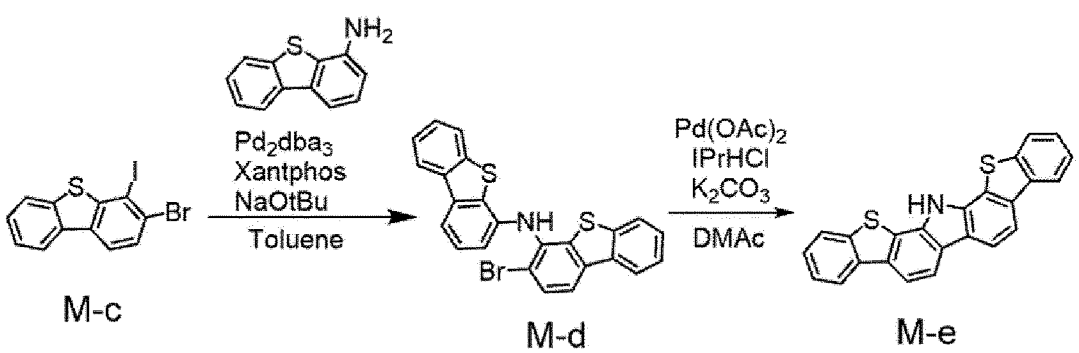

M-c

M-d

M-e

Under a nitrogen atmosphere, 3-bromodibenzothiophene (26.3 g, 100 mmol), chlorotrimethylsilane (33 g, 300 mmol), and THF (150 mL) were put into a 500-ml three-necked flask. After cooling the material in the three-necked flask

to -78 degrees C in a dry ice/acetone bath, 125 ml (2M, THF solution) of lithium diisopropylamide was added dropwise. The mixture was stirred at -78 degrees C for two hours, then returned to room temperature, and further stirred for two hours. After the stirring, water (100 mL) was added to the three-necked flask, and the organic layer was extracted with ethyl acetate. The extracted organic layer was washed with water and saline, dried over magnesium sulfate, and then the solvent was removed under reduced pressure using a rotary evaporator. 200 ml of dichloromethane was added to the obtained liquid, and then iodine monochloride (49 g, 300 mmol) was added dropwise at 0 degrees C, followed by stirring at 40 degrees C for six hours. The obtained mixture was returned to room temperature and added with saturated sodium bisulfite aqueous solution (100 mL). The organic layer was extracted with dichloromethane. The extracted organic layer was washed with water and saline. The washed organic layer was dried over magnesium sulfate. The dried organic layer was concentrated using a rotary evaporator. After the concentration, the obtained solid was purified by silica gel column chromatography to obtain an intermediate M-c (28 g, 72 mmol, a yield of 72%).

Under a nitrogen atmosphere, the intermediate M-c (24.5 g, 63.0 mmol), Dibenzo[b,d]thiophen-4-amine (12.55 g, 63.0 mmol), tris(dibenzylideneacetone)dipalladium(0) (0.865 g, 0.945 mmol), Xantphos (1.385 g, 1.889 mmol), sodium tert-butoxide (9.08 g, 94 mmol), and toluene (210 mL) were added to a 500-ml three-necked flask. The obtained mixture was heated at 60 degrees C for eight hours with stirring and then cooled to room temperature (25 degrees C). The deposited solid was collected by filtration and washed with toluene (200 ml) to obtain 25 g of a white solid. The obtained white solid was identified as an intermediate M-d by analysis of GC-MS (a yield of 86%).

Under a nitrogen atmosphere, the intermediate M-d (9.5 g, 20.7 mmol), 1,3-bis(2,6-diisopropylphenyl)imidazolium chloride (IPrHCl)(0.36 g, 0.82 mmol), palladium(II) acetate (0.093 g, 0.41 mmol), potassium carbonate (5.8 g, 42 mmol), and N,N-dimethylacetamide (DMAc) (60mL) were added to a 200-ml three-necked flask. The obtained mixture was stirred at 160 degrees C for 10 hours and then cooled to room temperature (25 degrees C). The deposited solid was collected by filtration and washed with acetone to obtain 6.9 g of a white solid. The obtained white solid was identified as an intermediate M-e by analysis of ASAP-MS (a yield of 86%). ASAP-MS is an abbreviation of Atmospheric Pressure solid Analysis Probe Mass Spectrometry.

[Formula 248]

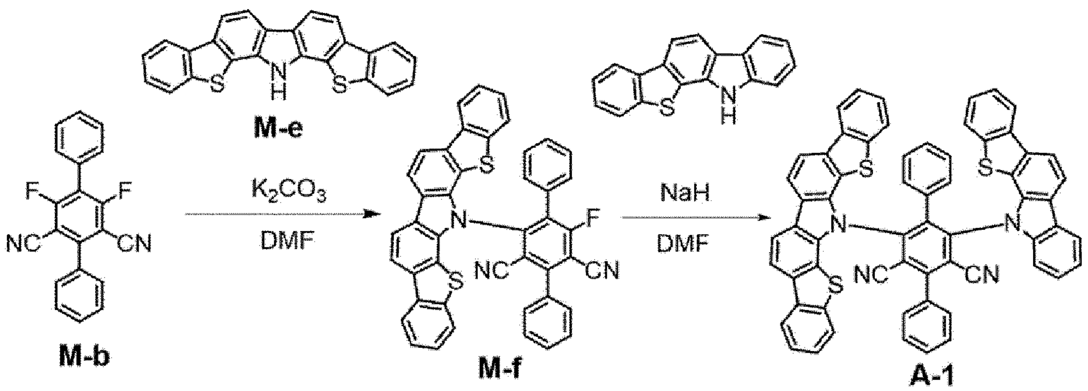

M-b M-f A-1

Under a nitrogen atmosphere, the intermediate M-b (3.0 g, 9.48 mmol), the intermediate M-e (3.6 g, 9.5 mmol), potassium carbonate (2.6 g, 19 mmol), and DMF (50 mL) were put into a 200-mL three-necked flask and stirred at 100 degrees C for four hours. 100 ml of ion-exchange water was added to the reaction solution, and the deposited solid was collected by filtration. The collected solid was purified by silica gel column chromatography to obtain 4.1 g of a yellow solid. The obtained yellow solid was identified as an intermediate M-f by analysis of ASAP-MS (a yield of 64%). DMF is an abbreviation of N,N-dimethylformamide.

Under a nitrogen atmosphere, 12H-[1]Benzothieno[2,3-a]carbazole (0.809 g, 2.96 mmol), sodium hydride (containing 40-mass% oil) (0.14 g, 3.55 mmol), and DMF (15 mL) were put into a 100-mL three-necked flask and stirred at 0 degrees C for 30 minutes. Next, the intermediate M-f (2 g, 2.96 mmol) was put into the reaction mixture and stirred at room temperature for two hours. Water (50 mL) was added to the reaction mixture. The deposited solid was purified by silica gel column chromatography to obtain 1.6 g of a yellow solid. The obtained yellow solid was identified as the compound A-1 by analysis of ASAP-MS (a yield of 58%).

Synthesis of Compound A-2

A synthesis method of the compound A-2 is described below.

[Formula 249]

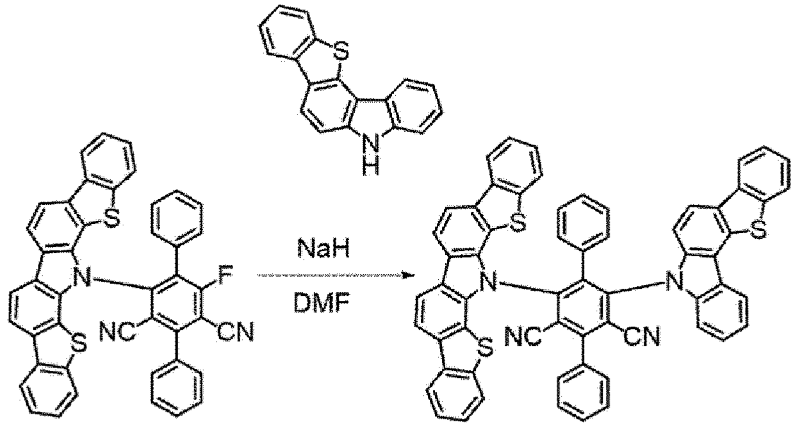


M-f A-2

Under a nitrogen atmosphere, 5H-benzo[4,5]thieno[3,2-c]carbazole (0.971 g, 3.55 mmol), sodium hydride (containing 40-mass% oil) (0.14 g, 3.55 mmol), and DMF (15 mL) were put into a 100-mL three-necked flask and stirred at 0 degrees C for 30 minutes. Next, the intermediate M-f (2 g, 2.96 mmol) was put into the reaction mixture and stirred at room temperature for two hours. Water (50 mL) was added to the reaction mixture. The deposited solid was purified by silica gel column chromatography to obtain 2.2 g of a yellow solid. The obtained yellow solid was identified as the compound A-2 by analysis of ASAP-MS (a yield of 80%).

Synthesis of Compound A-3

A synthesis method of the compound A-3 is described below.

[Formula 250]

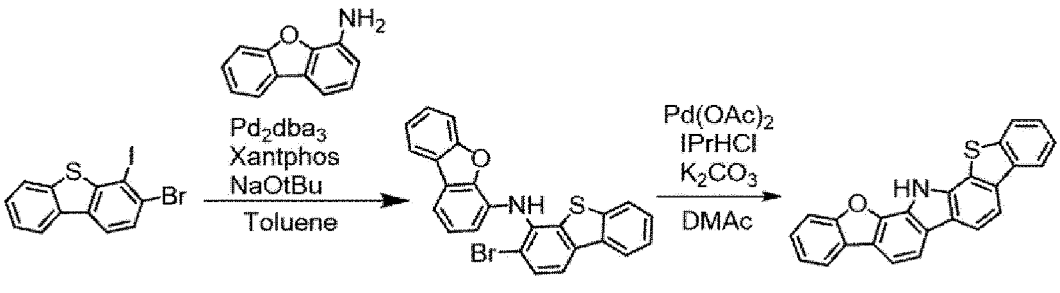


M-c M-g M-h

Under a nitrogen atmosphere, the intermediate M-c (20 g, 51.4 mmol), dibenzo[b,d]furan-4-amine (9.42 g, 51.4 mmol), $Pd_2dba_3$ (0.706 g, 0.771 mmol), (9,9-dimethyl-9H-xanthene-4,5-diyl)bis(triphenyl-14-phosphane) (1.130 g, 1.542 mmol), NaOt-Bu (7.41 g, 77 mmol), and toluene (171 ml) were added to a 500-ml three-necked flask, heated at 60 degrees C for eight hours with stirring, and then cooled to room temperature (25 degrees C). The deposited solid was collected by filtration and washed with toluene (200 ml) to obtain 19 g of a white solid. The obtained white solid was identified as an intermediate M-g by analysis of GC-MS (a yield of 83%).

Under a nitrogen atmosphere, the intermediate M-g (15 g, 33.8 mmol), 1,3-bis(2,6-diisopropylphenyl)imidazolium chloride (IPrHCl) (0.430 g, 1.013 mmol), palladium(II) acetate (0.114 g, 0.506 mmol), potassium carbonate (9.80 g, 70.9 mmol), and DMAc (169 ml) were added to a 300-ml three-necked flask. The obtained mixture was stirred at 140 degrees C for six hours and then cooled to room temperature (25 degrees C). The deposited solid was collected by filtration and washed with acetone to obtain 8.4 g of a white solid. The obtained white solid was identified as an intermediate M-h by analysis of ASAP-MS (a yield of 69%).

[Formula 251]

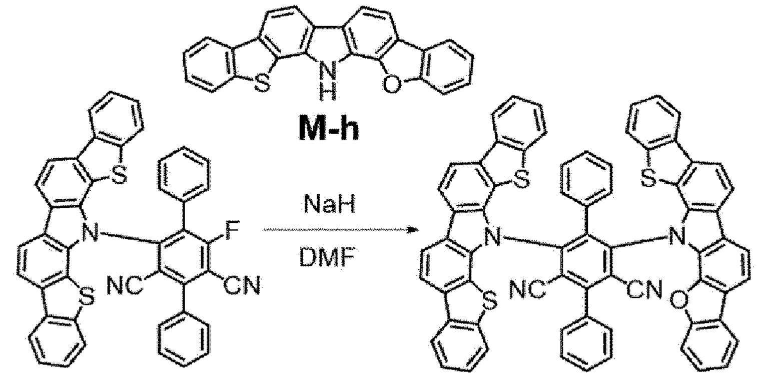


M-f A-3

Under a nitrogen atmosphere, the intermediate M-h (0.532 g,1.465 mmol), sodium hydride (containing 40-mass% oil) (0.064 g, 1.598 mmol), and DMF (15 mL) were put into a 100-mL three-necked flask and stirred at 0 degrees C for 30 minutes. Next, the intermediate M-f (0.9 g, 1.332 mmol) was put into the reaction mixture and stirred at room temperature for two hours. Water (50 mL) was added to the reaction mixture. The deposited solid was purified by silica gel column chromatography to obtain 0.7 g of a yellow solid. The obtained yellow solid was identified as the compound A-3 by analysis of ASAP-MS (a yield of 52%).

Synthesis of Compound A-4

A synthesis method of a compound A-4 is described below.

[Formula 252]

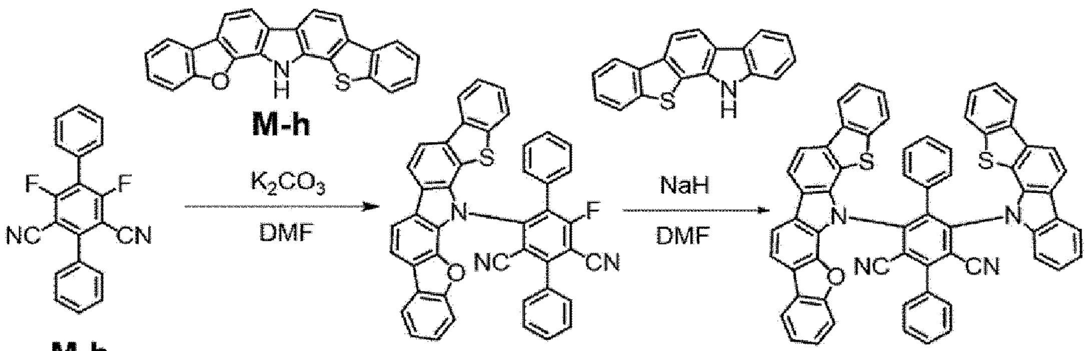


M-b M-i A-4

Under a nitrogen atmosphere, the intermediate M-b (3 g, 9.48 mmol), tripotassium phosphate (4.03 g, 18.97 mmol), the intermediate M-h (3.45 g, 9.48 mmol), and DMF (47.4 ml) were put into a 200-mL three-necked flask and stirred at 60 degrees C for four hours. 100 ml of ion-exchange water was added to the reaction solution, and the deposited solid was collected by filtration. The collected solid was purified by silica gel column chromatography to obtain 4.9 g of a yellow solid. The obtained yellow solid was identified as an intermediate M-i by analysis of ASAP-MS (a yield of 78%).

Under a nitrogen atmosphere, 12H-[1]Benzothieno[2,3-a]carbazole (0.912 g, 3.33 mmol), sodium hydride (containing 40-mass% oil) (0.133 g, 3.33 mmol), and DMF (15.16 ml) were put into a 100-mL three-necked flask and stirred at 0 degrees C for 30 minutes. Next, the intermediate M-i (2 g, 3.03 mmol) was put into the reaction mixture and stirred at room temperature for two hours. Water (50 mL) was added to the reaction mixture. The deposited solid was purified by silica gel column chromatography to obtain 1.1 g of a yellow solid. The obtained yellow solid was identified as the compound A-4 by analysis of ASAP-MS (a yield of 40%).

Synthesis of Compound A-5

A synthesis method of a compound A-5 is described below.

[Formula 253]

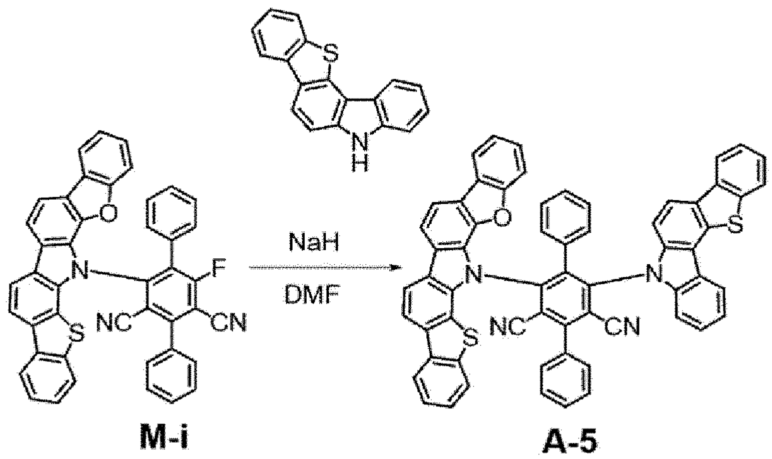


Under a nitrogen atmosphere, 5H-benzo[4,5]thieno[3,2-c]carbazole (0.646 g, 2.365 mmol), sodium hydride (containing 40-mass% oil) (0.095 g, 2.365 mmol), and DMF (19.70 ml) were put into a 100-mL three-necked flask and stirred at 0 degrees C for 30 minutes. Next, the intermediate M-i (1.3 g, 1.970 mmol) was put into the reaction mixture and stirred at room temperature for two hours. Water (50 mL) was added to the reaction mixture. The deposited solid was purified by silica gel column chromatography to obtain 1.4 g of a yellow solid. The obtained yellow solid was identified as the compound A-5 by analysis of ASAP-MS (a yield of 78%).

Synthesis of Compound A-6

A synthesis method of a compound A-6 is described below.

[Formula 254]

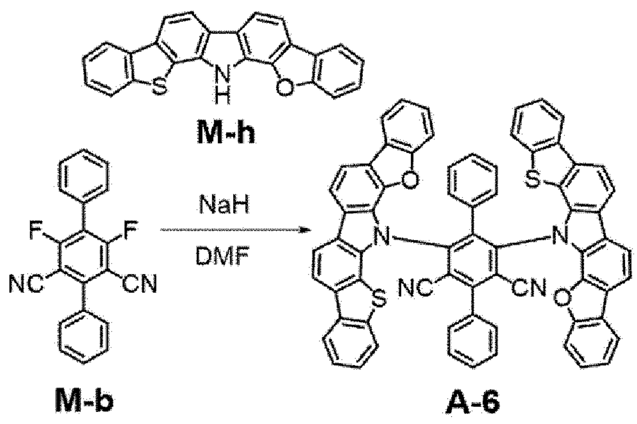


Under a nitrogen atmosphere, the intermediate M-h (1 g,3.16 mmol), sodium hydride (containing 40-mass% oil) (0.278 g, 6.96 mmol), and DMF (15.8 ml) were put into a 100-mL three-necked flask and stirred at 0 degrees C for 30 minutes. Next, the intermediate M-b (2.53 g, 6.96 mmol) was put into the reaction mixture and stirred at 100 degrees C for two hours. Water (50 mL) was added to the reaction mixture. The deposited solid was purified by silica gel column chromatography to obtain 2.1 g of a yellow solid. The obtained yellow solid was identified as the compound A-6 by analysis of ASAP-MS (a yield of 66%).

Synthesis of Compound A-7

A synthesis method of a compound A-7 is described below.

[Formula 255]

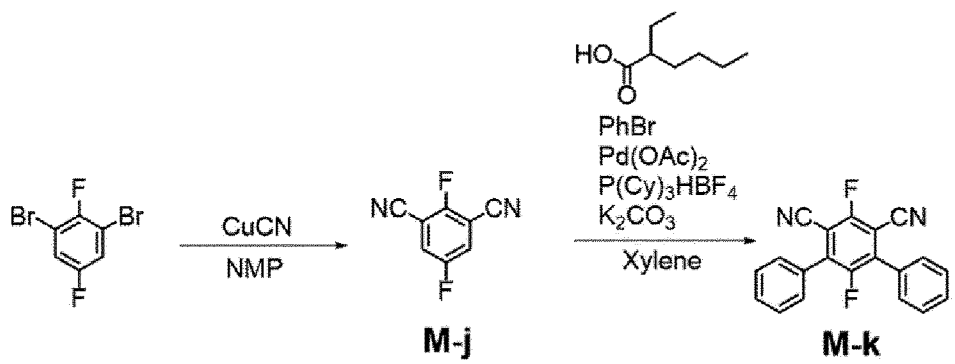


M-j

M-k

Under a nitrogen atmosphere, 1,3-dibromo-2,5-difluorobenzene (35 g, 129 mmol), cyanocopper (25.4 g, 283 mmol), and NMP (257 ml) were put into a 1-L three-necked flask and stirred for five hours at 150 degrees C. 500mL of methylene chloride was added to the reaction mixture, filtered through Celite, and the filtrate was concentrated using an evaporator. After the concentration, the obtained solid was purified by silica gel column chromatography and recrystallized with ethanol to obtain 12 g of a white solid. The obtained white solid was identified as an intermediate M-j by analysis of GC-MS (a yield of 57%).

Under a nitrogen atmosphere, 2,5-difluoroisophthalonitrile (6.3 g, 38.4 mmol), potassium carbonate (11.67 g, 84 mmol), diacetoxypalladium (0.431 g, 1.919 mmol), tricyclohexylphosphonium tetrafluoroborate (2.120 g, 5.76 mmol), bromobenzene (12.05 ml,115 mmol), 2-ethylhexanoic acid(2.460ml, 15.36 mmol), and xylene (80 ml) were put into a 300-mL three-necked flask and stirred at 100 degrees C for five hours. 100 ml of methylene chloride was added to the reaction solution and passed through Celite. Methylene chloride in the obtained solution was concentrated and the precipitated solid was filtered. The obtained solid was purified by silica gel column chromatography to obtain 5.2 g of a white solid. The obtained white solid was identified as an intermediate M-k by analysis of GC-MS (a yield of 43%).

[Formula 256]

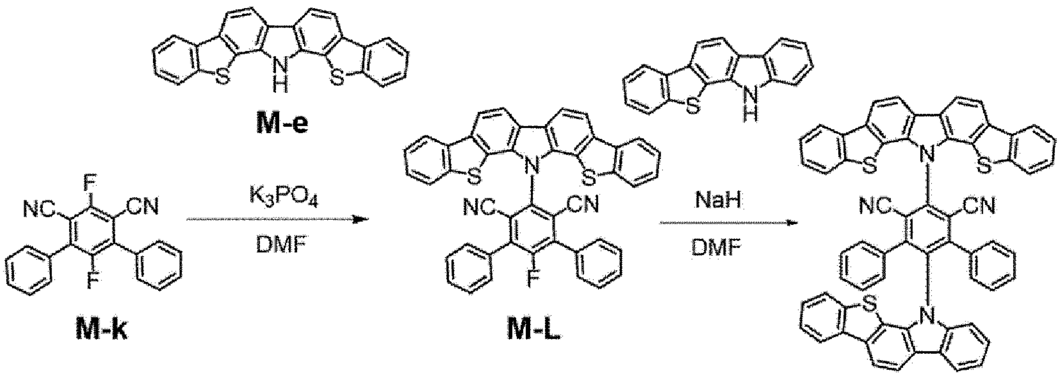


M-e

M-k

M-L

A-7

Under a nitrogen atmosphere, the intermediate M-k (1.5 g, 4.74 mmol), the intermediate M-e (1.8 g, 4.74 mmol), tripotassium phosphate (3.02 g, 14.23 mmol), and DMF (23.71 ml) were put into a 200-mL three-necked flask and stirred at 60 degrees C for four hours. 100 ml of ion-exchange water was added to the reaction solution, and the deposited solid was collected by filtration. The collected solid was purified by silica gel column chromatography to obtain 2 g of a yellow solid. The obtained yellow solid was identified as an intermediate M-L by analysis of ASAP-MS (a yield of 62%).

Under a nitrogen atmosphere, 12H-[1]Benzothieno[2,3-a]carbazole (0.922 g, 3.37 mmol), sodium hydride (containing 40-mass% oil) (0.124 g, 3.09 mmol), and DMF (14.06 ml) were put into a 100-mL three-necked flask and stirred at 0 degrees C for 30 minutes. Next, the intermediate M-L (1.9 g, 2.81 mmol) was put into the reaction mixture and

stirred at 70 degrees C for eight hours. Water (50 mL) was added to the reaction mixture. The deposited solid was purified by silica gel column chromatography to obtain 1.8 g of a yellow solid. The obtained yellow solid was identified as the compound A-7 by analysis of ASAP-MS (a yield of 69%).

Synthesis of Compound A-8

A synthesis method of a compound A-8 is described below.

[Formula 257]

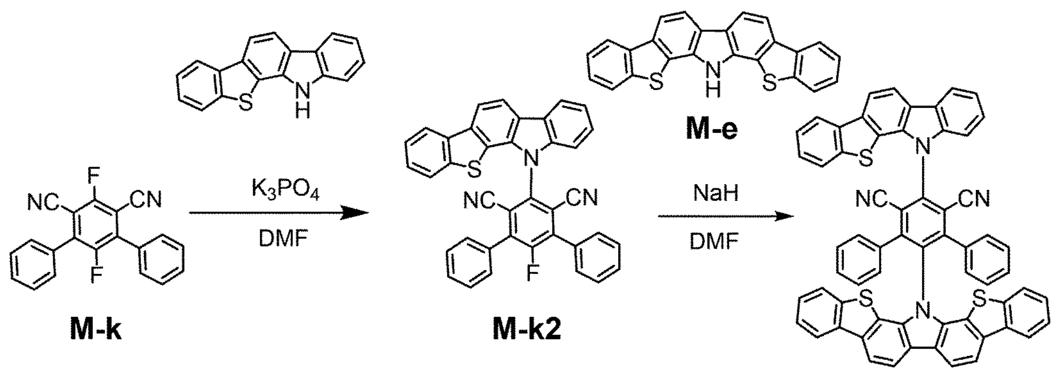


M-k M-e M-k2 A-8

Under a nitrogen atmosphere, the intermediate M-k (2.4 g, 7.59 mmol), 12H-[1]Benzothieno[2,3-a]carbazole (2.074 g, 7.59 mmol), tripotassium phosphate (4.83 g, 22.76 mmol), and DMF (37.9 ml) were put into a 200-mL three-necked flask and stirred at 50 degrees C for four hours. 100 ml of ion-exchange water was added to the reaction solution, and the deposited solid was collected by filtration. The collected solid was purified by silica gel column chromatography to obtain 4 g of a yellow solid. The obtained yellow solid was identified as an intermediate M-k2 by analysis of ASAP-MS (a yield of 93%).

Under a nitrogen atmosphere, the intermediate M-e (1.332 g, 3.51 mmol), sodium hydride (containing 40-mass% oil) (0.154 g, 3.86 mmol), and DMF (14.06 ml) were put into a 100-mL three-necked flask and stirred at 0 degrees C for 30 minutes. Next, the intermediate M-k2 (2 g, 3.51 mmol) was put into the reaction mixture and stirred at 170 degrees C for 14 hours. Water (50 mL) was added to the reaction mixture. The deposited solid was purified by silica gel column chromatography to obtain 0.98 g of a yellow solid. The obtained yellow solid was identified as the compound A-8 by analysis of ASAP-MS (a yield of 30%).

Synthesis of Compound A-9

A synthesis method of a compound A-9 is described below.

[Formula 258]

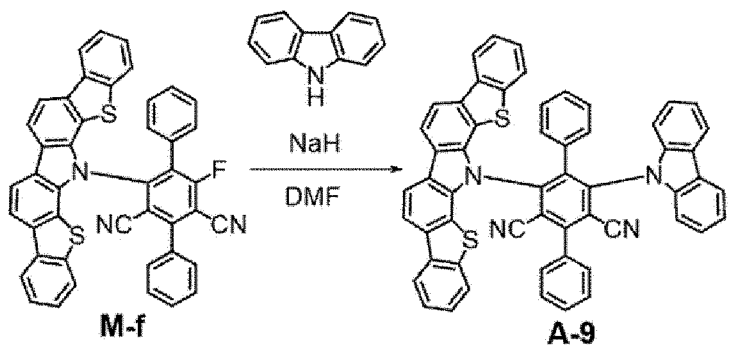


M-f A-9

Under a nitrogen atmosphere, 9H-carbazole (2.313 g, 13.84 mmol), sodium hydride (containing 40-mass% oil) (0.488 g, 12.21 mmol), and DMF (40.7 ml) were put into a 100-mL three-necked flask and stirred at 0 degrees C for 30 minutes. Next, the intermediate M-f (5.5 g, 8.14 mmol) was put into the reaction mixture and stirred at room temperature

for two hours. Methanol (20 mL) was added to the reaction mixture. The deposited solid was purified by silica gel column chromatography to obtain 6.3 g of a yellow solid. The obtained yellow solid was identified as the compound A-9 by analysis of ASAP-MS (a yield of 94%).

Synthesis of Compound A-10

A synthesis method of a compound A-10 is described below.

[Formula 259]

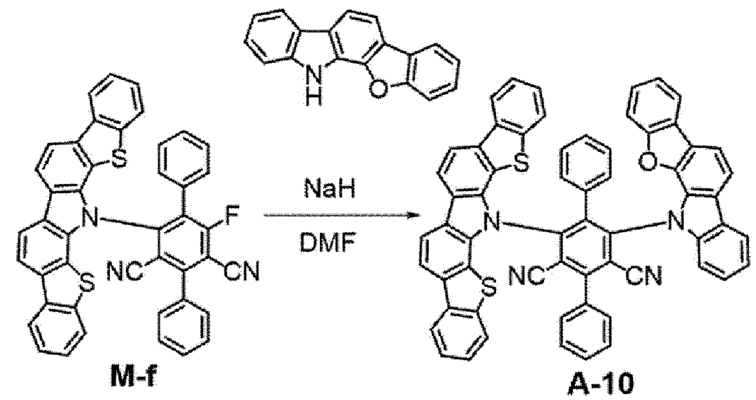


M-f

A-10

Under a nitrogen atmosphere, 12H-benzofuro[2,3-a]carbazole (1.466 g, 5.70 mmol), sodium hydride (containing 40-mass% oil) (0.228 g, 5.70 mmol), and DMF (17.26 ml) were put into a 100-mL three-necked flask and stirred at 0 degrees C for 30 minutes. Next, the intermediate M-f (3.5 g, 5.18 mmol) was put into the reaction mixture and stirred at room temperature for two hours. Methanol (20 mL) was added to the reaction mixture. The deposited solid was purified by silica gel column chromatography to obtain 3.7 g of a yellow solid. The obtained yellow solid was identified as the compound A-10 by analysis of ASAP-MS (a yield of 78%).

Synthesis of Compound A-11

A synthesis method of a compound A-11 is described below.

[Formula 260]

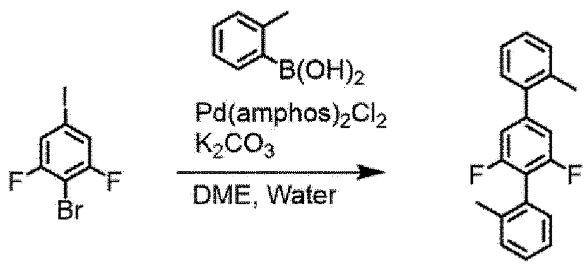


M-m

Under a nitrogen atmosphere, 5-bromo-1,3-difluoro-2-iodobenzene (4.00 g, 12.54 mmol), o-tolylboronic acid (3.75 g, 27.60 mmol), potassium carbonate (10.40 g, 75.00 mmol), bis[di-tert-butyl(4-dimethylaminophenyl)phosphine]dichloropalladium (0.266 g, 0.376 mmol), DME (66.9 ml) and ion-exchange water (16.7 ml) were put into a 200-ml three-necked flask and stirred at 90 degrees C for five hours. 100 ml of ion-exchange water was added to the reaction solution. The organic layer was extracted with ethyl acetate. The extracted organic layer was washed with water and saline. The washed organic layer was dried over magnesium sulfate, and then the solvent was removed under reduced pressure using a rotary evaporator. A compound obtained after the concentration was purified by silica gel column chromatography to obtain an intermediate M-m (3.50 g, 11.9 mmol, a yield of 95%).

[Formula 261]

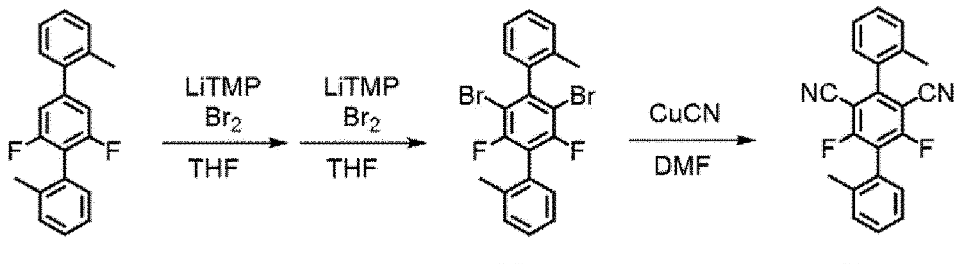


M-m M-n M-o

Under a nitrogen atmosphere, 2,2,6,6-tetramethylpiperidine (2.428 ml, 14.27 mmol) and THF (20 ml) were added to a 200-ml three-necked flask. After cooling the material in the three-necked flask to -78 degrees C in a dry ice/acetone bath, 14.3 ml (1.6M, hexane solution) of n-butyllithium was added dropwise. The obtained mixture solution was stirred for 20 minutes at 0 degrees C and cooled to -78 degrees C. Subsequently, a solution of the intermediate M-m (3.5 g, 11.89 mmol) dissolved in 20 ml of THF was added thereto and stirred for 15 minutes. After the stirring, bromine (1.218 ml, 23.78 mmol) was added to the reaction solution, returned to room temperature, and then stirred for 20 minutes. After the stirring, the reaction solution was added with saturated sodium bisulfite aqueous solution (100 mL). The organic layer was extracted with hexane. The extracted organic layer was washed with water and saline. The washed organic layer was dried over magnesium sulfate. The dried organic layer was concentrated using a rotary evaporator. A compound obtained after the concentration was passed through silica gel column chromatography and then the solvent was removed under reduced pressure using a rotary evaporator. A solution prepared by dissolving the obtained liquid in 20 ml of THF was added dropwise to another THF solution of LiTMP prepared as above at -78 degrees C and stirred for 15 minutes. Then, bromine (0.77 ml, 15.0 mmol) was added to the reaction solution, cooled to room temperature, and then stirred for 20 minutes. After the stirring, the reaction solution was added with saturated sodium bisulfite aqueous solution (100 mL). The organic layer was extracted with hexane, the extracted organic layer was washed with water and saline, the washed organic layer was dried over magnesium sulfate, and the dried organic layer was concentrated using a rotary evaporator. A compound obtained after the concentration was purified by silica gel column chromatography to obtain an intermediate M-n (2.75 g, 6.07 mmol, a yield of 51%). LiTMP is an abbreviation for lithium 2,2,6,6-tetramethylpiperidide.

Under a nitrogen atmosphere, the intermediate M-n (2.75 g, 6.07 mmol), cyanocopper (1.31 g, 14.6 mmol), and DMF (66ml) were put into a 1-L three-necked flask and stirred for five hours at 150 degrees C. 500mL of methylene chloride was added to the reaction mixture, filtered through Celite, and the filtrate was concentrated using an evaporator. The solid obtained after the concentration was purified by silica gel column chromatography to obtain 1.0 g of a white solid. The obtained white solid was identified as an intermediate M-o by analysis of GC-MS (a yield of 44%).

[Formula 262]

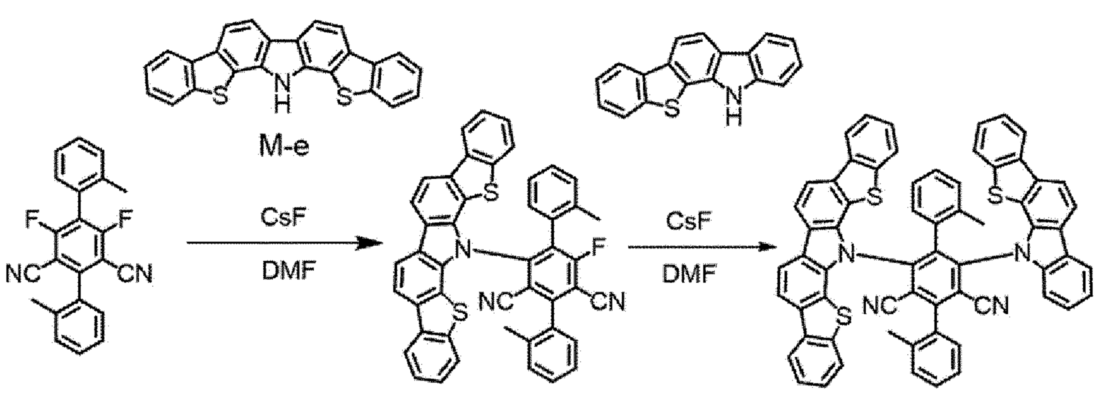


M-o M-p A-11

Under a nitrogen atmosphere, the intermediate M-o (1.0 g, 2.90 mmol), cesium fluoride (1.32 g, 8.71 mmol), the intermediate M-e (1.1 g, 2.90 mmol), and DMF (10.0 ml) were put into a 100-mL eggplant flask and stirred at room temperature for 20 hours. 50 ml of ion-exchange water was added to the reaction solution, and the deposited solid was collected by filtration. The collected solid was purified by silica gel column chromatography to obtain 1.5 g of a yellow

solid. The obtained yellow solid was identified as an intermediate M-p by analysis of ASAP-MS (a yield of 75%).

Under a nitrogen atmosphere, the intermediate M-p (1.54 g, 2.20 mmol), 12H-[1]Benzothieno[2,3-a]carbazole (0.718 g, 2.63 mmol), cesium fluoride (1.00 g, 6.56 mmol), and DMF (11.0 ml) were put into a 100-mL eggplant flask and stirred at 50 degrees C for one hour. Water (50 mL) was added to the reaction mixture. The deposited solid was purified by silica gel column chromatography to obtain 1.62 g of a yellow solid. The obtained yellow solid was identified as the compound A-11 by analysis of ASAP-MS (a yield of 77%).

Synthesis of Compound A-12

A synthesis method of a compound A-12 is described below.

[Formula 263]

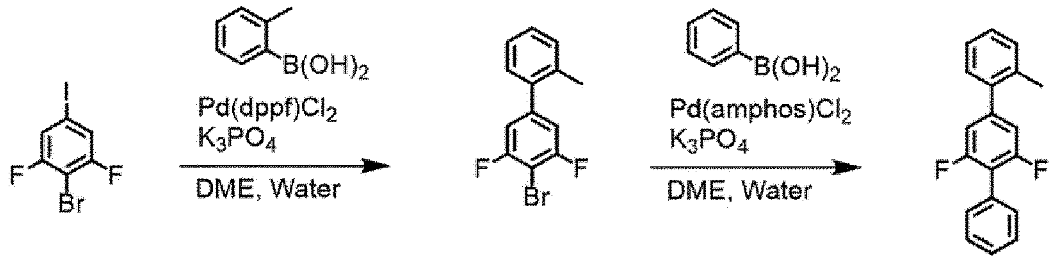


M-q M-r

Under a nitrogen atmosphere, 5-bromo-1,3-difluoro-2-iodobenzene (5.00 g, 15.68 mmol), o-tolylboronic acid (2.13 g, 15.68 mmol), potassium phosphate (9.98 g, 47.00 mmol), [1,1'-bis(diphenylphosphino)ferrocene]dichloropalladium(II) dichloromethane adduct (0.128 g, 0.157 mmol), DME (84.1 ml), and ion-exchange water (20.9 ml) were put into a 200-ml three-necked flask and stirred at room temperature for one hour. 100 ml of ion exchange water was added to the reaction solution. The organic layer was extracted with ethyl acetate. The extracted organic layer was washed with water and saline. The washed organic layer was dried over magnesium sulfate, and then the solvent was removed under reduced pressure using a rotary evaporator. A compound obtained after the concentration was purified by silica gel column chromatography to obtain an intermediate M-q (3.47 g, 12.3 mmol, a yield of 78%).

Under a nitrogen atmosphere, the intermediate M-q (3.47 g, 12.3 mmol), phenylboronic acid (2.26 g, 18.54 mmol), potassium phosphate (7.87 g, 37.1 mmol), bis[di-tert-butyl(4-dimethylaminophenyl)phosphine]dichloropalladium (0.101 g, 0.124 mmol), DME (65.9 ml), and ion-exchange water (16.5 ml) were put into a 200-ml three-necked flask and stirred at 60 degrees C for two hours. 100 ml of ion-exchange water was added to the reaction solution. The organic layer was extracted with ethyl acetate. The extracted organic layer was washed with water and saline. The washed organic layer was dried over magnesium sulfate, and then the solvent was removed under reduced pressure using a rotary evaporator. A compound obtained after the concentration was purified by silica gel column chromatography to obtain an intermediate M-r (3.15 g, 11.2 mmol, a yield of 90%).

[Formula 264]

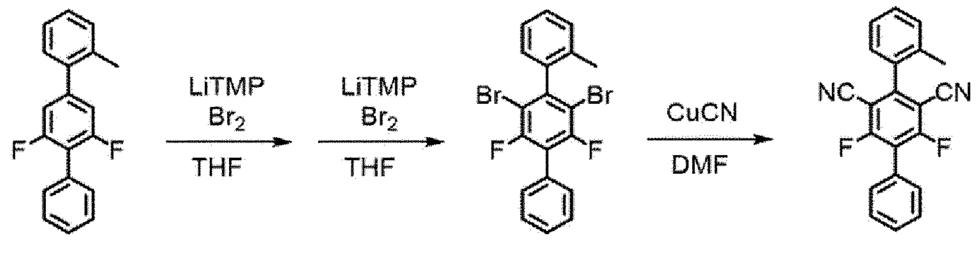


M-r M-s M-t

Under a nitrogen atmosphere, 2,2,6,6-tetramethylpiperidine (3.19 ml, 18.73 mmol) and THF (40 ml) were added to a 200-ml three-necked flask. After cooling the material in the three-necked flask to -78 degrees C in a dry ice/acetone bath, 18.7 ml (1.6M, hexane solution) of n-butyllithium was added dropwise. The obtained mixture solution was stirred

for 20 minutes at 0 degrees C and cooled to -78 degrees C. Subsequently, a solution of the intermediate M-r (3.15 g, 11.2 mmol) dissolved in 20 ml of THF was added thereto and stirred for 15 minutes. After the stirring, bromine (1.28 ml, 25.0 mmol) was added to the reaction solution, returned to room temperature, and then stirred for 20 minutes. After the stirring, the reaction solution was added with saturated sodium bisulfite aqueous solution (100 mL). The organic layer was extracted with hexane. The extracted organic layer was washed with water and saline. The washed organic layer was dried over magnesium sulfate. The dried organic layer was concentrated using a rotary evaporator. A compound obtained after the concentration was passed through silica gel column chromatography and then the solvent was removed under reduced pressure using a rotary evaporator. A solution prepared by dissolving the obtained liquid in 20 ml of THF was added dropwise to another THF solution of LiTMP prepared as above at -78 degrees C and stirred for 15 minutes. Then, bromine (1.28 ml, 25.0 mmol) was added to the reaction solution, cooled to room temperature, and then stirred for 20 minutes. After the stirring, the reaction solution was added with saturated sodium bisulfite aqueous solution (100 mL). The organic layer was extracted with hexane. The extracted organic layer was washed with water and saline. The washed organic layer was dried over magnesium sulfate. The dried organic layer was concentrated using a rotary evaporator. A compound obtained after the concentration was purified by silica gel column chromatography to obtain an intermediate M-s (4.16 g, 9.51 mmol, a yield of 85%).

Under a nitrogen atmosphere, the intermediate M-s (4.16 g, 9.51 mmol), cyanocopper (2.20 g, 24.6 mmol), and DMF (112 ml)were put into a 1-L three-necked flask and stirred for 10 hours at 160 degrees C. 500mL of methylene chloride was added to the reaction mixture, filtered through Celite, and the filtrate was concentrated using an evaporator. The solid obtained after the concentration was purified by silica gel column chromatography to obtain 1.57 g of a white solid. The obtained white solid was identified as an intermediate M-t by analysis of GC-MS (a yield of 42%).

[Formula 265]

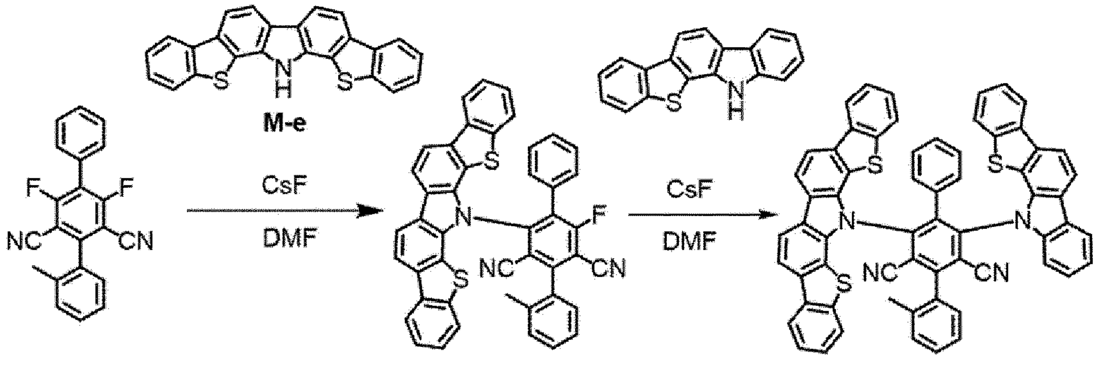


M-t M-u A-12

Under a nitrogen atmosphere, the intermediate M-t (1.0 g, 3.03 mmol), cesium fluoride (1.38 g, 9.08 mmol), the intermediate M-e (1.15 g, 3.03 mmol), and DMF (15.0 ml) were put into a 100-mL eggplant flask and stirred at room temperature for 20 hours. 50 ml of ion-exchange water was added to the reaction solution, and the deposited solid was collected by filtration. The collected solid was purified by silica gel column chromatography to obtain 1.55 g of a yellow solid. The obtained yellow solid was identified as an intermediate M-u by analysis of ASAP-MS (a yield of 74%).

Under a nitrogen atmosphere, the intermediate M-u (1.55 g, 2.25 mmol), 12H-[1]Benzothieno[2,3-a]carbazole (0.737 g, 2.70 mmol), cesium fluoride (1.02 g, 6.74 mmol), and DMF (11.0 ml) were put into a 100-mL eggplant flask and stirred at 50 degrees C for one hour. Water (50 mL) was added to the reaction mixture. The deposited solid was purified by silica gel column chromatography to obtain 1.6 g of a yellow solid. The obtained yellow solid was identified as the compound A-12 by analysis of ASAP-MS (a yield of 75%).

Synthesis of Compound A-13

A synthesis method of a compound A-13 is described below.

[Formula 266]

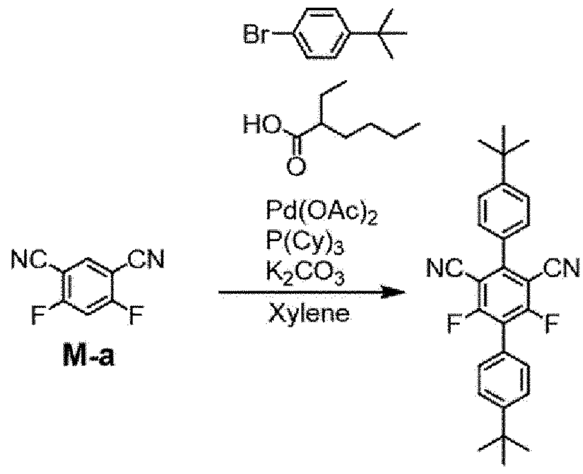


M-a

M-v

Under a nitrogen atmosphere, the intermediate M-a (20 g, 122 mmol), diacetoxypalladium (1.368 g, 6.09 mmol), tricyclohexylphosphine (5.13 g, 18.28 mmol), potassium carbonate (42.1 g, 305 mmol), and xylene (244 ml) were added to a 500-mL three-necked flask and stirred at room temperature for 30 minutes. Then, 2-ethylhexanoic acid (7.81 ml, 48.7 mmol) and 1-bromo-4-(tert-butyl)benzene (45.7 ml, 268 mmol) were added and stirred at 100 degrees C for five hours. The reaction solution was returned to room temperature. 200 ml of methylene chloride was added to the reaction solution and passed through Celite. Methylene chloride in the obtained solution was removed and the precipitated solid was filtered. The obtained solid was purified by silica gel column chromatography to obtain 36 g of a white solid. The obtained white solid was identified as an intermediate M-v by analysis of GC-MS (a yield of 69%).

[Formula 267]

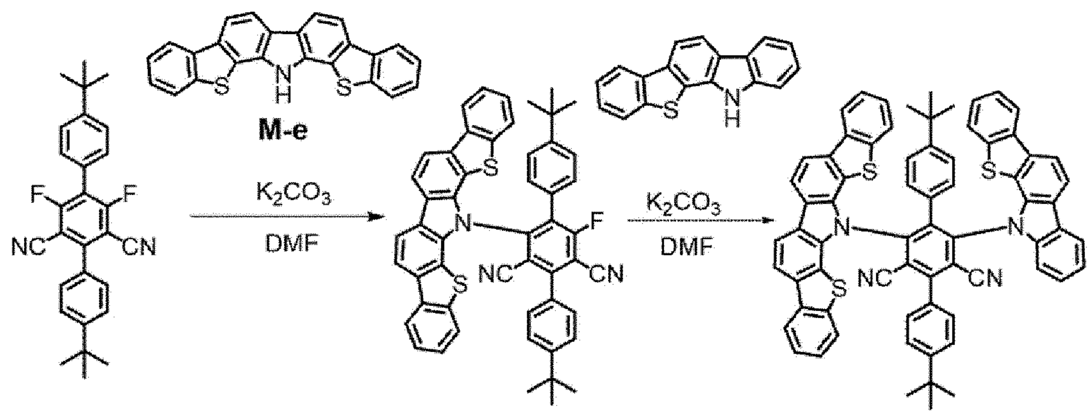


M-v M-w A-13

Under a nitrogen atmosphere, the intermediate M-v (7.2 g, 16.80 mmol), the intermediate M-e (6.38 g, 16.80 mmol), potassium carbonate (4.64 g, 33.6 mmol), and DMF (56.0 ml) were put into a 200-mL three-necked flask and stirred at 100 degrees C for four hours. 100 ml of ion-exchange water was added to the reaction solution, and the deposited solid was collected by filtration. The collected solid was purified by silica gel column chromatography to obtain 12 g of a yellow solid. The obtained yellow solid was identified as an intermediate M-w by analysis of ASAP-MS (a yield of 91%).

Under a nitrogen atmosphere, the intermediate M-w (3 g, 3.81 mmol), 12H-benzo[4,5]thieno[2,3-a]carbazole (1.249 g, 4.57 mmol), potassium carbonate (0.789 g, 5.71 mmol), and DMF (12.69 ml) were put into a 100 mL three-necked flask and stirred at 120 degrees C for four hours. Water (50 mL) was added to the reaction mixture. The deposited solid was purified by silica gel column chromatography to obtain 3.5 g of a yellow solid. The obtained yellow solid was identified as the compound A-13 by analysis of ASAP-MS (a yield of 88%).

Synthesis of Compound A-14

A synthesis method of a compound A-14 is described below.

[Formula 268]

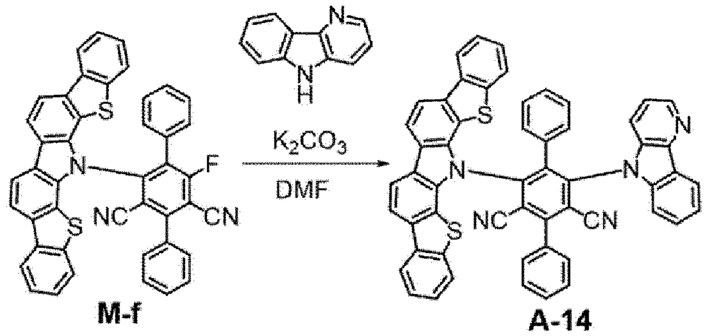


M-f

A-14

[0694]

Under a nitrogen atmosphere, 5H-pyrido[3,2-b]indole (0.684 g, 4.07 mmol), potassium carbonate (0.614 g, 4.44 mmol), the intermediate M-f (2.5 g, 3.70 mmol), and DMF (12.33 ml) were put into a 100-mL three-necked flask and stirred at 140 degrees C for four hours. Methanol (20 mL) was added to the reaction mixture. The deposited solid was purified by silica gel column chromatography to obtain 3.1 g of a yellow solid. The obtained yellow solid was identified as the compound A-14 by analysis of ASAP-MS (a yield of 88%).

Synthesis of Compound A-15

A synthesis method of a compound A-15 is described below.

[Formula 269]

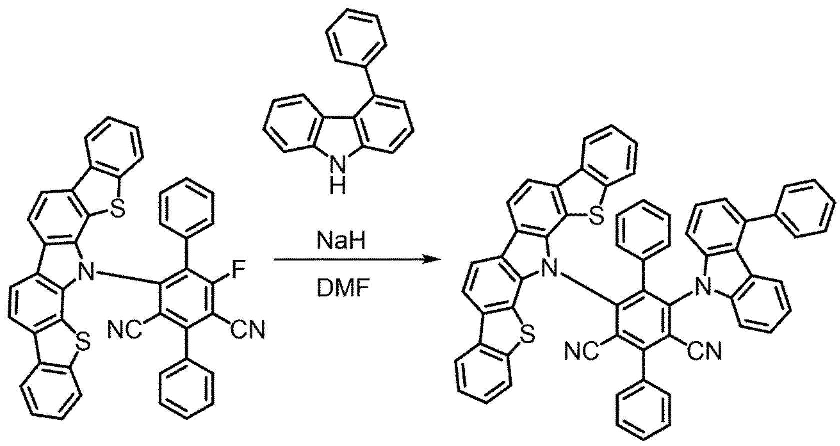


M-f

A-15

Under a nitrogen atmosphere, 4-phenyl-9H-carbazole (1.1 g, 4.44 mmol), sodium hydride (containing 40-mass% oil) (0.18 g, 4.44 mmol), and DMF (37 ml) were put into a 100-mL three-necked flask and stirred at 0 degrees C for 1 hour. Next, the intermediate M-f (2.5 g, 3.70 mmol) was put into the reaction solution at 0 degrees C and a temperature of the solution was slowly raised to room temperature. Then the solution was further stirred at room temperature for one hour. After the stirring, 30 ml of ion-exchange water was added to the reaction solution, and the deposited solid was collected by filtration. The obtained solid was purified by silica gel column chromatography to obtain a yellow solid. The obtained yellow solid was identified as the compound A-15 by analysis of ASAP-MS (a yield of 72%).

Synthesis of Compound A-16

A synthesis method of a compound A-16 is described below.

[Formula 270]

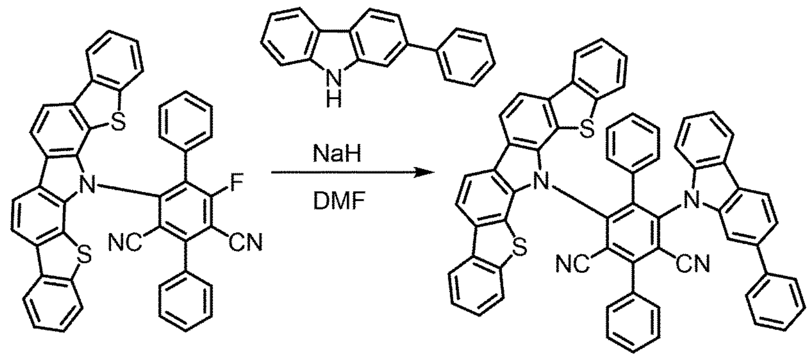


M-f A-16

Under a nitrogen atmosphere, 2-phenyl-9h-carbazol (1.1 g, 4.44 mmol), sodium hydride (containing 40-mass% oil) (0.18 g, 4.44 mmol), and DMF (37 ml) were put into a 100-mL three-necked flask and stirred at 0 degrees C for one hour. Next, the intermediate M-f (2.5 g, 3.70 mmol) was put into the reaction solution at 0 degrees C and a temperature of the solution was slowly raised to room temperature. Then the solution was further stirred at room temperature for one hour. 30 ml of ion-exchange water was added to the reaction mixture, and the deposited solid was collected by filtration. The obtained solid was purified by silica gel column chromatography to obtain a yellow solid. The obtained yellow solid was identified as the compound A-16 by analysis of ASAP-MS (a yield of 63%).

Synthesis of Compound A-17

A synthesis method of a compound A-17 is described below.

[Formula 271]

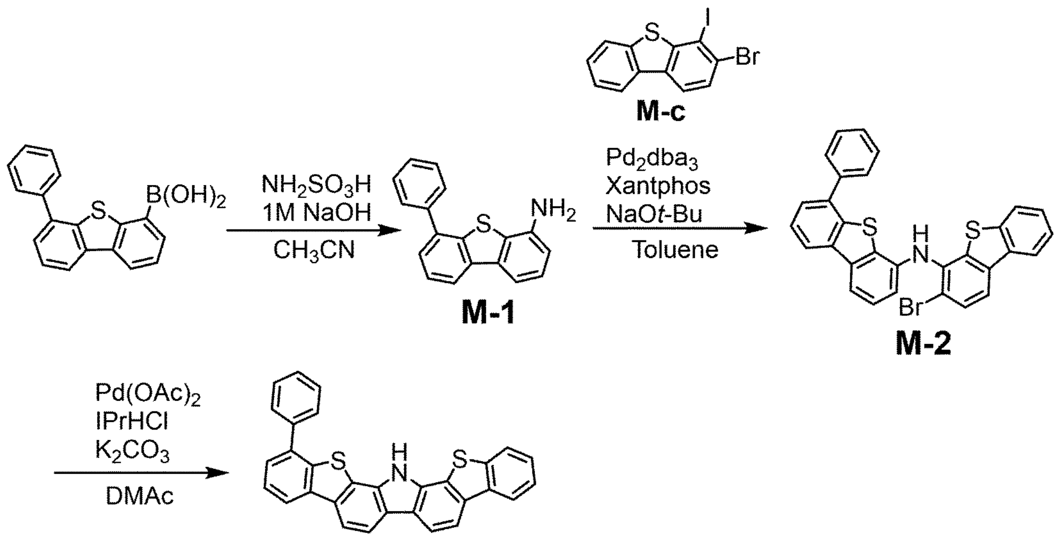


M-3

Under a nitrogen atmosphere, Boronic acid, B-(6-phenyl-4-dibenzothienyl) (40 g, 132 mmol), sulfamic acid (29.7 g, 263 mmol), acetonitrile (658 ml), and sodium hydroxide (1M) (881 ml, 881 mmol) were added to a 1000-mL three-necked flask and stirred at room temperature for 24 hours. After the stirring, the organic layer was extracted with toluene and collected. Next day, the solvent was removed using an evaporator from the collected organic layer. The

obtained solid was purified by silica gel column chromatography to obtain 17 g of a white solid. The obtained white solid was identified as an intermediate M-1 by analysis of GC-MS (a yield of 48%).

Under a nitrogen atmosphere, the intermediate M-1 (10.6 g, 38.6 mmol), the intermediate M-c (15 g, 38.6 mmol), tris(dibenzylideneacetone)dipalladium(0) (0.353 g, 0.386 mmol), Xantphos (1.13 g, 1.54 mmol), sodium tert-butoxide (5.56 g, 57.8 mmol), and toluene (129 ml) were added to a 500-ml three-necked flask. The obtained mixture was heated at 100 degrees C for eight hours with stirring and then cooled to room temperature (25 degrees C). The solution obtained after the cooling was purified by silica gel column chromatography to obtain 25 g of a white solid. The obtained white solid was identified as an intermediate M-2 by analysis of ASAP-MS (a yield of 77%).

Under a nitrogen atmosphere, the intermediate M-2 (8.5 g, 15.84 mmol), 1,3-bis(2,6-diisopropylphenyl)imidazolium chloride (IPrHCl) (0.202 g, 0.475 mmol), palladium(II) acetate (0.053 g, 0.238 mmol), potassium carbonate (4.60 g, 33.3 mmol), and N,N-dimethylacetamide (DMAc) (52.8 ml) were added to a 200-ml three-necked flask. The obtained mixture was stirred at 160 degrees C for 10 hours and then cooled to room temperature (25 degrees C). The deposited solid was collected by filtration and washed with methanol to obtain 7.2 g of a white solid. The obtained white solid was identified as an intermediate M-3 by analysis of ASAP-MS (a yield of 73%).

[Formula 272]

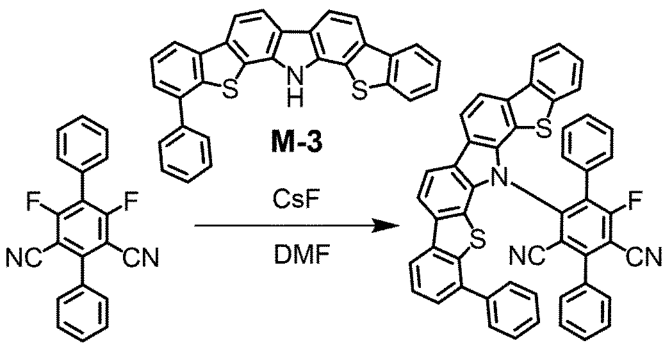


M-b M-4

Under a nitrogen atmosphere, the intermediate M-3 (4.0 g, 8.8 mmol), cesium fluoride (2.7 g, 17.6 mmol), the intermediate M-b (2.9 g, 9.22 mmol), and DMF (30 ml) were put into a 100-mL eggplant flask and stirred at room temperature for 12 hours. After the stirring, 50 ml of ion-exchange water was added to the reaction solution, and the deposited solid was collected by filtration. The collected solid was purified by silica gel column chromatography to obtain 5.5 g of a yellow solid. The obtained yellow solid was identified as an intermediate M-4 by analysis of ASAP-MS (a yield of 83%).

[Formula 273]

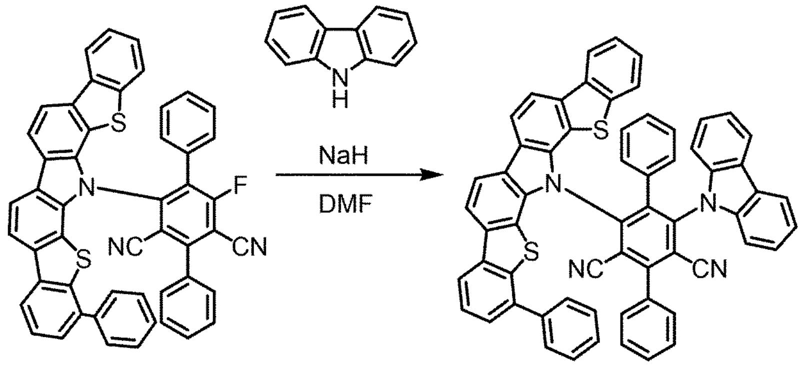


M-4 A-17

Under a nitrogen atmosphere, 9H-carbazole (1.0 g, 45.98 mmol), sodium hydride (containing 40-mass% oil) (0.24 g, 5.98 mmol), and DMF (40 ml) were put into a 100-mL three-necked flask and stirred at 0 degrees C for one hour. Next, the intermediate M-4 (3.0 g, 3.99 mmol) was put into the reaction solution at 0 degrees C and a temperature of the solution was slowly raised to room temperature. Then the solution was further stirred at room temperature for one

hour. After the stirring, 30 ml of ion-exchange water was added to the reaction solution, and the deposited solid was collected by filtration. The obtained solid was purified by silica gel column chromatography to obtain 2.9 g of a yellow solid. The obtained yellow solid was identified as the compound A-17 by analysis of ASAP-MS (a yield of 81%).

Synthesis of Compound A-18

A synthesis method of a compound A-18 is described below.

[Formula 274]

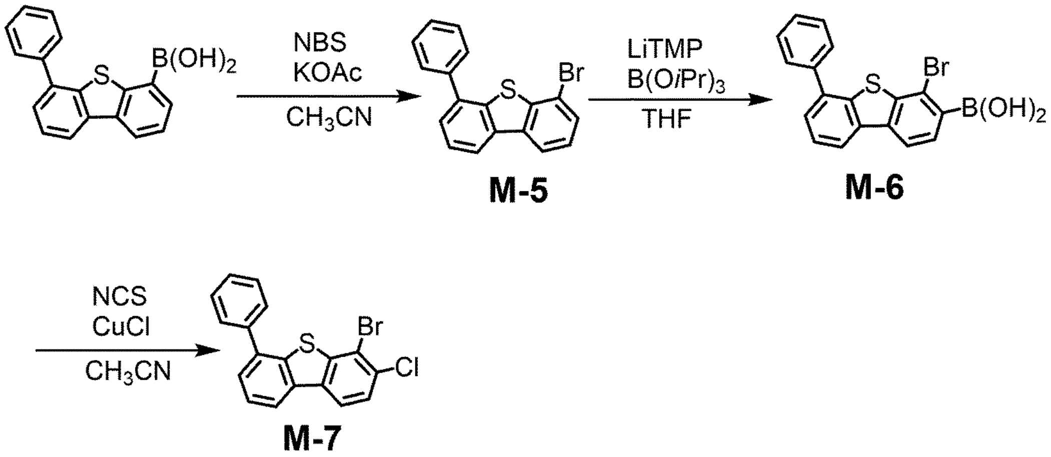


Under a nitrogen atmosphere, Boronic acid, B-(6-phenyl-4-dibenzothienyl)-(50 g, 164 mmol), N-bromosuccinimide (NBS) (32.2 g, 181 mmol), potassium acetate (KOAc) (3.23 g, 32.9 mmol), and acetonitrile (470 ml) were added to a 1000-mL three-necked flask and stirred at 50 degrees C for six hours. After the stirring, ion-exchange water (400 mL) was added to the reaction mixture. The deposited solid was purified by silica gel column chromatography to obtain 44 g of a white solid. The obtained white solid was identified as an intermediate M-5 by analysis of ASAP-MS (a yield of 79%).

Under a nitrogen atmosphere, 2,2,6,6-tetramethylpiperidine (26.1 ml, 153 mmol) and THF (236 ml) were added to a 1000-mL three-necked flask and cooled with ice to 0 degrees C using an ice bath. Normal butyl lithium (1.6M hexane solution) (96 ml, 153 mmol) was added dropwise to the reaction solution cooled using ice. After the addition, the reaction solution was stirred at 0 degrees C for 30 minutes. Next, the reaction solution was cooled to -78 degrees C using a dry ice/methanol bath. After the cooling, triisopropyl borate (33.3 g, 177 mmol) and the intermediate M-5 (40 g, 118 mmol) were sequentially added to the reaction solution. The obtained reaction solution was stirred while being slowly heated from -78 degrees C to room temperature. After the reaction was completed, 100 ml of 10% hydrochloric acid was added dropwise. After the addition, the organic layer was collected and the obtained solid was washed with toluene to obtain 40 g of a white solid. The obtained white solid was identified as an intermediate M-6 by analysis of ASAP-MS (a yield of 89%).

Under a nitrogen atmosphere, the intermediate M-6 (40 g, 104 mmol), N-chlorosuccinimide (NCS) (13.94 g, 104 mmol), copper(I) chloride (10.34 g, 104 mmol), and acetonitrile (348 ml) were added to 1000-mL three-necked flask and stirred at 60 degrees C for six hours. 300 mL of methylene chloride was added to the reaction mixture and passed through Celite. The obtained solution was concentrated. The obtained solid was purified by silica gel column chromatography to obtain 28 g of a white solid. The obtained white solid was identified as an intermediate M-7 by analysis of ASAP-MS (a yield of 72%).

[Formula 275]

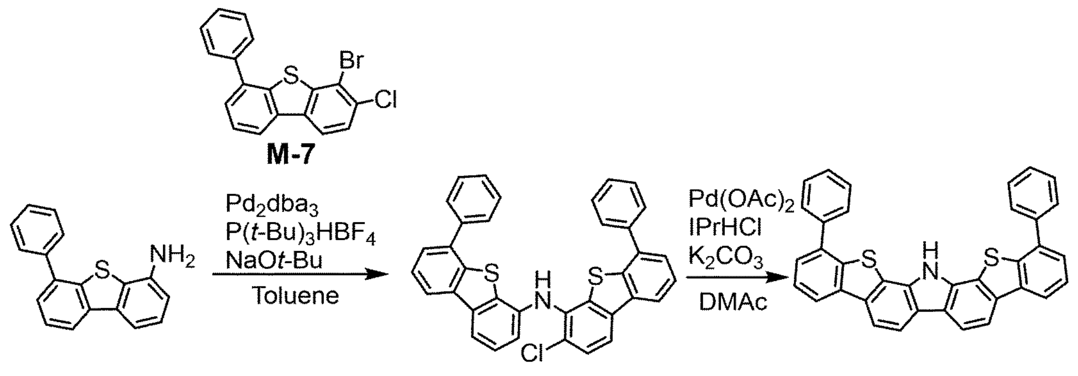


M-7

M-1 M-8 M-9

Under a nitrogen atmosphere, the intermediate M-1 (3.5 g, 12.6 mmol), the intermediate M-7 (4.7 g, 12.6 mmol), tris(dibenzylideneacetone)dipalladium(0) (0.353 g, 0.386 mmol), tri-tert-butylphosphonium tetrafluoroborate (0.17 g, 0.19 mmol), sodium tert-butoxide (1.8 g, 19.0 mmol), and toluene (42 ml) were added to a 100-ml three-necked flask. The obtained mixture was heated at 60 degrees C for eight hours with stirring and then cooled to room temperature (25 degrees C). The obtained solution was purified by silica gel column chromatography to obtain 5 g of a white solid. The obtained white solid was identified as an intermediate M-8 by analysis of ASAP-MS (a yield of 70%).

Under a nitrogen atmosphere, the intermediate M-8 (25 g, 44 mmol), 1,3-bis(2,6-diisopropylphenyl)imidazolium chloride (IPrHCl) (0.202 g, 0.475 mmol), palladium(II) acetate (0.15 g, 0.66 mmol), potassium carbonate (13 g, 92 mmol), and N,N-dimethylacetamide (DMAc) (220 ml) were added to a 500-ml three-necked flask. The obtained mixture was stirred at 160 degrees C for 10 hours and then cooled to room temperature (25 degrees C). The deposited solid was collected by filtration and washed with methanol to obtain 18 g of a white solid. The obtained white solid was identified as an intermediate M-9 by analysis of ASAP-MS (a yield of 77%).

[Formula 276]

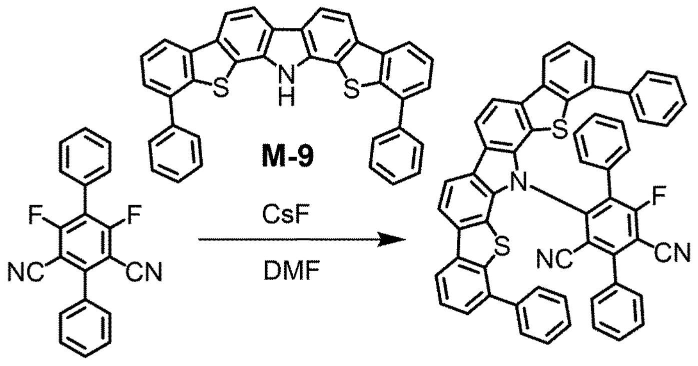


M-9

M-b M-10

Under a nitrogen atmosphere, the intermediate M-9 (18 g, 8.8 mmol), cesium fluoride (7.7 g, 51 mmol), the intermediate M-b (11 g, 35.5 mmol), and DMF (230 ml) were put into a 500-mL eggplant flask and stirred at room temperature for 12 hours. 200 ml of ion-exchange water was added to the reaction solution, and the deposited solid was collected by filtration. The collected solid was purified by silica gel column chromatography to obtain 20 g of a yellow solid. The obtained yellow solid was identified as an intermediate M-10 by analysis of ASAP-MS (a yield of 71%).

[Formula 277]

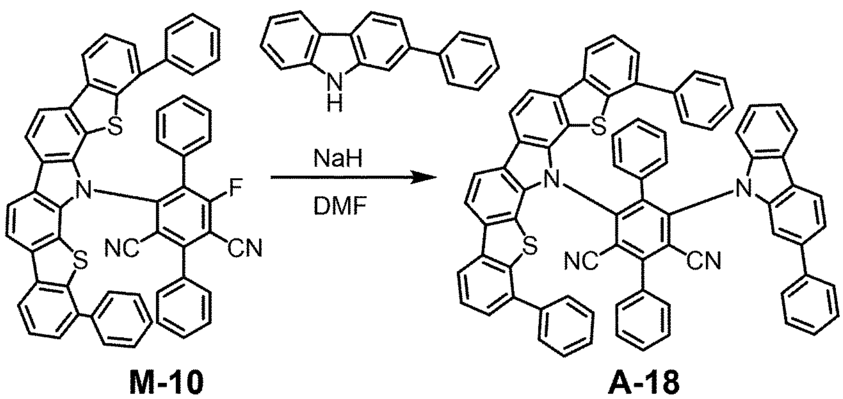


M-10 A-18

Under a nitrogen atmosphere, 2-phenyl-9h-carbazol (1.4 g, 5.80 mmol), sodium hydride (containing 40-mass% oil) (0.23 g, 5.80 mmol), and DMF (50 ml) were put into a 100-mL three-necked flask and stirred at 0 degrees C for one hour. Next, the intermediate M-10 (4.0 g, 4.83 mmol) was put into the reaction solution at 0 degrees C and a temperature of the solution was slowly raised to room temperature. Then the solution was further stirred at room temperature for one hour. 30 ml of ion-exchange water was added to the reaction mixture, and the deposited solid was collected by filtration. The obtained solid was purified by silica gel column chromatography to obtain 2.9 g of a yellow solid. The obtained yellow solid was identified as the compound A-18 by analysis of ASAP-MS (a yield of 89%).

Synthesis of Compound A-19

A synthesis method of a compound A-19 is described below.

[Formula 278]

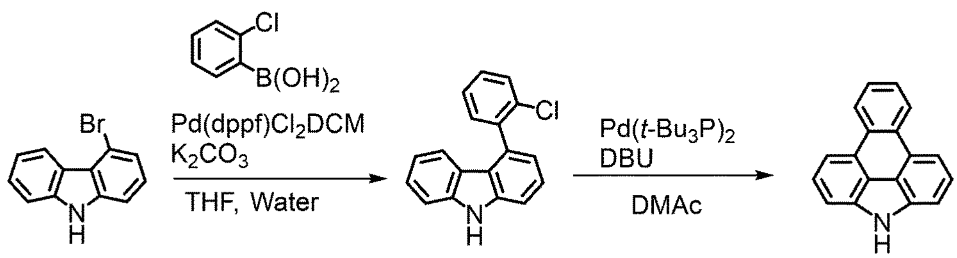


M-11 M-12

Under a nitrogen atmosphere, 4-bromo-9H-carbazole (66.6 g, 271 mmol), (2-chlorophenyl)boronic acid (44.4 g, 284 mmol), potassium carbonate (56.1 g, 406 mmol), [1,1'-bis(diphenylphosphino)ferrocene]dichloropalladium(II) dichloromethane adduct (1.1 g, 1.35 mmol), THF (200ml), and ion-exchange water (65 ml) were put into a 500-mL three-necked flask and stirred at room temperature for eight hours. After the stirring, the reaction solution was concentrated. 100 ml of ion-exchange water was added to the concentrated reaction solution. The organic layer was extracted with toluene. The extracted organic layer was washed with water and saline and dried over magnesium sulfate. Subsequently, the solvent was removed under reduced pressure using a rotary evaporator. A compound obtained after the solvent was removed under reduced pressure was purified by silica gel column chromatography to obtain a white solid. The obtained white solid was identified as an intermediate M-11 by analysis of ASAP-MS (a yield of 96%).

Under a nitrogen atmosphere, the intermediate M-11 (6 g, 21.6 mmol), diazabicycloundecene (DBU) (9.67 ml, 64.8 mmol), bis(tri-tert-butylphosphine)palladium(0) (0.552 g, 1.080 mmol), and N,N-dimethylacetamide (DMAc) (22 ml) were added to a 100-ml three-necked flask, and the obtained solution was heated under reflux with stirring for 20 hours. After the reaction was completed, 100 ml of ion-exchange water was added to the reaction solution. The organic layer was extracted with toluene. The extracted organic layer was washed with water and saline and dried over magnesium sulfate. Subsequently, the solvent was removed under reduced pressure using a rotary evaporator. A compound obtained after the solvent was removed under reduced pressure was purified by silica gel column chromatography to obtain 2.8 g of a white solid. The obtained white solid was identified as an intermediate M-12 by analysis of ASAP-MS (a yield of 54%).

[Formula 279]

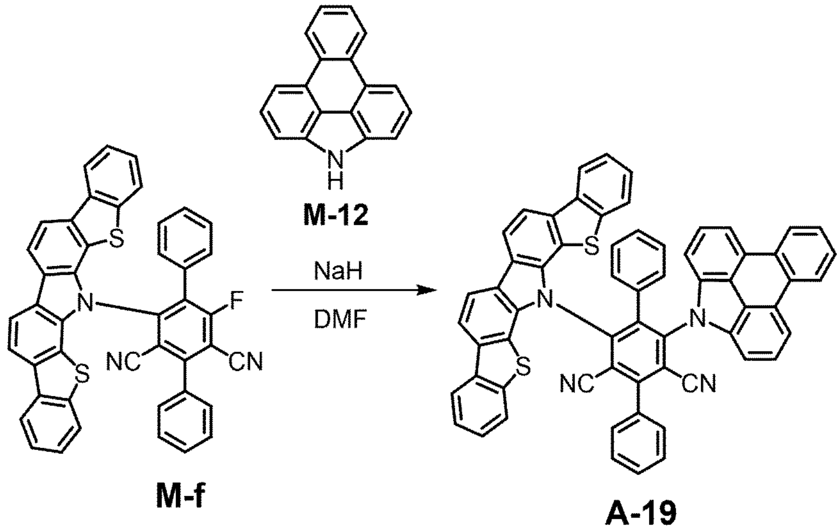


M-f

A-19

Under a nitrogen atmosphere, the intermediate M-12 (1.29 g, 5.33 mmol), sodium hydride (containing 40-mass% oil) (0.21 g, 5.33 mmol), and DMF (45 ml) were put into a 200-mL three-necked flask and stirred at 0 degrees C for one hour. Next, the intermediate M-f (3.0 g, 4.44 mmol) was put into the reaction solution at 0 degrees C and a temperature of the solution was slowly raised to room temperature. Then the solution was further stirred at room temperature for one hour. 50 ml of ethyl acetate was added to the reaction mixture, and the obtained solid was collected by filtration. The obtained solid was purified by silica gel column chromatography to obtain 3.4 g of a yellow solid. The obtained yellow solid was identified as the compound A-19 by analysis of ASAP-MS (a yield of 85%).

Synthesis of Compound A-20

A synthesis method of a compound A-20 is described below.

[Formula 280]

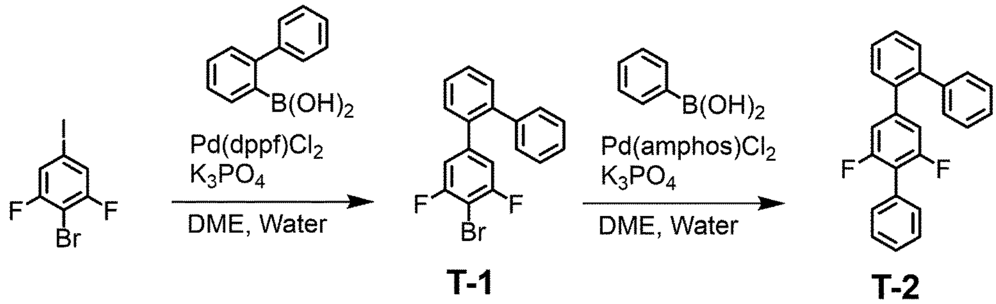


T-1

T-2

Under a nitrogen atmosphere, 2-bromo-1,3-difluoro-5-iodobenzene (7.00 g, 15.68 mmol), [1,1'-biphenyl]-2-yl boronic acid (4.78 g, 24.15 mmol), potassium phosphate (13.98 g, 65.90 mmol), [1,1'-bis(diphenylphosphino)ferrocene]dichloropalladium(II) dichloromethane adduct (0.359 g, 0.439 mmol), DME (117 ml), and ion-exchange water (29 ml) were put into a 200-ml three-necked flask and stirred at room temperature for eight hours. After the reaction solution was concentrated, 20 ml of ion-exchange water was added to the concentrated reaction solution. The organic layer was extracted with dichloromethane. The extracted organic layer was washed with water and saline and dried over magnesium sulfate. Subsequently, the solvent was removed under reduced pressure using a rotary evaporator. A compound obtained after the solvent was removed under reduced pressure was purified by silica gel column chromatography to obtain an intermediate T-1 (6.97 g, 20.05 mmol, a yield of 91%).

Under a nitrogen atmosphere, the intermediate T-1 (6.97 g, 20.05 mmol), phenylboronic acid (2.95 g, 24.23 mmol), potassium carbonate (12.86 g, 60.6 mmol), 1,1'-bis(diphenylphosphino)ferrocene]dichloropalladium(II) dichloromethane adduct (0.330 g, 0.404 mmol), DME (108 ml), and ion-exchange water (26.9 ml) were put into a 200-ml three-necked flask and stirred at 60 degrees C for four hours. After the reaction solution was concentrated, 20 ml of ion-exchange water was added to the concentrated reaction solution. The organic layer was extracted with dichloromethane. The extracted organic layer was washed with water and saline and dried over magnesium sulfate. Subsequently, the solvent was removed under reduced pressure using a rotary evaporator. A compound obtained after the solvent was removed under reduced pressure was purified by silica gel column chromatography to obtain an intermediate T-2 (6.23 g, 18.1 mmol, a yield of 90%).

[Formula 281]

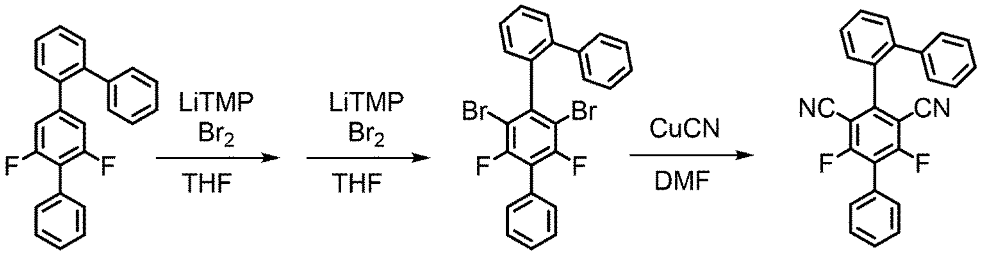


T-2 T-3 T-4

Under a nitrogen atmosphere, 2,2,6,6-tetramethylpiperidine (6.16 ml, 36.2 mmol) and THF (60 ml) were added to a 200-ml three-necked flask. After cooling the material in the three-necked flask to -78 degrees C in a dry ice/acetone bath, 22.6 ml (1.6M, hexane solution) of n-butyllithium was added dropwise. The obtained mixture solution was stirred for 20 minutes at 0 degrees C and cooled to -78 degrees C. Subsequently, a solution of the intermediate T-2 (6.2 g, 18.1 mmol) dissolved in 60 ml of THF was added thereto and stirred for 20 minutes. Bromine (2.32 ml, 45.0 mmol) was added to the reaction solution, returned to room temperature, and then stirred for 20 minutes. The reaction solution was added with saturated sodium bisulfite aqueous solution (100 mL). The organic layer was extracted with ethyl acetate, the extracted organic layer was washed with water and saline, the washed organic layer was dried over magnesium sulfate, and the dried organic layer was concentrated using a rotary evaporator. A compound obtained after the concentration was passed through silica gel column chromatography and then the solvent was removed under reduced pressure using a rotary evaporator. A solution prepared by dissolving the obtained liquid in 60 ml of THF was added dropwise to another THF solution of LiTMP prepared as above at -78°C and stirred for 15 minutes. Bromine (2.32 ml, 45.0 mmol) was added to the reaction solution, returned to room temperature, and then stirred for 20 minutes. The reaction solution was added with saturated sodium bisulfite aqueous solution (100 mL). The organic layer was extracted with hexane, the extracted organic layer was washed with water and saline, the washed organic layer was dried over magnesium sulfate, and the dried organic layer was concentrated using a rotary evaporator. A compound obtained after the concentration was purified by silica gel column chromatography to obtain an intermediate T-3 (8.1 g, 16.2 mmol, a yield of 89%).

Under a nitrogen atmosphere, the intermediate T-3 (8.1 g, 16.2 mmol), cyanocopper (3.19 g, 35.6 mmol), and NMP (162ml) were put into a 1-L three-necked flask and stirred at 180 degrees C for 10 hours. 500mL of methylene chloride was added to the reaction mixture, filtered through Celite, and the filtrate was concentrated using an evaporator. The obtained solid was purified by silica gel column chromatography to obtain 1.74 g of a white solid. The obtained white solid was identified as an intermediate T-4 by analysis of GC-MS (a yield of 27%).

[Formula 282]

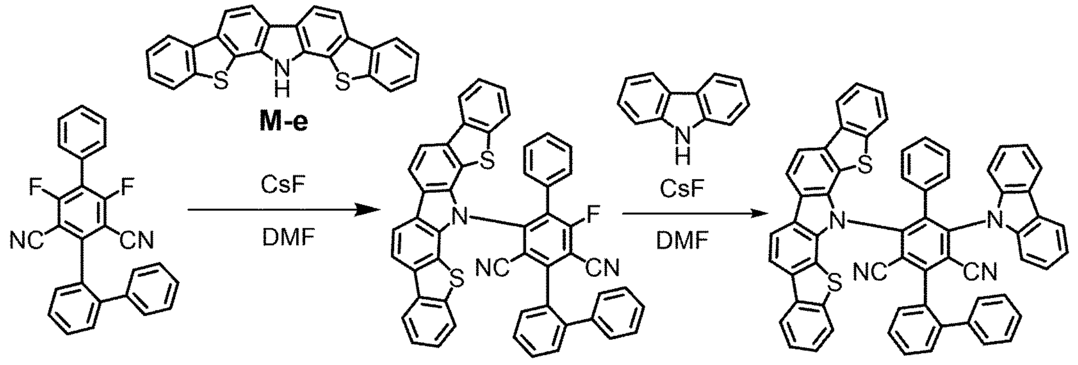


T-4 T-5 A-20

Under a nitrogen atmosphere, the intermediate T-4 (1.95 g, 4.97 mmol), cesium fluoride (2.27 g, 14.9 mmol), the intermediate M-e (1.89 g, 4.97 mmol), and DMF (49.7 ml) were put into a 100-mL eggplant flask and stirred at room temperature for 20 hours. 50 ml of ion-exchange water was added to the reaction solution, and the deposited solid was collected by filtration. The collected solid was purified by silica gel column chromatography to obtain 2.9 g of a yellow solid. The obtained yellow solid was identified as an intermediate T-5 by analysis of ASAP-MS (a yield of 78%).

Under a nitrogen atmosphere, the intermediate T-5 (1.5 g, 2.00 mmol), 9H-carbazole (0.567 g, 3.39 mmol), cesium fluoride (0.909 g, 5.98 mmol), and DMF (20 ml) were put into a 100-mL eggplant flask and stirred at 80 degrees C for four hours. Methanol (200mL) was added to the reaction mixture. The deposited solid was purified by column chromatography to obtain 0.9 g of a yellow solid. The obtained yellow solid was identified as the compound A-20 by analysis of ASAP-MS (a yield of 50%).

Synthesis of Compound A-21

A synthesis method of a compound A-21 is described below.

[Formula 283]

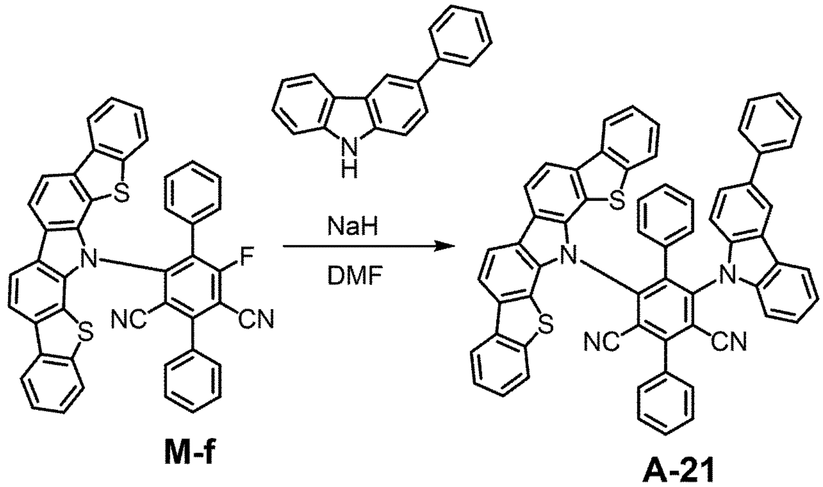


M-f A-21

Under a nitrogen atmosphere, 3-phenyl-9H-carbazole (1.1 g, 4.44 mmol), sodium hydride (containing 40-mass% oil) (0.18 g, 4.44 mmol), and DMF (37 ml) were put into a 100-mL three-necked flask and stirred at 0 degrees C for one hour. Next, the intermediate M-f (2.5 g, 3.70 mmol) was put into the reaction mixture at 0 degrees C and a temperature of the solution was slowly raised to room temperature. Then the solution was further stirred at room temperature for one hour. 30 ml of ion-exchange water was added to the reaction mixture, and the deposited solid was collected by filtration. The obtained solid was purified by silica gel column chromatography to obtain 2.7 g of a yellow solid. The obtained yellow solid was identified as the compound A-21 by analysis of ASAP-MS (a yield of 81%).

Synthesis of Compound A-22

A synthesis method of a compound A-22 is described below.

[Formula 284]

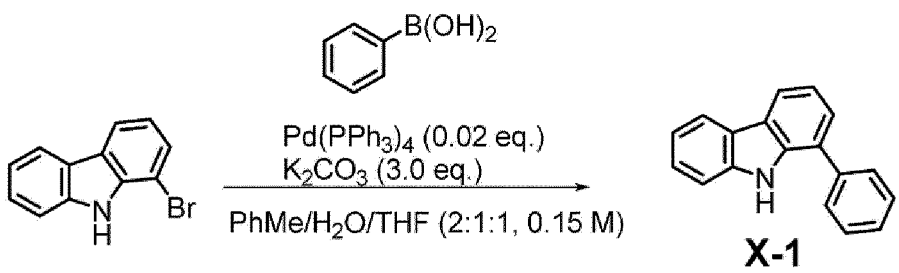


Under a nitrogen atmosphere, 1-bromo-9H-carbazole (10 g, 40.6 mmol), phenylboronic acid (7.43 g, 60.9 mmol), potassium carbonate (16.85 g, 122 mmol), tetrakistriphenylphosphine palladium (0.939 g, 0.813 mmol), toluene (135 ml), THF (67.7 ml), and ion-exchange water (67.7 ml) were put into a 500-ml three-necked flask and stirred at 100 degrees C for five hours. After the reaction solution was left to cool to room temperature, ion-exchange water was added to the reaction solution, from which the organic layer was extracted with toluene. The organic layer was dried over sodium sulfate. Subsequently, the solvent was removed under reduced pressure using a rotary evaporator. A compound obtained after the concentration was purified by silica gel column chromatography (hexane/dichloromethane = 80%:20%) to obtain an intermediate X-1 (8.50 g, 34.9 mmol, a yield of 86%).

[Formula 285]

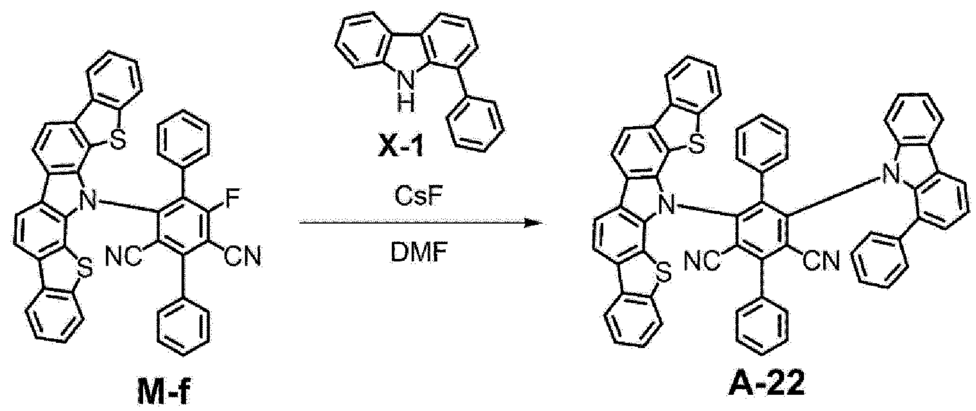


Under a nitrogen atmosphere, the intermediate M-f (1.58 g, 2.34 mmol), the intermediate X-1 (0.683 g, 2.81 mmol), cesium fluoride (1.776 g, 11.69 mmol), and DMF (23.4 ml) were put into a 100-mL three-necked flask and stirred at 130 degrees C for five hours and at 150 degrees C for four hours. After the reaction mixture was left to cool to room temperature, water was added to the reaction mixture. The deposited solid was washed with methanol. The washed solid was purified by column chromatography to obtain 0.339 g of a yellow solid. The obtained yellow solid was identified as the compound A-22 by analysis of ASAP-MS (a yield of 16%).

Synthesis of Compound A-23

A synthesis method of a compound A-23 is described below.

[Formula 286]

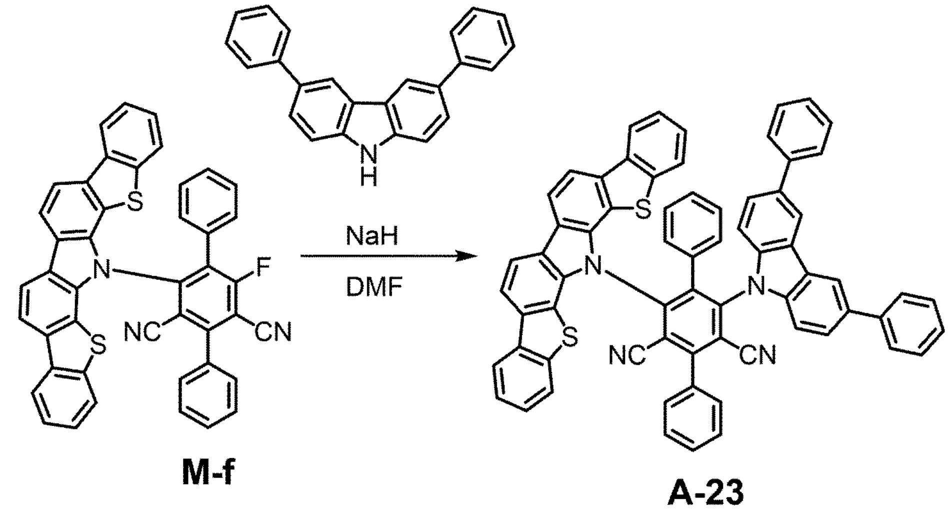


M-f

A-23

Under a nitrogen atmosphere, 3,6-diphenylcarbazole (1.418 g, 4.44 mmol) and DMF (37.0 ml) were put into a 100-ml three-necked flask and cooled at 0 degrees C using ice. Sodium hydride (containing 40-mass% oil) (0.178 g, 4.44 mmol) was put into the reaction solution after the ice-cooling, and stirred at 0 degrees C for one hour. The intermediate M-f (2.5 g, 3.70 mmol) was put into the reaction solution at 0 degrees C and a temperature of the solution was slowly raised to room temperature. Then the solution was further stirred at room temperature for one hour. 30 ml of ion-exchange water was added to the reaction mixture, and the deposited solid was collected by filtration. The obtained solid was purified by silica gel column chromatography to obtain 3.3 g of a yellow solid. The obtained yellow solid was identified as the compound A-23 by analysis of ASAP-MS (a yield of 91%).

Synthesis of Compound A-24

A synthesis method of a compound A-24 is described below.

[Formula 287]

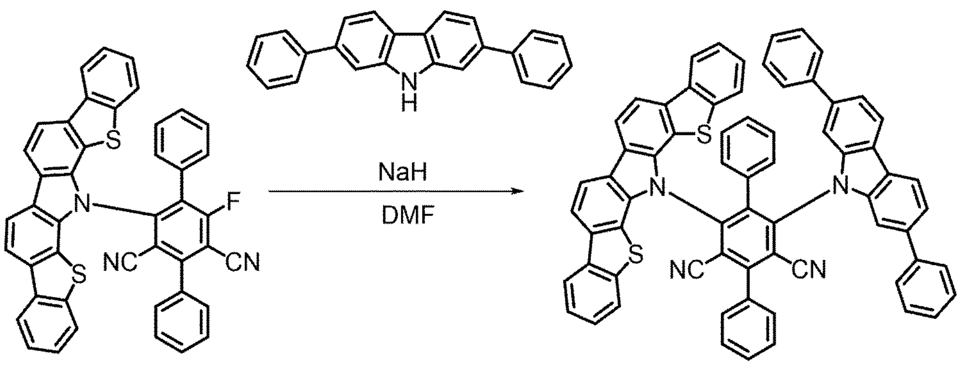


M-f

A-24

Under a nitrogen atmosphere, 2,7-diphenylcarbazole (1.418 g, 4.44 mmol) and DMF (37.0 ml) were put into a 100-ml three-necked flask and cooled at 0 degrees C using ice. Sodium hydride (containing 40-mass% oil) (0.178 g, 4.44 mmol) was put into the reaction solution after the ice-cooling, and stirred at 0 degrees C for one hour. The intermediate M-f (2.5 g, 3.70 mmol) was put into the reaction solution at 0 degrees C and a temperature of the solution was slowly raised to room temperature. Then the solution was further stirred at room temperature for one hour. 30 ml of ion-exchange water was added to the reaction mixture, and the deposited solid was collected by filtration. The obtained solid was purified by silica gel column chromatography to obtain 2.8 g of a yellow solid. The obtained yellow solid was identified as the compound A-24 by analysis of ASAP-MS (a yield of 78%).

Synthesis of Compound A-25

A synthesis method of a compound A-25 is described below.

[Formula 288]

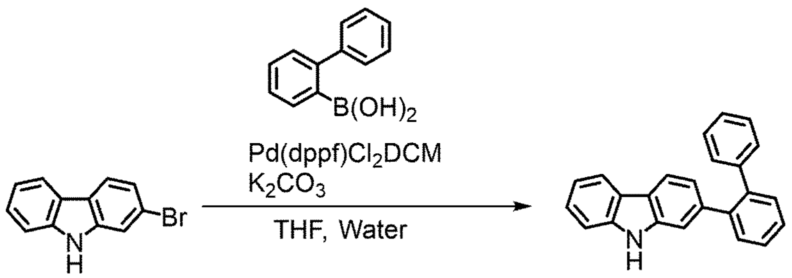


**M-13**

Under a nitrogen atmosphere, 2-bromocarbazole (10 g, 40.6 mmol), 2-biphenylboronic acid (8.45 g, 42.7 mmol), potassium carbonate (8.42 g, 60.9 mmol), [1,1'-bis(diphenylphosphino)ferrocene]dichloropalladium(II) dichloromethane adduct (1.1 g, 1.35 mmol), THF (30 ml), and ion-exchange water (10 ml) were put into a 100-ml three-necked flask and stirred at room temperature for eight hours. After the reaction solution was concentrated, 30 ml of ion-exchange water was added to the concentrated reaction solution. The organic layer was extracted with toluene. The extracted organic layer was washed with water and saline and dried over magnesium sulfate. Subsequently, the solvent was removed under reduced pressure using a rotary evaporator. A compound obtained after the solvent was removed under reduced pressure was purified by silica gel column chromatography to obtain 11 g of a white solid. The obtained white solid was identified as an intermediate M-13 by analysis of ASAP-MS (a yield of 85%).

[Formula 289]

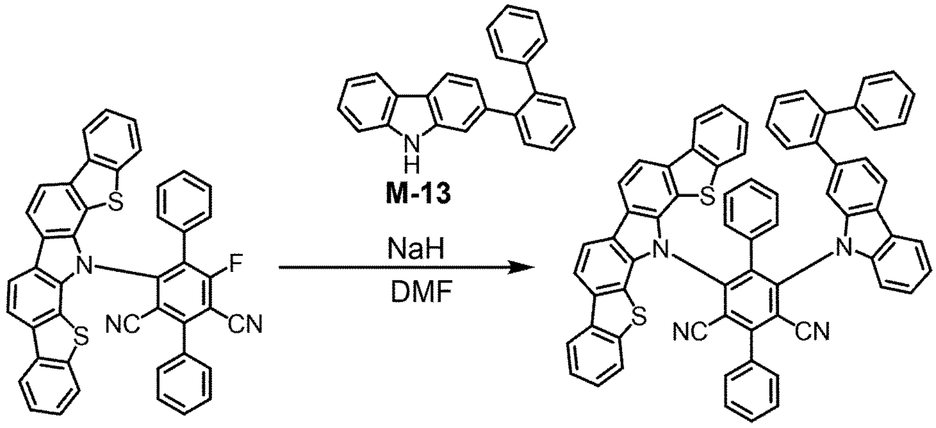


**M-f** **A-25**

Under a nitrogen atmosphere, the intermediate M-13 (1.42 g, 4.44 mmol) and DMF (40 ml) were put into a 100-ml three-necked flask and cooled at 0 degrees C using ice. Sodium hydride (containing 40-mass% oil) (0.18 g, 4.44 mmol) was put into the reaction solution after the ice-cooling, and stirred at 0 degrees C for one hour. Next, the intermediate M-f (2.5 g, 3.70 mmol) was put into the reaction solution at 0 degrees C and a temperature of the solution was slowly raised to room temperature. Then the solution was further stirred at room temperature for one hour. 30 ml of ion-exchange water was added to the reaction mixture, and the deposited solid was collected by filtration. The obtained solid was purified by silica gel column chromatography to obtain 1.9 g of a yellow solid. The obtained yellow solid was identified as the compound A-25 by analysis of ASAP-MS (a yield of 78%).

Synthesis of Compound A-26

A synthesis method of a compound A-26 is described below.

[Formula 290]

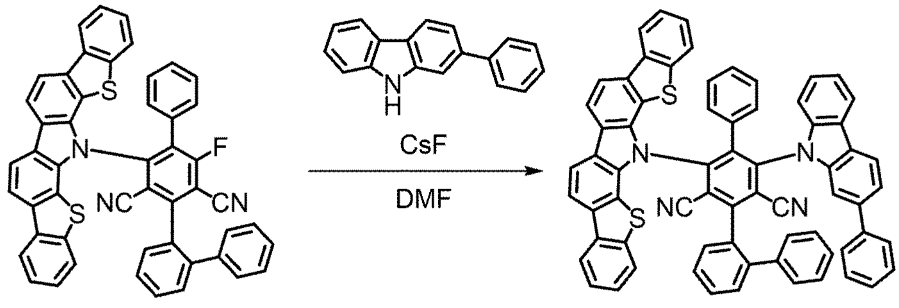


T-5 A-26

Under a nitrogen atmosphere, the intermediate T-5 (1.4 g, 1.86 mmol), 2-phenyl-9H-carbazole (0.770 g, 3.17 mmol), cesium fluoride (0.849 g, 5.59 mmol), and DMF (18.6 ml) were put into a 100-mL eggplant flask and stirred at 80 degrees C for five hours. Methanol (200mL) was added to the reaction mixture. The deposited solid was purified by silica gel column chromatography to obtain 0.8 g of a yellow solid. The obtained yellow solid was identified as the compound A-26 by analysis of ASAP-MS (a yield of 44%).

Synthesis of Compound A-27

A synthesis method of a compound A-27 is described below.

[Formula 291]

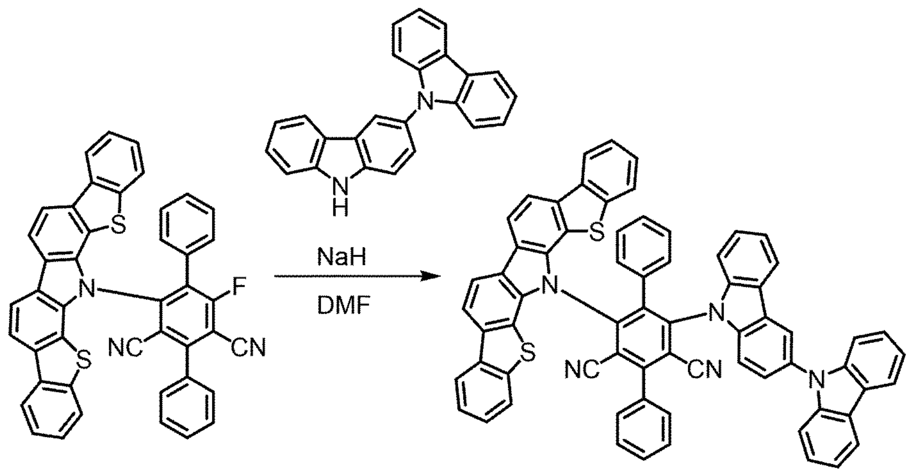


M-f A-27

Under a nitrogen atmosphere, 3,9'-bi[9H-carbazole] (1.77 g, 5.33 mmol), sodium hydride (containing 40-mass% oil) (0.21 g, 5.33 mmol), and DMF (45 ml) were put into a 100-mL three-necked flask and stirred at 0 degrees C for one hour. Next, the intermediate M-f (3.0 g, 4.44 mmol) was put into the reaction mixture at 0 degrees C and a temperature of the solution was slowly raised to room temperature. Then the solution was further stirred at room temperature for one hour. 30 ml of ion-exchange water was added to the reaction mixture, and the deposited solid was collected by filtration. The obtained solid was purified by silica gel column chromatography to obtain 2.7 g of a yellow solid. The obtained yellow solid was identified as the compound A-27 by analysis of ASAP-MS (a yield of 62%).

Synthesis of Compound A-28

A synthesis method of a compound A-28 is described below.

[Formula 292]

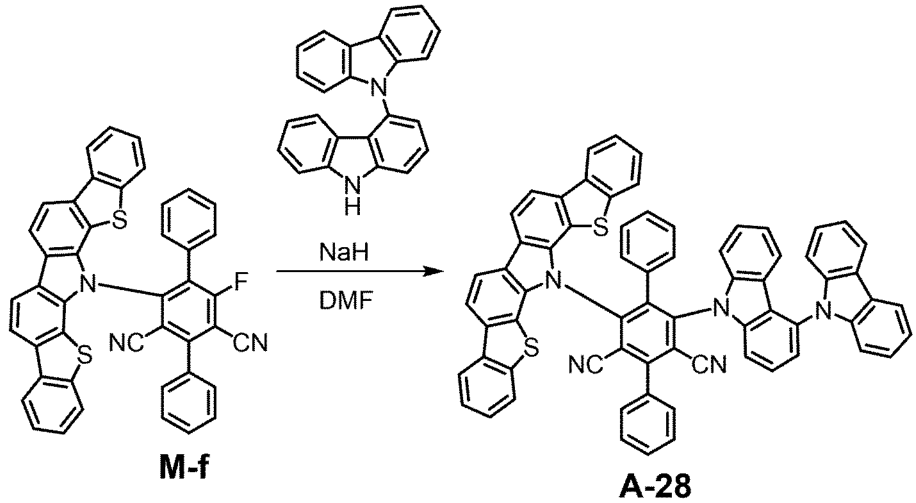


M-f

A-28

Under a nitrogen atmosphere, 4,9'-bi[9H-carbazole] (0.83 g, 2.49 mmol), sodium hydride (containing 40-mass% oil) (0.11 g, 2.84 mmol), and DMF (24 ml) were put into a 100-mL three-necked flask and stirred at 0 degrees C for one hour. Next, the intermediate M-f (1.6 g, 2.37 mmol) was put into the reaction mixture at 0 degrees C and a temperature of the solution was slowly raised to room temperature. Then the solution was further stirred at room temperature for one hour. 20 ml of ion-exchange water was added to the reaction mixture, and the deposited solid was collected by filtration. The obtained solid was purified by silica gel column chromatography to obtain 1.7 g of a yellow solid. The obtained yellow solid was identified as the compound A-28 by analysis of ASAP-MS (a yield of 73%).

Synthesis of Compound A-29

A synthesis method of a compound A-29 is described below.

[Formula 293]

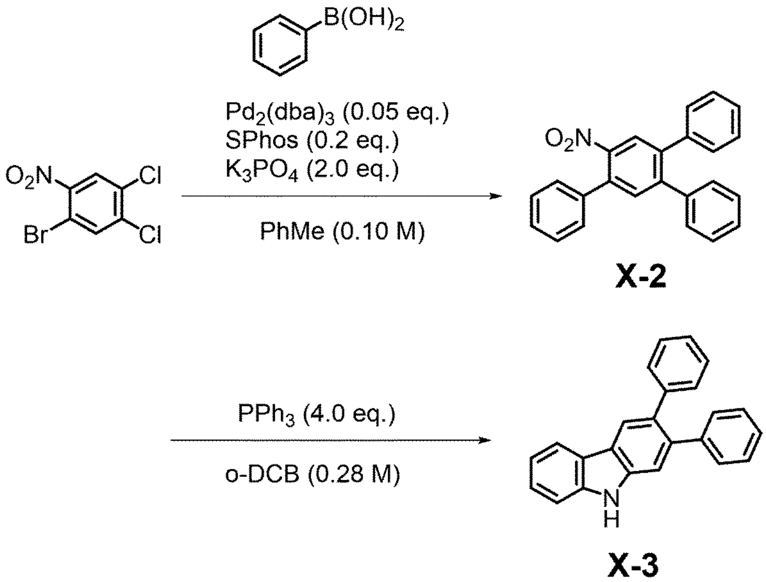


X-2

X-3

Under a nitrogen atmosphere, 1-bromo-4,5-dichloro-2-nitrobenzene (8 g, 29.5 mmol), phenylboronic acid (36 g, 295 mmol), tripotassium phosphate (16.85 g, 122 mmol), tris(dibenzylideneacetone)dipalladium(0) (1.30 g, 1.420 mmol), SPhos(2-dicyclohexylphosphino-2',6'-dimethoxybiphenyl) (2.40 g, 5.85 mmol), and toluene (135 ml) were added to a 1000-mL eggplant flask, and stirred at 110 degrees C for six hours. After the reaction solution was left to cool to room temperature, 20 ml of ion-exchange water was added to the reaction solution, from which the organic layer was extracted with ethyl acetate. The organic layer was washed with water and dried over sodium sulfate. Subsequently, the solvent was removed under reduced pressure using a rotary evaporator. A compound obtained after the solvent was removed under reduced pressure was purified by silica gel column chromatography (hexane/ethyl acetate = 90%:10% to 70%:30%) to obtain an intermediate X-2 (1.15 g, 3.23 mmol, a yield of 11%).

Under a nitrogen atmosphere, the intermediate X-2 (1 g, 2.85 mmol), triphenylphosphine (3 g, 11.44 mmol) and o-dichlorobenzene (10 ml) were put into a 100-mL eggplant flask and stirred at 180 degrees C for six hours. After the reaction solution was left to cool to room temperature, the reaction solution was concentrated by distillation under reduced pressure. A compound after the reaction solution was concentrated was purified by silica gel column chromatography (hexane/ethyl acetate = 95%:5% to 50%:50%) to obtain an intermediate X-3 (0.66 g, 2.08 mmol, a yield of 73%).

[Formula 294]

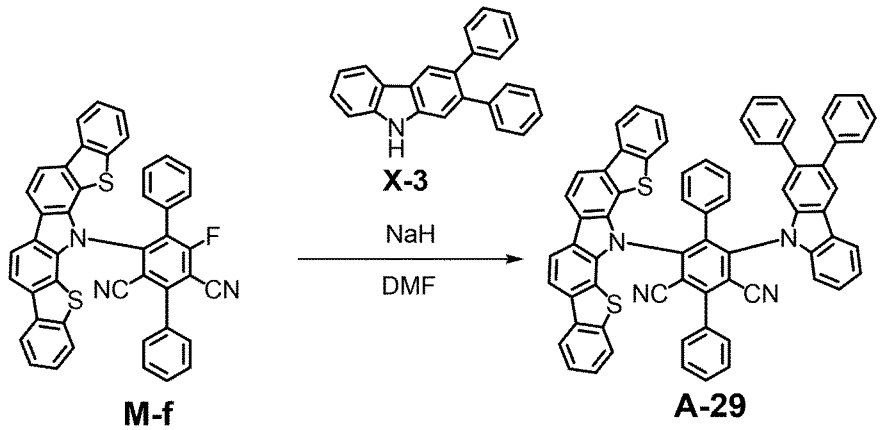


M-f

A-29

Under a nitrogen atmosphere, sodium hydride (60 mass%, 0.056 g, 1.40 mmol) was added to a solution of the intermediate X-3 (0.446 g, 1.40 mmol) in DMF (8.0 mL) under ice cooling in a 50-mL three-necked flask, and stirred for 30 minutes while the temperature was kept unchanged. A solution of the intermediate M-f (0.94 g, 1.40 mmol) in DMF (5.4 ml) was added dropwise to the reaction solution and a temperature of the solution was raised to room temperature. Then the solution was stirred for six hours. Water was added to the reaction mixture. The deposited solid was washed with methanol. The washed solid was purified by column chromatography to obtain 0.339 g of a yellow solid. The obtained

yellow solid was identified as the compound A-29 by analysis of ASAP-MS (a yield of 16%).

Synthesis of Compound A-30

A synthesis method of a compound A-30 is described below.

[Formula 295]

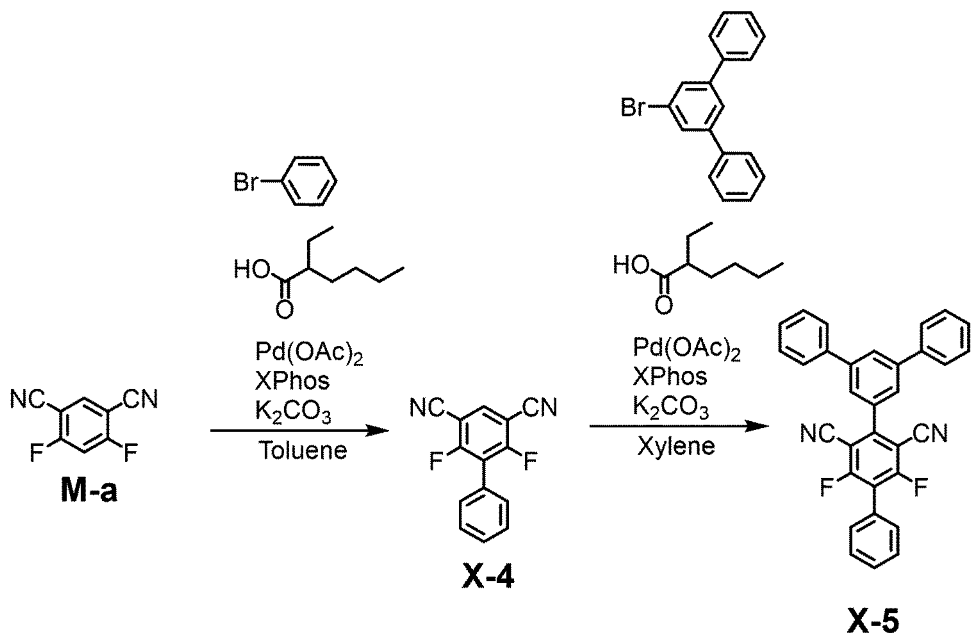


Under a nitrogen atmosphere, the intermediate M-a (20 g, 122 mmol), diacetoxypalladium (0.41 g, 1.83 mmol), XPhos (2-dicyclohexylphosphino-2',4',6'-triisopropylbiphenyl) (1.74 g, 3.66 mmol), potassium carbonate (25.3 g, 183 mmol), and toluene (300 ml) were added to a 1000-mL three-necked flask and stirred at room temperature for 30 minutes. Then, 2-ethylhexanoic acid (1.95 ml, 12.19 mmol) and bromobenzene (10.84 ml, 104 mmol) were put into the reaction solution and stirred at 40 degrees C overnight. After the stirring, the reaction solution was returned to room temperature, water was added to the reaction solution, and the precipitated solid was filtered. The obtained solid was passed through a silica pad and recrystallized with toluene to obtain 13.2 g of a white solid. The obtained white solid was identified as an intermediate X-4 by analysis of GC-MS (a yield of 44%).

Under a nitrogen atmosphere, the intermediate X-4 (2 g, 8.33 mmol), 5'-bromo-1,1':3',1"-terphenyl (3 g, 9.70 mmol), diacetoxypalladium (0.20 g, 0.82 mmol), XPhos(2-dicyclohexylphosphino-2',4',6'-triisopropylbiphenyl) (0.80 g, 1.678 mmol), potassium carbonate (3 g, 21.71 mmol), and xylene (40 ml) were added to a 200-mL three-necked flask and stirred at room temperature for 10 minutes. Then, 2-ethylhexanoic acid (0.06 ml, 0.374 mmol) was added to the reaction solution and stirred at 130 degrees C for five hours. After the stirring, the reaction solution was returned to room temperature, water was added to the reaction solution, and the organic layer was extracted twice with ethyl acetate. The extracted organic layer was washed with water and washed with sodium sulfate. Subsequently, the solvent was distilled away under reduced pressure. The obtained residue was purified by silica gel column chromatography to obtain 2.20 g of a white solid. The obtained white solid was identified as an intermediate X-5 by analysis of ASAP-MS (a yield of 56%).

[Formula 296]

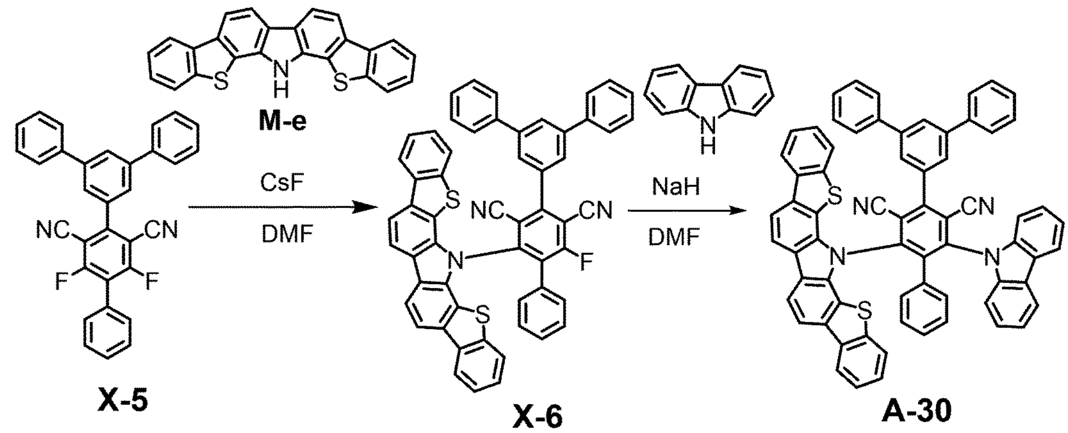


Under a nitrogen atmosphere, the intermediate X-5 (2.70 g, 5.76 mmol), cesium fluoride (2.50 g, 16.46 mmol), the intermediate M-e (2.20 g, 5.80 mmol), and DMF (50 ml) were put into a 300-mL eggplant flask and stirred at room temperature for 20 hours. 50 ml of ion-exchange water was added to the reaction solution. The deposited solid was collected by filtration and washed with methanol. The washed solid was purified by silica gel column chromatography to obtain 3.14 g of a yellow solid. The obtained yellow solid was identified as an intermediate X-6 by analysis of ASAP-MS (a yield of 65%).

Under a nitrogen atmosphere, sodium hydride (60 mass%, 0.080 g, 2.00 mmol) was added to a solution of carbazole (0.36 g, 2.51 mmol) in DMF (7 mL) under ice cooling in a 100-mL eggplant flask, and stirred for 30 minutes while the temperature was kept unchanged. A solution of the intermediate X-6 (1.50 g, 1.812 mmol) in DMF (7 ml) was added dropwise to the reaction solution. A temperature of the solution was raised to room temperature. Then the solution was stirred for 18 hours. Water was added to the reaction mixture. The deposited solid was washed with methanol. The washed solid was purified by column chromatography to obtain 0.925 g of a yellow solid. The obtained yellow solid was identified as the compound A-30 by analysis of ASAP-MS (a yield of 52%).

Synthesis of Compound A-31

A synthesis method of a compound A-31 is described below.

[Formula 297]

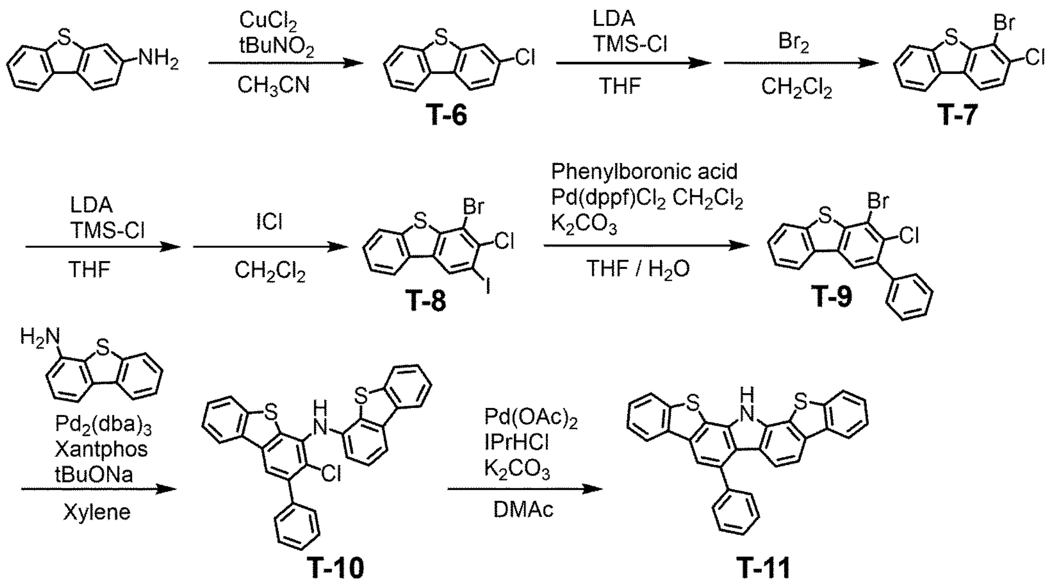


Under a nitrogen atmosphere, copper(II) chloride (12.1 g, 90 mmol), acetonitrile (70 ml), and tert-butyl nitrite

(13.18 ml, 113 mmol) were put into a 300-mL three-necked flask and heated to 65 degrees C. Dibenzo[b,d]thiophene-3-amine (15 g, 75 mmol) was dissolved in acetonitrile (90 ml) to prepare a solution, and this solution was added dropwise to the reaction solution over 15 minutes. The reaction solution after the addition was stirred for one hour, then left to cool, added with 6N hydrochloric acid (150 ml), and stirred. The stirred reaction solution was extracted with toluene, washed with brine, and then concentrated. A compound obtained after the concentration was purified by silica gel column chromatography to obtain 12 g of a white solid. The obtained white solid was identified as an intermediate T-6 by analysis of ASAP-MS (a yield of 73%).

Under a nitrogen atmosphere, the intermediate T-6 (12.0 g, 54.9 mmol), chlorotrimethylsilane (17.5 g, 110 mmol), and THF (180 mL) were put into a 500-ml three-necked flask. After cooling the material in the three-necked flask to -78 degrees C in a dry ice/acetone bath, 30 ml (2M, THF solution) of lithium diisopropylamide (LDA) was added dropwise. The reaction solution was stirred at -78 degrees C for 30 minutes, then returned to room temperature, and further stirred for three hours. After the stirring, water (100 mL) was added to the three-necked flask, and the organic layer was extracted with ethyl acetate. The extracted organic layer was washed with water and saline, dried over magnesium sulfate, and then the solvent was removed under reduced pressure using a rotary evaporator. 200 ml of dichloromethane was added to the obtained liquid, and then bromine (13.2 g, 83 mmol) was added dropwise at 0 degrees C, followed by stirring at room temperature for four hours. The stirred reaction solution was added with saturated sodium bisulfite aqueous solution (100 mL). The organic layer was extracted with dichloromethane, the extracted organic layer was washed with water and saline, the washed organic layer was dried over magnesium sulfate, and the dried organic layer was concentrated using a rotary evaporator. A compound obtained after the concentration was purified by silica gel column chromatography to obtain an intermediate T-7 (14 g, 47 mmol, a yield of 86%).

Under a nitrogen atmosphere, the intermediate T-7 (12.0 g, 54.9 mmol), chlorotrimethylsilane (15.0 g, 94 mmol), and THF (160 mL) were put into a 500-ml three-necked flask. After cooling the material in the three-necked flask to -78 degrees C in a dry ice/acetone bath, 26 ml (2M, THF solution) of lithium diisopropylamide was added dropwise. The reaction solution was stirred at -78 degrees C for 20 minutes, then returned to room temperature, and further stirred for three hours. After the stirring, water (100 mL) was added to the three-necked flask, and the organic layer was extracted with ethyl acetate. The extracted organic layer was washed with water and saline, dried over magnesium sulfate, and then the solvent was removed under reduced pressure using a rotary evaporator. 200 ml of dichloromethane was added to the obtained liquid, and then iodine monochloride (11.6 g, 71.4 mmol) was added dropwise at 0 degrees C, followed by stirring at room temperature for four hours. The stirred reaction solution was added with saturated sodium bisulfite aqueous solution (100 mL). The organic layer was extracted with dichloromethane, the extracted organic layer was washed with water and saline, the washed organic layer was dried over magnesium sulfate, and the dried organic layer was concentrated using a rotary evaporator. A compound obtained after the concentration was purified by silica gel column chromatography to obtain an intermediate T-8 (19.5 g, 46 mmol, a yield of 97%).

Under a nitrogen atmosphere, the intermediate T-8 (19.5 g, 46 mmol), phenylboronic acid (5.61 g, 46 mmol), potassium carbonate (19.1 g, 138 mmol), [1,1'-bis(diphenylphosphino)ferrocene]dichloropalladium(II) dichloromethane adduct (1.13 g, 1.38 mmol), THF (123 ml), and ion-exchange water (31 ml) were put into a 200-mL three-necked flask and stirred at 40 degrees C for seven hours. After the reaction solution was concentrated, 100 ml of ion-exchange water was added to the concentrated reaction solution. The organic layer was extracted with dichloromethane. The extracted organic layer was washed with water and saline and dried over magnesium sulfate. Subsequently, the solvent was removed under reduced pressure using a rotary evaporator. A compound obtained after the solvent was removed under reduced pressure was purified by silica gel column chromatography to obtain an intermediate T-9 (2.50 g, 5.89 mmol, a yield of 13%).

Under a nitrogen atmosphere, the intermediate T-9 (2.5 g, 5.89 mmol), dibenzo[b,d]thiophen-4-amine (1.33 g, 6.69 mmol), tris(dibenzylideneacetone)dipalladium(0) (0.092 g, 0.10 mmol) , Xantphos (0.116 g, 0.401 mmol), sodium tert-butoxide (0.964 g, 10.0 mmol), and xylene (35 mL) were added to a 500-ml three-necked flask. The obtained mixture was heated at 60 degrees C for 17 hours with stirring and then cooled to room temperature (25 degrees C). The reaction solution was concentrated and then purified by silica gel column chromatography to obtain 2.5 g of a white solid. The obtained white solid was identified as an intermediate T-10 by analysis of ASAP-MS (a yield of 68%).

Under a nitrogen atmosphere, the intermediate T-10 (2.5 g, 4.57 mmol), 1,3-bis(2,6-diisopropylphenyl)imidazolium chloride (IPrHCl) (0.086 g, 0.203 mmol), palladium(II) acetate (0.023 g, 0.102 mmol), potassium carbonate (1.76 g, 12.7 mmol), and N,N-dimethylacetamide (DMAc) (17 mL) were added to a 200-ml three-necked flask. The obtained mixture was stirred at 180 degrees C for nine hours and then cooled to room temperature (25 degrees C). 30 ml of ion-exchange water was added to the reaction solution, from which the organic layer was extracted with ethyl acetate and concentrated. The obtained solid was purified by silica gel column chromatography to obtain 1.7 g of a white solid. The obtained white solid was identified as an intermediate T-11 by analysis of ASAP-MS (a yield of 73%).

[Formula 298]

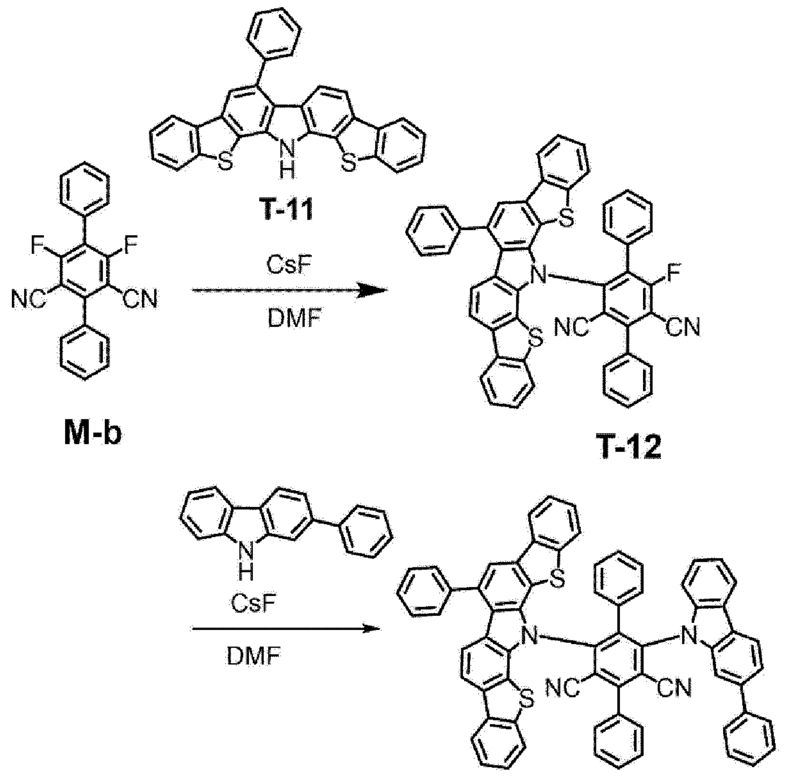


T-11

M-b

T-12

A-31

Under a nitrogen atmosphere, the intermediate M-b (0.791 g, 2.50 mmol), cesium fluoride (1.14 g, 7.51 mmol), the intermediate T-11 (1.14 g, 2.50 mmol), and DMF (20.9 ml) were put into a 100-mL eggplant flask and stirred at room temperature for 20 hours. 50 ml of ion-exchange water was added to the reaction solution, and the deposited solid was collected by filtration. The collected solid was purified by silica gel column chromatography to obtain 1.7 g of a yellow solid. The obtained yellow solid was identified as an intermediate T-12 by analysis of ASAP-MS (a yield of 92%).

Under a nitrogen atmosphere, the intermediate T-12 (1.73 g, 2.30 mmol), 2-phenyl-9H-carbazole (0.840 g, 3.45 mmol), cesium fluoride (1.05 g, 6.90 mmol), and DMF (7.7 ml) were put into a 100-mL eggplant flask and stirred at 80 degrees C for one hour. After the stirring, the deposited solid was collected by filtration and purified by silica gel column chromatography to obtain 0.9 g of a yellow solid. The obtained yellow solid was identified as the compound A-31 by analysis of ASAP-MS (a yield of 40%).

Synthesis of Compound A-32

A synthesis method of a compound A-32 is described below.

[Formula 299]

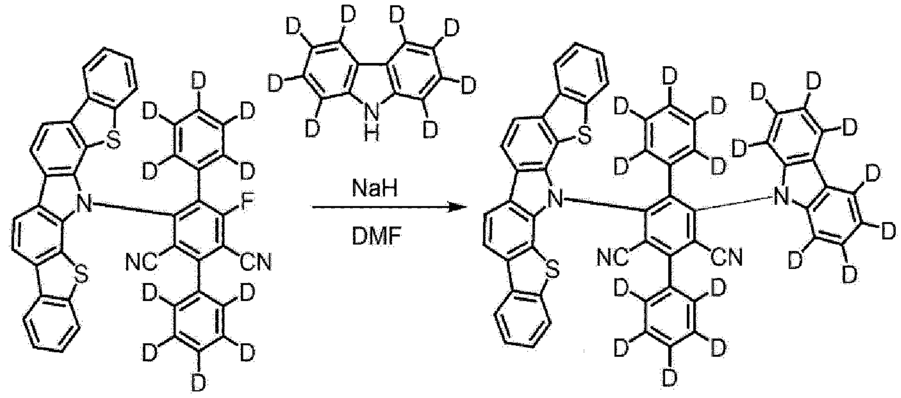


T-14

A-32

Synthesis of Compound A-32

Under a nitrogen atmosphere, carbazole-1,2,3,4,5,6,7,8-d8 (1.2 g, 6.6 mmol), sodium hydride (containing 40-mass% oil) (0.27 g, 6.6 mmol), and DMF (45 ml) were put into a 100-mL three-necked flask and stirred at 0 degrees C for 30 minutes. Next, the intermediate T-14 (3.0 g, 4.4 mmol) was put into the reaction mixture and stirred at room temperature for two hours. Methanol (20 mL) was added to the reaction mixture. The deposited solid was purified by silica gel column chromatography to obtain 3.2 g of a yellow solid. The obtained yellow solid was identified as the compound A-32 by analysis of ASAP-MS (a yield of 87%).

Synthesis of Compound A-33

A synthesis method of a compound A-33 is described below.

[Formula 300]

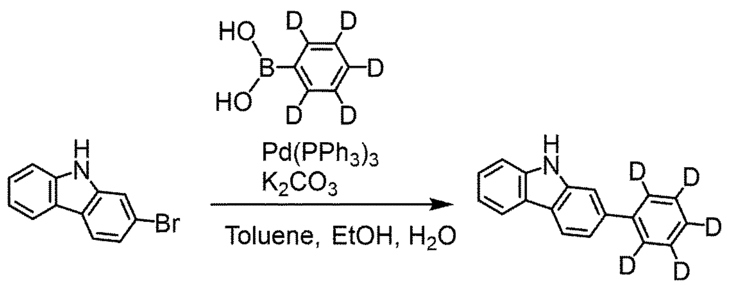


T-13

Under a nitrogen atmosphere, 2-bromo-9H-carbazole (16 g, 65 mmol), phenyl-d5-boronic acid (9.90 g, 78 mmol), potassium carbonate (27.0 g, 195 mmol), tetrakistriphenylphosphine palladium(0) (1.50 g, 1.30 mmol), toluene (108 ml), THF (54 ml), and ion-exchange water (54 ml) were put into a 300-mL three-necked flask and stirred at 80 degrees C for four hours. The organic layer was extracted with toluene from the reaction solution. The extracted organic layer was washed with water and saline and dried over magnesium sulfate. Subsequently, the solvent was removed under reduced pressure using a rotary evaporator. A compound obtained after the solvent was removed under reduced pressure was purified by silica gel column chromatography to obtain an intermediate T-13 (2.50 g, 5.89 mmol, a yield of 13%).

[Formula 301]

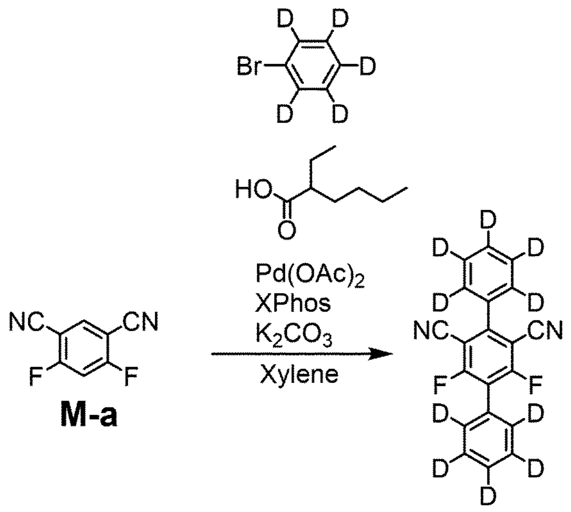


M-a

M-x

Under a nitrogen atmosphere, the intermediate M-a (34 g, 207 mmol), diacetoxypalladium (1.40g, 6.22 mmol), XPhos (2-dicyclohexylphosphino-2',4',6'-triisopropylbiphenyl) (5.93 g, 12.4 mmol), potassium carbonate (86 g, 622 mmol), and xylene (414 ml) were added to a 1000-mL three-necked flask and stirred at room temperature for 30 minutes. 2-ethylhexanoic acid (6.64 ml, 41.4 mmol) and bromobenzene-d5 (101 g, 622 mmol) were put into the stirred reaction solution and stirred at 100 degrees C for five hours. After the stirring, the reaction solution was returned to room temperature, and the precipitated solid was filtered. The obtained solid was recrystallized with xylene to obtain 48 g of a white solid. The obtained white solid was identified as an intermediate M-x by analysis of GC-MS (a yield of 71%).

[Formula 302]

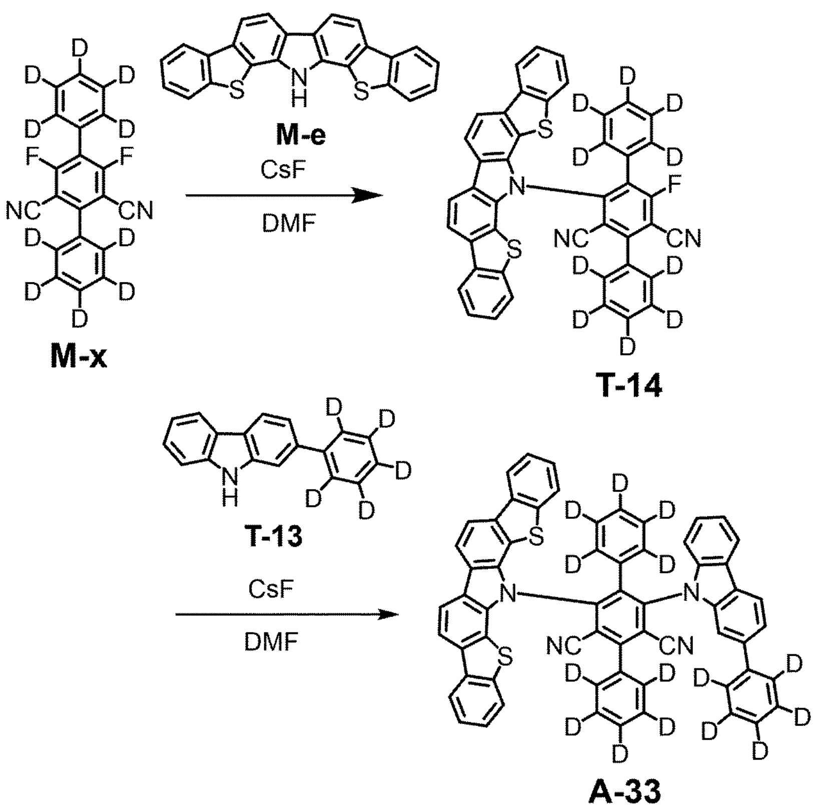


M-x

M-e

T-14

T-13

A-33

Under a nitrogen atmosphere, the intermediate M-x (19.6 g, 60.0 mmol), cesium fluoride (16.6 g, 109 mmol), the intermediate M-e (20.7 g, 54.5mmol), and DMF (182 ml) were put into a 300-mL three-necked flask and stirred at room temperature for 20 hours. After the stirring, 200 ml of ion-exchange water was added to the reaction solution, and the deposited solid was collected by filtration. The collected solid was purified by silica gel column chromatography to obtain 2.4 g of a yellow solid. The obtained yellow solid was identified as an intermediate T-14 by analysis of ASAP-MS (a yield of 91%).

Under a nitrogen atmosphere, the intermediate T-14 (2.5 g, 3.65 mmol), the intermediate T-13 (1.36 g, 5.47 mmol), cesium fluoride (1.66 g, 10.9 mmol), and DMF (37 ml) were put into a 100-mL eggplant flask and stirred at 80 degrees C for four hours. After being stirred and left to cool, 70 ml of methanol was added to the reaction solution, and the deposited solid was filtered. The obtained solid was purified by silica gel column chromatography to obtain 1.71 g of a yellow solid. The obtained yellow solid was identified as the compound A-33 by analysis of ASAP-MS (a yield of 51%).

Synthesis of Compound A-34

A synthesis method of a compound A-34 is described below.

[Formula 303]

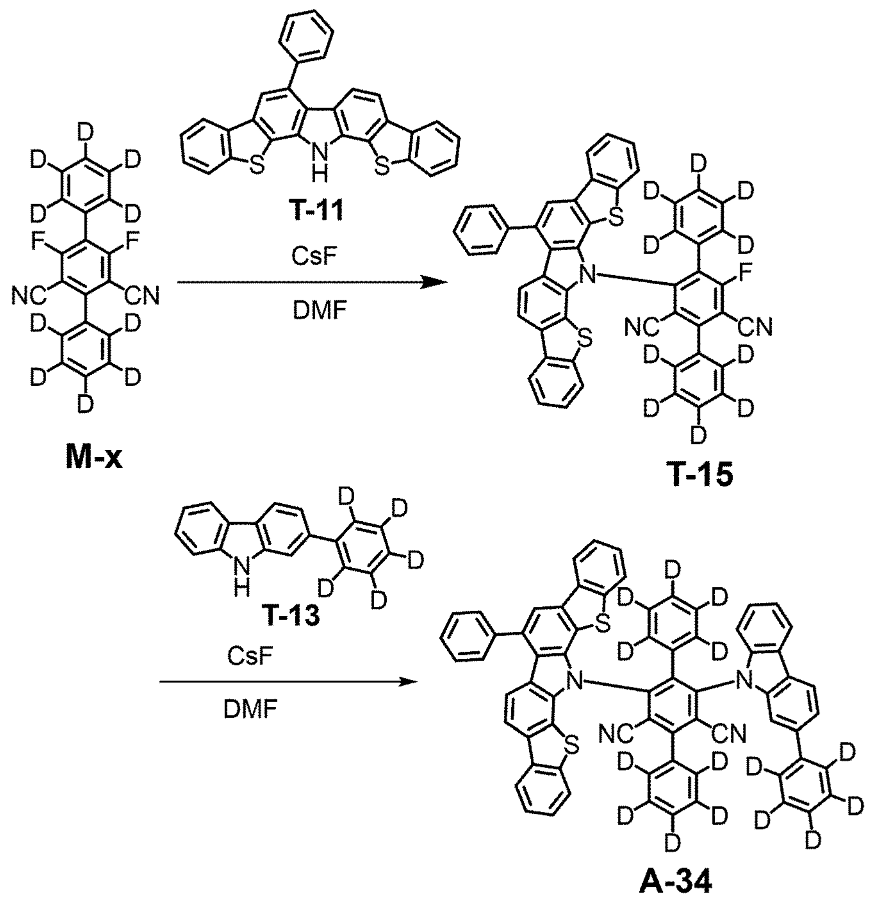


Under a nitrogen atmosphere, the intermediate M-x (1.11 g, 34.0 mmol), cesium fluoride (1.15 g, 10.2 mmol), the intermediate T-11 (1.55 g, 3.40 mmol), and DMF (11.3 ml) were put into a 100-mL eggplant flask and stirred at room temperature for 20 hours. After the stirring, 20 ml of ion-exchange water was added to the reaction solution, and the deposited solid was collected by filtration. The collected solid was purified by silica gel column chromatography to obtain 2.4 g of a yellow solid. The obtained yellow solid was identified as an intermediate T-15 by analysis of ASAP-MS (a yield of 91%).

Under a nitrogen atmosphere, the intermediate T-15 (2.4 g, 3.19 mmol), the intermediate T-13 (1.17 g, 4.72 mmol), cesium fluoride (1.44 g, 9.45 mmol), and DMF (11 ml) were put into a 100-mL eggplant flask and stirred at 80 degrees C for two hours. The deposited solid was filtered and purified by silica gel column chromatography to obtain 1.46 g of a yellow solid. The obtained yellow solid was identified as the compound A-34 by analysis of ASAP-MS (a yield

of 47%).

Synthesis of Compound A-35

A synthesis method of a compound A-35 is described below.

[Formula 304]

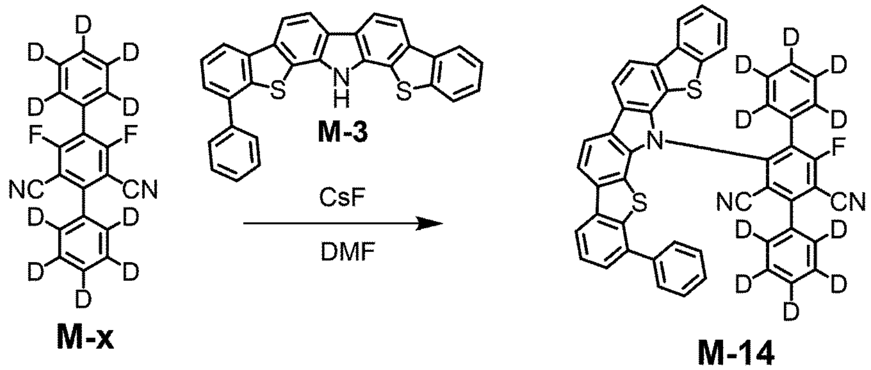


M-x M-14

Under a nitrogen atmosphere, the intermediate M-3 (3.5 g, 7.7 mmol), cesium fluoride (2.3 g, 15.4 mmol), the intermediate M-x (2.3 g, 8.07 mmol), and DMF (30 ml) were put into a 100-mL eggplant flask and stirred at room temperature for 12 hours. After the stirring, 50 ml of ion-exchange water was added to the reaction solution, and the deposited solid was collected by filtration. The collected solid was purified by silica gel column chromatography to obtain 5.1 g of a yellow solid. The obtained yellow solid was identified as an intermediate M-14 by analysis of ASAP-MS (a yield of 87%).

[Formula 305]

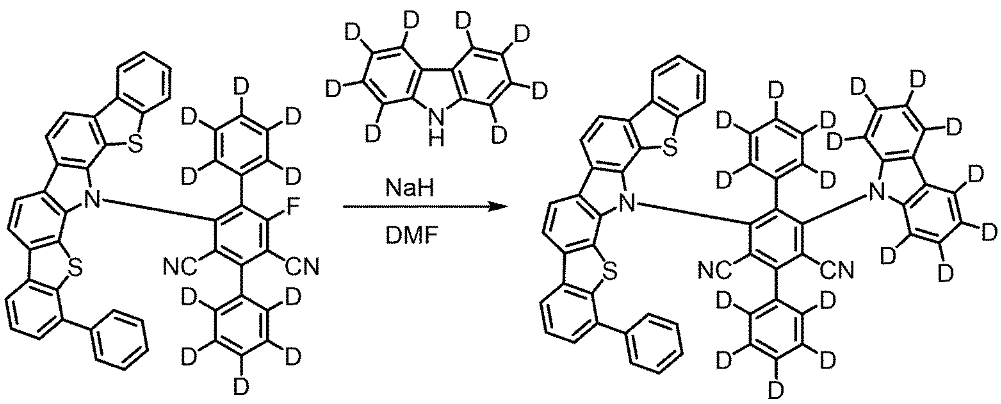


M-14 A-35

Under a nitrogen atmosphere, carbazole-1,2,3,4,5,6,7,8-d8 (0.24 g, 1.34 mmol), sodium hydride (containing 40-mass% oil) (0.058 g, 1.34 mmol), and DMF (10 ml) were put into a 50-mL three-necked flask and stirred at 0 degrees C for one hour. The intermediate M-14 (0.6 g, 0.79 mmol) was put into the stirred reaction solution at 0 degrees C and a temperature of the solution was slowly raised to room temperature. Then the solution was further stirred at room temperature for one hour. 10 ml of ion-exchange water was added to the reaction mixture, and the deposited solid was collected by filtration. The obtained solid was purified by silica gel column chromatography to obtain 0.47 g of a yellow solid. The obtained yellow solid was identified as the compound A-35 by analysis of ASAP-MS (a yield of 65%).

Synthesis of Compound A-36

A synthesis method of a compound A-36 is described below.

[Formula 306]

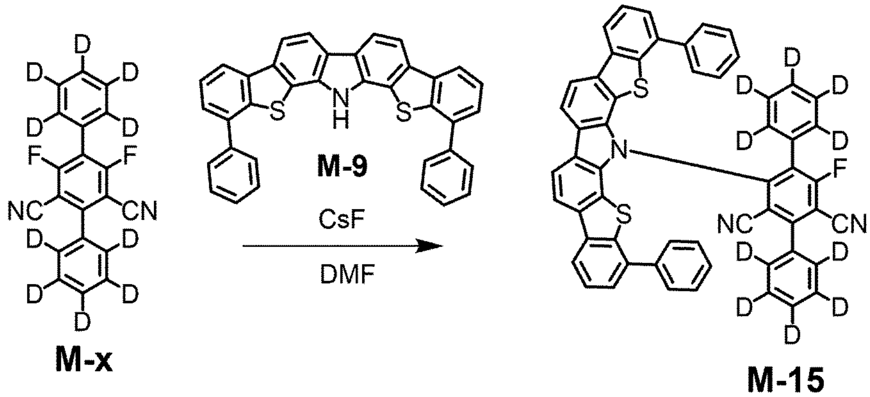


M-x

M-15

Under a nitrogen atmosphere, the intermediate M-9 (3.0 g, 5.60 mmol), cesium fluoride (2.6 g, 16.9 mmol), the intermediate M-x (1.9 g, 5.92 mmol), and DMF (20 ml) were put into a 100-mL eggplant flask and stirred at room temperature for 12 hours. After the stirring, 20 ml of ion-exchange water was added to the reaction solution, and the deposited solid was collected by filtration. The collected solid was purified by silica gel column chromatography to obtain 4.4 g of a yellow solid. The obtained yellow solid was identified as an intermediate M-15 by analysis of ASAP-MS (a yield of 93%).

[Formula 307]

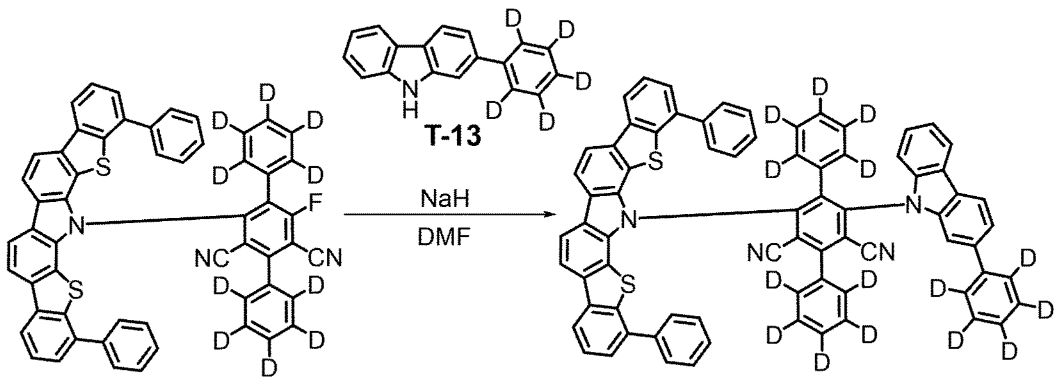


M-15

A-36

Under a nitrogen atmosphere, the intermediate T-13 (0.76 g, 3.04 mmol), sodium hydride (containing 40-mass% oil) (0.12 g, 3.04 mmol), and DMF (20 ml) were put into a 100-mL three-necked flask and stirred at 0 degrees C for one hour. The intermediate M-15 (1.7 g, 2.03 mmol) was put into the stirred reaction solution at 0 degrees C and a temperature of the solution was slowly raised to room temperature. Then the solution was further stirred at room temperature for one hour. 20 ml of ion-exchange water was added to the reaction mixture, and the deposited solid was collected by filtration. The obtained solid was purified by silica gel column chromatography to obtain 1.84 g of a yellow solid. The obtained yellow solid was identified as the compound A-36 by analysis of ASAP-MS (a yield of 85%).

Synthesis of Compound A-37

A synthesis method of a compound A-37 is described below.

[Formula 308]

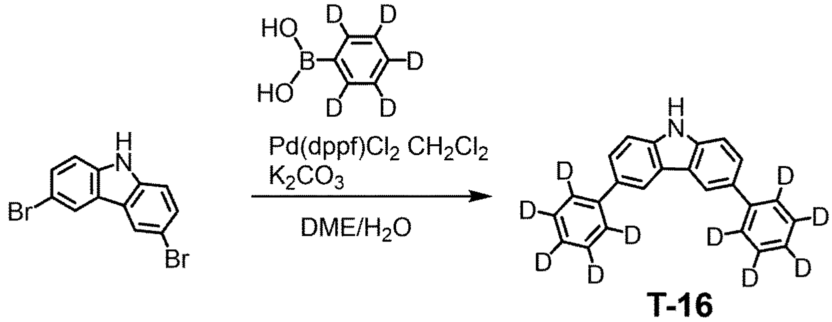


T-16

Under a nitrogen atmosphere, 3,6-dibromo-9H-carbazole (10 g, 30.8 mmol), phenyl-d5-boronic acid (8.59 g, 67.7 mmol), potassium carbonate (12.8.0 g, 92 mmol), [1,1'-bis(diphenylphosphino)ferrocene]dichloropalladium(II) dichloromethane adduct (0.754 g, 0.923 mmol), DME (82 ml) and ion-exchange water (20.5 ml) were put into a 200-mL three-necked flask and stirred at 80 degrees C for four hours. After the reaction solution was concentrated, the organic layer was extracted with dichloromethane. The extracted organic layer was washed with water and saline and dried over magnesium sulfate. Subsequently, the solvent was removed under reduced pressure using a rotary evaporator. A compound obtained after the solvent was removed under reduced pressure was purified by silica gel column chromatography and recrystallization with toluene to obtain an intermediate T-16 (7.64 g, 23.0 mmol, a yield of 75%).

[Formula 309]

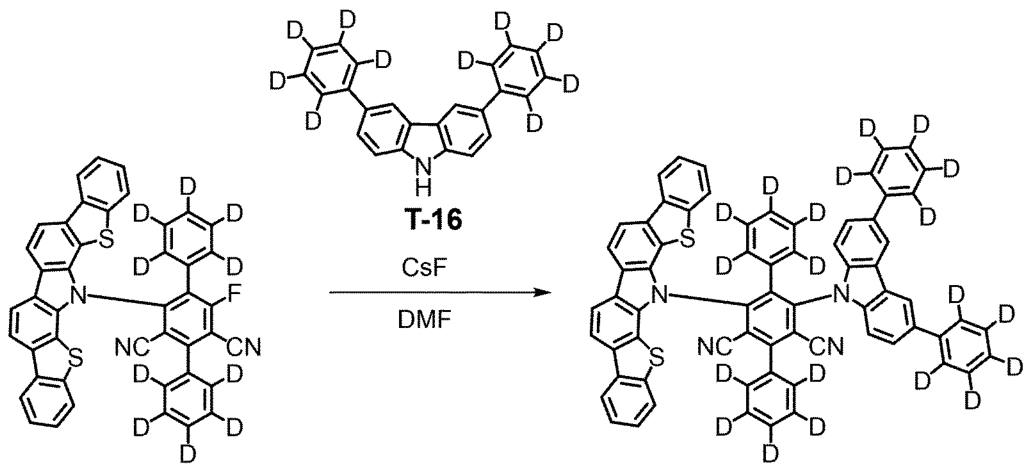


T-14

A-37

Under a nitrogen atmosphere, the intermediate T-14 (2.5 g, 3.65 mmol), the intermediate T-16 (1.80 g, 5.47 mmol), cesium fluoride (1.66 g, 10.9 mmol), and DMF (36.5 ml) were put into a 100-mL eggplant flask and stirred at 80 degrees C for three hours. After being stirred and left to cool, 70 ml of methanol was added to the reaction solution, and the deposited solid was filtered. The obtained solid was purified by silica gel column chromatography to obtain 1.50 g of a yellow solid. The obtained yellow solid was identified as the compound A-37 by analysis of ASAP-MS (a yield of 41%).

Synthesis of Compound A-38

A synthesis method of a compound A-38 is described below.

[Formula 310]

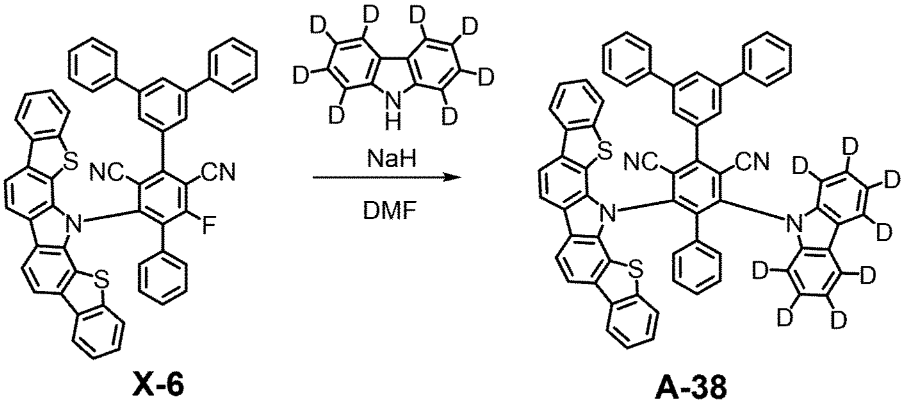


X-6 A-38

Under a nitrogen atmosphere, sodium hydride (60 mass%, 0.087 g, 2.17 mmol) was added to a solution of 9H-carbazole-1,2,3,4,5,6,7,8-d8 (0.41 g, 2.36 mmol) in DMF (10 mL) under ice cooling in a 100-mL eggplant flask, and stirred for 30 minutes while the temperature was kept unchanged. A solution of the intermediate X-6 (1.63 g, 1.969 mmol) in DMF (10 ml) was added dropwise to the stirred reaction solution. A temperature of the solution was raised to room temperature. Then the solution was stirred for 18 hours. Water was added to the reaction mixture. The deposited solid was washed with methanol. The washed solid was purified by silica gel column chromatography to obtain 1.14 g of a yellow solid. The obtained yellow solid was identified as the compound A-38 by analysis of ASAP-MS (a yield of 59%).

Synthesis of Compound A-39

A synthesis method of a compound A-39 is described below.

[Formula 311]

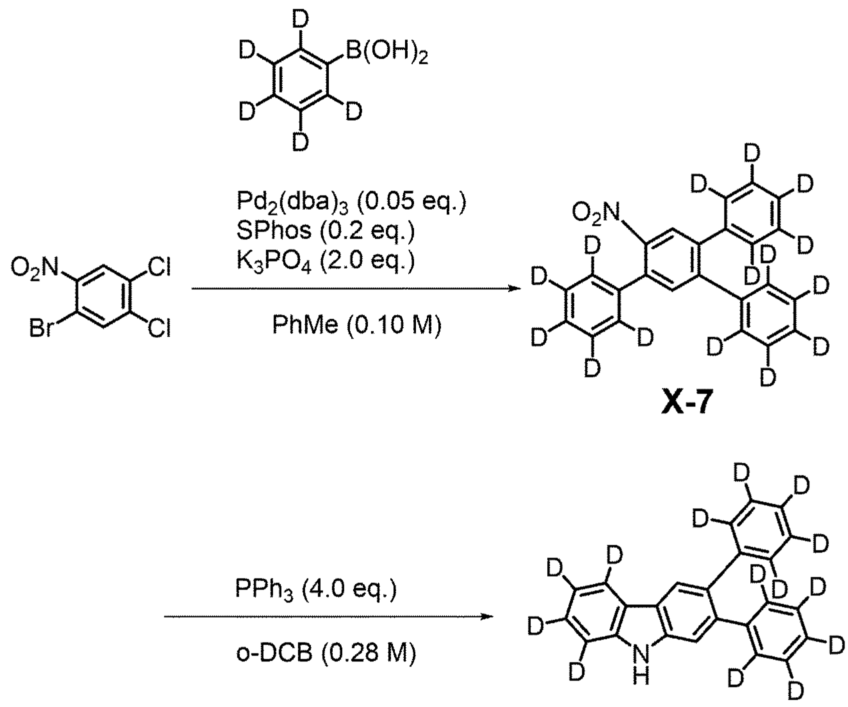


X-7

X-8

Under a nitrogen atmosphere, 1-bromo-4,5-dichloro-2-nitrobenzene (10 g, 36.9 mmol), (phenyl-d5)boronic acid

(42.1 g, 332 mmol), tripotassium phosphate (15.67 g, 73.8 mmol), tris(dibenzylideneacetone)dipalladium(0) (1.69 g, 1.846 mmol), SPhos (3.03 g, 7.38 mmol), and toluene (PhMe) (369 ml) were added to a 1000-mL three-necked flask, and stirred at 110 degrees C for nine hours. After the stirring, the reaction solution was left to cool to room temperature. Subsequently, ion-exchange water was added to the reaction solution, and the organic layer was extracted twice with ethyl acetate. The organic layer was dried over sodium sulfate. Subsequently, the solvent was removed under reduced pressure using a rotary evaporator. A compound obtained after the solvent was removed under reduced pressure was purified by silica gel column chromatography (hexane/dichloromethane = 67%:33%) to obtain an intermediate X-7 (3.01 g, 8.21 mmol, a yield of 22%).

Under a nitrogen atmosphere, the intermediate X-7 (3 g, 8.19 mmol), triphenylphosphine (7 g, 26.69 mmol) and o-dichlorobenzene (30 ml) were put into a 200-mL eggplant flask and stirred at 180 degrees C for 12 hours. After the stirring, the reaction solution was left to cool to room temperature. Subsequently, the reaction solution was concentrated by distillation under reduced pressure. The obtained compound was purified by silica gel column chromatography (hexane/ethyl acetate = 95%:5% to 50%:50%) to obtain an intermediate X-8 (1.47 g, 4.41 mmol, a yield of 58%).

[Formula 312]

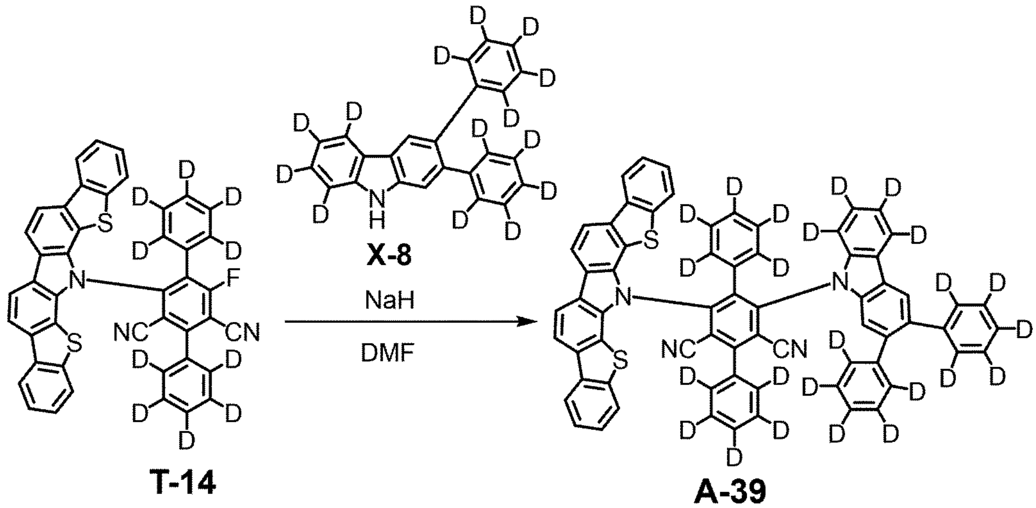


X-8

T-14

A-39

Under a nitrogen atmosphere, sodium hydride (60 mass%, 0.09 g, 2.29 mmol) was added to a solution of the intermediate X-8 (0.77 g, 2.31 mmol) in DMF (7.0 mL) under ice cooling in a 50-mL three-necked flask, and stirred for 30 minutes while the temperature was kept unchanged. A solution of the intermediate T-14 (1.50 g, 2.19 mmol) in DMF (7.0 ml) was added dropwise to the stirred reaction solution. A temperature of the solution was raised to room temperature. Then the solution was stirred for 15 hours. Water was added to the reaction mixture. The deposited solid was washed with methanol. The washed solid was purified by silica gel column chromatography to obtain 1.03 g of a yellow solid. The obtained yellow solid was identified as the compound A-39 by analysis of ASAP-MS (a yield of 47%).

Synthesis of Compound A-40

A synthesis method of a compound A-40 is described below.

[Formula 313]

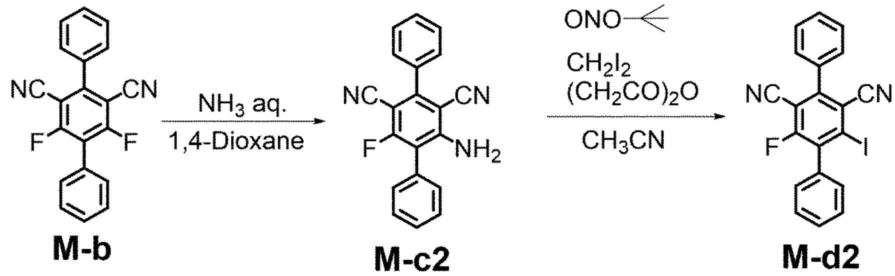


M-b

M-c2

M-d2

Under a nitrogen atmosphere, the intermediate M-b (25 g, 79 mmol) and 1,4-dioxane (160 mL) were put into a 500-mL three-necked flask and next 30-mass% ammonia water (25 mL) was put thereinto. The obtained solution was heated at 80 degrees C for 10 hours with stirring. The stirred reaction solution was returned to room temperature (25 degrees C). The solvent was distilled using an evaporator. The obtained solid was purified by silica gel column chromatography to obtain 23 g of a white solid. The obtained white solid was identified as an intermediate M-c2 by analysis of GC-MS (a yield of 93%).

Under a nitrogen atmosphere, the intermediate M-c2 (23 g, 73 mmol) and acetonitrile (150 mL) were put into a 1000-mL three-necked flask, and then diiodomethane (8.86 ml, 110 mmol) and tert-butyl nitrite (44.0 ml, 367 mmol) were put thereinto. The mixture was stirred for five minutes. Next, acetic anhydride (139 ml, 1468 mmol) was put into the mixture and stirred at 25 degrees C for four hours. After the reaction was completed, 500 ml of ion-exchange water was added to the reaction solution and the deposited solid was collected. The collected solid was purified by silica gel column chromatography to obtain 27 g of a white solid. The obtained white solid was identified as an intermediate M-d2 by analysis of GC-MS (a yield of 87%).

[Formula 314]

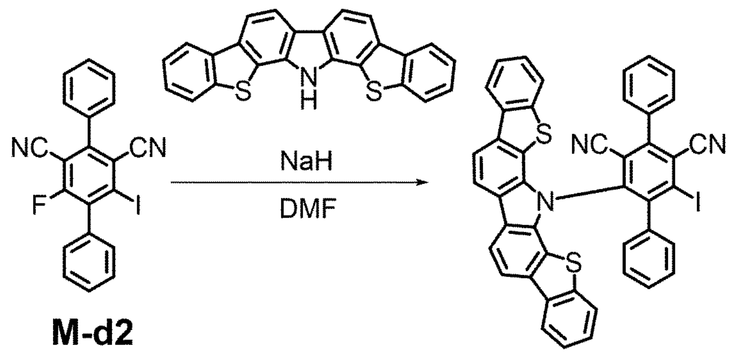


M-d2

M-e2

Under a nitrogen atmosphere, 14H-Bis[1]benzothieno[2,3-a:3',2'-i]carbazole (6.3 g, 16.5 mmol), sodium hydride (containing 40-mass% oil) (0.73 g, 18.2 mmol), and DMF (83 ml) were put into a 300-mL three-necked flask and stirred at 0 degrees C for one hour. Next, the intermediate M-d2 (7.0 g, 16.5 mmol) was put into the reaction mixture and stirred at room temperature for two hours. Water (100mL) was added to the reaction mixture. The deposited solid was purified by silica gel column chromatography to obtain 11 g of a yellow solid. The obtained yellow solid was identified as an intermediate M-e2 by analysis of ASAP-MS (a yield of 85%).

14H-Bis[1]benzothieno[2,3-a:3',2'-i]carbazole was synthesized by the same method described in WO2021/066059A1.

[Formula 315]

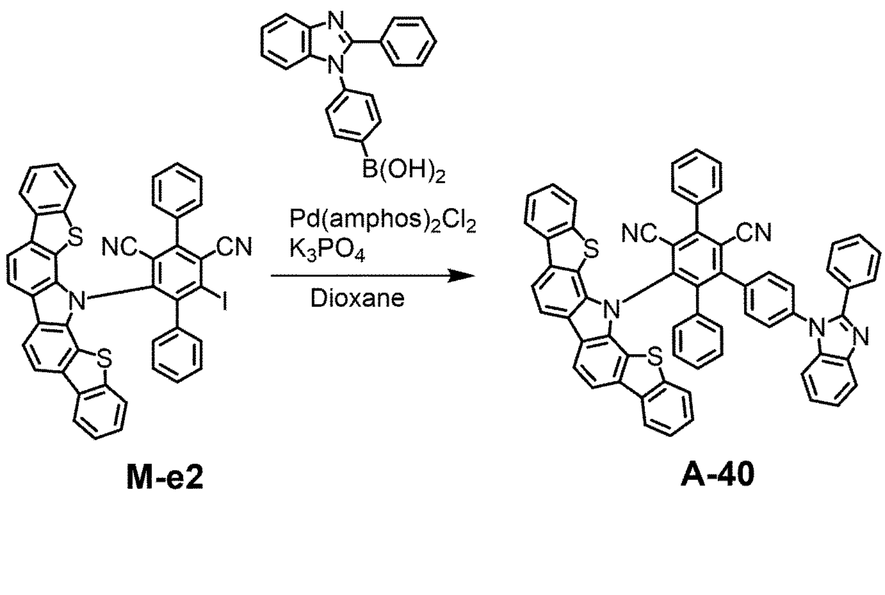


M-e2

A-40

Under a nitrogen atmosphere, the intermediate M-e2 (2.5 g, 3.20 mmol), 4-(2-Phenyl-1H-benzimidazol-1-yl)phenylboronic Acid (1.5 g, 4.80 mmol), bis[di-tert-butyl(4-dimethylaminophenyl)phosphine]dichloropalladium (0.111 g, 0.16 mmol), tripotassium phosphate (1.69g, 8.0 ml), and 1,4-dioxane(16 ml) were put into a 300-ml three-necked flask and stirred at 100 degrees C for five hours. 50 ml of ion-exchange water was added to the stirred reaction solution. The deposited solid was purified by silica gel column chromatography to obtain 2.6 g of a yellow solid. The obtained yellow solid was identified as the compound A-40 by analysis of ASAP-MS (a yield of 88%).

Synthesis of Compound A-41

A synthesis method of a compound A-41 is described below.

[Formula 316]

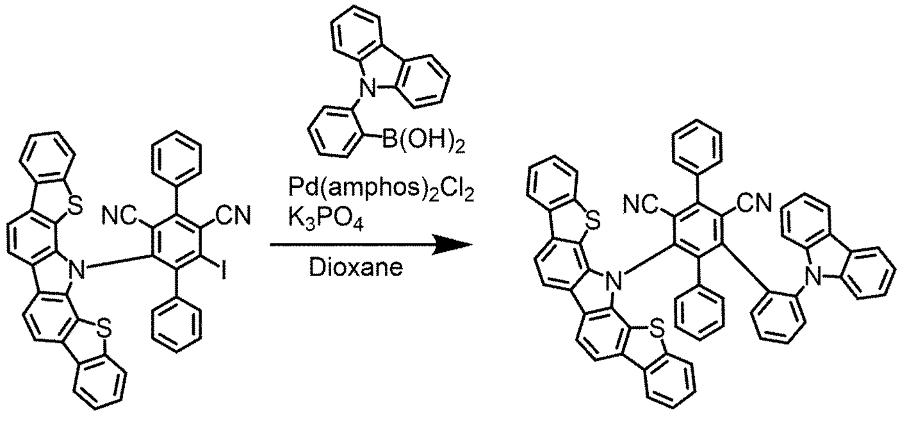


M-e2 A-41

Under a nitrogen atmosphere, the intermediate M-e2 (4.0 g, 5.10 mmol), (2-(9H-carbazol-9-yl)phenyl)boronic acid (2.2 g, 7.66 mmol), bis[di -tert-butyl(4-dimethylaminophenyl)phosphine]dichloropalladium (0.181 g, 0.255 mmol), tripotassium phosphate (2.71 g, 12.76 mmol), and 1,4-dioxane (25.5 ml) were added to a 300-mL three-necked flask and stirred at 100 degrees C for five hours. 100 ml of ion-exchange water was added to the stirred reaction solution. The deposited solid was purified by silica gel column chromatography to obtain 3.4 g of a yellow solid. The obtained yellow solid was identified as the compound A-41 by analysis of ASAP-MS (a yield of 74%).

Synthesis of Compound A-42

A synthesis method of a compound A-42 is described below.

[Formula 317]

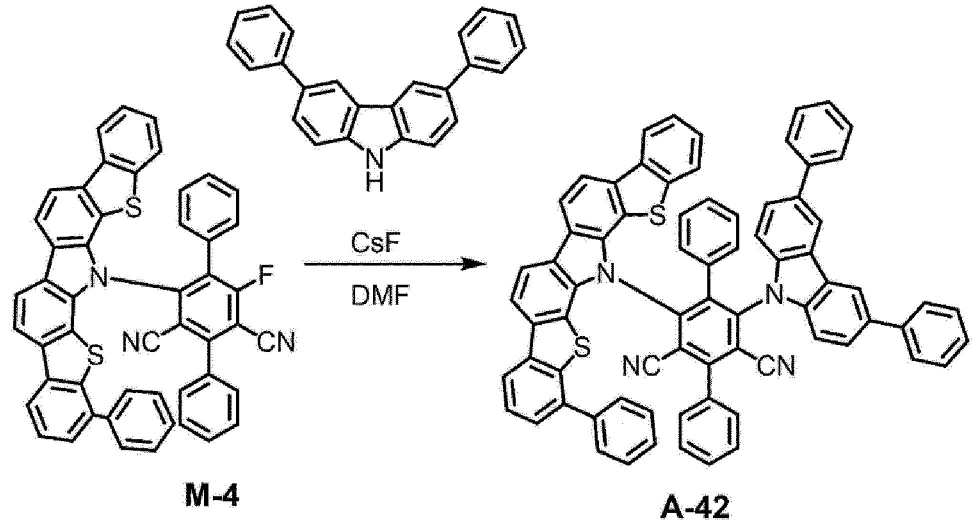


M-4

A-42

Under a nitrogen atmosphere, the intermediate M-4 (1.5 g, 2.0 mmol), 3,6-diphenylcarbazole (1.0 g, 3.0 mmmol), cesium fluoride (0.91 g, 5.98 mmol), and DMF (20 ml) were put into a 100-mL three-necked flask and stirred at room temperature for four hours. After the stirring, 30 ml of ion-exchange water was added to the reaction solution, and the deposited solid was collected by filtration. The obtained solid was purified by silica gel column chromatography to obtain 2.0 g of a yellow solid. The obtained yellow solid was identified as the compound A-42 by analysis of ASAP-MS (a yield of 95%).

Synthesis of Comparative Compound Ref-1

A comparative compound Ref-1 was synthesized in accordance with the description of WO2021/066059 A1.

EXPLANATION OF CODES

1... organic EL device, 2... substrate, 3...anode, 4... cathode, 5...emitting layer, 6... hole injecting layer, 7... hole transporting layer, 8...electron transporting layer, 9... electron injecting layer

**Claims**

**1.** A compound represented by a formula (1) below,

[Formula 1]

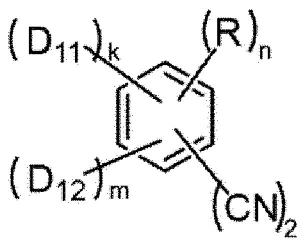

(1)

where, in the formula (1):

CN is a cyano group;
$D_{11}$ and $D_{12}$ are each independently a group represented by a formula (11), (12) or (13) below; at least one $D_{11}$ is a group represented by the formula (12) or (13);
R is each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2

to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a substituted or unsubstituted aralkyl group having 7 to 50 carbon atoms, a group represented by -C(=O)$R_{903}$, a group represented by -COOR$_{909}$, a cyano group, a nitro group, a group represented by -P(=O)($R_{931}$)($R_{932}$), a group represented by - Ge($R_{933}$)($R_{934}$)($R_{935}$), a group represented by -B($R_{936}$)($R_{937}$), a substituted or unsubstituted aryl group having 6 to 50 ring carbon atoms, or a substituted or unsubstituted heterocyclic group having 5 to 50 ring atoms;

at least one R is a substituent, and R as at least one substituent is bonded to a benzene ring in the formula (1) via carbon-carbon bonding;

k is 1 or 2, m is 0, 1, or 2, n is 1, 2, or 3, and k + m + n = 4 is satisfied;

when k is 2, a plurality of $D_{11}$ are mutually the same or different;

when m is 2, a plurality of $D_{12}$ are mutually the same or different; and

when n is 2 or 3, a plurality of R are mutually the same or different,

[Formula 2]

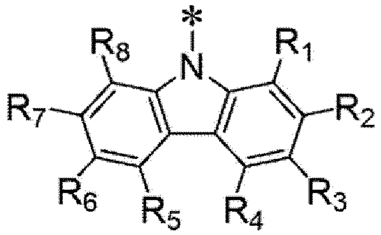

(11)

[Formula 3]

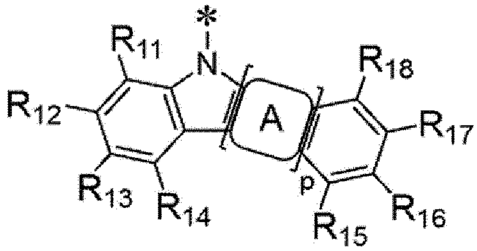

(12)

[Formula 4]

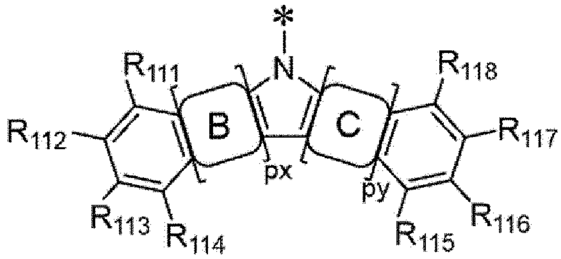

(13)

where, in the formula (12), at least one combination of adjacent two or more of $R_{11}$ to $R_{18}$ are mutually bonded to form a substituted or unsubstituted monocyclic ring, mutually bonded to form a substituted or unsubstituted fused ring, or not mutually bonded;

in the formula (13), at least one combination of adjacent two or more of $R_{111}$ to $R_{118}$ are mutually bonded to form a substituted or unsubstituted monocyclic ring, mutually bonded to form a substituted or unsubstituted fused ring, or not mutually bonded;

$R_1$ to $R_8$ in the formula (11), $R_{11}$ to $R_{18}$ forming neither the substituted or unsubstituted monocyclic ring nor

the substituted or unsubstituted fused ring in the formula (12), and $R_{111}$ to $R_{118}$ forming neither the substituted or unsubstituted monocyclic ring nor the substituted or unsubstituted fused ring in the formula (13) are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{903}$, a group represented by $-COOR_{909}$, a halogen atom, a cyano group, a nitro group, a group represented by $-P(=O)(R_{931})(R_{932})$, a group represented by $-Ge(R_{933})(R_{934})(R_{935})$, a group represented by $-B(R_{936})(R_{937})$, a substituted or unsubstituted aryl group having 6 to 50 ring carbon atoms, or a substituted or unsubstituted heterocyclic group having 5 to 50 ring atoms;

in the formulae (12) and (13):

a ring A, a ring B, and a ring C are each independently any cyclic structure selected from the group consisting of cyclic structures represented by formulae (14) and (15) below;
the ring A, the ring B, and the ring C are fused with adjacent rings at any positions;
p, px and py are each independently 1, 2, 3, or 4;
when p is 2, 3, or 4, a plurality of rings A are mutually the same or different;
when px is 2, 3, or 4, a plurality of rings B are mutually the same or different;
when py is 2, 3, or 4, a plurality of rings C are mutually the same or different; and
at least one $D_{11}$ is a group represented by the formula (12) or (13); p is 4 in the formula (12) when the at least one $D_{11}$ is represented by the formula (12), four rings A includes two cyclic structures each represented by a formula (14) below and two cyclic structures each represented by a formula (15) below; and px and py each are 2 when the at least one $D_{11}$ is represented by the formula (13), two rings B includes one cyclic structure represented by the formula (14) below and one cyclic structure represented by the formula (15) below, and two rings C includes one cyclic structure represented by the formula (14) below and one cyclic structure represented by the formula (15) below; and
each * in the formulae (11) to (13) represents a bonding position benzene ring in the formula (1),

[Formula 5]

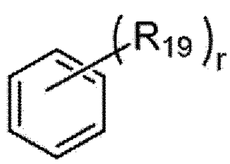

(14)

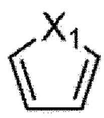

(15)

where in the formula (14):

r is 0, 2, or 4;
a combination of a plurality of $R_{19}$ are mutually bonded to form a substituted or unsubstituted monocyclic ring, mutually bonded to form a substituted or unsubstituted fused ring, or not mutually bonded;
in the formula (15), $X_1$ is a sulfur atom or an oxygen atom.
$R_{19}$ forming neither the substituted or unsubstituted monocyclic ring nor the substituted or unsubstituted fused ring is a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{903}$, a group represented by $-COOR_{909}$, a halogen atom, a cyano group, a nitro group, a group represented by $- P(=O)(R_{931})(R_{932})$, a group represented by $-Ge(R_{933})(R_{934})(R_{935})$, a group represented by $-B(R_{936})(R_{937})$, a substituted or unsubstituted aryl group

having 6 to 50 ring carbon atoms, or a substituted or unsubstituted heterocyclic group having 5 to 50 ring atoms;
a plurality of $R_{19}$ are mutually the same or different;
a plurality of $X_1$ are mutually the same or different;
$D_{11}$ that is a group represented by the formula (13) satisfies at least one of a condition (Pv1), a condition (Pv2), or a condition (Pv3),
condition (Pv1): when k is 2, at least one of $X_1$ in a cyclic structure represented by the formula (15) as the ring B or $X_1$ in a cyclic structure represented by the formula (15) as the ring C is an oxygen atom,
condition (Pv2): when k is 2, two $D_{11}$ are mutually different, and
condition (Pv3): when n is 3, $X_1$ in a cyclic structure represented by the formula (15) as the ring B and $X_1$ in a cyclic structure represented by the formula (15) as the ring C are each independently a sulfur atom or an oxygen atom;

in the formulae, $R_{901}$, $R_{902}$, $R_{903}$, $R_{904}$, $R_{905}$, $R_{906}$, $R_{907}$, $R_{908}$, $R_{909}$, $R_{931}$, $R_{932}$, $R_{933}$, $R_{934}$, $R_{935}$, $R_{936}$, and $R_{937}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{908}$ are present, the plurality of $R_{908}$ are mutually the same or different;
when a plurality of $R_{909}$ are present, the plurality of $R_{909}$ are mutually the same or different;
when a plurality of $R_{931}$ are present, the plurality of $R_{931}$ are mutually the same or different;
when a plurality of $R_{932}$ are present, the plurality of $R_{932}$ are mutually the same or different;
when a plurality of $R_{933}$ are present, the plurality of $R_{933}$ are mutually the same or different;
when a plurality of $R_{934}$ are present, the plurality of $R_{934}$ are mutually the same or different;
when a plurality of $R_{935}$ are present, the plurality of $R_{935}$ are mutually the same or different;
when a plurality of $R_{936}$ are present, the plurality of $R_{936}$ are mutually the same or different; and
when a plurality of $R_{937}$ are present, the plurality of $R_{937}$ are mutually the same or different.

**2.** The compound according to claim 1, wherein

at least one $D_{11}$ is a group represented by a formula (121), (122), or (131),

[Formula 6]

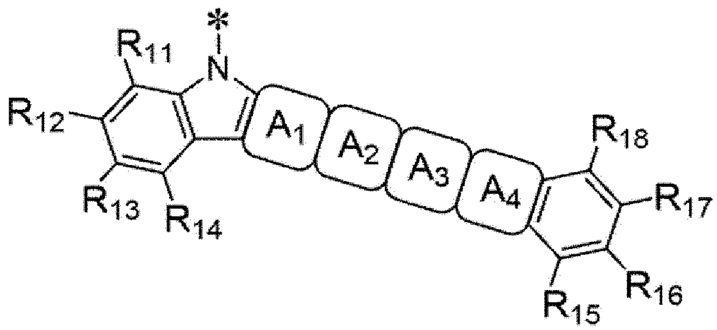

(121)

[Formula 7]

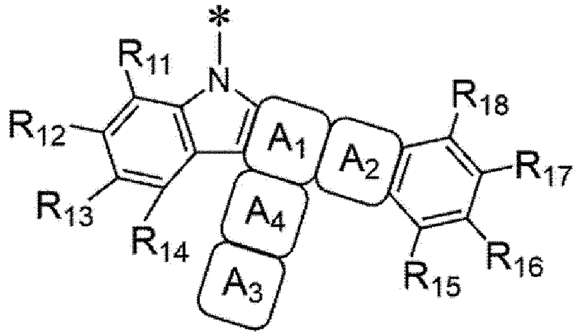

(122)

[Formula 8]

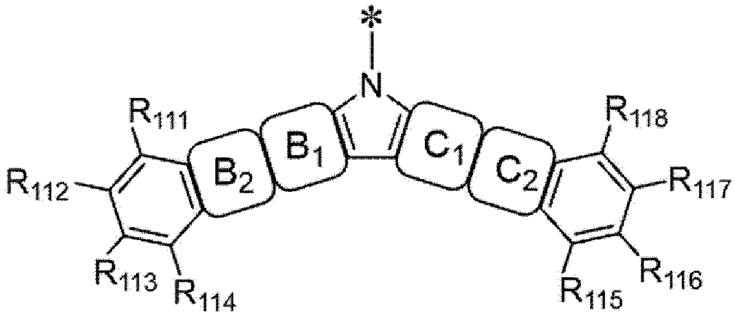

(131)

where in the formulae (121) and (122): $R_{11}$ to $R_{18}$ represent the same as $R_{11}$ to $R_{18}$ in the formula (12);
of a ring $A_1$, a ring $A_2$, a ring $A_3$, and a ring $A_4$, two of the rings $A_1$, $A_2$, $A_3$, and $A_4$ are each a cyclic structure represented by the formula (14) and the remaining two of the rings $A_1$, $A_2$, $A_3$, and $A_4$ are each a cyclic structure represented by the formula (15);
in the formula (131): $R_{111}$ to $R_{118}$ represent the same as $R_{111}$ to $R_{118}$ in the formula (13);
one of a ring $B_1$ and a ring $B_2$ is a cyclic structure represented by the formula (14) and the other of the ring $B_1$ and the ring $B_2$ is a cyclic structure represented by the formula (15); and
one of a ring C1 and a ring C2 is a cyclic structure represented by the formula (14) and the other of the ring C1 and the ring C2 is a cyclic structure represented by the formula (15), and
each * in the formulae (121), (122), and (131) represents a bonding position to the benzene ring in the formula (1).

**3.** The compound according to claim 2, wherein

the ring $A_1$ and the ring $A_3$ are each a cyclic structure represented by the formula (14) and the ring $A_2$ and the ring $A_4$ are each a cyclic structure represented by the formula (15),
the ring $B_1$ is a cyclic structure represented by the formula (14) and the ring $B_2$ is a cyclic structure represented by the formula (15), and
the ring $C_1$ is a cyclic structure represented by the formula (14) and the ring $C_2$ is a cyclic structure represented by the formula (15).

**4.** The compound according to claim 2 or 3, wherein at least one $D_{11}$ is a group represented by the formula (131).

**5.** The compound according to claim 2 or 3, wherein at least one $D_{11}$ is a group represented by a formula (123), (124), (125), or (132) below,

[Formula 9]

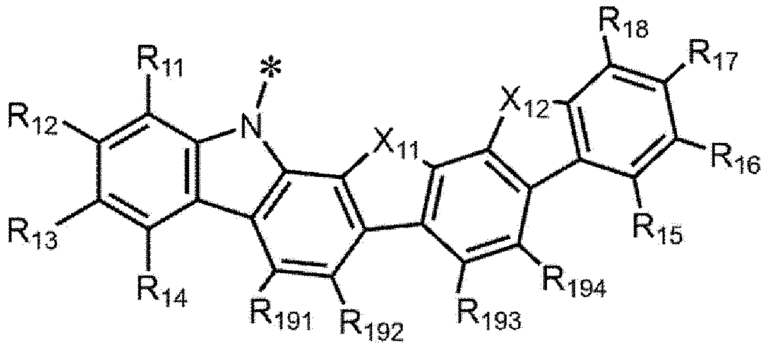

(123)

[Formula 10]

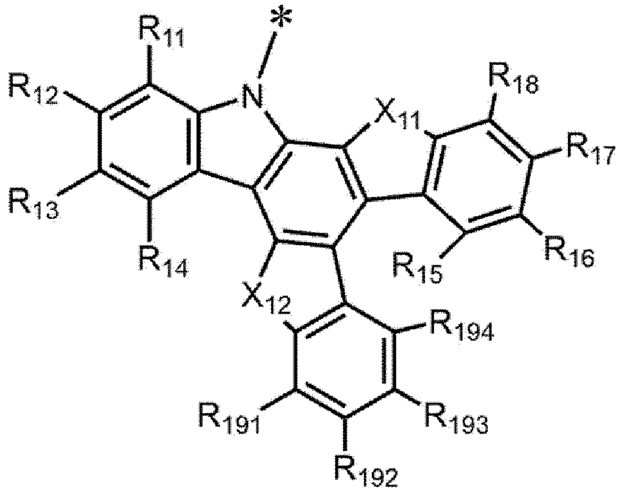

(124)

[Formula 11]

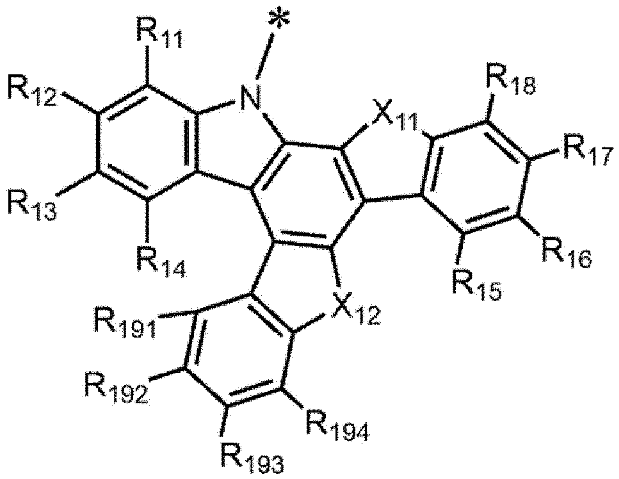

(125)

[Formula 12]

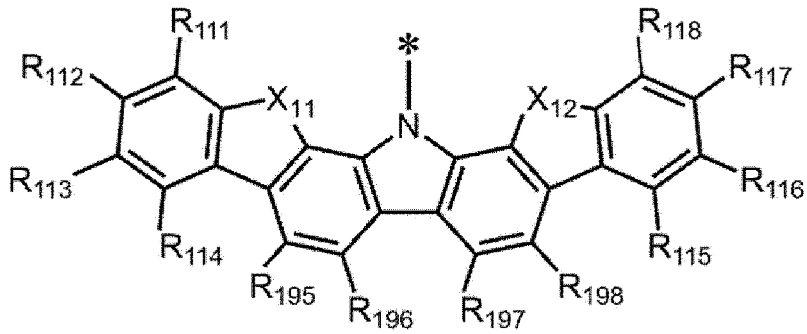

(132)

where in the formulae (123), (124), and (125): $R_{11}$ to $R_{18}$ represent the same as $R_{11}$ to $R_{18}$ in the formula (12); and $R_{191}$ to $R_{194}$ each independently represent the same as $R_{19}$ in the formula (14),

in the formula (132): $R_{111}$ to $R_{118}$ represent the same as $R_{111}$ to $R_{118}$ in the formula (13); and $R_{195}$ to $R_{198}$ each independently represent the same as $R_{19}$ in the formula (14), and
in the formulae (123), (124), (125), and (132): $X_{11}$ and $X_{12}$ each independently represent the same as $X_1$ in the formula (15), and each * represents a bonding position to the benzene ring in the formula (1).

**6.** The compound according to claim 5, wherein $X_{11}$ is a sulfur atom.

**7.** The compound according to claim 5 or 6, wherein at least one $D_{11}$ is a group represented by the formula (132).

**8.** The compound according to any one of claims 1 to 7, wherein $D_{12}$ is a group represented by the formula (11) or (12).

**9.** The compound according to any one of claims 1 to 8, wherein $D_{12}$ is a group represented by the formula (12).

**10.** The compound according to any one of claims 1 to 9, wherein a group represented by the formula (12) is any one group selected from the group consisting of groups represented by formulae (12A), (12B), (12C), (12D), (12E), and (12F) below,

[Formula 13]

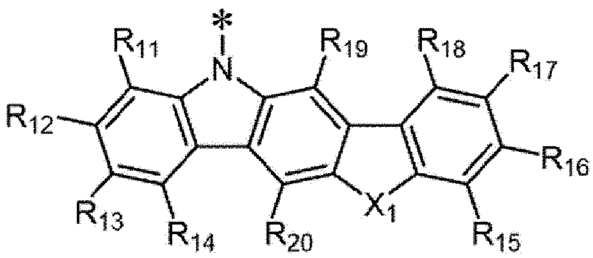

(12A)

[Formula 14]

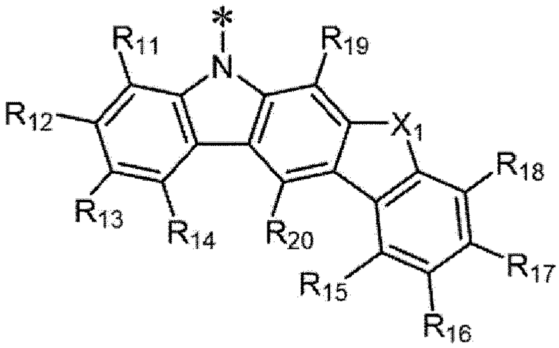

(12B)

[Formula 15]

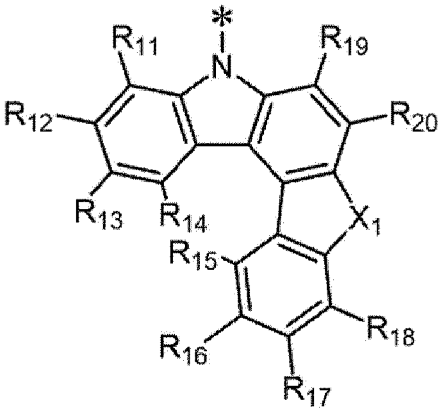

(12C)

[Formula 16]

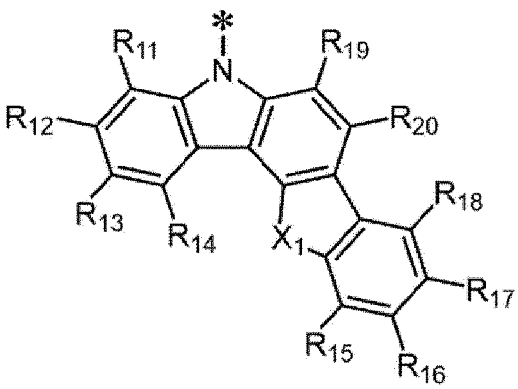

(12D)

[Formula 17]

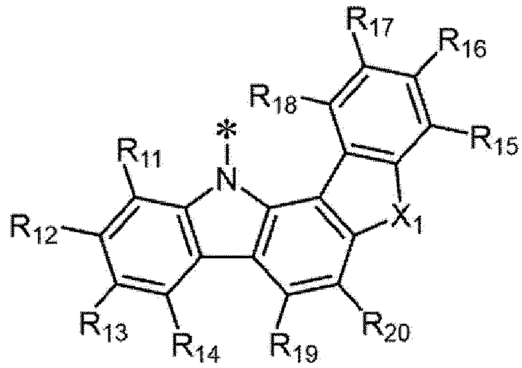

(12E)

[Formula 18]

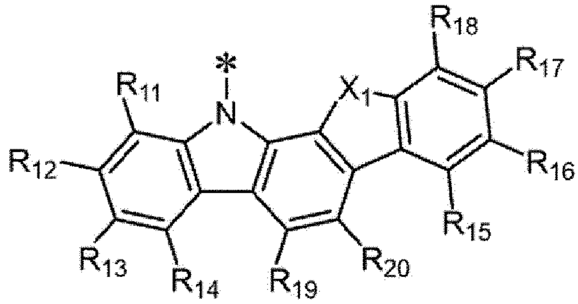

(12F)

where in the formulae (12A), (12B), (12C), (120), (12E), and (12F):

$R_{11}$ to $R_{18}$ each independently represent the same as $R_{11}$ to $R_{18}$ in the formula (12);
$R_{19}$ and $R_{20}$ each independently represent the same as $R_{19}$ in the formula (14);
$X_1$ represents the same as $X_1$ in the formula (15); and
* in the formulae (12A), (12B), (12C), (12D), (12E), and (12F) each represents a bonding position to a benzene ring in the formula (1).

**11.** The compound according to any one of claims 1 to 10, wherein a compound represented by the formula (1) is represented by a formula (110), (120), or (130) below,

[Formula 19]

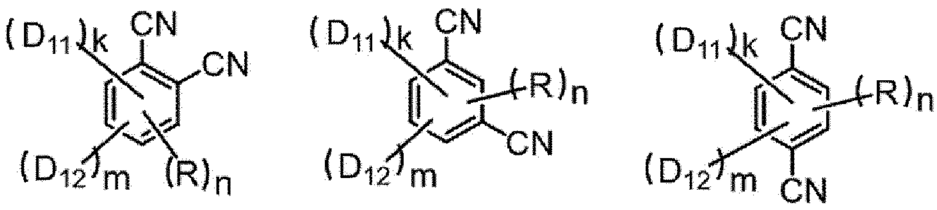

(110) (120) (130)

where in the formulae (110), (120), and (130): $D_{11}$, $D_{12}$, R, k, m, and n respectively represent the same as $D_{11}$, $D_{12}$, R, k, m, and n in the formula (1).

**12.** The compound according to any one of claims 1 to 11, wherein n in the formula (1) is 2.

**13.** The compound according to any one of claims 1 to 11, wherein a compound represented by the formula (1) is represented by a formula (111), (112), or (113) below,

[Formula 20]

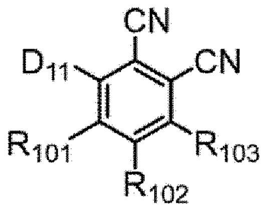
(111)

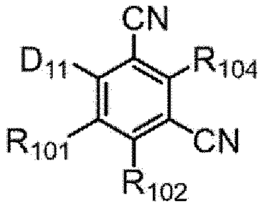
(112)

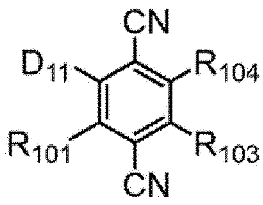
(113)

where in the formulae (111), (112), and (113): $D_{11}$ represents the same as $D_{11}$ in the formula (1); and $R_{101}$ to $R_{104}$ each independently represent the same as R in the formula (1).

**14.** The compound according to any one of claims 1 to 13, wherein R in the formula (1) is each independently a substituted or unsubstituted aryl group having 6 to 14 ring carbon atoms, or a substituted or unsubstituted heterocyclic group having 5 to 14 ring atoms.

**15.** The compound according to any one of claims 1 to 14, wherein R in the formula (1) is each independently a substituted or unsubstituted phenyl group, or a substituted or unsubstituted heterocyclic group having 6 ring atoms.

**16.** The compound according to any one of claims 1 to 15, wherein $R_1$ to $R_8$ in the formula (11), $R_{11}$ to $R_{18}$ represent in the formula (12), $R_{111}$ to $R_{118}$ in the formula (13), and $R_{19}$ in the formula (14) are each independently a hydrogen atom, a substituted or unsubstituted alkyl group having 1 to 50 carbon atoms, a substituted or unsubstituted cycloalkyl group having 3 to 50 ring carbon atoms, or a substituted or unsubstituted aryl group having 6 to 50 ring carbon atoms.

**17.** The compound according to any one of claims 1 to 16, wherein $R_1$ to $R_8$ in the formula (11), $R_{11}$ to $R_{18}$ represent in the formula (12), $R_{111}$ to $R_{118}$ in the formula (13), and $R_{19}$ in the formula (14) are each independently a hydrogen atom,
an unsubstituted alkyl group having 1 to 50 carbon atoms, an unsubstituted cycloalkyl group having 3 to 50 ring carbon atoms, or an unsubstituted arylene group having 6 to 50 ring carbon atoms.

**18.** An organic-electroluminescence-device material comprising the compound according to any one of claims 1 to 17.

**19.** An organic electroluminescence device comprising: an anode, a cathode, and organic layers, wherein
the organic layers comprise the compound according to any one of claims 1 to 17 as a compound M2.

**20.** The organic electroluminescence device according to claim 19, wherein

the organic layers comprise at least one emitting layer, and
the emitting layer comprises the compound M2.

**21.** The organic electroluminescence device according to claim 20, wherein

the emitting layer further comprises a compound M1 in addition to the compound M2,
the compound M1 is a fluorescent compound, and
a lowest singlet energy $S_1(M1)$ of the compound M1 and a lowest singlet energy $S_1(M2)$ of the compound M2 satisfy a relationship of a numerical formula (Numerical Formula 1) below,

$$S_1(M2) > S_1(M1) \qquad \text{...(Numerical Formula 1).}$$

**22.** The organic electroluminescence device according to claim 21, wherein the compound M1 is a compound represented by a formula (D1) below,

[Formula 21]

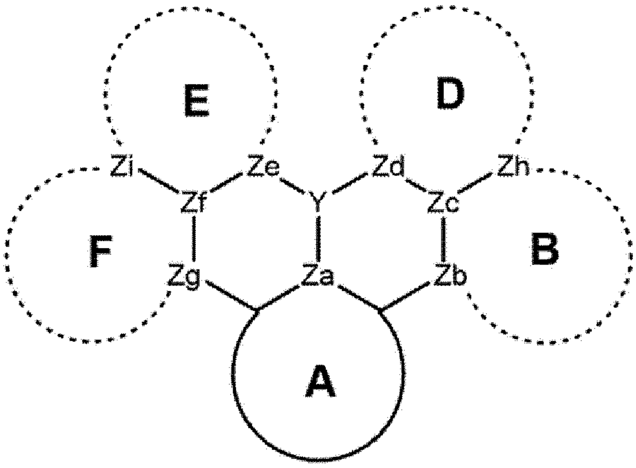

(D1)

where in the formula (D1):

a ring A, a ring B, a ring C, a ring D, a ring E, and a ring F are each independently a cyclic structure selected from the group consisting of a substituted or unsubstituted aryl ring having 6 to 30 ring carbon atoms, and a substituted or unsubstituted heterocyclic ring having 5 to 30 ring atoms;
one of the ring B and the ring D is present or both of the ring B and the ring D are present;
when both of the ring B and the ring D are present, the ring B and the ring D share a bond connecting Zc to Zh;
one of the ring E and the ring F is present or both of the ring E and the ring F are present;
when both of the ring E and the ring F are present, the ring E and the ring F share a bond connecting Zf to Zi;
Za is a nitrogen atom or a carbon atom;
Zb is a nitrogen atom or a carbon atom when the ring B is present, and Zb is an oxygen atom, a sulfur atom, NRb, $C(Rb_1)(Rb_2)$, or $Si(Rb_3)(Rb_4)$ when the ring B is not present;
Zc is a nitrogen atom or a carbon atom;
Zd is a nitrogen atom or a carbon atom when the ring D is present, and Zd is an oxygen atom, a sulfur atom, or NRd when the ring D is not present;
Ze is a nitrogen atom or a carbon atom when the ring E is present, and
Ze is an oxygen atom, a sulfur atom, or NRe when the ring E is not present;
Zf is a nitrogen atom or a carbon atom;
Zg is a nitrogen atom or a carbon atom when the ring F is present, and Zg is an oxygen atom, a sulfur atom, NRg, $C(Rg_1)(Rg_2)$, or $Si(Rg_3)(Rg_4)$ when the ring F is not present;
Zh is a nitrogen atom or a carbon atom;
Zi is a nitrogen atom or a carbon atom;
Y is a boron atom, a phosphorus atom, SiRh, P=O, or P=S;
Rb, $Rb_1$, $Rb_2$, $Rb_3$, $Rb_4$, Rd, Re, Rg, $Rg_1$, $Rg_2$, $Rg_3$, $Rg_4$, and Rh are each independently a hydrogen atom or a substituent;
Rb, $Rb_1$, $Rb_2$, $Rb_3$, $Rb_4$, Rd, Re, Rg, $Rg_1$, $Rg_2$, $Rg_3$, $Rg_4$, and Rh as a substituent are each independently a substituted or unsubstituted aryl group having 6 to 30 ring carbon atoms, a substituted or unsubstituted heterocyclic group having 5 to 30 ring atoms, a substituted or unsubstituted alkyl group having 1 to 30 carbon atoms, a substituted or unsubstituted cycloalkyl group having 3 to 30 ring carbon atoms, a group represented by $-Si(R_{911})(R_{912})(R_{913})$, a group represented by $-O-(R_{914})$, a group represented by $-S-(R_{915})$, or a group represented by $-N(R_{916})(R_{917})$;
a bond between Y and Za, a bond between Y and Zd, and a bond between Y and Ze are each a single bond;
in the compound M1, $R_{911}$ to $R_{917}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
when a plurality of $R_{911}$ are present, the plurality of $R_{911}$ are mutually the same or different;
when a plurality of $R_{912}$ are present, the plurality of $R_{912}$ are mutually the same or different;
when a plurality of $R_{913}$ are present, the plurality of $R_{913}$ are mutually the same or different;
when a plurality of $R_{914}$ are present, the plurality of $R_{914}$ are mutually the same or different;

when a plurality of $R_{915}$ are present, the plurality of $R_{915}$ are mutually the same or different;
when a plurality of $R_{916}$ are present, the plurality of $R_{916}$ are mutually the same or different; and
when a plurality of $R_{917}$ are present, the plurality of $R_{917}$ are mutually the same or different.

**23.** The organic electroluminescence device according to claim 21, wherein the compound M1 is a compound represented by a formula (20) below,

[Formula 22]

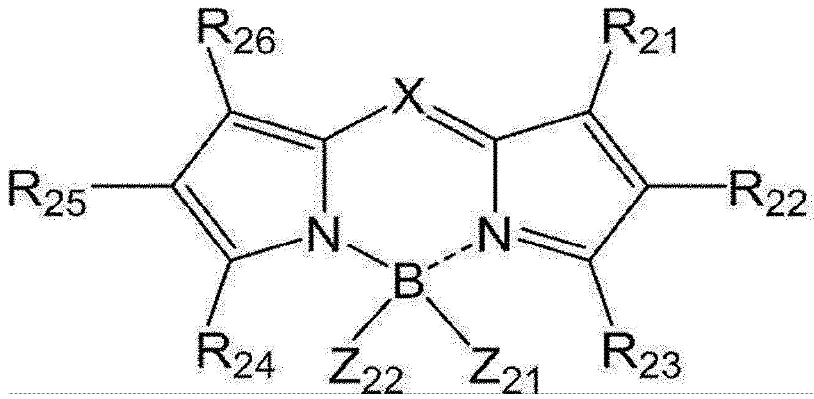

(20)

where in the formula (20):

X is a nitrogen atom or a carbon atom bonded to Y;
Y is a hydrogen atom or a substituent;
$R_{21}$ to $R_{26}$ are each independently a hydrogen atom or a substituent, or at least one combination of a combination of $R_{21}$ and $R_{22}$, a combination of $R_{22}$ and $R_{23}$, a combination of $R_{24}$ and $R_{25}$, or a combination of $R_{25}$ and $R_{26}$ are mutually bonded to form a ring;
Y and $R_{21}$ to $R_{26}$ as a substituent are each independently selected from the group consisting of a substituted or unsubstituted alkyl group having 1 to 30 carbon atoms, a substituted or unsubstituted alkyl halide group having 1 to 30 carbon atoms, a substituted or unsubstituted cycloalkyl group having 3 to 30 ring carbon atoms, a substituted or unsubstituted aryl group having 6 to 30 ring carbon atoms, a substituted or unsubstituted alkoxy group having 1 to 30 carbon atoms, a substituted or unsubstituted alkoxy halide group having 1 to 30 carbon atoms, a substituted or unsubstituted alkylthio group having 1 to 30 carbon atoms, a substituted or unsubstituted aryloxy group having 6 to 30 ring carbon atoms, a substituted or unsubstituted arylthio group having 6 to 30 ring carbon atoms, a substituted or unsubstituted alkenyl group having 2 to 30 carbon atoms, a substituted or unsubstituted aralkyl group having 7 to 30 carbon atoms, a substituted or unsubstituted heteroaryl group having 5 to 30 ring atoms, a halogen atom, a carboxy group, a substituted or unsubstituted ester group, a substituted or unsubstituted carbamoyl group, a substituted or unsubstituted amino group, a nitro group, a cyano group, a substituted or unsubstituted silyl group, and a substituted or unsubstituted siloxanyl group;
$Z_{21}$ and $Z_{22}$ are each independently a substituent, or $Z_{21}$ and $Z_{22}$ are mutually bonded to form a ring; and
$Z_{21}$ and $Z_{22}$ as a substituent are each independently selected from the group consisting of a halogen atom, a substituted or unsubstituted alkyl group having 1 to 30 carbon atoms, a substituted or unsubstituted alkyl halide group having 1 to 30 carbon atoms, a substituted or unsubstituted aryl group having 6 to 30 ring carbon atoms, a substituted or unsubstituted alkoxy group having 1 to 30 carbon atoms, a substituted or unsubstituted alkoxy halide group having 1 to 30 carbon atoms, and a substituted or unsubstituted aryloxy group having 6 to 30 ring carbon atoms.

**24.** The organic electroluminescence device according to any one of claims 20 to 23, wherein

the emitting layer further comprises a compound M3 in addition to the compound M2, and
a lowest singlet energy $S_1(M2)$ of the compound M2 and a lowest singlet energy $S_1(M3)$ of the compound M3 satisfy a relationship of a numerical formula (Numerical Formula 2) below,

$$S_1(M3) > S_1(M2) \quad ...\text{(Numerical Formula 2)}.$$

**25.** An electronic device comprising the organic electroluminescence device according to any one of claims 19 to 24.

FIG.1

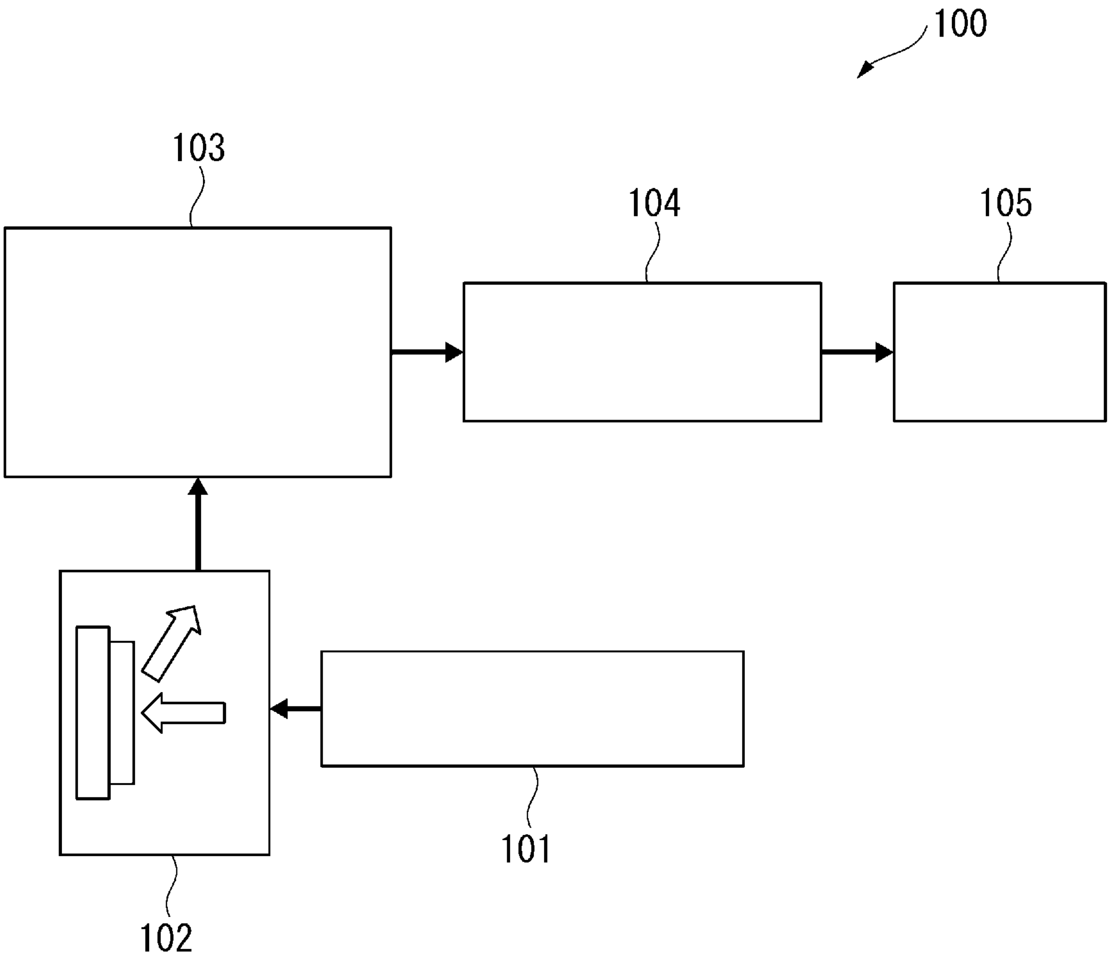

FIG. 2

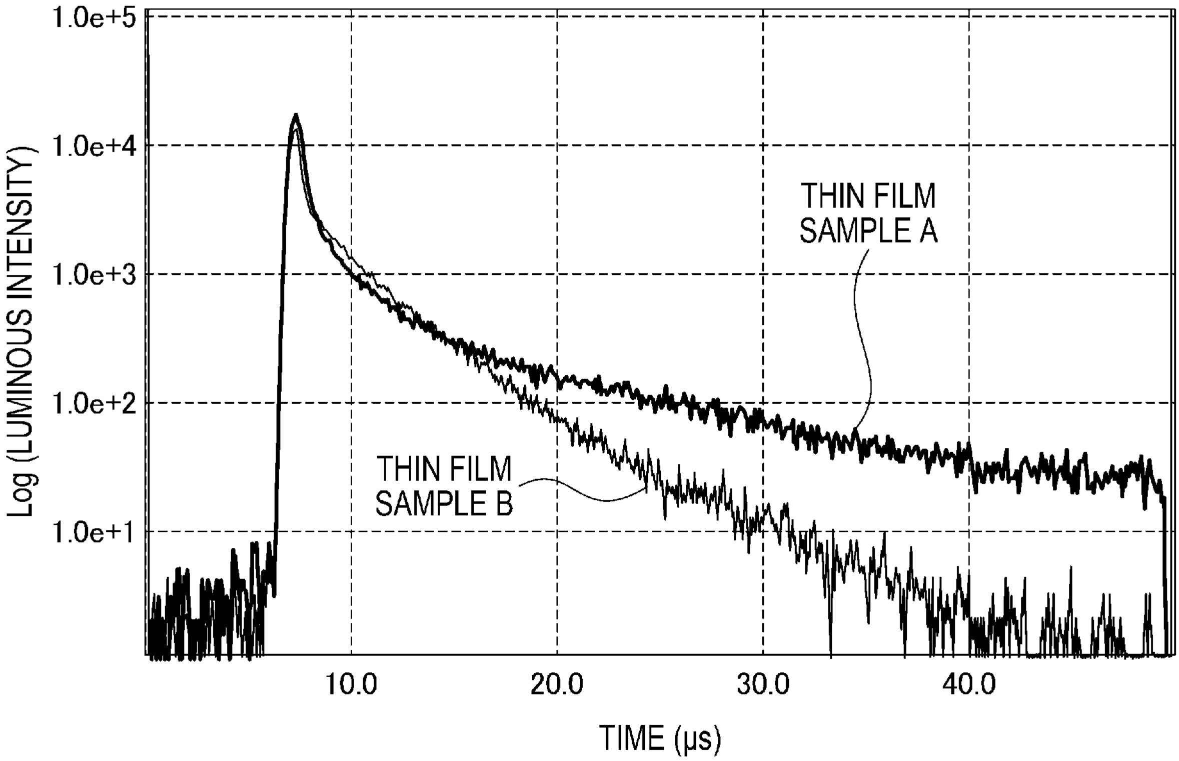
1.0e+5
1.0e+4
1.0e+3
1.0e+2
1.0e+1
Log (LUMINOUS INTENSITY)
10.0
20.0
30.0
40.0
TIME (μs)
THIN FILM SAMPLE A
THIN FILM SAMPLE B

FIG.3

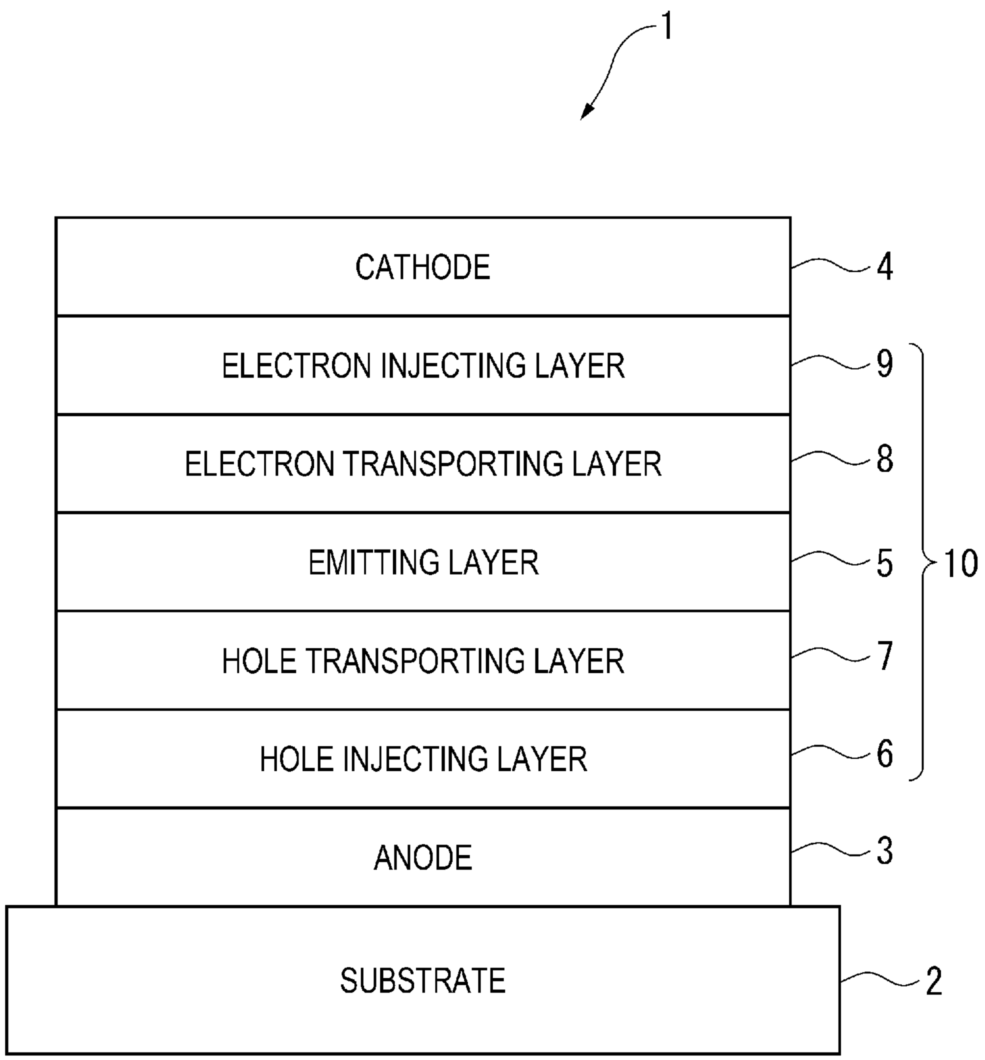
1
CATHODE
4
ELECTRON INJECTING LAYER
9
ELECTRON TRANSPORTING LAYER
8
EMITTING LAYER
5
10
HOLE TRANSPORTING LAYER
7
HOLE INJECTING LAYER
6
ANODE
3
SUBSTRATE
2

FIG.4

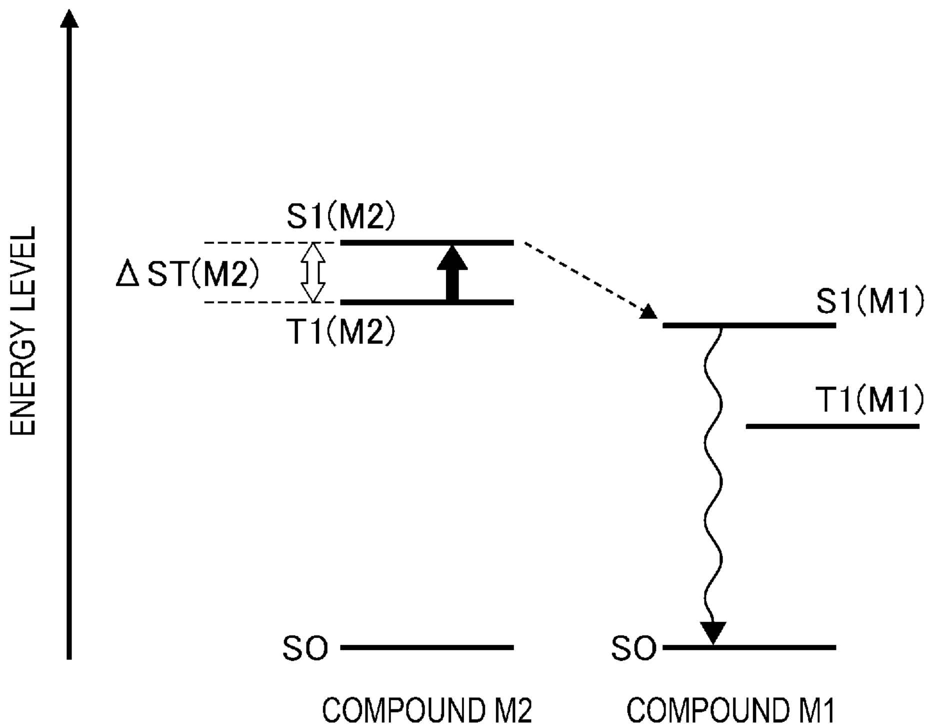
ENERGY LEVEL
S1(M2)
ΔST(M2)
T1(M2)
S1(M1)
T1(M1)
SO
SO
COMPOUND M2
COMPOUND M1

# FIG. 5

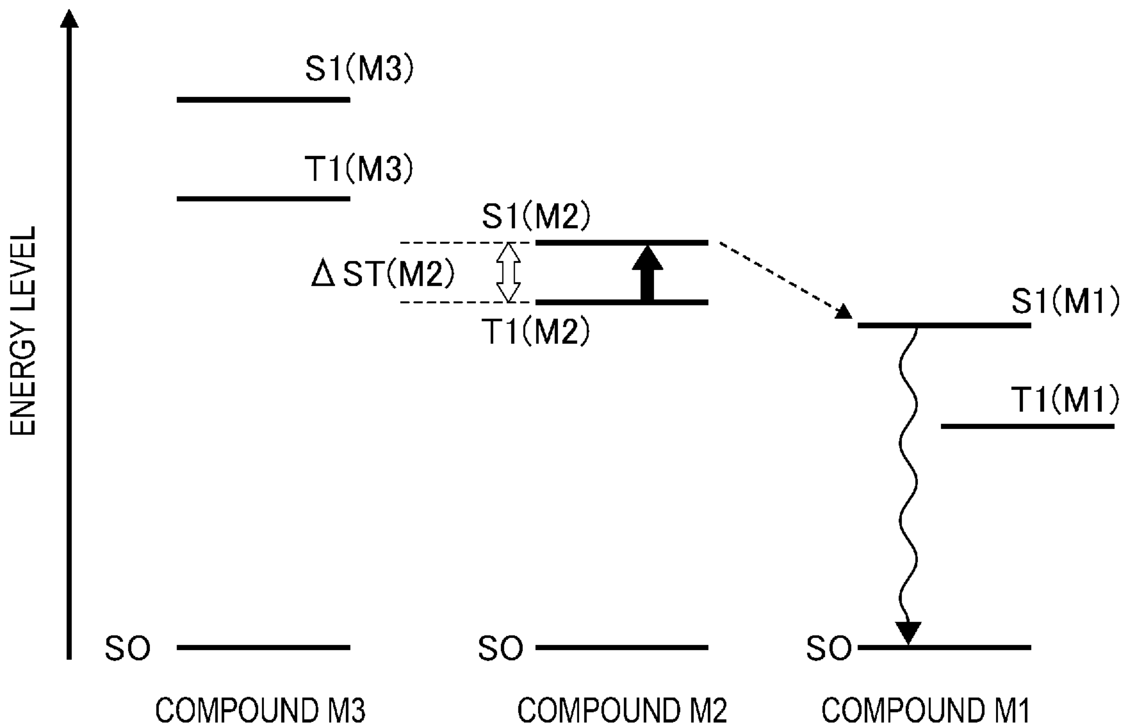
ENERGY LEVEL
S1(M3)
T1(M3)
S1(M2)
ΔST(M2)
T1(M2)
S1(M1)
T1(M1)
SO
SO
SO
COMPOUND M3
COMPOUND M2
COMPOUND M1

FIG.6

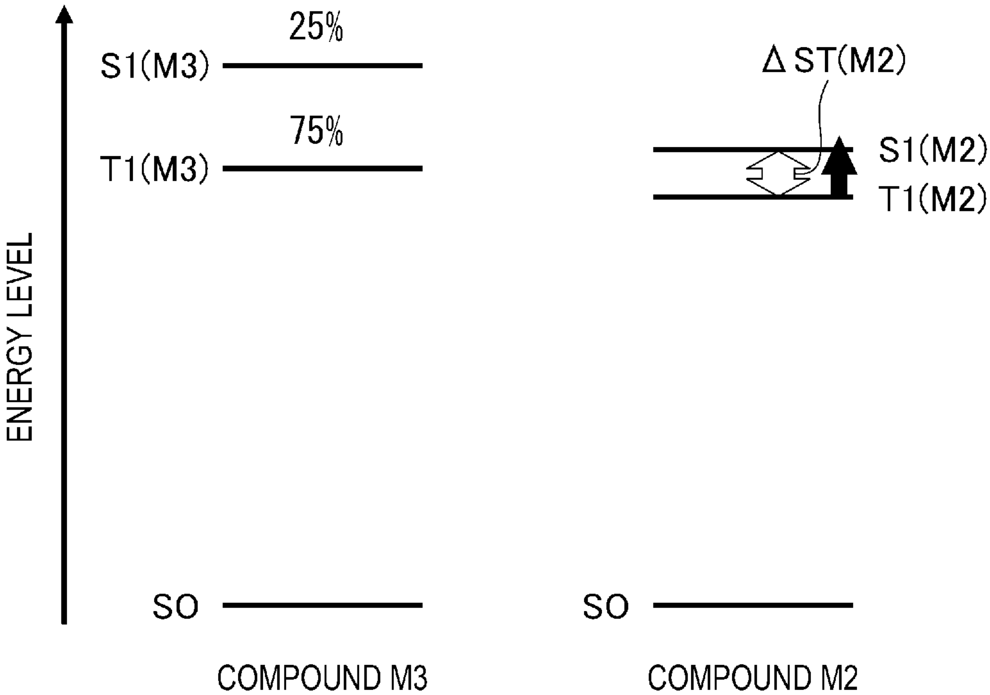
25%
S1(M3)
75%
T1(M3)
ENERGY LEVEL
SO
COMPOUND M3
ΔST(M2)
S1(M2)
T1(M2)
SO
COMPOUND M2

**INTERNATIONAL SEARCH REPORT**

International application No.
**PCT/JP2022/023247**

**A. CLASSIFICATION OF SUBJECT MATTER**

***C07D 495/14***(2006.01)i; ***C07D 519/00***(2006.01)i; ***C09K 11/06***(2006.01)i; ***H01L 51/50***(2006.01)i
FI: C07D495/14 A CSP; C07D519/00 301; C09K11/06 660; C09K11/06 690; H05B33/14 B

According to International Patent Classification (IPC) or to both national classification and IPC

**B. FIELDS SEARCHED**

Minimum documentation searched (classification system followed by classification symbols)

C07D495/14; C07D519/00; C09K11/06; H01L51/50

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2021/066059 A1 (IDEMITSU KOSAN CO., LTD.) 08 April 2021 (2021-04-08) claims, paragraphs [0114]-[0170], [0184]-[0200], [0218]-[0258], examples | 1-25 |
| A | WO 2014/208698 A1 (IDEMITSU KOSAN CO., LTD.) 31 December 2014 (2014-12-31) entire text | 1-25 |
| A | WO 2019/195104 A1 (KYULUX, INC.) 10 October 2019 (2019-10-10) entire text | 1-25 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 August 2022** | **23 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/023247**

| Patent document cited in search report | Publication date (day/month/year) | Patent family member(s) | Publication date (day/month/year) |
|---|---|---|---|
| WO 2021/066059 A1 | 08 April 2021 | (Family: none) | |
| WO 2014/208698 A1 | 31 December 2014 | US 2016/0130225 A1<br>EP 3015457 A1<br>CN 105209434 A<br>KR 10-2016-0023655 A | |
| WO 2019/195104 A1 | 10 October 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014208698 A
- WO 2019195104 A
- WO 2019190235 A
- WO 2021066059 A
- WO 2021066059 A1

### Non-patent literature cited in the description


- Yuki Hando-tai no Debaisu Bussei. Device Physics of Organic Semiconductors. Kodansha, 01 April 2012, 261-268
- Yuki Hando-tai no Debaisu Bussei. Device Physics of Organic Semiconductors. Kodansha, 261-268
- *Nature,* 2012, vol. 492, 234-238
- **MORRIS et al.** *J. Phys. Chem.,* 1976, vol. 80, 969